(19) Europäisches Patentamt
European Patent Office
Office européen des brevets

(11) EP 4 707 280 A1

(12) **EUROPEAN PATENT APPLICATION**
published in accordance with Art. 153(4) EPC

(43) Date of publication:
**11.03.2026 Bulletin 2026/11**

(21) Application number: **24800121.6**

(22) Date of filing: **01.05.2024**

(51) International Patent Classification (IPC):
***C07D 493/08*** *(2006.01)* ***A01N 43/90*** *(2006.01)*
***A01P 13/00*** *(2006.01)*

(52) Cooperative Patent Classification (CPC):
**A01N 43/90; A01P 13/00; C07D 493/08**

(86) International application number:
**PCT/JP2024/016782**

(87) International publication number:
**WO 2024/228390 (07.11.2024 Gazette 2024/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.05.2023 JP 2023076081**
**29.03.2024 JP 2024057466**

(71) Applicants:
- **HOKKO CHEMICAL INDUSTRY CO., LTD.**
  **Tokyo 103-8341 (JP)**
- **Meiji University**
  **Tokyo 101-8301 (JP)**

(72) Inventors:
- **OOTAKA, Akihito**
  **Atsugi-shi, Kanagawa 243-0023 (JP)**
- **HIRANO, Tatsuya**
  **Atsugi-shi, Kanagawa 243-0023 (JP)**
- **KOGURE, Yuki**
  **Atsugi-shi, Kanagawa 243-0023 (JP)**
- **MITANI, Yusuke**
  **Atsugi-shi, Kanagawa 243-0023 (JP)**
- **OGAWA, Narihito**
  **Kawasaki-shi, Kanagawa 214-8571 (JP)**

(74) Representative: **Reitstötter Kinzebach Patentanwälte**
**Sternwartstraße 4**
**81679 München (DE)**

(54) **1,4-CINEOLE DERIVATIVE AND INTERMEDIATE THEREOF, HERBICIDE CONTAINING SAID DERIVATIVE AS ACTIVE INGREDIENT, METHOD FOR USING HERBICIDE, AND METHOD FOR PREPARING AGROCHEMICAL COMPOSITION**

(57) The present invention provides a 1,4-cineole derivative represented by the following general formula (1), (1'), (2), (2'), (3) or (3') which exhibit an excellent herbicidal activity, an intermediate thereof, and a herbicide containing the derivative as an active ingredient. In the general formulae (1), (1'), (2), (2'), (3) and (3'), $R^1$ to $R^3$, X and W each represent a predetermined substituent as described in the specification:

[Chem. 1]

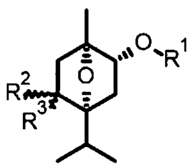
(1)

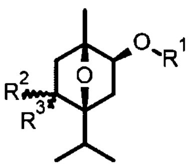
(1')

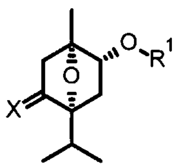
(2)

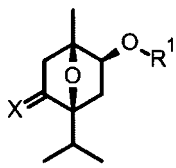
(2')

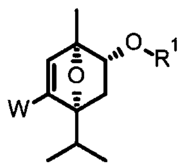
(3)

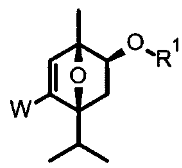
(3')

EP 4 707 280 A1

## Description

### TECHNICAL FIELD

The present invention relates to a 1,4-cineole derivative and an intermediate thereof, a herbicide containing the derivative as an active ingredient and having an extremely excellent control effect for a harmful weed in agricultural and horticultural fields and non-agricultural land, a method for using the herbicide, and a method for preparing an agrochemical composition.

### BACKGROUND ART

The use of a herbicide is essential for protecting useful crops such as rice, wheat, corn, soybeans, cotton, and beets from weeds and increasing a yield.
In recent years, in cultivated land where useful crops and weeds coexist, there has been a demand for a selective herbicide that can selectively kill only weeds without causing phytotoxicity to the crops. From the viewpoints of preventing environmental pollution and reducing the economic costs involved in transportation and spraying, there is a need for an agent that exhibits a high herbicidal effect at the lowest possible dosage.
Incidentally, a 1,4-cineole derivatives exhibiting herbicidal activity similar to that of the present invention include those related to a cinmethylin derivative reported in Patent Literature 1 and Non Patent Literature 1, and those reported in Non Patent Literature 2 that have a 1,4-cineole ring, but there is no compound having a substituent at C5 position of the 1,4-cineole ring (where a carbon atom to which a methyl group in the 1,4-cineol ring is bonded is defined as C1 position).

### CITATION LIST

### PATENT LITERATURE

Patent Literature 1: EP0081893A

### NON-PATENT LITERATURE

Non Patent Literature 1: Journal of Agricultural and Food Chemistry, Vol. 58, 2010, pp. 10147-10155
Non Patent Literature 2: Journal of Pesticide Science, Vol. 48, 2023, pp. 11-16

### SUMMARY OF INVENTION

### TECHNICAL PROBLEM

An object of the present invention is to provide a 1,4-cineole derivative and an intermediate thereof, and a herbicide having excellent herbicidal activity.
An object of the present invention is to provide a method for using a herbicide and a method for preparing an agrochemical composition.

### SOLUTION TO PROBLEM

As a result of intensive studies to solve the above problems, the inventors of the present invention have found that a 1,4-cineole derivative represented by the following general formula (1), (1'), (2), (2'), (3) or (3') exhibits excellent herbicidal activity, and have completed the present invention.
That is, the inventors of the present invention have found that the above problems can be solved by the following configuration.

[1] A 1,4-cineole derivative represented by the following general formula (1), (1'), (2), (2'), (3) or (3') (hereinafter may be referred to as "compound of the present invention" in the present description).

[Chem. 1]

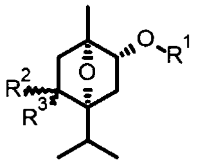
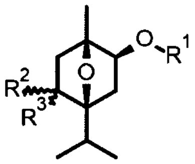
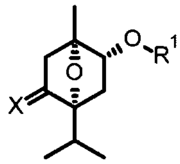
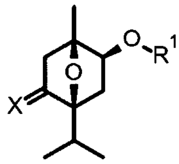
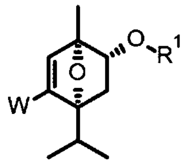
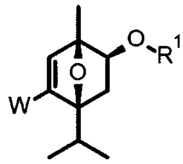

(1) (1') (2) (2') (3) (3')

(In the general formulae (1), (1'), (2), (2'), (3), and (3'), $R^1$'s each independently represent a C1-C6 alkyl group, a C1-C6 haloalkyl group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a benzoyl C1-C6 alkyl group (which may be mono-substituted or poly-substituted with a halogen atom, a C1-C6 alkyl group, or a C1-C6 haloalkyl group), or a C1-C6 alkoxy C1-C6 alkoxy C1-C6 alkyl group.

$R^2$'s and $R^3$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyl group, a C2-C6 alkenyloxy group, a C2-C6 alkynyl group, a C2-C6 alkynyloxy group, a C1-C6 alkylcarbonyloxy group, a C1-C6 alkoxycarbonyloxy group, a C1-C6 alkylthiocarbonyloxy group, a C1-C6 alkylthiothiocarbonyloxy group, a C1-C6 alkylsulfonyloxy group, a C1-C6 haloalkylsulfonyloxy group, a C3-C6 cycloalkylsulfonyloxy group, an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), an aminosulfonyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), and at least one of $R^2$ and $R^3$ is a substituent other than a hydrogen atom. The two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents.

X's each independently represent an oxygen atom, a sulfur atom, $CR^4R^5$ or $NR^6$.
$R^4$ and $R^5$ each independently represent a hydrogen atom or a C1-C6 alkyl group.
$R^6$ represents a hydrogen atom, a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group).
W's each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkoxy group, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkylsulfonyloxy group, a C1-C6 haloalkylsulfonyloxy group, a C3-C6 cycloalkylsulfonyloxy group, or an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group).)

[2] The 1,4-cineole derivative according to [1], in which

$R^1$'s each independently represent a C1-C6 alkyl group, a C1-C6 haloalkyl group, a C2-C6 alkenyl group, a C2-C6 alkynyl group, C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a benzoyl C1-C6 alkyl group (which may be mono-substituted or poly-substituted with a halogen atom, a C1-C6 alkyl group, or a C1-C6 haloalkyl group), or a C1-C6 alkoxy C1-C6 alkoxy C1-C6 alkyl group,
$R^2$'s and $R^3$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl

group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyloxy group, a C2-C6 alkynyloxy group, a C1-C6 alkylcarbonyloxy group, a C1-C6 alkylthiothiocarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), at least one of $R^2$ and $R^3$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

X's each independently represent an oxygen atom, a sulfur atom, $CR^4R^5$, or $NR^6$,

$R^4$ and $R^5$ each independently represent a hydrogen atom or a C1-C6 alkyl group,

$R^6$ represents a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and

W's each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C1-C6 alkylsulfonyloxy group, or a C1-C6 haloalkylsulfonyloxy group.

[3] The 1,4-cineole derivative according to [1], in which

$R^{1'}$s each independently represent a C1-C6 alkyl group, a C1-C6 haloalkyl group, a C2-C6 alkenyl group, a C2-C6 alkynyl group, a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), or a C1-C6 alkoxy C1-C6 alkoxy C1-C6 alkyl group,

$R^{2'}$s and $R^{3'}$s each independently represent a hydrogen atom, a halogen atom, a hydroxyl group, a C1-C6 alkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6

alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a C2-C6 alkenyloxy group, a C2-C6 alkynyloxy group, a C1-C6 alkylcarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), at least one of $R^2$ and $R^3$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

X's each independently represent an oxygen atom, $CR^4R^5$, or $NR^6$,

$R^4$ and $R^5$ each independently represent a hydrogen atom,

$R^6$ represents a hydroxyl group, and

W's each independently represent a hydrogen atom, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), or a C1-C6 haloalkylsulfonyloxy group.

[4] A synthetic intermediate represented by the following general formula (1a), (1a'), (2a), (2a'), (3a), or (3a'), which is useful for producing the 1,4-cineole derivative represented by the general formula (1), (1'), (2), (2'), (3), or (3') according to any one of [1] to [3]:

[Chem. 2]

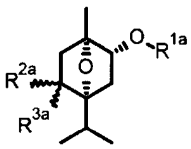
(1a)

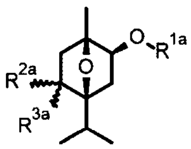
(1a')

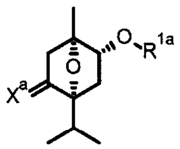
(2a)

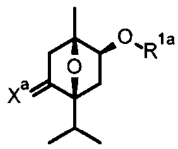
(2a')

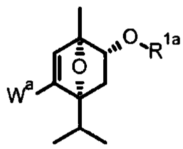
(3a)

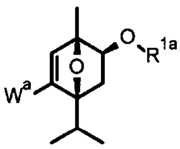
(3a')

(in the general formulae (1a), (1a'), (2a), (2a'), (3a), and (3a'),

$R^{1a}$'s each independently represent a hydrogen atom or a tri-C1-C6 alkylsilyl group which may be the same or different,

$R^{2a}$'s and $R^{3a}$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyl group, a C2-C6 alkenyloxy group, a C2-C6 alkynyl group, a C2-C6 alkynyloxy group, a C1-C6 alkylcarbonyloxy group, a C1-C6 alkoxycarbonyloxy group, a C1-C6 alkylthiocarbonyloxy group, a C1-C6 alkylthiothiocarbonyloxy group, a C1-C6 alkylsulfonyloxy group, a

C1-C6 haloalkylsulfonyloxy group, a C3-C6 cycloalkylsulfonyloxy group, an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), an aminosulfonyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), and at least one of $R^2$ and $R^3$ is a substituent other than a hydrogen atom, the two adjacent substituents, $R^{2a}$ and $R^{3a}$, together with a carbon atom to which $R^{2a}$ and $R^{3a}$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,
$X^a$'s each independently represent an oxygen atom, a sulfur atom, $CR^{4a}R^{5a}$ or $NR^{6a}$,
$R^{4a}$ and $R^{5a}$ each independently represent a hydrogen atom or a C1-C6 alkyl group,
$R^{6a}$ represents a hydrogen atom, a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and
$W^a$'s each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkoxy group, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkylsulfonyloxy group, a C1-C6 haloalkylsulfonyloxy group, a C3-C6 cycloalkylsulfonyloxy group, or an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group)).

[5] The synthetic intermediate according to [4], in which

$R^{1a}$'s each independently represent a hydrogen atom or a tri-C1-C6 alkylsilyl group which may be same or different,
$R^{2a}$'s and $R^{3a}$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which

may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyloxy group, a C2-C6 alkynyloxy group, a C1-C6 alkylcarbonyloxy group, a C1-C6 alkylthiothiocarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), at least one of $R^{2a}$ and $R^{3a}$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

$X^a$'s each independently represent an oxygen atom, a sulfur atom, $CR^4R^5$, or $NR^6$,

$R^{4a}$ and $R^{5a}$ each independently represent a hydrogen atom or a C1-C6 alkyl group,

$R^{6a}$ represents a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and

$W^a$'s each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C1-C6 alkylsulfonyloxy group, or a C1-C6 haloalkylsulfonyloxy group.

[6] The synthetic intermediate according to [4], in which

$R^{1a}$'s each independently represent a hydrogen atom or a tri-C1-C6 alkylsilyl group which may be same or different,

$R^{2a}$'s and $R^{3a}$'s each independently represent a hydrogen atom, a halogen atom, a hydroxyl group, a C1-C6 alkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a C2-C6 alkenyloxy group, a C2-C6 alkynyloxy group, a C1-C6 alkylcarbonyloxy group, a C1-C6 haloalkylsulfonyloxy group or a carbamoyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered

ring, a 5-membered ring, or a 6-membered ring), at least one of $R^{2a}$ and $R^{3a}$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

$X^a$'s each independently represent an oxygen atom, $CR^4R^5$, or $NR^6$,

$R^{4a}$ and $R^{5a}$ each independently represent a hydrogen atom,

$R^{6a}$ represents a hydroxyl group, and

$W^a$'s each independently represent a hydrogen atom, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), or a C1-C6 haloalkylsulfonyloxy group.

[7] A herbicide containing:
the 1,4-cineole derivative according to any one of [1] to [3] as an active ingredient.
[8] The herbicide according to [7], which is for use in agricultural land, pastureland, lawn, or non-agricultural land.
[9] A method for using the herbicide according to [7], the method including:
applying an effective amount of the 1,4-cineole derivative to at least one selected from a stem and leaf of a weed, soil, and a water surface.
[10] A method for preparing an agrochemical composition, the method including:
a step of mixing the herbicide according to [7] with at least one selected from a diluent and a surfactant.

ADVANTAGEOUS EFFECTS OF INVENTION

According to the present invention, a 1,4-cineole derivative and an intermediate thereof, and a herbicide having excellent herbicidal activity can be provided.
The novel 1,4-cineole derivative of the present invention that is represented by the above general formula (1), (1'), (2), (2'), (3) or (3') exhibits excellent herbicidal activity.
According to the present invention, a method for using a herbicide and a method for preparing an agrochemical composition can be provided.

DESCRIPTION OF EMBODIMENTS

A 1,4-cineole derivative related to a compound of the present invention, an intermediate of the present invention, and a herbicide containing the derivative as an active ingredient will now be described in detail.
The present invention relates to a 1,4-cineole derivative represented by the following general formula (1), (1'), (2), (2'), (3) or (3').

[Chem. 3]

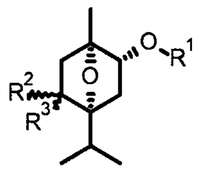
(1)
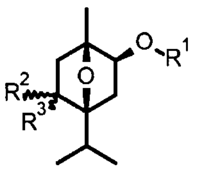
(1')
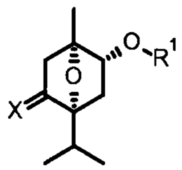
(2)
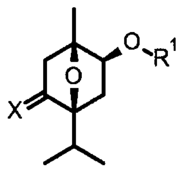
(2')
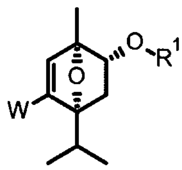
(3)
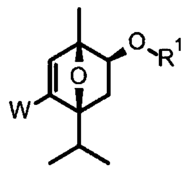
(3')

(In the general formulae (1), (1'), (2), (2'), (3), and (3'), $R^1$'s each independently represent a C1-C6 alkyl group, a C1-C6 haloalkyl group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with

a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a benzoyl C1-C6 alkyl group (which may be mono-substituted or poly-substituted with a halogen atom, a C1-C6 alkyl group, or a C1-C6 haloalkyl group), or a C1-C6 alkoxy C1-C6 alkoxy C1-C6 alkyl group.

$R^2$'s and $R^3$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyl group, a C2-C6 alkenyloxy group, a C2-C6 alkynyl group, a C2-C6 alkynyloxy group, a C1-C6 alkylcarbonyloxy group, a C1-C6 alkoxycarbonyloxy group, a C1-C6 alkylthiocarbonyloxy group, a C1-C6 alkylthiothiocarbonyloxy group, a C1-C6 alkylsulfonyloxy group, a C1-C6 haloalkylsulfonyloxy group, a C3-C6 cycloalkylsulfonyloxy group, an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), an aminosulfonyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), and at least one of $R^2$ and $R^3$ is a substituent other than a hydrogen atom. The two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents.

X's each independently represent an oxygen atom, a sulfur atom, $CR^4R^5$ or $NR^6$.

$R^4$ and $R^5$ each independently represent a hydrogen atom or a C1-C6 alkyl group.

$R^6$ represents a hydrogen atom, a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group).

W's each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkoxy group, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6

alkyl group, or a C1-C6 haloalkyl group), a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkylsulfonyloxy group, a C1-C6 haloalkylsulfonyloxy group, a C3-C6 cycloalkylsulfonyloxy group, or an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group).)

The present invention relates to a synthetic intermediate represented by the following general formula (1a), (1a'), (2a), (2a'), (3a), or (3a'), which is useful for producing the 1,4-cineole derivative represented by the general formula (1), (1'), (2), (2'), (3), or (3').

[Chem. 4]

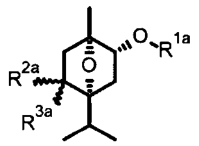

(1a)

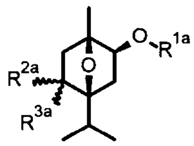

(1a')

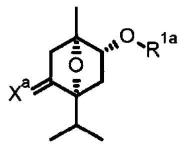

(2a)

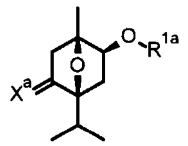

(2a')

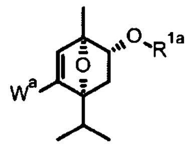

(3a)

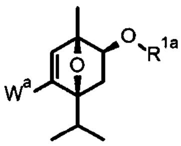

(3a')

(In the general formulae (1a), (1a'), (2a), (2a'), (3a), and (3a'),

$R^{1a}$'s each independently represent a hydrogen atom or a tri-C1-C6 alkylsilyl group which may be the same or different.

$R^{2a}$'s and $R^{3a}$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyl group, a C2-C6 alkenyloxy group, a C2-C6 alkynyl group, a C2-C6 alkynyloxy group, a C1-C6 alkylcarbonyloxy group, a C1-C6 alkoxycarbonyloxy group, a C1-C6 alkylthiocarbonyloxy group, a C1-C6 alkylthiothiocarbonyloxy group, a C1-C6 alkylsulfonyloxy group, a C1-C6 haloalkylsulfonyloxy group, a C3-C6 cycloalkylsulfonyloxy group, an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), an aminosulfonyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), and at least one of $R^2$ and $R^3$ is a substituent other than a hydrogen atom. The two adjacent substituents, $R^{2a}$ and $R^{3a}$, together with a carbon atom to which $R^{2a}$ and $R^{3a}$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents.

$X^a$'s each independently represent an oxygen atom, a sulfur atom, $CR^{4a}R^{5a}$ or $NR^{6a}$.

$R^{4a}$ and $R^{5a}$ each independently represent a hydrogen atom or a C1-C6 alkyl group.

$R^{6a}$ represents a hydrogen atom, a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group).

$W^a$'s each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkoxy group, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkylsulfonyloxy group, a C1-C6 haloalkylsulfonyloxy group, a C3-C6 cycloalkylsulfonyloxy group, or an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group).)

In the 1,4-cineole derivative of the present invention represented by the above general formula (1), (1'), (2), (2'), (3) or (3') and the synthetic intermediate of the present invention represented by the above general formula (1a), (1a'), (2a), (2a'), (3a) or (3a'), examples of the halogen atom represented by $R^2$, $R^3$, W, $R^{2a}$, $R^{3a}$, or $W^a$ or the halogen atom as a substituent include fluorine, chlorine, bromine, and iodine. The number of halogen atoms as the substituent may be one or two or more, and in the case of two or more, the respective halogen atoms may be the same or different. A substitution position of the halogen atom may be any position of the group substituted with the halogen atom.

The C1-C6 alkyl group represented by $R^1$, $R^2$, $R^3$, $R^4$, $R^5$, $R^6$, W, $R^{2a}$, $R^{3a}$, $R^{4a}$, $R^{5a}$, $R^{6a}$ or $W^a$, or the C1-C6 alkyl group as a substituent may be either linear or branched, and examples thereof include a methyl group, an ethyl group, an n-propyl group, an isopropyl group, an n-butyl group, an isobutyl group, a sec-butyl group, a tert-butyl group, an n-pentyl group, a neopentyl group, a 2-pentyl group, a 3-pentyl group, a tert-pentyl group, an n-hexyl group, an isohexyl group, a 2-hexyl group and a 3-hexyl group. The number of C1-C6 alkyl groups as the substituent may be one or two or more, and in the case of two or more, the respective C1-C6 alkyl groups may be the same or different. A substitution position of the C1-C6 alkyl group may be any position of the group substituted with the C1-C6 alkyl group.

The C1-C6 haloalkyl group represented by $R^1$, $R^2$, $R^3$, W, $R^{2a}$, $R^{3a}$ or $W^a$, or the C1-C6 haloalkyl group as a substituent may be either linear or branched, and examples thereof include a monofluoromethyl group, a difluoromethyl group, a trifluoromethyl group, a 2,2,2-trifluoroethyl group, a 2-chloroethyl group, a trichloromethyl group, a 1-fluoroethyl group, a 2-fluoroethyl group, a 6-fluorohexyl group and a monobromomethyl group. The number of C1-C6 haloalkyl groups as the substituent may be one or two or more, and in the case of two or more, the respective C1-C6 haloalkyl groups may be the same or different. A substitution position of the C1-C6 haloalkyl group may be any position of the group substituted with the C1-C6 haloalkyl group.

The C2-C6 alkenyl group represented by $R^1$, $R^2$, $R^3$, W, $R^{2a}$, $R^{3a}$, or $W^a$ may be either linear or branched, and examples thereof include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

The C2-C6 alkenyloxy group represented by $R^2$, $R^3$, $R^{2a}$, or $R^{3a}$ may be either linear or branched, and examples thereof include a vinyloxy group, a 1-propenyloxy group, a 2-propenyloxy group, a 1-butenyloxy group, a 2-butenyloxy group, a 3-butenyloxy group, a 1-methyl-2-propenyloxy group, a 2-methyl-2-propenyloxy group, a 1-pentenyloxy group, a 2-pentenyloxy group, a 3-pentenyloxy group, a 4-pentenyloxy group, a 1-methyl-2-butenyloxy group, a 2-methyl-2-butenyloxy group, a 1-hexenyloxy group, a 2-hexenyloxy group, a 3-hexenyloxy group, a 4-hexenyloxy group, and a 5-hexenyloxy group.

The C2-C6 alkynyl group represented by $R^1$, $R^2$, $R^3$, W, $R^{2a}$, $R^{3a}$, or $W^a$ may be either linear or branched, and examples thereof include an ethynyl group, a 1-propynyl group, a propargyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

The C2-C6 alkynyloxy group represented by $R^2$, $R^3$, $R^{2a}$, or $R^{3a}$ may be either linear or branched, and examples thereof include an ethynyloxy group, a 1-propynyloxy group, a propargyloxy group, a 1-butynyloxy group, a 2-butynyloxy group, a 3-butynyloxy group, a 1-methyl-2-propynyloxy group, a 2-methyl-3-butynyloxy group, a 1-pentynyloxy group, a 2-pentynyloxy group, a 3-pentynyloxy group, a 4-pentynyloxy group, a 1-methyl-2-butynyloxy group, a 2-methyl-3-pentynyloxy group, a 1-hexynyloxy group, and a 1,1-dimethyl-2-butynyloxy group.

Examples of the C3-C6 cycloalkyl group represented by $R^1$, $R^2$, $R^3$, W, $R^{2a}$, $R^{3a}$, or $W^a$ include a cyclopropyl group, a 1-methylcyclopropyl group, a 2-methylcyclopropyl group, a 2,2-dimethylpropyl group, a cyclobutyl group, a cyclopentyl group, and a cyclohexyl group.

The C3-C6 cycloalkyl C1-C6 alkyl group represented by $R^1$, $R^2$, $R^3$, W, $R^{2a}$, $R^{3a}$, or $W^a$ may be either linear or branched, and examples thereof include a cyclopropylmethyl group, a cyclopropylethyl group, a 1-methylcyclopropylmethyl group, a 2-methylcyclopropylmethyl group, a 2,2-dimethylcyclopropylmethyl group, a cyclobutylmethyl group, a cyclopentylmethyl group, and a cyclohexylmethyl group.

Examples of the C3-C6 cycloalkyl C1-C6 alkoxy group represented by $R^2$, $R^3$, $R^{2a}$, or $R^{3a}$ include a cyclopropylmethyloxy group, a cyclopropylethyloxy group, a 1-methylcyclopropylmethyloxy group, a 2-methylcyclopropylmethyloxy group, a 2,2-dimethylcyclopropylmethyloxy group, a cyclobutylmethyloxy group, a cyclopentylmethyloxy group, and a cyclohexylmethyloxy group.

The C1-C6 alkoxy C1-C6 alkyl group represented by $R^1$ may be either linear or branched, and examples thereof include a methoxymethyl group, an ethoxymethyl group, an n-propoxymethyl group, an isopropoxymethyl group, an n-butoxymethyl group, a sec-butoxymethyl group, a tert-butoxymethyl group, a 1-pentyloxymethyl group, a 1-hexyloxymethyl group, a 2-methoxyethyl group, a 2-ethoxyethyl group, a 2-isopropoxyethyl group, a 2-isobutoxyethyl group, a 3-methoxypropyl group, a 2-methoxypropyl group, and a 2-methoxy-1-methylethyl group.

The aryl group represented by $R^1$, $R^2$, $R^3$, W, $R^{2a}$, $R^{3a}$, or $W^a$ refers to a monocyclic or polycyclic aromatic group, and examples thereof include a phenyl group, a 1-naphthyl group, and a 2-naphthyl group.

Examples of the heterocyclic ring represented by $R^1$, $R^2$, $R^3$, W, $R^{2a}$, $R^{3a}$, or $W^a$ include a 2-pyridyl group, a 3-pyridyl group, a 4-pyridyl group, a 2-thienyl group, a 3-thienyl group, a 2-furyl group, a 3-furyl group, a 2-pyrimidyl group, a 4-pyrimidyl group, a 5-pyrimidyl group, a 6-pyrimidyl group, a 2-tetrahydrofuryl group, and a 3-tetrahydrofuryl group.

The C7-C11 aralkyl group represented by $R^1$, $R^2$, $R^3$, W, $R^{2a}$, $R^{3a}$, or $W^a$ may be either linear or branched, and examples thereof include a benzyl group, a 1-phenethyl group, a 2-phenethyl group, a 1-phenylpropyl group, a 2-phenylpropyl group, a 3-phenylpropyl group, a 1-phenyl-2-methylpropyl group, a 1-phenylbutyl group, and a 1-phenylpentyl group.

The heterocyclic C1-C6 alkyl group represented by $R^1$, $R^2$, $R^3$, W, $R^{2a}$, $R^{3a}$, or $W^a$ may be either linear or branched, and examples thereof include a 2-pyridylmethyl group, a 3-pyridylmethyl group, a 4-pyridylmethyl group, a 2-thienylmethyl group, a 3-thienylmethyl group, a 2-furfuryl group, a 3-furfuryl group, a 2-pyrimidylmethyl group, a 4-pyrimidylmethyl group, a 5-pyrimidylmethyl group, a 6-pyrimidylmethyl group, a 4-pyrazolylmethyl group, a 2-tetrahydrofurfuryl group, a 3-tetrahydrofurfuryl group, and an isoxazol-4yl-methyl group.

The phenoxy C1-C6 alkyl group represented by $R^1$, W, or $W^a$ may be either linear or branched, and examples thereof include a phenoxymethyl group, a 2-phenoxyethyl group, a 2-phenoxypropyl group, a 3-phenoxypropyl group, a 2-phenoxybutyl group, a 3-phenoxybutyl group, and a 4-phenoxybutyl group.

The C7-C11 aralkyloxy C1-C6 alkyl group represented by $R^1$, W, or $W^a$ may be either linear or branched, and examples thereof include a benzyloxymethyl group, a 1-phenethyloxymethyl group, a 2-phenethyloxymethyl group, a 1-phenylpropoxymethyl group, a 2-phenylpropoxymethyl group, a 3-phenylpropoxymethyl group, and a benzyloxyethyl group.

The C1-C6 alkoxy C1-C6 alkoxy C1-C6 alkyl group represented by $R^1$ may be either linear or branched, and examples thereof include a methoxymethoxymethyl group, an ethoxymethoxymethyl group, an n-propoxymethoxymethyl group, an isopropoxymethoxymethyl group, an n-butoxymethoxymethyl group, a sec-butoxymethoxymethyl group, a tert-butoxymethoxymethyl group, a 1-pentyloxymethoxymethyl group, a 1-hexyloxymethoxymethyl group, a 1-ethoxyethoxymethyl group, a 1-methoxyethoxymethyl group, and a 2-methoxyethoxymethyl group.

The benzoyl C1-C6 alkyl group represented by $R^1$ may be either linear or branched, and examples thereof include a phenacyl group, a 1-phenyl-1-oxopropyl group, and a 1-phenyl-2-oxopropyl group.

The C1-C6 alkoxy group represented by $R^2$, $R^3$, $R^6$, W, $R^{2a}$, $R^{3a}$, $R^{6a}$, or $W^a$, or the C1-C6 alkoxy group as a substituent may be either linear or branched, and examples thereof include a methoxy group, an ethoxy group, an n-propoxy group, an isopropoxy group, an n-butoxy group, an isobutoxy group, a sec-butoxy group, and a tert-butoxy group.

The number of C1-C6 alkoxy groups as the substituent may be one or two or more, and in the case of two or more, the respective C1-C6 alkoxy groups may be the same or different. A substitution position of the C1-C6 alkoxy group may be any position of the group substituted with the C1-C6 alkoxy group.

The C1-C6 haloalkoxy group represented by $R^2$, $R^3$, W, $R^{2a}$, $R^{3a}$ or $W^a$, or the C1-C6 haloalkoxy group as a substituent may be either linear or branched, and examples thereof include a monofluoromethyloxy group, a difluoromethyloxy group, a trifluoromethyloxy group, a 2,2,2-trifluoroethyloxy group, a 2-chloroethyloxy group, a trichloromethyloxy group, a 1-fluoroethyloxy group, a 2-fluoroethyloxy group, and a 6-fluorohexyloxy group. The number of C1-C6 haloalkoxy groups as the substituent may be one or two or more, and in the case of two or more, the respective C1-C6 haloalkoxy groups may be the same or different. A substitution position of the C1-C6 haloalkoxy group may be any position of the group substituted with the C1-C6 haloalkoxy group.

Examples of the C7-C11 aralkyloxy group represented by $R^2$, $R^3$, W, $R^{2a}$, $R^{3a}$, or $W^a$ include a benzyloxy group, a 1-phenethyloxy group, a 2-phenethyloxy group, a 1-phenylpropyloxy group, a 2-phenylpropyloxy group, a 3-phenylpropyloxy group, a 1-phenyl-2-methylpropyloxy group, a 1-phenylbutyloxy group, and a 1-phenylpentyloxy group.

The heterocyclic C1-C6 alkoxy group represented by $R^2$, $R^3$, W, $R^{2a}$, $R^{3a}$, or $W^a$ may be either linear or branched, and examples thereof include a 2-pyridylmethoxy group, a 3-pyridylmethoxy group, a 4-pyridylmethoxy group, a 2-thienylmethoxy group, a 3-thienylmethoxy group, a 2-furylmethoxy group, a 3-furylmethoxy group, a 2-pyrimidylmethoxy group, a 4-pyrimidylmethoxy group, a 5-pyrimidylmethoxy group, a 6-pyrimidylmethoxy group, a 2-tetrahydrofuryloxy group, and a 3-tetrahydrofuryloxy group.

The C1-C6 alkoxy C1-C6 alkoxy group represented by $R^2$, $R^3$, W, $R^{2a}$, $R^{3a}$, or $W^a$ may be either linear or branched, and examples thereof include a methoxymethoxy group, an ethoxymethoxy group, a 1-ethoxyethoxy group, an n-propoxymethoxy group, an isopropoxymethoxy group, an n-butoxymethoxy group, a sec-butoxymethoxy group, a tert-butoxymethoxy group, a 1-pentyloxymethoxy group, and a 1-hexyloxymethoxy group.

Examples of the C1-C6 alkylcarbonyloxy group represented by $R^2$, $R^3$, $R^{2a}$, or $R^{3a}$ include an acetyloxy group, an ethylcarbonyloxy group, an n-propylcarbonyloxy group, an isopropylcarbonyloxy group, an n-butylcarbonyloxy group, an isobutylcarbonyloxy group, a sec-butylcarbonyloxy group, a tert-butylcarbonyloxy group, a 1-pentylcarbonyloxy group, and a 1-hexylcarbonyloxy group.

Examples of the C1-C6 alkoxycarbonyloxy group represented by $R^2$, $R^3$, $R^{2a}$, or $R^{3a}$ include a methoxycarbonyloxy group, an ethoxycarbonyloxy group, an n-propoxycarbonyloxy group, an isopropoxycarbonyloxy group, an n-butoxycarbonyloxy group, a sec-butoxycarbonyloxy group, and a tert-butoxycarbonyloxy group.

Examples of the C1-C6 alkylthiocarbonyloxy group represented by $R^2$, $R^3$, $R^{2a}$, or $R^{3a}$ include a methylthiocarbonyloxy group, an ethylthiocarbonyloxy group, an n-propylthiocarbonyloxy group, an isopropylthiocarbonyloxy group, an n-butylthiocarbonyloxy group, a sec-butylthiocarbonyloxy group, a tert-butylthiocarbonyloxy group, a 1-pentylthiocarbonyloxy group, and a 1-hexylthiocarbonyloxy group.

Examples of the C1-C6 alkylthiothiocarbonyloxy group represented by $R^2$, $R^3$, $R^{2a}$, or $R^{3a}$ include a methylthiothiocarbonyloxy group, an ethylthiothiocarbonyloxy group, an n-propylthiothiocarbonyloxy group, an isopropylthiothiocarbonyloxy group, an n-butylthiothiocarbonyloxy group, a sec-butylthiothiocarbonyloxy group, a tert-butylthiothiocarbonyloxy group, a 1-pentylthiothiocarbonyloxy group, and a 1-hexylthiothiocarbonyloxy group.

Examples of the C1-C6 alkylsulfonyloxy group represented by $R^2$, $R^3$, W, $R^{2a}$, $R^{3a}$, or $W^a$ include a methanesulfonyloxy group, an ethanesulfonyloxy group, an n-propanesulfonyloxy group, an isopropanesulfonyloxy group, an n-butanesulfonyloxy group, an isobutanesulfonyloxy group, a sec-butanesulfonyloxy group, a tert-butanesulfonyloxy group, and an n-pentanesulfonyloxy group.

Examples of the C1-C6 haloalkylsulfonyloxy group represented by $R^2$, $R^3$, W, $R^{2a}$, $R^{3a}$, or $W^a$ include a monofluoromethylsulfonyloxy group, a difluoromethylsulfonyloxy group, a trifluoromethylsulfonyloxy group, a monochloromethylsulfonyloxy group, a trichloromethylsulfonyloxy group, and a 2,2,2-trifluoroethylsulfonyloxy group.

Examples of the C3-C6 cycloalkylsulfonyloxy group represented by $R^2$, $R^3$, W, $R^{2a}$, $R^{3a}$, or $W^a$ include a cyclopropylsulfonyloxy group, a 1-methylcyclopropylsulfonyloxy group, a 2-methylcyclopropylsulfonyloxy group, a 2,2-dimethylpropylsulfonyloxy group, a cyclobutylsulfonyloxy group, a cyclopentylsulfonyloxy group, and a cyclohexylsulfonyloxy group.

The arylsulfonyloxy group represented by $R^2$, $R^3$, W, $R^{2a}$, $R^{3a}$, or $W^a$ refers to a monocyclic or polycyclic aromatic sulfonyloxy group, and examples thereof include a phenylsulfonyloxy group, a 1-naphthylsulfonyloxy group, and a 2-naphthylsulfonyloxy group.

Examples of the C1-C6 alkylamino group represented by $R^6$ or $R^{6a}$ include a methylamino group, an ethylamino group, an n-propylamino group, an isopropylamino group, an n-butylamino group, an isobutylamino group, a sec-butylamino group, and a tert-butylamino group.

Examples of the tri-C1-C6 alkylsilyl group being represented by $R^{1a}$ which may be the same or different include a trimethylsilyl group, a triethylsilyl group, a triisopropylsilyl group, a dimethylisopropylsilyl group, a diethylisopropylsilyl group, a dimethylhexylsilyl group, a tert-butyldimethylsilyl group, and a di-tert-butylmethylsilyl group.

Examples of the heterocyclic oxy group represented by W or $W^a$ include a 2-pyridyloxy group, a 3-pyridyloxy

group, a 4-pyridyloxy group, a 2-thienyloxy group, a 3-thienyloxy group, a 2-furyloxy group, a 3-furyloxy group, a 2-pyrimidyloxy group, a 4-pyrimidyloxy group, a 5-pyrimidyloxy group, a 6-pyrimidyloxy group, a 2-tetrahydrofuryloxy group, and a 3-tetrahydrofuryloxy group.

The C1-C6 alkoxycarbonyl group as the substituent may be either linear or branched, and examples thereof include a methoxycarbonyl group, an ethoxycarbonyl group, an n-propoxycarbonyl group, an isopropoxycarbonyl group, an n-butoxycarbonyl group, an isobutoxycarbonyl group, a sec-butoxycarbonyl group, and a tert-butoxycarbonyl group. The number of C1-C6 alkoxycarbonyl groups as the substituent may be one or two or more, and in the case of two or more, the respective C1-C6 alkoxycarbonyl groups may be the same or different. A substitution position of the C1-C6 alkoxycarbonyl group may be any position of the substituent in the C1-C6 alkoxycarbonyl group.

In the 1,4-cineole derivative represented by the above general formula (1), (1'), (2), (2'), (3) or (3'),

$R^1$'s each independently represent a C1-C6 alkyl group, a C1-C6 haloalkyl group, a C2-C6 alkenyl group, a C2-C6 alkynyl group, C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a benzoyl C1-C6 alkyl group (which may be mono-substituted or poly-substituted with a halogen atom, a C1-C6 alkyl group, or a C1-C6 haloalkyl group), or a C1-C6 alkoxy C1-C6 alkoxy C1-C6 alkyl group,

$R^2$'s and $R^3$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyloxy group, a C2-C6 alkynyloxy group, a C1-C6 alkylcarbonyloxy group, a C1-C6 alkylthiothiocarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), at least one of $R^2$ and $R^3$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

X's each independently represent an oxygen atom, a sulfur atom, $CR^4R^5$, or $NR^6$,

$R^4$ and $R^5$ each independently represent a hydrogen atom or a C1-C6 alkyl group,

$R^6$ represents a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and

W's each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a

halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C1-C6 alkylsulfonyloxy group, or a C1-C6 haloalkylsulfonyloxy group.

More preferably, in the 1,4-cineole derivative represented by the above general formula (1), (1'), (2), (2'), (3) or (3'),

$R^1$'s each independently represent a C1-C6 alkyl group, a C1-C6 haloalkyl group, a C2-C6 alkenyl group, a C2-C6 alkynyl group, a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), or a C1-C6 alkoxy C1-C6 alkoxy C1-C6 alkyl group,

$R^2$'s and $R^3$'s each independently represent a hydrogen atom, a halogen atom, a hydroxyl group, a C1-C6 alkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a C2-C6 alkenyloxy group, a C2-C6 alkynyloxy group, a C1-C6 alkylcarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), at least one of $R^2$ and $R^3$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

X's each independently represent an oxygen atom, $CR^4R^5$, or $NR^6$,

$R^4$ and $R^5$ each independently represent a hydrogen atom,

$R^6$ represents a hydroxyl group, and

W's each independently represent a hydrogen atom, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), or a C1-C6 haloalkylsulfonyloxy group.

As a preferred aspect, in the 1,4-cineole derivative represented by the above general formula (1), (1'), (2), (2'), (3) or (3'),

$R^1$'s each independently preferably represent a C2-C6 alkenyl group, a C2-C6 alkynyl group,a C3-C6 cycloalkyl C1-C6 alkyl group (which may be mono-substituted or poly-substituted with a halogen atom, a C1-C6 alkyl group, or a C1-C6 haloalkyl group), an aryl group (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), or a benzoyl C1-C6 alkyl group (which may be mono-substituted or poly-substituted with a halogen atom, a C1-C6 alkyl group, or a C1-C6 haloalkyl group), and
more preferably represent a C2-C6 alkenyl group, a C2-C6 alkynyl group, a C3-C6 cycloalkyl C1-C6 alkyl group (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), and a phenoxy C1-C6 alkyl group (which may be mono-substituted or poly-substituted with a halogen atom, a C1-C6 alkyl group, or a C1-C6 haloalkyl group).
$R^2$'s and $R^3$'s each independently preferably represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyloxy group, a C2-C6 alkynyloxy group, a C1-C6 alkylcarbonyloxy group, a C1-C6 alkylthiothiocarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), at least one of $R^2$ and $R^3$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents, $R^2$ more preferably represents a halogen atom, a hydroxyl group, a C1-C6 alkyl group, a C1-C6 alkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a C2-C6 alkenyloxy group, a C2-C6 alkynyloxy group, a C1-C6 alkylcarbonyloxy group, a C1-C6 haloalkylsulfonyloxy group or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), and $R^3$ more preferably represents a hydrogen atom, and
$R^2$ still more preferably represents a halogen atom, a hydroxyl group, a C1-C6 alkyl group, a C1-C6 alkoxy group, a

C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), or an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), and $R^3$ still more preferably represents a hydrogen atom.

X's each independently preferably represent an oxygen atom, $CR^4R^5$, or $NR^6$.

$R^4$ and $R^5$ more preferably represent a hydrogen atom.

$R^6$ preferably represents a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and more preferably represents a hydroxyl group.

W's each independently preferably represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C1-C6 alkylsulfonyloxy group, or a C1-C6 haloalkylsulfonyloxy group, and more preferably represent a hydrogen atom, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), or a C1-C6 haloalkylsulfonyloxy group.

Examples of preferred groups for $R^{2a}$, $R^{3a}$, $X^2$, $R^{4a}$, $R^{5a}$, $R^{6a}$ and $W^a$ in the synthetic intermediate (intermediate of the present invention) represented by the general formula (1a), (1a'), (2a), (2a'), (3a) or (3a') which is useful for producing the 1,4-cineole derivative represented by the general formula (1), (1'), (2), (2'), (3) or (3') include groups same as the preferred groups for $R^2$, $R^3$, X, $R^4$, $R^5$, $R^6$ and W in the 1,4-cineole derivative represented by the general formula (1), (1'), (2), (2'), (3) or (3'), respectively.

Preferably, in the synthetic intermediate (intermediate of the present invention) represented by the general formula (1a), (1a'), (2a), (2a'), (3a) or (3a') which is useful for producing the 1,4-cineole derivative represented by the general formula (1), (1'), (2), (2'), (3) or (3'),

$R^{1a}$'s each independently represent a hydrogen atom or a tri-C1-C6 alkylsilyl group which may be the same or different,

$R^{2a}$'s and $R^{3a}$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyloxy group, a C2-C6 alkynyloxy group, a C1-C6 alkylcarbonyloxy group, a C1-C6 alkylthiothiocarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl group portions may be bonded to each other via an alkylene group to form a 3-membered ring, a 4-membered ring, a 5-membered ring, or a 6-membered ring), at least one of $R^2$ and $R^3$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

$X^a$'s each independently represent an oxygen atom, a sulfur atom, $CR^4R^5$ or $NR^6$,

$R^{4a}$ and $R^{5a}$ each independently represent a hydrogen atom or a C1-C6 alkyl group,

$R^{6a}$ represents a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and

$W^a$ each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C1-C6 alkylsulfonyloxy group, or a C1-C6 haloalkylsulfonyloxy group.

More preferably, in the synthetic intermediate (intermediate of the present invention) represented by the general formula (1a), (1a'), (2a), (2a'), (3a) or (3a') which is useful for producing the 1,4-cineole derivative represented by the general formula (1), (1'), (2), (2'), (3) or (3'),

$R^{1a}$'s each independently represent a hydrogen atom or a tri-C1-C6 alkylsilyl group which may be same or different,

$R^{2a}$'s and $R^{3a}$'s each independently represent a hydrogen atom, a halogen atom, a hydroxyl group, a C1-C6 alkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a C2-C6 alkenyloxy group, a C2-C6 alkynyloxy group, a C1-C6 alkylcarbonyloxy group, a C1-C6 haloalkylsulfonyloxy group or a carbamoyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), at least one of $R^{2a}$ and $R^{3a}$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

$X^a$'s each independently represent an oxygen atom, $CR^4R^5$, or $NR^6$,

$R^{4a}$ and $R^{5a}$ each independently represent a hydrogen atom,

$R^{6a}$ represents a hydroxyl group, and

$W^a$'s each independently represent a hydrogen atom, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), or a C1-C6 haloalkylsulfonyloxy group.

Representative examples of the 1,4-cineole derivative represented by the general formula (1) or (1') are listed in Table 1 below, representative examples of the 1,4-cineole derivative represented by the general formula (2) or (2') are listed in Table 2 below, and representative examples of the 1,4-cineole derivative represented by the general formula (3) or (3') are listed in Table 3 below, but the compounds are not limited thereto. The compounds include compounds containing an optical isomer, an E isomer, and a Z isomer. Compound numbers are referenced hereinafter.

The following notations in the table represent the corresponding groups as follows.

"H" represents a hydrogen atom, "Me" represents a methyl group, "Et" represents an ethyl group, "n-Pr" represents a normal propyl group, "i-Pr" represents an isopropyl group, "c-Pr" represents a cyclopropyl group, "n-Bu" represents a normal butyl group, "i-Bu" represents an isobutyl group, "t-Bu" represents a tert-butyl group, "c-Pen" represents a cyclopentyl group, "n-Hex" represents a normal hexyl group, "c-Hex" represents a cyclohexyl group, "Ph" represents a phenyl group, "Bn" represents a benzyl group, "=" represents a double bond, and "≡" represents a triple bond.

In Table 1, "stereochemistry at C-5 position" refers to optical isomerism observed in the 1,4-cineole derivative represented by the general formula (1) or (1') when focusing on C5 position, with the carbon atom substituted with a methyl group designated as C1 position.

[Table 1]

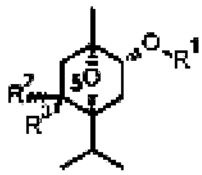

(1)

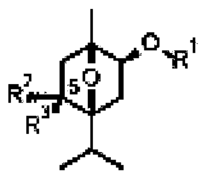

(1')

| No. | General formula | $R^1$ | $R^2$ | $R^3$ | Stereochemistr y at C-5 position | Remark |
|---|---|---|---|---|---|---|
| 1-1 | 1 | $CH_2Ph$ | F | H | S | |
| 1-2 | 1 | $CH_2$(2-MePh) | F | H | S | |
| 1-3 | 1 | $CH_2$(2,6-$Me_2$Ph) | F | H | S | |
| 1-4 | 1 | $CH_2$(2-MeOPh) | F | H | S | |
| 1-5 | 1 | $CH_2$(2-FPh) | F | H | S | |
| 1-6 | 1 | $CH_2$(2-ClPh) | F | H | S | |
| 1-7 | 1 | $CH_2$(2,6-$F_2$Ph) | F | H | S | |
| 1-8 | 1 | $CH_2Ph$ | Cl | H | S | |
| 1-9 | 1 | $CH_2$(2-MePh) | Cl | H | S | |
| 1-10 | 1 | $CH_2$(2,6-$Me_2$Ph) | Cl | H | S | |
| 1-11 | 1 | $CH_2$(2-MeOPh) | Cl | H | S | |
| 1-12 | 1 | $CH_2$(2-FPh) | Cl | H | S | |
| 1-13 | 1 | $CH_2$(2-ClPh) | Cl | H | S | |
| 1-14 | 1 | $CH_2$(2,6-$F_2$Ph) | Cl | H | S | |
| 1-15 | 1 | $CH_2Ph$ | Br | H | S | |
| 1-16 | 1 | $CH_2$(2-MePh) | Br | H | S | |
| 1-17 | 1 | $CH_2$(2,6-$Me_2$Ph) | Br | H | S | |
| 1-18 | 1 | $CH_2$(2-MeOPh) | Br | H | S | |

(continued)

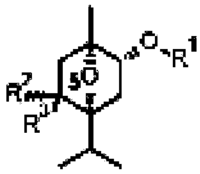 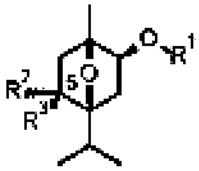

(1) (1')

| No. | General formula | $R^1$ | $R^2$ | $R^3$ | Stereochemistr y at C-5 position | Remark |
|---|---|---|---|---|---|---|
| 1-19 | 1 | $CH_2$(2-FPh) | Br | H | S | |
| 1-20 | 1 | $CH_2$(2-ClPh) | Br | H | S | |
| 1-21 | 1 | $CH_2$(2,6-$F_2$Ph) | Br | H | S | |
| 1-22 | 1 | $CH_2$Ph | OH | H | S | |
| 1-23 | 1' | $CH_2$Ph | OH | H | S | |
| 1-24 | 1 | $CH_2$(2-MePh) | OH | H | S | |
| 1-25 | 1' | $CH_2$(2-MePh) | OH | H | S | |
| 1-26 | 1 | $CH_2$(2,6-$Me_2$Ph) | OH | H | S | |
| 1-27 | 1' | $CH_2$(2,6-$Me_2$Ph) | OH | H | S | |
| 1-28 | 1 | $CH_2$(2-MeOPh) | OH | H | S | |
| 1-29 | 1' | $CH_2$(2-MeOPh) | OH | H | S | |
| 1-30 | 1 | $CH_2$(2-FPh) | OH | H | S | |

[Table 2]

Table 1-Continued

| No. | General formula | $R^1$ | $R^2$ | $R^3$ | Stereochemistr y at C-5 position | Remark |
|---|---|---|---|---|---|---|
| 1-31 | 1' | $CH_2$(2-FPh) | OH | H | S | |
| 1-32 | 1 | $CH_2$(2-ClPh) | OH | H | S | |
| 1-33 | 1' | $CH_2$(2-ClPh) | OH | H | S | |
| 1-34 | 1 | $CH_2$(2-BrPh) | OH | H | S | |
| 1-35 | 1 | $CH_2$(2,3-$F_2$Ph) | OH | H | S | |
| 1-36 | 1' | $CH_2$(2,3-$F_2$Ph) | OH | H | S | |
| 1-37 | 1 | $CH_2$(2,4-$F_2$Ph) | OH | H | S | |
| 1-38 | 1' | $CH_2$(2,4-$F_2$Ph) | OH | H | S | |
| 1-39 | 1 | $CH_2$(2,5-$F_2$Ph) | OH | H | S | |
| 1-40 | 1' | $CH_2$(2,5-$F_2$Ph) | OH | H | S | |
| 1-41 | 1 | $CH_2$(2,6-$F_2$Ph) | OH | H | S | |
| 1-42 | 1' | $CH_2$(2,6-$F_2$Ph) | OH | H | S | |
| 1-43 | 1 | $CH_2$(3,5-$F_2$Ph) | OH | H | S | |
| 1-44 | 1' | $CH_2$(3,5-$F_2$Ph) | OH | H | S | |
| 1-45 | 1 | $CH_2$(2,3,4,5,6-$F_5$Ph) | OH | H | S | |
| 1-46 | 1' | $CH_2$(2,3,4,5,6-$F_5$Ph) | OH | H | S | |
| 1-47 | 1 | $CH_2$(2,6-$Cl_2$Ph) | OH | H | S | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-48 | 1' | $CH_2(2,6\text{-}Cl_2Ph)$ | OH | H | S | |
| 1-49 | 1 | $CH_2$(2-F-6-ClPh) | OH | H | S | |
| 1-50 | 1 | $CH_2(2\text{-}CF_3Ph)$ | OH | H | S | |
| 1-51 | 1' | $CH_2(2\text{-}CF_3Ph)$ | OH | H | S | |
| 1-52 | 1 | $CH_2$(2-Pyridyl) | OH | H | S | |
| 1-53 | 1 | $CH_2$(3-Pyridyl) | OH | H | S | |
| 1-54 | 1' | $CH_2$(3-Pyridyl) | OH | H | S | |
| 1-55 | 1 | $CH_2$(4-Pyridyl) | OH | H | S | |
| 1-56 | 1' | $CH_2$(4-Pyridyl) | OH | H | S | |
| 1-57 | 1 | $CH_2$(2-Thienyl) | OH | H | S | |
| 1-58 | 1' | $CH_2$(2-Thienyl) | OH | H | S | |
| 1-59 | 1 | $CH_2$(3-Thienyl) | OH | H | S | |
| 1-60 | 1 | $CH_2Ph$ | OH | H | R | |
| 1-61 | 1' | $CH_2Ph$ | OH | H | R | |
| 1-62 | 1 | $CH_2$(2-MePh) | OH | H | R | |
| 1-63 | 1' | $CH_2$(2-MePh) | OH | H | R | |
| 1-64 | 1 | $CH_2(2,6\text{-}Me_2Ph)$ | OH | H | R | |
| 1-65 | 1' | $CH_2(2,6\text{-}Me_2Ph)$ | OH | H | R | |

[Table 3]

| Table 1-Continued | | | | | | |
|---|---|---|---|---|---|---|
| No. | General formula | $R^1$ | $R^2$ | $R^3$ | Stereochemistr y at C-5 position | Remark |
| 1-66 | 1 | $CH_2$(2-MeOPh) | OH | H | R | |
| 1-67 | 1' | $CH_2$(2-MeOPh) | OH | H | R | |
| 1-68 | 1 | $CH_2$(2-FPh) | OH | H | R | |
| 1-69 | 1' | $CH_2$(2-FPh) | OH | H | R | |
| 1-70 | 1 | $CH_2$(2-ClPh) | OH | H | R | |
| 1-71 | 1' | $CH_2$(2-ClPh) | OH | H | R | |
| 1-72 | 1 | $CH_2$(2-BrPh) | OH | H | R | |
| 1-73 | 1' | $CH_2$(2-BrPh) | OH | H | R | |
| 1-74 | 1 | $CH_2(2,6\text{-}F_2Ph)$ | OH | H | R | |
| 1-75 | 1' | $CH_2(2,6\text{-}F_2Ph)$ | OH | H | R | |
| 1-76 | 1 | $CH_2(2,6\text{-}Cl_2Ph)$ | OH | H | R | |
| 1-77 | 1' | $CH_2(2,6\text{-}Cl_2Ph)$ | OH | H | R | |
| 1-78 | 1 | $CH_2$(2-F-6-ClPh) | OH | H | R | |
| 1-79 | 1' | $CH_2$(2-F-6-ClPh) | OH | H | R | |
| 1-80 | 1 | $CH_2(2\text{-}CF_3Ph)$ | OH | H | R | |
| 1-81 | 1' | $CH_2(2\text{-}CF_3Ph)$ | OH | H | R | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-82 | 1 | $CH_2Ph$ | Me | H | Racemic | |
| 1-83 | 1 | $CH_2$(2-MePh) | Me | H | Racemic | |
| 1-84 | 1 | $CH_2$(2-MePh) | Me | H | S | |
| 1-85 | 1 | $CH_2$(2-MePh) | Me | H | R | |
| 1-86 | 1' | $CH_2$(2-MePh) | Me | H | Racemic | |
| 1-87 | 1' | $CH_2$(2-MePh) | Me | H | S | |
| 1-88 | 1' | $CH_2$(2-MePh) | Me | H | R | |
| 1-89 | 1 | $CH_2$(2,6-$Me_2$Ph) | Me | H | Racemic | |
| 1-90 | 1 | $CH_2$(2-MeOPh) | Me | H | Racemic | |
| 1-91 | 1' | $CH_2$(2-MeOPh) | Me | H | Racemic | |
| 1-92 | 1 | $CH_2$(2-FPh) | Me | H | Racemic | |
| 1-93 | 1 | $CH_2$(2-FPh) | Me | H | S | |
| 1-94 | 1 | $CH_2$(2-FPh) | Me | H | R | |
| 1-95 | 1' | $CH_2$(2-FPh) | Me | H | Racemic | |
| 1-96 | 1' | $CH_2$(2-FPh) | Me | H | S | |
| 1-97 | 1' | $CH_2$(2-FPh) | Me | H | R | |
| 1-98 | 1 | $CH_2$(2-ClPh) | Me | H | Racemic | |
| 1-99 | 1 | $CH_2$(2-ClPh) | Me | H | S | |
| 1-100 | 1 | $CH_2$(2-ClPh) | Me | H | R | |

[Table 4]

| Table 1-Continued | | | | | | |
|---|---|---|---|---|---|---|
| No. | General formula | $R^1$ | $R^2$ | $R^3$ | Stereochemistr y at C-5 position | Remark |
| 1-101 | 1' | $CH_2$(2-ClPh) | Me | H | Racemic | |
| 1-102 | 1' | $CH_2$(2-ClPh) | Me | H | S | |
| 1-103 | 1' | $CH_2$(2-ClPh) | Me | H | R | |
| 1-104 | 1 | $CH_2$(2-BrPh) | Me | H | Racemic | |
| 1-105 | 1 | $CH_2$(2,3-$F_2$Ph) | Me | H | Racemic | |
| 1-106 | 1' | $CH_2$(2,3-$F_2$Ph) | Me | H | Racemic | |
| 1-107 | 1 | $CH_2$(2,4-$F_2$Ph) | Me | H | Racemic | |
| 1-108 | 1' | $CH_2$(2,4-$F_2$Ph) | Me | H | Racemic | |
| 1-109 | 1 | $CH_2$(2,5-$F_2$Ph) | Me | H | Racemic | |
| 1-110 | 1' | $CH_2$(2,5-$F_2$Ph) | Me | H | Racemic | |
| 1-111 | 1 | $CH_2$(2,6-$F_2$Ph) | Me | H | Racemic | |
| 1-112 | 1 | $CH_2$(2,6-$F_2$Ph) | Me | H | S | |
| 1-113 | 1 | $CH_2$(2,6-$F_2$Ph) | Me | H | R | |
| 1-114 | 1' | $CH_2$(2,6-$F_2$Ph) | Me | H | Racemic | |
| 1-115 | 1' | $CH_2$(2,6-$F_2$Ph) | Me | H | S | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-116 | 1' | $CH_2(2,6\text{-}F_2Ph)$ | Me | H | R | |
| 1-117 | 1 | $CH_2(3,5\text{-}F_2Ph)$ | Me | H | Racemic | |
| 1-118 | 1' | $CH_2(3,5\text{-}F_2Ph)$ | Me | H | Racemic | |
| 1-119 | 1 | $CH_2(2,6\text{-}Cl_2Ph)$ | Me | H | Racemic | |
| 1-120 | 1 | $CH_2$(2-F-6-ClPh) | Me | H | Racemic | |
| 1-121 | 1 | $CH_2(2\text{-}CF_3Ph)$ | Me | H | Racemic | |
| 1-122 | 1 | $CH_2Ph$ | Et | H | Racemic | |
| 1-123 | 1 | $CH_2$(2-MePh) | Et | H | Racemic | |
| 1-124 | 1 | $CH_2(2,6\text{-}Me_2Ph)$ | Et | H | Racemic | |
| 1-125 | 1 | $CH_2$(2-MeOPh) | Et | H | Racemic | |
| 1-126 | 1 | $CH_2$(2-FPh) | Et | H | Racemic | |
| 1-127 | 1 | $CH_2$(2-ClPh) | Et | H | Racemic | |
| 1-128 | 1 | $CH_2(2,6\text{-}F_2Ph)$ | Et | H | Racemic | |
| 1-129 | 1 | $CH_2Ph$ | i-Pr | H | Racemic | |
| 1-130 | 1 | $CH_2$(2-MePh) | i-Pr | H | Racemic | |
| 1-131 | 1 | $CH_2(2,6\text{-}Me_2Ph)$ | i-Pr | H | Racemic | |
| 1-132 | 1 | $CH_2$(2-MeOPh) | i-Pr | H | Racemic | |
| 1-133 | 1 | $CH_2$(2-FPh) | i-Pr | H | Racemic | |
| 1-134 | 1 | $CH_2$(2-ClPh) | i-Pr | H | Racemic | |
| 1-135 | 1 | $CH_2(2,6\text{-}F_2Ph)$ | i-Pr | H | Racemic | |

[Table 5]

| Table 1-Continued | | | | | | |
|---|---|---|---|---|---|---|
| No. | General formula | $R^1$ | $R^2$ | $R^3$ | Stereochemistr y at C-5 position | Remark |
| 1-136 | 1 | $CH_2Ph$ | Me | Me | - | |
| 1-137 | 1 | $CH_2$(2-MePh) | Me | Me | - | |
| 1-138 | 1 | $CH_2(2,6\text{-}Me_2Ph)$ | Me | Me | - | |
| 1-139 | 1 | $CH_2$(2-MeOPh) | Me | Me | - | |
| 1-140 | 1 | $CH_2$(2-FPh) | Me | Me | - | |
| 1-141 | 1 | $CH_2$(2-ClPh) | Me | Me | - | |
| 1-142 | 1 | $CH_2(2,6\text{-}F_2Ph)$ | Me | Me | - | |
| 1-143 | 1 | $CH_2Ph$ | $CF_3$ | H | Racemic | |
| 1-144 | 1 | $CH_2$(2-MePh) | $CF_3$ | H | Racemic | |
| 1-145 | 1 | $CH_2(2,6\text{-}Me_2Ph)$ | $CF_3$ | H | Racemic | |
| 1-146 | 1 | $CH_2$(2-MeOPh) | $CF_3$ | H | Racemic | |
| 1-147 | 1 | $CH_2$(2-FPh) | $CF_3$ | H | Racemic | |
| 1-148 | 1 | $CH_2$(2-ClPh) | $CF_3$ | H | Racemic | |
| 1-149 | 1 | $CH_2(2,6\text{-}F_2Ph)$ | $CF_3$ | H | Racemic | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-150 | 1 | $CH_2Ph$ | OMe | H | S | |
| 1-151 | 1 | $CH_2$(2-MePh) | OMe | H | S | |
| 1-152 | 1' | $CH_2$(2-MePh) | OMe | H | S | |
| 1-153 | 1 | $CH_2$(2,6-$Me_2$Ph) | OMe | H | S | |
| 1-154 | 1 | $CH_2$(2-MeOPh) | OMe | H | S | |
| 1-155 | 1' | $CH_2$(2-MeOPh) | OMe | H | S | |
| 1-156 | 1 | $CH_2$(2-FPh) | OMe | H | S | |
| 1-157 | 1' | $CH_2$(2-FPh) | OMe | H | S | |
| 1-158 | 1 | $CH_2$(2-ClPh) | OMe | H | S | |
| 1-159 | 1' | $CH_2$(2-ClPh) | OMe | H | S | |
| 1-160 | 1 | $CH_2$(2-BrPh) | OMe | H | S | |
| 1-161 | 1 | $CH_2$(2,6-$F_2$Ph) | OMe | H | S | |
| 1-162 | 1 | $CH_2$(2,6-$Cl_2$Ph) | OMe | H | S | |
| 1-163 | 1 | $CH_2$(2-F-6-ClPh) | OMe | H | S | |
| 1-164 | 1 | $CH_2$(2-$CF_3$Ph) | OMe | H | S | |
| 1-165 | 1 | $CH_2Ph$ | OMe | H | R | |
| 1-166 | 1' | $CH_2Ph$ | OMe | H | R | |
| 1-167 | 1 | $CH_2$(2-MePh) | OMe | H | R | |
| 1-168 | 1' | $CH_2$(2-MePh) | OMe | H | R | |
| 1-169 | 1 | $CH_2$(2,6-$Me_2$Ph) | OMe | H | R | |
| 1-170 | 1' | $CH_2$(2,6-$Me_2$Ph) | OMe | H | R | |

[Table 6]

| Table 1-Continued | | | | | | |
|---|---|---|---|---|---|---|
| No. | General formula | $R^1$ | $R^2$ | $R^3$ | Stereochemistr y at C-5 position | Remark |
| 1-171 | 1 | $CH_2$(2-MeOPh) | OMe | H | R | |
| 1-172 | 1' | $CH_2$(2-MeOPh) | OMe | H | R | |
| 1-173 | 1 | $CH_2$(2-FPh) | OMe | H | R | |
| 1-174 | 1' | $CH_2$(2-FPh) | OMe | H | R | |
| 1-175 | 1 | $CH_2$(2-ClPh) | OMe | H | R | |
| 1-176 | 1' | $CH_2$(2-ClPh) | OMe | H | R | |
| 1-177 | 1 | $CH_2$(2,6-$F_2$Ph) | OMe | H | R | |
| 1-178 | 1' | $CH_2$(2,6-$F_2$Ph) | OMe | H | R | |
| 1-179 | 1 | $CH_2Ph$ | OEt | H | S | |
| 1-180 | 1 | $CH_2$(2-MePh) | OEt | H | S | |
| 1-181 | 1 | $CH_2$(2,6-$Me_2$Ph) | OEt | H | S | |
| 1-182 | 1 | $CH_2$(2-MeOPh) | OEt | H | S | |
| 1-183 | 1 | $CH_2$(2-FPh) | OEt | H | S | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-184 | 1 | $CH_2$(2-ClPh) | OEt | H | S | |
| 1-185 | 1 | $CH_2$(2,6-$F_2$Ph) | OEt | H | S | |
| 1-186 | 1 | $CH_2$Ph | $OCH_2CF_3$ | H | Racemic | |
| 1-187 | 1 | $CH_2$(2-MePh) | $OCH_2CF_3$ | H | Racemic | |
| 1-188 | 1 | $CH_2$(2,6-$Me_2$Ph) | $OCH_2CF_3$ | H | Racemic | |
| 1-189 | 1 | $CH_2$(2-MeOPh) | $OCH_2CF_3$ | H | Racemic | |
| 1-190 | 1 | $CH_2$(2-FPh) | $OCH_2CF_3$ | H | Racemic | |
| 1-191 | 1 | $CH_2$(2-ClPh) | $OCH_2CF_3$ | H | Racemic | |
| 1-192 | 1 | $CH_2$(2,6-$F_2$Ph) | $OCH_2CF_3$ | H | Racemic | |
| 1-193 | 1 | $CH_2$Ph | OBn | H | Racemic | |
| 1-194 | 1 | $CH_2$(2-MePh) | OBn | H | Racemic | |
| 1-195 | 1 | $CH_2$(2,6-$Me_2$Ph) | OBn | H | Racemic | |
| 1-196 | 1 | $CH_2$(2-MeOPh) | OBn | H | Racemic | |
| 1-197 | 1 | $CH_2$(2-FPh) | OBn | H | Racemic | |
| 1-198 | 1 | $CH_2$(2-ClPh) | OBn | H | Racemic | |
| 1-199 | 1 | $CH_2$(2,6-$F_2$Ph) | OBn | H | Racemic | |
| 1-200 | 1 | $CH_2$Ph | $OCH_2$(2-Pyridyl) | H | Racemic | |
| 1-201 | 1 | $CH_2$(2-MePh) | $OCH_2$(2-Pyridyl) | H | Racemic | |
| 1-202 | 1 | $CH_2$(2,6-$Me_2$Ph) | $OCH_2$(2-Pyridyl) | H | Racemic | |
| 1-203 | 1 | $CH_2$(2-MeOPh) | $OCH_2$(2-Pyridyl) | H | Racemic | |
| 1-204 | 1 | $CH_2$(2-FPh) | $OCH_2$(2-Pyridyl) | H | Racemic | |
| 1-205 | 1 | $CH_2$(2-ClPh) | $OCH_2$(2-Pyridyl) | H | Racemic | |

[Table 7]

| Table 1-Continued | | | | | | |
|---|---|---|---|---|---|---|
| No. | General formula | $R^1$ | $R^2$ | $R^3$ | Stereochemistr y at C-5 position | Remark |
| 1-206 | 1 | $CH_2$(2,6-$F_2$Ph) | $OCH_2$(2-Pyridyl) | H | Racemic | |
| 1-207 | 1 | $CH_2$Ph | OCH(Me)OEt | H | S | |
| 1-208 | 1 | $CH_2$(2-MePh) | OCH(Me)OEt | H | S | |
| 1-209 | 1 | $CH_2$(2,6-$Me_2$Ph) | OCH(Me)OEt | H | S | |
| 1-210 | 1’ | $CH_2$(2,6-$Me_2$Ph) | OCH(Me)OEt | H | R | |
| 1-211 | 1 | $CH_2$(2-MeOPh) | OCH(Me)OEt | H | S | |
| 1-212 | 1’ | $CH_2$(2-MeOPh) | OCH(Me)OEt | H | R | |
| 1-213 | 1 | $CH_2$(2-FPh) | OCH(Me)OEt | H | S | |
| 1-214 | 1’ | $CH_2$(2-FPh) | OCH(Me)OEt | H | R | |
| 1-215 | 1 | $CH_2$(2-ClPh) | OCH(Me)OEt | H | S | |
| 1-216 | 1’ | $CH_2$(2-ClPh) | OCH(Me)OEt | H | R | |
| 1-217 | 1 | $CH_2$(2,3-$F_2$Ph) | OCH(Me)OEt | H | S | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-218 | 1' | $CH_2(2,3\text{-}F_2Ph)$ | OCH(Me)OEt | H | R | |
| 1-219 | 1 | $CH_2(2,4\text{-}F_2Ph)$ | OCH(Me)OEt | H | S | |
| 1-220 | 1' | $CH_2(2,4\text{-}F_2Ph)$ | OCH(Me)OEt | H | R | |
| 1-221 | 1 | $CH_2(2,5\text{-}F_2Ph)$ | OCH(Me)OEt | H | S | |
| 1-222 | 1' | $CH_2(2,5\text{-}F_2Ph)$ | OCH(Me)OEt | H | R | |
| 1-223 | 1 | $CH_2(2,6\text{-}F_2Ph)$ | OCH(Me)OEt | H | S | |
| 1-224 | 1' | $CH_2(2,6\text{-}F_2Ph)$ | OCH(Me)OEt | H | R | |
| 1-225 | 1 | $CH_2(3,5\text{-}F_2Ph)$ | OCH(Me)OEt | H | S | |
| 1-226 | 1' | $CH_2(3,5\text{-}F_2Ph)$ | OCH(Me)OEt | H | R | |
| 1-227 | 1 | $CH_2(2,6\text{-}Cl_2Ph)$ | OCH(Me)OEt | H | S | |
| 1-228 | 1' | $CH_2(2,6\text{-}Cl_2Ph)$ | OCH(Me)OEt | H | R | |
| 1-229 | 1 | $CH_2(2\text{-}F\text{-}6\text{-}ClPh)$ | OCH(Me)OEt | H | S | |
| 1-230 | 1 | $CH_2(2\text{-}CF_3Ph)$ | OCH(Me)OEt | H | S | |
| 1-231 | 1' | $CH_2(2\text{-}CF_3Ph)$ | OCH(Me)OEt | H | R | |
| 1-232 | 1 | $CH_2Ph$ | c-Pr | H | Racemic | |
| 1-233 | 1 | $CH_2(2\text{-}MePh)$ | c-Pr | H | Racemic | |
| 1-234 | 1 | $CH_2(2\text{-}MePh)$ | c-Pr | H | S | |
| 1-235 | 1 | $CH_2(2\text{-}MePh)$ | c-Pr | H | R | |
| 1-236 | 1' | $CH_2(2\text{-}MePh)$ | c-Pr | H | Racemic | |
| 1-237 | 1' | $CH_2(2\text{-}MePh)$ | c-Pr | H | S | |
| 1-238 | 1' | $CH_2(2\text{-}MePh)$ | c-Pr | H | R | |
| 1-239 | 1 | $CH_2(2,6\text{-}Me_2Ph)$ | c-Pr | H | Racemic | |
| 1-240 | 1 | $CH_2(2\text{-}MeOPh)$ | c-Pr | H | Racemic | |

[Table 8]

| Table 1-Continued | | | | | | |
|---|---|---|---|---|---|---|
| No. | General formula | $R^1$ | $R^2$ | $R^3$ | Stereochemistr y at C-5 position | Remark |
| 1-241 | 1' | $CH_2(2\text{-}MeOPh)$ | c-Pr | H | Racemic | |
| 1-242 | 1 | $CH_2(2\text{-}FPh)$ | c-Pr | H | Racemic | |
| 1-243 | 1 | $CH_2(2\text{-}FPh)$ | c-Pr | H | S | |
| 1-244 | 1 | $CH_2(2\text{-}FPh)$ | c-Pr | H | R | |
| 1-245 | 1' | $CH_2(2\text{-}FPh)$ | c-Pr | H | Racemic | |
| 1-246 | 1' | $CH_2(2\text{-}FPh)$ | c-Pr | H | S | |
| 1-247 | 1' | $CH_2(2\text{-}FPh)$ | c-Pr | H | R | |
| 1-248 | 1 | $CH_2(2\text{-}ClPh)$ | c-Pr | H | Racemic | |
| 1-249 | 1 | $CH_2(2\text{-}ClPh)$ | c-Pr | H | S | |
| 1-250 | 1 | $CH_2(2\text{-}ClPh)$ | c-Pr | H | R | |
| 1-251 | 1' | $CH_2(2\text{-}ClPh)$ | c-Pr | H | Racemic | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-252 | 1’ | $CH_2$(2-ClPh) | c-Pr | H | S | |
| 1-253 | 1’ | $CH_2$(2-ClPh) | c-Pr | H | R | |
| 1-254 | 1 | $CH_2$(2-BrPh) | c-Pr | H | Racemic | |
| 1-255 | 1 | $CH_2$(2,3-$F_2$Ph) | c-Pr | H | Racemic | |
| 1-256 | 1’ | $CH_2$(2,3-$F_2$Ph) | c-Pr | H | Racemic | |
| 1-257 | 1 | $CH_2$(2,4-$F_2$Ph) | c-Pr | H | Racemic | |
| 1-258 | 1’ | $CH_2$(2,4-$F_2$Ph) | c-Pr | H | Racemic | |
| 1-259 | 1 | $CH_2$(2,5-$F_2$Ph) | c-Pr | H | Racemic | |
| 1-260 | 1’ | $CH_2$(2,5-$F_2$Ph) | c-Pr | H | Racemic | |
| 1-261 | 1 | $CH_2$(2,6-$F_2$Ph) | c-Pr | H | Racemic | |
| 1-262 | 1 | $CH_2$(2,6-$F_2$Ph) | c-Pr | H | S | |
| 1-263 | 1 | $CH_2$(2,6-$F_2$Ph) | c-Pr | H | R | |
| 1-264 | 1’ | $CH_2$(2,6-$F_2$Ph) | c-Pr | H | Racemic | |
| 1-265 | 1’ | $CH_2$(2,6-$F_2$Ph) | c-Pr | H | S | |
| 1-266 | 1’ | $CH_2$(2,6-$F_2$Ph) | c-Pr | H | R | |
| 1-267 | 1 | $CH_2$(3,5-$F_2$Ph) | c-Pr | H | Racemic | |
| 1-268 | 1’ | $CH_2$(3,5-$F_2$Ph) | c-Pr | H | Racemic | |
| 1-269 | 1 | $CH_2$(2,6-$Cl_2$Ph) | c-Pr | H | Racemic | |
| 1-270 | 1 | $CH_2$(2-F-6-ClPh) | c-Pr | H | Racemic | |
| 1-271 | 1 | $CH_2$(2-$CF_3$Ph) | c-Pr | H | Racemic | |
| 1-272 | 1 | $CH_2$Ph | $CH_2$c-Pr | H | Racemic | |
| 1-273 | 1 | $CH_2$(2-MePh) | $CH_2$c-Pr | H | Racemic | |
| 1-274 | 1 | $CH_2$(2-MePh) | $CH_2$c-Pr | H | S | |
| 1-275 | 1 | $CH_2$(2-MePh) | $CH_2$c-Pr | H | R | |

[Table 9]

| Table 1-Continued | | | | | | |
|---|---|---|---|---|---|---|
| No. | General formula | $R^1$ | $R^2$ | $R^3$ | Stereochemistr y at C-5 position | Remark |
| 1-276 | 1’ | $CH_2$(2-MePh) | $CH_2$c-Pr | H | Racemic | |
| 1-277 | 1’ | $CH_2$(2-MePh) | $CH_2$c-Pr | H | S | |
| 1-278 | 1’ | $CH_2$(2-MePh) | $CH_2$c-Pr | H | R | |
| 1-279 | 1 | $CH_2$(2,6-$Me_2$Ph) | $CH_2$c-Pr | H | Racemic | |
| 1-280 | 1 | $CH_2$(2-MeOPh) | $CH_2$c-Pr | H | Racemic | |
| 1-281 | 1’ | $CH_2$(2-MeOPh) | $CH_2$c-Pr | H | Racemic | |
| 1-282 | 1 | $CH_2$(2-FPh) | $CH_2$c-Pr | H | Racemic | |
| 1-283 | 1 | $CH_2$(2-FPh) | $CH_2$c-Pr | H | S | |
| 1-284 | 1 | $CH_2$(2-FPh) | $CH_2$c-Pr | H | R | |
| 1-285 | 1’ | $CH_2$(2-FPh) | $CH_2$c-Pr | H | Racemic | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-286 | 1' | $CH_2$(2-FPh) | $CH_2$c-Pr | H | S | |
| 1-287 | 1' | $CH_2$(2-FPh) | $CH_2$c-Pr | H | R | |
| 1-288 | 1 | $CH_2$(2-ClPh) | $CH_2$c-Pr | H | Racemic | |
| 1-289 | 1 | $CH_2$(2-ClPh) | $CH_2$c-Pr | H | S | |
| 1-290 | 1 | $CH_2$(2-ClPh) | $CH_2$c-Pr | H | R | |
| 1-291 | 1' | $CH_2$(2-ClPh) | $CH_2$c-Pr | H | Racemic | |
| 1-292 | 1' | $CH_2$(2-ClPh) | $CH_2$c-Pr | H | S | |
| 1-293 | 1' | $CH_2$(2-ClPh) | $CH_2$c-Pr | H | R | |
| 1-294 | 1 | $CH_2$(2-BrPh) | $CH_2$c-Pr | H | Racemic | |
| 1-295 | 1 | $CH_2$(2,3-$F_2$Ph) | $CH_2$c-Pr | H | Racemic | |
| 1-296 | 1' | $CH_2$(2,3-$F_2$Ph) | $CH_2$c-Pr | H | Racemic | |
| 1-297 | 1 | $CH_2$(2,4-$F_2$Ph) | $CH_2$c-Pr | H | Racemic | |
| 1-298 | 1' | $CH_2$(2,4-$F_2$Ph) | $CH_2$c-Pr | H | Racemic | |
| 1-299 | 1 | $CH_2$(2,5-$F_2$Ph) | $CH_2$c-Pr | H | Racemic | |
| 1-300 | 1' | $CH_2$(2,5-$F_2$Ph) | $CH_2$c-Pr | H | Racemic | |
| 1-301 | 1 | $CH_2$(2,6-$F_2$Ph) | $CH_2$c-Pr | H | Racemic | |
| 1-302 | 1 | $CH_2$(2,6-$F_2$Ph) | $CH_2$c-Pr | H | S | |
| 1-303 | 1 | $CH_2$(2,6-$F_2$Ph) | $CH_2$c-Pr | H | R | |
| 1-304 | 1' | $CH_2$(2,6-$F_2$Ph) | $CH_2$c-Pr | H | Racemic | |
| 1-305 | 1' | $CH_2$(2,6-$F_2$Ph) | $CH_2$c-Pr | H | S | |
| 1-306 | 1' | $CH_2$(2,6-$F_2$Ph) | $CH_2$c-Pr | H | R | |
| 1-307 | 1 | $CH_2$(3,5-$F_2$Ph) | $CH_2$c-Pr | H | Racemic | |
| 1-308 | 1' | $CH_2$(3,5-$F_2$Ph) | $CH_2$c-Pr | H | Racemic | |
| 1-309 | 1 | $CH_2$(2,6-$Cl_2$Ph) | $CH_2$c-Pr | H | Racemic | |
| 1-310 | 1 | $CH_2$(2-F-6-ClPh) | $CH_2$c-Pr | H | Racemic | |

[Table 10]

| Table 1-Continued | | | | | | |
|---|---|---|---|---|---|---|
| No. | General formula | $R^1$ | $R^2$ | $R^3$ | Stereochemistr y at C-5 position | Remark |
| 1-311 | 1 | $CH_2$(2-$CF_3$Ph) | $CH_2$c-Pr | H | Racemic | |
| 1-312 | 1 | $CH_2$Ph | Ph | H | Racemic | |
| 1-313 | 1 | $CH_2$(2-MePh) | Ph | H | Racemic | |
| 1-314 | 1 | $CH_2$(2-MePh) | Ph | H | S | |
| 1-315 | 1 | $CH_2$(2-MePh) | Ph | H | R | |
| 1-316 | 1' | $CH_2$(2-MePh) | Ph | H | Racemic | |
| 1-317 | 1' | $CH_2$(2-MePh) | Ph | H | S | |
| 1-318 | 1' | $CH_2$(2-MePh) | Ph | H | R | |
| 1-319 | 1 | $CH_2$(2,6-$Me_2$Ph) | Ph | H | Racemic | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-320 | 1 | $CH_2$(2-MeOPh) | Ph | H | Racemic | |
| 1-321 | 1' | $CH_2$(2-MeOPh) | Ph | H | Racemic | |
| 1-322 | 1 | $CH_2$(2-FPh) | Ph | H | Racemic | |
| 1-323 | 1 | $CH_2$(2-FPh) | Ph | H | S | |
| 1-324 | 1 | $CH_2$(2-FPh) | Ph | H | R | |
| 1-325 | 1' | $CH_2$(2-FPh) | Ph | H | Racemic | |
| 1-326 | 1' | $CH_2$(2-FPh) | Ph | H | S | |
| 1-327 | 1' | $CH_2$(2-FPh) | Ph | H | R | |
| 1-328 | 1 | $CH_2$(2-ClPh) | Ph | H | Racemic | |
| 1-329 | 1 | $CH_2$(2-ClPh) | Ph | H | S | |
| 1-330 | 1 | $CH_2$(2-ClPh) | Ph | H | R | |
| 1-331 | 1' | $CH_2$(2-ClPh) | Ph | H | Racemic | |
| 1-332 | 1' | $CH_2$(2-ClPh) | Ph | H | S | |
| 1-333 | 1' | $CH_2$(2-ClPh) | Ph | H | R | |
| 1-334 | 1 | $CH_2$(2-BrPh) | Ph | H | Racemic | |
| 1-335 | 1 | $CH_2$(2,3-$F_2$Ph) | Ph | H | Racemic | |
| 1-336 | 1' | $CH_2$(2,3-$F_2$Ph) | Ph | H | Racemic | |
| 1-337 | 1 | $CH_2$(2,4-$F_2$Ph) | Ph | H | Racemic | |
| 1-338 | 1' | $CH_2$(2,4-$F_2$Ph) | Ph | H | Racemic | |
| 1-339 | 1 | $CH_2$(2,5-$F_2$Ph) | Ph | H | Racemic | |
| 1-340 | 1' | $CH_2$(2,5-$F_2$Ph) | Ph | H | Racemic | |
| 1-341 | 1 | $CH_2$(2,6-$F_2$Ph) | Ph | H | Racemic | |
| 1-342 | 1 | $CH_2$(2,6-$F_2$Ph) | Ph | H | S | |
| 1-343 | 1 | $CH_2$(2,6-$F_2$Ph) | Ph | H | R | |
| 1-344 | 1' | $CH_2$(2,6-$F_2$Ph) | Ph | H | Racemic | |
| 1-345 | 1' | $CH_2$(2,6-$F_2$Ph) | Ph | H | S | |

[Table 11]

| Table 1-Continued | | | | | | |
|---|---|---|---|---|---|---|
| No. | General formula | $R^1$ | $R^2$ | $R^3$ | Stereochemistr y at C-5 position | Remark |
| 1-346 | 1' | $CH_2$(2,6-$F_2$Ph) | Ph | H | R | |
| 1-347 | 1 | $CH_2$(3,5-$F_2$Ph) | Ph | H | Racemic | |
| 1-348 | 1' | $CH_2$(3,5-$F_2$Ph) | Ph | H | Racemic | |
| 1-349 | 1 | $CH_2$(2,6-$Cl_2$Ph) | Ph | H | Racemic | |
| 1-350 | 1 | $CH_2$(2-F-6-ClPh) | Ph | H | Racemic | |
| 1-351 | 1 | $CH_2$(2-$CF_3$Ph) | Ph | H | Racemic | |
| 1-352 | 1 | $CH_2$Ph | 2-ClPh | H | Racemic | |
| 1-353 | 1 | $CH_2$(2-MePh) | 2-ClPh | H | Racemic | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-354 | 1 | $CH_2$(2,6-$Me_2$Ph) | 2-ClPh | H | Racemic | |
| 1-355 | 1 | $CH_2$(2-MeOPh) | 2-ClPh | H | Racemic | |
| 1-356 | 1 | $CH_2$(2-FPh) | 2-ClPh | H | Racemic | |
| 1-357 | 1 | $CH_2$(2-ClPh) | 2-ClPh | H | Racemic | |
| 1-358 | 1 | $CH_2$(2,6-$F_2$Ph) | 2-ClPh | H | Racemic | |
| 1-359 | 1 | $CH_2$Ph | 3-ClPh | H | Racemic | |
| 1-360 | 1 | $CH_2$(2-MePh) | 3-ClPh | H | Racemic | |
| 1-361 | 1 | $CH_2$(2,6-$Me_2$Ph) | 3-ClPh | H | Racemic | |
| 1-362 | 1 | $CH_2$(2-MeOPh) | 3-ClPh | H | Racemic | |
| 1-363 | 1 | $CH_2$(2-FPh) | 3-ClPh | H | Racemic | |
| 1-364 | 1 | $CH_2$(2-ClPh) | 3-ClPh | H | Racemic | |
| 1-365 | 1 | $CH_2$(2,6-$F_2$Ph) | 3-ClPh | H | Racemic | |
| 1-366 | 1 | $CH_2$Ph | 4-ClPh | H | Racemic | |
| 1-367 | 1 | $CH_2$(2-MePh) | 4-ClPh | H | Racemic | |
| 1-368 | 1 | $CH_2$(2,6-$Me_2$Ph) | 4-ClPh | H | Racemic | |
| 1-369 | 1 | $CH_2$(2-MeOPh) | 4-ClPh | H | Racemic | |
| 1-370 | 1 | $CH_2$(2-FPh) | 4-ClPh | H | Racemic | |
| 1-371 | 1 | $CH_2$(2-ClPh) | 4-ClPh | H | Racemic | |
| 1-372 | 1 | $CH_2$(2,6-$F_2$Ph) | 4-ClPh | H | Racemic | |
| 1-373 | 1 | $CH_2$Ph | 2-Pyridyl | H | Racemic | |
| 1-374 | 1 | $CH_2$(2-MePh) | 2-Pyridyl | H | Racemic | |
| 1-375 | 1 | $CH_2$(2,6-$Me_2$Ph) | 2-Pyridyl | H | Racemic | |
| 1-376 | 1 | $CH_2$(2-MeOPh) | 2-Pyridyl | H | Racemic | |
| 1-377 | 1 | $CH_2$(2-FPh) | 2-Pyridyl | H | Racemic | |
| 1-378 | 1 | $CH_2$(2-ClPh) | 2-Pyridyl | H | Racemic | |
| 1-379 | 1 | $CH_2$(2,6-$F_2$Ph) | 2-Pyridyl | H | Racemic | |
| 1-380 | 1 | $CH_2$Ph | 3-Pyridyl | H | Racemic | |

[Table 12]

| Table 1-Continued | | | | | | |
|---|---|---|---|---|---|---|
| No. | General formula | $R^1$ | $R^2$ | $R^3$ | Stereochemistr y at C-5 position | Remark |
| 1-381 | 1 | $CH_2$(2-MePh) | 3-Pyridyl | H | Racemic | |
| 1-382 | 1 | $CH_2$(2,6-$Me_2$Ph) | 3-Pyridyl | H | Racemic | |
| 1-383 | 1 | $CH_2$(2-MeOPh) | 3-Pyridyl | H | Racemic | |
| 1-384 | 1 | $CH_2$(2-FPh) | 3-Pyridyl | H | Racemic | |
| 1-385 | 1 | $CH_2$(2-ClPh) | 3-Pyridyl | H | Racemic | |
| 1-386 | 1 | $CH_2$(2,6-$F_2$Ph) | 3-Pyridyl | H | Racemic | |
| 1-387 | 1 | $CH_2$Ph | 4-Pyridyl | H | Racemic | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-388 | 1 | $CH_2$(2-MePh) | 4-Pyridyl | H | Racemic | |
| 1-389 | 1 | $CH_2$(2,6-$Me_2$Ph) | 4-Pyridyl | H | Racemic | |
| 1-390 | 1 | $CH_2$(2-MeOPh) | 4-Pyridyl | H | Racemic | |
| 1-391 | 1 | $CH_2$(2-FPh) | 4-Pyridyl | H | Racemic | |
| 1-392 | 1 | $CH_2$(2-ClPh) | 4-Pyridyl | H | Racemic | |
| 1-393 | 1 | $CH_2$(2,6-$F_2$Ph) | 4-Pyridyl | H | Racemic | |
| 1-394 | 1 | $CH_2$Ph | Bn | H | Racemic | |
| 1-395 | 1 | $CH_2$(2-MePh) | Bn | H | Racemic | |
| 1-396 | 1 | $CH_2$(2-MePh) | Bn | H | S | |
| 1-397 | 1 | $CH_2$(2-MePh) | Bn | H | R | |
| 1-398 | 1’ | $CH_2$(2-MePh) | Bn | H | Racemic | |
| 1-399 | 1’ | $CH_2$(2-MePh) | Bn | H | S | |
| 1-400 | 1’ | $CH_2$(2-MePh) | Bn | H | R | |
| 1-401 | 1 | $CH_2$(2,6-$Me_2$Ph) | Bn | H | Racemic | |
| 1-402 | 1 | $CH_2$(2-MeOPh) | Bn | H | Racemic | |
| 1-403 | 1’ | $CH_2$(2-MeOPh) | Bn | H | Racemic | |
| 1-404 | 1 | $CH_2$(2-FPh) | Bn | H | Racemic | |
| 1-405 | 1 | $CH_2$(2-FPh) | Bn | H | S | |
| 1-406 | 1 | $CH_2$(2-FPh) | Bn | H | R | |
| 1-407 | 1’ | $CH_2$(2-FPh) | Bn | H | Racemic | |
| 1-408 | 1’ | $CH_2$(2-FPh) | Bn | H | S | |
| 1-409 | 1’ | $CH_2$(2-FPh) | Bn | H | R | |
| 1-410 | 1 | $CH_2$(2-ClPh) | Bn | H | Racemic | |
| 1-411 | 1 | $CH_2$(2-ClPh) | Bn | H | S | |
| 1-412 | 1 | $CH_2$(2-ClPh) | Bn | H | R | |
| 1-413 | 1’ | $CH_2$(2-ClPh) | Bn | H | Racemic | |
| 1-414 | 1’ | $CH_2$(2-ClPh) | Bn | H | S | |
| 1-415 | 1’ | $CH_2$(2-ClPh) | Bn | H | R | |

[Table 13]

| Table 1-Continued | | | | | | |
|---|---|---|---|---|---|---|
| No. | General formula | $R^1$ | $R^2$ | $R^3$ | Stereochemistr y at C-5 position | Remark |
| 1-416 | 1 | $CH_2$(2-BrPh) | Bn | H | Racemic | |
| 1-417 | 1 | $CH_2$(2,3-$F_2$Ph) | Bn | H | Racemic | |
| 1-418 | 1’ | $CH_2$(2,3-$F_2$Ph) | Bn | H | Racemic | |
| 1-419 | 1 | $CH_2$(2,4-$F_2$Ph) | Bn | H | Racemic | |
| 1-420 | 1’ | $CH_2$(2,4-$F_2$Ph) | Bn | H | Racemic | |
| 1-421 | 1 | $CH_2$(2,5-$F_2$Ph) | Bn | H | Racemic | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-422 | 1' | $CH_2(2,5-F_2Ph)$ | Bn | H | Racemic | |
| 1-423 | 1 | $CH_2(2,6-F_2Ph)$ | Bn | H | Racemic | |
| 1-424 | 1 | $CH_2(2,6-F_2Ph)$ | Bn | H | S | |
| 1-425 | 1 | $CH_2(2,6-F_2Ph)$ | Bn | H | R | |
| 1-426 | 1' | $CH_2(2,6-F_2Ph)$ | Bn | H | Racemic | |
| 1-427 | 1' | $CH_2(2,6-F_2Ph)$ | Bn | H | S | |
| 1-428 | 1' | $CH_2(2,6-F_2Ph)$ | Bn | H | R | |
| 1-429 | 1 | $CH_2(3,5-F_2Ph)$ | Bn | H | Racemic | |
| 1-430 | 1' | $CH_2(3,5-F_2Ph)$ | Bn | H | Racemic | |
| 1-431 | 1 | $CH_2(2,6-Cl_2Ph)$ | Bn | H | Racemic | |
| 1-432 | 1 | $CH_2$(2-F-6-ClPh) | Bn | H | Racemic | |
| 1-433 | 1 | $CH_2(2-CF_3Ph)$ | Bn | H | Racemic | |
| 1-434 | 1 | $CH_2Ph$ | 2-ClBn | H | Racemic | |
| 1-435 | 1 | $CH_2$(2-MePh) | 2-ClBn | H | Racemic | |
| 1-436 | 1 | $CH_2(2,6-Me_2Ph)$ | 2-ClBn | H | Racemic | |
| 1-437 | 1 | $CH_2$(2-MeOPh) | 2-ClBn | H | Racemic | |
| 1-438 | 1 | $CH_2$(2-FPh) | 2-ClBn | H | Racemic | |
| 1-439 | 1 | $CH_2$(2-ClPh) | 2-ClBn | H | Racemic | |
| 1-440 | 1 | $CH_2(2,6-F_2Ph)$ | 2-ClBn | H | Racemic | |
| 1-441 | 1 | $CH_2Ph$ | 3-ClBn | H | Racemic | |
| 1-442 | 1 | $CH_2$(2-MePh) | 3-ClBn | H | Racemic | |
| 1-443 | 1 | $CH_2(2,6-Me_2Ph)$ | 3-ClBn | H | Racemic | |
| 1-444 | 1 | $CH_2$(2-MeOPh) | 3-ClBn | H | Racemic | |
| 1-445 | 1 | $CH_2$(2-FPh) | 3-ClBn | H | Racemic | |
| 1-446 | 1 | $CH_2$(2-ClPh) | 3-ClBn | H | Racemic | |
| 1-447 | 1 | $CH_2(2,6-F_2Ph)$ | 3-ClBn | H | Racemic | |
| 1-448 | 1 | $CH_2Ph$ | 4-ClBn | H | Racemic | |
| 1-449 | 1 | $CH_2$(2-MePh) | 4-ClBn | H | Racemic | |
| 1-450 | 1 | $CH_2(2,6-Me_2Ph)$ | 4-ClBn | H | Racemic | |

[Table 14]

| Table 1-Continued | | | | | | |
|---|---|---|---|---|---|---|
| No. | General formula | $R^1$ | $R^2$ | $R^3$ | Stereochemistr y at C-5 position | Remark |
| 1-451 | 1 | $CH_2$(2-MeOPh) | 4-ClBn | H | Racemic | |
| 1-452 | 1 | $CH_2$(2-FPh) | 4-ClBn | H | Racemic | |
| 1-453 | 1 | $CH_2$(2-ClPh) | 4-ClBn | H | Racemic | |
| 1-454 | 1 | $CH_2(2,6-F_2Ph)$ | 4-ClBn | H | Racemic | |
| 1-455 | 1 | $CH_2Ph$ | $CH_2$(2-Pyridyl) | H | Racemic | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-456 | 1 | $CH_2$(2-MePh) | $CH_2$(2-Pyridyl) | H | Racemic | |
| 1-457 | 1 | $CH_2$(2,6-$Me_2$Ph) | $CH_2$(2-Pyridyl) | H | Racemic | |
| 1-458 | 1 | $CH_2$(2-MeOPh) | $CH_2$(2-Pyridyl) | H | Racemic | |
| 1-459 | 1 | $CH_2$(2-FPh) | $CH_2$(2-Pyridyl) | H | Racemic | |
| 1-460 | 1 | $CH_2$(2-ClPh) | $CH_2$(2-Pyridyl) | H | Racemic | |
| 1-461 | 1 | $CH_2$(2,6-$F_2$Ph) | $CH_2$(2-Pyridyl) | H | Racemic | |
| 1-462 | 1 | $CH_2$Ph | $CH_2$(3-Pyridyl) | H | Racemic | |
| 1-463 | 1 | $CH_2$(2-MePh) | $CH_2$(3-Pyridyl) | H | Racemic | |
| 1-464 | 1 | $CH_2$(2,6-$Me_2$Ph) | $CH_2$(3-Pyridyl) | H | Racemic | |
| 1-465 | 1 | $CH_2$(2-MeOPh) | $CH_2$(3-Pyridyl) | H | Racemic | |
| 1-466 | 1 | $CH_2$(2-FPh) | $CH_2$(3-Pyridyl) | H | Racemic | |
| 1-467 | 1 | $CH_2$(2-ClPh) | $CH_2$(3-Pyridyl) | H | Racemic | |
| 1-468 | 1 | $CH_2$(2,6-$F_2$Ph) | $CH_2$(3-Pyridyl) | H | Racemic | |
| 1-469 | 1 | $CH_2$Ph | $CH_2$(4-Pyridyl) | H | Racemic | |
| 1-470 | 1 | $CH_2$(2-MePh) | $CH_2$(4-Pyridyl) | H | Racemic | |
| 1-471 | 1 | $CH_2$(2,6-$Me_2$Ph) | $CH_2$(4-Pyridyl) | H | Racemic | |
| 1-472 | 1 | $CH_2$(2-MeOPh) | $CH_2$(4-Pyridyl) | H | Racemic | |
| 1-473 | 1 | $CH_2$(2-FPh) | $CH_2$(4-Pyridyl) | H | Racemic | |
| 1-474 | 1 | $CH_2$(2-ClPh) | $CH_2$(4-Pyridyl) | H | Racemic | |
| 1-475 | 1 | $CH_2$(2,6-$F_2$Ph) | $CH_2$(4-Pyridyl) | H | Racemic | |
| 1-476 | 1 | $CH_2$Ph | OCOMe | H | S | |
| 1-477 | 1 | $CH_2$(2-MePh) | OCOMe | H | S | |
| 1-478 | 1 | $CH_2$(2,6-$Me_2$Ph) | OCOMe | H | S | |
| 1-479 | 1 | $CH_2$(2-MeOPh) | OCOMe | H | S | |
| 1-480 | 1 | $CH_2$(2-FPh) | OCOMe | H | S | |
| 1-481 | 1 | $CH_2$(2-ClPh) | OCOMe | H | S | |
| 1-482 | 1 | $CH_2$(2,6-$F_2$Ph) | OCOMe | H | S | |
| 1-483 | 1 | $CH_2$Ph | $OCO_2$Me | H | S | |
| 1-484 | 1 | $CH_2$(2-MePh) | $OCO_2$Me | H | S | |
| 1-485 | 1 | $CH_2$(2,6-$Me_2$Ph) | $OCO_2$Me | H | S | |

[Table 15]

| Table 1-Continued | | | | | | |
|---|---|---|---|---|---|---|
| No. | General formula | $R^1$ | $R^2$ | $R^3$ | Stereochemistr y at C-5 position | Remark |
| 1-486 | 1 | $CH_2$(2-MeOPh) | $OCO_2$Me | H | S | |
| 1-487 | 1 | $CH_2$(2-FPh) | $OCO_2$Me | H | S | |
| 1-488 | 1 | $CH_2$(2-ClPh) | $OCO_2$Me | H | S | |
| 1-489 | 1 | $CH_2$(2,6-$F_2$Ph) | $OCO_2$Me | H | S | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-490 | 1 | $CH_2Ph$ | OC(=S)Me | H | S | |
| 1-491 | 1 | $CH_2$(2-MePh) | OC(=S)Me | H | S | |
| 1-492 | 1 | $CH_2$(2,6-$Me_2$Ph) | OC(=S)Me | H | S | |
| 1-493 | 1 | $CH_2$(2-MeOPh) | OC(=S)Me | H | S | |
| 1-494 | 1 | $CH_2$(2-FPh) | OC(=S)Me | H | S | |
| 1-495 | 1 | $CH_2$(2-ClPh) | OC(=S)Me | H | S | |
| 1-496 | 1 | $CH_2$(2,6-$F_2$Ph) | OC(=S)Me | H | S | |
| 1-497 | 1 | $CH_2Ph$ | $OCS_2Me$ | H | S | |
| 1-498 | 1 | $CH_2$(2-MePh) | $OCS_2Me$ | H | S | |
| 1-499 | 1’ | $CH_2$(2-MePh) | $OCS_2Me$ | H | S | |
| 1-500 | 1 | $CH_2$(2,6-$Me_2$Ph) | $OCS_2Me$ | H | S | |
| 1-501 | 1 | $CH_2$(2-MeOPh) | $OCS_2Me$ | H | S | |
| 1-502 | 1 | $CH_2$(2-FPh) | $OCS_2Me$ | H | S | |
| 1-503 | 1 | $CH_2$(2-ClPh) | $OCS_2Me$ | H | S | |
| 1-504 | 1 | $CH_2$(2,6-$F_2$Ph) | $OCS_2Me$ | H | S | |
| 1-505 | 1 | $CH_2Ph$ | $OCS_2Me$ | H | R | |
| 1-506 | 1 | $CH_2$(2-MePh) | $OCS_2Me$ | H | R | |
| 1-507 | 1’ | $CH_2$(2-MePh) | $OCS_2Me$ | H | R | |
| 1-508 | 1 | $CH_2$(2,6-$Me_2$Ph) | $OCS_2Me$ | H | R | |
| 1-509 | 1 | $CH_2$(2-MeOPh) | $OCS_2Me$ | H | R | |
| 1-510 | 1 | $CH_2$(2-FPh) | $OCS_2Me$ | H | R | |
| 1-511 | 1 | $CH_2$(2-ClPh) | $OCS_2Me$ | H | R | |
| 1-512 | 1 | $CH_2$(2,6-$F_2$Ph) | $OCS_2Me$ | H | R | |
| 1-513 | 1 | $CH_2Ph$ | $OSO_2Me$ | H | S | |
| 1-514 | 1 | $CH_2$(2-MePh) | $OSO_2Me$ | H | S | |
| 1-515 | 1 | $CH_2$(2,6-$Me_2$Ph) | $OSO_2Me$ | H | S | |
| 1-516 | 1 | $CH_2$(2-MeOPh) | $OSO_2Me$ | H | S | |
| 1-517 | 1 | $CH_2$(2-FPh) | $OSO_2Me$ | H | S | |
| 1-518 | 1 | $CH_2$(2-ClPh) | $OSO_2Me$ | H | S | |
| 1-519 | 1 | $CH_2$(2,6-$F_2$Ph) | $OSO_2Me$ | H | S | |
| 1-520 | 1 | $CH_2Ph$ | $OSO_2CF_3$ | H | S | |

[Table 16]

| Table 1-Continued | | | | | | |
|---|---|---|---|---|---|---|
| No. | General formula | $R^1$ | $R^2$ | $R^3$ | Stereochemistr y at C-5 position | Remark |
| 1-521 | 1 | $CH_2$(2-MePh) | $OSO_2CF_3$ | H | S | |
| 1-522 | 1’ | $CH_2$(2-MePh) | $OSO_2CF_3$ | H | S | |
| 1-523 | 1 | $CH_2$(2,6-$Me_2$Ph) | $OSO_2CF_3$ | H | S | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-524 | 1 | $CH_2$(2-MeOPh) | $OSO_2CF_3$ | H | S | |
| 1-525 | 1 | $CH_2$(2-FPh) | $OSO_2CF_3$ | H | S | |
| 1-526 | 1 | $CH_2$(2-ClPh) | $OSO_2CF_3$ | H | S | |
| 1-527 | 1 | $CH_2$(2,6-$F_2$Ph) | $OSO_2CF_3$ | H | S | |
| 1-528 | 1 | $CH_2$Ph | OH | Me | Racemic | |
| 1-529 | 1 | $CH_2$(2-MePh) | OH | Me | Racemic | |
| 1-530 | 1’ | $CH_2$(2-MePh) | OH | Me | Racemic | |
| 1-531 | 1 | $CH_2$(2,6-$Me_2$Ph) | OH | Me | Racemic | |
| 1-532 | 1 | $CH_2$(2-MeOPh) | OH | Me | Racemic | |
| 1-533 | 1’ | $CH_2$(2-MeOPh) | OH | Me | Racemic | |
| 1-534 | 1 | $CH_2$(2-FPh) | OH | Me | Racemic | |
| 1-535 | 1’ | $CH_2$(2-FPh) | OH | Me | Racemic | |
| 1-536 | 1 | $CH_2$(2-ClPh) | OH | Me | Racemic | |
| 1-537 | 1’ | $CH_2$(2-ClPh) | OH | Me | Racemic | |
| 1-538 | 1 | $CH_2$(2,6-$F_2$Ph) | OH | Me | Racemic | |
| 1-539 | 1’ | $CH_2$(2,6-$F_2$Ph) | OH | Me | Racemic | |
| 1-540 | 1 | $CH_2$(2,6-$Cl_2$Ph) | OH | Me | Racemic | |
| 1-541 | 1’ | $CH_2$(2,6-$Cl_2$Ph) | OH | Me | Racemic | |
| 1-542 | 1 | $CH_2$(2-F-6-ClPh) | OH | Me | Racemic | |
| 1-543 | 1 | $CH_2$(2-$CF_3$Ph) | OH | Me | Racemic | |
| 1-544 | 1’ | $CH_2$(2-$CF_3$Ph) | OH | Me | Racemic | |
| 1-545 | 1 | $CH_2$(2-$CF_3$OPh) | OH | Me | Racemic | |
| 1-546 | 1’ | $CH_2$(2-$CF_3$OPh) | OH | Me | Racemic | |
| 1-547 | 1 | $CH_2$Ph | OH | Et | Racemic | |
| 1-548 | 1 | $CH_2$(2-MePh) | OH | Et | Racemic | |
| 1-549 | 1’ | $CH_2$(2-MePh) | OH | Et | Racemic | |
| 1-550 | 1 | $CH_2$(2,6-$Me_2$Ph) | OH | Et | Racemic | |
| 1-551 | 1 | $CH_2$(2-MeOPh) | OH | Et | Racemic | |
| 1-552 | 1 | $CH_2$(2-FPh) | OH | Et | Racemic | |
| 1-553 | 1’ | $CH_2$(2-FPh) | OH | Et | Racemic | |
| 1-554 | 1 | $CH_2$(2-ClPh) | OH | Et | Racemic | |
| 1-555 | 1’ | $CH_2$(2-ClPh) | OH | Et | Racemic | |

[Table 17]

| Table 1-Continued | | | | | | |
|---|---|---|---|---|---|---|
| No. | General formula | $R^1$ | $R^2$ | $R^3$ | Stereochemistr y at C-5 position | Remark |
| 1-556 | 1 | $CH_2$(2,6-$F_2$Ph) | OH | Et | Racemic | |
| 1-557 | 1’ | $CH_2$(2,6-$F_2$Ph) | OH | Et | Racemic | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-558 | 1 | $CH_2Ph$ | OH | n-Bu | Racemic | |
| 1-559 | 1' | $CH_2Ph$ | OH | n-Bu | Racemic | |
| 1-560 | 1 | $CH_2$(2-MePh) | OH | n-Bu | Racemic | |
| 1-561 | 1' | $CH_2$(2-MePh) | OH | n-Bu | Racemic | |
| 1-562 | 1 | $CH_2$(2,6-$Me_2$Ph) | OH | n-Bu | Racemic | |
| 1-563 | 1 | $CH_2$(2-MeOPh) | OH | n-Bu | Racemic | |
| 1-564 | 1 | $CH_2$(2-FPh) | OH | n-Bu | Racemic | |
| 1-565 | 1' | $CH_2$(2-FPh) | OH | n-Bu | Racemic | |
| 1-566 | 1 | $CH_2$(2-ClPh) | OH | n-Bu | Racemic | |
| 1-567 | 1' | $CH_2$(2-ClPh) | OH | n-Bu | Racemic | |
| 1-568 | 1 | $CH_2$(2,6-$F_2$Ph) | OH | n-Bu | Racemic | |
| 1-569 | 1' | $CH_2$(2,6-$F_2$Ph) | OH | n-Bu | Racemic | |
| 1-570 | 1 | $CH_2Ph$ | Br | $CH_2Br$ | Racemic | |
| 1-571 | 1 | $CH_2$(2-MePh) | Br | $CH_2Br$ | Racemic | |
| 1-572 | 1' | $CH_2$(2-MePh) | Br | $CH_2Br$ | Racemic | |
| 1-573 | 1 | $CH_2$(2,6-$Me_2$Ph) | Br | $CH_2Br$ | Racemic | |
| 1-574 | 1 | $CH_2$(2-MeOPh) | Br | $CH_2Br$ | Racemic | |
| 1-575 | 1 | $CH_2$(2-FPh) | Br | $CH_2Br$ | Racemic | |
| 1-576 | 1' | $CH_2$(2-FPh) | Br | $CH_2Br$ | Racemic | |
| 1-577 | 1 | $CH_2$(2-ClPh) | Br | $CH_2Br$ | Racemic | |
| 1-578 | 1' | $CH_2$(2-ClPh) | Br | $CH_2Br$ | Racemic | |
| 1-579 | 1 | $CH_2$(2,6-$F_2$Ph) | Br | $CH_2Br$ | Racemic | |
| 1-580 | 1' | $CH_2$(2,6-$F_2$Ph) | Br | $CH_2Br$ | Racemic | |
| 1-581 | 1 | $CH_2Ph$ | -$OCH_2$- | | R | |
| 1-582 | 1 | $CH_2$(2-MePh) | -$OCH_2$- | | R | |
| 1-583 | 1' | $CH_2$(2-MePh) | -$OCH_2$- | | R | |
| 1-584 | 1 | $CH_2$(2,6-$Me_2$Ph) | -$OCH_2$- | | R | |
| 1-585 | 1 | $CH_2$(2-MeOPh) | -$OCH_2$- | | R | |
| 1-586 | 1 | $CH_2$(2-FPh) | -$OCH_2$- | | R | |
| 1-587 | 1 | $CH_2$(2-ClPh) | -$OCH_2$- | | R | |
| 1-588 | 1 | $CH_2$(2,6-$F_2$Ph) | -$OCH_2$- | | R | |
| 1-589 | 1 | $CH_2Ph$ | -$CH_2CH_2$- | | - | |
| 1-590 | 1 | $CH_2$(2-MePh) | -$CH_2CH_2$- | | - | |

[Table 18]

| Table 1-Continued | | | | | | |
|---|---|---|---|---|---|---|
| No. | General formula | $R^1$ | $R^2$ | $R^3$ | Stereochemistr y at C-5 position | Remark |
| 1-591 | 1 | $CH_2$(2,6-$Me_2$Ph) | -$CH_2CH_2$- | | - | |

(continued)

| Table 1-Continued | | | | | | |
|---|---|---|---|---|---|---|
| No. | General formula | $R^1$ | $R^2$ | $R^3$ | Stereochemistr y at C-5 position | Remark |
| 1-592 | 1 | $CH_2$(2-MeOPh) | $-CH_2CH_2-$ | | - | |
| 1-593 | 1 | $CH_2$(2-FPh) | $-CH_2CH_2-$ | | - | |
| 1-594 | 1 | $CH_2$(2-ClPh) | $-CH_2CH_2-$ | | - | |
| 1-595 | 1 | $CH_2$(2,6-$F_2$Ph) | $-CH_2CH_2-$ | | - | |
| 1-596 | 1 | $CH_2$(2-MePh) | $OCH_2$(2-MePh) | H | Racemic | |
| 1-597 | 1 | $CH_2$(2-MePh) | $OCH_2$(2-MePh) | H | S | |
| 1-598 | 1 | $CH_2$(2-MePh) | $OCH_2$(2-MePh) | H | R | |
| 1-599 | 1’ | $CH_2$(2-MePh) | $OCH_2$(2-MePh) | H | Racemic | |
| 1-600 | 1’ | $CH_2$(2-MePh) | $OCH_2$(2-MePh) | H | S | |
| 1-601 | 1’ | $CH_2$(2-MePh) | $OCH_2$(2-MePh) | H | R | |
| 1-602 | 1 | $CH_2$(2-FPh) | $OCH_2$(2-FPh) | H | Racemic | |
| 1-603 | 1 | $CH_2$(2-FPh) | $OCH_2$(2-FPh) | H | S | |
| 1-604 | 1 | $CH_2$(2-FPh) | $OCH_2$(2-FPh) | H | R | |
| 1-605 | 1’ | $CH_2$(2-FPh) | $OCH_2$(2-FPh) | H | Racemic | |
| 1-606 | 1’ | $CH_2$(2-FPh) | $OCH_2$(2-FPh) | H | S | |
| 1-607 | 1’ | $CH_2$(2-FPh) | $OCH_2$(2-FPh) | H | R | |
| 1-608 | 1 | Me | Cl | H | S | |
| 1-609 | 1 | n-Hex | Cl | H | S | |
| 1-610 | 1 | $CH_2CF_3$ | Cl | H | S | |
| 1-611 | 1 | $CH_2CH=CH_2$ | Cl | H | S | |
| 1-612 | 1 | $CH_2CH=CMe_2$ | Cl | H | S | |
| 1-613 | 1 | $CH_2C\equiv CH$ | Cl | H | S | |
| 1-614 | 1 | $CH_2C\equiv CMe$ | Cl | H | S | |
| 1-615 | 1 | c-Hex | Cl | H | S | |
| 1-616 | 1 | $CH_2$(c-Hex) | Cl | H | S | |
| 1-617 | 1 | $CH_2OMe$ | Cl | H | S | |
| 1-618 | 1 | 2-Pyridyl | Cl | H | S | |
| 1-619 | 1 | 3-Me-2-Pyridyl | Cl | H | S | |
| 1-620 | 1 | $CH_2$(3-MePh) | Cl | H | S | |
| 1-621 | 1 | $CH_2$(4-MePh) | Cl | H | S | |
| 1-622 | 1 | $CH_2$(2-EtPh) | Cl | H | S | |
| 1-623 | 1 | $CH_2$(3-EtPh) | Cl | H | S | |
| 1-624 | 1 | $CH_2$(4-EtPh) | Cl | H | S | |
| 1-625 | 1 | $CH_2$(3-MeOPh) | Cl | H | S | |

[Table 19]

| Table 1-Continued | | | | | | |
|---|---|---|---|---|---|---|
| No. | General formula | $R^1$ | $R^2$ | $R^3$ | Stereochemistr y at C-5 position | Remark |
| 1-626 | 1 | $CH_2$(4-MeOPh) | Cl | H | S | |
| 1-627 | 1 | $CH_2$(2-$CF_3$OPh) | Cl | H | S | |
| 1-628 | 1 | $CH_2$(3-$CF_3$OPh) | Cl | H | S | |
| 1-629 | 1 | $CH_2$(4-$CF_3$OPh) | Cl | H | S | |
| 1-630 | 1 | $CH_2$(3-FPh) | Cl | H | S | |
| 1-631 | 1 | $CH_2$(4-FPh) | Cl | H | S | |
| 1-632 | 1 | $CH_2$(3-ClPh) | Cl | H | S | |
| 1-633 | 1 | $CH_2$(4-ClPh) | Cl | H | S | |
| 1-634 | 1 | $CH_2$(2-BrPh) | Cl | H | S | |
| 1-635 | 1 | $CH_2$(3-BrPh) | Cl | H | S | |
| 1-636 | 1 | $CH_2$(4-BrPh) | Cl | H | S | |
| 1-637 | 1 | $CH_2$(2-IPh) | Cl | H | S | |
| 1-638 | 1 | $CH_2$(3-IPh) | Cl | H | S | |
| 1-639 | 1 | $CH_2$(4-IPh) | Cl | H | S | |
| 1-640 | 1 | $CH_2$(2-$CF_3$Ph) | Cl | H | S | |
| 1-641 | 1 | $CH_2$(3-$CF_3$Ph) | Cl | H | S | |
| 1-642 | 1 | $CH_2$(4-$CF_3$Ph) | Cl | H | S | |
| 1-643 | 1 | $CH_2$(2-CNPh) | Cl | H | S | |
| 1-644 | 1 | $CH_2$(3-CNPh) | Cl | H | S | |
| 1-645 | 1 | $CH_2$(4-CNPh) | Cl | H | S | |
| 1-646 | 1 | $CH_2$(2-COOHPh) | Cl | H | S | |
| 1-647 | 1 | $CH_2$(3-COOHPh) | Cl | H | S | |
| 1-648 | 1 | $CH_2$(4-COOHPh) | Cl | H | S | |
| 1-649 | 1 | $CH_2$(2-COOMePh) | Cl | H | S | |
| 1-650 | 1 | $CH_2$(3-COOMePh) | Cl | H | S | |
| 1-651 | 1 | $CH_2$(4-COOMePh) | Cl | H | S | |
| 1-652 | 1 | $CH_2$(2,6-$Cl_2$Ph) | Cl | H | S | |
| 1-653 | 1 | $CH_2$(2-Cl-6-FPh) | Cl | H | S | |
| 1-654 | 1 | $CH_2$(2-Br-6-ClPh) | Cl | H | S | |
| 1-655 | 1 | $CH_2$(1-Naphthyl) | Cl | H | S | |
| 1-656 | 1 | $CH_2$(2-Naphthyl) | Cl | H | S | |
| 1-657 | 1 | $CH_2$(2,4-$Cl_2$Ph) | Cl | H | S | |
| 1-658 | 1 | $CH_2$(2-Pyridyl) | Cl | H | S | |
| 1-659 | 1 | $CH_2$(3-Pyridyl) | Cl | H | S | |
| 1-660 | 1 | $CH_2$(4-Pyridyl) | Cl | H | S | |

[Table 20]

| Table 1-Continued | | | | | | |
|---|---|---|---|---|---|---|
| No. | General formula | $R^1$ | $R^2$ | $R^3$ | Stereochemistr y at C-5 position | Remark |
| 1-661 | 1 | $CH_2$(2-Thienyl) | Cl | H | S | |
| 1-662 | 1 | $CH_2$(3-Thienyl) | Cl | H | S | |
| 1-663 | 1 | $CH_2$(3,5-$Me_2$-Isoxazol-4-ly) | Cl | H | S | |
| 1-664 | 1 | $CH_2CH_2OPh$ | Cl | H | S | |
| 1-665 | 1 | $CH_2CH_2OCH_2Ph$ | Cl | H | S | |
| 1-666 | 1 | $CH_2COPh$ | Cl | H | S | |
| 1-667 | 1 | $CH_2OCH_2CH_2OMe$ | Cl | H | S | |
| 1-668 | 1 | Me | Br | H | S | |
| 1-669 | 1 | n-Hex | Br | H | S | |
| 1-670 | 1 | $CH_2CF_3$ | Br | H | S | |
| 1-671 | 1 | $CH_2CH=CH_2$ | Br | H | S | |
| 1-672 | 1 | $CH_2CH=CMe_2$ | Br | H | S | |
| 1-673 | 1 | $CH_2C\equiv CH$ | Br | H | S | |
| 1-674 | 1 | $CH_2C\equiv CMe$ | Br | H | S | |
| 1-675 | 1 | c-Hex | Br | H | S | |
| 1-676 | 1 | $CH_2$(c-Hex) | Br | H | S | |
| 1-677 | 1 | $CH_2OMe$ | Br | H | S | |
| 1-678 | 1 | 2-Pyridyl | Br | H | S | |
| 1-679 | 1 | 3-Me-2-Pyridyl | Br | H | S | |
| 1-680 | 1 | $CH_2$(3-MePh) | Br | H | S | |
| 1-681 | 1 | $CH_2$(4-MePh) | Br | H | S | |
| 1-682 | 1 | $CH_2$(2-EtPh) | Br | H | S | |
| 1-683 | 1 | $CH_2$(3-EtPh) | Br | H | S | |
| 1-684 | 1 | $CH_2$(4-EtPh) | Br | H | S | |
| 1-685 | 1 | $CH_2$(3-MeOPh) | Br | H | S | |
| 1-686 | 1 | $CH_2$(4-MeOPh) | Br | H | S | |
| 1-687 | 1 | $CH_2$(2-$CF_3$OPh) | Br | H | S | |
| 1-688 | 1 | $CH_2$(3-$CF_3$OPh) | Br | H | S | |
| 1-689 | 1 | $CH_2$(4-$CF_3$OPh) | Br | H | S | |
| 1-690 | 1 | $CH_2$(3-FPh) | Br | H | S | |
| 1-691 | 1 | $CH_2$(4-FPh) | Br | H | S | |
| 1-692 | 1 | $CH_2$(3-ClPh) | Br | H | S | |
| 1-693 | 1 | $CH_2$(4-ClPh) | Br | H | S | |
| 1-694 | 1 | $CH_2$(2-BrPh) | Br | H | S | |
| 1-695 | 1 | $CH_2$(3-BrPh) | Br | H | S | |

[Table 21]

| Table 1-Continued | | | | | | |
|---|---|---|---|---|---|---|
| No. | General formula | $R^1$ | $R^2$ | $R^3$ | Stereochemistr y at C-5 position | Remark |
| 1-696 | 1 | $CH_2$(4-BrPh) | Br | H | S | |
| 1-697 | 1 | $CH_2$(2-IPh) | Br | H | S | |
| 1-698 | 1 | $CH_2$(3-IPh) | Br | H | S | |
| 1-699 | 1 | $CH_2$(4-IPh) | Br | H | S | |
| 1-700 | 1 | $CH_2$(2-$CF_3$Ph) | Br | H | S | |
| 1-701 | 1 | $CH_2$(3-$CF_3$Ph) | Br | H | S | |
| 1-702 | 1 | $CH_2$(4-$CF_3$Ph) | Br | H | S | |
| 1-703 | 1 | $CH_2$(2-CNPh) | Br | H | S | |
| 1-704 | 1 | $CH_2$(3-CNPh) | Br | H | S | |
| 1-705 | 1 | $CH_2$(4-CNPh) | Br | H | S | |
| 1-706 | 1 | $CH_2$(2-COOHPh) | Br | H | S | |
| 1-707 | 1 | $CH_2$(3-COOHPh) | Br | H | S | |
| 1-708 | 1 | $CH_2$(4-COOHPh) | Br | H | S | |
| 1-709 | 1 | $CH_2$(2-COOMePh) | Br | H | S | |
| 1-710 | 1 | $CH_2$(3-COOMePh) | Br | H | S | |
| 1-711 | 1 | $CH_2$(4-COOMePh) | Br | H | S | |
| 1-712 | 1 | $CH_2$(2,6-$Cl_2$Ph) | Br | H | S | |
| 1-713 | 1 | $CH_2$(2-Cl-6-FPh) | Br | H | S | |
| 1-714 | 1 | $CH_2$(2-Br-6-ClPh) | Br | H | S | |
| 1-715 | 1 | $CH_2$(1-Naphthyl) | Br | H | S | |
| 1-716 | 1 | $CH_2$(2-Naphthyl) | Br | H | S | |
| 1-717 | 1 | $CH_2$(2,4-$Cl_2$Ph) | Br | H | S | |
| 1-718 | 1 | $CH_2$(2-Pyridyl) | Br | H | S | |
| 1-719 | 1 | $CH_2$(3-Pyridyl) | Br | H | S | |
| 1-720 | 1 | $CH_2$(4-Pyridyl) | Br | H | S | |
| 1-721 | 1 | $CH_2$(2-Thienyl) | Br | H | S | |
| 1-722 | 1 | $CH_2$(3-Thienyl) | Br | H | S | |
| 1-723 | 1 | $CH_2$(3,5-$Me_2$-Isoxazol-4-ly) | Br | H | S | |
| 1-724 | 1 | $CH_2CH_2OPh$ | Br | H | S | |
| 1-725 | 1 | $CH_2CH_2OCH_2Ph$ | Br | H | S | |
| 1-726 | 1 | $CH_2COPh$ | Br | H | S | |
| 1-727 | 1 | $CH_2OCH_2CH_2OMe$ | Br | H | S | |
| 1-728 | 1 | Me | I | H | S | |
| 1-729 | 1 | n-Hex | I | H | S | |
| 1-730 | 1 | $CH_2CF_3$ | I | H | S | |

[Table 22]

| Table 1-Continued | | | | | | |
|---|---|---|---|---|---|---|
| No. | General formula | $R^1$ | $R^2$ | $R^3$ | Stereochemistr y at C-5 position | Remark |
| 1-731 | 1 | $CH_2CH=CH_2$ | I | H | S | |
| 1-732 | 1 | $CH_2CH=CMe_2$ | I | H | S | |
| 1-733 | 1 | $CH_2C\equiv CH$ | I | H | S | |
| 1-734 | 1 | $CH_2C\equiv CMe$ | I | H | S | |
| 1-735 | 1 | c-Hex | I | H | S | |
| 1-736 | 1 | $CH_2$(c-Hex) | I | H | S | |
| 1-737 | 1 | $CH_2OMe$ | I | H | S | |
| 1-738 | 1 | 2-Pyridyl | I | H | S | |
| 1-739 | 1 | 3-Me-2-Pyridyl | I | H | S | |
| 1-740 | 1 | $CH_2Ph$ | I | H | S | |
| 1-741 | 1 | $CH_2$(2-MePh) | I | H | S | |
| 1-742 | 1 | $CH_2$(3-MePh) | I | H | S | |
| 1-743 | 1 | $CH_2$(4-MePh) | I | H | S | |
| 1-744 | 1 | $CH_2$(2-EtPh) | I | H | S | |
| 1-745 | 1 | $CH_2$(3-EtPh) | I | H | S | |
| 1-746 | 1 | $CH_2$(4-EtPh) | I | H | S | |
| 1-747 | 1 | $CH_2$(2-MeOPh) | I | H | S | |
| 1-748 | 1 | $CH_2$(3-MeOPh) | I | H | S | |
| 1-749 | 1 | $CH_2$(4-MeOPh) | I | H | S | |
| 1-750 | 1 | $CH_2$(2-$CF_3$OPh) | I | H | S | |
| 1-751 | 1 | $CH_2$(3-$CF_3$OPh) | I | H | S | |
| 1-752 | 1 | $CH_2$(4-$CF_3$OPh) | I | H | S | |
| 1-753 | 1 | $CH_2$(2-FPh) | I | H | S | |
| 1-754 | 1 | $CH_2$(3-FPh) | I | H | S | |
| 1-755 | 1 | $CH_2$(4-FPh) | I | H | S | |
| 1-756 | 1 | $CH_2$(2-ClPh) | I | H | S | |
| 1-757 | 1 | $CH_2$(3-ClPh) | I | H | S | |
| 1-758 | 1 | $CH_2$(4-ClPh) | I | H | S | |
| 1-759 | 1 | $CH_2$(2-BrPh) | I | H | S | |
| 1-760 | 1 | $CH_2$(3-BrPh) | I | H | S | |
| 1-761 | 1 | $CH_2$(4-BrPh) | I | H | S | |
| 1-762 | 1 | $CH_2$(2-IPh) | I | H | S | |
| 1-763 | 1 | $CH_2$(3-IPh) | I | H | S | |
| 1-764 | 1 | $CH_2$(4-IPh) | I | H | S | |
| 1-765 | 1 | $CH_2$(2-$CF_3$Ph) | I | H | S | |

[Table 23]

| Table 1-Continued | | | | | | |
|---|---|---|---|---|---|---|
| No. | General formula | $R^1$ | $R^2$ | $R^3$ | Stereochemistr y at C-5 position | Remark |
| 1-766 | 1 | $CH_2(3\text{-}CF_3Ph)$ | I | H | S | |
| 1-767 | 1 | $CH_2(4\text{-}CF_3Ph)$ | I | H | S | |
| 1-768 | 1 | $CH_2$(2-CNPh) | I | H | S | |
| 1-769 | 1 | $CH_2$(3-CNPh) | I | H | S | |
| 1-770 | 1 | $CH_2$(4-CNPh) | I | H | S | |
| 1-771 | 1 | $CH_2$(2-COOHPh) | I | H | S | |
| 1-772 | 1 | $CH_2$(3-COOHPh) | I | H | S | |
| 1-773 | 1 | $CH_2$(4-COOHPh) | I | H | S | |
| 1-774 | 1 | $CH_2$(2-COOMePh) | I | H | S | |
| 1-775 | 1 | $CH_2$(3-COOMePh) | I | H | S | |
| 1-776 | 1 | $CH_2$(4-COOMePh) | I | H | S | |
| 1-777 | 1 | $CH_2(2,6\text{-}Me_2Ph)$ | I | H | S | |
| 1-778 | 1 | $CH_2(2,6\text{-}F_2Ph)$ | I | H | S | |
| 1-779 | 1 | $CH_2(2,6\text{-}Cl_2Ph)$ | I | H | S | |
| 1-780 | 1 | $CH_2$(2-Cl-6-FPh) | I | H | S | |
| 1-781 | 1 | $CH_2$(2-Br-6-ClPh) | I | H | S | |
| 1-782 | 1 | $CH_2$(1-Naphthyl) | I | H | S | |
| 1-783 | 1 | $CH_2$(2-Naphthyl) | I | H | S | |
| 1-784 | 1 | $CH_2(2,4\text{-}Cl_2Ph)$ | I | H | S | |
| 1-785 | 1 | $CH_2$(2-Pyridyl) | I | H | S | |
| 1-786 | 1 | $CH_2$(3-Pyridyl) | I | H | S | |
| 1-787 | 1 | $CH_2$(4-Pyridyl) | I | H | S | |
| 1-788 | 1 | $CH_2$(2-Thienyl) | I | H | S | |
| 1-789 | 1 | $CH_2$(3-Thienyl) | I | H | S | |
| 1-790 | 1 | $CH_2(3,5\text{-}Me_2\text{-}$Isoxazol-4-ly) | I | H | S | |
| 1-791 | 1 | $CH_2CH_2OPh$ | I | H | S | |
| 1-792 | 1 | $CH_2CH_2OCH_2Ph$ | I | H | S | |
| 1-793 | 1 | $CH_2COPh$ | I | H | S | |
| 1-794 | 1 | $CH_2OCH_2CH_2OMe$ | I | H | S | |
| 1-795 | 1 | Me | OMe | H | S | |
| 1-796 | 1 | n-Hex | OMe | H | S | |
| 1-797 | 1 | $CH_2CF_3$ | OMe | H | S | |
| 1-798 | 1 | $CH_2CH{=}CH_2$ | OMe | H | S | |
| 1-799 | 1 | $CH_2CH{=}CMe_2$ | OMe | H | S | |
| 1-800 | 1 | $CH_2C{\equiv}CH$ | OMe | H | S | |

[Table 24]

| Table 1-Continued | | | | | | |
|---|---|---|---|---|---|---|
| No. | General formula | $R^1$ | $R^2$ | $R^3$ | Stereochemistr y at C-5 position | Remark |
| 1-801 | 1 | $CH_2C{\equiv}CMe$ | OMe | H | S | |
| 1-802 | 1 | c-Hex | OMe | H | S | |
| 1-803 | 1 | $CH_2$(c-Hex) | OMe | H | S | |
| 1-804 | 1 | $CH_2OMe$ | OMe | H | S | |
| 1-805 | 1 | 2-Pyridyl | OMe | H | S | |
| 1-806 | 1 | 3-Me-2-Pyridyl | OMe | H | S | |
| 1-807 | 1 | $CH_2$(3-MePh) | OMe | H | S | |
| 1-808 | 1 | $CH_2$(4-MePh) | OMe | H | S | |
| 1-809 | 1 | $CH_2$(2-EtPh) | OMe | H | S | |
| 1-810 | 1 | $CH_2$(3-EtPh) | OMe | H | S | |
| 1-811 | 1 | $CH_2$(4-EtPh) | OMe | H | S | |
| 1-812 | 1 | $CH_2$(3-MeOPh) | OMe | H | S | |
| 1-813 | 1 | $CH_2$(4-MeOPh) | OMe | H | S | |
| 1-814 | 1 | $CH_2$(2-$CF_3$OPh) | OMe | H | S | |
| 1-815 | 1 | $CH_2$(3-$CF_3$OPh) | OMe | H | S | |
| 1-816 | 1 | $CH_2$(4-$CF_3$OPh) | OMe | H | S | |
| 1-817 | 1 | $CH_2$(3-FPh) | OMe | H | S | |
| 1-818 | 1 | $CH_2$(4-FPh) | OMe | H | S | |
| 1-819 | 1 | $CH_2$(3-ClPh) | OMe | H | S | |
| 1-820 | 1 | $CH_2$(4-ClPh) | OMe | H | S | |
| 1-821 | 1 | $CH_2$(3-BrPh) | OMe | H | S | |
| 1-822 | 1 | $CH_2$(4-BrPh) | OMe | H | S | |
| 1-823 | 1 | $CH_2$(2-IPh) | OMe | H | S | |
| 1-824 | 1 | $CH_2$(3-IPh) | OMe | H | S | |
| 1-825 | 1 | $CH_2$(4-IPh) | OMe | H | S | |
| 1-826 | 1 | $CH_2$(3-$CF_3$Ph) | OMe | H | S | |
| 1-827 | 1 | $CH_2$(4-$CF_3$Ph) | OMe | H | S | |
| 1-828 | 1 | $CH_2$(2-CNPh) | OMe | H | S | |
| 1-829 | 1 | $CH_2$(3-CNPh) | OMe | H | S | |
| 1-830 | 1 | $CH_2$(4-CNPh) | OMe | H | S | |
| 1-831 | 1 | $CH_2$(2-COOHPh) | OMe | H | S | |
| 1-832 | 1 | $CH_2$(3-COOHPh) | OMe | H | S | |
| 1-833 | 1 | $CH_2$(4-COOHPh) | OMe | H | S | |
| 1-834 | 1 | $CH_2$(2-COOMePh) | OMe | H | S | |
| 1-835 | 1 | $CH_2$(3-COOMePh) | OMe | H | S | |

[Table 25]

| Table 1-Continued | | | | | | |
|---|---|---|---|---|---|---|
| No. | General formula | $R^1$ | $R^2$ | $R^3$ | Stereochemistr y at C-5 position | Remark |
| 1-836 | 1 | $CH_2$(4-COOMePh) | OMe | H | S | |
| 1-837 | 1 | $CH_2$(2-Cl-6-FPh) | OMe | H | S | |
| 1-838 | 1 | $CH_2$(2-Br-6-ClPh) | OMe | H | S | |
| 1-839 | 1 | $CH_2$(1-Naphthyl) | OMe | H | S | |
| 1-840 | 1 | $CH_2$(2-Naphthyl) | OMe | H | S | |
| 1-841 | 1 | $CH_2(2,4\text{-}Cl_2Ph)$ | OMe | H | S | |
| 1-842 | 1 | $CH_2$(2-Pyridyl) | OMe | H | S | |
| 1-843 | 1 | $CH_2$(3-Pyridyl) | OMe | H | S | |
| 1-844 | 1 | $CH_2$(4-Pyridyl) | OMe | H | S | |
| 1-845 | 1 | $CH_2$(2-Thienyl) | OMe | H | S | |
| 1-846 | 1 | $CH_2$(3-Thienyl) | OMe | H | S | |
| 1-847 | 1 | $CH_2(3,5\text{-}Me_2$-Isoxazol-4-ly) | OMe | H | S | |
| 1-848 | 1 | $CH_2CH_2OPh$ | OMe | H | S | |
| 1-849 | 1 | $CH_2CH_2OCH_2Ph$ | OMe | H | S | |
| 1-850 | 1 | $CH_2COPh$ | OMe | H | S | |
| 1-851 | 1 | $CH_2OCH_2CH_2OMe$ | OMe | H | S | |
| 1-852 | 1 | Me | Me | H | S | |
| 1-853 | 1 | n-Hex | Me | H | S | |
| 1-854 | 1 | $CH_2CF_3$ | Me | H | S | |
| 1-855 | 1 | $CH_2CH=CH_2$ | Me | H | S | |
| 1-856 | 1 | $CH_2CH=CMe_2$ | Me | H | S | |
| 1-857 | 1 | $CH_2C{\equiv}CH$ | Me | H | S | |
| 1-858 | 1 | $CH_2C{\equiv}CMe$ | Me | H | S | |
| 1-859 | 1 | c-Hex | Me | H | S | |
| 1-860 | 1 | $CH_2$(c-Hex) | Me | H | S | |
| 1-861 | 1 | $CH_2OMe$ | Me | H | S | |
| 1-862 | 1 | 2-Pyridyl | Me | H | S | |
| 1-863 | 1 | 3-Me-2-Pyridyl | Me | H | S | |
| 1-864 | 1 | $CH_2Ph$ | Me | H | S | |
| 1-865 | 1 | $CH_2$(3-MePh) | Me | H | S | |
| 1-866 | 1 | $CH_2$(4-MePh) | Me | H | S | |
| 1-867 | 1 | $CH_2$(2-EtPh) | Me | H | S | |
| 1-868 | 1 | $CH_2$(3-EtPh) | Me | H | S | |
| 1-869 | 1 | $CH_2$(4-EtPh) | Me | H | S | |
| 1-870 | 1 | $CH_2$(3-MeOPh) | Me | H | S | |

[Table 26]

| Table 1-Continued | | | | | | |
|---|---|---|---|---|---|---|
| No. | General formula | $R^1$ | $R^2$ | $R^3$ | Stereochemistr y at C-5 position | Remark |
| 1-871 | 1 | $CH_2$(4-MeOPh) | Me | H | S | |
| 1-872 | 1 | $CH_2$(2-$CF_3$OPh) | Me | H | S | |
| 1-873 | 1 | $CH_2$(3-$CF_3$OPh) | Me | H | S | |
| 1-874 | 1 | $CH_2$(4-$CF_3$OPh) | Me | H | S | |
| 1-875 | 1 | $CH_2$(3-FPh) | Me | H | S | |
| 1-876 | 1 | $CH_2$(4-FPh) | Me | H | S | |
| 1-877 | 1 | $CH_2$(3-ClPh) | Me | H | S | |
| 1-878 | 1 | $CH_2$(4-ClPh) | Me | H | S | |
| 1-879 | 1 | $CH_2$(2-BrPh) | Me | H | S | |
| 1-880 | 1 | $CH_2$(3-BrPh) | Me | H | S | |
| 1-881 | 1 | $CH_2$(4-BrPh) | Me | H | S | |
| 1-882 | 1 | $CH_2$(2-IPh) | Me | H | S | |
| 1-883 | 1 | $CH_2$(3-IPh) | Me | H | S | |
| 1-884 | 1 | $CH_2$(4-IPh) | Me | H | S | |
| 1-885 | 1 | $CH_2$(2-$CF_3$Ph) | Me | H | S | |
| 1-886 | 1 | $CH_2$(3-$CF_3$Ph) | Me | H | S | |
| 1-887 | 1 | $CH_2$(4-$CF_3$Ph) | Me | H | S | |
| 1-888 | 1 | $CH_2$(2-CNPh) | Me | H | S | |
| 1-889 | 1 | $CH_2$(3-CNPh) | Me | H | S | |
| 1-890 | 1 | $CH_2$(4-CNPh) | Me | H | S | |
| 1-891 | 1 | $CH_2$(2-COOHPh) | Me | H | S | |
| 1-892 | 1 | $CH_2$(3-COOHPh) | Me | H | S | |
| 1-893 | 1 | $CH_2$(4-COOHPh) | Me | H | S | |
| 1-894 | 1 | $CH_2$(2-COOMePh) | Me | H | S | |
| 1-895 | 1 | $CH_2$(3-COOMePh) | Me | H | S | |
| 1-896 | 1 | $CH_2$(4-COOMePh) | Me | H | S | |
| 1-897 | 1 | $CH_2$(2,6-$Me_2$Ph) | Me | H | S | |
| 1-898 | 1 | $CH_2$(2,6-$Cl_2$Ph) | Me | H | S | |
| 1-899 | 1 | $CH_2$(2-Cl-6-FPh) | Me | H | S | |
| 1-900 | 1 | $CH_2$(2-Br-6-ClPh) | Me | H | S | |
| 1-901 | 1 | $CH_2$(1-Naphthyl) | Me | H | S | |
| 1-902 | 1 | $CH_2$(2-Naphthyl) | Me | H | S | |
| 1-903 | 1 | $CH_2$(2,4-$Cl_2$Ph) | Me | H | S | |
| 1-904 | 1 | $CH_2$(2-Pyridyl) | Me | H | S | |
| 1-905 | 1 | $CH_2$(3-Pyridyl) | Me | H | S | |

[Table 27]

| Table 1-Continued | | | | | | |
|---|---|---|---|---|---|---|
| No. | General formula | $R^1$ | $R^2$ | $R^3$ | Stereochemistr y at C-5 position | Remark |
| 1-906 | 1 | $CH_2$(4-Pyridyl) | Me | H | S | |
| 1-907 | 1 | $CH_2$(2-Thienyl) | Me | H | S | |
| 1-908 | 1 | $CH_2$(3-Thienyl) | Me | H | S | |
| 1-909 | 1 | $CH_2$(3,5-$Me_2$-Isoxazol-4-ly) | Me | H | S | |
| 1-910 | 1 | $CH_2CH_2OPh$ | Me | H | S | |
| 1-911 | 1 | $CH_2CH_2OCH_2Ph$ | Me | H | S | |
| 1-912 | 1 | $CH_2COPh$ | Me | H | S | |
| 1-913 | 1 | $CH_2OCH_2CH_2OMe$ | Me | H | S | |
| 1-914 | 1 | $CH_2$(2-FPh) | n-Pr | H | R | |
| 1-915 | 1 | $CH_2$(2-FPh) | i-Bu | H | Racemic | |
| 1-916 | 1 | $CH_2$(2-FPh) | OEt | H | R | |
| 1-917 | 1 | $CH_2$(2-FPh) | On-Pr | H | R | |
| 1-918 | 1 | $CH_2$(2-FPh) | OBn | H | R | |
| 1-919 | 1 | $CH_2$(2-FPh) | $OCH_2$(2-Pyridyl) | H | R | |
| 1-920 | 1 | $CH_2$(2-FPh) | c-Pen | H | Racemic | |
| 1-921 | 1 | $CH_2$(2-FPh) | $OCH_2$c-Pr | H | R | |
| 1-922 | 1 | $CH_2$(2-FPh) | 2-FPh | H | Racemic | |
| 1-923 | 1 | $CH_2$(2-FPh) | 3-FPh | H | Racemic | |
| 1-924 | 1 | $CH_2$(2-FPh) | 4-FPh | H | Racemic | |
| 1-925 | 1 | $CH_2$(2-FPh) | 2-MePh | H | Racemic | |
| 1-926 | 1 | $CH_2$(2-FPh) | 3-MePh | H | Racemic | |
| 1-927 | 1 | $CH_2$(2-FPh) | 4-MePh | H | Racemic | |
| 1-928 | 1 | $CH_2$(2-FPh) | 3-Thienyl | H | Racemic | |
| 1-929 | 1 | $CH_2$(2-FPh) | $CH_2CH_2Ph$ | H | Racemic | |
| 1-930 | 1 | $CH_2$(2-FPh) | $OCH_2CH=CH_2$ | H | R | |
| 1-931 | 1 | $CH_2$(2-FPh) | $OCH_2C\equiv CH$ | H | R | |
| 1-932 | 1 | $CH_2$(2-FPh) | OCOMe | H | R | |
| 1-933 | 1 | $CH_2$(2-FPh) | OCONHEt | H | R | |
| 1-934 | 1 | $CH_2$(2-FPh) | n-Pr | OH | S | |
| 1-935 | 1 | $CH_2$(2-FPh) | OMe | OMe | - | |
| 1-936 | 1 | $CH_2$(2-FPh) | $-OCH_2CH_2O-$ | | - | |
| 1-937 | 1’ | Me | Cl | H | R | |
| 1-938 | 1’ | n-Hex | Cl | H | R | |
| 1-939 | 1’ | $CH_2CF_3$ | Cl | H | R | |
| 1-940 | 1’ | $CH_2CH=CH_2$ | Cl | H | R | |

[Table 28]

| Table 1-Continued | | | | | | |
|---|---|---|---|---|---|---|
| No. | General formula | $R^1$ | $R^2$ | $R^3$ | Stereochemistr y at C-5 position | Remark |
| 1-941 | 1' | $CH_2CH=CMe_2$ | Cl | H | R | |
| 1-942 | 1' | $CH_2C≡CH$ | Cl | H | R | |
| 1-943 | 1' | $CH_2C≡CMe$ | Cl | H | R | |
| 1-944 | 1' | c-Hex | Cl | H | R | |
| 1-945 | 1' | $CH_2$(c-Hex) | Cl | H | R | |
| 1-946 | 1' | $CH_2OMe$ | Cl | H | R | |
| 1-947 | 1' | 2-Pyridyl | Cl | H | R | |
| 1-948 | 1' | 3-Me-2-Pyridyl | Cl | H | R | |
| 1-949 | 1' | $CH_2Ph$ | Cl | H | R | |
| 1-950 | 1' | $CH_2$(2-MePh) | Cl | H | R | |
| 1-951 | 1' | $CH_2$(3-MePh) | Cl | H | R | |
| 1-952 | 1' | $CH_2$(4-MePh) | Cl | H | R | |
| 1-953 | 1' | $CH_2$(2-EtPh) | Cl | H | R | |
| 1-954 | 1' | $CH_2$(3-EtPh) | Cl | H | R | |
| 1-955 | 1' | $CH_2$(4-EtPh) | Cl | H | R | |
| 1-956 | 1' | $CH_2$(2-MeOPh) | Cl | H | R | |
| 1-957 | 1' | $CH_2$(3-MeOPh) | Cl | H | R | |
| 1-958 | 1' | $CH_2$(4-MeOPh) | Cl | H | R | |
| 1-959 | 1' | $CH_2$(2-$CF_3$OPh) | Cl | H | R | |
| 1-960 | 1' | $CH_2$(3-$CF_3$OPh) | Cl | H | R | |
| 1-961 | 1' | $CH_2$(4-$CF_3$OPh) | Cl | H | R | |
| 1-962 | 1' | $CH_2$(2-FPh) | Cl | H | R | |
| 1-963 | 1' | $CH_2$(3-FPh) | Cl | H | R | |
| 1-964 | 1' | $CH_2$(4-FPh) | Cl | H | R | |
| 1-965 | 1' | $CH_2$(2-ClPh) | Cl | H | R | |
| 1-966 | 1' | $CH_2$(3-ClPh) | Cl | H | R | |
| 1-967 | 1' | $CH_2$(4-ClPh) | Cl | H | R | |
| 1-968 | 1' | $CH_2$(2-BrPh) | Cl | H | R | |
| 1-969 | 1' | $CH_2$(3-BrPh) | Cl | H | R | |
| 1-970 | 1' | $CH_2$(4-BrPh) | Cl | H | R | |
| 1-971 | 1' | $CH_2$(2-IPh) | Cl | H | R | |
| 1-972 | 1' | $CH_2$(3-IPh) | Cl | H | R | |
| 1-973 | 1' | $CH_2$(4-IPh) | Cl | H | R | |
| 1-974 | 1' | $CH_2$(2-$CF_3$Ph) | Cl | H | R | |
| 1-975 | 1' | $CH_2$(3-$CF_3$Ph) | Cl | H | R | |

[Table 29]

| Table 1-Continued | | | | | | |
|---|---|---|---|---|---|---|
| No. | General formula | $R^1$ | $R^2$ | $R^3$ | Stereochemistr y at C-5 position | Remark |
| 1-976 | 1' | $CH_2(4\text{-}CF_3Ph)$ | Cl | H | R | |
| 1-977 | 1' | $CH_2$(2-CNPh) | Cl | H | R | |
| 1-978 | 1' | $CH_2$(3-CNPh) | Cl | H | R | |
| 1-979 | 1' | $CH_2$(4-CNPh) | Cl | H | R | |
| 1-980 | 1' | $CH_2$(2-COOHPh) | Cl | H | R | |
| 1-981 | 1' | $CH_2$(3-COOHPh) | Cl | H | R | |
| 1-982 | 1' | $CH_2$(4-COOHPh) | Cl | H | R | |
| 1-983 | 1' | $CH_2$(2-COOMePh) | Cl | H | R | |
| 1-984 | 1' | $CH_2$(3-COOMePh) | Cl | H | R | |
| 1-985 | 1' | $CH_2$(4-COOMePh) | Cl | H | R | |
| 1-986 | 1' | $CH_2(2,6\text{-}F_2Ph)$ | Cl | H | R | |
| 1-987 | 1' | $CH_2(2,6\text{-}Cl_2Ph)$ | Cl | H | R | |
| 1-988 | 1' | $CH_2$(2-Cl-6-FPh) | Cl | H | R | |
| 1-989 | 1' | $CH_2$(2-Br-6-ClPh) | Cl | H | R | |
| 1-990 | 1' | $CH_2$(1-Naphthyl) | Cl | H | R | |
| 1-991 | 1' | $CH_2$(2-Naphthyl) | Cl | H | R | |
| 1-992 | 1' | $CH_2(2,4\text{-}Cl_2Ph)$ | Cl | H | R | |
| 1-993 | 1' | $CH_2$(2-Pyridyl) | Cl | H | R | |
| 1-994 | 1' | $CH_2$(3-Pyridyl) | Cl | H | R | |
| 1-995 | 1' | $CH_2$(4-Pyridyl) | Cl | H | R | |
| 1-996 | 1' | $CH_2$(2-Thienyl) | Cl | H | R | |
| 1-997 | 1' | $CH_2$(3-Thienyl) | Cl | H | R | |
| 1-998 | 1' | $CH_2(3,5\text{-}Me_2\text{-}Isoxazol\text{-}4\text{-}ly)$ | Cl | H | R | |
| 1-999 | 1' | $CH_2CH_2OPh$ | Cl | H | R | |
| 1-1000 | 1' | $CH_2CH_2OCH_2Ph$ | Cl | H | R | |
| 1-1001 | 1' | $CH_2COPh$ | Cl | H | R | |
| 1-1002 | 1' | $CH_2OCH_2CH_2OMe$ | Cl | H | R | |
| 1-1003 | 1' | Me | Br | H | R | |
| 1-1004 | 1' | n-Hex | Br | H | R | |
| 1-1005 | 1' | $CH_2CF_3$ | Br | H | R | |
| 1-1006 | 1' | $CH_2CH=CH_2$ | Br | H | R | |
| 1-1007 | 1' | $CH_2CH=CMe_2$ | Br | H | R | |
| 1-1008 | 1' | $CH_2C\equiv CH$ | Br | H | R | |
| 1-1009 | 1' | $CH_2C\equiv CMe$ | Br | H | R | |
| 1-1010 | 1' | c-Hex | Br | H | R | |

[Table 30]

| Table 1-Continued | | | | | | |
|---|---|---|---|---|---|---|
| No. | General formula | $R^1$ | $R^2$ | $R^3$ | Stereochemistr y at C-5 position | Remark |
| 1-1011 | 1' | $CH_2$(c-Hex) | Br | H | R | |
| 1-1012 | 1' | $CH_2OMe$ | Br | H | R | |
| 1-1013 | 1' | 2-Pyridyl | Br | H | R | |
| 1-1014 | 1' | 3-Me-2-Pyridyl | Br | H | R | |
| 1-1015 | 1' | $CH_2Ph$ | Br | H | R | |
| 1-1016 | 1' | $CH_2$(2-MePh) | Br | H | R | |
| 1-1017 | 1' | $CH_2$(3-MePh) | Br | H | R | |
| 1-1018 | 1' | $CH_2$(4-MePh) | Br | H | R | |
| 1-1019 | 1' | $CH_2$(2-EtPh) | Br | H | R | |
| 1-1020 | 1' | $CH_2$(3-EtPh) | Br | H | R | |
| 1-1021 | 1' | $CH_2$(4-EtPh) | Br | H | R | |
| 1-1022 | 1' | $CH_2$(2-MeOPh) | Br | H | R | |
| 1-1023 | 1' | $CH_2$(3-MeOPh) | Br | H | R | |
| 1-1024 | 1' | $CH_2$(4-MeOPh) | Br | H | R | |
| 1-1025 | 1' | $CH_2$(2-$CF_3$OPh) | Br | H | R | |
| 1-1026 | 1' | $CH_2$(3-$CF_3$OPh) | Br | H | R | |
| 1-1027 | 1' | $CH_2$(4-$CF_3$OPh) | Br | H | R | |
| 1-1028 | 1' | $CH_2$(2-FPh) | Br | H | R | |
| 1-1029 | 1' | $CH_2$(3-FPh) | Br | H | R | |
| 1-1030 | 1' | $CH_2$(4-FPh) | Br | H | R | |
| 1-1031 | 1' | $CH_2$(2-ClPh) | Br | H | R | |
| 1-1032 | 1' | $CH_2$(3-ClPh) | Br | H | R | |
| 1-1033 | 1' | $CH_2$(4-ClPh) | Br | H | R | |
| 1-1034 | 1' | $CH_2$(2-BrPh) | Br | H | R | |
| 1-1035 | 1' | $CH_2$(3-BrPh) | Br | H | R | |
| 1-1036 | 1' | $CH_2$(4-BrPh) | Br | H | R | |
| 1-1037 | 1' | $CH_2$(2-IPh) | Br | H | R | |
| 1-1038 | 1' | $CH_2$(3-IPh) | Br | H | R | |
| 1-1039 | 1' | $CH_2$(4-IPh) | Br | H | R | |
| 1-1040 | 1' | $CH_2$(2-$CF_3$Ph) | Br | H | R | |
| 1-1041 | 1' | $CH_2$(3-$CF_3$Ph) | Br | H | R | |
| 1-1042 | 1' | $CH_2$(4-$CF_3$Ph) | Br | H | R | |
| 1-1043 | 1' | $CH_2$(2-CNPh) | Br | H | R | |
| 1-1044 | 1' | $CH_2$(3-CNPh) | Br | H | R | |
| 1-1045 | 1' | $CH_2$(4-CNPh) | Br | H | R | |

[Table 31]

| Table 1-Continued | | | | | | |
|---|---|---|---|---|---|---|
| No. | General formula | $R^1$ | $R^2$ | $R^3$ | Stereochemistr y at C-5 position | Remark |
| 1-1046 | 1’ | $CH_2$(2-COOHPh) | Br | H | R | |
| 1-1047 | 1’ | $CH_2$(3-COOHPh) | Br | H | R | |
| 1-1048 | 1’ | $CH_2$(4-COOHPh) | Br | H | R | |
| 1-1049 | 1’ | $CH_2$(2-COOMePh) | Br | H | R | |
| 1-1050 | 1’ | $CH_2$(3-COOMePh) | Br | H | R | |
| 1-1051 | 1’ | $CH_2$(4-COOMePh) | Br | H | R | |
| 1-1052 | 1’ | $CH_2$(2,6-$F_2$Ph) | Br | H | R | |
| 1-1053 | 1’ | $CH_2$(2,6-$Cl_2$Ph) | Br | H | R | |
| 1-1054 | 1’ | $CH_2$(2-Cl-6-FPh) | Br | H | R | |
| 1-1055 | 1’ | $CH_2$(2-Br-6-ClPh) | Br | H | R | |
| 1-1056 | 1’ | $CH_2$(1-Naphthyl) | Br | H | R | |
| 1-1057 | 1’ | $CH_2$(2-Naphthyl) | Br | H | R | |
| 1-1058 | 1’ | $CH_2$(2,4-$Cl_2$Ph) | Br | H | R | |
| 1-1059 | 1’ | $CH_2$(2-Pyridyl) | Br | H | R | |
| 1-1060 | 1’ | $CH_2$(3-Pyridyl) | Br | H | R | |
| 1-1061 | 1’ | $CH_2$(4-Pyridyl) | Br | H | R | |
| 1-1062 | 1’ | $CH_2$(2-Thienyl) | Br | H | R | |
| 1-1063 | 1’ | $CH_2$(3-Thienyl) | Br | H | R | |
| 1-1064 | 1’ | $CH_2$(3,5-$Me_2$-Isoxazol-4-ly) | Br | H | R | |
| 1-1065 | 1’ | $CH_2CH_2OPh$ | Br | H | R | |
| 1-1066 | 1’ | $CH_2CH_2OCH_2Ph$ | Br | H | R | |
| 1-1067 | 1’ | $CH_2COPh$ | Br | H | R | |
| 1-1068 | 1’ | $CH_2OCH_2CH_2OMe$ | Br | H | R | |
| 1-1069 | 1’ | Me | I | H | R | |
| 1-1070 | 1’ | n-Hex | I | H | R | |
| 1-1071 | 1’ | $CH_2CF_3$ | I | H | R | |
| 1-1072 | 1’ | $CH_2CH=CH_2$ | I | H | R | |
| 1-1073 | 1’ | $CH_2CH=CMe_2$ | I | H | R | |
| 1-1074 | 1’ | $CH_2C≡CH$ | I | H | R | |
| 1-1075 | 1’ | $CH_2C≡CMe$ | I | H | R | |
| 1-1076 | 1’ | c-Hex | I | H | R | |
| 1-1077 | 1’ | $CH_2$(c-Hex) | I | H | R | |
| 1-1078 | 1’ | $CH_2OMe$ | I | H | R | |
| 1-1079 | 1’ | 2-Pyridyl | I | H | R | |
| 1-1080 | 1’ | 3-Me-2-Pyridyl | I | H | R | |

[Table 32]

| Table 1-Continued | | | | | | |
|---|---|---|---|---|---|---|
| No. | General formula | $R^1$ | $R^2$ | $R^3$ | Stereochemistr y at C-5 position | Remark |
| 1-1081 | 1' | $CH_2Ph$ | I | H | R | |
| 1-1082 | 1' | $CH_2$(2-MePh) | I | H | R | |
| 1-1083 | 1' | $CH_2$(3-MePh) | I | H | R | |
| 1-1084 | 1' | $CH_2$(4-MePh) | I | H | R | |
| 1-1085 | 1' | $CH_2$(2-EtPh) | I | H | R | |
| 1-1086 | 1' | $CH_2$(3-EtPh) | I | H | R | |
| 1-1087 | 1' | $CH_2$(4-EtPh) | I | H | R | |
| 1-1088 | 1' | $CH_2$(2-MeOPh) | I | H | R | |
| 1-1089 | 1' | $CH_2$(3-MeOPh) | I | H | R | |
| 1-1090 | 1' | $CH_2$(4-MeOPh) | I | H | R | |
| 1-1091 | 1' | $CH_2$(2-$CF_3$OPh) | I | H | R | |
| 1-1092 | 1' | $CH_2$(3-$CF_3$OPh) | I | H | R | |
| 1-1093 | 1' | $CH_2$(4-$CF_3$OPh) | I | H | R | |
| 1-1094 | 1' | $CH_2$(2-FPh) | I | H | R | |
| 1-1095 | 1' | $CH_2$(3-FPh) | I | H | R | |
| 1-1096 | 1' | $CH_2$(4-FPh) | I | H | R | |
| 1-1097 | 1' | $CH_2$(2-ClPh) | I | H | R | |
| 1-1098 | 1' | $CH_2$(3-ClPh) | I | H | R | |
| 1-1099 | 1' | $CH_2$(4-ClPh) | I | H | R | |
| 1-1100 | 1' | $CH_2$(2-BrPh) | I | H | R | |
| 1-1101 | 1' | $CH_2$(3-BrPh) | I | H | R | |
| 1-1102 | 1' | $CH_2$(4-BrPh) | I | H | R | |
| 1-1103 | 1' | $CH_2$(2-IPh) | I | H | R | |
| 1-1104 | 1' | $CH_2$(3-IPh) | I | H | R | |
| 1-1105 | 1' | $CH_2$(4-IPh) | I | H | R | |
| 1-1106 | 1' | $CH_2$(2-$CF_3$Ph) | I | H | R | |
| 1-1107 | 1' | $CH_2$(3-$CF_3$Ph) | I | H | R | |
| 1-1108 | 1' | $CH_2$(4-$CF_3$Ph) | I | H | R | |
| 1-1109 | 1' | $CH_2$(2-CNPh) | I | H | R | |
| 1-1110 | 1' | $CH_2$(3-CNPh) | I | H | R | |
| 1-1111 | 1' | $CH_2$(4-CNPh) | I | H | R | |
| 1-1112 | 1' | $CH_2$(2-COOHPh) | I | H | R | |
| 1-1113 | 1' | $CH_2$(3-COOHPh) | I | H | R | |
| 1-1114 | 1' | $CH_2$(4-COOHPh) | I | H | R | |
| 1-1115 | 1' | $CH_2$(2-COOMePh) | I | H | R | |

[Table 33]

| Table 1-Continued | | | | | | |
|---|---|---|---|---|---|---|
| No. | General formula | $R^1$ | $R^2$ | $R^3$ | Stereochemistr y at C-5 position | Remark |
| 1-1116 | 1’ | $CH_2$(3-COOMePh) | I | H | R | |
| 1-1117 | 1’ | $CH_2$(4-COOMePh) | I | H | R | |
| 1-1118 | 1’ | $CH_2$(2,6-$Me_2$Ph) | I | H | R | |
| 1-1119 | 1’ | $CH_2$(2,6-$F_2$Ph) | I | H | R | |
| 1-1120 | 1’ | $CH_2$(2,6-$Cl_2$Ph) | I | H | R | |
| 1-1121 | 1’ | $CH_2$(2-Cl-6-FPh) | I | H | R | |
| 1-1122 | 1’ | $CH_2$(2-Br-6-ClPh) | I | H | R | |
| 1-1123 | 1’ | $CH_2$(1-Naphthyl) | I | H | R | |
| 1-1124 | 1’ | $CH_2$(2-Naphthyl) | I | H | R | |
| 1-1125 | 1’ | $CH_2$(2,4-$Cl_2$Ph) | I | H | R | |
| 1-1126 | 1’ | $CH_2$(2-Pyridyl) | I | H | R | |
| 1-1127 | 1’ | $CH_2$(3-Pyridyl) | I | H | R | |
| 1-1128 | 1’ | $CH_2$(4-Pyridyl) | I | H | R | |
| 1-1129 | 1’ | $CH_2$(2-Thienyl) | I | H | R | |
| 1-1130 | 1’ | $CH_2$(3-Thienyl) | I | H | R | |
| 1-1131 | 1’ | $CH_2$(3,5-$Me_2$-Isoxazol-4-ly) | I | H | R | |
| 1-1132 | 1’ | $CH_2CH_2OPh$ | I | H | R | |
| 1-1133 | 1’ | $CH_2CH_2OCH_2Ph$ | I | H | R | |
| 1-1134 | 1’ | $CH_2COPh$ | I | H | R | |
| 1-1135 | 1’ | $CH_2OCH_2CH_2OMe$ | I | H | R | |
| 1-1136 | 1’ | Me | OMe | H | R | |
| 1-1137 | 1’ | n-Hex | OMe | H | R | |
| 1-1138 | 1’ | $CH_2CF_3$ | OMe | H | R | |
| 1-1139 | 1’ | $CH_2CH=CH_2$ | OMe | H | R | |
| 1-1140 | 1’ | $CH_2CH=CMe_2$ | OMe | H | R | |
| 1-1141 | 1’ | $CH_2C\equiv CH$ | OMe | H | R | |
| 1-1142 | 1’ | $CH_2C\equiv CMe$ | OMe | H | R | |
| 1-1143 | 1’ | c-Hex | OMe | H | R | |
| 1-1144 | 1’ | $CH_2$(c-Hex) | OMe | H | R | |
| 1-1145 | 1’ | $CH_2OMe$ | OMe | H | R | |
| 1-1146 | 1’ | 2-Pyridyl | OMe | H | R | |
| 1-1147 | 1’ | 3-Me-2-Pyridyl | OMe | H | R | |
| 1-1148 | 1’ | $CH_2$(3-MePh) | OMe | H | R | |
| 1-1149 | 1’ | $CH_2$(4-MePh) | OMe | H | R | |
| 1-1150 | 1’ | $CH_2$(2-EtPh) | OMe | H | R | |

[Table 34]

| Table 1-Continued | | | | | | |
|---|---|---|---|---|---|---|
| No. | General formula | $R^1$ | $R^2$ | $R^3$ | Stereochemistr y at C-5 position | Remark |
| 1-1151 | 1' | $CH_2$(3-EtPh) | OMe | H | R | |
| 1-1152 | 1' | $CH_2$(4-EtPh) | OMe | H | R | |
| 1-1153 | 1' | $CH_2$(3-MeOPh) | OMe | H | R | |
| 1-1154 | 1' | $CH_2$(4-MeOPh) | OMe | H | R | |
| 1-1155 | 1' | $CH_2$(2-$CF_3$OPh) | OMe | H | R | |
| 1-1156 | 1' | $CH_2$(3-$CF_3$OPh) | OMe | H | R | |
| 1-1157 | 1' | $CH_2$(4-$CF_3$OPh) | OMe | H | R | |
| 1-1158 | 1' | $CH_2$(3-FPh) | OMe | H | R | |
| 1-1159 | 1' | $CH_2$(4-FPh) | OMe | H | R | |
| 1-1160 | 1' | $CH_2$(3-ClPh) | OMe | H | R | |
| 1-1161 | 1' | $CH_2$(4-ClPh) | OMe | H | R | |
| 1-1162 | 1' | $CH_2$(2-BrPh) | OMe | H | R | |
| 1-1163 | 1' | $CH_2$(3-BrPh) | OMe | H | R | |
| 1-1164 | 1' | $CH_2$(4-BrPh) | OMe | H | R | |
| 1-1165 | 1' | $CH_2$(2-IPh) | OMe | H | R | |
| 1-1166 | 1' | $CH_2$(3-IPh) | OMe | H | R | |
| 1-1167 | 1' | $CH_2$(4-IPh) | OMe | H | R | |
| 1-1168 | 1' | $CH_2$(2-$CF_3$Ph) | OMe | H | R | |
| 1-1169 | 1' | $CH_2$(3-$CF_3$Ph) | OMe | H | R | |
| 1-1170 | 1' | $CH_2$(4-$CF_3$Ph) | OMe | H | R | |
| 1-1171 | 1' | $CH_2$(2-CNPh) | OMe | H | R | |
| 1-1172 | 1' | $CH_2$(3-CNPh) | OMe | H | R | |
| 1-1173 | 1' | $CH_2$(4-CNPh) | OMe | H | R | |
| 1-1174 | 1' | $CH_2$(2-COOHPh) | OMe | H | R | |
| 1-1175 | 1' | $CH_2$(3-COOHPh) | OMe | H | R | |
| 1-1176 | 1' | $CH_2$(4-COOHPh) | OMe | H | R | |
| 1-1177 | 1' | $CH_2$(2-COOMePh) | OMe | H | R | |
| 1-1178 | 1' | $CH_2$(3-COOMePh) | OMe | H | R | |
| 1-1179 | 1' | $CH_2$(4-COOMePh) | OMe | H | R | |
| 1-1180 | 1' | $CH_2$(2,6-$Cl_2$Ph) | OMe | H | R | |
| 1-1181 | 1' | $CH_2$(2-Cl-6-FPh) | OMe | H | R | |
| 1-1182 | 1' | $CH_2$(2-Br-6-ClPh) | OMe | H | R | |
| 1-1183 | 1' | $CH_2$(1-Naphthyl) | OMe | H | R | |
| 1-1184 | 1' | $CH_2$(2-Naphthyl) | OMe | H | R | |
| 1-1185 | 1' | $CH_2$(2,4-$Cl_2$Ph) | OMe | H | R | |

[Table 35]

| Table 1-Continued | | | | | | |
|---|---|---|---|---|---|---|
| No. | General formula | $R^1$ | $R^2$ | $R^3$ | Stereochemistr y at C-5 position | Remark |
| 1-1186 | 1' | $CH_2$(2-Pyridyl) | OMe | H | R | |
| 1-1187 | 1' | $CH_2$(3-Pyridyl) | OMe | H | R | |
| 1-1188 | 1' | $CH_2$(4-Pyridyl) | OMe | H | R | |
| 1-1189 | 1' | $CH_2$(2-Thienyl) | OMe | H | R | |
| 1-1190 | 1' | $CH_2$(3-Thienyl) | OMe | H | R | |
| 1-1191 | 1' | $CH_2$(3,5-$Me_2$-Isoxazol-4-ly) | OMe | H | R | |
| 1-1192 | 1' | $CH_2CH_2OPh$ | OMe | H | R | |
| 1-1193 | 1' | $CH_2CH_2OCH_2Ph$ | OMe | H | R | |
| 1-1194 | 1' | $CH_2COPh$ | OMe | H | R | |
| 1-1195 | 1' | $CH_2OCH_2CH_2OMe$ | OMe | H | R | |
| 1-1196 | 1' | Me | Me | H | R | |
| 1-1197 | 1' | n-Hex | Me | H | R | |
| 1-1198 | 1' | $CH_2CF_3$ | Me | H | R | |
| 1-1199 | 1' | $CH_2CH=CH_2$ | Me | H | R | |
| 1-1200 | 1' | $CH_2CH=CMe_2$ | Me | H | R | |
| 1-1201 | 1' | $CH_2C\equiv CH$ | Me | H | R | |
| 1-1202 | 1' | $CH_2C\equiv CMe$ | Me | H | R | |
| 1-1203 | 1' | c-Hex | Me | H | R | |
| 1-1204 | 1' | $CH_2$(c-Hex) | Me | H | R | |
| 1-1205 | 1' | $CH_2OMe$ | Me | H | R | |
| 1-1206 | 1' | 2-Pyridyl | Me | H | R | |
| 1-1207 | 1' | 3-Me-2-Pyridyl | Me | H | R | |
| 1-1208 | 1' | $CH_2Ph$ | Me | H | R | |
| 1-1209 | 1' | $CH_2$(2-MePh) | Me | H | R | |
| 1-1210 | 1' | $CH_2$(3-MePh) | Me | H | R | |
| 1-1211 | 1' | $CH_2$(4-MePh) | Me | H | R | |
| 1-1212 | 1' | $CH_2$(2-EtPh) | Me | H | R | |
| 1-1213 | 1' | $CH_2$(3-EtPh) | Me | H | R | |
| 1-1214 | 1' | $CH_2$(4-EtPh) | Me | H | R | |
| 1-1215 | 1' | $CH_2$(2-MeOPh) | Me | H | R | |
| 1-1216 | 1' | $CH_2$(3-MeOPh) | Me | H | R | |
| 1-1217 | 1' | $CH_2$(4-MeOPh) | Me | H | R | |
| 1-1218 | 1' | $CH_2$(2-$CF_3$OPh) | Me | H | R | |
| 1-1219 | 1' | $CH_2$(3-$CF_3$OPh) | Me | H | R | |
| 1-1220 | 1' | $CH_2$(4-$CF_3$OPh) | Me | H | R | |

[Table 36]

| Table 1-Continued | | | | | | |
|---|---|---|---|---|---|---|
| No. | General formula | $R^1$ | $R^2$ | $R^3$ | Stereochemistr y at C-5 position | Remark |
| 1-1221 | 1' | $CH_2$(2-FPh) | Me | H | R | |
| 1-1222 | 1' | $CH_2$(2-FPh) | Me | H | S | |
| 1-1223 | 1' | $CH_2$(3-FPh) | Me | H | R | |
| 1-1224 | 1' | $CH_2$(4-FPh) | Me | H | R | |
| 1-1225 | 1' | $CH_2$(2-ClPh) | Me | H | R | |
| 1-1226 | 1' | $CH_2$(3-ClPh) | Me | H | R | |
| 1-1227 | 1' | $CH_2$(4-ClPh) | Me | H | R | |
| 1-1228 | 1' | $CH_2$(2-BrPh) | Me | H | R | |
| 1-1229 | 1' | $CH_2$(3-BrPh) | Me | H | R | |
| 1-1230 | 1' | $CH_2$(4-BrPh) | Me | H | R | |
| 1-1231 | 1' | $CH_2$(2-IPh) | Me | H | R | |
| 1-1232 | 1' | $CH_2$(3-IPh) | Me | H | R | |
| 1-1233 | 1' | $CH_2$(4-IPh) | Me | H | R | |
| 1-1234 | 1' | $CH_2$(2-$CF_3$Ph) | Me | H | R | |
| 1-1235 | 1' | $CH_2$(3-$CF_3$Ph) | Me | H | R | |
| 1-1236 | 1' | $CH_2$(4-$CF_3$Ph) | Me | H | R | |
| 1-1237 | 1' | $CH_2$(2-CNPh) | Me | H | R | |
| 1-1238 | 1' | $CH_2$(3-CNPh) | Me | H | R | |
| 1-1239 | 1' | $CH_2$(4-CNPh) | Me | H | R | |
| 1-1240 | 1' | $CH_2$(2-COOHPh) | Me | H | R | |
| 1-1241 | 1' | $CH_2$(3-COOHPh) | Me | H | R | |
| 1-1242 | 1' | $CH_2$(4-COOHPh) | Me | H | R | |
| 1-1243 | 1' | $CH_2$(2-COOMePh) | Me | H | R | |
| 1-1244 | 1' | $CH_2$(3-COOMePh) | Me | H | R | |
| 1-1245 | 1' | $CH_2$(4-COOMePh) | Me | H | R | |
| 1-1246 | 1' | $CH_2$(2,6-$Me_2$Ph) | Me | H | R | |
| 1-1247 | 1' | $CH_2$(2,6-$F_2$Ph) | Me | H | R | |
| 1-1248 | 1' | $CH_2$(2,6-$Cl_2$Ph) | Me | H | R | |
| 1-1249 | 1' | $CH_2$(2-Cl-6-FPh) | Me | H | R | |
| 1-1250 | 1' | $CH_2$(2-Br-6-ClPh) | Me | H | R | |
| 1-1251 | 1' | $CH_2$(1-Naphthyl) | Me | H | R | |
| 1-1252 | 1' | $CH_2$(2-Naphthyl) | Me | H | R | |
| 1-1253 | 1' | $CH_2$(2,4-$Cl_2$Ph) | Me | H | R | |
| 1-1254 | 1' | $CH_2$(2-Pyridyl) | Me | H | R | |
| 1-1255 | 1' | $CH_2$(3-Pyridyl) | Me | H | R | |

[Table 37]

| Table 1-Continued | | | | | | |
|---|---|---|---|---|---|---|
| No. | General formula | $R^1$ | $R^2$ | $R^3$ | Stereochemistr y at C-5 position | Remark |
| 1-1256 | 1' | $CH_2$(4-Pyridyl) | Me | H | R | |
| 1-1257 | 1' | $CH_2$(2-Thienyl) | Me | H | R | |
| 1-1258 | 1' | $CH_2$(3-Thienyl) | Me | H | R | |
| 1-1259 | 1' | $CH_2$(3,5-$Me_2$-Isoxazol-4-ly) | Me | H | R | |
| 1-1260 | 1' | $CH_2CH_2OPh$ | Me | H | R | |
| 1-1261 | 1' | $CH_2CH_2OCH_2Ph$ | Me | H | R | |
| 1-1262 | 1' | $CH_2COPh$ | Me | H | R | |
| 1-1263 | 1' | $CH_2OCH_2CH_2OMe$ | Me | H | R | |
| 1-1264 | 1+1' | Me | Cl | H | S:(1) R:(1') | Racemate of 1 and 1' |
| 1-1265 | 1+1' | n-Hex | Cl | H | S:(1) R:(1') | Racemate of 1 and 1' |
| 1-1266 | 1+1' | $CH_2CF_3$ | Cl | H | S:(1) R:(1') | Racemate of 1 and 1' |
| 1-1267 | 1+1' | $CH_2CH=CH_2$ | Cl | H | S:(1) R:(1') | Racemate of 1 and 1' |
| 1-1268 | 1+1' | $CH_2CH=CMe_2$ | Cl | H | S:(1) R:(1') | Racemate of 1 and 1' |
| 1-1269 | 1+1' | $CH_2C{\equiv}CH$ | Cl | H | S:(1) R:(1') | Racemate of 1 and 1' |
| 1-1270 | 1+1' | $CH_2C{\equiv}CMe$ | Cl | H | S:(1) R:(1') | Racemate of 1 and 1' |
| 1-1271 | 1+1' | c-Hex | Cl | H | S:(1) R:(1') | Racemate of 1 and 1' |
| 1-1272 | 1+1' | $CH_2$(c-Hex) | Cl | H | S:(1) R:(1') | Racemate of 1 and 1' |
| 1-1273 | 1+1' | $CH_2OMe$ | Cl | H | S:(1) R:(1') | Racemate of 1 and 1' |
| 1-1274 | 1+1' | 2-Pyridyl | Cl | H | S:(1) R:(1') | Racemate of 1 and 1' |
| 1-1275 | 1+1' | 3-Me-2-Pyridyl | Cl | H | S:(1) R:(1') | Racemate of 1 and 1' |
| 1-1276 | 1+1' | $CH_2Ph$ | Cl | H | S:(1) R:(1') | Racemate of 1 and 1' |
| 1-1277 | 1+1' | $CH_2$(2-MePh) | Cl | H | S:(1) R:(1') | Racemate of 1 and 1' |
| 1-1278 | 1+1' | $CH_2$(3-MePh) | Cl | H | S:(1) R:(1') | Racemate of 1 and 1' |
| 1-1279 | 1+1' | $CH_2$(4-MePh) | Cl | H | S:(1) R:(1') | Racemate of 1 and 1' |
| 1-1280 | 1+1' | $CH_2$(2-EtPh) | Cl | H | S:(1) R:(1') | Racemate of 1 and 1' |
| 1-1281 | 1+1' | $CH_2$(3-EtPh) | Cl | H | S:(1) R:(1') | Racemate of 1 and 1' |
| 1-1282 | 1+1' | $CH_2$(4-EtPh) | Cl | H | S:(1) R:(1') | Racemate of 1 and 1' |
| 1-1283 | 1+1' | $CH_2$(2-MeOPh) | Cl | H | S:(1) R:(1') | Racemate of 1 and 1' |
| 1-1284 | 1+1' | $CH_2$(3-MeOPh) | Cl | H | S:(1) R:(1') | Racemate of 1 and 1' |
| 1-1285 | 1+1' | $CH_2$(4-MeOPh) | Cl | H | S:(1) R:(1') | Racemate of 1 and 1' |
| 1-1286 | 1+1' | $CH_2$(2-$CF_3$OPh) | Cl | H | S:(1) R:(1') | Racemate of 1 and 1' |
| 1-1287 | 1+1' | $CH_2$(3-$CF_3$OPh) | Cl | H | S:(1) R:(1') | Racemate of 1 and 1' |
| 1-1288 | 1+1' | $CH_2$(4-$CF_3$OPh) | Cl | H | S:(1) R:(1') | Racemate of 1 and 1' |
| 1-1289 | 1+1' | $CH_2$(2-FPh) | Cl | H | S:(1) R:(1') | Racemate of 1 and 1' |
| 1-1290 | 1+1' | $CH_2$(3-FPh) | Cl | H | S:(1) R:(1') | Racemate of 1 and 1' |

[Table 38]

| Table 1-Continued | | | | | | |
|---|---|---|---|---|---|---|
| No. | General formula | $R^1$ | $R^2$ | $R^3$ | Stereochemistr y at C-5 position | Remark |
| 1-1291 | 1+1' | $CH_2$(4-FPh) | Cl | H | S:(1) R:(1') | Racemate of 1 and 1' |
| 1-1292 | 1+1' | $CH_2$(2-ClPh) | Cl | H | S:(1) R:(1') | Racemate of 1 and 1' |
| 1-1293 | 1+1' | $CH_2$(3-ClPh) | Cl | H | S:(1) R:(1') | Racemate of 1 and 1' |
| 1-1294 | 1+1' | $CH_2$(4-ClPh) | Cl | H | S:(1) R:(1') | Racemate of 1 and 1' |
| 1-1295 | 1+1' | $CH_2$(2-BrPh) | Cl | H | S:(1) R:(1') | Racemate of 1 and 1' |
| 1-1296 | 1+1' | $CH_2$(3-BrPh) | Cl | H | S:(1) R:(1') | Racemate of 1 and 1' |
| 1-1297 | 1+1' | $CH_2$(4-BrPh) | Cl | H | S:(1) R:(1') | Racemate of 1 and 1' |
| 1-1298 | 1+1' | $CH_2$(2-IPh) | Cl | H | S:(1) R:(1') | Racemate of 1 and 1' |
| 1-1299 | 1+1' | $CH_2$(3-IPh) | Cl | H | S:(1) R:(1') | Racemate of 1 and 1' |
| 1-1300 | 1+1' | $CH_2$(4-IPh) | Cl | H | S:(1) R:(1') | Racemate of 1 and 1' |
| 1-1301 | 1+1' | $CH_2$(2-$CF_3$Ph) | Cl | H | S:(1) R:(1') | Racemate of 1 and 1' |
| 1-1302 | 1+1' | $CH_2$(3-$CF_3$Ph) | Cl | H | S:(1) R:(1') | Racemate of 1 and 1' |
| 1-1303 | 1+1' | $CH_2$(4-$CF_3$Ph) | Cl | H | S:(1) R:(1') | Racemate of 1 and 1' |
| 1-1304 | 1+1' | $CH_2$(2-CNPh) | Cl | H | S:(1) R:(1') | Racemate of 1 and 1' |
| 1-1305 | 1+1' | $CH_2$(3-CNPh) | Cl | H | S:(1) R:(1') | Racemate of 1 and 1' |
| 1-1306 | 1+1' | $CH_2$(4-CNPh) | Cl | H | S:(1) R:(1') | Racemate of 1 and 1' |
| 1-1307 | 1+1' | $CH_2$(2-COOHPh) | Cl | H | S:(1) R:(1') | Racemate of 1 and 1' |
| 1-1308 | 1+1' | $CH_2$(3-COOHPh) | Cl | H | S:(1) R:(1') | Racemate of 1 and 1' |
| 1-1309 | 1+1' | $CH_2$(4-COOHPh) | Cl | H | S:(1) R:(1') | Racemate of 1 and 1' |
| 1-1310 | 1+1' | $CH_2$(2-COOMePh) | Cl | H | S:(1) R:(1') | Racemate of 1 and 1' |
| 1-1311 | 1+1' | $CH_2$(3-COOMePh) | Cl | H | S:(1) R:(1') | Racemate of 1 and 1' |
| 1-1312 | 1+1' | $CH_2$(4-COOMePh) | Cl | H | S:(1) R:(1') | Racemate of 1 and 1' |
| 1-1313 | 1+1' | $CH_2$(2,6-$F_2$Ph) | Cl | H | S:(1) R:(1') | Racemate of 1 and 1' |
| 1-1314 | 1+1' | $CH_2$(2,6-$Cl_2$Ph) | Cl | H | S:(1) R:(1') | Racemate of 1 and 1' |
| 1-1315 | 1+1' | $CH_2$(2-Cl-6-FPh) | Cl | H | S:(1) R:(1') | Racemate of 1 and 1' |
| 1-1316 | 1+1' | $CH_2$(2-Br-6-ClPh) | Cl | H | S:(1) R:(1') | Racemate of 1 and 1' |
| 1-1317 | 1+1' | $CH_2$(1-Naphthyl) | Cl | H | S:(1) R:(1') | Racemate of 1 and 1' |
| 1-1318 | 1+1' | $CH_2$(2-Naphthyl) | Cl | H | S:(1) R:(1') | Racemate of 1 and 1' |
| 1-1319 | 1+1' | $CH_2$(2,4-$Cl_2$Ph) | Cl | H | S:(1) R:(1') | Racemate of 1 and 1' |
| 1-1320 | 1+1' | $CH_2$(2-Pyridyl) | Cl | H | S:(1) R:(1') | Racemate of 1 and 1' |
| 1-1321 | 1+1' | $CH_2$(3-Pyridyl) | Cl | H | S:(1) R:(1') | Racemate of 1 and 1' |
| 1-1322 | 1+1' | $CH_2$(4-Pyridyl) | Cl | H | S:(1) R:(1') | Racemate of 1 and 1' |
| 1-1323 | 1+1' | $CH_2$(2-Thienyl) | Cl | H | S:(1) R:(1') | Racemate of 1 and 1' |
| 1-1324 | 1+1' | $CH_2$(3-Thienyl) | Cl | H | S:(1) R:(1') | Racemate of 1 and 1' |
| 1-1325 | 1+1' | $CH_2$(3,5-$Me_2$-Isoxazol-4-ly) | Cl | H | S:(1) R:(1') | Racemate of 1 and 1' |

[Table 39]

| Table 1-Continued | | | | | | |
|---|---|---|---|---|---|---|
| No. | General formula | $R^1$ | $R^2$ | $R^3$ | Stereochemistr y at C-5 position | Remark |
| 1-1326 | 1+1' | $CH_2CH_2OPh$ | Cl | H | S:(1) R:(1') | Racemate of 1 and 1' |
| 1-1327 | 1+1' | $CH_2CH_2OCH_2Ph$ | Cl | H | S:(1) R:(1') | Racemate of 1 and 1' |
| 1-1328 | 1+1' | $CH_2COPh$ | Cl | H | S:(1) R:(1') | Racemate of 1 and 1' |
| 1-1329 | 1+1' | $CH_2OCH_2CH_2OMe$ | Cl | H | S:(1) R:(1') | Racemate of 1 and 1' |
| 1-1330 | 1+1' | Me | Br | H | S:(1) R:(1') | Racemate of 1 and 1' |
| 1-1331 | 1+1' | n-Hex | Br | H | S:(1) R:(1') | Racemate of 1 and 1' |
| 1-1332 | 1+1' | $CH_2CF_3$ | Br | H | S:(1) R:(1') | Racemate of 1 and 1' |
| 1-1333 | 1+1' | $CH_2CH=CH_2$ | Br | H | S:(1) R:(1') | Racemate of 1 and 1' |
| 1-1334 | 1+1' | $CH_2CH=CMe_2$ | Br | H | S:(1) R:(1') | Racemate of 1 and 1' |
| 1-1335 | 1+1' | $CH_2C\equiv CH$ | Br | H | S:(1) R:(1') | Racemate of 1 and 1' |
| 1-1336 | 1+1' | $CH_2C\equiv CMe$ | Br | H | S:(1) R:(1') | Racemate of 1 and 1' |
| 1-1337 | 1+1' | c-Hex | Br | H | S:(1) R:(1') | Racemate of 1 and 1' |
| 1-1338 | 1+1' | $CH_2$(c-Hex) | Br | H | S:(1) R:(1') | Racemate of 1 and 1' |
| 1-1339 | 1+1' | $CH_2OMe$ | Br | H | S:(1) R:(1') | Racemate of 1 and 1' |
| 1-1340 | 1+1' | 2-Pyridyl | Br | H | S:(1) R:(1') | Racemate of 1 and 1' |
| 1-1341 | 1+1' | 3-Me-2-Pyridyl | Br | H | S:(1) R:(1') | Racemate of 1 and 1' |
| 1-1342 | 1+1' | $CH_2Ph$ | Br | H | S:(1) R:(1') | Racemate of 1 and 1' |
| 1-1343 | 1+1' | $CH_2$(2-MePh) | Br | H | S:(1) R:(1') | Racemate of 1 and 1' |
| 1-1344 | 1+1' | $CH_2$(3-MePh) | Br | H | S:(1) R:(1') | Racemate of 1 and 1' |
| 1-1345 | 1+1' | $CH_2$(4-MePh) | Br | H | S:(1) R:(1') | Racemate of 1 and 1' |
| 1-1346 | 1+1' | $CH_2$(2-EtPh) | Br | H | S:(1) R:(1') | Racemate of 1 and 1' |
| 1-1347 | 1+1' | $CH_2$(3-EtPh) | Br | H | S:(1) R:(1') | Racemate of 1 and 1' |
| 1-1348 | 1+1' | $CH_2$(4-EtPh) | Br | H | S:(1) R:(1') | Racemate of 1 and 1' |
| 1-1349 | 1+1' | $CH_2$(2-MeOPh) | Br | H | S:(1) R:(1') | Racemate of 1 and 1' |
| 1-1350 | 1+1' | $CH_2$(3-MeOPh) | Br | H | S:(1) R:(1') | Racemate of 1 and 1' |
| 1-1351 | 1+1' | $CH_2$(4-MeOPh) | Br | H | S:(1) R:(1') | Racemate of 1 and 1' |
| 1-1352 | 1+1' | $CH_2$(2-$CF_3$OPh) | Br | H | S:(1) R:(1') | Racemate of 1 and 1' |
| 1-1353 | 1+1' | $CH_2$(3-$CF_3$OPh) | Br | H | S:(1) R:(1') | Racemate of 1 and 1' |
| 1-1354 | 1+1' | $CH_2$(4-$CF_3$OPh) | Br | H | S:(1) R:(1') | Racemate of 1 and 1' |
| 1-1355 | 1+1' | $CH_2$(2-FPh) | Br | H | S:(1) R:(1') | Racemate of 1 and 1' |
| 1-1356 | 1+1' | $CH_2$(3-FPh) | Br | H | S:(1) R:(1') | Racemate of 1 and 1' |
| 1-1357 | 1+1' | $CH_2$(4-FPh) | Br | H | S:(1) R:(1') | Racemate of 1 and 1' |
| 1-1358 | 1+1' | $CH_2$(2-ClPh) | Br | H | S:(1) R:(1') | Racemate of 1 and 1' |
| 1-1359 | 1+1' | $CH_2$(3-ClPh) | Br | H | S:(1) R:(1') | Racemate of 1 and 1' |
| 1-1360 | 1+1' | $CH_2$(4-ClPh) | Br | H | S:(1) R:(1') | Racemate of 1 and 1' |

[Table 40]

| Table 1-Continued | | | | | | |
|---|---|---|---|---|---|---|
| No. | General formula | $R^1$ | $R^2$ | $R^3$ | Stereochemistr y at C-5 position | Remark |
| 1-1361 | 1+1’ | $CH_2$(2-BrPh) | Br | H | S:(1) R:(1’) | Racemate of 1 and 1’ |
| 1-1362 | 1+1’ | $CH_2$(3-BrPh) | Br | H | S:(1) R:(1’) | Racemate of 1 and 1’ |
| 1-1363 | 1+1’ | $CH_2$(4-BrPh) | Br | H | S:(1) R:(1’) | Racemate of 1 and 1’ |
| 1-1364 | 1+1’ | $CH_2$(2-IPh) | Br | H | S:(1) R:(1’) | Racemate of 1 and 1’ |
| 1-1365 | 1+1’ | $CH_2$(3-IPh) | Br | H | S:(1) R:(1’) | Racemate of 1 and 1’ |
| 1-1366 | 1+1’ | $CH_2$(4-IPh) | Br | H | S:(1) R:(1’) | Racemate of 1 and 1’ |
| 1-1367 | 1+1’ | $CH_2$(2-$CF_3$Ph) | Br | H | S:(1) R:(1’) | Racemate of 1 and 1’ |
| 1-1368 | 1+1’ | $CH_2$(3-$CF_3$Ph) | Br | H | S:(1) R:(1’) | Racemate of 1 and 1’ |
| 1-1369 | 1+1’ | $CH_2$(4-$CF_3$Ph) | Br | H | S:(1) R:(1’) | Racemate of 1 and 1’ |
| 1-1370 | 1+1’ | $CH_2$(2-CNPh) | Br | H | S:(1) R:(1’) | Racemate of 1 and 1’ |
| 1-1371 | 1+1’ | $CH_2$(3-CNPh) | Br | H | S:(1) R:(1’) | Racemate of 1 and 1’ |
| 1-1372 | 1+1’ | $CH_2$(4-CNPh) | Br | H | S:(1) R:(1’) | Racemate of 1 and 1’ |
| 1-1373 | 1+1’ | $CH_2$(2-COOHPh) | Br | H | S:(1) R:(1’) | Racemate of 1 and 1’ |
| 1-1374 | 1+1’ | $CH_2$(3-COOHPh) | Br | H | S:(1) R:(1’) | Racemate of 1 and 1’ |
| 1-1375 | 1+1’ | $CH_2$(4-COOHPh) | Br | H | S:(1) R:(1’) | Racemate of 1 and 1’ |
| 1-1376 | 1+1’ | $CH_2$(2-COOMePh) | Br | H | S:(1) R:(1’) | Racemate of 1 and 1’ |
| 1-1377 | 1+1’ | $CH_2$(3-COOMePh) | Br | H | S:(1) R:(1’) | Racemate of 1 and 1’ |
| 1-1378 | 1+1’ | $CH_2$(4-COOMePh) | Br | H | S:(1) R:(1’) | Racemate of 1 and 1’ |
| 1-1379 | 1+1’ | $CH_2$(2,6-$F_2$Ph) | Br | H | S:(1) R:(1’) | Racemate of 1 and 1’ |
| 1-1380 | 1+1’ | $CH_2$(2,6-$Cl_2$Ph) | Br | H | S:(1) R:(1’) | Racemate of 1 and 1’ |
| 1-1381 | 1+1’ | $CH_2$(2-Cl-6-FPh) | Br | H | S:(1) R:(1’) | Racemate of 1 and 1’ |
| 1-1382 | 1+1’ | $CH_2$(2-Br-6-ClPh) | Br | H | S:(1) R:(1’) | Racemate of 1 and 1’ |
| 1-1383 | 1+1’ | $CH_2$(1-Naphthyl) | Br | H | S:(1) R:(1’) | Racemate of 1 and 1’ |
| 1-1384 | 1+1’ | $CH_2$(2-Naphthyl) | Br | H | S:(1) R:(1’) | Racemate of 1 and 1’ |
| 1-1385 | 1+1’ | $CH_2$(2,4-$Cl_2$Ph) | Br | H | S:(1) R:(1’) | Racemate of 1 and 1’ |
| 1-1386 | 1+1’ | $CH_2$(2-Pyridyl) | Br | H | S:(1) R:(1’) | Racemate of 1 and 1’ |
| 1-1387 | 1+1’ | $CH_2$(3-Pyridyl) | Br | H | S:(1) R:(1’) | Racemate of 1 and 1’ |
| 1-1388 | 1+1’ | $CH_2$(4-Pyridyl) | Br | H | S:(1) R:(1’) | Racemate of 1 and 1’ |
| 1-1389 | 1+1’ | $CH_2$(2-Thienyl) | Br | H | S:(1) R:(1’) | Racemate of 1 and 1’ |
| 1-1390 | 1+1’ | $CH_2$(3-Thienyl) | Br | H | S:(1) R:(1’) | Racemate of 1 and 1’ |
| 1-1391 | 1+1’ | $CH_2$(3,5-$Me_2$-Isoxazol-4-ly) | Br | H | S:(1) R:(1’) | Racemate of 1 and 1’ |
| 1-1392 | 1+1’ | $CH_2CH_2OPh$ | Br | H | S:(1) R:(1’) | Racemate of 1 and 1’ |
| 1-1393 | 1+1’ | $CH_2CH_2OCH_2Ph$ | Br | H | S:(1) R:(1’) | Racemate of 1 and 1’ |
| 1-1394 | 1+1’ | $CH_2COPh$ | Br | H | S:(1) R:(1’) | Racemate of 1 and 1’ |
| 1-1395 | 1+1’ | $CH_2OCH_2CH_2OMe$ | Br | H | S:(1) R:(1’) | Racemate of 1 and 1’ |

[Table 41]

| Table 1-Continued | | | | | | |
|---|---|---|---|---|---|---|
| No. | General formula | $R^1$ | $R^2$ | $R^3$ | Stereochemistry at C-5 position | Remark |
| 1-1396 | 1+1’ | Me | I | H | S:(1) R:(1’) | Racemate of 1 and 1’ |
| 1-1397 | 1+1’ | n-Hex | I | H | S:(1) R:(1’) | Racemate of 1 and 1’ |
| 1-1398 | 1+1’ | $CH_2CF_3$ | I | H | S:(1) R:(1’) | Racemate of 1 and 1’ |
| 1-1399 | 1+1’ | $CH_2CH=CH_2$ | I | H | S:(1) R:(1’) | Racemate of 1 and 1’ |
| 1-1400 | 1+1’ | $CH_2CH=CMe_2$ | I | H | S:(1) R:(1’) | Racemate of 1 and 1’ |
| 1-1401 | 1+1’ | $CH_2C≡CH$ | I | H | S:(1) R:(1’) | Racemate of 1 and 1’ |
| 1-1402 | 1+1’ | $CH_2C≡CMe$ | I | H | S:(1) R:(1’) | Racemate of 1 and 1’ |
| 1-1403 | 1+1’ | c-Hex | I | H | S:(1) R:(1’) | Racemate of 1 and 1’ |
| 1-1404 | 1+1’ | $CH_2$(c-Hex) | I | H | S:(1) R:(1’) | Racemate of 1 and 1’ |
| 1-1405 | 1+1’ | $CH_2OMe$ | I | H | S:(1) R:(1’) | Racemate of 1 and 1’ |
| 1-1406 | 1+1’ | 2-Pyridyl | I | H | S:(1) R:(1’) | Racemate of 1 and 1’ |
| 1-1407 | 1+1’ | 3-Me-2-Pyridyl | I | H | S:(1) R:(1’) | Racemate of 1 and 1’ |
| 1-1408 | 1+1’ | $CH_2Ph$ | I | H | S:(1) R:(1’) | Racemate of 1 and 1’ |
| 1-1409 | 1+1’ | $CH_2$(2-MePh) | I | H | S:(1) R:(1’) | Racemate of 1 and 1’ |
| 1-1410 | 1+1’ | $CH_2$(3-MePh) | I | H | S:(1) R:(1’) | Racemate of 1 and 1’ |
| 1-1411 | 1+1’ | $CH_2$(4-MePh) | I | H | S:(1) R:(1’) | Racemate of 1 and 1’ |
| 1-1412 | 1+1’ | $CH_2$(2-EtPh) | I | H | S:(1) R:(1’) | Racemate of 1 and 1’ |
| 1-1413 | 1+1’ | $CH_2$(3-EtPh) | I | H | S:(1) R:(1’) | Racemate of 1 and 1’ |
| 1-1414 | 1+1’ | $CH_2$(4-EtPh) | I | H | S:(1) R:(1’) | Racemate of 1 and 1’ |
| 1-1415 | 1+1’ | $CH_2$(2-MeOPh) | I | H | S:(1) R:(1’) | Racemate of 1 and 1’ |
| 1-1416 | 1+1’ | $CH_2$(3-MeOPh) | I | H | S:(1) R:(1’) | Racemate of 1 and 1’ |
| 1-1417 | 1+1’ | $CH_2$(4-MeOPh) | I | H | S:(1) R:(1’) | Racemate of 1 and 1’ |
| 1-1418 | 1+1’ | $CH_2$(2-$CF_3$OPh) | I | H | S:(1) R:(1’) | Racemate of 1 and 1’ |
| 1-1419 | 1+1’ | $CH_2$(3-$CF_3$OPh) | I | H | S:(1) R:(1’) | Racemate of 1 and 1’ |
| 1-1420 | 1+1’ | $CH_2$(4-$CF_3$OPh) | I | H | S:(1) R:(1’) | Racemate of 1 and 1’ |
| 1-1421 | 1+1’ | $CH_2$(2-FPh) | I | H | S:(1) R:(1’) | Racemate of 1 and 1’ |
| 1-1422 | 1+1’ | $CH_2$(3-FPh) | I | H | S:(1) R:(1’) | Racemate of 1 and 1’ |
| 1-1423 | 1+1’ | $CH_2$(4-FPh) | I | H | S:(1) R:(1’) | Racemate of 1 and 1’ |
| 1-1424 | 1+1’ | $CH_2$(2-ClPh) | I | H | S:(1) R:(1’) | Racemate of 1 and 1’ |
| 1-1425 | 1+1’ | $CH_2$(3-ClPh) | I | H | S:(1) R:(1’) | Racemate of 1 and 1’ |
| 1-1426 | 1+1’ | $CH_2$(4-ClPh) | I | H | S:(1) R:(1’) | Racemate of 1 and 1’ |
| 1-1427 | 1+1’ | $CH_2$(2-BrPh) | I | H | S:(1) R:(1’) | Racemate of 1 and 1’ |
| 1-1428 | 1+1’ | $CH_2$(3-BrPh) | I | H | S:(1) R:(1’) | Racemate of 1 and 1’ |
| 1-1429 | 1+1’ | $CH_2$(4-BrPh) | I | H | S:(1) R:(1’) | Racemate of 1 and 1’ |
| 1-1430 | 1+1’ | $CH_2$(2-IPh) | I | H | S:(1) R:(1’) | Racemate of 1 and 1’ |

[Table 42]

| Table 1-Continued | | | | | | |
|---|---|---|---|---|---|---|
| No. | General formula | $R^1$ | $R^2$ | $R^3$ | Stereochemistry at C-5 position | Remark |
| 1-1431 | 1+1' | $CH_2$(3-IPh) | I | H | S:(1) R:(1') | Racemate of 1 and 1' |
| 1-1432 | 1+1' | $CH_2$(4-IPh) | I | H | S:(1) R:(1') | Racemate of 1 and 1' |
| 1-1433 | 1+1' | $CH_2$(2-$CF_3$Ph) | I | H | S:(1) R:(1') | Racemate of 1 and 1' |
| 1-1434 | 1+1' | $CH_2$(3-$CF_3$Ph) | I | H | S:(1) R:(1') | Racemate of 1 and 1' |
| 1-1435 | 1+1' | $CH_2$(4-$CF_3$Ph) | I | H | S:(1) R:(1') | Racemate of 1 and 1' |
| 1-1436 | 1+1' | $CH_2$(2-CNPh) | I | H | S:(1) R:(1') | Racemate of 1 and 1' |
| 1-1437 | 1+1' | $CH_2$(3-CNPh) | I | H | S:(1) R:(1') | Racemate of 1 and 1' |
| 1-1438 | 1+1' | $CH_2$(4-CNPh) | I | H | S:(1) R:(1') | Racemate of 1 and 1' |
| 1-1439 | 1+1' | $CH_2$(2-COOHPh) | I | H | S:(1) R:(1') | Racemate of 1 and 1' |
| 1-1440 | 1+1' | $CH_2$(3-COOHPh) | I | H | S:(1) R:(1') | Racemate of 1 and 1' |
| 1-1441 | 1+1' | $CH_2$(4-COOHPh) | I | H | S:(1) R:(1') | Racemate of 1 and 1' |
| 1-1442 | 1+1' | $CH_2$(2-COOMePh) | I | H | S:(1) R:(1') | Racemate of 1 and 1' |
| 1-1443 | 1+1' | $CH_2$(3-COOMePh) | I | H | S:(1) R:(1') | Racemate of 1 and 1' |
| 1-1444 | 1+1' | $CH_2$(4-COOMePh) | I | H | S:(1) R:(1') | Racemate of 1 and 1' |
| 1-1445 | 1+1' | $CH_2$(2,6-$Me_2$Ph) | I | H | S:(1) R:(1') | Racemate of 1 and 1' |
| 1-1446 | 1+1' | $CH_2$(2,6-$F_2$Ph) | I | H | S:(1) R:(1') | Racemate of 1 and 1' |
| 1-1447 | 1+1' | $CH_2$(2,6-$Cl_2$Ph) | I | H | S:(1) R:(1') | Racemate of 1 and 1' |
| 1-1448 | 1+1' | $CH_2$(2-Cl-6-FPh) | I | H | S:(1) R:(1') | Racemate of 1 and 1' |
| 1-1449 | 1+1' | $CH_2$(2-Br-6-ClPh) | I | H | S:(1) R:(1') | Racemate of 1 and 1' |
| 1-1450 | 1+1' | $CH_2$(1-Naphthyl) | I | H | S:(1) R:(1') | Racemate of 1 and 1' |
| 1-1451 | 1+1' | $CH_2$(2-Naphthyl) | I | H | S:(1) R:(1') | Racemate of 1 and 1' |
| 1-1452 | 1+1' | $CH_2$(2,4-$Cl_2$Ph) | I | H | S:(1) R:(1') | Racemate of 1 and 1' |
| 1-1453 | 1+1' | $CH_2$(2-Pyridyl) | I | H | S:(1) R:(1') | Racemate of 1 and 1' |
| 1-1454 | 1+1' | $CH_2$(3-Pyridyl) | I | H | S:(1) R:(1') | Racemate of 1 and 1' |
| 1-1455 | 1+1' | $CH_2$(4-Pyridyl) | I | H | S:(1) R:(1') | Racemate of 1 and 1' |
| 1-1456 | 1+1' | $CH_2$(2-Thienyl) | I | H | S:(1) R:(1') | Racemate of 1 and 1' |
| 1-1457 | 1+1' | $CH_2$(3-Thienyl) | I | H | S:(1) R:(1') | Racemate of 1 and 1' |
| 1-1458 | 1+1' | $CH_2$(3,5-$Me_2$-Isoxazol-4-ly) | I | H | S:(1) R:(1') | Racemate of 1 and 1' |
| 1-1459 | 1+1' | $CH_2CH_2OPh$ | I | H | S:(1) R:(1') | Racemate of 1 and 1' |
| 1-1460 | 1+1' | $CH_2CH_2OCH_2Ph$ | I | H | S:(1) R:(1') | Racemate of 1 and 1' |
| 1-1461 | 1+1' | $CH_2COPh$ | I | H | S:(1) R:(1') | Racemate of 1 and 1' |
| 1-1462 | 1+1' | $CH_2OCH_2CH_2OMe$ | I | H | S:(1) R:(1') | Racemate of 1 and 1' |
| 1-1463 | 1+1' | Me | OMe | H | S:(1) R:(1') | Racemate of 1 and 1' |
| 1-1464 | 1+1' | n-Hex | OMe | H | S:(1) R:(1') | Racemate of 1 and 1' |
| 1-1465 | 1+1' | $CH_2CF_3$ | OMe | H | S:(1) R:(1') | Racemate of 1 and 1' |

[Table 43]

| Table 1-Continued | | | | | | |
|---|---|---|---|---|---|---|
| No. | General formula | $R^1$ | $R^2$ | $R^3$ | Stereochemistry at C-5 position | Remark |
| 1-1466 | 1+1' | $CH_2CH=CH_2$ | OMe | H | S:(1) R:(1') | Racemate of 1 and 1' |
| 1-1467 | 1+1' | $CH_2CH=CMe_2$ | OMe | H | S:(1) R:(1') | Racemate of 1 and 1' |
| 1-1468 | 1+1' | $CH_2C≡CH$ | OMe | H | S:(1) R:(1') | Racemate of 1 and 1' |
| 1-1469 | 1+1' | $CH_2C≡CMe$ | OMe | H | S:(1) R:(1') | Racemate of 1 and 1' |
| 1-1470 | 1+1' | c-Hex | OMe | H | S:(1) R:(1') | Racemate of 1 and 1' |
| 1-1471 | 1+1' | $CH_2$(c-Hex) | OMe | H | S:(1) R:(1') | Racemate of 1 and 1' |
| 1-1472 | 1+1' | $CH_2OMe$ | OMe | H | S:(1) R:(1') | Racemate of 1 and 1' |
| 1-1473 | 1+1' | 2-Pyridyl | OMe | H | S:(1) R:(1') | Racemate of 1 and 1' |
| 1-1474 | 1+1' | 3-Me-2-Pyridyl | OMe | H | S:(1) R:(1') | Racemate of 1 and 1' |
| 1-1475 | 1+1' | $CH_2Ph$ | OMe | H | S:(1) R:(1') | Racemate of 1 and 1' |
| 1-1476 | 1+1' | $CH_2$(2-MePh) | OMe | H | S:(1) R:(1') | Racemate of 1 and 1' |
| 1-1477 | 1+1' | $CH_2$(3-MePh) | OMe | H | S:(1) R:(1') | Racemate of 1 and 1' |
| 1-1478 | 1+1' | $CH_2$(4-MePh) | OMe | H | S:(1) R:(1') | Racemate of 1 and 1' |
| 1-1479 | 1+1' | $CH_2$(2-EtPh) | OMe | H | S:(1) R:(1') | Racemate of 1 and 1' |
| 1-1480 | 1+1' | $CH_2$(3-EtPh) | OMe | H | S:(1) R:(1') | Racemate of 1 and 1' |
| 1-1481 | 1+1' | $CH_2$(4-EtPh) | OMe | H | S:(1) R:(1') | Racemate of 1 and 1' |
| 1-1482 | 1+1' | $CH_2$(2-MeOPh) | OMe | H | S:(1) R:(1') | Racemate of 1 and 1' |
| 1-1483 | 1+1' | $CH_2$(3-MeOPh) | OMe | H | S:(1) R:(1') | Racemate of 1 and 1' |
| 1-1484 | 1+1' | $CH_2$(4-MeOPh) | OMe | H | S:(1) R:(1') | Racemate of 1 and 1' |
| 1-1485 | 1+1' | $CH_2$(2-$CF_3$OPh) | OMe | H | S:(1) R:(1') | Racemate of 1 and 1' |
| 1-1486 | 1+1' | $CH_2$(3-$CF_3$OPh) | OMe | H | S:(1) R:(1') | Racemate of 1 and 1' |
| 1-1487 | 1+1' | $CH_2$(4-$CF_3$OPh) | OMe | H | S:(1) R:(1') | Racemate of 1 and 1' |
| 1-1488 | 1+1' | $CH_2$(2-FPh) | OMe | H | S:(1) R:(1') | Racemate of 1 and 1' |
| 1-1489 | 1+1' | $CH_2$(3-FPh) | OMe | H | S:(1) R:(1') | Racemate of 1 and 1' |
| 1-1490 | 1+1' | $CH_2$(4-FPh) | OMe | H | S:(1) R:(1') | Racemate of 1 and 1' |
| 1-1491 | 1+1' | $CH_2$(2-ClPh) | OMe | H | S:(1) R:(1') | Racemate of 1 and 1' |
| 1-1492 | 1+1' | $CH_2$(3-ClPh) | OMe | H | S:(1) R:(1') | Racemate of 1 and 1' |
| 1-1493 | 1+1' | $CH_2$(4-ClPh) | OMe | H | S:(1) R:(1') | Racemate of 1 and 1' |
| 1-1494 | 1+1' | $CH_2$(2-BrPh) | OMe | H | S:(1) R:(1') | Racemate of 1 and 1' |
| 1-1495 | 1+1' | $CH_2$(3-BrPh) | OMe | H | S:(1) R:(1') | Racemate of 1 and 1' |
| 1-1496 | 1+1' | $CH_2$(4-BrPh) | OMe | H | S:(1) R:(1') | Racemate of 1 and 1' |
| 1-1497 | 1+1' | $CH_2$(2-IPh) | OMe | H | S:(1) R:(1') | Racemate of 1 and 1' |
| 1-1498 | 1+1' | $CH_2$(3-IPh) | OMe | H | S:(1) R:(1') | Racemate of 1 and 1' |
| 1-1499 | 1+1' | $CH_2$(4-IPh) | OMe | H | S:(1) R:(1') | Racemate of 1 and 1' |
| 1-1500 | 1+1' | $CH_2$(2-$CF_3$Ph) | OMe | H | S:(1) R:(1') | Racemate of 1 and 1' |

[Table 44]

| Table 1-Continued | | | | | | |
|---|---|---|---|---|---|---|
| No. | General formula | $R^1$ | $R^2$ | $R^3$ | Stereochemistry at C-5 position | Remark |
| 1-1501 | 1+1’ | $CH_2(3-CF_3Ph)$ | OMe | H | S:(1) R:(1’) | Racemate of 1 and 1’ |
| 1-1502 | 1+1’ | $CH_2(4-CF_3Ph)$ | OMe | H | S:(1) R:(1’) | Racemate of 1 and 1’ |
| 1-1503 | 1+1’ | $CH_2$(2-CNPh) | OMe | H | S:(1) R:(1’) | Racemate of 1 and 1’ |
| 1-1504 | 1+1’ | $CH_2$(3-CNPh) | OMe | H | S:(1) R:(1’) | Racemate of 1 and 1’ |
| 1-1505 | 1+1’ | $CH_2$(4-CNPh) | OMe | H | S:(1) R:(1’) | Racemate of 1 and 1’ |
| 1-1506 | 1+1’ | $CH_2$(2-COOHPh) | OMe | H | S:(1) R:(1’) | Racemate of 1 and 1’ |
| 1-1507 | 1+1’ | $CH_2$(3-COOHPh) | OMe | H | S:(1) R:(1’) | Racemate of 1 and 1’ |
| 1-1508 | 1+1’ | $CH_2$(4-COOHPh) | OMe | H | S:(1) R:(1’) | Racemate of 1 and 1’ |
| 1-1509 | 1+1’ | $CH_2$(2-COOMePh) | OMe | H | S:(1) R:(1’) | Racemate of 1 and 1’ |
| 1-1510 | 1+1’ | $CH_2$(3-COOMePh) | OMe | H | S:(1) R:(1’) | Racemate of 1 and 1’ |
| 1-1511 | 1+1’ | $CH_2$(4-COOMePh) | OMe | H | S:(1) R:(1’) | Racemate of 1 and 1’ |
| 1-1512 | 1+1’ | $CH_2(2,6-Me_2Ph)$ | OMe | H | S:(1) R:(1’) | Racemate of 1 and 1’ |
| 1-1513 | 1+1’ | $CH_2(2,6-F_2Ph)$ | OMe | H | S:(1) R:(1’) | Racemate of 1 and 1’ |
| 1-1514 | 1+1’ | $CH_2(2,6-Cl_2Ph)$ | OMe | H | S:(1) R:(1’) | Racemate of 1 and 1’ |
| 1-1515 | 1+1’ | $CH_2$(2-Cl-6-FPh) | OMe | H | S:(1) R:(1’) | Racemate of 1 and 1’ |
| 1-1516 | 1+1’ | $CH_2$(2-Br-6-ClPh) | OMe | H | S:(1) R:(1’) | Racemate of 1 and 1’ |
| 1-1517 | 1+1’ | $CH_2$(1-Naphthyl) | OMe | H | S:(1) R:(1’) | Racemate of 1 and 1’ |
| 1-1518 | 1+1’ | $CH_2$(2-Naphthyl) | OMe | H | S:(1) R:(1’) | Racemate of 1 and 1’ |
| 1-1519 | 1+1’ | $CH_2(2,4-Cl_2Ph)$ | OMe | H | S:(1) R:(1’) | Racemate of 1 and 1’ |
| 1-1520 | 1+1’ | $CH_2$(2-Pyridyl) | OMe | H | S:(1) R:(1’) | Racemate of 1 and 1’ |
| 1-1521 | 1+1’ | $CH_2$(3-Pyridyl) | OMe | H | S:(1) R:(1’) | Racemate of 1 and 1’ |
| 1-1522 | 1+1’ | $CH_2$(4-Pyridyl) | OMe | H | S:(1) R:(1’) | Racemate of 1 and 1’ |
| 1-1523 | 1+1’ | $CH_2$(2-Thienyl) | OMe | H | S:(1) R:(1’) | Racemate of 1 and 1’ |
| 1-1524 | 1+1’ | $CH_2$(3-Thienyl) | OMe | H | S:(1) R:(1’) | Racemate of 1 and 1’ |
| 1-1525 | 1+1’ | $CH_2(3,5-Me_2$-Isoxazol-4-ly) | OMe | H | S:(1) R:(1’) | Racemate of 1 and 1’ |
| 1-1526 | 1+1’ | $CH_2CH_2OPh$ | OMe | H | S:(1) R:(1’) | Racemate of 1 and 1’ |
| 1-1527 | 1+1’ | $CH_2CH_2OCH_2Ph$ | OMe | H | S:(1) R:(1’) | Racemate of 1 and 1’ |
| 1-1528 | 1+1’ | $CH_2COPh$ | OMe | H | S:(1) R:(1’) | Racemate of 1 and 1’ |
| 1-1529 | 1+1’ | $CH_2OCH_2CH_2OMe$ | OMe | H | S:(1) R:(1’) | Racemate of 1 and 1’ |
| 1-1530 | 1+1’ | Me | Me | H | S:(1) R:(1’) | Racemate of 1 and 1’ |
| 1-1531 | 1+1’ | n-Hex | Me | H | S:(1) R:(1’) | Racemate of 1 and 1’ |
| 1-1532 | 1+1’ | $CH_2CF_3$ | Me | H | S:(1) R:(1’) | Racemate of 1 and 1’ |
| 1-1533 | 1+1’ | $CH_2CH=CH_2$ | Me | H | S:(1) R:(1’) | Racemate of 1 and 1’ |
| 1-1534 | 1+1’ | $CH_2CH=CMe_2$ | Me | H | S:(1) R:(1’) | Racemate of 1 and 1’ |
| 1-1535 | 1+1’ | $CH_2C\equiv CH$ | Me | H | S:(1) R:(1’) | Racemate of 1 and 1’ |

[Table 45]

| Table 1-Continued | | | | | | |
|---|---|---|---|---|---|---|
| No. | General formula | $R^1$ | $R^2$ | $R^3$ | Stereochemistry at C-5 position | Remark |
| 1-1536 | 1+1’ | $CH_2C\equiv CMe$ | Me | H | S:(1) R:(1’) | Racemate of 1 and 1’ |
| 1-1537 | 1+1’ | c-Hex | Me | H | S:(1) R:(1’) | Racemate of 1 and 1’ |
| 1-1538 | 1+1’ | $CH_2$(c-Hex) | Me | H | S:(1) R:(1’) | Racemate of 1 and 1’ |
| 1-1539 | 1+1’ | $CH_2OMe$ | Me | H | S:(1) R:(1’) | Racemate of 1 and 1’ |
| 1-1540 | 1+1’ | 2-Pyridyl | Me | H | S:(1) R:(1’) | Racemate of 1 and 1’ |
| 1-1541 | 1+1’ | 3-Me-2-Pyridyl | Me | H | S:(1) R:(1’) | Racemate of 1 and 1’ |
| 1-1542 | 1+1’ | $CH_2Ph$ | Me | H | S:(1) R:(1’) | Racemate of 1 and 1’ |
| 1-1543 | 1+1’ | $CH_2$(2-MePh) | Me | H | S:(1) R:(1’) | Racemate of 1 and 1’ |
| 1-1544 | 1+1’ | $CH_2$(3-MePh) | Me | H | S:(1) R:(1’) | Racemate of 1 and 1’ |
| 1-1545 | 1+1’ | $CH_2$(4-MePh) | Me | H | S:(1) R:(1’) | Racemate of 1 and 1’ |
| 1-1546 | 1+1’ | $CH_2$(2-EtPh) | Me | H | S:(1) R:(1’) | Racemate of 1 and 1’ |
| 1-1547 | 1+1’ | $CH_2$(3-EtPh) | Me | H | S:(1) R:(1’) | Racemate of 1 and 1’ |
| 1-1548 | 1+1’ | $CH_2$(4-EtPh) | Me | H | S:(1) R:(1’) | Racemate of 1 and 1’ |
| 1-1549 | 1+1’ | $CH_2$(2-MeOPh) | Me | H | S:(1) R:(1’) | Racemate of 1 and 1’ |
| 1-1550 | 1+1’ | $CH_2$(3-MeOPh) | Me | H | S:(1) R:(1’) | Racemate of 1 and 1’ |
| 1-1551 | 1+1’ | $CH_2$(4-MeOPh) | Me | H | S:(1) R:(1’) | Racemate of 1 and 1’ |
| 1-1552 | 1+1’ | $CH_2$(2-$CF_3$OPh) | Me | H | S:(1) R:(1’) | Racemate of 1 and 1’ |
| 1-1553 | 1+1’ | $CH_2$(3-$CF_3$OPh) | Me | H | S:(1) R:(1’) | Racemate of 1 and 1’ |
| 1-1554 | 1+1’ | $CH_2$(4-$CF_3$OPh) | Me | H | S:(1) R:(1’) | Racemate of 1 and 1’ |
| 1-1555 | 1+1’ | $CH_2$(2-FPh) | Me | H | S:(1) R:(1’) | Racemate of 1 and 1’ |
| 1-1556 | 1+1’ | $CH_2$(3-FPh) | Me | H | S:(1) R:(1’) | Racemate of 1 and 1’ |
| 1-1557 | 1+1’ | $CH_2$(4-FPh) | Me | H | S:(1) R:(1’) | Racemate of 1 and 1’ |
| 1-1558 | 1+1’ | $CH_2$(2-ClPh) | Me | H | S:(1) R:(1’) | Racemate of 1 and 1’ |
| 1-1559 | 1+1’ | $CH_2$(3-ClPh) | Me | H | S:(1) R:(1’) | Racemate of 1 and 1’ |
| 1-1560 | 1+1’ | $CH_2$(4-ClPh) | Me | H | S:(1) R:(1’) | Racemate of 1 and 1’ |
| 1-1561 | 1+1’ | $CH_2$(2-BrPh) | Me | H | S:(1) R:(1’) | Racemate of 1 and 1’ |
| 1-1562 | 1+1’ | $CH_2$(3-BrPh) | Me | H | S:(1) R:(1’) | Racemate of 1 and 1’ |
| 1-1563 | 1+1’ | $CH_2$(4-BrPh) | Me | H | S:(1) R:(1’) | Racemate of 1 and 1’ |
| 1-1564 | 1+1’ | $CH_2$(2-IPh) | Me | H | S:(1) R:(1’) | Racemate of 1 and 1’ |
| 1-1565 | 1+1’ | $CH_2$(3-IPh) | Me | H | S:(1) R:(1’) | Racemate of 1 and 1’ |
| 1-1566 | 1+1’ | $CH_2$(4-IPh) | Me | H | S:(1) R:(1’) | Racemate of 1 and 1’ |
| 1-1567 | 1+1’ | $CH_2$(2-$CF_3$Ph) | Me | H | S:(1) R:(1’) | Racemate of 1 and 1’ |
| 1-1568 | 1+1’ | $CH_2$(3-$CF_3$Ph) | Me | H | S:(1) R:(1’) | Racemate of 1 and 1’ |
| 1-1569 | 1+1’ | $CH_2$(4-$CF_3$Ph) | Me | H | S:(1) R:(1’) | Racemate of 1 and 1’ |
| 1-1570 | 1+1’ | $CH_2$(2-CNPh) | Me | H | S:(1) R:(1’) | Racemate of 1 and 1’ |

[Table 46]

| Table 1-Continued | | | | | | |
|---|---|---|---|---|---|---|
| No. | General formula | $R^1$ | $R^2$ | $R^3$ | Stereochemistry at C-5 position | Remark |
| 1-1571 | 1+1’ | $CH_2$(3-CNPh) | Me | H | S:(1) R:(1’) | Racemate of 1 and 1’ |
| 1-1572 | 1+1’ | $CH_2$(4-CNPh) | Me | H | S:(1) R:(1’) | Racemate of 1 and 1’ |
| 1-1573 | 1+1’ | $CH_2$(2-COOHPh) | Me | H | S:(1) R:(1’) | Racemate of 1 and 1’ |
| 1-1574 | 1+1’ | $CH_2$(3-COOHPh) | Me | H | S:(1) R:(1’) | Racemate of 1 and 1’ |
| 1-1575 | 1+1’ | $CH_2$(4-COOHPh) | Me | H | S:(1) R:(1’) | Racemate of 1 and 1’ |
| 1-1576 | 1+1’ | $CH_2$(2-COOMePh) | Me | H | S:(1) R:(1’) | Racemate of 1 and 1’ |
| 1-1577 | 1+1’ | $CH_2$(3-COOMePh) | Me | H | S:(1) R:(1’) | Racemate of 1 and 1’ |
| 1-1578 | 1+1’ | $CH_2$(4-COOMePh) | Me | H | S:(1) R:(1’) | Racemate of 1 and 1’ |
| 1-1579 | 1+1’ | $CH_2$(2,6-$Me_2$Ph) | Me | H | S:(1) R:(1’) | Racemate of 1 and 1’ |
| 1-1580 | 1+1’ | $CH_2$(2,6-$F_2$Ph) | Me | H | S:(1) R:(1’) | Racemate of 1 and 1’ |
| 1-1581 | 1+1’ | $CH_2$(2,6-$Cl_2$Ph) | Me | H | S:(1) R:(1’) | Racemate of 1 and 1’ |
| 1-1582 | 1+1’ | $CH_2$(2-Cl-6-FPh) | Me | H | S:(1) R:(1’) | Racemate of 1 and 1’ |
| 1-1583 | 1+1’ | $CH_2$(2-Br-6-ClPh) | Me | H | S:(1) R:(1’) | Racemate of 1 and 1’ |
| 1-1584 | 1+1’ | $CH_2$(1-Naphthyl) | Me | H | S:(1) R:(1’) | Racemate of 1 and 1’ |
| 1-1585 | 1+1’ | $CH_2$(2-Naphthyl) | Me | H | S:(1) R:(1’) | Racemate of 1 and 1’ |
| 1-1586 | 1+1’ | $CH_2$(2,4-$Cl_2$Ph) | Me | H | S:(1) R:(1’) | Racemate of 1 and 1’ |
| 1-1587 | 1+1’ | $CH_2$(2-Pyridyl) | Me | H | S:(1) R:(1’) | Racemate of 1 and 1’ |
| 1-1588 | 1+1’ | $CH_2$(3-Pyridyl) | Me | H | S:(1) R:(1’) | Racemate of 1 and 1’ |
| 1-1589 | 1+1’ | $CH_2$(4-Pyridyl) | Me | H | S:(1) R:(1’) | Racemate of 1 and 1’ |
| 1-1590 | 1+1’ | $CH_2$(2-Thienyl) | Me | H | S:(1) R:(1’) | Racemate of 1 and 1’ |
| 1-1591 | 1+1’ | $CH_2$(3-Thienyl) | Me | H | S:(1) R:(1’) | Racemate of 1 and 1’ |
| 1-1592 | 1+1’ | $CH_2$(3,5-$Me_2$-Isoxazol-4-ly) | Me | H | S:(1) R:(1’) | Racemate of 1 and 1’ |

(continued)

| Table 1-Continued | | | | | | |
|---|---|---|---|---|---|---|
| No. | General formula | $R^1$ | $R^2$ | $R^3$ | Stereochemistry at C-5 position | Remark |
| 1-1593 | 1+1’ | $CH_2CH_2OPh$ | Me | H | S:(1) R:(1’) | Racemate of 1 and 1’ |
| 1-1594 | 1+1’ | $CH_2CH_2OCH_2Ph$ | Me | H | S:(1) R:(1’) | Racemate of 1 and 1’ |
| 1-1595 | 1+1’ | $CH_2COPh$ | Me | H | S:(1) R:(1’) | Racemate of 1 and 1’ |
| 1-1596 | 1+1’ | $CH_2OCH_2CH_2OMe$ | Me | H | S:(1) R:(1’) | Racemate of 1 and 1’ |
| 1-1597 | 1+1’ | $CH_2$(2-FPh) | Me | H | R:(1) S:(1’) | Racemate of 1 and 1’ |
| 1-1598 | 1+1’ | $CH_2$(2-Br-6-FPh) | Br | H | S:(1) R:(1’) | Racemate of 1 and 1’ |
| 1-1599 | 1+1’ | $CH_2$(2,6-$Me_2$Ph) | Br | H | S:(1) R:(1’) | Racemate of 1 and 1’ |
| 1-1600 | 1 | $CH_2$(2-Br-6-FPh) | Br | H | S | |
| 1-1601 | 1 | $CH_2$(2-Br-6-FPh) | I | H | R | |
| 1-1602 | 1 | $CH_2$(2-FPh) | $OCH_2CH=CH_2$ | H | S | |
| 1-1603 | 1 | $CH_2$(2-FPh) | $OCH_2$c-Pr | H | S | |
| 1-1604 | 1 | $CH_2$(2-FPh) | On-Pr | H | S | |
| 1-1605 | 1 | $CH_2$(2-FPh) | $OCH_2CF_3$ | H | S | |

[Table 47]

| Table 1-Continued | | | | | | |
|---|---|---|---|---|---|---|
| No. | General formula | $R^1$ | $R^2$ | $R^3$ | Stereochemistry at C-5 position | Remark |
| 1-1606 | 1 | $CH_2$(2-FPh) | OBn | H | S | |
| 1-1607 | 1 | $CH_2$(2-FPh) | $OCH_2C\equiv CH$ | H | S | |
| 1-1608 | 1’ | $CH_2$(2,6-$Me_2$Ph) | Br | H | R | |
| 1-1609 | 1’ | $CH_2$(2-Br-6-FPh) | Br | H | R | |
| 1-1610 | 1+1’ | $CH_2$(2,6-$Me_2$Ph) | Cl | H | S:(1) R:(1’) | Racemate of 1 and 1’ |
| 1-1611 | 1+1’ | $CH_2$(2-Br-6-FPh) | Cl | H | S:(1) R:(1’) | Racemate of 1 and 1’ |
| 1-1612 | 1+1’ | $CH_2$(2-Br-6-FPh) | I | H | S:(1) R:(1’) | Racemate of 1 and 1’ |
| 1-1613 | 1 | $CH_2$(2-Br-6-FPh) | Cl | H | S | |
| 1-1614 | 1 | $CH_2$(2-Br-6-FPh) | I | H | S | |
| 1-1615 | 1 | $CH_2$(2-FPh) | OCONHEt | H | S | |

[Table 48]

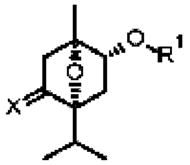 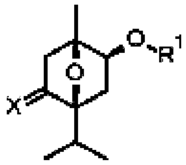

(2) (2')

| No. | General formula | $R^1$ | X | Remark |
|---|---|---|---|---|
| 2-1 | 2 | $CH_2Ph$ | O | |
| 2-2 | 2’ | $CH_2Ph$ | O | |
| 2-3 | 2 | $CH_2$(2-MePh) | O | |
| 2-4 | 2’ | $CH_2$(2-MePh) | O | |
| 2-5 | 2 | $CH_2$(2,6-$Me_2$Ph) | O | |
| 2-6 | 2’ | $CH_2$(2,6-$Me_2$Ph) | O | |
| 2-7 | 2 | $CH_2$(2-MeOPh) | O | |
| 2-8 | 2’ | $CH_2$(2-MeOPh) | O | |
| 2-9 | 2 | $CH_2$(2-FPh) | O | |
| 2-10 | 2’ | $CH_2$(2-FPh) | O | |
| 2-11 | 2 | $CH_2$(2-ClPh) | O | |
| 2-12 | 2’ | $CH_2$(2-ClPh) | O | |
| 2-13 | 2 | $CH_2$(2-BrPh) | O | |
| 2-14 | 2 | $CH_2$(2,3-$F_2$Ph) | O | |
| 2-15 | 2’ | $CH_2$(2,3-$F_2$Ph) | O | |
| 2-16 | 2 | $CH_2$(2,4-$F_2$Ph) | O | |
| 2-17 | 2’ | $CH_2$(2,4-$F_2$Ph) | O | |
| 2-18 | 2 | $CH_2$(2,5-$F_2$Ph) | O | |
| 2-19 | 2’ | $CH_2$(2,5-$F_2$Ph) | O | |
| 2-20 | 2 | $CH_2$(2,6-$F_2$Ph) | O | |
| 2-21 | 2’ | $CH_2$(2,6-$F_2$Ph) | O | |
| 2-22 | 2 | $CH_2$(3,5-$F_2$Ph) | O | |
| 2-23 | 2’ | $CH_2$(3,5-$F_2$Ph) | O | |
| 2-24 | 2 | $CH_2$(2,3,4,5,6-$F_5$Ph) | O | |
| 2-25 | 2’ | $CH_2$(2,3,4,5,6-$F_5$Ph) | O | |
| 2-26 | 2 | $CH_2$(2,6-$Cl_2$Ph) | O | |
| 2-27 | 2’ | $CH_2$(2,6-$Cl_2$Ph) | O | |
| 2-28 | 2 | $CH_2$(2-F-6-ClPh) | O | |
| 2-29 | 2 | $CH_2$(2-$CF_3$Ph) | O | |
| 2-30 | 2’ | $CH_2$(2-$CF_3$Ph) | O | |

[Table 49]

| Table 2-Continued | | | | |
|---|---|---|---|---|
| No. | General formula | $R^1$ | X | Remark |
| 2-31 | 2 | $CH_2$(2-Pyridyl) | O | |
| 2-32 | 2 | $CH_2$(3-Pyridyl) | O | |
| 2-33 | 2' | $CH_2$(3-Pyridyl) | O | |
| 2-34 | 2 | $CH_2$(4-Pyridyl) | O | |
| 2-35 | 2' | $CH_2$(4-Pyridyl) | O | |
| 2-36 | 2 | $CH_2$(2-Thienyl) | O | |
| 2-37 | 2' | $CH_2$(2-Thienyl) | O | |
| 2-38 | 2 | $CH_2$(3-Thienyl) | O | |
| 2-39 | 2 | $CH_2Ph$ | S | |
| 2-40 | 2 | $CH_2$(2-MePh) | S | |
| 2-41 | 2 | $CH_2$(2,6-$Me_2$Ph) | S | |
| 2-42 | 2 | $CH_2$(2-FPh) | S | |
| 2-43 | 2 | $CH_2$(2-ClPh) | S | |
| 2-44 | 2 | $CH_2$(2,6-$F_2$Ph) | S | |
| 2-45 | 2 | Me | $CH_2$ | |
| 2-46 | 2 | Et | $CH_2$ | |
| 2-47 | 2 | i-Pr | $CH_2$ | |
| 2-48 | 2 | i-Bu | $CH_2$ | |
| 2-49 | 2 | t-Bu | $CH_2$ | |
| 2-50 | 2 | $CH_2$t-Bu | $CH_2$ | |
| 2-51 | 2 | $CH_2CF_3$ | $CH_2$ | |
| 2-52 | 2 | $CH_2CH=CH_2$ | $CH_2$ | |
| 2-53 | 2 | $CH_2C\equiv CH$ | $CH_2$ | |
| 2-54 | 2 | c-Pr | $CH_2$ | |
| 2-55 | 2 | c-Hex | $CH_2$ | |
| 2-56 | 2 | $CH_2$c-Pr | $CH_2$ | |
| 2-57 | 2 | $CH_2$c-Hex | $CH_2$ | |
| 2-58 | 2 | $CH_2OMe$ | $CH_2$ | |
| 2-59 | 2 | $CH_2OEt$ | $CH_2$ | |
| 2-60 | 2 | $CH_2CH_2OMe$ | $CH_2$ | |
| 2-61 | 2 | Ph | $CH_2$ | |
| 2-62 | 2 | 2-MePh | $CH_2$ | |
| 2-63 | 2 | 2-FPh | $CH_2$ | |
| 2-64 | 2 | 2-ClPh | $CH_2$ | |
| 2-65 | 2 | 2,6-$F_2$Ph | $CH_2$ | |

[Table 50]

| [able 2-Continued | | | | |
|---|---|---|---|---|
| No. | General formula | $R^1$ | X | Remark |
| 2-66 | 2 | 2-Pyridyl | $CH_2$ | |
| 2-67 | 2 | 3-Pyridyl | $CH_2$ | |
| 2-68 | 2 | 4-Pyridyl | $CH_2$ | |
| 2-69 | 2 | 2-Thienyl | $CH_2$ | |
| 2-70 | 2 | 3-Thienyl | $CH_2$ | |
| 2-71 | 2 | $CH_2Ph$ | $CH_2$ | |
| 2-72 | 2 | $CH_2$(2-MePh) | $CH_2$ | |
| 2-73 | 2' | $CH_2$(2-MePh) | $CH_2$ | |
| 2-74 | 2 | $CH_2$(3-MePh) | $CH_2$ | |
| 2-75 | 2' | $CH_2$(3-MePh) | $CH_2$ | |
| 2-76 | 2 | $CH_2$(4-MePh) | $CH_2$ | |
| 2-77 | 2' | $CH_2$(4-MePh) | $CH_2$ | |
| 2-78 | 2 | $CH_2$(2,6-$Me_2$Ph) | $CH_2$ | |
| 2-79 | 2' | $CH_2$(2,6-$Me_2$Ph) | $CH_2$ | |
| 2-80 | 2 | $CH_2$(2,4,6-$Me_3$Ph) | $CH_2$ | |
| 2-81 | 2' | $CH_2$(2,4,6-$Me_3$Ph) | $CH_2$ | |
| 2-82 | 2 | $CH_2$(2-MeOPh) | $CH_2$ | |
| 2-83 | 2' | $CH_2$(2-MeOPh) | $CH_2$ | |
| 2-84 | 2 | $CH_2$(3-MeOPh) | $CH_2$ | |
| 2-85 | 2' | $CH_2$(3-MeOPh) | $CH_2$ | |
| 2-86 | 2 | $CH_2$(4-MeOPh) | $CH_2$ | |
| 2-87 | 2' | $CH_2$(4-MeOPh) | $CH_2$ | |
| 2-88 | 2 | $CH_2$(2-FPh) | $CH_2$ | |
| 2-89 | 2' | $CH_2$(2-FPh) | $CH_2$ | |
| 2-90 | 2 | $CH_2$(3-FPh) | $CH_2$ | |
| 2-91 | 2' | $CH_2$(3-FPh) | $CH_2$ | |
| 2-92 | 2 | $CH_2$(4-FPh) | $CH_2$ | |
| 2-93 | 2' | $CH_2$(4-FPh) | $CH_2$ | |
| 2-94 | 2 | $CH_2$(2-ClPh) | $CH_2$ | |
| 2-95 | 2' | $CH_2$(2-ClPh) | $CH_2$ | |
| 2-96 | 2 | $CH_2$(2-BrPh) | $CH_2$ | |
| 2-97 | 2 | $CH_2$(2-IPh) | $CH_2$ | |
| 2-98 | 2 | $CH_2$(2,3-$F_2$Ph) | $CH_2$ | |
| 2-99 | 2' | $CH_2$(2,3-$F_2$Ph) | $CH_2$ | |
| 2-100 | 2 | $CH_2$(2,4-$F_2$Ph) | $CH_2$ | |

[Table 51]

| Table 2-Continued | | | | |
|---|---|---|---|---|
| No. | General formula | $R^1$ | X | Remark |
| 2-101 | 2' | $CH_2$(2,4-$F_2$Ph) | $CH_2$ | |
| 2-102 | 2 | $CH_2$(2,5-$F_2$Ph) | $CH_2$ | |
| 2-103 | 2' | $CH_2$(2,5-$F_2$Ph) | $CH_2$ | |
| 2-104 | 2 | $CH_2$(2,6-$F_2$Ph) | $CH_2$ | |
| 2-105 | 2' | $CH_2$(2,6-$F_2$Ph) | $CH_2$ | |
| 2-106 | 2 | $CH_2$(3,5-$F_2$Ph) | $CH_2$ | |
| 2-107 | 2' | $CH_2$(3,5-$F_2$Ph) | $CH_2$ | |
| 2-108 | 2 | $CH_2$(2,4,6-$F_3$Ph) | $CH_2$ | |
| 2-109 | 2 | $CH_2$(2,3,4,5,6-$F_5$Ph) | $CH_2$ | |
| 2-110 | 2 | $CH_2$(2,6-$Cl_2$Ph) | $CH_2$ | |
| 2-111 | 2' | $CH_2$(2,6-$Cl_2$Ph) | $CH_2$ | |
| 2-112 | 2 | $CH_2$(2-F-6-ClPh) | $CH_2$ | |
| 2-113 | 2' | $CH_2$(2-F-6-ClPh) | $CH_2$ | |
| 2-114 | 2 | $CH_2$(2-CNPh) | $CH_2$ | |
| 2-115 | 2 | $CH_2$(2-$NO_2$Ph) | $CH_2$ | |
| 2-116 | 2 | $CH_2$(2-$CF_3$Ph) | $CH_2$ | |
| 2-117 | 2' | $CH_2$(2-$CF_3$Ph) | $CH_2$ | |
| 2-118 | 2 | $CH_2$(3-$CF_3$Ph) | $CH_2$ | |
| 2-119 | 2' | $CH_2$(3-$CF_3$Ph) | $CH_2$ | |
| 2-120 | 2 | $CH_2$(4-$CF_3$Ph) | $CH_2$ | |
| 2-121 | 2' | $CH_2$(4-$CF_3$Ph) | $CH_2$ | |
| 2-122 | 2 | $CH_2$(2-F-6-$CF_3$Ph) | $CH_2$ | |
| 2-123 | 2' | $CH_2$(2-F-6-$CF_3$Ph) | $CH_2$ | |
| 2-124 | 2 | $CH_2$(2-$CF_3$OPh) | $CH_2$ | |
| 2-125 | 2' | $CH_2$(2-$CF_3$OPh) | $CH_2$ | |
| 2-126 | 2 | $CH_2$(3-$CF_3$OPh) | $CH_2$ | |
| 2-127 | 2' | $CH_2$(3-$CF_3$OPh) | $CH_2$ | |
| 2-128 | 2 | $CH_2$(4-$CF_3$OPh) | $CH_2$ | |
| 2-129 | 2' | $CH_2$(4-$CF_3$OPh) | $CH_2$ | |
| 2-130 | 2 | $CH_2CH_2Ph$ | $CH_2$ | |
| 2-131 | 2 | $CH_2CH_2$(2-MePh) | $CH_2$ | |
| 2-132 | 2 | $CH_2CH_2$(2-FPh) | $CH_2$ | |
| 2-133 | 2 | $CH_2CH_2$(2-ClPh) | $CH_2$ | |
| 2-134 | 2 | $CH_2CH_2$(2,6-$F_2$Ph) | $CH_2$ | |
| 2-135 | 2 | $CH_2CH_2CH_2Ph$ | $CH_2$ | |

[Table 52]

| Table 2-Continued | | | | |
|---|---|---|---|---|
| No. | General formula | $R^1$ | X | Remark |
| 2-136 | 2 | $CH_2CH_2CH_2$(2-MePh) | $CH_2$ | |
| 2-137 | 2 | $CH_2CH_2CH_2$(2-FPh) | $CH_2$ | |
| 2-138 | 2 | $CH_2CH_2CH_2$(2-ClPh) | $CH_2$ | |
| 2-139 | 2 | $CH_2CH_2CH_2$(2,6-$F_2$Ph) | $CH_2$ | |
| 2-140 | 2 | $CH_2$(2-Pyridyl) | $CH_2$ | |
| 2-141 | 2 | $CH_2$(3-Pyridyl) | $CH_2$ | |
| 2-142 | 2 | $CH_2$(4-Pyridyl) | $CH_2$ | |
| 2-143 | 2 | $CH_2$(3-Me-2-Pyridyl) | $CH_2$ | |
| 2-144 | 2 | $CH_2$(3-F-2-Pyridyl) | $CH_2$ | |
| 2-145 | 2 | $CH_2$(3-Cl-2-Pyridyl) | $CH_2$ | |
| 2-146 | 2 | $CH_2$(2-Me-3-Pyridyl) | $CH_2$ | |
| 2-147 | 2 | $CH_2$(2-F-3-Pyridyl) | $CH_2$ | |
| 2-148 | 2 | $CH_2$(2-Cl-3-Pyridyl) | $CH_2$ | |
| 2-149 | 2 | $CH_2$(2-Pyrimidyl) | $CH_2$ | |
| 2-150 | 2 | $CH_2$(4-Pyrimidyl) | $CH_2$ | |
| 2-151 | 2 | $CH_2$(5-Pyrimidyl) | $CH_2$ | |
| 2-152 | 2 | 2-Furfuryl | $CH_2$ | |
| 2-153 | 2 | 3-Furfuryl | $CH_2$ | |
| 2-154 | 2 | $CH_2$(2-Thienyl) | $CH_2$ | |
| 2-155 | 2 | $CH_2$(3-Thienyl) | $CH_2$ | |
| 2-156 | 2 | $CH_2$(1-Me-4-Pyrazolyl) | $CH_2$ | |
| 2-157 | 2 | $CH_2CH_2OPh$ | $CH_2$ | |
| 2-158 | 2 | $CH_2CH_2O$(2-MePh) | $CH_2$ | |
| 2-159 | 2 | $CH_2CH_2O$(2-FPh) | $CH_2$ | |
| 2-160 | 2 | $CH_2CH_2O$(2-ClPh) | $CH_2$ | |
| 2-161 | 2 | $CH_2CH_2O$(2,6-$F_2$Ph) | $CH_2$ | |
| 2-162 | 2 | $CH_2CH_2OCH_2Ph$ | $CH_2$ | |
| 2-163 | 2 | $CH_2CH_2OCH_2$(2-MePh) | $CH_2$ | |
| 2-164 | 2 | $CH_2CH_2OCH_2$(2-FPh) | $CH_2$ | |
| 2-165 | 2 | $CH_2CH_2OCH_2$(2-ClPh) | $CH_2$ | |
| 2-166 | 2 | $CH_2CH_2OCH_2$(2,6-$F_2$Ph) | $CH_2$ | |
| 2-167 | 2 | $CH_2COPh$ | $CH_2$ | |
| 2-168 | 2 | $CH_2CO$(2-MePh) | $CH_2$ | |
| 2-169 | 2 | $CH_2CO$(2-FPh) | $CH_2$ | |
| 2-170 | 2 | $CH_2CO$(2-ClPh) | $CH_2$ | |

[Table 53]

| Table 2-Continued | | | | |
|---|---|---|---|---|
| No. | General formula | $R^1$ | X | Remark |
| 2-171 | 2 | $CH_2CO(2,6-F_2Ph)$ | $CH_2$ | |
| 2-172 | 2 | $CH_2Ph$ | CH(Me) | E isomer |
| 2-173 | 2 | $CH_2(2-MePh)$ | CH(Me) | E isomer |
| 2-174 | 2 | $CH_2(2,6-Me_2Ph)$ | CH(Me) | E isomer |
| 2-175 | 2 | $CH_2(2-FPh)$ | CH(Me) | E isomer |
| 2-176 | 2 | $CH_2(2-ClPh)$ | CH(Me) | E isomer |
| 2-177 | 2 | $CH_2(2,6-F_2Ph)$ | CH(Me) | E isomer |
| 2-178 | 2 | $CH_2Ph$ | CH(Me) | Z isomer |
| 2-179 | 2 | $CH_2(2-MePh)$ | CH(Me) | Z isomer |
| 2-180 | 2 | $CH_2(2,6-Me_2Ph)$ | CH(Me) | Z isomer |
| 2-181 | 2 | $CH_2(2-FPh)$ | CH(Me) | Z isomer |
| 2-182 | 2 | $CH_2(2-ClPh)$ | CH(Me) | Z isomer |
| 2-183 | 2 | $CH_2(2,6-F_2Ph)$ | CH(Me) | Z isomer |
| 2-184 | 2 | $CH_2Ph$ | $C(Me)_2$ | |
| 2-185 | 2 | $CH_2(2-MePh)$ | $C(Me)_2$ | |
| 2-186 | 2 | $CH_2(2,6-Me_2Ph)$ | $C(Me)_2$ | |
| 2-187 | 2 | $CH_2(2-FPh)$ | $C(Me)_2$ | |
| 2-188 | 2 | $CH_2(2-ClPh)$ | $C(Me)_2$ | |
| 2-189 | 2 | $CH_2(2,6-F_2Ph)$ | $C(Me)_2$ | |
| 2-190 | 2 | $CH_2Ph$ | NOH | E isomer |
| 2-191 | 2 | $CH_2(2-MePh)$ | NOH | E isomer |
| 2-192 | 2' | $CH_2(2-MePh)$ | NOH | E isomer |
| 2-193 | 2 | $CH_2(2,6-Me_2Ph)$ | NOH | E isomer |
| 2-194 | 2' | $CH_2(2,6-Me_2Ph)$ | NOH | E isomer |
| 2-195 | 2 | $CH_2(2-MeOPh)$ | NOH | E isomer |
| 2-196 | 2' | $CH_2(2-MeOPh)$ | NOH | E isomer |
| 2-197 | 2 | $CH_2(2-FPh)$ | NOH | E isomer |
| 2-198 | 2' | $CH_2(2-FPh)$ | NOH | E isomer |
| 2-199 | 2 | $CH_2(2-ClPh)$ | NOH | E isomer |
| 2-200 | 2' | $CH_2(2-ClPh)$ | NOH | E isomer |
| 2-201 | 2 | $CH_2(2,3-F_2Ph)$ | NOH | E isomer |
| 2-202 | 2' | $CH_2(2,3-F_2Ph)$ | NOH | E isomer |
| 2-203 | 2 | $CH_2(2,4-F_2Ph)$ | NOH | E isomer |
| 2-204 | 2' | $CH_2(2,4-F_2Ph)$ | NOH | E isomer |
| 2-205 | 2 | $CH_2(2,5-F_2Ph)$ | NOH | E isomer |

[Table 54]

| Table 2-Continued | | | | |
|---|---|---|---|---|
| No. | General formula | $R^1$ | X | Remark |
| 2-206 | 2’ | $CH_2(2,5\text{-}F_2Ph)$ | NOH | E isomer |
| 2-207 | 2 | $CH_2(2,6\text{-}F_2Ph)$ | NOH | E isomer |
| 2-208 | 2’ | $CH_2(2,6\text{-}F_2Ph)$ | NOH | E isomer |
| 2-209 | 2 | $CH_2(3,5\text{-}F_2Ph)$ | NOH | E isomer |
| 2-210 | 2’ | $CH_2(3,5\text{-}F_2Ph)$ | NOH | E isomer |
| 2-211 | 2 | $CH_2(2,3,4,5,6\text{-}F_5Ph)$ | NOH | E isomer |
| 2-212 | 2’ | $CH_2(2,3,4,5,6\text{-}F_5Ph)$ | NOH | E isomer |
| 2-213 | 2 | $CH_2(2,6\text{-}Cl_2Ph)$ | NOH | E isomer |
| 2-214 | 2’ | $CH_2(2,6\text{-}Cl_2Ph)$ | NOH | E isomer |
| 2-215 | 2 | $CH_2$(2-F-6-ClPh) | NOH | E isomer |
| 2-216 | 2 | $CH_2(2\text{-}CF_3Ph)$ | NOH | E isomer |
| 2-217 | 2’ | $CH_2(2\text{-}CF_3Ph)$ | NOH | E isomer |
| 2-218 | 2 | $CH_2$(2-Pyridyl) | NOH | E isomer |
| 2-219 | 2 | $CH_2$(3-Pyridyl) | NOH | E isomer |
| 2-220 | 2’ | $CH_2$(3-Pyridyl) | NOH | E isomer |
| 2-221 | 2 | $CH_2$(4-Pyridyl) | NOH | E isomer |
| 2-222 | 2’ | $CH_2$(4-Pyridyl) | NOH | E isomer |
| 2-223 | 2 | $CH_2$(2-Thienyl) | NOH | E isomer |
| 2-224 | 2’ | $CH_2$(2-Thienyl) | NOH | E isomer |
| 2-225 | 2 | $CH_2$(3-Thienyl) | NOH | E isomer |
| 2-226 | 2 | $CH_2Ph$ | NOH | Z isomer |
| 2-227 | 2 | $CH_2$(2-MePh) | NOH | Z isomer |
| 2-228 | 2’ | $CH_2$(2-MePh) | NOH | Z isomer |
| 2-229 | 2 | $CH_2(2,6\text{-}Me_2Ph)$ | NOH | Z isomer |
| 2-230 | 2 | $CH_2$(2-FPh) | NOH | Z isomer |
| 2-231 | 2’ | $CH_2$(2-FPh) | NOH | Z isomer |
| 2-232 | 2 | $CH_2$(2-ClPh) | NOH | Z isomer |
| 2-233 | 2 | $CH_2(2,6\text{-}F_2Ph)$ | NOH | Z isomer |
| 2-234 | 2 | $CH_2Ph$ | NMe | E isomer |
| 2-235 | 2 | $CH_2$(2-MePh) | NMe | E isomer |
| 2-236 | 2’ | $CH_2$(2-MePh) | NMe | E isomer |
| 2-237 | 2 | $CH_2(2,6\text{-}Me_2Ph)$ | NMe | E isomer |
| 2-238 | 2 | $CH_2$(2-FPh) | NMe | E isomer |
| 2-239 | 2’ | $CH_2$(2-FPh) | NMe | E isomer |
| 2-240 | 2 | $CH_2$(2-ClPh) | NMe | E isomer |

[Table 55]

| Table 2-Continued | | | | |
|---|---|---|---|---|
| No. | General formula | $R^1$ | X | Remark |
| 2-241 | 2 | $CH_2(2,6-F_2Ph)$ | NMe | E isomer |
| 2-242 | 2 | $CH_2Ph$ | NMe | Z isomer |
| 2-243 | 2 | $CH_2(2-MePh)$ | NMe | Z isomer |
| 2-244 | 2' | $CH_2(2-MePh)$ | NMe | Z isomer |
| 2-245 | 2 | $CH_2(2,6-Me_2Ph)$ | NMe | Z isomer |
| 2-246 | 2 | $CH_2(2-FPh)$ | NMe | Z isomer |
| 2-247 | 2' | $CH_2(2-FPh)$ | NMe | Z isomer |
| 2-248 | 2 | $CH_2(2-ClPh)$ | NMe | Z isomer |
| 2-249 | 2 | $CH_2(2,6-F_2Ph)$ | NMe | Z isomer |
| 2-250 | 2 | $CH_2Ph$ | NOMe | E isomer |
| 2-251 | 2 | $CH_2(2-MePh)$ | NOMe | E isomer |
| 2-252 | 2' | $CH_2(2-MePh)$ | NOMe | E isomer |
| 2-253 | 2 | $CH_2(2,6-Me_2Ph)$ | NOMe | E isomer |
| 2-254 | 2 | $CH_2(2-FPh)$ | NOMe | E isomer |
| 2-255 | 2' | $CH_2(2-FPh)$ | NOMe | E isomer |
| 2-256 | 2 | $CH_2(2-ClPh)$ | NOMe | E isomer |
| 2-257 | 2 | $CH_2(2,6-F_2Ph)$ | NOMe | E isomer |
| 2-258 | 2 | $CH_2Ph$ | NOMe | Z isomer |
| 2-259 | 2 | $CH_2(2-MePh)$ | NOMe | Z isomer |
| 2-260 | 2' | $CH_2(2-MePh)$ | NOMe | Z isomer |
| 2-261 | 2 | $CH_2(2,6-Me_2Ph)$ | NOMe | Z isomer |
| 2-262 | 2 | $CH_2(2-FPh)$ | NOMe | Z isomer |
| 2-263 | 2' | $CH_2(2-FPh)$ | NOMe | Z isomer |
| 2-264 | 2 | $CH_2(2-ClPh)$ | NOMe | Z isomer |
| 2-265 | 2 | $CH_2(2,6-F_2Ph)$ | NOMe | Z isomer |
| 2-266 | 2 | $CH_2Ph$ | NOEt | E isomer |
| 2-267 | 2 | $CH_2(2-MePh)$ | NOEt | E isomer |
| 2-268 | 2' | $CH_2(2-MePh)$ | NOEt | E isomer |
| 2-269 | 2 | $CH_2(2,6-Me_2Ph)$ | NOEt | E isomer |
| 2-270 | 2 | $CH_2(2-FPh)$ | NOEt | E isomer |
| 2-271 | 2' | $CH_2(2-FPh)$ | NOEt | E isomer |
| 2-272 | 2 | $CH_2(2-ClPh)$ | NOEt | E isomer |
| 2-273 | 2 | $CH_2(2,6-F_2Ph)$ | NOEt | E isomer |
| 2-274 | 2 | $CH_2Ph$ | NOEt | Z isomer |
| 2-275 | 2 | $CH_2(2-MePh)$ | NOEt | Z isomer |

[Table 56]

| Table 2-Continued | | | | |
|---|---|---|---|---|
| No. | General formula | $R^1$ | X | Remark |
| 2-276 | 2' | $CH_2$(2-MePh) | NOEt | Z isomer |
| 2-277 | 2 | $CH_2$(2,6-$Me_2$Ph) | NOEt | Z isomer |
| 2-278 | 2 | $CH_2$(2-FPh) | NOEt | Z isomer |
| 2-279 | 2' | $CH_2$(2-FPh) | NOEt | Z isomer |
| 2-280 | 2 | $CH_2$(2-ClPh) | NOEt | Z isomer |
| 2-281 | 2 | $CH_2$(2,6-$F_2$Ph) | NOEt | Z isomer |
| 2-282 | 2 | $CH_2$Ph | $NNH_2$ | E isomer |
| 2-283 | 2 | $CH_2$(2-MePh) | $NNH_2$ | E isomer |
| 2-284 | 2' | $CH_2$(2-MePh) | $NNH_2$ | E isomer |
| 2-285 | 2 | $CH_2$(2,6-$Me_2$Ph) | $NNH_2$ | E isomer |
| 2-286 | 2 | $CH_2$(2-FPh) | $NNH_2$ | E isomer |
| 2-287 | 2' | $CH_2$(2-FPh) | $NNH_2$ | E isomer |
| 2-288 | 2 | $CH_2$(2-ClPh) | $NNH_2$ | E isomer |
| 2-289 | 2 | $CH_2$(2,6-$F_2$Ph) | $NNH_2$ | E isomer |
| 2-290 | 2 | $CH_2$Ph | $NNH_2$ | Z isomer |
| 2-291 | 2 | $CH_2$(2-MePh) | $NNH_2$ | Z isomer |
| 2-292 | 2' | $CH_2$(2-MePh) | $NNH_2$ | Z isomer |
| 2-293 | 2 | $CH_2$(2,6-$Me_2$Ph) | $NNH_2$ | Z isomer |
| 2-294 | 2 | $CH_2$(2-FPh) | $NNH_2$ | Z isomer |
| 2-295 | 2' | $CH_2$(2-FPh) | $NNH_2$ | Z isomer |
| 2-296 | 2 | $CH_2$(2-ClPh) | $NNH_2$ | Z isomer |
| 2-297 | 2 | $CH_2$(2,6-$F_2$Ph) | $NNH_2$ | Z isomer |
| 2-298 | 2 | $CH_2$Ph | NNHMe | E isomer |
| 2-299 | 2 | $CH_2$(2-MePh) | NNHMe | E isomer |
| 2-300 | 2' | $CH_2$(2-MePh) | NNHMe | E isomer |
| 2-301 | 2 | $CH_2$(2,6-$Me_2$Ph) | NNHMe | E isomer |
| 2-302 | 2 | $CH_2$(2-FPh) | NNHMe | E isomer |
| 2-303 | 2' | $CH_2$(2-FPh) | NNHMe | E isomer |
| 2-304 | 2 | $CH_2$(2-ClPh) | NNHMe | E isomer |
| 2-305 | 2 | $CH_2$(2,6-$F_2$Ph) | NNHMe | E isomer |
| 2-306 | 2 | $CH_2$Ph | NNHMe | Z isomer |
| 2-307 | 2 | $CH_2$(2-MePh) | NNHMe | Z isomer |
| 2-308 | 2' | $CH_2$(2-MePh) | NNHMe | Z isomer |
| 2-309 | 2 | $CH_2$(2,6-$Me_2$Ph) | NNHMe | Z isomer |
| 2-310 | 2 | $CH_2$(2-FPh) | NNHMe | Z isomer |

[Table 57]

| Table 2-Continued | | | | |
|---|---|---|---|---|
| No. | General formula | $R^1$ | X | Remark |
| 2-311 | 2’ | $CH_2$(2-FPh) | NNHMe | Z isomer |
| 2-312 | 2 | $CH_2$(2-ClPh) | NNHMe | Z isomer |
| 2-313 | 2 | $CH_2$(2,6-$F_2$Ph) | NNHMe | Z isomer |
| 2-314 | 2 | $CH_2$Ph | $NNHCONH_2$ | E isomer |
| 2-315 | 2 | $CH_2$(2-MePh) | $NNHCONH_2$ | E isomer |
| 2-316 | 2’ | $CH_2$(2-MePh) | $NNHCONH_2$ | E isomer |
| 2-317 | 2 | $CH_2$(2,6-$Me_2$Ph) | $NNHCONH_2$ | E isomer |
| 2-318 | 2 | $CH_2$(2-FPh) | $NNHCONH_2$ | E isomer |
| 2-319 | 2’ | $CH_2$(2-FPh) | $NNHCONH_2$ | E isomer |
| 2-320 | 2 | $CH_2$(2-ClPh) | $NNHCONH_2$ | E isomer |
| 2-321 | 2 | $CH_2$(2,6-$F_2$Ph) | $NNHCONH_2$ | E isomer |
| 2-322 | 2 | $CH_2$Ph | $NNHCONH_2$ | Z isomer |
| 2-323 | 2 | $CH_2$(2-MePh) | $NNHCONH_2$ | Z isomer |
| 2-324 | 2’ | $CH_2$(2-MePh) | $NNHCONH_2$ | Z isomer |
| 2-325 | 2 | $CH_2$(2,6-$Me_2$Ph) | $NNHCONH_2$ | Z isomer |
| 2-326 | 2 | $CH_2$(2-FPh) | $NNHCONH_2$ | Z isomer |
| 2-327 | 2’ | $CH_2$(2-FPh) | $NNHCONH_2$ | Z isomer |
| 2-328 | 2 | $CH_2$(2-ClPh) | $NNHCONH_2$ | Z isomer |
| 2-329 | 2 | $CH_2$(2,6-$F_2$Ph) | $NNHCONH_2$ | Z isomer |
| 2-330 | 2 | $CH_2$Ph | NNHPh | E isomer |
| 2-331 | 2 | $CH_2$(2-MePh) | NNHPh | E isomer |
| 2-332 | 2’ | $CH_2$(2-MePh) | NNHPh | E isomer |
| 2-333 | 2 | $CH_2$(2,6-$Me_2$Ph) | NNHPh | E isomer |
| 2-334 | 2 | $CH_2$(2-FPh) | NNHPh | E isomer |
| 2-335 | 2’ | $CH_2$(2-FPh) | NNHPh | E isomer |
| 2-336 | 2 | $CH_2$(2-ClPh) | NNHPh | E isomer |
| 2-337 | 2 | $CH_2$(2,6-$F_2$Ph) | NNHPh | E isomer |
| 2-338 | 2 | $CH_2$Ph | NNHPh | Z isomer |
| 2-339 | 2 | $CH_2$(2-MePh) | NNHPh | Z isomer |
| 2-340 | 2’ | $CH_2$(2-MePh) | NNHPh | Z isomer |
| 2-341 | 2 | $CH_2$(2,6-$Me_2$Ph) | NNHPh | Z isomer |
| 2-342 | 2 | $CH_2$(2-FPh) | NNHPh | Z isomer |
| 2-343 | 2’ | $CH_2$(2-FPh) | NNHPh | Z isomer |
| 2-344 | 2 | $CH_2$(2-ClPh) | NNHPh | Z isomer |
| 2-345 | 2 | $CH_2$(2,6-$F_2$Ph) | NNHPh | Z isomer |

[Table 58]

| Table 2-Continued | | | | |
|---|---|---|---|---|
| No. | General formula | $R^1$ | X | Remark |
| 2-346 | 2 | $CH_2$(4-MeOPh) | O | |
| 2-347 | 2 | $CH_2$(3-MeOPh) | O | |
| 2-348 | 2 | $CH_2$(2-$CF_3$OPh) | O | |
| 2-349 | 2 | $CH_2$(4-FPh) | O | |
| 2-350 | 2 | $CH_2$(4-MePh) | O | |
| 2-351 | 2 | $CH_2$(3-FPh) | O | |
| 2-352 | 2 | $CH_2$(3-MePh) | O | |
| 2-353 | 2 | $CH_2$(2-F-6-MePh) | O | |
| 2-354 | 2 | $CH_2$(2-CNPh) | O | |
| 2-355 | 2 | $CH_2$(2-Br-6-FPh) | O | |
| 2-356 | 2 | $CH_2CH=CH_2$ | O | |
| 2-357 | 2 | $CH_2CH=CMe_2$ | O | |
| 2-358 | 2 | $CH_2C \equiv CH$ | O | |
| 2-359 | 2 | $CH_2C \equiv CMe$ | O | |

[Table 59]

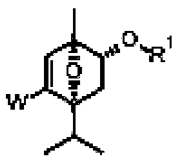
(3)
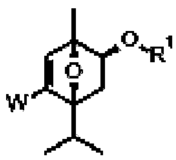
(3')

| No. | General formula | $R^1$ | W | Remark |
|---|---|---|---|---|
| 3-1 | 3 | $CH_2Ph$ | H | |
| 3-2 | 3 | $CH_2$(2-MePh) | H | |
| 3-3 | 3 | $CH_2$(2,6-$Me_2$Ph) | H | |
| 3-4 | 3 | $CH_2$(2-MeOPh) | H | |
| 3-5 | 3 | $CH_2$(2-FPh) | H | |
| 3-6 | 3 | $CH_2$(2-ClPh) | H | |
| 3-7 | 3 | $CH_2$(2,6-$F_2$Ph) | H | |
| 3-8 | 3 | $CH_2Ph$ | F | |
| 3-9 | 3 | $CH_2$(2-MePh) | F | |
| 3-10 | 3 | $CH_2$(2,6-$Me_2$Ph) | F | |
| 3-11 | 3 | $CH_2$(2-MeOPh) | F | |
| 3-12 | 3 | $CH_2$(2-FPh) | F | |
| 3-13 | 3 | $CH_2$(2-ClPh) | F | |
| 3-14 | 3 | $CH_2$(2,6-$F_2$Ph) | F | |
| 3-15 | 3 | $CH_2Ph$ | Cl | |
| 3-16 | 3 | $CH_2$(2-MePh) | Cl | |

(continued)

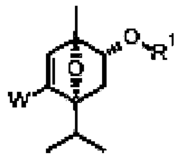 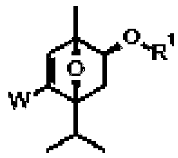

(3) (3')

| No. | General formula | $R^1$ | W | Remark |
|---|---|---|---|---|
| 3-17 | 3 | $CH_2(2,6\text{-}Me_2Ph)$ | Cl | |
| 3-18 | 3 | $CH_2(2\text{-}MeOPh)$ | Cl | |
| 3-19 | 3 | $CH_2(2\text{-}FPh)$ | Cl | |
| 3-20 | 3 | $CH_2(2\text{-}ClPh)$ | Cl | |
| 3-21 | 3 | $CH_2(2,6\text{-}F_2Ph)$ | Cl | |
| 3-22 | 3 | $CH_2Ph$ | Br | |
| 3-23 | 3 | $CH_2(2\text{-}MePh)$ | Br | |
| 3-24 | 3 | $CH_2(2,6\text{-}Me_2Ph)$ | Br | |
| 3-25 | 3 | $CH_2(2\text{-}MeOPh)$ | Br | |
| 3-26 | 3 | $CH_2(2\text{-}FPh)$ | Br | |
| 3-27 | 3 | $CH_2(2\text{-}ClPh)$ | Br | |
| 3-28 | 3 | $CH_2(2,6\text{-}F_2Ph)$ | Br | |
| 3-29 | 3 | $CH_2Ph$ | Me | |
| 3-30 | 3 | $CH_2(2\text{-}MePh)$ | Me | |

[Table 60]

Table 3-Continued

| No. | General formula | $R^1$ | W | Remark |
|---|---|---|---|---|
| 3-31 | 3' | $CH_2(2\text{-}MePh)$ | Me | |
| 3-32 | 3 | $CH_2(2,6\text{-}Me_2Ph)$ | Me | |
| 3-33 | 3 | $CH_2(2\text{-}MeOPh)$ | Me | |
| 3-34 | 3' | $CH_2(2\text{-}MeOPh)$ | Me | |
| 3-35 | 3 | $CH_2(2\text{-}FPh)$ | Me | |
| 3-36 | 3' | $CH_2(2\text{-}FPh)$ | Me | |
| 3-37 | 3 | $CH_2(2\text{-}ClPh)$ | Me | |
| 3-38 | 3' | $CH_2(2\text{-}ClPh)$ | Me | |
| 3-39 | 3 | $CH_2(2\text{-}BrPh)$ | Me | |
| 3-40 | 3 | $CH_2(2,3\text{-}F_2Ph)$ | Me | |
| 3-41 | 3' | $CH_2(2,3\text{-}F_2Ph)$ | Me | |
| 3-42 | 3 | $CH_2(2,4\text{-}F_2Ph)$ | Me | |
| 3-43 | 3' | $CH_2(2,4\text{-}F_2Ph)$ | Me | |
| 3-44 | 3 | $CH_2(2,5\text{-}F_2Ph)$ | Me | |
| 3-45 | 3' | $CH_2(2,5\text{-}F_2Ph)$ | Me | |
| 3-46 | 3 | $CH_2(2,6\text{-}F_2Ph)$ | Me | |

(continued)

| Table 3-Continued | | | | |
|---|---|---|---|---|
| No. | General formula | $R^1$ | W | Remark |
| 3-47 | 3' | $CH_2(2,6-F_2Ph)$ | Me | |
| 3-48 | 3 | $CH_2(3,5-F_2Ph)$ | Me | |
| 3-49 | 3 | $CH_2(2,6-Cl_2Ph)$ | Me | |
| 3-50 | 3 | $CH_2$(2-F-6-ClPh) | Me | |
| 3-51 | 3 | $CH_2(2-CF_3Ph)$ | Me | |
| 3-52 | 3 | $CH_2Ph$ | Et | |
| 3-53 | 3 | $CH_2$(2-MePh) | Et | |
| 3-54 | 3 | $CH_2(2,6-Me_2Ph)$ | Et | |
| 3-55 | 3 | $CH_2$(2-MeOPh) | Et | |
| 3-56 | 3 | $CH_2$(2-FPh) | Et | |
| 3-57 | 3 | $CH_2$(2-ClPh) | Et | |
| 3-58 | 3 | $CH_2(2,6-F_2Ph)$ | Et | |
| 3-59 | 3 | $CH_2Ph$ | i-Pr | |
| 3-60 | 3 | $CH_2$(2-MePh) | i-Pr | |
| 3-61 | 3 | $CH_2(2,6-Me_2Ph)$ | i-Pr | |
| 3-62 | 3 | $CH_2$(2-MeOPh) | i-Pr | |
| 3-63 | 3 | $CH_2$(2-FPh) | i-Pr | |
| 3-64 | 3 | $CH_2$(2-ClPh) | i-Pr | |
| 3-65 | 3 | $CH_2(2,6-F_2Ph)$ | i-Pr | |

[Table 61]

| Table 3-Continued | | | | |
|---|---|---|---|---|
| No. | General formula | $R^1$ | W | Remark |
| 3-66 | 3 | $CH_2Ph$ | n-Pr | |
| 3-67 | 3 | $CH_2$(2-MePh) | n-Pr | |
| 3-68 | 3 | $CH_2(2,6-Me_2Ph)$ | n-Pr | |
| 3-69 | 3 | $CH_2$(2-MeOPh) | n-Pr | |
| 3-70 | 3 | $CH_2$(2-FPh) | n-Pr | |
| 3-71 | 3 | $CH_2$(2-ClPh) | n-Pr | |
| 3-72 | 3 | $CH_2(2,6-F_2Ph)$ | n-Pr | |
| 3-73 | 3 | $CH_2Ph$ | $CF_3$ | |
| 3-74 | 3 | $CH_2$(2-MePh) | $CF_3$ | |
| 3-75 | 3 | $CH_2(2,6-Me_2Ph)$ | $CF_3$ | |
| 3-76 | 3 | $CH_2$(2-MeOPh) | $CF_3$ | |
| 3-77 | 3 | $CH_2$(2-FPh) | $CF_3$ | |
| 3-78 | 3 | $CH_2$(2-ClPh) | $CF_3$ | |
| 3-79 | 3 | $CH_2(2,6-F_2Ph)$ | $CF_3$ | |
| 3-80 | 3 | $CH_2Ph$ | OMe | |

(continued)

| Table 3-Continued | | | | |
|---|---|---|---|---|
| No. | General formula | $R^1$ | W | Remark |
| 3-81 | 3 | $CH_2$(2-MePh) | OMe | |
| 3-82 | 3' | $CH_2$(2-MePh) | OMe | |
| 3-83 | 3 | $CH_2$(2,6-$Me_2$Ph) | OMe | |
| 3-84 | 3 | $CH_2$(2-MeOPh) | OMe | |
| 3-85 | 3' | $CH_2$(2-MeOPh) | OMe | |
| 3-86 | 3 | $CH_2$(2-FPh) | OMe | |
| 3-87 | 3' | $CH_2$(2-FPh) | OMe | |
| 3-88 | 3 | $CH_2$(2-ClPh) | OMe | |
| 3-89 | 3' | $CH_2$(2-ClPh) | OMe | |
| 3-90 | 3 | $CH_2$(2-BrPh) | OMe | |
| 3-91 | 3 | $CH_2$(2,6-$F_2$Ph) | OMe | |
| 3-92 | 3 | $CH_2$(2,6-$Cl_2$Ph) | OMe | |
| 3-93 | 3 | $CH_2$(2-F-6-ClPh) | OMe | |
| 3-94 | 3 | $CH_2$(2-$CF_3$Ph) | OMe | |
| 3-95 | 3 | $CH_2$Ph | OEt | |
| 3-96 | 3 | $CH_2$(2-MePh) | OEt | |
| 3-97 | 3 | $CH_2$(2,6-$Me_2$Ph) | OEt | |
| 3-98 | 3 | $CH_2$(2-MeOPh) | OEt | |
| 3-99 | 3 | $CH_2$(2-FPh) | OEt | |
| 3-100 | 3 | $CH_2$(2-ClPh) | OEt | |

[Table 62]

| Table 3-Continued | | | | |
|---|---|---|---|---|
| No. | General formula | $R^1$ | W | Remark |
| 3-101 | 3 | $CH_2$(2,6-$F_2$Ph) | OEt | |
| 3-102 | 3 | $CH_2$Ph | $OCH_2CF_3$ | |
| 3-103 | 3 | $CH_2$(2-MePh) | $OCH_2CF_3$ | |
| 3-104 | 3 | $CH_2$(2,6-$Me_2$Ph) | $OCH_2CF_3$ | |
| 3-105 | 3 | $CH_2$(2-MeOPh) | $OCH_2CF_3$ | |
| 3-106 | 3 | $CH_2$(2-FPh) | $OCH_2CF_3$ | |
| 3-107 | 3 | $CH_2$(2-ClPh) | $OCH_2CF_3$ | |
| 3-108 | 3 | $CH_2$(2,6-$F_2$Ph) | $OCH_2CF_3$ | |
| 3-109 | 3 | $CH_2$Ph | $CH=CH_2$ | |
| 3-110 | 3 | $CH_2$(2-MePh) | $CH=CH_2$ | |
| 3-111 | 3 | $CH_2$(2,6-$Me_2$Ph) | $CH=CH_2$ | |
| 3-112 | 3 | $CH_2$(2-MeOPh) | $CH=CH_2$ | |
| 3-113 | 3 | $CH_2$(2-FPh) | $CH=CH_2$ | |
| 3-114 | 3 | $CH_2$(2-ClPh) | $CH=CH_2$ | |

(continued)

| Table 3-Continued | | | | |
|---|---|---|---|---|
| No. | General formula | $R^1$ | W | Remark |
| 3-115 | 3 | $CH_2(2,6-F_2Ph)$ | $CH=CH_2$ | |
| 3-116 | 3 | $CH_2Ph$ | C=CH | |
| 3-117 | 3 | $CH_2(2-MePh)$ | C=CH | |
| 3-118 | 3 | $CH_2(2,6-Me_2Ph)$ | C=CH | |
| 3-119 | 3 | $CH_2(2-MeOPh)$ | C=CH | |
| 3-120 | 3 | $CH_2(2-FPh)$ | C=CH | |
| 3-121 | 3 | $CH_2(2-ClPh)$ | C=CH | |
| 3-122 | 3 | $CH_2(2,6-F_2Ph)$ | C=CH | |
| 3-123 | 3 | $CH_2Ph$ | c-Pr | |
| 3-124 | 3 | $CH_2(2-MePh)$ | c-Pr | |
| 3-125 | 3 | $CH_2(2,6-Me_2Ph)$ | c-Pr | |
| 3-126 | 3 | $CH_2(2-MeOPh)$ | c-Pr | |
| 3-127 | 3 | $CH_2(2-FPh)$ | c-Pr | |
| 3-128 | 3 | $CH_2(2-ClPh)$ | c-Pr | |
| 3-129 | 3 | $CH_2(2,6-F_2Ph)$ | c-Pr | |
| 3-130 | 3 | $CH_2Ph$ | $CH_2$c-Pr | |
| 3-131 | 3 | $CH_2(2-MePh)$ | $CH_2$c-Pr | |
| 3-132 | 3 | $CH_2(2,6-Me_2Ph)$ | $CH_2$c-Pr | |
| 3-133 | 3 | $CH_2(2-MeOPh)$ | $CH_2$c-Pr | |
| 3-134 | 3 | $CH_2(2-FPh)$ | $CH_2$c-Pr | |
| 3-135 | 3 | $CH_2(2-ClPh)$ | $CH_2$c-Pr | |

[Table 63]

| Table 3-Continued | | | | |
|---|---|---|---|---|
| No. | General formula | $R^1$ | W | Remark |
| 3-136 | 3 | $CH_2(2,6-F_2Ph)$ | $CH_2$c-Pr | |
| 3-137 | 3 | $CH_2(2-FPh)$ | $OCH_2OMe$ | |
| 3-138 | 3’ | $CH_2(2-FPh)$ | $OCH_2OMe$ | |
| 3-139 | 3 | $CH_2(2-ClPh)$ | $OCH_2OMe$ | |
| 3-140 | 3’ | $CH_2(2-ClPh)$ | $OCH_2OMe$ | |
| 3-141 | 3 | $CH_2(2,3-F_2Ph)$ | $OCH_2OMe$ | |
| 3-142 | 3’ | $CH_2(2,3-F_2Ph)$ | $OCH_2OMe$ | |
| 3-143 | 3 | $CH_2(2,4-F_2Ph)$ | $OCH_2OMe$ | |
| 3-144 | 3’ | $CH_2(2,4-F_2Ph)$ | $OCH_2OMe$ | |
| 3-145 | 3 | $CH_2(2,5-F_2Ph)$ | $OCH_2OMe$ | |
| 3-146 | 3’ | $CH_2(2,5-F_2Ph)$ | $OCH_2OMe$ | |
| 3-147 | 3 | $CH_2(2,6-F_2Ph)$ | $OCH_2OMe$ | |
| 3-148 | 3’ | $CH_2(2,6-F_2Ph)$ | $OCH_2OMe$ | |

(continued)

| Table 3-Continued | | | | |
|---|---|---|---|---|
| No. | General formula | $R^1$ | W | Remark |
| 3-149 | 3 | $CH_2(3,5\text{-}F_2Ph)$ | $OCH_2OMe$ | |
| 3-150 | 3' | $CH_2(3,5\text{-}F_2Ph)$ | $OCH_2OMe$ | |
| 3-151 | 3 | $CH_2(2,6\text{-}Cl_2Ph)$ | $OCH_2OMe$ | |
| 3-152 | 3' | $CH_2(2,6\text{-}Cl_2Ph)$ | $OCH_2OMe$ | |
| 3-153 | 3 | $CH_2$(2-F-6-ClPh) | $OCH_2OMe$ | |
| 3-154 | 3 | $CH_2(2\text{-}CF_3Ph)$ | $OCH_2OMe$ | |
| 3-155 | 3' | $CH_2(2\text{-}CF_3Ph)$ | $OCH_2OMe$ | |
| 3-156 | 3 | $CH_2$(2-FPh) | OCH(Me)OEt | |
| 3-157 | 3' | $CH_2$(2-FPh) | OCH(Me)OEt | |
| 3-158 | 3 | $CH_2$(2-ClPh) | OCH(Me)OEt | |
| 3-159 | 3' | $CH_2$(2-ClPh) | OCH(Me)OEt | |
| 3-160 | 3 | $CH_2(2,3\text{-}F_2Ph)$ | OCH(Me)OEt | |
| 3-161 | 3' | $CH_2(2,3\text{-}F_2Ph)$ | OCH(Me)OEt | |
| 3-162 | 3 | $CH_2(2,4\text{-}F_2Ph)$ | OCH(Me)OEt | |
| 3-163 | 3' | $CH_2(2,4\text{-}F_2Ph)$ | OCH(Me)OEt | |
| 3-164 | 3 | $CH_2(2,5\text{-}F_2Ph)$ | OCH(Me)OEt | |
| 3-165 | 3' | $CH_2(2,5\text{-}F_2Ph)$ | OCH(Me)OEt | |
| 3-166 | 3 | $CH_2(2,6\text{-}F_2Ph)$ | OCH(Me)OEt | |
| 3-167 | 3' | $CH_2(2,6\text{-}F_2Ph)$ | OCH(Me)OEt | |
| 3-168 | 3 | $CH_2(3,5\text{-}F_2Ph)$ | OCH(Me)OEt | |
| 3-169 | 3' | $CH_2(3,5\text{-}F_2Ph)$ | OCH(Me)OEt | |
| 3-170 | 3 | $CH_2(2,6\text{-}Cl_2Ph)$ | OCH(Me)OEt | |

[Table 64]

| Table 3-Continued | | | | |
|---|---|---|---|---|
| No. | General formula | $R^1$ | W | Remark |
| 3-171 | 3' | $CH_2(2,6\text{-}Cl_2Ph)$ | OCH(Me)OEt | |
| 3-172 | 3 | $CH_2$(2-F-6-ClPh) | OCH(Me)OEt | |
| 3-173 | 3 | $CH_2(2\text{-}CF_3Ph)$ | OCH(Me)OEt | |
| 3-174 | 3' | $CH_2(2\text{-}CF_3Ph)$ | OCH(Me)OEt | |
| 3-175 | 3 | $CH_2$(2-MePh) | Ph | |
| 3-176 | 3' | $CH_2$(2-MePh) | Ph | |
| 3-177 | 3 | $CH_2$(2-FPh) | Ph | |
| 3-178 | 3' | $CH_2$(2-FPh) | Ph | |
| 3-179 | 3 | $CH_2$(2-ClPh) | Ph | |
| 3-180 | 3' | $CH_2$(2-ClPh) | Ph | |
| 3-181 | 3 | $CH_2(2,3\text{-}F_2Ph)$ | Ph | |
| 3-182 | 3' | $CH_2(2,3\text{-}F_2Ph)$ | Ph | |

(continued)

| Table 3-Continued | | | | |
|---|---|---|---|---|
| No. | General formula | $R^1$ | W | Remark |
| 3-183 | 3 | $CH_2(2,4-F_2Ph)$ | Ph | |
| 3-184 | 3' | $CH_2(2,4-F_2Ph)$ | Ph | |
| 3-185 | 3 | $CH_2(2,5-F_2Ph)$ | Ph | |
| 3-186 | 3' | $CH_2(2,5-F_2Ph)$ | Ph | |
| 3-187 | 3 | $CH_2(2,6-F_2Ph)$ | Ph | |
| 3-188 | 3' | $CH_2(2,6-F_2Ph)$ | Ph | |
| 3-189 | 3 | $CH_2(3,5-F_2Ph)$ | Ph | |
| 3-190 | 3' | $CH_2(3,5-F_2Ph)$ | Ph | |
| 3-191 | 3 | $CH_2(2,6-Cl_2Ph)$ | Ph | |
| 3-192 | 3' | $CH_2(2,6-Cl_2Ph)$ | Ph | |
| 3-193 | 3 | $CH_2(2-F-6-ClPh)$ | Ph | |
| 3-194 | 3 | $CH_2(2-CF_3Ph)$ | Ph | |
| 3-195 | 3' | $CH_2(2-CF_3Ph)$ | Ph | |
| 3-196 | 3 | $CH_2(2-MePh)$ | 2-ClPh | |
| 3-197 | 3' | $CH_2(2-MePh)$ | 2-ClPh | |
| 3-198 | 3 | $CH_2(2-FPh)$ | 2-ClPh | |
| 3-199 | 3' | $CH_2(2-FPh)$ | 2-ClPh | |
| 3-200 | 3 | $CH_2(2-ClPh)$ | 2-ClPh | |
| 3-201 | 3' | $CH_2(2-ClPh)$ | 2-ClPh | |
| 3-202 | 3 | $CH_2(2,3-F_2Ph)$ | 2-ClPh | |
| 3-203 | 3' | $CH_2(2,3-F_2Ph)$ | 2-ClPh | |
| 3-204 | 3 | $CH_2(2,4-F_2Ph)$ | 2-ClPh | |
| 3-205 | 3' | $CH_2(2,4-F_2Ph)$ | 2-ClPh | |

[Table 65]

| Table 3-Continued | | | | |
|---|---|---|---|---|
| No. | General formula | $R^1$ | W | Remark |
| 3-206 | 3 | $CH_2(2,5-F_2Ph)$ | 2-ClPh | |
| 3-207 | 3' | $CH_2(2,5-F_2Ph)$ | 2-ClPh | |
| 3-208 | 3 | $CH_2(2,6-F_2Ph)$ | 2-ClPh | |
| 3-209 | 3' | $CH_2(2,6-F_2Ph)$ | 2-ClPh | |
| 3-210 | 3 | $CH_2(3,5-F_2Ph)$ | 2-ClPh | |
| 3-211 | 3' | $CH_2(3,5-F_2Ph)$ | 2-ClPh | |
| 3-212 | 3 | $CH_2(2,6-Cl_2Ph)$ | 2-ClPh | |
| 3-213 | 3' | $CH_2(2,6-Cl_2Ph)$ | 2-ClPh | |
| 3-214 | 3 | $CH_2(2-F-6-ClPh)$ | 2-ClPh | |
| 3-215 | 3 | $CH_2(2-CF_3Ph)$ | 2-ClPh | |
| 3-216 | 3' | $CH_2(2-CF_3Ph)$ | 2-ClPh | |

(continued)

| Table 3-Continued | | | | |
|---|---|---|---|---|
| No. | General formula | $R^1$ | W | Remark |
| 3-217 | 3 | $CH_2$(2-MePh) | 3-ClPh | |
| 3-218 | 3' | $CH_2$(2-MePh) | 3-ClPh | |
| 3-219 | 3 | $CH_2$(2-FPh) | 3-ClPh | |
| 3-220 | 3' | $CH_2$(2-FPh) | 3-ClPh | |
| 3-221 | 3 | $CH_2$(2-ClPh) | 3-ClPh | |
| 3-222 | 3' | $CH_2$(2-ClPh) | 3-ClPh | |
| 3-223 | 3 | $CH_2$(2,3-$F_2$Ph) | 3-ClPh | |
| 3-224 | 3' | $CH_2$(2,3-$F_2$Ph) | 3-ClPh | |
| 3-225 | 3 | $CH_2$(2,4-$F_2$Ph) | 3-ClPh | |
| 3-226 | 3' | $CH_2$(2,4-$F_2$Ph) | 3-ClPh | |
| 3-227 | 3 | $CH_2$(2,5-$F_2$Ph) | 3-ClPh | |
| 3-228 | 3' | $CH_2$(2,5-$F_2$Ph) | 3-ClPh | |
| 3-229 | 3 | $CH_2$(2,6-$F_2$Ph) | 3-ClPh | |
| 3-230 | 3' | $CH_2$(2,6-$F_2$Ph) | 3-ClPh | |
| 3-231 | 3 | $CH_2$(3,5-$F_2$Ph) | 3-ClPh | |
| 3-232 | 3' | $CH_2$(3,5-$F_2$Ph) | 3-ClPh | |
| 3-233 | 3 | $CH_2$(2,6-$Cl_2$Ph) | 3-ClPh | |
| 3-234 | 3' | $CH_2$(2,6-$Cl_2$Ph) | 3-ClPh | |
| 3-235 | 3 | $CH_2$(2-F-6-ClPh) | 3-ClPh | |
| 3-236 | 3 | $CH_2$(2-$CF_3$Ph) | 3-ClPh | |
| 3-237 | 3' | $CH_2$(2-$CF_3$Ph) | 3-ClPh | |
| 3-238 | 3 | $CH_2$(2-MePh) | 4-ClPh | |
| 3-239 | 3' | $CH_2$(2-MePh) | 4-ClPh | |
| 3-240 | 3 | $CH_2$(2-FPh) | 4-ClPh | |

[Table 66]

| Table 3-Continued | | | | |
|---|---|---|---|---|
| No. | General formula | $R^1$ | W | Remark |
| 3-241 | 3' | $CH_2$(2-FPh) | 4-ClPh | |
| 3-242 | 3 | $CH_2$(2-ClPh) | 4-ClPh | |
| 3-243 | 3' | $CH_2$(2-ClPh) | 4-ClPh | |
| 3-244 | 3 | $CH_2$(2,3-$F_2$Ph) | 4-ClPh | |
| 3-245 | 3' | $CH_2$(2,3-$F_2$Ph) | 4-ClPh | |
| 3-246 | 3 | $CH_2$(2,4-$F_2$Ph) | 4-ClPh | |
| 3-247 | 3' | $CH_2$(2,4-$F_2$Ph) | 4-ClPh | |
| 3-248 | 3 | $CH_2$(2,5-$F_2$Ph) | 4-ClPh | |
| 3-249 | 3' | $CH_2$(2,5-$F_2$Ph) | 4-ClPh | |
| 3-250 | 3 | $CH_2$(2,6-$F_2$Ph) | 4-ClPh | |

(continued)

| Table 3-Continued | | | | |
|---|---|---|---|---|
| No. | General formula | $R^1$ | W | Remark |
| 3-251 | 3' | $CH_2(2,6-F_2Ph)$ | 4-ClPh | |
| 3-252 | 3 | $CH_2(3,5-F_2Ph)$ | 4-ClPh | |
| 3-253 | 3' | $CH_2(3,5-F_2Ph)$ | 4-ClPh | |
| 3-254 | 3 | $CH_2(2,6-Cl_2Ph)$ | 4-ClPh | |
| 3-255 | 3' | $CH_2(2,6-Cl_2Ph)$ | 4-ClPh | |
| 3-256 | 3 | $CH_2$(2-F-6-ClPh) | 4-ClPh | |
| 3-257 | 3 | $CH_2(2-CF_3Ph)$ | 4-ClPh | |
| 3-258 | 3' | $CH_2(2-CF_3Ph)$ | 4-ClPh | |
| 3-259 | 3 | $CH_2$(2-MePh) | 2-Pyridyl | |
| 3-260 | 3' | $CH_2$(2-MePh) | 2-Pyridyl | |
| 3-261 | 3 | $CH_2$(2-FPh) | 2-Pyridyl | |
| 3-262 | 3' | $CH_2$(2-FPh) | 2-Pyridyl | |
| 3-263 | 3 | $CH_2$(2-ClPh) | 2-Pyridyl | |
| 3-264 | 3' | $CH_2$(2-ClPh) | 2-Pyridyl | |
| 3-265 | 3 | $CH_2(2,3-F_2Ph)$ | 2-Pyridyl | |
| 3-266 | 3' | $CH_2(2,3-F_2Ph)$ | 2-Pyridyl | |
| 3-267 | 3 | $CH_2(2,4-F_2Ph)$ | 2-Pyridyl | |
| 3-268 | 3' | $CH_2(2,4-F_2Ph)$ | 2-Pyridyl | |
| 3-269 | 3 | $CH_2(2,5-F_2Ph)$ | 2-Pyridyl | |
| 3-270 | 3' | $CH_2(2,5-F_2Ph)$ | 2-Pyridyl | |
| 3-271 | 3 | $CH_2(2,6-F_2Ph)$ | 2-Pyridyl | |
| 3-272 | 3' | $CH_2(2,6-F_2Ph)$ | 2-Pyridyl | |
| 3-273 | 3 | $CH_2(3,5-F_2Ph)$ | 2-Pyridyl | |
| 3-274 | 3' | $CH_2(3,5-F_2Ph)$ | 2-Pyridyl | |
| 3-275 | 3 | $CH_2(2,6-Cl_2Ph)$ | 2-Pyridyl | |

[Table 67]

| Table 3-Continued | | | | |
|---|---|---|---|---|
| No. | General formula | $R^1$ | W | Remark |
| 3-276 | 3' | $CH_2(2,6-Cl_2Ph)$ | 2-Pyridyl | |
| 3-277 | 3 | $CH_2$(2-F-6-ClPh) | 2-Pyridyl | |
| 3-278 | 3 | $CH_2(2-CF_3Ph)$ | 2-Pyridyl | |
| 3-279 | 3' | $CH_2(2-CF_3Ph)$ | 2-Pyridyl | |
| 3-280 | 3 | $CH_2$(2-MePh) | 3-Pyridyl | |
| 3-281 | 3' | $CH_2$(2-MePh) | 3-Pyridyl | |
| 3-282 | 3 | $CH_2$(2-FPh) | 3-Pyridyl | |
| 3-283 | 3' | $CH_2$(2-FPh) | 3-Pyridyl | |
| 3-284 | 3 | $CH_2$(2-ClPh) | 3-Pyridyl | |

(continued)

| Table 3-Continued | | | | |
|---|---|---|---|---|
| No. | General formula | $R^1$ | W | Remark |
| 3-285 | 3’ | $CH_2$(2-ClPh) | 3-Pyridyl | |
| 3-286 | 3 | $CH_2$(2,3-$F_2$Ph) | 3-Pyridyl | |
| 3-287 | 3’ | $CH_2$(2,3-$F_2$Ph) | 3-Pyridyl | |
| 3-288 | 3 | $CH_2$(2,4-$F_2$Ph) | 3-Pyridyl | |
| 3-289 | 3’ | $CH_2$(2,4-$F_2$Ph) | 3-Pyridyl | |
| 3-290 | 3 | $CH_2$(2,5-$F_2$Ph) | 3-Pyridyl | |
| 3-291 | 3’ | $CH_2$(2,5-$F_2$Ph) | 3-Pyridyl | |
| 3-292 | 3 | $CH_2$(2,6-$F_2$Ph) | 3-Pyridyl | |
| 3-293 | 3’ | $CH_2$(2,6-$F_2$Ph) | 3-Pyridyl | |
| 3-294 | 3 | $CH_2$(3,5-$F_2$Ph) | 3-Pyridyl | |
| 3-295 | 3’ | $CH_2$(3,5-$F_2$Ph) | 3-Pyridyl | |
| 3-296 | 3 | $CH_2$(2,6-$Cl_2$Ph) | 3-Pyridyl | |
| 3-297 | 3’ | $CH_2$(2,6-$Cl_2$Ph) | 3-Pyridyl | |
| 3-298 | 3 | $CH_2$(2-F-6-ClPh) | 3-Pyridyl | |
| 3-299 | 3 | $CH_2$(2-$CF_3$Ph) | 3-Pyridyl | |
| 3-300 | 3’ | $CH_2$(2-$CF_3$Ph) | 3-Pyridyl | |
| 3-301 | 3 | $CH_2$(2-MePh) | 4-Pyridyl | |
| 3-302 | 3’ | $CH_2$(2-MePh) | 4-Pyridyl | |
| 3-303 | 3 | $CH_2$(2-FPh) | 4-Pyridyl | |
| 3-304 | 3’ | $CH_2$(2-FPh) | 4-Pyridyl | |
| 3-305 | 3 | $CH_2$(2-ClPh) | 4-Pyridyl | |
| 3-306 | 3’ | $CH_2$(2-ClPh) | 4-Pyridyl | |
| 3-307 | 3 | $CH_2$(2,3-$F_2$Ph) | 4-Pyridyl | |
| 3-308 | 3’ | $CH_2$(2,3-$F_2$Ph) | 4-Pyridyl | |
| 3-309 | 3 | $CH_2$(2,4-$F_2$Ph) | 4-Pyridyl | |
| 3-310 | 3’ | $CH_2$(2,4-$F_2$Ph) | 4-Pyridyl | |

[Table 68]

| Table 3-Continued | | | | |
|---|---|---|---|---|
| No. | General formula | $R^1$ | W | Remark |
| 3-311 | 3 | $CH_2$(2,5-$F_2$Ph) | 4-Pyridyl | |
| 3-312 | 3’ | $CH_2$(2,5-$F_2$Ph) | 4-Pyridyl | |
| 3-313 | 3 | $CH_2$(2,6-$F_2$Ph) | 4-Pyridyl | |
| 3-314 | 3’ | $CH_2$(2,6-$F_2$Ph) | 4-Pyridyl | |
| 3-315 | 3 | $CH_2$(3,5-$F_2$Ph) | 4-Pyridyl | |
| 3-316 | 3’ | $CH_2$(3,5-$F_2$Ph) | 4-Pyridyl | |
| 3-317 | 3 | $CH_2$(2,6-$Cl_2$Ph) | 4-Pyridyl | |
| 3-318 | 3’ | $CH_2$(2,6-$Cl_2$Ph) | 4-Pyridyl | |

(continued)

| Table 3-Continued | | | | |
|---|---|---|---|---|
| No. | General formula | $R^1$ | W | Remark |
| 3-319 | 3 | $CH_2$(2-F-6-ClPh) | 4-Pyridyl | |
| 3-320 | 3 | $CH_2$(2-$CF_3$Ph) | 4-Pyridyl | |
| 3-321 | 3' | $CH_2$(2-$CF_3$Ph) | 4-Pyridyl | |
| 3-322 | 3 | $CH_2$(2-MePh) | Bn | |
| 3-323 | 3' | $CH_2$(2-MePh) | Bn | |
| 3-324 | 3 | $CH_2$(2-FPh) | Bn | |
| 3-325 | 3' | $CH_2$(2-FPh) | Bn | |
| 3-326 | 3 | $CH_2$(2-ClPh) | Bn | |
| 3-327 | 3' | $CH_2$(2-ClPh) | Bn | |
| 3-328 | 3 | $CH_2$(2,3-$F_2$Ph) | Bn | |
| 3-329 | 3' | $CH_2$(2,3-$F_2$Ph) | Bn | |
| 3-330 | 3 | $CH_2$(2,4-$F_2$Ph) | Bn | |
| 3-331 | 3' | $CH_2$(2,4-$F_2$Ph) | Bn | |
| 3-332 | 3 | $CH_2$(2,5-$F_2$Ph) | Bn | |
| 3-333 | 3' | $CH_2$(2,5-$F_2$Ph) | Bn | |
| 3-334 | 3 | $CH_2$(2,6-$F_2$Ph) | Bn | |
| 3-335 | 3' | $CH_2$(2,6-$F_2$Ph) | Bn | |
| 3-336 | 3 | $CH_2$(3,5-$F_2$Ph) | Bn | |
| 3-337 | 3' | $CH_2$(3,5-$F_2$Ph) | Bn | |
| 3-338 | 3 | $CH_2$(2,6-$Cl_2$Ph) | Bn | |
| 3-339 | 3' | $CH_2$(2,6-$Cl_2$Ph) | Bn | |
| 3-340 | 3 | $CH_2$(2-F-6-ClPh) | Bn | |
| 3-341 | 3 | $CH_2$(2-$CF_3$Ph) | Bn | |
| 3-342 | 3' | $CH_2$(2-$CF_3$Ph) | Bn | |
| 3-343 | 3 | $CH_2$(2-MePh) | 2-ClBn | |
| 3-344 | 3' | $CH_2$(2-MePh) | 2-ClBn | |
| 3-345 | 3 | $CH_2$(2-FPh) | 2-ClBn | |

[Table 69]

| Table 3-Continued | | | | |
|---|---|---|---|---|
| No. | General formula | $R^1$ | W | Remark |
| 3-346 | 3' | $CH_2$(2-FPh) | 2-ClBn | |
| 3-347 | 3 | $CH_2$(2-ClPh) | 2-ClBn | |
| 3-348 | 3' | $CH_2$(2-ClPh) | 2-ClBn | |
| 3-349 | 3 | $CH_2$(2,3-$F_2$Ph) | 2-ClBn | |
| 3-350 | 3' | $CH_2$(2,3-$F_2$Ph) | 2-ClBn | |
| 3-351 | 3 | $CH_2$(2,4-$F_2$Ph) | 2-ClBn | |
| 3-352 | 3' | $CH_2$(2,4-$F_2$Ph) | 2-ClBn | |

(continued)

| Table 3-Continued | | | | |
|---|---|---|---|---|
| No. | General formula | $R^1$ | W | Remark |
| 3-353 | 3 | $CH_2$(2,5-$F_2$Ph) | 2-ClBn | |
| 3-354 | 3' | $CH_2$(2,5-$F_2$Ph) | 2-ClBn | |
| 3-355 | 3 | $CH_2$(2,6-$F_2$Ph) | 2-ClBn | |
| 3-356 | 3' | $CH_2$(2,6-$F_2$Ph) | 2-ClBn | |
| 3-357 | 3 | $CH_2$(3,5-$F_2$Ph) | 2-ClBn | |
| 3-358 | 3' | $CH_2$(3,5-$F_2$Ph) | 2-ClBn | |
| 3-359 | 3 | $CH_2$(2,6-$Cl_2$Ph) | 2-ClBn | |
| 3-360 | 3' | $CH_2$(2,6-$Cl_2$Ph) | 2-ClBn | |
| 3-361 | 3 | $CH_2$(2-F-6-ClPh) | 2-ClBn | |
| 3-362 | 3 | $CH_2$(2-$CF_3$Ph) | 2-ClBn | |
| 3-363 | 3' | $CH_2$(2-$CF_3$Ph) | 2-ClBn | |
| 3-364 | 3 | $CH_2$(2-MePh) | 3-ClBn | |
| 3-365 | 3' | $CH_2$(2-MePh) | 3-ClBn | |
| 3-366 | 3 | $CH_2$(2-FPh) | 3-ClBn | |
| 3-367 | 3' | $CH_2$(2-FPh) | 3-ClBn | |
| 3-368 | 3 | $CH_2$(2-ClPh) | 3-ClBn | |
| 3-369 | 3' | $CH_2$(2-ClPh) | 3-ClBn | |
| 3-370 | 3 | $CH_2$(2,3-$F_2$Ph) | 3-ClBn | |
| 3-371 | 3' | $CH_2$(2,3-$F_2$Ph) | 3-ClBn | |
| 3-372 | 3 | $CH_2$(2,4-$F_2$Ph) | 3-ClBn | |
| 3-373 | 3' | $CH_2$(2,4-$F_2$Ph) | 3-ClBn | |
| 3-374 | 3 | $CH_2$(2,5-$F_2$Ph) | 3-ClBn | |
| 3-375 | 3' | $CH_2$(2,5-$F_2$Ph) | 3-ClBn | |
| 3-376 | 3 | $CH_2$(2,6-$F_2$Ph) | 3-ClBn | |
| 3-377 | 3' | $CH_2$(2,6-$F_2$Ph) | 3-ClBn | |
| 3-378 | 3 | $CH_2$(3,5-$F_2$Ph) | 3-ClBn | |
| 3-379 | 3' | $CH_2$(3,5-$F_2$Ph) | 3-ClBn | |
| 3-380 | 3 | $CH_2$(2,6-$Cl_2$Ph) | 3-ClBn | |

[Table 70]

| Table 3-Continued | | | | |
|---|---|---|---|---|
| No. | General formula | $R^1$ | W | Remark |
| 3-381 | 3' | $CH_2$(2,6-$Cl_2$Ph) | 3-ClBn | |
| 3-382 | 3 | $CH_2$(2-F-6-ClPh) | 3-ClBn | |
| 3-383 | 3 | $CH_2$(2-$CF_3$Ph) | 3-ClBn | |
| 3-384 | 3' | $CH_2$(2-$CF_3$Ph) | 3-ClBn | |
| 3-385 | 3 | $CH_2$(2-MePh) | 4-ClBn | |
| 3-386 | 3' | $CH_2$(2-MePh) | 4-ClBn | |

(continued)

| Table 3-Continued | | | | |
|---|---|---|---|---|
| No. | General formula | $R^1$ | W | Remark |
| 3-387 | 3 | $CH_2$(2-FPh) | 4-ClBn | |
| 3-388 | 3' | $CH_2$(2-FPh) | 4-ClBn | |
| 3-389 | 3 | $CH_2$(2-ClPh) | 4-ClBn | |
| 3-390 | 3' | $CH_2$(2-ClPh) | 4-ClBn | |
| 3-391 | 3 | $CH_2$(2,3-$F_2$Ph) | 4-ClBn | |
| 3-392 | 3' | $CH_2$(2,3-$F_2$Ph) | 4-ClBn | |
| 3-393 | 3 | $CH_2$(2,4-$F_2$Ph) | 4-ClBn | |
| 3-394 | 3' | $CH_2$(2,4-$F_2$Ph) | 4-ClBn | |
| 3-395 | 3 | $CH_2$(2,5-$F_2$Ph) | 4-ClBn | |
| 3-396 | 3' | $CH_2$(2,5-$F_2$Ph) | 4-ClBn | |
| 3-397 | 3 | $CH_2$(2,6-$F_2$Ph) | 4-ClBn | |
| 3-398 | 3' | $CH_2$(2,6-$F_2$Ph) | 4-ClBn | |
| 3-399 | 3 | $CH_2$(3,5-$F_2$Ph) | 4-ClBn | |
| 3-400 | 3' | $CH_2$(3,5-$F_2$Ph) | 4-ClBn | |
| 3-401 | 3 | $CH_2$(2,6-$Cl_2$Ph) | 4-ClBn | |
| 3-402 | 3' | $CH_2$(2,6-$Cl_2$Ph) | 4-ClBn | |
| 3-403 | 3 | $CH_2$(2-F-6-ClPh) | 4-ClBn | |
| 3-404 | 3 | $CH_2$(2-$CF_3$Ph) | 4-ClBn | |
| 3-405 | 3' | $CH_2$(2-$CF_3$Ph) | 4-ClBn | |
| 3-406 | 3 | $CH_2$(2-MePh) | $CH_2$(2-Pyridyl) | |
| 3-407 | 3' | $CH_2$(2-MePh) | $CH_2$(2-Pyridyl) | |
| 3-408 | 3 | $CH_2$(2-FPh) | $CH_2$(2-Pyridyl) | |
| 3-409 | 3' | $CH_2$(2-FPh) | $CH_2$(2-Pyridyl) | |
| 3-410 | 3 | $CH_2$(2-ClPh) | $CH_2$(2-Pyridyl) | |
| 3-411 | 3' | $CH_2$(2-ClPh) | $CH_2$(2-Pyridyl) | |
| 3-412 | 3 | $CH_2$(2,3-$F_2$Ph) | $CH_2$(2-Pyridyl) | |
| 3-413 | 3' | $CH_2$(2,3-$F_2$Ph) | $CH_2$(2-Pyridyl) | |
| 3-414 | 3 | $CH_2$(2,4-$F_2$Ph) | $CH_2$(2-Pyridyl) | |
| 3-415 | 3' | $CH_2$(2,4-$F_2$Ph) | $CH_2$(2-Pyridyl) | |

[Table 71]

| Table 3-Continued | | | | |
|---|---|---|---|---|
| No. | General formula | $R^1$ | W | Remark |
| 3-416 | 3 | $CH_2$(2,5-$F_2$Ph) | $CH_2$(2-Pyridyl) | |
| 3-417 | 3' | $CH_2$(2,5-$F_2$Ph) | $CH_2$(2-Pyridyl) | |
| 3-418 | 3 | $CH_2$(2,6-$F_2$Ph) | $CH_2$(2-Pyridyl) | |
| 3-419 | 3' | $CH_2$(2,6-$F_2$Ph) | $CH_2$(2-Pyridyl) | |
| 3-420 | 3 | $CH_2$(3,5-$F_2$Ph) | $CH_2$(2-Pyridyl) | |

(continued)

| Table 3-Continued | | | | |
|---|---|---|---|---|
| No. | General formula | $R^1$ | W | Remark |
| 3-421 | 3' | $CH_2$(3,5-$F_2$Ph) | $CH_2$(2-Pyridyl) | |
| 3-422 | 3 | $CH_2$(2,6-$Cl_2$Ph) | $CH_2$(2-Pyridyl) | |
| 3-423 | 3' | $CH_2$(2,6-$Cl_2$Ph) | $CH_2$(2-Pyridyl) | |
| 3-424 | 3 | $CH_2$(2-F-6-ClPh) | $CH_2$(2-Pyridyl) | |
| 3-425 | 3 | $CH_2$(2-$CF_3$Ph) | $CH_2$(2-Pyridyl) | |
| 3-426 | 3' | $CH_2$(2-$CF_3$Ph) | $CH_2$(2-Pyridyl) | |
| 3-427 | 3 | $CH_2$(2-MePh) | $CH_2$(3-Pyridyl) | |
| 3-428 | 3' | $CH_2$(2-MePh) | $CH_2$(3-Pyridyl) | |
| 3-429 | 3 | $CH_2$(2-FPh) | $CH_2$(3-Pyridyl) | |
| 3-430 | 3' | $CH_2$(2-FPh) | $CH_2$(3-Pyridyl) | |
| 3-431 | 3 | $CH_2$(2-ClPh) | $CH_2$(3-Pyridyl) | |
| 3-432 | 3' | $CH_2$(2-ClPh) | $CH_2$(3-Pyridyl) | |
| 3-433 | 3 | $CH_2$(2,3-$F_2$Ph) | $CH_2$(3-Pyridyl) | |
| 3-434 | 3' | $CH_2$(2,3-$F_2$Ph) | $CH_2$(3-Pyridyl) | |
| 3-435 | 3 | $CH_2$(2,4-$F_2$Ph) | $CH_2$(3-Pyridyl) | |
| 3-436 | 3' | $CH_2$(2,4-$F_2$Ph) | $CH_2$(3-Pyridyl) | |
| 3-437 | 3 | $CH_2$(2,5-$F_2$Ph) | $CH_2$(3-Pyridyl) | |
| 3-438 | 3' | $CH_2$(2,5-$F_2$Ph) | $CH_2$(3-Pyridyl) | |
| 3-439 | 3 | $CH_2$(2,6-$F_2$Ph) | $CH_2$(3-Pyridyl) | |
| 3-440 | 3' | $CH_2$(2,6-$F_2$Ph) | $CH_2$(3-Pyridyl) | |
| 3-441 | 3 | $CH_2$(3,5-$F_2$Ph) | $CH_2$(3-Pyridyl) | |
| 3-442 | 3' | $CH_2$(3,5-$F_2$Ph) | $CH_2$(3-Pyridyl) | |
| 3-443 | 3 | $CH_2$(2,6-$Cl_2$Ph) | $CH_2$(3-Pyridyl) | |
| 3-444 | 3' | $CH_2$(2,6-$Cl_2$Ph) | $CH_2$(3-Pyridyl) | |
| 3-445 | 3 | $CH_2$(2-F-6-ClPh) | $CH_2$(3-Pyridyl) | |
| 3-446 | 3 | $CH_2$(2-$CF_3$Ph) | $CH_2$(3-Pyridyl) | |
| 3-447 | 3' | $CH_2$(2-$CF_3$Ph) | $CH_2$(3-Pyridyl) | |
| 3-448 | 3 | $CH_2$(2-MePh) | $CH_2$(4-Pyridyl) | |
| 3-449 | 3' | $CH_2$(2-MePh) | $CH_2$(4-Pyridyl) | |
| 3-450 | 3 | $CH_2$(2-FPh) | $CH_2$(4-Pyridyl) | |

[Table 72]

| Table 3-Continued | | | | |
|---|---|---|---|---|
| No. | General formula | $R^1$ | W | Remark |
| 3-451 | 3' | $CH_2$(2-FPh) | $CH_2$(4-Pyridyl) | |
| 3-452 | 3 | $CH_2$(2-ClPh) | $CH_2$(4-Pyridyl) | |
| 3-453 | 3' | $CH_2$(2-ClPh) | $CH_2$(4-Pyridyl) | |
| 3-454 | 3 | $CH_2$(2,3-$F_2$Ph) | $CH_2$(4-Pyridyl) | |

(continued)

| Table 3-Continued | | | | |
|---|---|---|---|---|
| No. | General formula | $R^1$ | W | Remark |
| 3-455 | 3' | $CH_2(2,3-F_2Ph)$ | $CH_2$(4-Pyridyl) | |
| 3-456 | 3 | $CH_2(2,4-F_2Ph)$ | $CH_2$(4-Pyridyl) | |
| 3-457 | 3' | $CH_2(2,4-F_2Ph)$ | $CH_2$(4-Pyridyl) | |
| 3-458 | 3 | $CH_2(2,5-F_2Ph)$ | $CH_2$(4-Pyridyl) | |
| 3-459 | 3' | $CH_2(2,5-F_2Ph)$ | $CH_2$(4-Pyridyl) | |
| 3-460 | 3 | $CH_2(2,6-F_2Ph)$ | $CH_2$(4-Pyridyl) | |
| 3-461 | 3' | $CH_2(2,6-F_2Ph)$ | $CH_2$(4-Pyridyl) | |
| 3-462 | 3 | $CH_2(3,5-F_2Ph)$ | $CH_2$(4-Pyridyl) | |
| 3-463 | 3' | $CH_2(3,5-F_2Ph)$ | $CH_2$(4-Pyridyl) | |
| 3-464 | 3 | $CH_2(2,6-Cl_2Ph)$ | $CH_2$(4-Pyridyl) | |
| 3-465 | 3' | $CH_2(2,6-Cl_2Ph)$ | $CH_2$(4-Pyridyl) | |
| 3-466 | 3 | $CH_2$(2-F-6-ClPh) | $CH_2$(4-Pyridyl) | |
| 3-467 | 3 | $CH_2(2-CF_3Ph)$ | $CH_2$(4-Pyridyl) | |
| 3-468 | 3' | $CH_2(2-CF_3Ph)$ | $CH_2$(4-Pyridyl) | |
| 3-469 | 3 | $CH_2Ph$ | $CH_2OPh$ | |
| 3-470 | 3 | $CH_2$(2-MePh) | $CH_2OPh$ | |
| 3-471 | 3 | $CH_2(2,6-Me_2Ph)$ | $CH_2OPh$ | |
| 3-472 | 3 | $CH_2$(2-MeOPh) | $CH_2OPh$ | |
| 3-473 | 3 | $CH_2$(2-FPh) | $CH_2OPh$ | |
| 3-474 | 3 | $CH_2$(2-ClPh) | $CH_2OPh$ | |
| 3-475 | 3 | $CH_2(2,6-F_2Ph)$ | $CH_2OPh$ | |
| 3-476 | 3 | $CH_2Ph$ | $CH_2OBn$ | |
| 3-477 | 3 | $CH_2$(2-MePh) | $CH_2OBn$ | |
| 3-478 | 3 | $CH_2(2,6-Me_2Ph)$ | $CH_2OBn$ | |
| 3-479 | 3 | $CH_2$(2-MeOPh) | $CH_2OBn$ | |
| 3-480 | 3 | $CH_2$(2-FPh) | $CH_2OBn$ | |
| 3-481 | 3 | $CH_2$(2-ClPh) | $CH_2OBn$ | |
| 3-482 | 3 | $CH_2(2,6-F_2Ph)$ | $CH_2OBn$ | |
| 3-483 | 3 | $CH_2$(2-MePh) | OPh | |
| 3-484 | 3' | $CH_2$(2-MePh) | OPh | |
| 3-485 | 3 | $CH_2$(2-FPh) | OPh | |

[Table 73]

| Table 3-Continued | | | | |
|---|---|---|---|---|
| No. | General formula | $R^1$ | W | Remark |
| 3-486 | 3' | $CH_2$(2-FPh) | OPh | |
| 3-487 | 3 | $CH_2$(2-ClPh) | OPh | |
| 3-488 | 3' | $CH_2$(2-ClPh) | OPh | |

(continued)

| Table 3-Continued | | | | |
|---|---|---|---|---|
| No. | General formula | $R^1$ | W | Remark |
| 3-489 | 3 | $CH_2(2,3-F_2Ph)$ | OPh | |
| 3-490 | 3' | $CH_2(2,3-F_2Ph)$ | OPh | |
| 3-491 | 3 | $CH_2(2,4-F_2Ph)$ | OPh | |
| 3-492 | 3' | $CH_2(2,4-F_2Ph)$ | OPh | |
| 3-493 | 3 | $CH_2(2,5-F_2Ph)$ | OPh | |
| 3-494 | 3' | $CH_2(2,5-F_2Ph)$ | OPh | |
| 3-495 | 3 | $CH_2(2,6-F_2Ph)$ | OPh | |
| 3-496 | 3' | $CH_2(2,6-F_2Ph)$ | OPh | |
| 3-497 | 3 | $CH_2(3,5-F_2Ph)$ | OPh | |
| 3-498 | 3' | $CH_2(3,5-F_2Ph)$ | OPh | |
| 3-499 | 3 | $CH_2(2,6-Cl_2Ph)$ | OPh | |
| 3-500 | 3' | $CH_2(2,6-Cl_2Ph)$ | OPh | |
| 3-501 | 3 | $CH_2(2-F-6-ClPh)$ | OPh | |
| 3-502 | 3 | $CH_2(2-CF_3Ph)$ | OPh | |
| 3-503 | 3' | $CH_2(2-CF_3Ph)$ | OPh | |
| 3-504 | 3 | $CH_2(2-MePh)$ | O(2-ClPh) | |
| 3-505 | 3' | $CH_2(2-MePh)$ | O(2-ClPh) | |
| 3-506 | 3 | $CH_2(2-FPh)$ | O(2-ClPh) | |
| 3-507 | 3' | $CH_2(2-FPh)$ | O(2-ClPh) | |
| 3-508 | 3 | $CH_2(2-ClPh)$ | O(2-ClPh) | |
| 3-509 | 3' | $CH_2(2-ClPh)$ | O(2-ClPh) | |
| 3-510 | 3 | $CH_2(2,3-F_2Ph)$ | O(2-ClPh) | |
| 3-511 | 3' | $CH_2(2,3-F_2Ph)$ | O(2-ClPh) | |
| 3-512 | 3 | $CH_2(2,4-F_2Ph)$ | O(2-ClPh) | |
| 3-513 | 3' | $CH_2(2,4-F_2Ph)$ | O(2-ClPh) | |
| 3-514 | 3 | $CH_2(2,5-F_2Ph)$ | O(2-ClPh) | |
| 3-515 | 3' | $CH_2(2,5-F_2Ph)$ | O(2-ClPh) | |
| 3-516 | 3 | $CH_2(2,6-F_2Ph)$ | O(2-ClPh) | |
| 3-517 | 3' | $CH_2(2,6-F_2Ph)$ | O(2-ClPh) | |
| 3-518 | 3 | $CH_2(3,5-F_2Ph)$ | O(2-ClPh) | |
| 3-519 | 3' | $CH_2(3,5-F_2Ph)$ | O(2-ClPh) | |
| 3-520 | 3 | $CH_2(2,6-Cl_2Ph)$ | O(2-ClPh) | |

[Table 74]

| Table 3-Continued | | | | |
|---|---|---|---|---|
| No. | General formula | $R^1$ | W | Remark |
| 3-521 | 3' | $CH_2(2,6-Cl_2Ph)$ | O(2-ClPh) | |
| 3-522 | 3 | $CH_2(2-F-6-ClPh)$ | O(2-ClPh) | |

(continued)

| Table 3-Continued | | | | |
|---|---|---|---|---|
| No. | General formula | $R^1$ | W | Remark |
| 3-523 | 3 | $CH_2(2-CF_3Ph)$ | O(2-ClPh) | |
| 3-524 | 3' | $CH_2(2-CF_3Ph)$ | O(2-ClPh) | |
| 3-525 | 3 | $CH_2(2-MePh)$ | O(3-ClPh) | |
| 3-526 | 3' | $CH_2(2-MePh)$ | O(3-ClPh) | |
| 3-527 | 3 | $CH_2(2-FPh)$ | O(3-ClPh) | |
| 3-528 | 3' | $CH_2(2-FPh)$ | O(3-ClPh) | |
| 3-529 | 3 | $CH_2(2-ClPh)$ | O(3-ClPh) | |
| 3-530 | 3' | $CH_2(2-ClPh)$ | O(3-ClPh) | |
| 3-531 | 3 | $CH_2(2,3-F_2Ph)$ | O(3-ClPh) | |
| 3-532 | 3' | $CH_2(2,3-F_2Ph)$ | O(3-ClPh) | |
| 3-533 | 3 | $CH_2(2,4-F_2Ph)$ | O(3-ClPh) | |
| 3-534 | 3' | $CH_2(2,4-F_2Ph)$ | O(3-ClPh) | |
| 3-535 | 3 | $CH_2(2,5-F_2Ph)$ | O(3-ClPh) | |
| 3-536 | 3' | $CH_2(2,5-F_2Ph)$ | O(3-ClPh) | |
| 3-537 | 3 | $CH_2(2,6-F_2Ph)$ | O(3-ClPh) | |
| 3-538 | 3' | $CH_2(2,6-F_2Ph)$ | O(3-ClPh) | |
| 3-539 | 3 | $CH_2(3,5-F_2Ph)$ | O(3-ClPh) | |
| 3-540 | 3' | $CH_2(3,5-F_2Ph)$ | O(3-ClPh) | |
| 3-541 | 3 | $CH_2(2,6-Cl_2Ph)$ | O(3-ClPh) | |
| 3-542 | 3' | $CH_2(2,6-Cl_2Ph)$ | O(3-ClPh) | |
| 3-543 | 3 | $CH_2(2-F-6-ClPh)$ | O(3-ClPh) | |
| 3-544 | 3 | $CH_2(2-CF_3Ph)$ | O(3-ClPh) | |
| 3-545 | 3' | $CH_2(2-CF_3Ph)$ | O(3-ClPh) | |
| 3-546 | 3 | $CH_2(2-MePh)$ | O(4-ClPh) | |
| 3-547 | 3' | $CH_2(2-MePh)$ | O(4-ClPh) | |
| 3-548 | 3 | $CH_2(2-FPh)$ | O(4-ClPh) | |
| 3-549 | 3' | $CH_2(2-FPh)$ | O(4-ClPh) | |
| 3-550 | 3 | $CH_2(2-ClPh)$ | O(4-ClPh) | |
| 3-551 | 3' | $CH_2(2-ClPh)$ | O(4-ClPh) | |
| 3-552 | 3 | $CH_2(2,3-F_2Ph)$ | O(4-ClPh) | |
| 3-553 | 3' | $CH_2(2,3-F_2Ph)$ | O(4-ClPh) | |
| 3-554 | 3 | $CH_2(2,4-F_2Ph)$ | O(4-ClPh) | |
| 3-555 | 3' | $CH_2(2,4-F_2Ph)$ | O(4-ClPh) | |

[Table 75]

| Table 3-Continued | | | | |
|---|---|---|---|---|
| No. | General formula | $R^1$ | W | Remark |
| 3-556 | 3 | $CH_2(2,5-F_2Ph)$ | O(4-ClPh) | |

(continued)

| Table 3-Continued | | | | |
|---|---|---|---|---|
| No. | General formula | $R^1$ | W | Remark |
| 3-557 | 3' | $CH_2$(2,5-$F_2$Ph) | O(4-ClPh) | |
| 3-558 | 3 | $CH_2$(2,6-$F_2$Ph) | O(4-ClPh) | |
| 3-559 | 3' | $CH_2$(2,6-$F_2$Ph) | O(4-ClPh) | |
| 3-560 | 3 | $CH_2$(3,5-$F_2$Ph) | O(4-ClPh) | |
| 3-561 | 3' | $CH_2$(3,5-$F_2$Ph) | O(4-ClPh) | |
| 3-562 | 3 | $CH_2$(2,6-$Cl_2$Ph) | O(4-ClPh) | |
| 3-563 | 3' | $CH_2$(2,6-$Cl_2$Ph) | O(4-ClPh) | |
| 3-564 | 3 | $CH_2$(2-F-6-ClPh) | O(4-ClPh) | |
| 3-565 | 3 | $CH_2$(2-$CF_3$Ph) | O(4-ClPh) | |
| 3-566 | 3' | $CH_2$(2-$CF_3$Ph) | O(4-ClPh) | |
| 3-567 | 3 | $CH_2$(2-MePh) | O(2-Pyridyl) | |
| 3-568 | 3' | $CH_2$(2-MePh) | O(2-Pyridyl) | |
| 3-569 | 3 | $CH_2$(2-FPh) | O(2-Pyridyl) | |
| 3-570 | 3' | $CH_2$(2-FPh) | O(2-Pyridyl) | |
| 3-571 | 3 | $CH_2$(2-ClPh) | O(2-Pyridyl) | |
| 3-572 | 3' | $CH_2$(2-ClPh) | O(2-Pyridyl) | |
| 3-573 | 3 | $CH_2$(2,3-$F_2$Ph) | O(2-Pyridyl) | |
| 3-574 | 3' | $CH_2$(2,3-$F_2$Ph) | O(2-Pyridyl) | |
| 3-575 | 3 | $CH_2$(2,4-$F_2$Ph) | O(2-Pyridyl) | |
| 3-576 | 3' | $CH_2$(2,4-$F_2$Ph) | O(2-Pyridyl) | |
| 3-577 | 3 | $CH_2$(2,5-$F_2$Ph) | O(2-Pyridyl) | |
| 3-578 | 3' | $CH_2$(2,5-$F_2$Ph) | O(2-Pyridyl) | |
| 3-579 | 3 | $CH_2$(2,6-$F_2$Ph) | O(2-Pyridyl) | |
| 3-580 | 3' | $CH_2$(2,6-$F_2$Ph) | O(2-Pyridyl) | |
| 3-581 | 3 | $CH_2$(3,5-$F_2$Ph) | O(2-Pyridyl) | |
| 3-582 | 3' | $CH_2$(3,5-$F_2$Ph) | O(2-Pyridyl) | |
| 3-583 | 3 | $CH_2$(2,6-$Cl_2$Ph) | O(2-Pyridyl) | |
| 3-584 | 3' | $CH_2$(2,6-$Cl_2$Ph) | O(2-Pyridyl) | |
| 3-585 | 3 | $CH_2$(2-F-6-ClPh) | O(2-Pyridyl) | |
| 3-586 | 3 | $CH_2$(2-$CF_3$Ph) | O(2-Pyridyl) | |
| 3-587 | 3' | $CH_2$(2-$CF_3$Ph) | O(2-Pyridyl) | |
| 3-588 | 3 | $CH_2$(2-MePh) | O(3-Pyridyl) | |
| 3-589 | 3' | $CH_2$(2-MePh) | O(3-Pyridyl) | |
| 3-590 | 3 | $CH_2$(2-FPh) | O(3-Pyridyl) | |

[Table 76]

| Table 3-Continued | | | | |
|---|---|---|---|---|
| No. | General formula | $R^1$ | W | Remark |
| 3-591 | 3’ | $CH_2$(2-FPh) | O(3-Pyridyl) | |
| 3-592 | 3 | $CH_2$(2-ClPh) | O(3-Pyridyl) | |
| 3-593 | 3’ | $CH_2$(2-ClPh) | O(3-Pyridyl) | |
| 3-594 | 3 | $CH_2$(2,3-$F_2$Ph) | O(3-Pyridyl) | |
| 3-595 | 3’ | $CH_2$(2,3-$F_2$Ph) | O(3-Pyridyl) | |
| 3-596 | 3 | $CH_2$(2,4-$F_2$Ph) | O(3-Pyridyl) | |
| 3-597 | 3’ | $CH_2$(2,4-$F_2$Ph) | O(3-Pyridyl) | |
| 3-598 | 3 | $CH_2$(2,5-$F_2$Ph) | O(3-Pyridyl) | |
| 3-599 | 3’ | $CH_2$(2,5-$F_2$Ph) | O(3-Pyridyl) | |
| 3-600 | 3 | $CH_2$(2,6-$F_2$Ph) | O(3-Pyridyl) | |
| 3-601 | 3’ | $CH_2$(2,6-$F_2$Ph) | O(3-Pyridyl) | |
| 3-602 | 3 | $CH_2$(3,5-$F_2$Ph) | O(3-Pyridyl) | |
| 3-603 | 3’ | $CH_2$(3,5-$F_2$Ph) | O(3-Pyridyl) | |
| 3-604 | 3 | $CH_2$(2,6-$Cl_2$Ph) | O(3-Pyridyl) | |
| 3-605 | 3’ | $CH_2$(2,6-$Cl_2$Ph) | O(3-Pyridyl) | |
| 3-606 | 3 | $CH_2$(2-F-6-ClPh) | O(3-Pyridyl) | |
| 3-607 | 3 | $CH_2$(2-$CF_3$Ph) | O(3-Pyridyl) | |
| 3-608 | 3’ | $CH_2$(2-$CF_3$Ph) | O(3-Pyridyl) | |
| 3-609 | 3 | $CH_2$(2-MePh) | O(4-Pyridyl) | |
| 3-610 | 3’ | $CH_2$(2-MePh) | O(4-Pyridyl) | |
| 3-611 | 3 | $CH_2$(2-FPh) | O(4-Pyridyl) | |
| 3-612 | 3’ | $CH_2$(2-FPh) | O(4-Pyridyl) | |
| 3-613 | 3 | $CH_2$(2-ClPh) | O(4-Pyridyl) | |
| 3-614 | 3’ | $CH_2$(2-ClPh) | O(4-Pyridyl) | |
| 3-615 | 3 | $CH_2$(2,3-$F_2$Ph) | O(4-Pyridyl) | |
| 3-616 | 3’ | $CH_2$(2,3-$F_2$Ph) | O(4-Pyridyl) | |
| 3-617 | 3 | $CH_2$(2,4-$F_2$Ph) | O(4-Pyridyl) | |
| 3-618 | 3’ | $CH_2$(2,4-$F_2$Ph) | O(4-Pyridyl) | |
| 3-619 | 3 | $CH_2$(2,5-$F_2$Ph) | O(4-Pyridyl) | |
| 3-620 | 3’ | $CH_2$(2,5-$F_2$Ph) | O(4-Pyridyl) | |
| 3-621 | 3 | $CH_2$(2,6-$F_2$Ph) | O(4-Pyridyl) | |
| 3-622 | 3’ | $CH_2$(2,6-$F_2$Ph) | O(4-Pyridyl) | |
| 3-623 | 3 | $CH_2$(3,5-$F_2$Ph) | O(4-Pyridyl) | |
| 3-624 | 3’ | $CH_2$(3,5-$F_2$Ph) | O(4-Pyridyl) | |
| 3-625 | 3 | $CH_2$(2,6-$Cl_2$Ph) | O(4-Pyridyl) | |

[Table 77]

| Table 3-Continued | | | | |
|---|---|---|---|---|
| No. | General formula | $R^1$ | W | Remark |
| 3-626 | 3' | $CH_2$(2,6-$Cl_2$Ph) | O(4-Pyridyl) | |
| 3-627 | 3 | $CH_2$(2-F-6-ClPh) | O(4-Pyridyl) | |
| 3-628 | 3 | $CH_2$(2-$CF_3$Ph) | O(4-Pyridyl) | |
| 3-629 | 3' | $CH_2$(2-$CF_3$Ph) | O(4-Pyridyl) | |
| 3-630 | 3 | $CH_2$Ph | OBn | |
| 3-631 | 3' | $CH_2$Ph | OBn | |
| 3-632 | 3 | $CH_2$(2-MePh) | OBn | |
| 3-633 | 3' | $CH_2$(2-MePh) | OBn | |
| 3-634 | 3 | $CH_2$(2,6-$Me_2$Ph) | OBn | |
| 3-635 | 3' | $CH_2$(2,6-$Me_2$Ph) | OBn | |
| 3-636 | 3 | $CH_2$(2-MeOPh) | OBn | |
| 3-637 | 3' | $CH_2$(2-MeOPh) | OBn | |
| 3-638 | 3 | $CH_2$(2-FPh) | OBn | |
| 3-639 | 3' | $CH_2$(2-FPh) | OBn | |
| 3-640 | 3 | $CH_2$(2-ClPh) | OBn | |
| 3-641 | 3' | $CH_2$(2-ClPh) | OBn | |
| 3-642 | 3 | $CH_2$(2,6-$F_2$Ph) | OBn | |
| 3-643 | 3' | $CH_2$(2,6-$F_2$Ph) | OBn | |
| 3-644 | 3 | $CH_2$Ph | $OCH_2$(2-Pyridyl) | |
| 3-645 | 3' | $CH_2$Ph | $OCH_2$(2-Pyridyl) | |
| 3-646 | 3 | $CH_2$(2-MePh) | $OCH_2$(2-Pyridyl) | |
| 3-647 | 3' | $CH_2$(2-MePh) | $OCH_2$(2-Pyridyl) | |
| 3-648 | 3 | $CH_2$(2,6-$Me_2$Ph) | $OCH_2$(2-Pyridyl) | |
| 3-649 | 3' | $CH_2$(2,6-$Me_2$Ph) | $OCH_2$(2-Pyridyl) | |
| 3-650 | 3 | $CH_2$(2-MeOPh) | $OCH_2$(2-Pyridyl) | |
| 3-651 | 3' | $CH_2$(2-MeOPh) | $OCH_2$(2-Pyridyl) | |
| 3-652 | 3 | $CH_2$(2-FPh) | $OCH_2$(2-Pyridyl) | |
| 3-653 | 3' | $CH_2$(2-FPh) | $OCH_2$(2-Pyridyl) | |
| 3-654 | 3 | $CH_2$(2-ClPh) | $OCH_2$(2-Pyridyl) | |
| 3-655 | 3' | $CH_2$(2-ClPh) | $OCH_2$(2-Pyridyl) | |
| 3-656 | 3 | $CH_2$(2,6-$F_2$Ph) | $OCH_2$(2-Pyridyl) | |
| 3-657 | 3' | $CH_2$(2,6-$F_2$Ph) | $OCH_2$(2-Pyridyl) | |
| 3-658 | 3 | $CH_2$Ph | $OSO_2$Me | |
| 3-659 | 3' | $CH_2$Ph | $OSO_2$Me | |
| 3-660 | 3 | $CH_2$(2-MePh) | $OSO_2$Me | |

[Table 78]

| Table 3-Continued | | | | |
|---|---|---|---|---|
| No. | General formula | $R^1$ | W | Remark |
| 3-661 | 3' | $CH_2$(2-MePh) | $OSO_2Me$ | |
| 3-662 | 3 | $CH_2$(2,6-$Me_2$Ph) | $OSO_2Me$ | |
| 3-663 | 3' | $CH_2$(2,6-$Me_2$Ph) | $OSO_2Me$ | |
| 3-664 | 3 | $CH_2$(2-MeOPh) | $OSO_2Me$ | |
| 3-665 | 3' | $CH_2$(2-MeOPh) | $OSO_2Me$ | |
| 3-666 | 3 | $CH_2$(2-FPh) | $OSO_2Me$ | |
| 3-667 | 3' | $CH_2$(2-FPh) | $OSO_2Me$ | |
| 3-668 | 3 | $CH_2$(2-ClPh) | $OSO_2Me$ | |
| 3-669 | 3' | $CH_2$(2-ClPh) | $OSO_2Me$ | |
| 3-670 | 3 | $CH_2$(2,6-$F_2$Ph) | $OSO_2Me$ | |
| 3-671 | 3' | $CH_2$(2,6-$F_2$Ph) | $OSO_2Me$ | |
| 3-672 | 3 | $CH_2Ph$ | $OSO_2CF_3$ | |
| 3-673 | 3' | $CH_2Ph$ | $OSO_2CF_3$ | |
| 3-674 | 3 | $CH_2$(2-MePh) | $OSO_2CF_3$ | |
| 3-675 | 3' | $CH_2$(2-MePh) | $OSO_2CF_3$ | |
| 3-676 | 3 | $CH_2$(2,6-$Me_2$Ph) | $OSO_2CF_3$ | |
| 3-677 | 3' | $CH_2$(2,6-$Me_2$Ph) | $OSO_2CF_3$ | |
| 3-678 | 3 | $CH_2$(2-MeOPh) | $OSO_2CF_3$ | |
| 3-679 | 3' | $CH_2$(2-MeOPh) | $OSO_2CF_3$ | |
| 3-680 | 3 | $CH_2$(2-FPh) | $OSO_2CF_3$ | |
| 3-681 | 3' | $CH_2$(2-FPh) | $OSO_2CF_3$ | |
| 3-682 | 3 | $CH_2$(2-ClPh) | $OSO_2CF_3$ | |
| 3-683 | 3' | $CH_2$(2-ClPh) | $OSO_2CF_3$ | |
| 3-684 | 3 | $CH_2$(2,6-$F_2$Ph) | $OSO_2CF_3$ | |
| 3-685 | 3' | $CH_2$(2,6-$F_2$Ph) | $OSO_2CF_3$ | |
| 3-686 | 3 | $CH_2$(2-FPh) | 2-FPh | |
| 3-687 | 3 | 4-Pyridyl | H | |
| 3-688 | 3 | 2-Pyridyl | H | |
| 3-689 | 3 | Me | H | |
| 3-690 | 3 | n-Hex | H | |
| 3-691 | 3 | $CH_2CF_3$ | H | |
| 3-692 | 3 | $CH_2CH=CH_2$ | H | |
| 3-693 | 3 | $CH_2CH=CMe_2$ | H | |
| 3-694 | 3 | $CH_2C\equiv CH$ | H | |
| 3-695 | 3 | $CH_2C\equiv CMe$ | H | |

[Table 79]

| Table 3-Continued | | | | |
|---|---|---|---|---|
| No. | General formula | $R^1$ | W | Remark |
| 3-696 | 3 | c-Hex | H | |
| 3-697 | 3 | $CH_2$(c-Hex) | H | |
| 3-698 | 3 | $CH_2OMe$ | H | |
| 3-699 | 3 | 3-Me-2-Pyridyl | H | |
| 3-700 | 3 | $CH_2$(3-MePh) | H | |
| 3-701 | 3 | $CH_2$(4-MePh) | H | |
| 3-702 | 3 | $CH_2$(2-EtPh) | H | |
| 3-703 | 3 | $CH_2$(3-EtPh) | H | |
| 3-704 | 3 | $CH_2$(4-EtPh) | H | |
| 3-705 | 3 | $CH_2$(3-MeOPh) | H | |
| 3-706 | 3 | $CH_2$(4-MeOPh) | H | |
| 3-707 | 3 | $CH_2$(2-$CF_3$OPh) | H | |
| 3-708 | 3 | $CH_2$(3-$CF_3$OPh) | H | |
| 3-709 | 3 | $CH_2$(4-$CF_3$OPh) | H | |
| 3-710 | 3 | $CH_2$(3-FPh) | H | |
| 3-711 | 3 | $CH_2$(4-FPh) | H | |
| 3-712 | 3 | $CH_2$(3-ClPh) | H | |
| 3-713 | 3 | $CH_2$(4-ClPh) | H | |
| 3-714 | 3 | $CH_2$(2-BrPh) | H | |
| 3-715 | 3 | $CH_2$(3-BrPh) | H | |
| 3-716 | 3 | $CH_2$(4-BrPh) | H | |
| 3-717 | 3 | $CH_2$(2-IPh) | H | |
| 3-718 | 3 | $CH_2$(3-IPh) | H | |
| 3-719 | 3 | $CH_2$(4-IPh) | H | |
| 3-720 | 3 | $CH_2$(2-$CF_3$Ph) | H | |
| 3-721 | 3 | $CH_2$(3-$CF_3$Ph) | H | |
| 3-722 | 3 | $CH_2$(4-$CF_3$Ph) | H | |
| 3-723 | 3 | $CH_2$(2-CNPh) | H | |
| 3-724 | 3 | $CH_2$(3-CNPh) | H | |
| 3-725 | 3 | $CH_2$(4-CNPh) | H | |
| 3-726 | 3 | $CH_2$(2-COOHPh) | H | |
| 3-727 | 3 | $CH_2$(3-COOHPh) | H | |
| 3-728 | 3 | $CH_2$(4-COOHPh) | H | |
| 3-729 | 3 | $CH_2$(2-COOMePh) | H | |
| 3-730 | 3 | $CH_2$(3-COOMePh) | H | |

[Table 80]

| Table 3-Continued | | | | |
|---|---|---|---|---|
| No. | General formula | $R^1$ | W | Remark |
| 3-731 | 3 | $CH_2$(4-COOMePh) | H | |
| 3-732 | 3 | $CH_2$(2,6-$Cl_2$Ph) | H | |
| 3-733 | 3 | $CH_2$(2-Cl-6-FPh) | H | |
| 3-734 | 3 | $CH_2$(2-Br-6-ClPh) | H | |
| 3-735 | 3 | $CH_2$(1-Naphthyl) | H | |
| 3-736 | 3 | $CH_2$(2-Naphthyl) | H | |
| 3-737 | 3 | $CH_2$(2,4-$Cl_2$Ph) | H | |
| 3-738 | 3 | $CH_2$(2-Pyridyl) | H | |
| 3-739 | 3 | $CH_2$(3-Pyridyl) | H | |
| 3-740 | 3 | $CH_2$(4-Pyridyl) | H | |
| 3-741 | 3 | $CH_2$(2-Thienyl) | H | |
| 3-742 | 3 | $CH_2$(3-Thienyl) | H | |
| 3-743 | 3 | $CH_2$(3,5-$Me_2$-Isoxazol-4-1y) | H | |
| 3-744 | 3 | $CH_2CH_2OPh$ | H | |
| 3-745 | 3 | $CH_2CH_2OCH_2Ph$ | H | |
| 3-746 | 3 | $CH_2COPh$ | H | |
| 3-747 | 3 | $CH_2OCH_2CH_2OMe$ | H | |
| 3-748 | 3' | $CH_2Ph$ | H | |
| 3-749 | 3' | $CH_2$(2-MePh) | H | |
| 3-750 | 3' | $CH_2$(2-FPh) | H | |
| 3-751 | 3' | $CH_2$(2,4-$Cl_2$Ph) | H | |
| 3-752 | 3+3' | $CH_2Ph$ | H | Racemate of 3 and 3' |
| 3-753 | 3+3' | $CH_2$(2-MePh) | H | Racemate of 3 and 3' |
| 3-754 | 3+3' | $CH_2$(2-FPh) | H | Racemate of 3 and 3' |
| 3-755 | 3+3' | $CH_2$(2-Pyridyl) | H | Racemate of 3 and 3' |
| 3-756 | 3+3' | $CH_2$(2-Thienyl) | H | Racemate of 3 and 3' |
| 3-757 | 3+3' | $CH_2$(3-Thienyl) | H | Racemate of 3 and 3' |

Representative examples of the synthetic intermediate represented by the general formula (1a), (1a'), (2a), (2a'), (3a), or (3a') include compounds in which $R^1$ in the compounds of Tables 1 to 3 is substituted with a hydrogen atom, a trimethylsilyl group, a triethylsilyl group, a triisopropylsilyl group, a dimethylisopropylsilyl group, a diethylisopropylsilyl group, a dimethylhexylsilyl group, a tert-butyldimethylsilyl group, or a di-tert-butylmethylsilyl group.

Next, a method for producing the 1,4-cineole derivative represented by the above general formula (1), (1'), (2), (2'), (3) or (3') of the present invention will be described in detail, but the present invention is not limited to the method. For a reactor, the reaction can be performed using a magnetic stirrer, a mechanical stirrer, or even a microwave synthesis device.

[Production Method 1]

[Chem. 5]

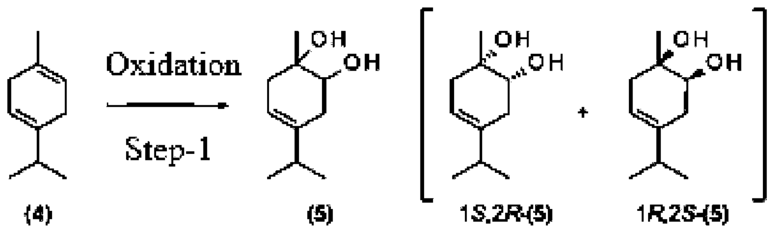


(4) (5) 1*S*,2*R*-(5) 1*R*,2*S*-(5)

Step-1 is a step of producing a diol derivative (5) by subjecting γ-terpinene represented by a formula (4) to an oxidation reaction. The γ-terpinene represented by the formula (4) is known and can be obtained from Tokyo Chemical Industry Co., Ltd., and the like.

Examples of the oxidation method in the present step include a method using an oxidizing agent such as osmium tetroxide, potassium osmium ate, lead tetraacetate, potassium permanganate, sodium periodate and ruthenium chloride, and iodine and silver acetate, and oxidation in the presence of a reoxidizing agent such as potassium ferricyanide, N-methylmorpholine N-oxide, or tert-butyl hydroperoxide. Among the oxidation method, osmium tetroxide and potassium osmate are preferred in terms of good yield.

The present reaction may be performed in the presence of a base, and examples of the base that can be used include organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, and N-methylimidazole; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium amide, butyllithium, tert-butyllithium, sec-butyllithium, lithium diisopropylamide, trimethylsilyllithium, lithium hexamethyldisilazide, sodium hexamethyldisilazide, and potassium hexamethyldisilazide. An amount of the base to be used is in a range of 0.01 equivalents to 10 equivalents with respect to γ-terpinene (4) without adversely affecting the progress of the reaction, but in order to obtain a target compound in good yield, the amount of the base to be used is more preferable in a range of 0.05 equivalents to 5 equivalents.

The present reaction is preferably performed in a solvent. Any solvent that is not harmful to the reaction can be used as the solvent, and examples of such a solvent include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, hydrochloric acid, acetic acid, and mixtures thereof.

The reaction can be performed at a temperature appropriately selected from a range of -90°C to 100°C, depending on the oxidizing agent to be used and reaction conditions, and a range of -20°C to 100°C is preferred in terms of good yield. After the reaction is complete, the target compound can be obtained by an ordinary post-treatment operation, and if necessary, the target compound can be purified by column chromatography or recrystallization.

In the present step, two isomers can also be obtained individually by performing an asymmetric reaction using an asymmetric ligand such as $(DHQ)_2PHAL$ or $(DHQD)_2PHAL$.

[Production Method 2]

[Chem. 6]

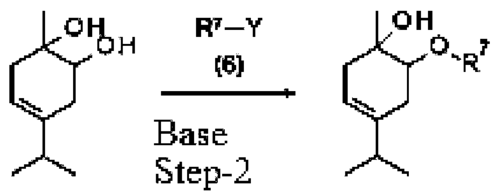


(5) (7)

($R^7$ represents a C1-C6 alkyl group, a C1-C6 haloalkyl group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl groups), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-Cll aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a benzoyl C1-C6 alkyl group (which may be mono-substituted or poly-substituted with a halogen atom, a C1-C6 alkyl group, or a C1-C6 haloalkyl group), a C1-C6 alkoxy C1-C6 alkoxy C1-C6 alkyl group, or a tri-C1-C6 alkylsilyl group which may be the same or different, and Y is a leaving group such as a halogen atom, a methanesulfonyloxy group, a trifluoromethanesulfonyloxy group, or a p-toluenesulfonyloxy group.)

The C1-C6 alkyl group represented by $R^7$ may be either linear or branched, and examples thereof include a methyl group, an ethyl group, an n-propyl group, an isopropyl group, an n-butyl group, an isobutyl group, a secbutyl group, a tert-butyl group, an n-pentyl group, a neopentyl group, a 2-pentyl group, a 3-pentyl group, a tert-pentyl group, an n-hexyl group, an isohexyl group, a 2-hexyl group and a 3-hexyl group.

The C1-C6 haloalkyl group represented by $R^7$ may be either linear or branched, and examples thereof include a monofluoromethyl group, a difluoromethyl group, a trifluoromethyl group, a 2,2,2-trifluoroethyl group, a 2-chloroethyl group, a trichloromethyl group, a 1-fluoroethyl group, a 2-fluoroethyl group, a 6-fluorohexyl group and a monobromomethyl group. The number of C1-C6 haloalkyl groups as the substituent may be one or two or more, and in the case of two or more, the respective C1-C6 haloalkyl groups may be the same or different. A substitution position of the C1-C6 haloalkyl group may be any position of the group substituted with the C1-C6 haloalkyl group.

The C2-C6 alkenyl group represented by $R^7$ may be either linear or branched, and examples thereof include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

The C2-C6 alkynyl group represented by $R^7$ may be either linear or branched, and examples thereof include an ethynyl group, a 1-propynyl group, a propargyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the C3-C6 cycloalkyl group represented by $R^7$ include a cyclopropyl group, a 1-methylcyclopropyl group, a 2-methylcyclopropyl group, a 2,2-dimethylpropyl group, a cyclobutyl group, a cyclopentyl group, and a cyclohexyl group.

The C3-C6 cycloalkyl C1-C6 alkyl group represented by $R^7$ may be either linear or branched, and examples thereof include a cyclopropylmethyl group, a cyclopropylethyl group, a 1-methylcyclopropylmethyl group, a 2-methylcyclopropylmethyl group, a 2,2-dimethylcyclopropylmethyl group, a cyclobutylmethyl group, a cyclopentylmethyl group, and a cyclohexylmethyl group.

The C1-C6 alkoxy C1-C6 alkyl group represented by $R^7$ may be either linear or branched, and examples thereof include a methoxymethyl group, an ethoxymethyl group, an n-propoxymethyl group, an isopropoxymethyl group, an n-butoxymethyl group, a sec-butoxymethyl group, a tert-butoxymethyl group, a 1-pentyloxymethyl group, a 1-hexyloxymethyl group, a 2-methoxyethyl group, a 2-ethoxyethyl group, a 2-isopropoxyethyl group, a 2-isobutoxyethyl group, a 3-methoxypropyl group, a 2-methoxypropyl group, and a 2-methoxy-1-methylethyl group.

The aryl group represented by $R^7$ refers to a monocyclic or polycyclic aromatic group, and examples thereof include a phenyl group, a 1-naphthyl group, and a 2-naphthyl group.

Examples of the heterocyclic ring represented by $R^7$ include a 2-pyridyl group, a 3-pyridyl group, a 4-pyridyl group, a 2-thienyl group, a 3-thienyl group, a 2-furyl group, a 3-furyl group, a 2-pyrimidyl group, a 4-pyrimidyl group, a 5-pyrimidyl group, a 6-pyrimidyl group, a 2-tetrahydrofuryl group, and a 3-tetrahydrofuryl group.

The C7-C11 aralkyl group represented by $R^7$ may be either linear or branched, and examples thereof include a benzyl group, a 1-phenethyl group, a 2-phenethyl group, a 1-phenylpropyl group, a 2-phenylpropyl group, a 3-phenylpropyl group, a 1-phenyl-2-methylpropyl group, a 1-phenylbutyl group, and a 1-phenylpentyl group.

The heterocyclic C1-C6 alkyl group represented by $R^7$ may be either linear or branched, and examples thereof include a 2-pyridylmethyl group, a 3-pyridylmethyl group, a 4-pyridylmethyl group, a 2-thienylmethyl group, a 3-thienylmethyl group, a 2-furfuryl group, a 3-furfuryl group, a 2-pyrimidylmethyl group, a 4-pyrimidylmethyl group, a 5-pyrimidylmethyl group, a 6-pyrimidylmethyl group, a 4-pyrazolylmethyl group, a 2-tetrahydrofurfuryl group, a 3-tetrahydrofurfuryl group, and an isoxazol-4yl-methyl group.

The phenoxy C1-C6 alkyl group represented by $R^7$ may be either linear or branched, and examples thereof include a phenoxymethyl group, a 2-phenoxyethyl group, a 2-phenoxypropyl group, a 3-phenoxypropyl group, a 2-phenoxybutyl group, a 3-phenoxybutyl group, and a 4-phenoxybutyl group.

The C7-C11 aralkyloxy C1-C6 alkyl group represented by $R^7$ may be either linear or branched, and examples thereof include a benzyloxymethyl group, a 1-phenethyloxymethyl group, a 2-phenethyloxymethyl group, a 1-phenylpropoxymethyl group, a 2-phenylpropoxymethyl group, a 3-phenylpropoxymethyl group, and a benzyloxyethyl group.

The C1-C6 alkoxy C1-C6 alkoxy C1-C6 alkyl group represented by $R^7$ may be either linear or branched, and examples thereof include a methoxymethoxymethyl group, an ethoxymethoxymethyl group, an n-propoxymethoxymethyl group, an isopropoxymethoxymethyl group, an n-butoxymethoxymethyl group, a secbutoxymethoxymethyl group, a tert-butoxymethoxymethyl group, a 1-pentyloxymethoxymethyl group, a 1-hexyloxymethoxymethyl group, a 1-ethoxyethoxymethyl group, a 1-methoxyethoxymethyl group, and a 2-methoxyethoxymethyl group.

Examples of the tri-C1-C6 alkylsilyl group being represented by $R^7$ which may be the same or different include a trimethylsilyl group, a triethylsilyl group, a triisopropylsilyl group, a dimethylisopropylsilyl group, a diethylisopropylsilyl group, a dimethylhexylsilyl group, a tert-butyldimethylsilyl group, and a di-tert-butylmethylsilyl group.

Examples of the halogen atom represented by Y include fluorine, chlorine, bromine and iodine.

Step-2 is a step of producing an olefin derivative (7) by reacting a diol derivative represented by a formula (5) (hereinafter also referred to as substrate (5) or diol (5)) with a compound represented by a formula (6) (hereinafter also referred to as reaction substrate (6)) in the presence of a base.

The present reaction may be performed in the presence of a base, and examples of the base that can be used include organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, and N-methylimidazole; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium amide, butyllithium, tert-butyllithium, sec-butyllithium, lithium diisopropylamide, trimethylsilyllithium, lithium hexamethyldisilazide, sodium hexamethyldisilazide, and potassium hexamethyldisilazide. The base can be used in an amount within a range of 0.1 equivalents to 5 equivalents with respect to the substrate (5) without adversely affecting the progress of the reaction, and in order to obtain the target compound in good yield, the base is preferably used in an amount within a range of 0.5 equivalents to 3 equivalents, and more preferably 1 equivalent to 1.5 equivalents. The reaction substrate (6) can be used in an amount within a range of 0.5 equivalents to 5 equivalents with respect to the substrate (5) without adversely affecting the progress of the reaction, and in order to obtain the target compound in good yield, the reaction substrate (6) is preferably used in an amount within a range of 1 equivalent to 3 equivalents, and more preferably within a range of 1.1 equivalents to 1.5 equivalents.

In the present reaction, the target compound may be obtained in good yield by using a reoxidizing agent, and examples of the reoxidizing agent that can be used include N-methylmorpholine oxide, trimethylamine oxide, tert-butyl hydroperoxide, and potassium ferricyanide. The reoxidizing agent can be used in an amount within a range of 0.1 equivalents to 20 equivalents with respect to the substrate (5) without adversely affecting the progress of the reaction, and in order to obtain the target compound in good yield, the reoxidizing agent is preferably used in an amount within a range of 1 equivalent to 10 equivalents, and more preferably 1.5 equivalents to 5 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide; water; and mixtures thereof.

The reaction can be performed at a temperature appropriately selected from a range of -90°C to 200°C, depending on the base to be used and reaction conditions, and a range of 0°C to 100°C is preferred in terms of good yield. After the reaction is complete, the target compound can be obtained by an ordinary post-treatment operation, and if necessary, the target compound can be purified by column chromatography or recrystallization.

In the present step, two isomers can also be obtained individually with stereochemistry thereof retained by using 1S,2R-(5) and 1R,2S-(5) instead of the diol (5).

[Production Method 3]

[Chem. 7]

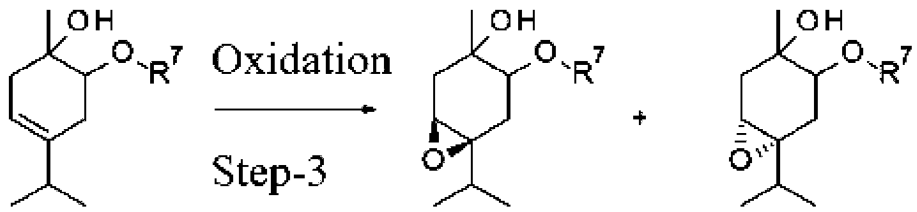


(7) 1*S*,6*S*-(8) 1*R*,6*R*-(8)

($R^7$ has the same meaning as above.)

Step-3 is a step of producing an epoxy derivative (8) by subjecting an olefin derivative represented by a formula (7) (hereinafter also referred to as substrate (7)) to an oxidation reaction. The obtained epoxy derivative (8) is a mixture of 1S,6S-(8) and 1R,6R-(8), and can be easily separated and purified by column chromatography or the like.

Examples of an oxidizing agent to be used in the present reaction include peroxides such as hydrogen peroxide, m-chloroperbenzoic acid, peracetic acid, tert-butyl hydroperoxide, sodium periodate, and OXONE, E.I. DuPont (trade name); containing potassium hydrogen peroxosulfate), N-chlorosuccinimide, N-bromosuccinimide, tert-butyl hypochlorite, sodium hypochlorite, and oxygen. Among the oxidizing agent, hydrogen peroxide and m-chloroperbenzoic acid are preferred in terms of good yield. The oxidizing agent can be used in an amount within a range of 0.1 equivalents to 10 equivalents with respect to the substrate (7) without adversely affecting the progress of the reaction, and in order to obtain the target compound in good yield, the oxidizing agent is preferably used in an amount within a range of 0.5 equivalents to 5 equivalents, and more preferably 1 equivalent to 3 equivalents.

The present reaction can be performed in the presence of a catalyst to synthesize the target compound in good yield. Examples of a catalyst to be used in the present reaction include molybdenum oxide, boric acid, iron tris(acetylacetonate), and sodium tungstate. Among the catalyst, sodium tungstate is preferred in terms of good yield. The catalyst can be used in an amount within a range of 0.01 mol% to 30 mol% with respect to the substrate (7) without adversely affecting the progress of the reaction, and in order to obtain the target compound in good yield, the catalyst is preferably used in an amount within a range of 0.1 mol% to 10 mol%, and more preferably within a range of 0.5 mol% to 1 mol% with respect to the substrate (7).

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; water; and mixed solvents thereof.

The reaction can be performed at a temperature appropriately selected from a range of -90°C to 200°C, depending on the oxidizing agent to be used and reaction conditions, and a range of -10°C to 50°C is preferred in terms of good yield. After the reaction is complete, the target compound can be obtained by an ordinary post-treatment operation, and if necessary, the target compound can be purified by column chromatography or recrystallization.

In the present step, two isomers can also be obtained individually with stereochemistry thereof retained by using, instead of the olefin derivative (7), the two isomeric olefin derivatives (7) obtained by performing step-2 using, as a raw material, 1S,2R-(5) and 1R,2S-(5) obtained by performing the asymmetric reaction in step-1.

[Production Method 4]

[Chem. 8]

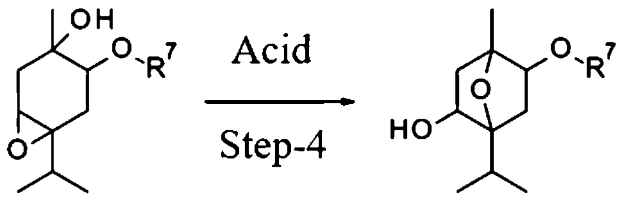


(8) (9)

($R^7$ has the same meaning as above.)

Step-4 is a step of producing a 1,4-cineole derivative (9) by ring-opening an epoxy derivative represented by a formula (8) (hereinafter also referred to as substrate (8)) using an acid and simultaneously performing intramolecular cyclization.

Examples of the acid that can be used in the present reaction include organic acids and inorganic acids such as hydrochloric acid, sulfuric acid, nitric acid, acetic acid, trifluoroacetic acid, p-tosylic acid, pyridinium p-toluenesulfonate, methanesulfonic acid, trifluoromethanesulfonic acid, camphorsulfonic acid, benzenesulfonic acid, fluorosulfonic acid, chloric acid, bromic acid, iodic acid, perbromic acid, thiocyanic acid, metaperiodic acid, hexafluorophosphoric acid, tetrafluoroboric acid, chlorobenzoic acid, and fluorobenzoic acid. The acid can be used in an amount within a range of 0.1 equivalents to 5 equivalents with respect to the substrate (8) without adversely affecting the progress of the reaction, and in order to obtain a target compound in good yield, the acid is preferably used in an amount within a range of 0.5 equivalents to 3 equivalents, and more preferably 1 equivalent to 1.5 equivalents.

The present reaction is preferably performed in a solvent. Any solvent that is not harmful to the reaction can be used as the solvent, and examples of such a solvent include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, hydrochloric acid, acetic acid, and mixtures thereof.

Depending on the acid to be used and reaction conditions, the reaction can be performed at a temperature appropriately selected from a range of -90°C to 200°C, and a range of 0°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the epoxy derivative (8), each isomeric epoxy derivative (8) obtained by performing step-2 and step-3 using, as a raw material, 1S,2R-(5) and 1R,2S-(5) obtained by performing the asymmetric reaction in step-1.

[Production Method 5]

[Chem. 9]

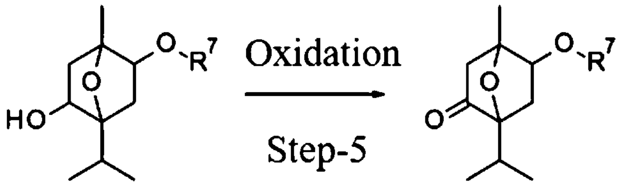


(9) (10)

($R^7$ has the same meaning as above.)

Step-5 is a step of producing a ketone derivative (10) by subjecting a 1,4-cineole derivative represented by a formula (9) (hereinafter also referred to as a substrate (9)) to an oxidation reaction.

Examples of the oxidation reaction to be used in the present reaction include Swern oxidation using dimethyl sulfoxide and oxalyl chloride or triethylamine, PCC oxidation or PDC oxidation using pyridinium chlorochromate or pyridinium nichromate, oxidation using Dess-Martin periodinane, 2-iodoxybenzoic acid (IBX), or the like, TPAP oxidation using tetrapropylammonium pertenate, oxidation using nitroxy radicals such as 2,2,6,6-tetramethylpiperidine-1-oxyl radical (TEMPO), 2-azaadamantane-N-oxyl (AZADO), and 2-hydroxy-2-azaadamantane (AZADOL), oxidation using hypervalent iodine such as iodobenzene diacetate (PIDA), 1-acetoxy-1,2-benziodoxol-3(1H)-one (ABX), and 1-(tert-butylperoxy)-1,2-benziodoxol-3-one, and oxidation using N-tert-butylbenzenesulfinimidoyl chloride. The oxidizing agent can be used in an amount within a range of 0.1 equivalents to 10 equivalents with respect to the substrate (9) without adversely affecting the progress of the reaction, and in order to obtain a target compound in good yield, the oxidizing agent is preferably used in an amount within a range of 0.5 equivalents to 3 equivalents, and more preferably 1 equivalent to 1.5 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; water; and mixed solvents thereof.

The reaction can be performed at a temperature appropriately selected from a range of -90°C to 200°C, depending on the oxidizing agent to be used and reaction conditions, and a range of 0°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the 1,4-cineole derivative (9), each isomeric 1,4-cineole derivative (9) obtained by performing step-2, step-3, and step-4 using, as a raw material, 1S,2R-(5) and 1R,2S-(5) obtained by performing the asymmetric reaction in step-1.

[Production Method 6]

[Chem. 10]

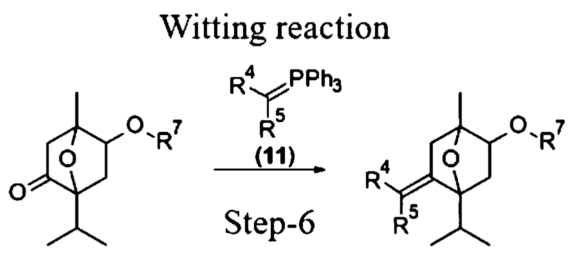


(10) (12)

($R^4$, $R^5$ and $R^7$ have the same meaning as above.)

Step-6 is a step of producing an olefin derivative (12) by subjecting a ketone derivative represented by a formula (10) (hereinafter also referred to as substrate (10)) to a Wittig reaction with a phosphonium ylide (11) (hereinafter also referred to as reaction substrate (11)).

The reaction substrate (11) can be used in an amount within a range of 0.5 equivalents to 5 equivalents with respect to the substrate (10) without adversely affecting the progress of the reaction, and in order to obtain the target compound in good yield, the reaction substrate (11) is preferably used in an amount within a range of 1 equivalent to 3 equivalents, and more preferably within a range of 1.1 equivalents to 1.5 equivalents.

The present reaction may be performed in the presence of a base, and examples of the base that can be used include alkali metal salts such as sodium carbonate, potassium carbonate, sodium hydrogencarbonate, potassium hydrogencarbonate, sodium acetate, potassium acetate, sodium methoxide, sodium ethoxide, potassium tert-butoxide, sodium hydride, potassium hydride, sodium amide, butyllithium, tert-butyllithium, sec-butyllithium, lithium diisopropylamide, trimethylsilyllithium, sodium hexamethyldisilazide, potassium hexamethyldisilazide, and lithium hexamethyldisilazide. The base can be used in an amount within a range of 0.1 equivalents to 5 equivalents with respect to the substrate (10) without adversely affecting the progress of the reaction, and in order to obtain a target compound in good yield, the base is preferably used in an amount within a range of 0.5 equivalents to 3 equivalents, and more preferably 1 equivalent to 1.5 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of -90°C to 200°C, depending on the base to be used and reaction conditions, and a range of 0°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the ketone derivative (10), each isomeric ketone derivative (10) obtained by performing step-2, step-3, step-4 and step-5 using, as a raw material, 1S,2R-(5) and 1R,2S-(5) obtained by performing the asymmetric reaction in step-1.

[Production Method 7]

[Chem. 11]

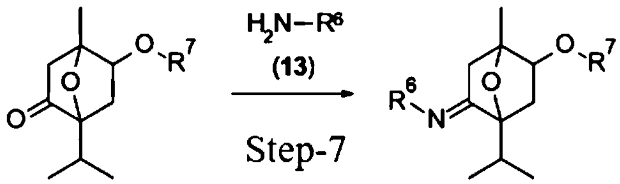


(10) (14)

($R^6$ and $R^7$ have the same meaning as above.)

Step-7 is a step of producing an imine derivative (14) by reacting a ketone derivative represented by a formula (10) with an amine compound represented by a formula (13) (hereinafter also referred to as reaction substrate (13)).

The amine compound (13) to be used in the present reaction can also be used as a salt, and examples of the salt that can be used include hydrochloride, sulfate, and carbonate.

The reaction substrate (13) can be used in an amount within a range of 0.5 equivalents to 5 equivalents with respect to the substrate (10) without adversely affecting the progress of the reaction, and in order to obtain the target compound in good yield, the reaction substrate (13) is preferably used in an amount within a range of 1 equivalent to 3 equivalents, and more preferably within a range of 1.1 equivalents to 1.5 equivalents.

The present reaction may be performed in the presence of an acid or a base. Examples of the acid that can be used include hydrochloric acid, sulfuric acid, and acetic acid. Examples of the base that can be used include organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, and N-methylimidazole; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium amide, butyllithium, tert-butyllithium, lithium diisopropylamide, trimethylsilyllithium, and lithium hexamethyldisilazide. The acid or base can be used in an amount within a range of 0.1 equivalents to 10 equivalents with respect to the substrate (10) without adversely affecting the progress of the reaction, and in order to obtain a target compound in good yield, the acid or base is preferably used in an amount within a range of 0.5 equivalents to 5 equivalents, and more preferably 1 equivalent to 3 equivalents.

The present reaction is preferably performed in a solvent. Any solvent that is not harmful to the reaction can be used as the solvent, and examples of the solvent that can be used include aromatic hydrocarbon solvents such as

benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the base to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the ketone derivative (10), each isomeric ketone derivative (10) obtained by performing step-2, step-3, step-4 and step-5 using, as a raw material, 1S,2R-(5) and 1R,2S-(5) obtained by performing the asymmetric reaction in step-1.

[Production Method 8]

[Chem. 12]

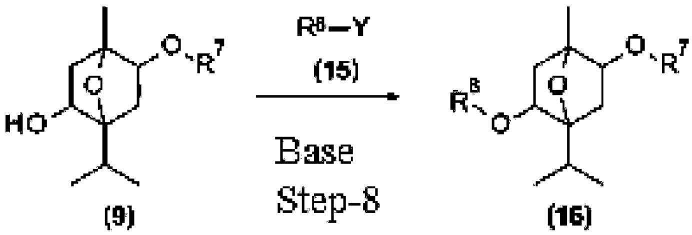


(9) (16)

($R^7$ and Y have the same meaning as above. $R^8$ represents a C1-C6 alkyl group, a C1-C6 haloalkyl group, a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C1-C6 alkoxy C1-C6 alkyl group, a C1-C6 alkylcarbonyl group, a C1-C6 alkoxycarbonyl group, a C1-C6 alkylthiocarbonyl group, a C1-C6 alkylthiothiocarbonyl group, a C1-C6 alkylsulfonyl group, or a C1-C6 haloalkylsulfonyl group.)

The C1-C6 alkyl group represented by $R^8$ may be either linear or branched, and examples thereof include a methyl group, an ethyl group, an n-propyl group, an isopropyl group, an n-butyl group, an isobutyl group, a secbutyl group, a tert-butyl group, an n-pentyl group, a neopentyl group, a 2-pentyl group, a 3-pentyl group, a tert-pentyl group, an n-hexyl group, an isohexyl group, a 2-hexyl group and a 3-hexyl group.

The C1-C6 haloalkyl group represented by $R^8$ may be either linear or branched, and examples thereof include a monofluoromethyl group, a difluoromethyl group, a trifluoromethyl group, a 2,2,2-trifluoroethyl group, a 2-chloroethyl group, a trichloromethyl group, a 1-fluoroethyl group, a 2-fluoroethyl group, and a 6-fluorohexyl group.

The C7-C11 aralkyl group represented by $R^8$ may be either linear or branched, and examples thereof include a benzyl group, a 1-phenethyl group, a 2-phenethyl group, a 1-phenylpropyl group, a 2-phenylpropyl group, a 3-phenylpropyl group, a 1-phenyl-2-methylpropyl group, a 1-phenylbutyl group, and a 1-phenylpentyl group.

The heterocyclic C1-C6 alkyl group represented by $R^8$ may be either linear or branched, and examples thereof include a 2-pyridylmethyl group, a 3-pyridylmethyl group, a 4-pyridylmethyl group, a 2-thienylmethyl group, a 3-thienylmethyl group, a 2-furfuryl group, a 3-furfuryl group, a 2-pyrimidylmethyl group, a 4-pyrimidylmethyl group, a 5-pyrimidylmethyl group, a 6-pyrimidylmethyl group, a 2-tetrahydrofurfuryl group, and a 3-tetrahydrofurfuryl group.

The C1-C6 alkoxy C1-C6 alkyl group represented by $R^8$ may be either linear or branched, and examples thereof include a methoxymethyl group, an ethoxymethyl group, an n-propoxymethyl group, an isopropoxymethyl group, an n-butoxymethyl group, a sec-butoxymethyl group, a tert-butoxymethyl group, a 1-pentyloxymethyl group, a 1-hexyloxymethyl group, a 2-methoxyethyl group, a 2-ethoxyethyl group, a 2-isopropoxyethyl group, a 2-isobutoxyethyl group, a 3-methoxypropyl group, a 2-methoxypropyl group, and a 2-methoxy-1-methylethyl group.

Examples of the C1-C6 alkylcarbonyl group represented by $R^8$ include an acetyl group, an ethylcarbonyl group, an n-propylcarbonyl group, an isopropylcarbonyl group, an n-butylcarbonyl group, an isobutylcarbonyl group, a sec-butylcarbonyl group, a tert-butylcarbonyl group, a 1-pentylcarbonyl group, and a 1-hexylcarbonyl group.

Examples of the C1-C6 alkoxycarbonyl group represented by $R^8$ include a methoxycarbonyl group, an ethoxycarbonyl group, an n-propoxycarbonyl group, an isopropoxycarbonyl group, an n-butoxycarbonyl group, a secbutoxycarbonyl group, and a tert-butoxycarbonyl group.

Examples of the C1-C6 alkylthiocarbonyl group represented by $R^8$ include a methylthiocarbonyl group, an ethylthiocarbonyl group, an n-propylthiocarbonyl group, an isopropylthiocarbonyl group, an n-butylthiocarbonyl group, a sec-butylthiocarbonyl group, a tert-butylthiocarbonyl group, a 1-pentylthiocarbonyl group, and a 1-hexylthiocarbonyl group.

Examples of the C1-C6 alkylthiothiocarbonyl group represented by $R^8$ include a methylthiothiocarbonyl group, an ethylthiothiocarbonyl group, an n-propylthiothiocarbonyl group, an isopropylthiothiocarbonyl group, an n-butylthiothiocarbonyl group, a sec-butylthiothiocarbonyl group, a tert-butylthiothiocarbonyl group, a 1-pentylthiothiocarbonyl group, and a 1-hexylthiothiocarbonyl group.

Examples of the C1-C6 alkylsulfonyl group represented by $R^8$ include a methanesulfonyl group, an ethanesulfonyl group, an n-propanesulfonyl group, an isopropanesulfonyl group, an n-butanesulfonyl group, an isobutanesulfonyl group, a sec-butanesulfonyl group, a tert-butanesulfonyl group, and an n-pentanesulfonyl group.

Examples of the C1-C6 haloalkylsulfonyl group represented by $R^8$ include a monofluoromethylsulfonyl group, a difluoromethylsulfonyl group, a trifluoromethylsulfonyl group, a monochloromethylsulfonyl group, a trichloromethylsulfonyl group, and a 2,2,2-trifluoroethylsulfonyl group.

Step-8 is a step of producing a 1,4-cineole derivative (16) by reacting a compound represented by a formula (15) (hereinafter also referred to as reaction substrate (15)) with the hydroxyl group at 5-position of the 1,4-cineole derivative represented by the formula (9).

The reaction substrate (15) can be used in an amount within a range of 0.5 equivalents to 5 equivalents with respect to the substrate (9) without adversely affecting the progress of the reaction, and in order to obtain the target compound in good yield, the reaction substrate (15) is preferably used in an amount within a range of 1 equivalent to 3 equivalents, and more preferably within a range of 1.1 equivalents to 1.5 equivalents.

The present reaction may be performed in the presence of a base, and examples of the base that can be used include organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, and N-methylimidazole; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium amide, butyllithium, tert-butyllithium, sec-butyllithium, lithium diisopropylamide, trimethylsilyllithium, sodium hexamethyldisilazide, potassium hexamethyldisilazide, and lithium hexamethyldisilazide. The base can be used in an amount within a range of 0.1 equivalents to 10 equivalents with respect to the substrate (9) without adversely affecting the progress of the reaction, and in order to obtain the target compound in good yield, the base is preferably used in an amount within a range of 0.5 equivalents to 3 equivalents, and more preferably 1 equivalent to 1.5 equivalents.

Any solvent that is not harmful to the reaction can be used as the solvent to be used in the present reaction, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the base to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the 1,4-cineole derivative (9), each isomeric 1,4-cineole derivative (9) obtained by performing step-2, step-3, and step-4 using, as a raw material, 1S,2R-(5) and 1R,2S-(5) obtained by performing the asymmetric reaction in step-1.

[Production Method 9]

[Chem. 13]

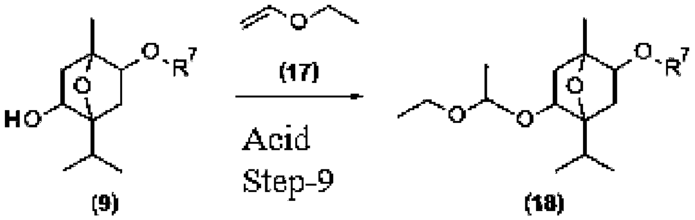


(9) (17) (18)

($R^7$ has the same meaning as above.)

Step-9 is a step of producing an ether derivative represented by a formula (18) by reacting the 1,4-cineole derivative represented by the formula (9) with ethyl vinyl ether (17) (hereinafter also referred to as reaction substrate (17)) in the presence of an acid catalyst.

The reaction substrate (17) can be used in an amount within a range of 0.5 equivalents to 5 equivalents with respect to the substrate (9) without adversely affecting the progress of the reaction, and in order to obtain the target compound in good yield, the reaction substrate (17) is preferably used in an amount within a range of 1 equivalent to 3 equivalents, and more preferably within a range of 1.1 equivalents to 1.5 equivalents.

The acid to be used as a catalyst in the present reaction may be an organic acid or an inorganic acid such as hydrochloric acid, sulfuric acid, acetic acid, p-tosylic acid, or pyridinium p-toluenesulfonate. The acid can be used in an amount within a range of 0.001 equivalents to 1 equivalent with respect to the substrate (9) without adversely affecting the progress of the reaction, and in order to obtain the target compound in good yield, the acid is preferably used in an amount within a range of 0.005 equivalents to 0.5 equivalents, and more preferably 0.05 equivalents to 0.1 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the acid to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the 1,4-cineole derivative (9), each isomeric 1,4-cineole derivative (9) obtained by performing step-2, step-3, and step-4 using, as a raw material, 1S,2R-(5) and 1R,2S-(5) obtained by performing the asymmetric reaction in step-1.

[Production Method 10]

[Chem. 14]

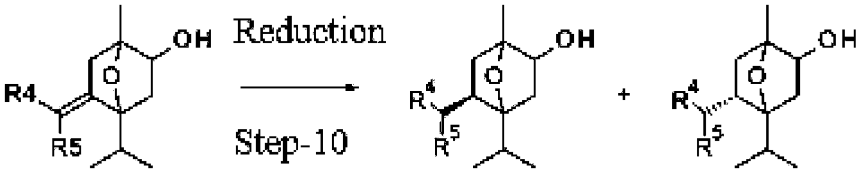


(19) 5*R*-(20) 5*S*-(20)

($R^4$ and $R^5$ have the same meaning as above.)

Step-10 is a step of producing a 1,4-cineole derivative (20) by reducing an olefin derivative represented by a formula (19). The obtained 1,4-cineole derivative (20) is a mixture of 5R-(20) and 5S-(20), and can be easily separated and

purified by column chromatography or the like.

The reduction method used in the present reaction may include a method using a reducing agent such as zinc powder, reduced iron, tin powder, stannous chloride, or titanium chloride; a method using a hydrogen donor such as hydrazine in the presence of Raney nickel; catalytic hydrogen reduction in the presence of a catalyst such as Raney nickel, palladium carbon, palladium hydroxide, platinum oxide, or rhodium carbon; or catalytic hydrogen transfer reduction.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the olefin derivative (19), each isomeric olefin derivative (19) obtained by performing step-2, step-3, step-4, step-5, step-6 and step-15 using, as a raw material, 1S,2R-(5) and 1R,2S-(5) obtained by performing the asymmetric reaction in step-1.

[Production Method 11]

[Chem. 15]

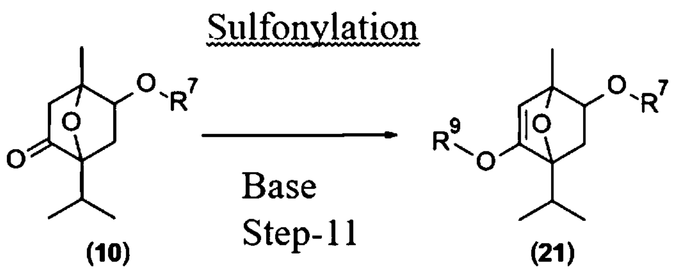


**(10)** **(21)**

($R^7$ has the same meaning as above. $R^9$ represents a C1-C6 alkylsulfonyl group, a C1-C6 haloalkylsulfonyl group, or a p-toluenesulfonyl group.)

Examples of the C1-C6 alkylsulfonyl group represented by $R^9$ include a methanesulfonyl group, an ethanesulfonyl group, an n-propanesulfonyl group, an isopropanesulfonyl group, an n-butanesulfonyl group, an isobutanesulfonyl group, a sec-butanesulfonyl group, a tert-butanesulfonyl group, and an n-pentanesulfonyl group.

Examples of the C1-C6 haloalkylsulfonyl group represented by $R^9$ include a monofluoromethylsulfonyl group, a difluoromethylsulfonyl group, a trifluoromethylsulfonyl group, a monochloromethylsulfonyl group, a trichloromethylsulfonyl group, and a 2,2,2-trifluoroethylsulfonyl group.

Step-11 is a step of producing a 1,4-cineole derivative (21) by reacting a ketone derivative represented by a formula (10) with a sulfonylating agent.

Examples of the sulfonylating agent to be used in the present reaction include methanesulfonyl chloride, methanesulfonyl bromide, methanesulfonic anhydride, trifluoromethanesulfonyl fluoride, trifluoromethanesulfonyl chloride, trifluoromethanesulfonyl bromide, trifluoromethanesulfonic anhydride, 1-(trifluoromethylsulfonyl)imidazole, 4-nitrophenyl trifluoromethanesulfonate, 1-(trifluoromethanesulfonyl)-1H-benzotriazole, N-phenylbis(trifluoromethanesulfonimide), trifluoromethanesulfonanilide, N-(2-pyridyl)bis(trifluoromethanesulfonimide), 2-[N,N-bis(trifluoromethanesulfonyl)amino]-5-chloropyridine, p-toluenesulfonyl chloride, 1-(p-toluenesulfonyl)imidazole, and p-toluenesulfonic anhydride. The sulfonylating agent can be used in an amount within a range of 0.1 equivalents to 10 equivalents with respect to the substrate (10) without adversely affecting the progress of the reaction, and in order to obtain the target compound in good yield, the sulfonylating agent is preferably used in an amount within a range of 0.5 equivalents to 5 equivalents, and more preferably 1 equivalent to 3 equivalents.

The present reaction may be performed in the presence of a base. Examples of the base that can be used include

organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, and N-methylimidazole; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium amide, butyllithium, tert-butyllithium, lithium diisopropylamide, trimethylsilyllithium, and lithium hexamethyldisilazide. The base can be used in an amount within a range of 0.1 equivalents to 10 equivalents with respect to the substrate (10) without adversely affecting the progress of the reaction, and in order to obtain the target compound in good yield, the base is preferably used in an amount within a range of 0.5 equivalents to 5 equivalents, and more preferably 1 equivalent to 3 equivalents.

Any solvent that is not harmful to the reaction can be used as the solvent to be used in the present reaction, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of -90°C to 200°C, depending on the base to be used and reaction conditions, and a range of 0°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the ketone derivative (10), each isomeric ketone derivative (10) obtained by performing step-2, step-3, step-4 and step-5 using, as a raw material, 1S,2R-(5) and 1R,2S-(5) obtained by performing the asymmetric reaction in step-1.

[Production Method 12]

[Chem. 16]

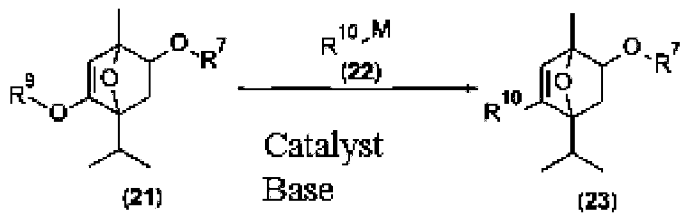


(21) (23)

Step-12

($R^7$ and $R^9$ have the same meaning as above. $R^{10}$ represents a C1-C6 alkyl group, a C1-C6 haloalkyl group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), or a heterocyclic C1-C6 alkyl group (which may be mono-substituted or poly-substituted with a halogen atom, a C1-C6 alkyl group, or a C1-C6 haloalkyl group). M represents ZnZ, $Sn(R^{11})_3$, $B(OR^{11})_2$, or MgZ (Z represents a halogen atom, and $R^{11}$ represents a hydrogen atom, a C1-C6 alkyl group, or a C3-C6 cycloalkyl group, and $R^{11}$'s may be the same or different, each independently. In $B(OR^{11})_2$, $R^{11}$'s may be bonded to each other to form a 5-membered ring or a 6-membered ring.).)

The C1-C6 alkyl group represented by $R^{10}$ may be either linear or branched, and examples thereof include a

methyl group, an ethyl group, an n-propyl group, an isopropyl group, an n-butyl group, an isobutyl group, a secbutyl group, a tert-butyl group, an n-pentyl group, a neopentyl group, a 2-pentyl group, a 3-pentyl group, a tert-pentyl group, an n-hexyl group, an isohexyl group, a 2-hexyl group and a 3-hexyl group.

The C1-C6 haloalkyl group represented by $R^{10}$ may be either linear or branched, and examples thereof include a monofluoromethyl group, a difluoromethyl group, a trifluoromethyl group, a 2,2,2-trifluoroethyl group, a 2-chloroethyl group, a trichloromethyl group, a 1-fluoroethyl group, a 2-fluoroethyl group, a 6-fluorohexyl group and a monobromomethyl group.

The C2-C6 alkenyl group represented by $R^{10}$ may be either linear or branched, and examples thereof include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

The C2-C6 alkynyl group represented by $R^{10}$ may be either linear or branched, and examples thereof include an ethynyl group, a 1-propynyl group, a propargyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the C3-C6 cycloalkyl group represented by $R^{10}$ include a cyclopropyl group, a 1-methylcyclopropyl group, a 2-methylcyclopropyl group, a 2,2-dimethylpropyl group, a cyclobutyl group, a cyclopentyl group, and a cyclohexyl group.

The C3-C6 cycloalkyl C1-C6 alkyl group represented by $R^{10}$ may be either linear or branched, and examples thereof include a cyclopropylmethyl group, a cyclopropylethyl group, a 1-methylcyclopropylmethyl group, a 2-methylcyclopropylmethyl group, a 2,2-dimethylcyclopropylmethyl group, a cyclobutylmethyl group, a cyclopentylmethyl group, and a cyclohexylmethyl group.

The aryl group represented by $R^{10}$ refers to a monocyclic or polycyclic aromatic group, and examples thereof include a phenyl group, a 1-naphthyl group, and a 2-naphthyl group.

Examples of the heterocyclic ring represented by $R^{10}$ include a 2-pyridyl group, a 3-pyridyl group, a 4-pyridyl group, a 2-thienyl group, a 3-thienyl group, a 2-furyl group, a 3-furyl group, a 2-pyrimidyl group, a 4-pyrimidyl group, a 5-pyrimidyl group, a 6-pyrimidyl group, a 2-tetrahydrofuryl group, and a 3-tetrahydrofuryl group.

The C7-C11 aralkyl group represented by $R^{10}$ may be either linear or branched, and examples thereof include a benzyl group, a 1-phenethyl group, a 2-phenethyl group, a 1-phenylpropyl group, a 2-phenylpropyl group, a 3-phenylpropyl group, a 1-phenyl-2-methylpropyl group, a 1-phenylbutyl group, and a 1-phenylpentyl group.

The heterocyclic C1-C6 alkyl group represented by $R^{10}$ may be either linear or branched, and examples thereof include a 2-pyridylmethyl group, a 3-pyridylmethyl group, a 4-pyridylmethyl group, a 2-thienylmethyl group, a 3-thienylmethyl group, a 2-furfuryl group, a 3-furfuryl group, a 2-pyrimidylmethyl group, a 4-pyrimidylmethyl group, a 5-pyrimidylmethyl group, a 6-pyrimidylmethyl group, a 4-pyrazolylmethyl group, a 2-tetrahydrofurfuryl group, and a 3-tetrahydrofurfuryl group.

Examples of the halogen atom represented by Z include chlorine, bromine and iodine.

The C1-C6 alkyl group represented by $R^{11}$ may be either linear or branched, and examples thereof include a methyl group, an ethyl group, an n-propyl group, an isopropyl group, an n-butyl group, an isobutyl group, a secbutyl group, a tert-butyl group, an n-pentyl group, a neopentyl group, a 2-pentyl group, a 3-pentyl group, a tert-pentyl group, an n-hexyl group, an isohexyl group, a 2-hexyl group and a 3-hexyl group.

Examples of the C3-C6 cycloalkyl group represented by $R^{11}$ include a cyclopropyl group, a 1-methylcyclopropyl group, a 2-methylcyclopropyl group, a 2,2-dimethylpropyl group, a cyclobutyl group, a cyclopentyl group, and a cyclohexyl group.

Step-12 is a step of producing an olefin derivative (23) by reacting an organometallic reagent (22) (hereinafter also referred to as reaction substrate (22)) with the group of a 1,4-cineole derivative represented by a formula (21) (hereinafter also referred to as substrate (21)) at 5-position.

The reaction substrate (22) can be used in an amount within a range of 0.5 equivalents to 5 equivalents with respect to the substrate (21) without adversely affecting the progress of the reaction, and in order to obtain the target compound in good yield, the reaction substrate (22) is preferably used in an amount within a range of 1 equivalent to 3 equivalents, and more preferably within a range of 1.1 equivalents to 1.5 equivalents.

The present reaction may be performed in the presence of a base. Examples of the base that can be used include organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, and N-methylimidazole; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium

amide, butyllithium, tert-butyllithium, lithium diisopropylamide, trimethylsilyllithium, and lithium hexamethyldisilazide. The base can be used in an amount within a range of 0.1 equivalents to 10 equivalents with respect to the substrate (21) without adversely affecting the progress of the reaction, and in order to obtain the target compound in good yield, the base is preferably used in an amount within a range of 0.5 equivalents to 3 equivalents, and more preferably 1 equivalent to 1.5 equivalents.

Examples of a catalyst to be used in the present reaction includes a copper compound, a tin compound, a palladium compound, and palladium black, and a palladium compound is preferred. Examples of the palladium compound include palladium chloride, palladium bromide, palladium iodide, palladium acetate, palladium trifluoroacetate, palladium nitrate, palladium oxide, dichlorobis(triphenylphosphine)palladium, tetrakis(triphenylphosphine)palladium, palladium cyanide, dichlorobis(tri-ortho-tolylphosphine)palladium, dichlorobis(tricyclohexylphosphine)palladium, bis(dibenzylideneacetone)palladium, tris(dibenzylideneacetone)dipalladium, bis(acetylacetonato)palladium, dichloro-(1,5-cyclooctadiene)-palladium, dichlorodiamminepalladium, tetraamminepalladium nitrate, tetraamminepalladium tetrachloropalladate, dichlorodipyridinepalladium, dichloro(2,2'-bipyridyl)palladium, dichloro(phenanthroline)palladium, dichloro[1,2-bis(diphenylphosphino)ethane]palladium, dichloro[1,3-bis(diphenylphosphino)propane]palladium, dichloro[1,4-bis(diphenylphosphino)butane]palladium, dichloro[1,1'-bis(diphenylphosphino)ferrocene]palladium, allylpalladium(II) chloride dimer, allylchloro-[1,3-bis-(diisopropylphenyl)-imidazol-2-ylidene]palladium, phenylallylchloro-[1,3-bis-(diisopropylphenyl)-imidazol-2-ylidene]palladium, and dichloro-[1,3-bis(diisopropylphenyl)imidazolylidene]-(3-chloropyridyl)palladium. Among the metal catalyst, palladium chloride, palladium acetate, tetrakis(triphenylphosphine)palladium, and dichloro[1,1'-bis(diphenylphosphino)ferrocene]palladium are more preferred in terms of good yield. The amount of the catalyst to be used is within a range of 0.001 mol% to 50 mol% with respect to the substrate (21) without adversely affecting the progress of the reaction, and in order to obtain the target compound in a good yield, the catalyst is preferably used within a range of 0.01 mol% to 30 mol%, and more preferably within a range of 0.1 mol% to 10 mol%.

The palladium catalyst may be used alone or in combination with a tertiary phosphine. Specific examples of the tertiary phosphine include triphenylphosphine, tri(orthotolyl)phosphine, trimethylphosphine, triethylphosphine, tripropylphosphine, triisopropylphosphine, tributylphosphine, triisobutylphosphine, tri(tert-butyl)phosphine, trineopentylphosphine, tricyclohexylphosphine, trioctylphosphine, 1,2-bis(diphenylphosphino)ethane, 1,3-bis(diphenylphosphino)propane, 1,4-bis(diphenylphosphino)butane, 1,1'-bis(diphenylphosphino)ferrocene, 4,5-bis(diphenylphosphino)-9,9-dimethylxanthene, tris(hydroxymethyl)phosphine, 2-dicyclohexylphosphino-2',6'-dimethoxy-1,1'-biphenyl, 2-dicyclohexylphosphino-2',6'-diisopropoxybiphenyl, and 2-(dicyclohexylphosphino)-2',4',6'-triisopropyl-1,1'-biphenyl. Among the tertiary phosphine, triphenylphosphine, 1,1'-bis(diphenylphosphino)ferrocene, and 2-dicyclohexylphosphino-2',6'-dimethoxy-1,1'-biphenyl are preferred in terms of good yield.

Any solvent that is not harmful to the reaction can be used as the solvent to be used in the present reaction, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the base to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the 1,4-cineole derivative (21), each isomeric 1,4-cineole derivative (21) obtained by performing step-2, step-3, step-4, step-5 and step-11 using, as a raw material, 1S,2R-(5) and 1R,2S-(5) obtained by performing the asymmetric reaction in step-1.

[Production Method 13]

[Chem. 17]

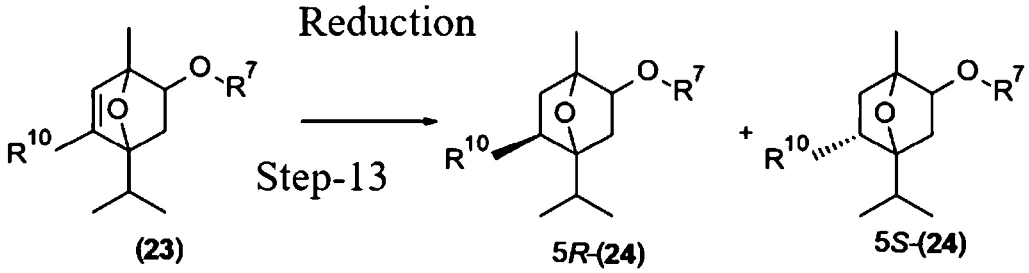


(23) 5R-(24) 5S-(24)

($R^7$ and $R^{10}$ have the same meaning as above.)

Step-13 is a step of producing a 1,4-cineole derivative (24) by reducing an olefin derivative represented by a formula (23). The obtained 1,4-cineole derivative (24) is a mixture of 5R-(24) and 5S-(24), and can be easily separated and purified by column chromatography or the like.

The reduction method used in the present reaction may include a method using a reducing agent such as zinc powder, reduced iron, tin powder, stannous chloride, or titanium chloride; a method using a hydrogen donor such as hydrazine in the presence of Raney nickel; catalytic hydrogen reduction in the presence of a catalyst such as Raney nickel, palladium carbon, palladium hydroxide, platinum oxide, or rhodium carbon; or catalytic hydrogen transfer reduction.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of -90°C to 200°C, depending on reaction conditions, and a range of 0°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the olefin derivative (23), each isomeric olefin derivative (23) obtained by performing step-2, step-3, step-4, step-5, step-11 and step-12 using, as a raw material, 1S,2R-(5) and 1R,2S-(5) obtained by performing the asymmetric reaction in step-1.

[Production Method 14]

[Chem. 18]

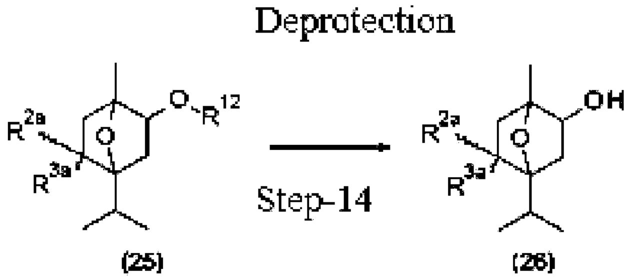


(25) (26)

($R^{2a}$ and $R^{3a}$ have the same meanings as above. $R^{12}$ may be the same or different and represents a tri-C1-C6 alkylsilyl group.)

Examples of the tri-C1-C6 alkylsilyl group being represented by $R^{12}$ which may be the same or different include a trimethylsilyl group, a triethylsilyl group, a triisopropylsilyl group, a dimethylisopropylsilyl group, a diethylisopropylsilyl group, a dimethylhexylsilyl group, a tert-butyldimethylsilyl group, and a di-tert-butylmethylsilyl group.

Step-14 is a step of producing an alcohol derivative represented by a formula (26) by deprotecting a silyl ether

derivative represented by a formula (25) (hereinafter also referred to as substrate (25)) in the presence of an acid or fluoride ion.

Examples of the acid that can be used in the present reaction include organic acids and inorganic acids such as hydrochloric acid, sulfuric acid, nitric acid, acetic acid, trifluoroacetic acid, p-tosylic acid, pyridinium p-toluenesulfonate, methanesulfonic acid, trifluoromethanesulfonic acid, camphorsulfonic acid, benzenesulfonic acid, fluorosulfonic acid, chloric acid, bromic acid, iodic acid, perbromic acid, thiocyanic acid, metaperiodic acid, hexafluorophosphoric acid, tetrafluoroboric acid, chlorobenzoic acid, and fluorobenzoic acid. The acid can be used in an amount within a range of 0.1 equivalent to 10 equivalents with respect to the substrate (25) without adversely affecting the progress of the reaction, and in order to obtain the target compound in good yield, the acid is preferably used in an amount within a range of 0.5 equivalents to 3 equivalents, and more preferably 1 equivalent to 1.5 equivalents.

Examples of the fluoride ion to be used in the present reaction include potassium fluoride, cesium fluoride, hydrofluoric acid and a salt thereof, and tetrabutylammonium fluoride. The fluoride ion can be used in an amount within a range of 0.1 equivalent to 10 equivalents with respect to the substrate (25) without adversely affecting the progress of the reaction, and in order to obtain the target compound in good yield, the fluoride ion is preferably used in an amount within a range of 0.5 equivalents to 3 equivalents, and more preferably 1 equivalent to 1.5 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the acid to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the silyl ether derivative (25), each isomeric silyl ether derivative (25) obtained by performing step-2, step-3, step-4 and step-8, or step-2, step-3, step-4 and step-8, or step-2, step-3, step-4 and step-9, or step-2, step-3, step-4, step-5, step-11, step-12, and step-13 using, as a raw material, 1S,2R-(5) and 1R,2S-(5) obtained by performing the asymmetric reaction in step-1.

[Production Method 15]

[Chem. 19]

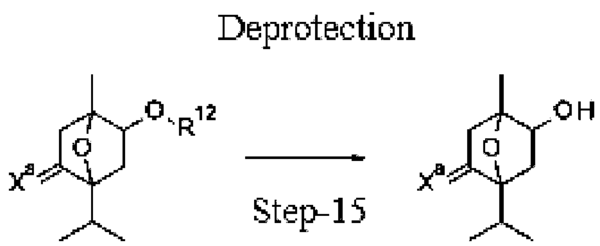


(27) (28)

($X^a$ and $R^{12}$ have the same meanings as above.)

Step- 15 is a step of producing an alcohol derivative represented by a formula (28) by deprotecting a silyl ether derivative represented by a formula (27) (hereinafter also referred to as substrate (27)) in the presence of an acid or fluoride ion.

Examples of the acid that can be used in the present reaction include organic acids and inorganic acids such as hydrochloric acid, sulfuric acid, nitric acid, acetic acid, trifluoroacetic acid, p-tosylic acid, pyridinium p-toluenesulfonate, methanesulfonic acid, trifluoromethanesulfonic acid, camphorsulfonic acid, benzenesulfonic acid, fluorosulfonic acid, chloric acid, bromic acid, iodic acid, perbromic acid, thiocyanic acid, metaperiodic acid, hexafluorophosphoric acid,

tetrafluoroboric acid, chlorobenzoic acid, and fluorobenzoic acid. The acid can be used in an amount within a range of 0.1 equivalent to 10 equivalents with respect to the substrate (27) without adversely affecting the progress of the reaction, and in order to obtain the target compound in good yield, the acid is preferably used in an amount within a range of 0.5 equivalents to 3 equivalents, and more preferably 1 equivalent to 1.5 equivalents.

Examples of the fluoride ion to be used in the present reaction include sodium fluoride, potassium fluoride, cesium fluoride, hydrofluoric acid and a salt thereof, and tetrabutylammonium fluoride. The fluoride ion can be used in an amount within a range of 0.1 equivalent to 10 equivalents with respect to the substrate (27) without adversely affecting the progress of the reaction, and in order to obtain a target compound in good yield, the fluoride ion is preferably used in an amount within a range of 0.5 equivalents to 3 equivalents, and more preferably 1 equivalent to 1.5 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the acid to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the silyl ether derivative (27), each isomeric silyl ether derivative (27) obtained by performing step-2, step-3, step-4, step-5 and step-6 or step-2, step-3, step-4, step-5 and step-7 using, as a raw material, 1S,2R-(5) and 1R,2S-(5) obtained by performing the asymmetric reaction in step-1.

[Production Method 16]

[Chem. 20]

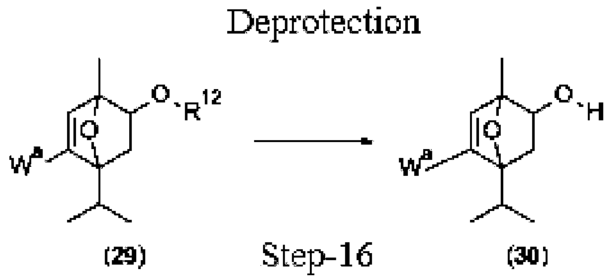


(29) (30)

($W^a$ and $R^{12}$ have the same meanings as above.)

Step-16 is a step of producing an alcohol derivative represented by a formula (30) by deprotecting a silyl ether derivative represented by a formula (29) (hereinafter also referred to as substrate (29)) in the presence of an acid or fluoride ion.

Examples of the acid that can be used in the present reaction include organic acids and inorganic acids such as hydrochloric acid, sulfuric acid, nitric acid, acetic acid, trifluoroacetic acid, p-tosylic acid, pyridinium p-toluenesulfonate, methanesulfonic acid, trifluoromethanesulfonic acid, camphorsulfonic acid, benzenesulfonic acid, fluorosulfonic acid, chloric acid, bromic acid, iodic acid, perbromic acid, thiocyanic acid, metaperiodic acid, hexafluorophosphoric acid, tetrafluoroboric acid, chlorobenzoic acid, and fluorobenzoic acid. The acid can be used in an amount within a range of 0.1 equivalent to 10 equivalents with respect to the substrate (29) without adversely affecting the progress of the reaction, and in order to obtain the target compound in good yield, the acid is preferably used in an amount within a range of 0.5 equivalents to 3 equivalents, and more preferably 1 equivalent to 1.5 equivalents.

Examples of the fluoride ion to be used in the present reaction include sodium fluoride, potassium fluoride, cesium fluoride, hydrofluoric acid and a salt thereof, and tetrabutylammonium fluoride. The fluoride ion can be used in an

amount within a range of 0.1 equivalent to 10 equivalents with respect to the substrate (29) without adversely affecting the progress of the reaction, and in order to obtain the target compound in good yield, the fluoride ion is preferably used in an amount within a range of 0.5 equivalents to 3 equivalents, and more preferably 1 equivalent to 1.5 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the acid to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the silyl ether derivative (29), each isomeric silyl ether derivative (29) obtained by performing step-2, step-3, step-4, step-5, step-11 and step-12 using, as a raw material, 1S,2R-(5) and 1R,2S-(5) obtained by performing the asymmetric reaction in step-1.

[Production Method 17]

[Chem. 21]

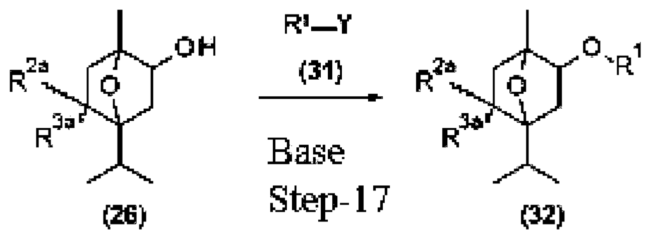


($R^1$, $R^{2a}$, $R^{3a}$ and Y have the same meanings as above.)

Step-17 is a step of producing a 1,4-cineole derivative (32) by reacting an alcohol derivative represented by a formula (26) (hereinafter also referred to as substrate (26)) with a compound represented by a formula (31) (hereinafter also referred to as reaction substrate (31)).

The reaction substrate (31) can be used in an amount within a range of 0.5 equivalents to 5 equivalents with respect to the substrate (26) without adversely affecting the progress of the reaction, and in order to obtain the target compound in good yield, the reaction substrate (31) is preferably used in an amount within a range of 1 equivalent to 3 equivalents, and more preferably within a range of 1.1 equivalents to 1.5 equivalents.

The present reaction may be performed in the presence of a base. Examples of the base that can be used include organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, and N-methylimidazole; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium amide, butyllithium, tert-butyllithium, lithium diisopropylamide, trimethylsilyllithium, and lithium hexamethyldisilazide. The base can be used in an amount within a range of 0.1 equivalents to 10 equivalents with respect to the substrate (26) without adversely affecting the progress of the reaction, and in order to obtain the target compound in good yield, the base is preferably used in an amount within a range of 0.5 equivalents to 3 equivalents, and more preferably 1 equivalent to 1.5 equivalents.

Any solvent that is not harmful to the reaction can be used as the solvent to be used in the present reaction, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether,

diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the base to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the alcohol derivative (26), each isomeric alcohol derivative (26) obtained by performing step-2, step-3, step-4, step-5, step-6, step-15 and step-10, or step-2, step-3, step-4, step-8 and step-14, or step-2, step-3, step-4, step-9 and step-14, or step-2, step-3, step-4, step-5, step-11, step-12, step-13 and step-14 using, as a raw material, 1S,2R-(5) and 1R,2S-(5) obtained by performing the asymmetric reaction in step-1.

[Production Method 18]

[Chem. 22]

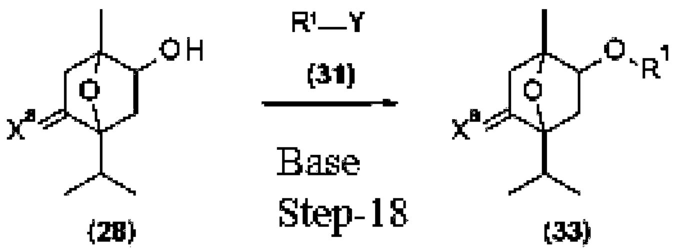


(28) (33)

($R^1$, $X^a$, and Y have the same meanings as above.)

Step-18 is a step of producing a 1,4-cineole derivative (33) by reacting an alcohol derivative represented by a formula (28) (hereinafter also referred to as substrate (28)) with the compound represented by the formula (31).

The reaction substrate (31) can be used in an amount within a range of 0.5 equivalents to 5 equivalents with respect to the substrate (28) without adversely affecting the progress of the reaction, and in order to obtain the target compound in good yield, the reaction substrate (31) is preferably used in an amount within a range of 1 equivalent to 3 equivalents, and more preferably within a range of 1.1 equivalents to 1.5 equivalents.

The present reaction may be performed in the presence of a base, and examples of the base that can be used include organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, and N-methylimidazole; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium amide, butyllithium, tert-butyllithium, sec-butyllithium, lithium diisopropylamide, trimethylsilyllithium, sodium hexamethyldisilazide, potassium hexamethyldisilazide, lithium diisopropylamide, trimethylsilyllithium, and lithium hexamethyldisilazide. The base can be used in an amount within a range of 0.1 equivalents to 10 equivalents with respect to the substrate (28) without adversely affecting the progress of the reaction, and in order to obtain the target compound in good yield, the base is preferably used in an amount within a range of 0.5 equivalents to 3 equivalents, and more preferably 1 equivalent to 1.5 equivalents.

Any solvent that is not harmful to the reaction can be used as the solvent to be used in the present reaction, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone;

alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the base to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the alcohol derivative (28), each isomeric alcohol derivative (28) obtained by performing step-2, step-3, step-4, step-5, step-6 and step-15, or step-2, step-3, step-4, step-5, step-7 and step-15 using, as a raw material, 1S,2R-(5) and 1R,2S-(5) obtained by performing the asymmetric reaction in step-1.

[Production Method 19]

[Chem. 23]

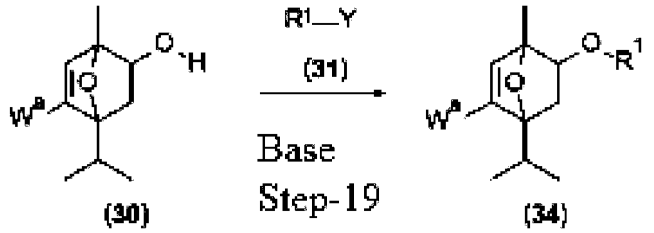


($R^1$, $W^a$, and Y have the same meanings as above.)

Step-19 is a step of producing a 1,4-cineole derivative (34) by reacting an alcohol derivative represented by a formula (30) (hereinafter also referred to as substrate (30)) with the compound represented by the formula (31).

The reaction substrate (31) can be used in an amount within a range of 0.5 equivalents to 5 equivalents with respect to the substrate (30) without adversely affecting the progress of the reaction, and in order to obtain the target compound in good yield, the reaction substrate (31) is preferably used in an amount within a range of 1 equivalent to 3 equivalents, and more preferably within a range of 1.1 equivalents to 1.5 equivalents.

The present reaction may be performed in the presence of a base, and examples of the base that can be used include organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, and N-methylimidazole; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium amide, butyllithium, tert-butyllithium, sec-butyllithium, lithium diisopropylamide, trimethylsilyllithium, sodium hexamethyldisilazide, potassium hexamethyldisilazide, lithium diisopropylamide, trimethylsilyllithium, and lithium hexamethyldisilazide. The base can be used in an amount within a range of 0.1 equivalents to 10 equivalents with respect to the substrate (30) without adversely affecting the progress of the reaction, and in order to obtain the target compound in good yield, the base is preferably used in an amount within a range of 0.5 equivalents to 3 equivalents, and more preferably 1 equivalent to 1.5 equivalents.

Any solvent that is not harmful to the reaction can be used as the solvent to be used in the present reaction, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the base to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the alcohol derivative (30), each isomeric alcohol derivative (30) obtained by performing step-2, step-3, step-4, step-5, step-11, step-12, and step-16 using, as a raw material, 1S,2R-(5) and 1R,2S-(5) obtained by performing the asymmetric reaction in step-1.

[Production Method 20]

[Chem. 24]

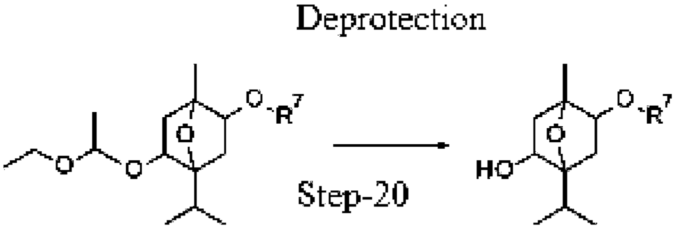


(18) (35)

($R^7$ has the same meaning as above.)

Step-20 is a step of producing an alcohol derivative represented by a formula (35) by deprotecting the ether derivative represented by the formula (18) (hereinafter also referred to as substrate (18)) in the presence of an acid.

Examples of the acid that can be used in the present reaction include organic acids and inorganic acids such as hydrochloric acid, sulfuric acid, nitric acid, acetic acid, trifluoroacetic acid, p-tosylic acid, pyridinium p-toluenesulfonate, methanesulfonic acid, trifluoromethanesulfonic acid, camphorsulfonic acid, benzenesulfonic acid, fluorosulfonic acid, chloric acid, bromic acid, iodic acid, perbromic acid, thiocyanic acid, metaperiodic acid, hexafluorophosphoric acid, tetrafluoroboric acid, chlorobenzoic acid, and fluorobenzoic acid. The acid can be used in an amount within a range of 0.1 equivalents to 10 equivalents with respect to the substrate (18) without adversely affecting the progress of the reaction, and in order to obtain the target compound in good yield, the acid is preferably used in an amount within a range of 0.5 equivalents to 5 equivalents, and more preferably 1 equivalent to 3 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of 0°C to 200°C, depending on the acid to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the ether derivative (18), each isomeric ether derivative (18) obtained by performing step-2, step-3, step-4, step-9, step-14 and step-17 using, as a raw material, 1S,2R-(5) and 1R,2S-(5) obtained by performing the asymmetric reaction in step-1.

[Production Method 21]

[Chem. 25]

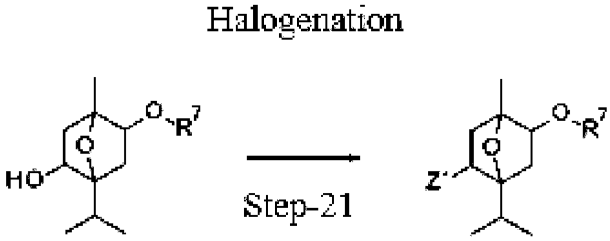


(35) (36)

($R^7$ has the same meaning as above. Z' represents a halogen atom.)

Examples of the halogen atom represented by Z' include fluorine, chlorine, bromine and iodine.

Step-21 is a step of producing a 1,4-cineole derivative represented by a formula (36) by halogenating an alcohol derivative represented by a formula (35) (hereinafter also referred to as substrate (35)).

Examples of a halogenating reagent that can be used in the present reaction include fluorinating reagents such as DAST, bromine trifluoride, and cesium fluoride; chlorinating reagents such as chlorine, thionyl chloride, oxalyl chloride, phosphorus pentachloride, phosphoryl chloride, N-chlorosuccinimide, 1,3-dichloro-5,5-dimethylhydantoin, and trichloroisocyanuric acid; brominating reagents such as bromine, thionyl bromide, oxalyl bromide, phosphorus pentabromide, phosphoryl bromide, N-bromosuccinimide, N-bromoacetamide, 1,3-dibromo-5,5-dimethylhydantoin, and dibromoisocyanuric acid; and iodinating reagents such as iodine, N-iodosuccinimide, 1,3-diiodo-5,5-dimethylhydantoin, and N-iodosaccharin. The halogenating agent can be used in an amount within a range of 0.1 equivalents to 10 equivalents with respect to the substrate (35) without adversely affecting the progress of the reaction, and in order to obtain a target compound in good yield, the halogenating agent is preferably used in an amount within a range of 0.5 equivalents to 3 equivalents, and more preferably 1 equivalent to 1.5 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; pyridine, dimethyl sulfoxide, water, and mixtures thereof.

The reaction can be performed at a temperature appropriately selected from a range of 0°C to 200°C, depending on the halogenating agent to be used and reaction conditions, and a range of 20°C to 100°C is preferred in terms of good yield. After the reaction is complete, the target compound can be obtained by an ordinary post-treatment operation, and if necessary, the target compound can be purified by column chromatography or recrystallization.

In the present step, each isomer can be obtained with stereochemistry thereof retained by using, instead of the alcohol derivative (35), each isomeric alcohol derivative (35) obtained by performing step-2, step-3, step-4, step-9, step-14, step-17, and step-20 using, as a raw material, 1S,2R-(5) and 1R,2S-(5) obtained by performing the asymmetric reaction in step-1.

[Production Method 22]

[Chem. 26]

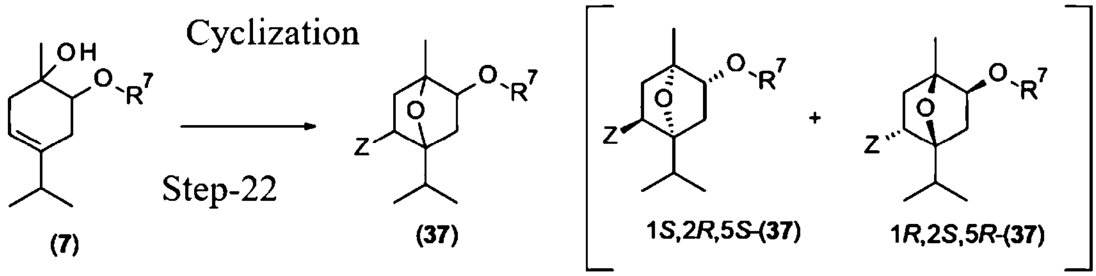


(7) (37) 1*S*,2*R*,5*S*-(37) 1*R*,2*S*,5*R*-(37)

($R^7$ and Z have the same meaning as above.)

Step-22 is a step of producing a 1,4-cineole derivative (37) by reacting the olefin derivative represented by the formula (7) (hereinafter also referred to as substrate (7)) with a halogenating agent to cause intramolecular cyclization.

Examples of the halogenating agent to be used in the present reaction include chlorinating reagents such as chlorine, thionyl chloride, oxalyl chloride, phosphorus pentachloride, phosphoryl chloride, N-chlorosuccinimide, 1,3-dichloro-5,5-dimethylhydantoin, and trichloroisocyanuric acid; brominating reagents such as bromine, thionyl bromide, oxalyl bromide, phosphorus pentabromide, phosphoryl bromide, N-bromosuccinimide, N-bromoacetamide, 1,3-dibromo-5,5-dimethylhydantoin, and dibromoisocyanuric acid; and iodinating reagents such as iodine, N-iodosuccinimide, 1,3-diiodo-5,5-dimethylhydantoin, and N-iodosaccharin. Among the halogenating agent, chlorine, N-chlorosuccinimide, bromine, N-bromosuccinimide, 1,3-dibromo-5,5-dimethylhydantoin, iodine, and N-iodosuccinimide are preferred in terms of good yield. The halogenating agent can be used in an amount within a range of 0.1 equivalents to 10 equivalents with respect to the substrate (7) without adversely affecting the progress of the reaction, and in order to obtain the target compound in good yield, the halogenating agent is preferably used in an amount within a range of 0.5 equivalents to 5 equivalents, and more preferably 1 equivalent to 3 equivalents.

In the present reaction, the target compound may be synthesized with good yield by adding an additive. Examples of the additive to be used in the present reaction include triphenylphosphine, 4-dimethylaminopyridine, sodium hydrogen carbonate, potassium hydrogen carbonate, sodium carbonate, and potassium carbonate. Among the additive, triphenylphosphine is preferred in terms of good yield. The additive can be used in an amount within a range of 0.01 mol% to 60 mol% with respect to the substrate (7) without adversely affecting the progress of the reaction, and in order to obtain the target compound in good yield, the additive is preferably used in an amount within a range of 0.05 mol% to 50 mol%, more preferably within a range of 0.1 mol% to 30 mol%, and still more preferably within a range of 1 mol% to 10 mol% with respect to the substrate (7).

The present reaction can be performed in the absence or presence of a solvent. Any solvent that is not harmful to the reaction can be used as the solvent to be used, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of -90°C to 200°C, depending on the halogenating agent to be used and reaction conditions, and a range of -10°C to 50°C is preferred in terms of good yield. After the reaction is complete, the target compound can be obtained by an ordinary post-treatment operation, and if necessary, the target compound can be purified by column chromatography or recrystallization.

In the present step, two isomers can also be obtained individually with stereochemistry thereof retained by using, instead of the olefin derivative (7), the two isomeric olefin derivatives (7) obtained by performing step-2 using, as a raw material, 1S,2R-(5) and 1R,2S-(5) obtained by performing the asymmetric reaction in step-1.

[Production Method 23]

[Chem. 27]

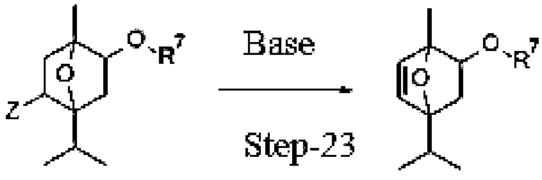


(37) (38)

($R^7$ and Z have the same meaning as above.)

Step-23 is a step of producing an olefin derivative (38) by reacting a 1,4-cineole derivative represented by a formula (37) (hereinafter also referred to as substrate (37)) with a base.

Examples of the base to be used in the present reaction include organic bases such as triethylamine, diisopropylethylamine, tributylamine, propylamine, butylamine, tert-butylamine, benzylamine, N-methylmorpholine, N,N-dimethylaniline, N,N-diethylaniline, 4-tert-butyl-N,N-dimethylaniline, pyridine, 4-dimethylaminopyridine, picoline, lutidine, pyrazine, imidazole, N-methylimidazole, 1,8-diazabicyclo[5.4.0]undecene, 7-methyl-1,5,7-triazabicyclo[4.4.0]dec-5-ene, and 1,5,7-triazabicyclo[4.4.0]dec-5-ene; and alkali metal salts such as sodium carbonate, potassium carbonate, sodium bicarbonate, potassium bicarbonate, sodium acetate, potassium acetate, sodium methoxide, sodium ethoxide, potassium tert-butoxide, sodium hydride, potassium hydride, sodium amide, butyllithium, tert-butyllithium, sec-butyllithium, lithium diisopropylamide, trimethylsilyllithium, sodium hexamethyldisilazide, potassium hexamethyldisilazide, lithium diisopropylamide, trimethylsilyllithium, and lithium hexamethyldisilazide. Among the base, potassium tert-butoxide, sodium hydride, and 1,8-diazabicyclo[5.4.0]undecene are preferred in terms of good yield. The base can be used in an amount within a range of 0.1 equivalents to 10 equivalents with respect to the substrate (37) without adversely affecting the progress of the reaction, and in order to obtain the target compound in good yield, the base is preferably used in an amount within a range of 0.5 equivalents to 3 equivalents, and more preferably 1 equivalent to 1.5 equivalents.

The present reaction is preferably performed in a solvent. Any solvent that is not harmful to the reaction can be used as the solvent, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of -90°C to 200°C, depending on the base to be used and reaction conditions, and a range of 0°C to 100°C is preferred in terms of good yield. After the reaction is complete, the target compound can be obtained by an ordinary post-treatment operation, and if necessary, the target compound can be purified by column chromatography or recrystallization.

In the present step, two isomers can also be obtained individually with stereochemistry thereof retained by using, instead of the 1,4-cineole derivative (37), the two isomeric olefin derivatives (37) obtained by performing step-2 and step-22 using, as a raw material, 1S,2R-(5) and 1R,2S-(5) obtained by performing the asymmetric reaction in step-1.

[Production Method 24]

[Chem. 28]

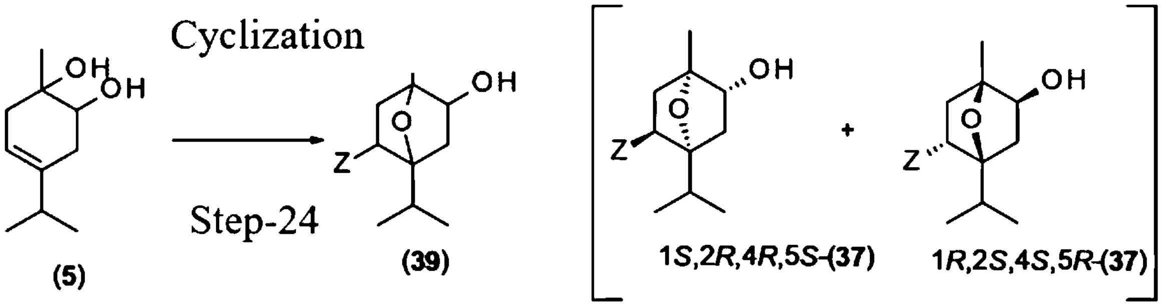


(5) (39) 1S,2R,4R,5S-(37) 1R,2S,4S,5R-(37)

(Z has the same meaning as above.)

Step-24 is a step of producing a 1,4-cineole derivative (39) by reacting the diol derivative represented by the formula (5) (hereinafter also referred to as substrate (5)) with a halogenating agent to cause intramolecular cyclization.

Examples of the halogenating agent to be used in the present reaction include chlorinating reagents such as chlorine, thionyl chloride, oxalyl chloride, phosphorus pentachloride, phosphoryl chloride, N-chlorosuccinimide, 1,3-dichloro-5,5-dimethylhydantoin, and trichloroisocyanuric acid; brominating reagents such as bromine, thionyl bromide, oxalyl bromide, phosphorus pentabromide, phosphoryl bromide, N-bromosuccinimide, N-bromoacetamide, 1,3-dibromo-5,5-dimethylhydantoin, and dibromoisocyanuric acid; and iodinating reagents such as iodine, N-iodosuccinimide, 1,3-diiodo-5,5-dimethylhydantoin, and N-iodosaccharin. Among the halogenating agent, chlorine, N-chlorosuccinimide, bromine, N-bromosuccinimide, N-bromoacetamide, 1,3-dibromo-5,5-dimethylhydantoin, iodine, and N-iodosuccinimide are preferred in terms of good yield. The halogenating agent can be used in an amount within a range of 0.1 equivalents to 10 equivalents with respect to the substrate (5) without adversely affecting the progress of the reaction, and in order to obtain the target compound in good yield, the halogenating agent is preferably used in an amount within a range of 0.5 equivalents to 5 equivalents, and more preferably 1 equivalent to 3 equivalents.

In the present reaction, the target compound may be synthesized with good yield by adding an additive. Examples of the additive to be used in the present reaction include triphenylphosphine, 4-dimethylaminopyridine, sodium hydrogen carbonate, potassium hydrogen carbonate, sodium carbonate, and potassium carbonate. Among the additive, triphenylphosphine and 4-dimethylaminopyridine are preferred in terms of good yield. The additive can be used in an amount within a range of 0.01 mol% to 60 mol% with respect to the substrate (5) without adversely affecting the progress of the reaction, and in order to obtain the target compound in good yield, the additive is preferably used in an amount within a range of 0.05 mol% to 50 mol%, more preferably within a range of 0.1 mol% to 30 mol%, and still more preferably within a range of 1 mol% to 10 mol% with respect to the substrate (5).

The present reaction can be performed in the absence or presence of a solvent. Any solvent that is not harmful to the reaction can be used as the solvent to be used, and examples of the solvent that can be used include aromatic hydrocarbon solvents such as benzene, toluene, xylene, and chlorobenzene; aliphatic hydrocarbon solvents such as pentane, hexane, and octane; ether solvents such as diethyl ether, diisopropyl ether, cyclopentyl methyl ether, tetrahydrofuran, dimethoxyethane, and 1,4-dioxane; ketone solvents such as acetone, methyl ethyl ketone, and cyclohexanone; halogenated solvents such as chloroform and dichloromethane; nitrile solvents such as acetonitrile and propionitrile; ester solvents such as ethyl acetate, propyl acetate, butyl acetate, and methyl propionate; amide solvents such as N,N-dimethylformamide, N,N-dimethylacetamide, and N-methylpyrrolidone; alcohol solvents such as methanol, ethanol, 1-propanol, 2-propanol, and tert-butanol; dimethyl sulfoxide, water, and mixtures thereof.

The reaction can be performed at a temperature appropriately selected from a range of -90°C to 200°C, depending on the halogenating agent to be used and reaction conditions, and a range of -10°C to 50°C is preferred in terms of good yield. After the reaction is complete, the target compound can be obtained by an ordinary post-treatment operation, and if necessary, the target compound can be purified by column chromatography or recrystallization.

In the present step, two isomers can also be obtained individually with stereochemistry thereof retained by using, instead of the diol derivative (5), 1S,2R-(5) and 1R,2S-(5) obtained by performing the asymmetric reaction in step-1 as a raw material.

The compound of the present invention and the intermediate of the present invention can be analyzed, confirmed, and identified by melting point, infrared absorption spectrum, $^{1}$H-NMR, $^{13}$C-NMR, mass spectrometry, X-ray structural analysis, or the like, as necessary.

The compound of the present invention can be produced by any organic synthesis method without being limited to the above-mentioned method.

The present invention relates to a herbicide containing the compound of the present invention as an active ingredient (hereinafter also referred to as "herbicide of the present invention").

The herbicide of the present invention is preferably for use in agricultural land, pastureland, lawn, or non-agricultural land.

Examples of the agricultural land include a field, a paddy field, an orchard, fallow land, and uncultivated land.

As will be shown in Test Examples to be described later, the compound of the present invention exhibit excellent herbicidal activity and can be used against a wide range of weeds, including those in paddy rice cultivation and upland field cultivation. Specific examples of the weeds include the following.

Specifically, the compound of the present invention can control various harmful weeds, for example, gramineae weeds such as Echinochloa crus-galli, Echinochloa oryzicola, Echinochloa crus-galli var. formosensis, Digitaria ciliaris (Digitaria sanguinalis, Digitaria ischaem, Digitaria adscendens, Digitaria microbachne, Digitaria horizontalis), Setaria viridis, Setaria faberi, Setaria lutescens, Eleusine indica, Avena fatua, Sorghum halepense, Aropyron repens, Brachiaria plantaginea, Panicum maximum, Panicum purpurascens, Panicum dichotomiflorum, Leptochloa chinensis, Leptochloa panicea, Poa annua, Alopecurus aequalis, Alopecurus myosuroides, Agropyron tsukushiense, Brachiaria platyphylla, Cenchrus echinatus, Lolium multiflorum, Cynodon dactylon, Beckmannia syzigache, Bromus catharticus, Leersia japonica, Leersia sayanuka, Lolium rigidum, Paspalum distichum, and Phleum pratense; cyperaceae weeds such as Cyperus iria, Cyperus rotundus, Cyperus esculentus, Scirpus hotarui, Cyperus serotinus, Eleocharis acicularis, Eleocharis kuroguwai, Cyperus flaccidus, Kyllinga brevifolia, and Scirpus juncoides; alismataceae weeds such as Sagittaria pygmaea, Sagittaria trifolia, and Alisma canaliculatum; pontderiaceae weeds such as Monochoria vaginalis, Heteranthera limosa, and Monochoria kosakowii; linderniaceae weeds such as Lindernia pyxidaria; plantaginaceae weeds such as Plantago asiatica, Gratiola japonica, Dopatrium junceum, and Veronica polita; lythraceae weeds such as Rotala india and Ammannia multifflora; elatinaceae weeds such as Elatine triandra; malvaceae weeds such as Abutiol theophrsti and Sida spinosa; compositae weeds such as Xanthium strumarim, Ambrosia elatior, Breea serosa, Galinsoga ciliata, Matricaria chamomilla, Taraxacum officinale, Erigeron canadensis, Bidens frondosa, Bidens pilosa, Bidens tripartita, Gnaphalium affine, and Senecio vulgaris; lamiaceae weeds such as Lamium amplexinale weber; solanaceae weeds such as Solanum nigrum and Datura stramonium; amaranthaceae weeds such as Amaranthus viridis, Chenopodium album, Kochia scoparia, and Amaranthus hybridus; polygonaceae weeds such as Polygonum lapathifolium, Polygonum persicaria, Polygonum convolvulus, Polygonum aviculare, Persicaria longiseta, and Persicaria nepalensis; crpurea weeds such as Cardamine flexuosa, Capsella bursapastoris, Brassica juncea, and Rorippa indica; convolvulaceae weeds such as Ipomoea purpurea, Convolvulus arvensis, Ipomoea hederacea, Calystegia pubescens, and Ipomoea coccinea; portulacaceae weeds such as portulaca aleracea; fabaceae weeds such as Cassia obtusifolia, Aeschynomene indica, Sesbania exaltata, Trifolium repens, and Vicia sativa; caryophyllaceae weeds such as Stellaria media, Stellaria neglecta, and Stellaria uliginosa; euphorbiaceae weeds such as Euphorbia helioscopia and Acalypha australis; commelinaceae weeds such as Commelina communis and Murdannia keisak; potamogetonaceae weeds such as Potamogeton distinctus; araceae weeds such as Spirodela polyrhiza; cucurbitaceae weeds such as Sicyos angulatus; rubiaceae weeds such as Galium spurium; apiaceae weeds such as Oenanthe javanica; violaceae weeds such as Viola mandshuria; onagraceae weeds such as Ludwigia epilobioides) and Oenothera odorata; oxalidaceae weeds such as Oxalis corniculata; equisetaceae weeds such as Equisetum arvense; and Zygnemataceae weeds such as Spirogyra sp. Therefore, the compound of the present invention can be effectively used to control harmful weeds in the cultivation of useful crops such as Oryza sativa L., Zea mays, Glycine max, Gossypium spp., Triticum spp., Hordeum vulgare, Secale cereale, Avena sativa, Sorghum bicolor, Brassica napus, Helianthus annuus, Beta vulgaris, Saccharum officinarum, Zoysia japonica, Arachis hypogaea, Linum usitatissimum, Nicotiana tabacum, and Coffea spp.

The application of the herbicide of the present invention is not limited to the weeds and crops exemplified above.

The compound of the present invention may be mixed with other herbicides, various insecticides, acaricides, nematicides, fungicides (fungicides, bactericides, antivirals, plant resistance inducers), bird repellents, plant growth regulators, phytotoxicity reducers (safeners), fertilizers, soil improvers, synergists, and the like during formulation or during spraying, as required, or may be mixed and applied in a tank mix at the time of spraying.

Particularly, by applying the compound of the present invention in combination with other herbicides, the amount of the herbicide to be used can be reduced, resulting in labor savings. In addition, it can be expected that an application target of the herbicide (herbicidal spectrum) is expanded by the cooperative action of the two agents, and further, a stronger effect is obtained by the synergistic action of the two agents. In this case, a plurality of known herbicides and phytotoxicity reducers (safeners) can also be used in combination.

Among the optional ingredients, representative examples of the herbicide are shown below, but are not limited thereto.

(1) A compound that exhibits herbicidal activity by disturbing a hormone effect of a plant, such as phenoxy compounds such as 2,4-D, 2,4-D-butotyl, 2,4-D-butyl, 2,4-D-dimthylammonium, 2,4-D-diolamine, 2,4-D-ethyl, 2,4-D-2-ethylhexyl, 2,4-D-isobutyl, 2,4-D isoctyl, 2,4-D-isopropyl, 2,4-D-isopropylammonium, 2,4-D-sodium, 2,4-D-isopropanolammonium, 2,4-D-trolamine, 2,4-DB, 2,4-DB-butyl, 2,4-DB-dimethylammonium, 2,4-DB-isoctyl, 2,4-DB-potassium, 2,4-DB-sodium, a 2,4-D chloinesalt, dichlorprop, dichlorprop-butotyl, dichlorprop-dimethylammonium, dichlorprop-isooctyl, dichlorprop-potassium, dichlorprop-P, dichlorprop-P-dimethylammonium, dichlorprop-P-potassium, dichlorprop-P-sodium, MCPA, MCPA-butotyl, MCPA-dimethylammonium, MCPA-2-ethylhexyl, MCPA-potassium, MCPA-sodium, MCPA-thioethyl, MCPB, MCPB-ethyl, MCPB-sodium, mecoprop, mecoprop-butotyl, mecoprop-sodium, mecoprop-P, mecoprop-P-butotyl, mecoprop-P-dimethylammonium, mecoprop-P-2-ethylhexyl, mecoprop-P-potassium, naproanilide, clomeprop, and HIA-1; aromatic carboxylic acid compounds such as 2,3,6-TBA, dicamba, dicamba-butotyl, dicamba-diglycolamine, dicamba-dimethylammonium, dicamba-diolamine, dicamba-isopropylammonium, dicamba-potassium, dicamba-sodium, picloram, picloram-dimethylammonium, picloram-isoctyl, picloram-potassium, picloram-triisopropanolammonium, picloram-triisopropylammonium, picloram-trolamine, tricolopyr, tricolopyr-butotyl, tricolopyr-triethylammonium, clopyralid, clopyralid-olamine, clopyralid-potassium, clopyralid-triisopropanolammonium, aminopyralid, aminocyclopyrachlor, aminocyclopyrachlor, halauxifen, florpyrauxifen, halauxifen-methyl, and DAS-534; and other compounds such as naptalam, naptalam-sodium, benazolin, benazolin-ethyl, quinclorac, quinmerac, diflufenzopyr, diflufenzopyr-sodium, fluroxypyr, fluroxypyr-2-butoxy-1-methylethyl, fluroxypyr-meptyl, chlorflurenol, chlorflurenol-methyl, clacyfos, fluchloraminopyr, and indolauxipyr.

(2) A compound that exhibits herbicidal activity by inhibiting photosynthesis of a plant, such as urea compounds such as chlorotoluron, diuron, fluometuron, linuron, isoproturon, metobenzuron, tebuthiuron, dimefuron, isouron, karbutilate, methabenzthiazolone, metoxuron, metoburomuron, monolinuron, neburon, siduron, terbumeton, and trietazine; triazine compounds such as simazine, atrazine, atratone, simetryn, prometryn, dimethametryn, hexazinone, metribuzin, terbuthylazine, cyanazine, ametryn, cybutryne, terbutryn, propazine, metamitron, and prometon; uracil compounds such as bromacil, bromacyl-lithium, lenacil, and terbacil; anilide compounds such as propanil and cypromid; carbamate compounds such as swep, desmedipham, and phenmedipham; hydroxybenzonitrile compounds such as bromoxynil, bromoxynil-octanoate, bromoxynil-heptanoate, ioxynil, ioxynil-octanoate, ioxynil-potassium, and Ioxynil-sodium; and other compounds such as pyridate, bentazone, bentazone-sodium, amicarbazone, methazole, pentanochlor, and phenmedipham.

(3) A quaternary ammonium salt compound such as paraquat and diquat, which themselves are thought to become free radicals in a plant and generate active oxygen, thereby exhibiting a rapid-acting herbicidal effect.

(4) A compound that inhibits chlorophyll biosynthesis of a plant and exhibits herbicidal activity by abnormally accumulating a photoenhanced peroxide in the plant, such as diphenyl ether compounds such as nitrofen, chlomethoxyfen, bifenox, acifluorfen, acifluorfen-sodium, fomesafen, fomesafen-sodium, oxyfluorfen, lactofen, aclonifen, ethoxyfen-ethyl, fluoroglycofen-ethyl, fluoroglycofen; cyclic imide compounds such as chlorphthalim, flumioxazin, flumiclorac, flumiclorac-pentyl, cinidon-ethyl, fluthiacet-methyl, and EK-5385; and other compounds such as oxadiargyl, oxadiazon, sulfentrazone, carfentrazone-ethyl, thidiazimin, pentoxazone, azafenidin, isopropazole, pyraflufen-ethyl, benzfendizone, butafenacil, saflufenacil, fluazolate, profluazol, flufenpyr-ethyl, bencarbazone, tiafenacil, pyrachlonil, cyclopyranil, epyrifenacil, trifludimoxazin, flufenoximacil, HNPC-B4047, IR-6396, EK-5498, SYN-523, and compounds described in WO2008/008763 (FMC).

(5) A compound that inhibits biosynthesis of a dye of a plant such as a carotenoid and exhibits herbicidal activity characterized by a whitening action, such as pyridazinone compounds such as norflurazon, chloridazon, and metflurazon; pyrazole compounds such as pyrazolynate, pyrazoxyfen, benzofenap, topramezone, pyrasulfotole, tolpyralate, tripyrasulfone, fenpyrazone, and bipyrazone; and other compounds such as amitrole, fluridone, flurtamone, diflufenican, methoxyphenone, clomazone, bixlozone, sulcotrione, mesotrione, tembotrione, tefuryltrione, fenquinotrione, lancotrione, dioxopyritrione, benquitrione, cyclopyrimorate, isoxaflutole, difenzoquat, difenzoquat-metilsulfate, isoxachlortole, benzobicyclon, bicyclopyron, picolinafen, beflubutamid, beflubutamid-M, ketospiradox, ketospiradox-potassium, iptriazopyrid, flusulfinam, broclozone, pyraquinate, and compounds described in JP2012/2571 (Sumitomo Chemical Co., Ltd.).

(6) A compound that inhibits biosynthesis of fatty acid and exhibits herbicidal activity on a plant, such as arixyphenoxypropionic acid compounds such as diclofop-methyl, diclofop, pyriphenop-sodium, fluazifop-butyl, fluazifop, fluazifop-P, fluazifop-P-butyl, haloxyfop, haloxyfop-etotyl, haloxyfop-P, haloxyfop-P-methyl, quizalofop-ethyl, quizalofop-P, quizalofop-P-ethyl, quizalofop-P-tefuryl, cyhalofop-butyl, fenoxaprop-ethyl, fenoxaprop-P, fenoxaprop-P-ethyl, metamifop-propyl, metamifop, clodinafop-propargyl, propaquizafop, HNPC-A8169, SYP-1924; cyclohexanedione compounds such as alloxydim-sodium, alloxydim, clethodim, sethoxydim, tralkoxydim, butroxydim, tepraloxydim, profoxydim, cycloxydim; and phenylpyrazoline compounds such as pinoxaden.

(7) A compound that exhibits herbicidal activity by inhibiting biosynthesis of an amino acid of a plant, such as sulfonylurea compounds such as chlorimuron-ethyl, chlorimuron, sulfometuron-methyl, sulfometuron, primisulfuron-methyl, primisulfuron, bensulfuron-methyl, bensulfuron, chlorsulfuron, metsulfuron-methyl, metsulfuron, cinosulfuron, pyrazosulfuron-ethyl, pyrazosulfuron, flazasulfuron, rimsulfuron, nicosulfuron, imazosulfuron, flucetosulfuron, cyclosulfamuron, prosulfuron, flupyrsulfuron-methyl-sodium, flupyrsulfuron, triflusulfuron, triflusulfuron-methyl, halosulfuron-methyl, halosulfuron, thifensulfuron-methyl, thifensulfuron, ethoxysulfuron, oxasulfuron, ethametsulfuron, ethametsulfuron-methyl, iodosulfuron, iodosulfuron-methyl-sodium, sulfosulfuron, triasulfuron, tribenuron-methyl, tribenuron, tritosulfuron, foramsulfuron, trifloxysulfuron, trifloxysulfuron-sodium, mesosulfuron-methyl, mesosulfuron, orthosulfamuron, amidosulfuron, azimsulfuron, propyrisulfuron, metazosulfuron, methiopyrsulfuron, monosulfuron-methyl, orsosulfuron, iofensulfuron, and iofensulfuron-sodium; triazolopyrimidine sulfonamide compounds such as flumetsulam, metosulam, diclosulam, cloransulam-methyl, florasulam, penoxsulam, and pyroxsulam; imidazolinone compounds such as imazapyr, imazapyr-isopropylammonium, imazethapyr, imazethapyr-ammonium, imazaquin, imazaquin-ammonium, imazamox, imazamox-ammonium, imazamethabenz, imazamethabenz-methyl, and imazapic; pyrimidinylsalicylic acid compounds such as pyrithiobac-sodium, bispyribac-sodium, pyriminobac-methyl, pyribenzoxim, pyriftalid, pyrimisulfan, and triafamone; sulfonylaminocarbonyltriazolinone compounds such as flucarbazone, flucarbazone- sodium, propoxycarbazone-sodium, propoxycarbazone, and thiencarbazone-methyl; and other compounds such as glyphosate, glyphosate-sodium, glyphosate-potassium, glyphosate-ammonium, glyphosate-isopropylammonium, glyphosate-trimesium, glyphosate-sesquisodium, glufosinate, glufosinate-ammonium, glufosinate-P, glufosinate-P-ammonium, glufosinate-P-sodium, bilanafos, bilanafos-sodium, and cinmethylin.

(8) A compound that exhibits herbicidal activity by inhibiting cell fission of a plant, such as dinitroaniline compounds such as trifluralin, oryzalin, nitralin, pendimethalin, ethalfluralin, benfluralin, prodiamine, butralin, and dinitramine; amide compounds such as bensulide, napropamide, napropamide-M, propyzamide, and pronamide; organophosphate compounds such as amiprofos-methyl, butamifos, anilofos, and piperophos; phenylcarbamate compounds such as propham, chlorpropham, barban, and carbetamide; cumylamine compounds such as daimuron, cumyluron, bromobutide, and methyldymron; and other compounds such as asulam, asulam-sodium, dithiopyr, thiazopyr, chlorthal-dimethyl, chlorthal, diphenamid, flamprop-M-methyl, flamprop-M, and flamprop-M-isopropyl.

(9) A compound that exhibits herbicidal activity by inhibiting plant protein biosynthesis or lipid biosynthesis, such as chloroacetamide compounds such as alachlor, metazachlor, butachlor, pretilachlor, metolachlor, S-metolachlor, thenylchlor, pethoxamid, acetochlor, propachlor, dimethenamide, dimethenamide-P, propisochlor, and dimethachlor; thiocarbamate compounds such as molinate, dimepiperate, pyributicarb, EPTC, butylate, vernolate, cycloate, prosulfocarb, esprocarb, thiobencarb, diallate, tri-allate, orbencarb; and other compounds such as etobenzanid, mefenacet, flufenacet, tridiphane, cafenstrole, fentrazamide, ipfencarbazone, oxaziclomefone, indanofan, benfuresate, pyroxasulfone, fenoxasulfone, methiozolin, dalapon, dalapon-sodium, TCA-sodium, and trichloroacetic acid.

(10) A compound that exhibits herbicidal activity by inhibiting cellulose biosynthesis of a plant, such as dichlobenil, triaziflam, indaziflam, flupoxam, and isoxaben.

(11) Other herbicides such as icafolin, tetflupyrolimet, dimesulfazet, rimisoxafen, MSMA, DSMA, CMA, endothall, endothall-dipotassium, endothall-sodium, endothall-mono(N,N-dimethylalkylammonium), ethofumesate, sodium chlorate, pelargonic acid, nonanoic acid, fosamine, fosamine-ammonium, aclolein, ammonium sulfamate, borax, chloroacetic acid, sodium chloroacetate, cyanamide, methylarsonic acid, dimethylarsonic acid, sodium dimethylarsonate, dinoterb, dinoterb-ammonium, dinoterb-diolamine, dinoterb-acetate, DNOC, ferrous sulfate, flupropanate, flupropanate-sodium, mefluidide, mefluidide-diolamine, metam, metam-ammonium, metam-potassium, metam-sodium, methyl isothiocyanate, pentachlorophenol, sodium pentachlorophenoxide, pentachlorophenol laurate, quinoclamine, sulfuric acid, urea sulfate, zanthinosin, herbimycin, unguinol, metatyrosine, sarmentine, thaxtomin A, mevalocidin, alpha-limonene, pyribambenz-propyl, pyribambenz-isopropyl, pyriflubenzoxime, cypyrafluone, JS-913, KHG-23844, H-9201, SIOC-0163, SIOC-0171, SIOC-0172, SIOC-0285, SIOC-0426, SIOC-H-057, ZJ-0166, ZJ-1835, ZJ-0453, ZJ-0777, ZJ-0862, and compounds described in WO2008/096398 (Kumiai Chemical Industry Co.).

(12) Those that exhibit herbicidal activity by parasitizing a plant, such as Xanthomonas campestris, Epicoccosirus nematosorus, Epicoccosirus nematosperus, Exserohilum monoseras, and Drechslera monoceras.

When the compound of the present invention is used as a herbicide, the compound of the present invention can be used as it is, and the compound of the present invention can also be used after being formulated. In the formulation, suitable carriers, adjuvants, surfactants, binders, stabilizers, and the like described in Pesticide Formulation Guide (edited by the Pesticide Science Society of Japan Application Method Research Group, published by the Japan Plant Protection Association, 1997) may be blended.

The herbicide containing the compound of the present invention can be formulated into any type generally used in the form of, but is not limited to, a granule, a microgranule, a fine granule, a wettable powder, a water dispersible granule (dry flowable formulation), an emulsifiable concentrate, a soluble powder, a suspension concentrate (flowable formulation), a liquid formulation, a dustable powder, a coarse powder, a drift-less (DL) dustable powder, a flowable dust, an oil solution, an microcapsule suspension, a paste formulation, and a jumbo formulation.

In the formulation, any carrier, whether solid or liquid, commonly used for agricultural chemical formulations can be used. Such a carrier is not limited to a particular one, but specific examples include the following. Examples of the solid carrier include a mineral powder (kaolin, bentonite, clay, montmorillonite, talc, diatomaceous earth, mica, vermiculite, quartz, calcium carbonate, apatite, white carbon, hydrated lime, silica sand, acid clay, zeolite, sepiolite, crushed expanded perlite, a shirasu balloon, an alumina balloon, and a microsphere composed of phenol resin, epoxy resin, polyacrylonitrile,

or polyurethane); a plant-based powder (soybean powder, wheat flour, wood flour, tobacco powder, starch, and crystalline cellulose); a polymeric compound (petroleum resin, polyvinyl chloride, and ketone resin); alumina; a silicate; glucose; sucrose; lactose; a sugar polymer; ammonium sulfate; sodium chloride; potassium chloride; urea; highly dispersed silicic acid; and waxes.

Examples of the liquid carrier include water; alcohols (methyl alcohol, ethyl alcohol, n-propyl alcohol, isopropyl alcohol, butanol, ethylene glycol, and benzyl alcohol); aromatic hydrocarbons (toluene, benzene, xylene, ethylbenzene, and methylnaphthalene); ethers (ethyl ether, ethylene oxide, dioxane, and tetrahydrofuran); ketone solvents (acetone, methyl ethyl ketone, cyclohexanone, methyl isobutyl ketone, and isophorone); esters (ethyl acetate, butyl acetate, ethylene glycol acetate, and amyl acetate); acid amides (dimethylformamide, and dimethylacetamide); nitriles (acetonitrile, propionitrile, and acrylonitrile); sulfoxides (dimethyl sulfoxide); glycol ethers (ethylene glycol monomethyl ether, and ethylene glycol monoethyl ether); aliphatic or alicyclic hydrocarbons (n-hexane, and cyclohexane); industrial gasoline (petroleum ether, and solvent naphtha); and petroleum fractions (paraffins, kerosene, and light oil).

When the herbicide is formulated into an emulsifiable concentrate, a wettable powder, and a flowable formulation, various surfactants are added for the purposes of emulsification, dispersion, solubilization, wetting, foaming, lubrication, and spreading. Examples of such surfactants include nonionic surfactants such as a polyoxyethylene alkyl ether, a polyoxyethylene alkyl ester, a polyoxyethylene sorbitan alkyl ester, a polyoxyethylene alkylaryl ether, a polyoxyethylene polyoxypropylene block polymer, and a polyoxyethylene styrylphenyl ether; anionic surfactants such as an alkylbenzenesulfonate, an alkyl sulfosuccinate, an alkyl sulfate, a polyoxyethylene alkyl sulfate, an arylsulfonate, an alkylnaphthalenesulfonate, a polyoxyethylenestyrylphenyl ether sulfate, a lignin sulfonate, a naphthalenesulfonic acid formaldehyde condensate, and a polycarboxylate; cationic surfactants such as an alkylamine (laurylamine and stearyltrimethylammonium chloride), a polyoxyethylene alkylamine, an alkylpyridinium salt, an alkyltrimethylammonium salt, and an alkyldimethylammonium salt; and amphoteric surfactants such as a carboxylic acid (betaine type) and a sulfate ester salt, but the present invention is not limited thereto.

In addition to these, various adjuvants and additives such as polyvinyl alcohol (PVA), carboxymethyl cellulose (CMC), gum arabic, polyvinyl acetate, sodium alginate, gelatin, tragacanth gum, dextrin, hydroxypropyl methyl cellulose (HPMC), and methyl cellulose (MC) can be used.

Preferred methods of application of the herbicide containing the compound of the present invention as an active ingredient include soil application, water surface application, stem and leaf parts application, and the like, and particularly excellent effects can be obtained by application from before weed emergence to the early seedling stage.

The amount of the compound of the present invention to be applied as a herbicide varies depending on the application site, application time, application method, target weeds, cultivated crops, and the like, and generally, the amount of active ingredient of about 0.001 kg per hectare (ha) to 10 kg per hectare (ha), and preferably about 0.01 kg per hectare (ha) to 1 kg per hectare (ha) is appropriate.

The present invention also relates to a method for using the herbicide of the present invention, which includes applying an effective amount of the compound of the present invention to at least one selected from a stem and leaf of a weed, soil, and a water surface.

The weeds are not particularly limited, and include, for example, the weeds mentioned above.

It is preferable that the soil is agricultural land such as a field or a paddy field where agricultural and horticultural plants are cultivated.

The water surface may be a water surface in waterlogged soil.

The treatment step is not particularly limited. Examples of the treatment step include a step of spraying an effective amount of the compound of the present invention onto at least one selected from a stem and leaf of a weed, soil, and a water surface.

Further, the present invention relates to a method for preparing an agrochemical composition, which includes a step of mixing a herbicide containing the compound of the present invention as an active ingredient with at least one selected from a diluent and a surfactant.

The diluent is not particularly limited, and examples thereof include natural minerals such as clay, quartz, calcite, sepiolite, dolomite, chalk, kaolin, pyrophyllite, sericite, halloysite, metahalloysite, kibushi clay, gaerome clay, pottery stone, zeclite, allophane, shirasu, mica, talc, pumice, hectorite, zeolite, and diatomaceous earth; calcined products of natural minerals such as calcined clay, perlite, a shirasu balloon, vermiculite, attapulgus clay, and calcined diatomaceous earth; inorganic salts such as magnesium carbonate, calcium carbonate, sodium carbonate, sodium bicarbonate, ammonium sulfate, sodium sulfate, magnesium sulfate, diammonium hydrogen phosphate, ammonium dihydrogen phosphate, and potassium chloride; sugars such as glucose, fructose, sucrose, and lactose; polysaccharides such as starch, powdered cellulose, and dextrin; organic substances such as urea, a urea derivative, benzoic acid, and a benzoic acid salt; plant-based materials such as wood flour, a corn cob, a walnut shell, and a tobacco stem; fly ash, white carbon, and water.

The surfactant is not particularly limited, and examples thereof include surfactants same as those described above in the formulation of the herbicide.

The method for mixing the herbicide containing the compound of the present invention as an active ingredient with at least one selected from a diluent and a surfactant is not particularly limited, and known methods can be used.

[Example]

The present invention will be described in more detail below with reference to synthesis examples, formulation examples and test examples of the compound of the present invention and the intermediate of the present invention, but the present invention is not limited thereto.
Compounds obtained in the following Synthesis Examples 3 to 5, 8 to 13, 15, 16, 18 to 21, 24, 25, 28, 29, 31, 32, 35, 37, 40, 45, 46, 49, 52, 55, 58, 61, 64, 67, 70, 73, 76, 77, 80 to 102, and 104 to 391 are the compound of the present invention, and compounds obtained in Synthesis Examples other than those mentioned above are the intermediate of the present invention.

Synthesis Example 1

Synthesis of (1R,4S,5R)-5-((tert-butyldimethylsilyl)oxy)-1-isopropyl-4-methyl-7-oxabicyclo[2.2.1]heptan-2-one

To a dichloromethane solution (5 mL) of (1R,2S,4S,5R)-5-((tert-butyldimethylsilyl)oxy)-1-isopropyl-4-methyl-7-oxabicyclo[2.2.1]heptan-2-ol (466 mg, 1.55 mmol), pyridinium chlorochromate (689 mg, 3.10 mmol) and Celite (1.34 g) were added, followed by stirring at room temperature (25°C) for 19 hours. The reaction mixture was filtered and then concentrated under reduced pressure. The concentrate was purified by silica gel column chromatography (eluent: ethyl acetate/n-hexane) to obtain a colorless liquid title compound (yield 361 mg, 78%).
$^1H$ NMR (400 MHz, $CDCl_3$) δ 0.06 (s, 3H), 0.07 (s, 3H), 0.90 (s, 9H), 1.05 (d, J = 6.8 Hz, 3H), 1.06 (d, J = 6.8 Hz, 3H), 1.47 (s, 3H), 1.65 (dd, J = 13.4, 2.4 Hz, 1H), 2.02 (dd, J = 13.4, 6.8 Hz, 1H), 2.03 (d, J = 17.2 Hz, 1H), 2.15 (sept, J = 6.8 Hz, 1H), 2.23 (d, J = 17.2 Hz, 1H), 3.93 (dd, J = 6.8, 2.4 Hz, 1H).

Synthesis Example 2

Synthesis of (1S,2R,4R)-4-isopropyl-1-methyl-5-methylene-7-oxabicyclo[2.2.1]heptan-2-ol

To a tetrahydrofuran solution (9 mL) of methyltriphenylphosphonium bromide (629 mg, 1.58 mmol), n-butyllithium (1.00 mL, 1.59 mol/L hexane solution, 1.59 mmol) was added, followed by stirring at 0 °C for 1 hour. To the reaction mixture, (1R,4S,5R)-5-((tert-butyldimethylsilyl)oxy)-1-isopropyl-4-methyl-7-oxabicyclo[2.2.1]heptan-2-one (262 mg, 0.878 mmol) was added, followed by stirring at 0°C for 2 hours. A saturated ammonium chloride aqueous solution was poured into the reaction mixture, which was then extracted with ethyl acetate. The extract was dried over anhydrous magnesium sulfate and then concentrated under reduced pressure to obtain a crude product of tert-butyl(((1S,2R,4R)-4-isopropyl-1-methyl-5-methylene-7-oxabicyclo[2.2.1]heptan-2-yl)oxy)dimethylsilane.
To a tetrahydrofuran solution (7 mL) of the crude product of tert-butyl(((1S,2R,4R)-4-isopropyl-1-methyl-5-methylene-7-oxabicyclo[2.2.1]heptan-2-yl)oxy)dimethylsilane, tetrabutylammonium fluoride (1.04 mL, 1 mol/L tetrahydrofuran solution, 1.04 mmol) was added, followed by stirring at room temperature for 18 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 96 mg, 60%).
$^1H$ NMR (400 MHz, $CDCl_3$) δ 1.07 (d, J = 6.8 Hz, 3H), 1.09 (d, J = 6.8 Hz, 3H), 1.44 (s, 3H), 1.50-1.65 (m, 2H), 2.08 (dd, J = 13.2, 6.8 Hz, 1H), 2.20 (m, 2H), 2.29 (dt, J = 16.0, 2.8 Hz, 1H), 3.80-3.88 (m, 1H), 4.75 (t, J = 2.4 Hz, 1H), 4.84 (t, J = 2.4 Hz, 1H).

Synthesis Example 3

Synthesis of (1R,4S,5R)-1-isopropyl-4-methyl-5-((2-methylbenzyl)oxy)-2-methylene-7-oxabicyclo[2.2.1]heptane (2-72)

To a dimethylformamide solution (1.4 mL) of (1S,2R,4R)-4-isopropyl-1-methyl-5-methylene-7-oxabicyclo[2.2.1]heptan-2-ol (80.0 mg, 0.439 mmol), sodium hydride (55% dispersion in mineral oil, 29.0 mg, 0.658 mmol) was added, followed by stirring at 0°C for 1 hour. To the reaction mixture, 2-methylbenzyl bromide (0.059 mL, 0.44 mmol) was added, followed by stirring at room temperature for 14 hours. A saturated ammonium chloride aqueous solution was poured into the reaction mixture, which was then extracted with ethyl acetate. The extract was dried over anhydrous magnesium

sulfate and then concentrated under reduced pressure. The concentrate was purified by silica gel column chromatography (eluent: ethyl acetate/n-hexane) to obtain a colorless liquid title compound (yield 119 mg, 95%).

$^1$H NMR (400 MHz, $CDCl_3$) δ 1.08 (d, J = 6.8 Hz, 3H), 1.11 (d, J = 6.8 Hz, 3H), 1.49 (s, 3H), 1.82 (dd, J = 12.4, 2.8 Hz, 1H), 1.92 (dd, J = 12.4, 6.8 Hz, 1H), 2.13-2.29 (m, 3H), 2.32 (s, 3H), 3.61 (dd, J = 6.8, 2.8 Hz, 1H), 4.37 (d, J = 12.4 Hz, 1H), 4.55 (d, J = 12.4 Hz, 1H), 4.74 (t, J = 2.4 Hz, 1H), 4.83 (t, J = 2.4 Hz, 1H), 7.13 -7.22 (m, 3H), 7.30-7.34 (m, 1H).

Synthesis Example 4

Synthesis of (1R,4S,5R)-5-((2-fluorobenzyl)oxy)-1-isopropyl-4-methyl-2-methylene-7-oxabicyclo[2.2.1]heptane (2-88)

The same reaction and treatment as in Synthesis Example 3 were performed using 2-fluorobenzyl bromide instead of 2-methylbenzyl bromide to obtain a colorless liquid title compound (yield 64%).

$^1$H NMR (400 MHz, $CDCl_3$) δ 1.08 (d, J = 6.8 Hz, 3H), 1.11 (d, J = 6.8 Hz, 3H), 1.40 (s, 3H), 1.82 (dd, J = 12.4, 2.8 Hz, 1H), 1.95 (dd, J = 12.4, 6.8 Hz, 1H), 2.14-2.31 (m, 3H), 3.65 (dd, J = 6.8, 2.8 Hz, 1H), 4.50 (d, J = 12.8 Hz, 1H), 4.60 (d, J = 12.8 Hz, 1H), 4.75 (t, J = 2.4 Hz, 1H), 4.84 (t, J = 2.4 Hz, 1H), 6.98-7.05 (m, 1H), 7.13 (td, J = 7.2, 0.8 Hz, 1H), 7.22-7.29 (m, 1H), 7.45 (td, J = 7.2, 1.6 Hz, 1H).

Synthesis Example 5

Synthesis of (1R,4S,5R)-1-isopropyl-4-methyl-2-methylene-5-(2-methylphenoxy)-7-oxabicyclo[2.2.1]heptane (2-62)

To a toluene solution (0.3 mL) of (1S,2R,4R)-4-isopropyl-1-methyl-5-methylene-7-oxabicyclo[2.2.1]heptan-2-ol (51.0 mg, 0.280 mmol), tripotassium phosphate (139 mg, 0.655 mmol), copper iodide (5.3 mg, 0.028 mmol), 4-pyrrolidinopyridine (49.8 mg, 0.336 mmol), and 2-iodotoluene (0.035 mL, 0.28 mmol) were added, followed by stirring at 140°C for 23 hours. Hexane was poured into the reaction mixture, the solid was filtered using filter paper, and the filtrate was concentrated under reduced pressure. The concentrate was purified by silica gel column chromatography (eluent: ethyl acetate/n-hexane) to obtain a colorless liquid title compound (yield 77.3 mg, 100%).

$^1$H NMR (400 MHz, $CDCl_3$) δ 1.07 (d, J = 6.8 Hz, 3H), 1.08 (d, J = 6.8 Hz, 3H), 1.59 (s, 3H), 1.77 (dd, J = 12.4, 2.8 Hz, 1H), 2.14-2.23 (m, 2H), 2.25 (s, 3H), 2.29-2.41 (m, 2H), 4.31 (dd, J = 6.8, 2.8 Hz, 1H), 4.83 (t, J = 2.4 Hz, 1H), 4.89 (t, J = 2.4 Hz, 1H), 6.69 (d, J = 8.0 Hz, 1H), 6.84 (td, J = 8.0, 0.8 Hz, 1H), 7.06-7.17 (m, 2H).

Synthesis Example 6

Synthesis of tert-butyl(((1S,2R,4R,5S)-5-(1-ethoxyethoxy)-4-isopropyl-1-methyl-7-oxabicyclo[2.2.1]heptan-2-yl)oxy)dimethylsilane

To a dichloromethane solution (1.7 mL) of (1R,2S,4S,5R)-5-((tert-butyldimethylsilyl)oxy)-1-isopropyl-4-methyl-7-oxabicyclo[2.2.1]heptan-2-ol (175 mg, 0.582 mmol), pyridinium paratoluenesulfonate (11.8 mg, 0.0465 mmol) and ethyl vinyl ether (50.4 mg, 0.699 mmol) were added, followed by stirring at room temperature for 2 hours. A saturated sodium hydrogen carbonate aqueous solution was poured into the reaction mixture, which was then extracted with dichloromethane. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 172 mg, 79%).

$^1$H NMR (400 MHz, $CDCl_3$) δ 0.051 (s, 3H), 0.054 (s, 3H), 0.89 (s, 9H), 0.95-1.03 (m, 6H), 1.196 (t, J = 7.2 Hz, 1.5H), 1.198 (t, J = 7.2 Hz, 1.5H), 1.25-1.37 (m, 8H), 1.84-1.98 (m, 1H), 2.00-2.10 (m, 1H), 2.61-2.69 (m, 1H), 3.41-3.52 (m, 1H), 3.56-3.69 (m, 1H), 3.80-3.87 (m, 1.5H), 4.00 (ddd, J = 8.4, 3.2, 1.2 Hz, 0.5H), 4.63 (q, J = 7.6 Hz, 1H).

Synthesis Example 7

Synthesis of (1S,2R,4R,5S)-5-(1-ethoxyethoxy)-4-isopropyl-1-methyl-7-oxabicyclo[2.2.1]heptan-2-ol

To a tetrahydrofuran solution (5 mL) of tert-butyl(((1S,2R,4R,5S)-5-(1-ethoxyethoxy)-4-isopropyl-1-methyl-7-oxabicyclo[2.2.1]heptan-2-yl)oxy)dimethylsilane (172 mg, 0.462 mmol), tetrabutylammonium fluoride (0.69 mL, 1 mol/L tetrahydrofuran solution, 0.69 mmol) was added, followed by stirring at room temperature for 22 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 113 mg, 95%).

$^1H$ NMR (400 MHz, $CDCl_3$) δ 0.99 (d, J = 6.8 Hz, 3H), 1.00 (t, J = 6.8 Hz, 3H), 1.02 (d, J = 6.8 Hz, 3H), 1.19 (t, J = 6.8 Hz, 1.5H), 1.20 (t, J = 6.8 Hz, 1.5H), 1.30-1.34 (m, 1H), 1.35 (s, 1.5H), 1.36 (s, 1.5H), 1.40-1.56 (m, 2H), 1.89-2.09 (m, 2H), 2.70-2.77 (m, 1H), 3.41-3.52 (m, 1H), 3.55-3.67 (m, 1H), 3.74-3.86 (m, 1.5H), 4.01 (ddd, J = 8.4, 3.2, 1.2 Hz, 0.5H), 4.60-4.66 (m, 1H).

Synthesis Example 8

Synthesis of (1R,2S,4S,5R)-2-(1-ethoxyethoxy)-1-isopropyl-4-methyl-5-((2-methylbenzyl)oxy)-7-oxabicyclo[2.2.1]heptane (1-208)

To a dimethylformamide solution (2 mL) of (1S,2R,4R,5S)-5-(1-ethoxyethoxy)-4-isopropyl-1-methyl-7-oxabicyclo[2.2.1]heptan-2-ol (113 mg, 0.437 mmol), sodium hydride (55% dispersion in mineral oil, 28.6 mg, 0.655 mmol) was added, followed by stirring at 0°C for 1 hour. To the reaction mixture, 2-methylbenzyl bromide (0.059 mL, 0.44 mmol) was added, followed by stirring at room temperature for 7 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 158 mg, 100%).

$^1H$ NMR (400 MHz, $CDCl_3$) δ 0.98-1.06 (m, 6H), 1.15-1.22 (m, 3H), 1.24-1.29 (m, 4H), 1.32-1.38 (m, 1H), 1.40 (s, 1.5H), 1.41 (s, 1.5H), 1.84-1.99 (m, 1H), 2.01-2.13 (m, 1H), 2.32 (s, 3H), *2.57-2.66* (m, 1H), 3.38-3.53 (m, 1H), 3.54-3.67 (m, 2H), 3.81-3.88 (m, 0.5H), 4.00-4.06 (m, 0.5H), 4.39 (d, J = 12.4 Hz, 1H), 4.57 (d, J = 12.4 Hz, 1H), 4.59-4.68 (m, 1H), 7.13-7.22 (m, 3H), 7.32-7.36 (m, 1H).

Synthesis Example 9

Synthesis of (1R,28,4S,5R)-2-(1-ethoxyethoxy)-5-((2-fluorobenzyl)oxy)-1-isopropyl-4-methyl-7-oxabicyclo[2.2.1]heptane (1-213)

The same reaction and treatment as in Synthesis Example 8 were performed using 2-fluorobenzyl bromide instead of 2-methylbenzyl bromide to obtain a colorless liquid title compound (yield 49%).

$^1H$ NMR (400 MHz, $CDCl_3$) δ 0.97-1.05 (m, 6H), 1.17 (t, J = 7.2 Hz, 1.5H), 1.19 (t, J = 7.2 Hz, 1.5H), 1.23-1.28 (m, 3H), 1.33-1.38 (m, 1H), 1.408 (s, 1.5H), 1.413 (s, 1.5H), 1.47-1.54 (m, 1H), 1.85-1.99 (m, 1H), 2.01-2.13 (m, 1H), 2.63 (dd, J = 12.8, 6.8 Hz, 1H), 3.38-3.52 (m, 1H), 3.54-3.68 (m, 2H), 3.80-3.87 (m, 0.5H), 4.00-4.06 (m, 0.5H), 4.35-4.67 (m, 3H), 6.97-7.49 (m, 4H).

Synthesis Example 10

Synthesis of (1R,2S,4S,5R)-1-isopropyl-4-methyl-5-((2-methylbenzyl)oxy)-7-oxabicyclo[2.2.1]heptan-2-ol (1-24)

To a tetrahydrofuran solution (1 mL) of (1R,2S,4S,5R)-2-(1-ethoxyethoxy)-1-isopropyl-4-methyl-5-((2-methylbenzyl)oxy)-7-oxabicyclo[2.2.1]heptane (133 mg, 0.367 mmol), 3N hydrochloric acid (0.24 mL) was added, followed by stirring at room temperature for 3 hours. A saturated sodium hydrogen carbonate aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 98.2 mg, 92%).

$^1H$ NMR (400 MHz, $CDCl_3$) δ 1.02 (d, J = 6.8 Hz, 3H), 1.03 (d, J = 6.8 Hz, 3H), 1.19 (dd, J = 13.2, 3.6 Hz, 1H), 1.39 (s, 3H), 1.46-1.55 (m, 2H), 1.99-2.14 (m, 2H), 2.32 (s, 3H), 2.64 (dd, J = 12.8, 6.8 Hz, 1H), 3.64 (dd, J = 6.8, 2.4 Hz, 1H), 4.12-4.18 (m, 1H), 4.39 (d, J = 12.4 Hz, 1H), 4.58 (d, J = 12.4 Hz, 1H), 7.12-7.22 (m, 3H), 7.30-7.35 (m, 1H).

Synthesis Example 11

Synthesis of (1R,2S,4S,5R)-5-((2-fluorobenzyl)oxy)-1-isopropyl-4-methyl-7-oxabicyclo[2.2.1]heptan-2-ol (1-30)

The same reaction and treatment as in Synthesis Example 10 were performed using (1R,2S,4S,5R)-2-(1-ethoxyethoxy)-5-((2-fluorobenzyl)oxy)-1-isopropyl-4-methyl-7-oxabicyclo[2.2.1]heptane instead of (1R,2S,4S,5R)-2-(1-ethoxyethoxy)-1-isopropyl-4-methyl-5-((2-methylbenzyl)oxy)-7-oxabicyclo[2.2.1]heptane to obtain a colorless liquid title compound (yield 50%).

$^1H$ NMR (400 MHz, $CDCl_3$) δ 1.02 (d, J = 6.8 Hz, 3H), 1.03 (d, J = 6.8 Hz, 3H), 1.21 (dd, J = 13.2, 3.6 Hz, 1H), 1.41 (s, 3H), 1.48-1.55 (m, 2H), 2.01-2.2.16 (m, 2H), 2.67 (dd, J = 12.8, 6.8 Hz, 1H), 3.69 (dd, J = 6.8, 2.0 Hz, 1H), 4.13-4.20 (m,

1H), 4.53 (d, J = 12.8 Hz, 1H), 4.64 (d, J = 12.8 Hz, 1H), 6.99-7.05 (m, 1H), 7.13 (td, J = 7.2, 0.8 Hz, 1H), 7.22-7.30 (m, 1H), 7.55 (td, J = 7.2, 2.0 Hz, 1H).

Synthesis Example 12

Synthesis of (1R,2S,4S,5R)-2-fluoro-1-isopropyl-4-methyl-5-((2-methylbenzyl)oxy)-7-oxabicyclo[2.2.1]heptane (1-2)

To a dichloromethane solution (3 mL) of (1R,2S,4S,5R)-1-isopropyl-4-methyl-5-((2-methylbenzyl)oxy)-7-oxabicyclo[2.2.1]heptan-2-ol (98.7 mg, 0.400 mmol), pyridine (0.38 mL, 5.0 mmol) and DAST (0.22 mL, 1.7 mmol) were added, and an oil bath temperature was set to 45°C, followed by stirring under heating and reflux for 21 hours. A saturated sodium hydrogen carbonate aqueous solution was poured into the reaction mixture, which was then extracted with dichloromethane. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 44.5 mg, 38%).

$^1$H NMR (400 MHz, $CDCl_3$) δ 1.00 (d, J = 6.8 Hz, 3H), 1.02 (d, J = 6.8 Hz, 3H), 1.42 (s, 3H), 1.44-1.61 (m, 2H), 1.95-2.18 (m, 2H), 2.33 (s, 3H), 2.51-2.59 (m, 1H), 3.69 (dd, J = 6.8, 2.4 Hz, 1H), 4.40 (d, J = 12.0 Hz, 1H), 4.59 (d, J = 12.0 Hz, 1H), 4.83 (dddd, J = 58.4, 9.2, 2.4, 1.6 Hz, 1H), 7.13-7.23 (m, 3H), 7.31-7.35 (m, 1H).

Synthesis Example 13

Synthesis of (1R,2S,4S,5R)-2-fluoro-5-((2-fluorobenzyl)oxy)-1-isopropyl-4-methyl-7-oxabicyclo[2.2.1]heptane (1-5)

The same reaction and treatment as in Synthesis Example 12 were performed using (1R,2S,4S,5R)-5-((2-fluorobenzyl)oxy)-1-isopropyl-4-methyl-7-oxabicyclo[2.2.1]heptan-2-ol instead of (1R,2S,4S,5R)-1-isopropyl-4-methyl-5-((2-methylbenzyl)oxy)-7-oxabicyclo[2.2.1]heptan-2-ol to obtain a colorless liquid title compound (yield 32%).

$^1$H NMR (400 MHz, $CDCl_3$) δ 1.00 (dd, J = 6.8, 0.8 Hz, 3H), 1.01 (dd, J = 6.8, 0.8 Hz, 3H), 1.43 (s, 3H), 1.44-1.62 (m, 2H), 1.97-2.15 (m, 2H), 2.52 (ddd, J = 12.8, 6.8, 3.6 Hz, 1H), 3.72 (dd, J = 6.8, 2.8 Hz, 1H), 4.53 (d, J = 12.4 Hz, 1H), 4.63 (d, J = 12.4 Hz, 1H), 4.90 (ddq, J = 58.4, 9.2, 1.2 Hz, 1H), 6.99-7.08 (m, 1H), 7.10-7.17 (m, 1H), 7.23-7.30 (m, 1H), 7.41-7.49 (m, 1H).

Synthesis Example 14

Synthesis of (1S,2R,4R)-4-isopropyl-1,5-dimethyl-7-oxabicyclo[2.2.1]heptan-2-ol

To a methanol solution (5 mL) of (1S,2R,4R)-4-isopropyl-1-methyl-5-methylene-7-oxabicyclo[2.2.1]heptan-2-ol (95.0 mg, 0.522 mmol), palladium carbon (20.0 mg, water content 55 wt%) was added, followed by stirring under a hydrogen atmosphere at room temperature for 4 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 86.8 mg, 91%).

$^1$H NMR (400 MHz, $CDCl_3$) δ 0.83-0.93 (m, 3H), 0.94-01.04 (m, 6H), 1.24-1.55 (m, 1H), 1.36 (s, 2H), 1.40 (s, 1H), 1.50-1.70 (brs, 1H), 1.73-2.20 (m, 4.33H), 2.38 (dd, J = 13.8, 6.4 Hz, 0.67H), 3.65-3.70 (m, 1H).

Synthesis Example 15

Synthesis of (1S,2R,4R)-4-isopropyl-1,5-dimethyl-2-((2-methylbenzyl)oxy)-7-oxabicyclo[2.2.1]heptane (1-83)

To a dimethylformamide solution (1 mL) of (1S,2R,4R)-4-isopropyl-1,5-dimethyl-7-oxabicyclo[2.2.1]heptan-2-ol (43.0 mg, 0.233 mmol), sodium hydride (55% dispersion in mineral oil, 15.0 mg, 0.344 mmol) was added, followed by stirring at 0°C for 1 hour. To the reaction mixture, 2-methylbenzyl bromide (0.028 mL, 0.209 mmol) was added, followed by stirring at room temperature for 12 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 60.2 mg, 100%).

$^1$H NMR (400 MHz, $CDCl_3$) δ 0.85-1.06 (m, 9H), 1.41 (s, 2.16H), 1.44 (s, 0.84H), 1.49-1.91 (m, 3H), 2.01-2.21 (m, 2H), 2.25-2.32 (m, 1H), 2.32 (s, 3H), 3.47 (dd, J = 6.8, 2.4 Hz, 0.28H), 3.50 (dd, J = 6.8, 2.4 Hz, 0.72H), 4.37 (d, J = 12.4 Hz, 0.28H), 4.39 (d, J = 12.4 Hz, 0.72H), 4.55 (d, J = 12.4 Hz, 1H), 7.13-7.22 (m, 3H), 7.31-7.36 (m, 1H).

Synthesis Example 16

Synthesis of (1S,2R,4R)-2-((2-fluorobenzyl)oxy)-4-isopropyl-1,5-dimethyl-7-oxabicyclo[2.2.1]heptane (1-92)

The same reaction and treatment as in Synthesis Example 15 were performed using 2-fluorobenzyl bromide instead of 2-methylbenzyl bromide to obtain a colorless liquid title compound (yield 92%).

$^1$H NMR (400 MHz, $CDCl_3$) δ 0.82-1.05 (m, 9H), 1.41 (s, 2.01H), 1.44 (s, 0.99H), 1.47-1.53 (m, 0.67H), 1.63-1.91 (m, 2.66H), 1.99-2.20 (m, 1.67H), 2.25-2.32 (dd, J = 13.2, 6.4 Hz, 1H), 3.49 (dd, J = 6.8, 2.4 Hz, 0.33H), 3.52 (dd, J = 6.8, 2.4 Hz, 0.67H), 4.50 (d, J = 12.8 Hz, 1H), 4.59 (d, J = 12.8 Hz, 0.33H), 4.60 (d, J = 12.8 Hz, 0.67H), 7.00 (t, J = 8.8 Hz, 1H), 7.08-7.15 (m, 1H), 7.20-7.28 (m, 1H), 7.41-7.48 (m, 1H).

Synthesis Example 17

Synthesis of (1R,4S,5R)-5-hydroxy-1-isopropyl-4-methyl-7-oxabicyclo[2.2.1]heptan-2-one

To a tetrahydrofuran solution (6 mL) of (1R,4S,5R)-5-((tert-butyldimethylsilyl)oxy)-1-isopropyl-4-methyl-7-oxabicyclo[2.2.1]heptan-2-one (177 mg, 0.593 mmol), tetrabutylammonium fluoride (0.89 mL, 1 mol/L tetrahydrofuran solution, 0.89 mmol) was added, followed by stirring at room temperature for 16 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a white solid title compound (yield 96.2 mg, 88%).

Melting point: 175°C to 181°C; $^1$H NMR (400 MHz, $CDCl_3$) δ 1.05 (d, J = 6.8 Hz, 3H), 1.07 (d, J = 6.8 Hz, 3H), 1.53 (s, 3H), 1.58 (d, J = 6.8 Hz, 1H), 1.64-1.70 (m, 2H), 2.11-2.28 (m, 3H), 3.94-4.00 (m, 1H).

Synthesis Example 18

Synthesis of (1R,4S,5R)-1-isopropyl-4-methyl-5-((2-methylbenzyl)oxy)-7-oxabicyclo[2.2.1]heptan-2-one (2-3)

To a dimethylformamide solution (2 mL) of (1R,48,5R)-5-hydroxy-1-isopropyl-4-methyl-7-oxabicyclo[2.2.1]heptan-2-one (47.9 mg, 0.260 mmol), sodium hydride (55% dispersion in mineral oil, 17.0 mg, 0.390 mmol) was added, followed by stirring at 0°C for 1 hour. To the reaction mixture, 2-methylbenzyl bromide (0.052 mL, 0.39 mmol) was added, followed by stirring at room temperature for 12 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 74.9 mg, 100%).

$^1$H NMR (400 MHz, $CDCl_3$) δ 1.07 (d, J = 6.8 Hz, 3H), 1.08 (d, J = 6.8 Hz, 3H), 1.57 (s, 3H), 1.83 (dd, J = 14.0, 2.8 Hz, 1H), 1.98 (dd, J = 14.0, 6.8 Hz, 1H), 2.01-2.26 (m, 3H), 2.34 (s, 3H), 3.69 (dd, J = 6.4, 2.8 Hz, 1H), 4.41 (d, J = 12.4 Hz, 1H), 4.59 (d, J = 12.4 Hz, 1H), 7.10-7.25 (m, 3H), 7.29-7.33 (m, 1H).

Synthesis Example 19

Synthesis of (1R,4S,5R)-5-((2-fluorobenzyl)oxy)-1-isopropyl-4-methyl-7-oxabicyclo[2.2.1]heptan-2-one (2-9)

The same reaction and treatment as in Synthesis Example 18 were performed using 2-fluorobenzyl bromide instead of 2-methylbenzyl bromide to obtain a colorless liquid title compound (yield 100%).

$^1$H NMR (400 MHz, $CDCl_3$) δ 1.06 (d, J = 6.8 Hz, 3H), 1.07 (d, J = 6.8 Hz, 3H), 1.58 (s, 3H), 1.83 (dd, J = 11.2, 2.8 Hz, 1H), 2.00 (dd, J = 9.2, 6.8 Hz, 1H), 2.04-2.27 (m, 3H), 3.73 (dd, J = 6.8, 2.8 Hz, 1H), 4.53 (d, J = 12.4 Hz, 1H), 4.63 (d, J = 12.4 Hz, 1H), 7.00-7.07 (m, 1H), 7.14 (td, J = 7.2, 0.8 Hz, 1H), 7.25-7.32 (m, 1H), 7.43 (td, J = 7.2, 1.6 Hz, 1H).

Synthesis Example 20

Synthesis of (1R,4S,5R,E)-1-isopropyl-4-methyl-5-((2-methylbenzyl)oxy)-7-oxabicyclo[2.2.1]heptan-2-one oxime (2-191)

To a mixed solution of (1R,4S,5R)-1-isopropyl-4-methyl-5-((2-methylbenzyl)oxy)-7-oxabicyclo[2.2.1]heptan-2-one (80.5 mg, 0.279 mmol) in ethanol (4 mL) and water (1 mL), hydroxylamine hydrochloride (29.1 mg, 0.419 mmol) and sodium acetate (45.8 mg, 1.10 mmol) were added, and the oil bath was set to 110°C, followed by stirring under heating and reflux for 31 hours. The reaction mixture was extracted with ethyl acetate, and the extract was dried over anhydrous magnesium sulfate and then concentrated under reduced pressure. The concentrate was purified by silica gel column chromatography (eluent: ethyl acetate/n-hexane) to obtain a colorless liquid title compound (yield 43.5 mg, 51%).

$^1H$ NMR (400 MHz, $CDCl_3$) δ 1.11 (d, J = 7.2 Hz, 3H), 1.12 (d, J = 7.2 Hz, 3H), 1.54 (s, 3H), 1.58 (s, 1H), 1.84 (dd, J = 12.8, 2.8 Hz, 1H), 2.08 (dd, J = 12.8, 2.8 Hz, 1H), 2.26 (sept, J = 7.2 Hz, 1H), 2.33 (s, 3H), 2.36 (s, 2H), 3.62 (dd, J = 6.8, 2.8 Hz, 1H), 4.39 (d, J = 12.4 Hz, 1H), 4.57 (d, J = 12.4 Hz, 1H), 7.14-7.24 (m, 3H), 7.30 (d, J = 7.2 Hz, 1H).

Synthesis Example 21

Synthesis of (1R,4S,5R,E)-5-((2-fluorobenzyl)oxy)-1-isopropyl-4-methyl-7-oxabicyclo[2.2.1]heptan-2-one oxime (2-197)

The same reaction and treatment as in Synthesis Example 20 were performed using (1R,4S,5R)-5-((2-fluorobenzyl)oxy)-1-isopropyl-4-methyl-7-oxabicyclo[2.2.1]heptan-2-one instead of (1R,4S,5R)-1-isopropyl-4-methyl-5-((2-methylbenzyl)oxy)-7-oxabicyclo[2.2.1]heptan-2-one to obtain a colorless liquid title compound (yield 33%).
$^1H$ NMR (400 MHz, $CDCl_3$) δ 1.11 (d, J = 6.8 Hz, 6H), 1.55 (s, 3H), 1.58 (s, 1H), 1.84 (dd, J = 12.8, 2.4 Hz, 1H), 2.08 (dd, J = 12.8, 2.4 Hz, 1H), 2.26 (sept, J = 6.8 Hz, 1H), 2.38 (s, 2H), 3.67 (dd, J = 6.8, 2.8 Hz, 1H), 4.51 (d, J = 12.4 Hz, 1H), 4.61 (d, J = 12.4 Hz, 1H), 7.00-7.06 (m, 1H), 7.14 (td, J = 7.2, 2.0 Hz, 1H), 7.24-7.31 (m, 1H), 7.43 (td, J = 7.2, 2.0 Hz, 1H).

Synthesis Example 22

Synthesis of tert-butyl(((1S,2R,4R,5S)-4-isopropyl-5-methoxy-1-methyl-7-oxabicyclo[2.2.1]heptan-2-yl)oxy)dimethylsilane

To a dimethylformamide solution (3 mL) of (1R,2S,4S,5R)-5-((tert-butyldimethylsilyl)oxy)-1-isopropyl-4-methyl-7-oxabicyclo[2.2.1]heptan-2-ol (274 mg, 0.912 mmol), sodium hydride (55% dispersion in mineral oil, 79.0 mg, 1.81 mmol) was added, followed by stirring at 0°C for 2 hours. Iodomethane (0.33 mL, 5.3 mmol) was added to the reaction mixture, followed by stirring at room temperature for 26 hours. A saturated sodium hydrogen carbonate aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 289 mg, 100%).
$^1H$ NMR (400 MHz, $CDCl_3$) δ 0.05 (s, 6H), 0.89 (s, 9H), 0.97 (d, J = 6.8 Hz, 3H), 0.98 (d, J = 6.8 Hz, 3H), 1.30 (s, 3H), 1.20-1.36 (m, 2H), 1.86 (dd, J = 12.8, 5.2 Hz, 1H), 2.05 (sept, J = 6.8 Hz, 1H), 2.60 (dd, J = 12.8, 7.2 Hz, 1H), 3.27 (s, 3H), 3.57 (ddd, J = 7.2, 3.2, 1.2 Hz, 1H), 3.84 (dd, J = 7.2, 2.4 Hz, 1H).

Synthesis Example 23

Synthesis of (1S,2R,4R,5S)-4-isopropyl-5-methoxy-1-methyl-7-oxabicyclo[2.2.1]heptan-2-ol

To a tetrahydrofuran solution (9 mL) of tert-butyl(((1S,2R,4R,5S)-4-isopropyl-5-methoxy-1-methyl-7-oxabicyclo[2.2.1]heptan-2-yl)oxy)dimethylsilane (289 mg, 0.918 mmol), tetrabutylammonium fluoride (1.38 mL, 1 mol/L tetrahydrofuran solution, 1.38 mmol) was added, followed by stirring at room temperature for 20 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a white solid title compound (yield 130 mg, 71%).
Melting point: 118°C to 122°C; $^1H$ NMR (400 MHz, $CDCl_3$) δ 0.99 (d, J = 6.8 Hz, 3H), 1.00 (d, J = 6.8 Hz, 3H), 1.30 (s, 3H), 1.23-1.30 (m, 1H), 1.45-1.53 (m, 1H), 1.54-1.59 (m, 1H), 1.86 (dd, J = 12.8, 9.6 Hz, 1H), 2.04 (sept, J = 6.8 Hz, 1H), 2.70 (dd, J = 13.6, 7.2 Hz, 1H), 3.27 (s, 3H), 3.57 (ddd, J = 9.6, 3.6, 1.2 Hz, 1H), 3.77-3.82 (m, 1H).

Synthesis Example 24

Synthesis of (1 S,2R,4R,5S)-4-isopropyl-5-methoxy-l-methyl-2-((2-methylbenzyl)oxy)-7-oxabicyclo[2.2.1]heptane (1-151)

To a dimethylformamide solution (1 mL) of (1S,2R,4R,5S)-4-isopropyl-5-methoxy-1-methyl-7-oxabicyclo[2.2.1]heptan-2-ol (65.1 mg, 0.325 mmol), sodium hydride (55% dispersion in mineral oil, 21.0 mg, 0.481 mmol) was added, followed by stirring at 0°C for 2 hours. To the reaction mixture, 2-methylbenzyl bromide (0.052 mL, 0.39 mmol) was added, followed by stirring at room temperature for 24 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography

(eluent: ethyl acetate/n-hexane) to obtain a colorless liquid title compound (yield 81.0 mg, 82%).

$^1$H NMR (400 MHz, $CDCl_3$) δ 0.99 (d, J = 6.8 Hz, 6H), 1.28 (dd, J = 12.8, 3.2 Hz, 1H), 1.37 (s, 3H), 1.45-1.53 (m, 1H), 1.86 (dd, J = 12.8, 9.6 Hz, 1H), 2.08 (sept, J = 6.8 Hz, 1H), 2.32 (s, 3H), 2.56 (dd, J = 12.8, 6.8 Hz, 1H), 3.26 (s, 3H), 3.57-3.62 (m, 2H), 4.37 (d, J = 12.4 Hz, 1H), 4.57 (d, J = 12.4 Hz, 1H), 7.12-7.22 (m, 3H), 7.31-7.36 (m, 1H).

Synthesis Example 25

Synthesis of (1S,2R,4R,5S)-2-((2-fluorobenzyl)oxy)-4-isopropyl-5-methoxy-1-methyl-7-oxabicyclo[2.2.1]heptane (1-156)

The same reaction and treatment as in Synthesis Example 24 were performed using 2-fluorobenzyl bromide instead of 2-methylbenzyl bromide to obtain a colorless liquid title compound (yield 100%).

$^1$H NMR (400 MHz, $CDCl_3$) δ 0.99 (d, J = 6.8 Hz, 6H), 1.30 (dd, J = 12.8, 3.2 Hz, 1H), 1.43 (s, 3H), 1.46-1.52 (m, 1H), 1.88 (dd, J = 12.8, 9.2 Hz, 1H), 2.08 (sept, J = 6.8 Hz, 1H), 2.60 (dd, J = 12.8, 6.8 Hz, 1H), 3.27 (s, 3H), 3.60 (ddd, J = 9.2, 3.2, 2.0 Hz, 1H), 3.65 (dd, J = 7.2, 2.4 Hz, 1H), 4.51 (d, J = 12.8 Hz, 1H), 4.62 (d, J = 12.8 Hz, 1H), 6.99-7.05 (m, 1H), 7.13 (td, J = 7.2, 1.6 Hz, 1H), 7.22-7.29 (m, 1H), 7.46 (td, J = 7.2, 1.6 Hz, 1H).

Synthesis Example 26

Synthesis of tert-butyl(((1S,2R,4R)-4-isopropyl-1-methyl-5-phenyl-7-oxabicyclo[2.2.1]hept-5-en-2-yl)oxy)dimethylsilane

To a tetrahydrofuran solution (7 mL) of (1R,4S,5R)-5-((tert-butyldimethylsilyl)oxy)-1-isopropyl-4-methyl-7-oxabicyclo[2.2.1]heptan-2-one (352 mg, 1.18 mmol), lithium bis(trimethylsilyl)amide (1.76 mL, 1 mol/L tetrahydrofuran solution, 1.76 mmol) was added, followed by stirring for 2 hours. To the reaction mixture, N-phenylbis(trifluoromethanesulfonimide) (630 mg, 1.76 mmol) was added, followed by stirring at room temperature for 30 minutes. A saturated ammonium chloride aqueous solution was poured into the reaction mixture, which was then extracted with ethyl acetate. The extract was dried over anhydrous magnesium sulfate and then concentrated under reduced pressure to obtain a crude product of (1R,4S,5R)-5-((tert-butyldimethylsilyl)oxy)-1-isopropyl-4-methyl-7-oxabicyclo[2.2.1]hept-2-en-2-yl trifluoromethanesulfonate.

To a 1:1 mixture (5 mL) of 1,2-dimethoxyethane and dichloromethane of the crude product of (1R,4S,5R)-5-((tert-butyldimethylsilyl)oxy)-1-isopropyl-4-methyl-7-oxabicyclo[2.2.1]hept-2-en-2-yl trifluoromethanesulfonate, potassium carbonate (1.34 g, 9.75 mmol) and phenylboronic acid (87.0 mg, 0.715 mmol) were added, and argon was bubbled through the mixture for 5 minutes. To the reaction mixture, tetrakistriphenylphosphine palladium (150 mg, 0.130 mmol) was added, followed by stirring at room temperature for 17 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 187 mg, 44%).

$^1$H NMR (400 MHz, $CDCl_3$) δ 0.05 (s, 3H), 0.06 (s, 3H), 0.84 (d, J = 6.8 Hz, 3H), 0.90 (s, 9H), 1.08 (d, J = 6.8 Hz, 3H), 1.55 (s, 3H), 1.64-1.71 (m, 1H), 1.97-2.06 (m, 1H), 2.33-2.45 (m, 1H), 3.86-3.96 (m, 1H), 5.98 (s, 1H), 7.18-7.62 (m, 5H).

Synthesis Example 27

Synthesis of (1S,2R,4R)-4-isopropyl-1-methyl-5-phenyl-7-oxabicyclo[2.2.1]heptan-2-ol

To a methanol solution (3.5 mL) of tert-butyl(((1S,2R,4R)-4-isopropyl-1-methyl-5-phenyl-7-oxabicyclo[2.2.1]hept-5-en-2-yl)oxy)dimethylsilane (316 mg, 0.882 mmol), palladium carbon (47.0 mg, water content 55 wt%) was added, followed by stirring at room temperature for 21 hours under a hydrogen atmosphere. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 217 mg, 100%).

$^1$H NMR (400 MHz, $CDCl_3$) δ 0.91 (d, J = 6.4 Hz, 3H), 0.93 (d, J = 6.4 Hz, 3H), 1.26-1.33 (m, 1H), 1.47 (s, 3H), 1.58 (s, 1H), 1.65 (dd, J = 13.2, 6.8 Hz, 1H), 2.06 (sept, J = 6.4, Hz, 1H), 2.14 (t, J = 12 Hz, 1H), 2.53 (dd, J = 13.2, 6.4 Hz, 1H), 3.32-3.40 (m, 1H), 3.95 (d, J = 6.4 Hz, 1H), 7.17-7.35 (m, 3H), 7.38-7.45 (m, 1H), 7.48-7.56 (m, 1H).

Synthesis Example 28

Synthesis of (1S,2R,4R)-4-isopropyl-1-methyl-2-((2-methylbenzyl)oxy)-5-phenyl-7-oxabicyclo[2.2.1]heptane (1-313)

To a dimethylformamide solution (1.5 mL) of (1S,2R,4R)-4-isopropyl-1-methyl-5-phenyl-7-oxabicyclo[2.2.1]heptan-2-ol (109 mg, 0.441 mmol), sodium hydride (55% dispersion in mineral oil, 28.9 g, 0.662 mmol) was added, followed by stirring at 0°C for 1 hour. To the reaction mixture, 2-methylbenzyl bromide (0.050 mL, 0.37 mmol) was added, followed by stirring at room temperature for 16 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 55.0 mg, 40%).

$^1$H NMR (400 MHz, $CDCl_3$) δ 0.90 (d, J = 7.2 Hz, 3H), 0.95 (d, J = 7.2 Hz, 3H), 1.51-1.60 (m, 1H), 1.53 (s, 3H), 1.63 (dd, J = 12.8, 6.0 Hz, 1H), 2.05-2.19 (m, 2H), 2.34 (s, 3H), 2.39 (dd, J = 12.8, 6.0 Hz, 1H), 3.35-3.43 (m, 1H), 3.78 (d, J = 6.0 Hz, 1H), 4.44 (d, J = 12.8 Hz, 1H), 4.60 (d, J = 12.8 Hz, 1H), 7.11-7.33 (m, 8H), 7.36-7.41 (m, 1H).

Synthesis Example 29

Synthesis of (1S,2R,4R)-2-((2-fluorobenzyl)oxy)-4-isopropyl-1-methyl-5-phenyl-7-oxabicyclo[2.2.1]heptane (1-322)

The same reaction and treatment as in Synthesis Example 28 were performed using 2-fluorobenzyl bromide instead of 2-methylbenzyl bromide to obtain a colorless liquid title compound (yield 68%).

$^1$H NMR (400 MHz, $CDCl_3$) δ 0.89 (d, J = 6.8 Hz, 3H), 0.95 (d, J = 6.8 Hz, 3H), 1.51-1.58 (m, 1H), 1.53 (s, 3H), 1.64 (dd, J = 13.2, 6.4 Hz, 1H), 2.04-2.19 (m, 2H), 2.42 (dd, J = 13.2, 6.8 Hz, 1H), 3.39 (ddd, J = 11.2, 6.4, 1.6 Hz, 1H), 3.82 (dd, J = 6.8, 3.6 Hz, 1H), 4.57 (d, J = 12.4 Hz, 1H), 4.66 (d, J = 12.4 Hz, 1H), 6.89-7.35 (m, 8H), 7.46-7.55 (m, 1H).

Synthesis Example 30

Synthesis of (1S,2R,4R)-4-isopropyl-1-methyl-5-phenyl-7-oxabicyclo[2.2.1]hept-5-en-2-ol

To a tetrahydrofuran solution (7 mL) of tert-butyl(((1S,2R,4R)-4-isopropyl-1-methyl-5-phenyl-7-oxabicyclo[2.2.1]hept-5-en-2-yl)oxy)dimethylsilane (233 mg, 0.650 mmol), tetrabutylammonium fluoride (0.98 mL, 1 mol/L tetrahydrofuran solution, 0.98 mmol) was added, followed by stirring at room temperature for 29 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 97.3 mg, 61%).

$^1$H NMR (400 MHz, $CDCl_3$) δ 0.84 (d, J = 6.8 Hz, 3H), 1.07 (d, J = 6.8 Hz, 3H), 1.48-1.70 (m, 2H), 1.61 (s, 3H), 2.15-2.23 (m, 1H), 2.43 (sept, J = 6.8 Hz, 1H), 3.95 (ddd, J = 10.1, 6.4, 1.6 Hz, 1H), 6.00 (s, 1H), 7.21-7.34 (m, 5H).

Synthesis Example 31

Synthesis of (1R,4S,5R)-1-isopropyl-4-methyl-5-((2-methylbenzyl)oxy)-2-phenyl-7-oxabicyclo[2.2.1]hept-2-ene (3-175)

To a dimethylformamide solution (1 mL) of (1S,2R,4R)-4-isopropyl-1-methyl-5-phenyl-7-oxabicyclo[2.2.1]hept-5-en-2-ol (50.0 mg, 0.205 mmol), sodium hydride (55% dispersion in mineral oil, 13.4 mg, 0.307 mmol) was added, followed by stirring at 0°C for 1 hour. To the reaction mixture, 2-methylbenzyl bromide (0.030 mL, 0.22 mmol) was added, followed by stirring at room temperature for 15 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 48.3 mg, 68%).

$^1$H NMR (400 MHz, $CDCl_3$) δ 0.85 (d, J = 6.8 Hz, 3H), 1.09 (d, J = 6.8 Hz, 3H), 1.64 (s, 3H), 1.86 (dd, J = 12.0, 2.4 Hz, 1H), 1.97 (dd, J = 12.0, 6.4 Hz, 1H), 2.35 (s, 3H), 2.43 (sept, J = 6.8 Hz, 1H), 3.68 (dd, J = 6.4, 1.6 Hz, 1H), 4.45 (d, J = 12.4 Hz, 1H), 4.63 (d, J = 12.4 Hz, 1H), 5.97 (s, 1H), 7.11-7.35 (m, 9H).

Synthesis Example 32

Synthesis of (1R,4S,5R)-5-((2-fluorobenzyl)oxy)-1-isopropyl-4-methyl-2-phenyl-7-oxabicyclo[2.2.1]hept-2-ene (3-177)

The same reaction and treatment as in Synthesis Example 31 were performed using 2-fluorobenzyl bromide

instead of 2-methylbenzyl bromide to obtain a colorless liquid title compound (yield 54%).

$^1$H NMR (400 MHz, $CDCl_3$) δ 0.85 (d, J = 6.8 Hz, 3H), 1.09 (d, J = 6.8 Hz, 3H), 1.65 (s, 3H), 1.84-1.91 (m, 1H), 1.88 (dd, J = 12.8, 9.2 Hz, 1H), 2.43 (sept, J = 6.8 Hz, 1H), 3.72 (dd, J = 6.0, 1.6 Hz, 1H), 4.59 (d, J = 12.8 Hz, 1H), 4.68 (d, J = 12.8 Hz, 1H), 5.98 (s, 1H), 7.02 (t, J = 8.0 Hz, 1H), 7.13 (t, J = 7.2 Hz, 1H), 7.18-7.36 (m, 6H), 7.48 (td, J = 7.2 Hz, 1H).

Synthesis Example 33

Synthesis of tert-butyl(((1R,4S,5R)-1-isopropyl-4-methyl-7-oxaspiro[bicyclo[2.2.1]heptane-2,2'-[1,3]dioxolan]-5-yl)oxy) dimethylsilane

To a toluene solution (5 mL) of (1R,4S,5R)-5-((tert-butyldimethylsilyl)oxy)-1-isopropyl-4-methyl-7-oxabicyclo [2.2.1]heptan-2-one (120 mg, 0.402 mmol), ethylene glycol (0.11 mL, 2.01 mmol) and p-toluenesulfonic acid monohydrate (7.0 mg, 0.040 mmol) were added, followed by stirring under heating and reflux for 21 hours. To the reaction mixture, triethylamine was added, and the mixture was concentrated under reduced pressure. The concentrate was purified by silica gel column chromatography (eluent: ethyl acetate/n-hexane) to obtain a colorless liquid title compound (yield 95.6 mg, 69%).

$^1$H NMR (400 MHz, $CDCl_3$) δ 0.049 (s, 3H), 0.051 (s, 3H), 0.87 (d, J = 6.8 Hz, 3H), 0.89 (s, 9H), 1.01 (d, J = 6.8 Hz, 3H), 1.32 (s, 3H), 1.46 (dd, J = 13.6, 2.4 Hz, 1H), 1.62 (d, J = 11.2 Hz, 1H), 1.81 (d, J = 11.2 Hz, 1H), 2.30-2.41 (m, 2H), 3.75-3.99 (m, 5H).

Synthesis Example 34

Synthesis of (1R,4S,5R)-1-isopropyl-4-methyl-7-oxaspiro[bicyclo[2.2.1]heptane-2,2'-[1,3]dioxolan]-5-ol

To a tetrahydrofuran solution (3 mL) of tert-butyl(((IR,4S,5R)-1-isopropyl-4-methyl-7-oxaspiro[bicyclo[2.2.1] heptane-2,2'-[1,3]dioxolan]-5-yl)oxy)dimethylsilane (95.6 mg, 0.279 mmol), tetrabutylammonium fluoride (0.40 mL, 1 mol/L tetrahydrofuran solution, 0.40 mmol) was added, followed by stirring at room temperature (25°C) for 18 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 53.0 mg, 83%).

$^1$H NMR (400 MHz, $CDCl_3$) δ 0.88 (d, J = 6.8 Hz, 3H), 1.00 (d, J = 6.8 Hz, 3H), 1.39 (s, 3H), 1.44 (dd, J = 14.4, 1.6 Hz, 1H), 1.55 (d, J = 10.0 Hz, 1H), 1.68 (d, J = 13.2 Hz, 1H), 1.86 (d, J = 13.2 Hz, 1H), 2.39 (sept, J = 6.8 Hz, 1H), 2.50 (dd, J = 14.4, 6.8 Hz, 1H), 3.77-3.85 (m, 2H), 3.87-4.02 (m, 3H).

Synthesis Example 35

Synthesis of (1R,4S,5R)-5-((2-fluorobenzyl)oxy)-1-isopropyl-4-methyl-7-oxaspiro[bicyclo[2.2.1]heptane-2,2'-[1,3]dioxolane] (1-936)

To a dimethylformamide solution (1 mL) of (1R,4S,5R)-1-isopropyl-4-methyl-7-oxaspiro[bicyclo[2.2.1]heptane-2,2'-[1,3]dioxolan]-5-ol (53.0 mg, 0.232 mmol), sodium hydride (55% dispersion in mineral oil, 15.0 mg, 0.348 mmol) was added, followed by stirring at 0°C for 1 hour. To the reaction mixture, 2-fluorobenzyl bromide (0.033 mL, 0.28 mmol) was added, followed by stirring at room temperature (25°C) for 17 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (71.3 mg, 92%).

$^1$H NMR (400 MHz, $CDCl_3$) δ 0.89 (d, J = 6.8 Hz, 3H), 1.03 (d, J = 6.8 Hz, 3H), 1.44 (s, 3H), 1.64 (dd, J = 13.2, 2.4 Hz, 1H), 1.66 (d, J = 12.8 Hz, 1H), 1.83 (d, J = 12.8 Hz, 1H), 2.34-2.45 (m, 2H), 3.64 (dd, J = 6.8, 2.4 Hz, 1H), 3.75-3.81 (m, 1H), 3.88-3.99 (m, 3H), 4.53 (d, J = 12.8 Hz, 1H), 4.62 (d, J = 12.8 Hz, 1H), 7.00-7.05 (m, 1H), 7.13 (dt, J = 7.2, 0.8 Hz, 1H), 7.23-7.29 (m, 1H), 7.45 (dt, J = 8.0, 1.2 Hz, 1H).

Synthesis Example 36

Synthesis of (1S,2R,4R)-4-isopropyl-5,5-dimethoxy-1-methyl-7-oxabicyclo[2.2.1]heptan-2-ol

To a methanol solution (3 mL) of (1R,4S,5R)-5-((tert-butyldimethylsilyl)oxy)-1-isopropyl-4-methyl-7-oxabicyclo [2.2.1]heptan-2-one (120 mg, 0.402 mmol), trimethyl orthoformate (0.21 mL, 2.01 mmol) and p-toluenesulfonic acid

monohydrate (15.0 mg, 0.0789 mmol) were added, followed by stirring under heating and reflux for 2 hours. To the reaction mixture, triethylamine was added, and the mixture was concentrated under reduced pressure. The concentrate was purified by silica gel column chromatography (eluent: ethyl acetate/n-hexane) to obtain a colorless liquid title compound (yield 49.9 mg, 54%).

$^1$H NMR (400 MHz, $CDCl_3$) δ 0.97 (d, J = 6.8 Hz, 3H), 1.01 (d, J = 6.8 Hz, 3H), 1.35 (dd, J = 14.0, 1.6 Hz, 1H), 1.39 (s, 3H), 1.53 (d, J = 9.6 Hz, 1H), 1.70 (d, J = 13.2 Hz, 1H), 1.87 (d, J = 13.2 Hz, 1H), 2.36 (sept, J = 6.8 Hz, 1H), 2.44 (dd, J = 14.0, 6.8 Hz, 1H), 3.28 (s, 3H), 3.40 (s, 3H), 3.73 (ddd, J = 9.6, 6.8, 1.6 Hz, 1H).

Synthesis Example 37

Synthesis of (1R,4S,5R)-5-((2-fluorobenzyl)oxy)-1-isopropyl-2,2-dimethoxy-4-methyl-7-oxabicyclo[2.2.1]heptane (1-935)

To a dimethylformamide solution (1 mL) of (1S,2R,4R)-4-isopropyl-5,5-dimethoxy-1-methyl-7-oxabicyclo[2.2.1]heptan-2-ol (49.9 mg, 0.217 mmol), sodium hydride (55% dispersion in mineral oil, 14.0 mg, 0.321 mmol) was added, followed by stirring at 0°C for 1 hour. To the reaction mixture, 2-fluorobenzyl bromide (0.031 mL, 0.26 mmol) was added, followed by stirring at room temperature (25°C) for 16 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (62.7 mg, 85%).

$^1$H NMR (400 MHz, $CDCl_3$) δ 0.98 (d, J = 6.8 Hz, 3H), 1.04 (d, J = 6.8 Hz, 3H), 1.44 (s, 3H), 1.56 (dd, J = 13.6, 2.8 Hz, 1H), 1.68 (d, J = 12.8 Hz, 1H), 1.87 (d, J = 12.8 Hz, 1H), 2.30 (dd, J = 13.6, 6.8 Hz, 1H), 2.36 (sept, J = 6.8 Hz, 1H), 3.25 (s, 3H), 3.37 (s, 3H), 3.58 (dd, J = 6.8, 2.8 Hz, 1H), 4.52 (d, J = 13.2 Hz, 1H), 4.62 (d, J = 13.2 Hz, 1H), 7.02 (ddd, J = 9.6, 7.2, 0.8 Hz, 1H), 7.13 (dt, J = 7.6, 0.8 Hz, 1H), 7.23-7.29 (m, 1H), 7.45 (dt, J = 7.6, 1.6 Hz, 1H).

Synthesis Example 38

Synthesis of (1S,2R,4R)-4-isopropyl-1-methyl-5-(thiophen-3-yl)-7-oxabicyclo[2.2.1]hept-5-en-2-ol

To a 1,2-dimethoxyethane solution (2.2 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (230 mg, 0.643 mmol), potassium carbonate (133 mg, 0.962 mmol) and 3-thiopheneboronic acid (91.0 mg, 0.711 mmol) were added, and argon was bubbled through the mixture for 5 minutes. To the reaction mixture, tetrakistriphenylphosphine palladium (148 mg, 0.128 mmol) was added, followed by stirring at room temperature (25°C) for 29 hours. A saturated ammonium chloride aqueous solution was poured into the reaction mixture, which was then extracted with ethyl acetate. The extract was dried over anhydrous magnesium sulfate and then concentrated under reduced pressure to obtain a crude product of tert-butyl(((1S,2R,4R)-4-isopropyl-1-methyl-5-(thiophen-3-yl)-7-oxabicyclo[2.2.1]hept-5-en-2-yl)oxy)dimethylsilane.

To a tetrahydrofuran solution (4.8 mL) of tert-butyl(((1S,2R,4R)-4-isopropyl-1-methyl-5-(thiophen-3-yl)-7-oxabicyclo[2.2.1]hept-5-en-2-yl)oxy)dimethylsilane, tetrabutylammonium fluoride (0.72 mL, 1 mol/L tetrahydrofuran solution, 0.72 mmol) was added, followed by stirring at room temperature (25°C) for 18 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 89.3 mg, 55%).

$^1$H NMR (400 MHz, $CDCl_3$) δ 0.95 (d, J = 6.8 Hz, 3H), 1.10 (d, J = 6.8 Hz, 3H), 1.55 (d, J = 8.0 Hz, 1H), 1.58-1.64 (m, 1H), 1.60 (s, 3H), 2.04 (dd, J = 12.4, 6.4 Hz, 1H), 2.43 (sept, J = 6.8 Hz, 1H), 3.91 (t, J = 8.0, 6.4 Hz, 1H), 6.04 (s, 1H), 7.06 (dd, J = 5.2, 1.2 Hz, 1H), 7.12 (dd, J = 2.8, 1.2 Hz, 1H), 7.30 (dd, J = 5.2, 2.8 Hz, 1H).

Synthesis Example 39

Synthesis of (1S,2R,4R)-4-isopropyl-1-methyl-5-(thiophen-3-yl)-7-oxabicyclo[2.2.1]heptan-2-ol

To a methanol solution (1 mL) of (1S,2R,4R)-4-isopropyl-1-methyl-5-(thiophen-3-yl)-7-oxabicyclo[2.2.1]hept-5-en-2-ol (61.8 mg, 0.247 mmol), palladium hydroxide (12.0 mg, water content 50 wt%) was added, followed by stirring under a hydrogen atmosphere at room temperature (25°C) for 17 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 53.8 mg, 86%).

$^1$H NMR (400 MHz, $CDCl_3$) δ 0.97 (d, J = 6.8 Hz, 3H), 0.98 (d, J = 6.8 Hz, 3H), 1.34 (dt, J = 14.0, 2.0 Hz, 1H), 1.46 (s,

3H), 1.59 (dd, J = 14.0, 6.4 Hz, 1H), 1.59-1.70 (m, 1H), 2.04 (sept, J = 6.8 Hz, 1H), 2.41 (dd, J = 12.8, 11.6 Hz, 1H), 2.44 (dd, J = 14.0, 6.8 Hz, 1H), 3.42 (ddd, J = 11.6, 6.4, 2.4 Hz, 1H), 3.85-3.92 (m, 1H), 6.96 (dd, J = 5.2, 1.6 Hz, 1H), 6.98-7.02 (m, 1H), 7.27 (dd, J = 5.2, 2.8 Hz, 1H).

Synthesis Example 40

Synthesis of (1S,2R,4R)-2-((2-fluorobenzyl)oxy)-4-isopropyl-1-methyl-5-(thiophen-3-yl)-7-oxabicyclo[2.2.1]heptane (1-928)

To a dimethylformamide solution (1 mL) of (1S,2R,4R)-4-isopropyl-1-methyl-5-(thiophen-3-yl)-7-oxabicyclo [2.2.1]heptan-2-ol (53.8 mg, 0.213 mmol), sodium hydride (55% dispersion in mineral oil, 13.9 mg, 0.319 mmol) was added, followed by stirring at 0°C for 1 hour. To the reaction mixture, 2-fluorobenzyl bromide (0.030 mL, 0.25 mmol) was added, followed by stirring at room temperature (25°C) for 15 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (52.8 mg, 69%).

$^1$H NMR (400 MHz, $CDCl_3$) δ 0.97 (d, J = 6.8 Hz, 3H), 0.99 (d, J = 6.8 Hz, 3H), 1.51 (s, 3H), 1.53-1.61 (m, 2H), 2.09 (sept, J = 6.8 Hz, 1H), 2.13 (dd, J = 12.8, 11.6 Hz, 1H), 2.34 (dd, J = 13.6, 6.8 Hz, 1H), 3.45 (ddd, J = 11.6, 6.4, 2.0 Hz, 1H), 3.74 (dd, J = 6.8, 2.8 Hz, 1H), 4.54 (d, J = 12.8 Hz, 1H), 4.63 (d, J = 12.8 Hz, 1H), 6.93-7.06 (m, 3H), 7.14 (td, J = 7.2, 0.8 Hz, 1H), 7.24-7.29 (m, 2H), 7.48 (dt, J = 7.2, 1.6 Hz, 1H).

Synthesis Example 41

Synthesis of tert-butyl(((1S,2R,4R)-4-isopropyl-1-methyl-5-((trimethylsilyl )ethynyl)-7-oxabicyclo[2.2.1]hept-5-en-2-yl) oxy)dimethylsilane

To a t-butylamine solution (1.8 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (233 mg, 0.542 mmol), trimethylsilylacetylene (0.38 mL, 2.7 mmol), copper iodide (10.0 mg, 0.0542 mmol), and tetrakistriphenylphosphinepalladium (62.6 mg, 0.0542 mmol) were added, followed by stirring at room temperature (25°C) for 22 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (163 mg, 79%). $^1$H NMR (400 MHz, $CDCl_3$) δ 0.04 (s, 3H), 0.05 (s, 3H), 0.19 (s, 9H), 0.89 (s, 9H), 1.15 (d, J = 6.8 Hz, 3H), 1.16 (d, J = 6.8 Hz, 3H), 1.38 (dd, J = 11.6, 2.0 Hz, 1H), 1.47 (s, 3H), 2.00 (dd, J = 11.6, 6.4 Hz, 1H), 2.19 (sept, J = 6.8 Hz, 1H), 3.87 (dd, J = 6.4, 2.0 Hz, 1H), 6.27 (s, 1H).

Synthesis Example 42

Synthesis of (1S,2R,4R)-4-isopropyl-1-methyl-5-[(trimethylsilyl)ethynyl]-7-oxabicyclo[2,2,1]hept-5-en-2-ol

To a tetrahydrofuran solution (1.4 mL) of tert-butyl(((1S,2R,4R)-4-isopropyl-1-methyl-5-((trimethylsilyl)ethynyl)-7-oxabicyclo[2.2.1]hept-5-en-2-yl)oxy)dimethylsilane (163 mg, 0.428 mmol), hydrochloric acid (3 N aqueous solution, 0.29 mL, 0.87 mmol) was added, followed by stirring at room temperature (25°C) for 4 hours. Water was poured into the reaction mixture, which was then extracted with diethyl ether. The extract was dried over anhydrous magnesium sulfate and then concentrated under reduced pressure. The concentrate was purified by silica gel column chromatography (eluent: ethyl acetate/n-hexane) to obtain a colorless liquid title compound (92.8 mg, 100%).

$^1$H NMR (400 MHz, $CDCl_3$) δ 0.19 (s, 9H), 1.15 (d, J = 6.8 Hz, 6H), 1.37 (dd, J = 12.8, 1.2 Hz, 1H), 1.53 (s, 3H), 2.12 (dd, J = 12.8, 6.4 Hz, 1H), 2.19 (sept, J = 7.6 Hz, 1H), 3.86-3.89 (m, 1H), 6.27 (s, 1H).

Synthesis Example 43

Synthesis of (1S,2R,4R)-5-ethynyl-4-isopropyl-1-methyl-7-oxabicyclo[2.2.1]hept-5-en-2-ol

To a methanol solution (9.7 mL) of (1S,2R,4R)-4-isopropyl-1-methyl-5-[(trimethylsilyl)ethynyl]-7-oxabicyclo [2.2.1]hept-5-en-2-ol (259 mg, 0.970 mmol), potassium carbonate (0.201 mg, 1.46 mmol) was added, followed by stirring at room temperature (25°C) for 3 hours. A saturated ammonium chloride aqueous solution was poured into the reaction mixture, which was then extracted with ethyl acetate. The extract was dried over anhydrous magnesium sulfate and then

concentrated under reduced pressure. The concentrate was purified by silica gel column chromatography (eluent: ethyl acetate/n-hexane) to obtain a colorless liquid title compound (145 mg, 78%).

$^1$H NMR (400 MHz, $CDCl_3$) δ 1.14 (d, J = 5.6 Hz, 3H), 1.15 (d, J = 5.6 Hz, 3H), 1.37 (dd, J = 10.0, 1.2 Hz, 1H), 1.54 (s, 3H), 1.64 (s, 1H), 2.08 (dd, J = 10.0, 5.6 Hz, 1H), 2.21 (sept, J = 5.6 Hz, 1H), 3.38 (s, 1H), 3.87-3.89 (m, 1H), 6.35 (s, 1H).

Synthesis Example 44

Synthesis of (1S,2R,4R)-5-ethyl-4-isopropyl-1-methyl-7-oxabicyclo[2.2.1]heptan-2-ol

To a methanol solution (3.0 mL) of (1S,2R,4R)-5-ethynyl-4-isopropyl-1-methyl-7-oxabicyclo[2.2.1]hept-5-en-2-ol (150 mg, 0.753 mmol), palladium hydroxide (29.0 mg, water content 50 wt%) was added, followed by stirring under a hydrogen atmosphere at room temperature (25°C) for 2 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 94.0 mg, 63%).

$^1$H NMR (400 MHz, $CDCl_3$) δ 0.79-1.04 (m, 11H), 1.16-1.22 (m, 0.3H), 1.27-1.34 (m, 0.7H), 1.36 (s, 2.1H), 1.39 (s, 0.9H), 1.46-1.68 (m, 3H), 1.78 (dd, J = 14.0, 6.4 Hz, 0.3H), 1.98 (sept, J = 6.8 Hz, 0.7H), 2.17 (sept, J = 6.8 Hz, 0.3H), 2.32 (dd, J = 14.0, 6.8 Hz, 0.7H), 3.62-3.71 (m, 1H).

Synthesis Example 45

Synthesis of (1R,4S,5R)-2-ethyl-1-isopropyl-4-methyl-5-((2-methylbenzyl)oxy)-7-oxabicyclo[2.2.1]heptane (1-123)

To a dimethylformamide solution (1.0 mL) of (1S,2R,4R)-5-ethyl-4-isopropyl-1-methyl-7-oxabicyclo[2.2.1]heptan-2-ol (48.0 mg, 0.242 mmol), sodium hydride (55% dispersion in mineral oil, 16.0 mg, 0.367 mmol) was added, followed by stirring at 0°C for 1 hour. To the reaction mixture, 2-methylbenzyl bromide (0.031 mL, 0.23 mmol) was added, followed by stirring at room temperature (25°C) for 39 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (64.0 mg, 92%). $^1$H NMR (400 MHz, $CDCl_3$) δ 0.77-1.06 (m, 11H), 1.42 (s, 2.1H), 1.45 (s, 0.9H), 1.51-1.71 (m, 3H), 1.80-1.92 (m, 1.3H), 2.05 (sept, J = 6.8 Hz, 0.7H), 2.16-2.26 (m, 1H), 2.32 (s, 2.1H), 2.33 (s, 0.9H), 3.48 (dd, J = 6.4, 2.8 Hz, 0.3H), 3.49 (dd, J = 6.4, 2.8 Hz, 0.7H), 4.37 (d, J = 12.4 Hz, 1H), 4.54 (d, J = 12.4 Hz, 0.7H), 4.55 (d, J = 12.4 Hz, 0.3H), 7.13-7.21 (m, 3H), 7.31-7.36 (m, 1H).

Synthesis Example 46

Synthesis of (1R,4S,5R)-2-ethyl-5-[(2-fluorobenzyl)oxy]-1-isopropyl-4-methyl-7-oxabicyclo[2.2.1]heptane (1-126)

The same reaction and treatment as in Synthesis Example 28 were performed using 2-fluorobenzyl bromide instead of 2-methylbenzyl bromide to obtain a colorless liquid title compound (yield 71%).

$^1$H NMR (400 MHz, $CDCl_3$) δ 0.80-1.06 (m, 11H), 1.43 (s, 2.1H), 1.46 (s, 0.9H), 1.50-1.70 (m, 3H), 1.81-1.93 (m, 1.3H), 2.05 (sept, J = 6.8 Hz, 0.7H), 2.16-2.28 (m, 1H), 3.51-3.54 (m, 1H), 4.50 (d, J = 12.8 Hz, 0.7H), 4.51 (d, J = 12.8 Hz, 0.3H), 4.55 (d, J = 12.8 Hz, 1H), 6.99-7.04 (m, 1H), 7.10-7.15 (m, 1H), 7.22-7.28 (m, 1H), 7.44-7.48 (m, 1H).

Synthesis Example 47

Synthesis of tert-butyl(((1S,2R,4R)-4-isopropyl-1-methyl-5-(prop-1-en-2-yl)-7-oxabicyclo[2.2.1]hept-5-en-2-yl)oxy)dimethylsilane

To a 1,2-dimethoxyethane solution (2.2 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (280 mg, 0.650 mmol), potassium carbonate (135 mg, 0.977 mmol) and 2-isopropenyl-4,4,5,5-tetramethyl-1,3,2-dioxaborolane (0.14 mL, 0.74 mmol) were added, and argon was bubbled through the mixture for 5 minutes. To the reaction mixture, tetrakistriphenylphosphine palladium (150 mg, 0.130 mmol) was added, followed by stirring at room temperature (25°C) for 25 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 159 mg, 76%).

$^1$H NMR (400 MHz, $CDCl_3$) δ 0.04 (s, 3H), 0.05 (s, 3H), 0.90 (s, 9H), 0.98 (d, J = 6.8 Hz, 3H), 1.08 (d, J = 6.8 Hz,

3H), 1.49 (s, 3H), 1.56 (dt, J = 11.6, 2.0 Hz, 1H), 1.76 (dd, J = 11.6, 6.4 Hz, 1H), 1.87 (s, 3H), 2.36 (sept, J = 6.8 Hz, 1H), 3.81 (dd, J = 6.4, 2.0 Hz, 1H), 4.83-4.89 (m, 2H), 5.90 (s, 1H).

Synthesis Example 48

Synthesis of (1S,2R,4R)-4,5-isopropyl-1-methyl-7-oxabicyclo[2.2.1]heptan-2-ol

To a methanol solution (2.0 mL) of tert-butyl(((1S,2R,4R)-4-isopropyl-1-methyl-5-(prop-1-en-2-yl)-7-oxabicyclo [2.2.1]hept-5-en-2-yl)oxy)dimethylsilane (159 mg, 0.493 mmol), palladium carbon (31.9 mg, water content 55 wt%) was added, followed by stirring under a hydrogen atmosphere at room temperature (25°C) for 17 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 76.1 mg, 73%).

$^1$H NMR (400 MHz, $CDCl_3$) δ 0.91 (d, J = 6.8 Hz, 3H), 0.92 (d, J = 6.8 Hz, 3H), 1.00-1.05 (m, 1H), 1.06

(d, J = 6.8 Hz, 3H), 1.07 (d, J = 6.8 Hz, 3H), 1.35 (s, 3H), 1.30-1.45 (m, 1H), 1.49-1.54 (m, 2H), 1.76-1.87 (m, 2H), 1.98 (sept, J = 6.8 Hz, 1H), 2.23 (dd, J = 14.0, 6.8 Hz, 1H), 3.63-3.71 (m, 1H).

Synthesis Example 49

Synthesis of (1S,2R,4R)-2-[(2-fluorobenzyl)oxy]-4,5-isopropyl-1-methyl-7-oxabicyclo[2.2.1]heptane (1-133)

To a dimethylformamide solution (1.2 mL) of (1S,2R,4R)-4,5-isopropyl-1-methyl-7-oxabicyclo[2.2.1]heptan-2-ol (76.1 mg, 0.358 mmol), sodium hydride (60% dispersion in mineral oil, 21.5 mg, 0.538 mmol) was added, followed by stirring at 0°C for 1 hour. To the reaction mixture, 2-fluorobenzyl bromide (0.052 mL, 0.43 mmol) was added, followed by stirring at room temperature (25°C) for 17 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (97.2 mg, 81%).

$^1$H NMR (400 MHz, $CDCl_3$) δ 0.89 (d, J = 6.8 Hz, 3H), 0.91 (d, J = 6.8 Hz, 3H), 1.00-1.05 (m, 1H), 1.05 (d, J = 7.2 Hz, 3H), 1.07 (d, J = 7.2 Hz, 3H), 1.33 (sept, J = 7.2 Hz, 1H), 1.40 (s, 3H), 1.69-1.84 (m, 3H), 1.99 (sept, J = 6.8 Hz, 1H), 2.12 (dd, J = 13.2, 6.8 Hz, 1H), 3.51 (dd, J = 6.8, 2.8 Hz, 1H), 4.49 (d, J = 13.2 Hz, 1H), 4.58 (d, J = 13.2 Hz, 1H), 7.00 (t, J = 7.2 Hz, 1H), 7.11 (t, J = 7.2 Hz, 1H), 7.20-7.28 (m, 1H), 7.45 (dt, J = 7.2, 1.6 Hz, 1H).

Synthesis Example 50

Synthesis of tert-butyl(((1S,2R,4R)-4-isopropyl-1-methyl-5-(2-methylprop-1-en-2-yl)-7-oxabicyclo[2.2.1]hept-5-en-2-yl) oxy)dimethylsilane

To a 1,2-dimethoxyethane solution (2.2 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (280 mg, 0.650 mmol), potassium carbonate (135 mg, 0.977 mmol) and 4,4,5,5-tetramethyl-2-(2-methyl-1-prorenyl)-1,3,2-dioxaborolane (0.15 mL, 0.74 mmol) were added, and argon was bubbled through the mixture for 5 minutes. To the reaction mixture, tetrakistriphenylphosphine palladium (150 mg, 0.130 mmol) was added, followed by stirring at room temperature (25°C) for 19 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 164 mg, 75%).

$^1$H NMR (400 MHz, $CDCl_3$) δ 0.04 (s, 3H), 0.05 (s, 3H), 0.89 (s, 9H), 1.02 (d, J = 6.8 Hz, 3H), 1.07 (d, J = 6.8 Hz, 3H), 1.41 (dd, J = 11.6, 2.0 Hz, 1H), 1.50 (s, 3H), 1.71-1.79 (m, 1H), 1.75 (s, 3H), 1.83 (s, 3H), 2.09 (sept, J = 6.8 Hz, 1H), 3.84 (dd, J = 6.0, 2.0 Hz, 1H), 5.57 (s, 1H), 5.73 (s, 1H).

Synthesis Example 51

Synthesis of (1S,2R,4R)-5-isobutyl-4-isopropyl-1-methyl-7-oxabicyclo[2.2.1]heptan-2-ol

To a methanol solution (1.9 mL) of tert-butyl(((1S,2R,4R)-4-isopropyl-1-methyl-5-(2-methylprop-1-en-2-yl)-7-oxabicyclo[2.2.1]hept-5-en-2-yl)oxy)dimethylsilane (164 mg, 0.487 mmol), palladium carbon (32.8 mg, water content 55 wt%) was added, followed by stirring under a hydrogen atmosphere at room temperature (25°C) for 17 hours. The reaction

mixture was filtered through Celite, and the filtrate was concentrated under reduced pressure. The concentrate was purified by silica gel column chromatography (eluent: ethyl acetate/n-hexane) to obtain a colorless liquid title compound (yield 84.4 mg, 77%).

$^1$H NMR (400 MHz, $CDCl_3$) δ 0.88 (d, J = 6.8 Hz, 3H), 0.90 (d, J = 6.8 Hz, 3H), 0.92-0.98 (m, 2H), 1.01 (d, J = 6.8 Hz, 3H), 1.04 (d, J = 6.8 Hz, 3H), 1.22-1.29 (m, 1H), 1.33 (dt, J = 14.0, 1.6 Hz, 1H), 1.37 (s, 3H), 1.45-1.56 (m, 2H), 1.85 (dd, J = 12.4, 10.8 Hz, 1H), 1.97 (sept, J = 6.8 Hz, 1H), 2.00-2.11 (m, 1H), 2.32 (dd, J = 13.6, 6.8 Hz, 1H), 3.63-3.72 (m, 1H).

Synthesis Example 52

Synthesis of (1S,2R,4R)-2-((2-fluorobenzyl)oxy)-5-isobutyl-4-isopropyl-1-methyl-7-oxabicyclo[2.2.1]heptane (1-915)

To a dimethylformamide solution (1.2 mL) of (1S,2R,4R)-5-isobutyl-4-isopropyl-1-methyl-7-oxabicyclo[2.2.1]heptan-2-ol (84.4 mg, 0.373 mmol), sodium hydride (60% dispersion in mineral oil, 22.4 mg, 0.560 mmol) was added, followed by stirring at 0°C for 2 hours. To the reaction mixture, 2-fluorobenzyl bromide (0.055 mL, 0.45 mmol) was added, followed by stirring at room temperature (25°C) for 15 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (97.7 mg, 79%).

$^1$H NMR (400 MHz, $CDCl_3$) δ 0.87 (d, J = 6.8 Hz, 3H), 0.89 (d, J = 6.8 Hz, 3H), 0.96-1.03 (m, 2H), 1.02 (d, J = 6.8 Hz, 3H), 1.05 (d, J = 6.8 Hz, 3H), 1.26 (ddd, J = 12.8, 9.2, 2.4 Hz, 1H), 1.43 (s, 3H), 1.47-1.58 (m, 2H), 1.84 (t, J = 12.4 Hz, 1H), 1.99-2.11 (m, 2H), 2.26 (dd, J = 12.8, 6.8 Hz, 1H), 3.53 (dd, J = 6.8, 2.4 Hz, 1H), 4.50 (d, J = 12.8 Hz, 1H), 4.60 (d, J = 12.8 Hz, 1H), 6.98-7.05 (m, 1H), 7.12 (td, J = 7.2, 1.6 Hz, 1H), 7.21-7.28 (m, 1H), 7.46 (td, J = 7.2, 1.6 Hz, 1H).

Synthesis Example 53

Synthesis of tert-butyl(((1S,2R,4R)-5-(cyclopenten-1-yl)-4-isopropyl-1-methyl-7-oxabicyclo[2.2.1]hept-5-en-2-yl)oxy)dimethylsilane

To a 1,2-dimethoxyethane solution (1.2 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (150 mg, 0.348 mmol), potassium carbonate (72.0 mg, 0.521 mmol) and 1-cyclopentenylboronic acid (42.9 mg, 0.383 mmol) were added, and argon was bubbled through the mixture for 5 minutes. To the reaction mixture, tetrakistriphenylphosphine palladium (80.4 mg, 0.0696 mmol) was added, followed by stirring at room temperature (25°C) for 25 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 78.6 mg, 65%).

$^1$H NMR (400 MHz, $CDCl_3$) δ 0.03 (s, 3H), 0.04 (s, 3H), 0.89 (s, 9H), 1.01 (d, J = 6.8 Hz, 3H), 1.10 (d, J = 6.8 Hz, 3H), 1.49 (s, 3H), 1.53-1.58 (m, 1H), 1.70 (dd, J = 11.2, 6.4 Hz, 1H), 1.77-1.93 (m, 2H), 2.31-2.53 (m, 5H), 3.80 (dd, J = 6.4, 2.0 Hz, 1H), 5.94 (s, 1H), 5.77 (s, 1H).

Synthesis Example 54

Synthesis of (1S,2R,4R)-5-cyclopentyl-4-isopropyl-1-methyl-7-oxabicyclo[2.2.1]heptan-2-ol

To a methanol solution (1.2 mL) of tert-butyl(((1S,2R,4R)-5-(cyclopenten-1-yl)-4-isopropyl-1-methyl-7-oxabicyclo[2.2.1]hept-5-en-2-yl)oxy)dimethylsilane (104 mg, 0.298 mmol), palladium carbon (20.7 mg, water content 55 wt%) was added, followed by stirring under a hydrogen atmosphere at room temperature (25°C) for 15 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 44.0 mg, 62%).

$^1$H NMR (400 MHz, $CDCl_3$) δ 1.04-1.14 (m, 2H), 1.06 (d, J = 6.8 Hz, 6H), 1.36 (s, 3H), 1.43-1.84 (m, 11H), 1.94 (sept, J = 6.8 Hz, 1H), 1.97-2.01 (m, 1H), 2.26 (dd, J = 14.0, 6.8 Hz, 1H), 3.64-3.68 (m, 1H).

Synthesis Example 55

Synthesis of (1R,4S,5R)-2-cyclopentyl-5-[(2-fluorobenzyl)oxy]-1-isopropyl-4-methyl-7-oxabicyclo[2.2.1]heptane (1-920)

To a dimethylformamide solution (1.0 mL) of (1S,2R,4R)-5-cyclopentyl-4-isopropyl-1-methyl-7-oxabicyclo [2.2.1]heptan-2-ol (44.0 mg, 0.185 mmol), sodium hydride (60% dispersion in mineral oil, 11.1 mg, 0.278 mmol) was added, followed by stirring at 0°C for 1 hour. To the reaction mixture, 2-fluorobenzyl bromide (0.027 mL, 0.22 mmol) was added, followed by stirring at room temperature (25°C) for 16 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (52.0 mg, 81%).

$^1$H NMR (400 MHz, $CDCl_3$) δ 1.02-1.17 (m, 2H), 1.06 (d, J = 6.8 Hz, 3H), 1.08 (d, J = 6.8 Hz, 3H), 1.42 (s, 3H), 1.45-1.81 (m, 10H), 1.91-2.03 (m, 2H), 2.16 (dd, J = 13.2, 6.8 Hz, 1H), 3.53 (dd, J = 6.8, 2.8 Hz, 1H), 4.50 (d, J = 12.8 Hz, 1H), 4.60 (d, J = 12.8 Hz, 1H), 6.98-7.04 (m, 1H), 7.12 (td, J = 7.6, 1.6 Hz, 1H), 7.22-7.28 (m, 1H), 7.46 (dt, J = 7.6, 1.6 Hz, 1H).

Synthesis Example 56

Synthesis of tert-butyl(((1S,2R,4R)-4-isopropyl-1-methyl-5-[(E)-styryl]-7-oxabicyclo[2.2.1]hept-5-en-2-yl)oxy)dimethylsilane

To a 1,2-dimethoxyethane solution (2.2 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (278 mg, 0.646 mmol), potassium carbonate (134 mg, 0.970 mmol) and (E)-styrylboronic acid (105 mg, 0.710 mmol) were added, and argon was bubbled through the mixture for 5 minutes. To the reaction mixture, tetrakistriphenylphosphine palladium (149 mg, 0.129 mmol) was added, followed by stirring at room temperature (25°C) for 18 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 103 mg, 41%).

$^1$H NMR (400 MHz, $CDCl_3$) δ 0.05 (s, 3H), 0.06 (s, 3H), 0.91 (s, 9H), 1.10 (d, J = 6.4 Hz, 3H), 1.14 (d, J = 6.4 Hz, 3H), 1.27-1.37 (m, 1H), 1.52 (s, 3H), 1.83 (dd, J = 10.8, 6.4 Hz, 1H), 2.31 (sept, J = 6.8 Hz, 1H), 3.88 (d, J = 6.4 Hz, 1H), 6.10 (s, 1H), 6.63 (d, J = 12.4 Hz, 1H), 6.73 (d, J = 12.4 Hz, 1H), 7.21-7.28 (m, 1H), 7.33 (t, J = 7.2 Hz, 2H), 7.39 (d, J = 8.0 Hz, 2H).

Synthesis Example 57

Synthesis of (1S,2R,4R)-4-isopropyl-1-methyl-5-phenethyl-7-oxabicyclo[2.2.1]heptan-2-ol

To a methanol solution (1.1 mL) of tert-butyl(((1S,2R,4R)-4-isopropyl-1-methyl-5-[(E)-styryl]-7-oxabicyclo [2.2.1]-5-en-2-yl)oxy)dimethylsilane (103 mg, 0.268 mmol), palladium carbon (20.6 mg, water content 55 wheptt%) was added, followed by stirring under a hydrogen atmosphere at room temperature (25°C) for 15 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 46.7 mg, 63%).

$^1$H NMR (400 MHz, $CDCl_3$) δ 0.95 (d, J = 6.8 Hz, 3H), 0.98 (d, J = 6.8 Hz, 3H), 1.04 (dd, J = 14.0, 5.2 Hz, 1H), 1.31-1.35 (m, 2H), 1.38 (s, 3H), 1.70-2.03 (m, 5H), 2.33 (dd, J = 13.6, 6.8 Hz, 1H), 2.50 (dd, J = 9.6, 7.2 Hz, 0.44H), 2.53 (dd, J = 9.6, 7.2 Hz, 0.56H), 2.66 (dd, J = 9.6, 4.8 Hz, 0.56H), 2.69 (dd, J = 9.6, 4.8 Hz, 0.44H), 3.69 (dd, J = 6.8, 2.4 Hz, 0.44H), 3.71 (dd, J = 6.8, 2.4 Hz, 0.56H), 7.13-7.22 (m, 3H), 7.24-7.31 (m, 2H).

Synthesis Example 58

Synthesis of (1S,2R,4R)-2-((2-fluorobenzyl)oxy)-4-isopropyl-1-methyl-5-phenethyl-7-oxabicyclo[2.2.1]heptane (1-929)

To a dimethylformamide solution (1.0 mL) of (1S,2R,4R)-4-isopropyl-1-methyl-5-phenethyl-7-oxabicyclo[2.2.1] heptan-2-ol (46.7 mg, 0.168 mmol), sodium hydride (60% dispersion in mineral oil, 10.1 mg, 0.252 mmol) was added, followed by stirring at 0°C for 1 hour. To the reaction mixture, 2-fluorobenzyl bromide (0.025 mL, 0.20 mmol) was added, followed by stirring at room temperature (25°C) for 15 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (53.2 mg, 83%).

$^1H$ NMR (400 MHz, $CDCl_3$) δ 0.97 (d, J = 7.2 Hz, 3H), 0.99 (d, J = 6.8 Hz, 3H), 1.04 (dd, J = 12.0, 6.8 Hz, 1H), 1.44 (s, 3H), 1.63-1.93 (m, 4H), 1.95-2.07 (m, 2H), 2.26 (dd, J = 13.2, 6.8 Hz, 1H), 2.49 (dd, J = 9.6, 6.8 Hz, 0.44H), 2.53 (dd, J = 9.6, 6.8 Hz, 0.56H), 2.65 (dd, J = 9.6, 4.4 Hz, 0.56H), 2.68 (dd, J = 9.6, 4.4 Hz, 0.44H), 3.55 (dd, J = 6.8, 2.8 Hz, 1H), 4.50 (d, J = 12.8 Hz, 1H), 4.59 (d, J = 12.8 Hz, 1H), 6.97-7.05 (m, 1H), 7.09-7.31 (m, 7H), 7.45 (td, J = 7.2, 2.0 Hz, 1H).

Synthesis Example 59

Synthesis of tert-butyl(((1S,2R,4R)-4-isopropyl-1-methyl-5-(p-tolyl)-7-oxabicyclo[2.2.1]hept-5-en-2-yl)oxy)dimethylsilane

To a 1,2-dimethoxyethane solution (1.5 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (109 mg, 0.253 mmol), potassium carbonate (52.3 mg, 0.378 mmol) and 4-methylphenylboronic acid (37.7 mg, 0.277 mmol) were added, and argon was bubbled through the mixture for 5 minutes. To the reaction mixture, tetrakistriphenylphosphine palladium (63.3 mg, 0.0548 mmol) was added, followed by stirring at room temperature (25°C) for 17 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 69.1 mg, 73%).

$^1H$ NMR (400 MHz, $CDCl_3$) δ 0.05 (s, 3H), 0.06 (s, 3H), 0.85 (d, J = 6.8 Hz, 3H), 0.91 (s, 9H), 1.08 (d, J = 6.8 Hz, 3H), 1.54 (s, 3H), 1.67 (dd, J = 11.6, 2.0 Hz, 1H), 2.00 (dd, J = 11.6, 6.4 Hz, 1H), 2.34 (s, 3H), 2.34-2.43 (m, 1H), 3.93 (dd, J = 6.4, 2.0 Hz, 1H), 5.94 (s, 1H), 7.11 (s, 4H).

Synthesis Example 60

Synthesis of (1S,2R,4R)-4-isopropyl-1-methyl-5-(p-tolyl)-7-oxabicyclo[2.2.1]hept-5-en-2-ol

To a methanol solution (2 mL) of tert-butyl(((1S,2R,4R)-4-isopropyl-1-methyl-5-(p-tolyl)-7-oxabicyclo[2.2.1]hept-5-en-2-yl)oxy)dimethylsilane (70.0 mg, 0.188 mmol), palladium carbon (7.3 mg, water content 55 wt%) was added, followed by stirring under a hydrogen atmosphere at room temperature (25°C) for 19 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 21.9 mg, 63%).

$^1H$ NMR (400 MHz, $CDCl_3$) δ 0.91 (d, J = 7.2 Hz, 3H), 0.93 (d, J = 7.2 Hz, 3H), 1.29 (dt, J = 13.6, 2.4 Hz, 1H), 1.46 (s, 3H), 1.58 (brs, 1H), 1.63 (dd, J = 13.6, 7.2 Hz, 1H), 2.03 (sept, J = 7.2 Hz, 1H), 2.12 (dd, J = 13.6, 12.0 Hz, 1H), 2.32 (s, 3H), 2.52 (dd, J = 13.6, 7.2 Hz, 1H), 3.33 (ddd, J = 12.0, 7.2, 2.4 Hz, 1H), 3.91-3.96 (m, 1H), 7.10 (s, 4H).

Synthesis Example 61

Synthesis of (1S,2R,4R)-2-((2-fluorobenzyl)oxy)-4-isopropyl-1-methyl-5-(p-tolyl)-7-oxabicyclo[2.2.1]heptane (1-927)

To a dimethylformamide solution (0.5 mL) of (1S,2R,4R)-4-isopropyl-1-methyl-5-(p-tolyl)-7-oxabicyclo[2.2.1]hept-5-en-2-ol (35.1 mg, 0.135 mmol), sodium hydride (55% dispersion in mineral oil, 8.8 mg, 0.20 mmol) was added, followed by stirring at 0°C for 1 hour. To the reaction mixture, 2-fluorobenzyl bromide (0.019 mL, 0.16 mmol) was added, followed by stirring at room temperature (25°C) for 38 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (27.8 mg, 56%).

$^1H$ NMR (400 MHz, $CDCl_3$) δ 0.89 (d, J = 6.8 Hz, 3H), 0.95 (d, J = 6.8 Hz, 3H), 1.50-1.53 (m, 1H), 1.53 (s, 3H), 1.63 (dd, J = 13.6, 7.2 Hz, 1H), 2.07 (sept, J = 6.8 Hz, 1H), 2.12-2.16 (m, 1H), 2.32 (s, 3H), 2.46 (dd, J = 13.2, 6.8 Hz, 1H), 3.35 (ddd, J = 12.0, 6.8, 1.6 Hz, 1H), 3.80 (dd, J = 7.2, 2.4 Hz, 1H), 4.57 (d, J = 12.8 Hz, 1H), 4.65 (d, J = 12.8 Hz, 1H), 7.01-7.16 (m, 6H), 7.24-7.29 (m, 1H), 7.45 (dt, J = 7.6, 1.6 Hz, 1H).

Synthesis Example 62

Synthesis of tert-butyl(((1S,2R,4R)-4-isopropyl-1-methyl-5-(m-tolyl)-7-oxabicyclo[2.2.1]heptan-2-yl)oxy)dimethylsilane

To a 1,2-dimethoxyethane solution (2 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (162 mg, 0.376 mmol), potassium carbonate (78.0 mg, 0.564 mmol) and 3-methylphenylboronic acid (56.2 mg, 0.414 mmol) were added, and argon was bubbled through the mixture for

5 minutes. To the reaction mixture, tetrakistriphenylphosphine palladium (87.0 mg, 0.0753 mmol) was added, followed by stirring at room temperature (25°C) for 18 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 51.1 mg, 36%).

$^1$H NMR (400 MHz, $CDCl_3$) δ 0.05 (s, 3H), 0.06 (s, 3H), 0.84 (d, J = 6.8 Hz, 3H), 0.91 (s, 9H), 1.09 (d, J = 6.8 Hz, 3H), 1.54 (s, 3H), 1.68 (dd, J = 11.6, 2.4 Hz, 1H), 2.02 (dd, J = 11.6, 6.0 Hz, 1H), 2.34 (s, 3H), 2.39 (sept, J = 6.8 Hz, 1H), 3.93 (dd, J = 6.0, 2.4 Hz, 1H), 5.94 (s, 1H), 7.01-7.07 (m, 3H), 7.19 (t, J = 7.6 Hz, 1H).

Synthesis Example 63

Synthesis of (1S,2R,4R)-4-isopropyl-1-methyl-5-(m-tolyl)-7-oxabicyclo[2.2.1]heptan-2-ol

To a methanol solution (0.8 mL) of tert-butyl(((1S,2R,4R)-4-isopropyl-1-methyl-5-(m-tolyl)-7-oxabicyclo[2.2.1]heptan-2-yl)oxy)dimethylsilane (61.4 mg, 0.165 mmol), palladium carbon (10.0 mg, water content 55 wt%) was added, followed by stirring under a hydrogen atmosphere at room temperature (25°C) for 25 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 40.7 mg, 95%).

$^1$H NMR (400 MHz, $CDCl_3$) δ 0.91 (d, J = 6.8 Hz, 3H), 0.94 (d, J = 6.8 Hz, 3H), 1.30 (dt, J = 14.0, 2.0 Hz, 1H), 1.47 (s, 3H), 1.59 (d, J = 6.8 Hz, 1H), 1.65 (dd, J = 14.0, 6.8 Hz, 1H), 2.03 (sept, J = 6.8 Hz, 1H), 2.10 (dd, J = 12.8, 12.0 Hz, 1H), 2.34 (s, 3H), 2.54 (dd, J = 12.8, 6.8 Hz, 1H), 3.33 (ddd, J = 12.0, 6.8, 2.0 Hz, 1H), 3.94-3.97 (m, 1H), 7.01-7.04 (m, 3H), 7.19 (t, J = 8.0 Hz, 1H).

Synthesis Example 64

Synthesis of (1S,2R,4R)-2-((2-fluorobenzyl)oxy)-4-isopropyl-1-methyl-5-(m-tolyl)-7-oxabicyclo[2.2.1]heptane (1-926)

To a dimethylformamide solution (1 mL) of (1S,2R,4R)-4-isopropyl-1-methyl-5-(m-tolyl)-7-oxabicyclo[2.2.1]heptan-2-ol (50.0 mg, 0.192 mmol), sodium hydride (55% dispersion in mineral oil, 12.0 mg, 0.275 mmol) was added, followed by stirring at 0°C for 1 hour. To the reaction mixture, 2-fluorobenzyl bromide (0.028 mL, 0.23 mmol) was added, followed by stirring at room temperature (25°C) for 14 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (45.7 mg, 65%).

$^1$H NMR (400 MHz, $CDCl_3$) δ 0.90 (d, J = 6.8 Hz, 3H), 0.95 (d, J = 6.8 Hz, 3H), 1.51-1.55 (m, 1H), 1.53 (s, 3H), 1.63 (dd, J = 13.2, 6.4 Hz, 1H), 2.04-2.15 (m, 2H), 2.35 (s, 3H), 2.41 (dd, J = 13.2, 6.8 Hz, 1H), 3.35 (ddd, J = 12.0, 6.8, 2.0 Hz, 1H), 3.81 (dd, J = 6.8, 2.4 Hz, 1H), 4.57 (d, J = 12.8 Hz, 1H), 4.66 (d, J = 12.8 Hz, 1H), 7.00-7.06 (m, 4H), 7.14 (dt, J = 7.2, 0.8 Hz, 1H), 7.19 (dt, J = 17.6, 7.6 Hz, 1H), 7.24-7.30 (m, 1H), 7.50 (td, J = 7.6, 1.6 Hz, 1H).

Synthesis Example 65

Synthesis of tert-butyl(((1S,2R,4R)-4-isopropyl-1-methyl-5-(o-tolyl)-7-oxabicyclo[2.2.1]hept-5-en-2-yl)oxy)dimethylsilane

To a 1,2-dimethoxyethane solution (2 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (162 mg, 0.376 mmol), potassium carbonate (78.0 mg, 0.564 mmol) and 2-methylphenylboronic acid (87.0 mg, 0.753 mmol) were added, and argon was bubbled through the mixture for 5 minutes. To the reaction mixture, tetrakistriphenylphosphine palladium (87.0 mg, 0.0753 mmol) was added, followed by stirring at room temperature (25°C) for 19 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 86.0 mg, 61%).

$^1$H NMR (400 MHz, $CDCl_3$) δ 0.07 (s, 3H), 0.08 (s, 3H), 0.79 (d, J = 6.8 Hz, 3H), 0.92 (s, 9H), 0.96 (d, J = 6.8 Hz, 3H), 1.56 (s, 3H), 1.65 (dd, J = 12.0, 2.0 Hz, 1H), 2.17 (dd, J = 12.0, 6.4 Hz, 1H), 2.22 (sept, J = 6.8 Hz, 1H), 2.31 (s, 3H), 3.97 (dd, J = 6.4, 2.0 Hz, 1H), 5.92 (s, 1H), 6.99-7.03 (m, 1H), 7.08-7.17 (m, 2H), 7.19-7.23 (m, 1H).

Synthesis Example 66

Synthesis of (1S,2R,4R)-4-isopropyl-1-methyl-5-(o-tolyl)-7-oxabicyclo[2.2.1]heptan-2-ol

To a methanol solution (0.8 mL) of tert-butyl(((1S,2R,4R)-4-isopropyl-1-methyl-5-(o-tolyl)-7-oxabicyclo[2.2.1]hept-5-en-2-yl)oxy)dimethylsilane (46.4 mg, 0.125 mmol), palladium carbon (10.0 mg, water content 55 wt%) was added, followed by stirring under a hydrogen atmosphere at room temperature (25°C) for 15 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 20.0 mg, 61%).
$^1$H NMR (400 MHz, $CDCl_3$) δ 0.61 (d, J = 6.8 Hz, 3H), 0.96 (d, J = 6.8 Hz, 3H), 1.36 (dt, J = 13.2, 2.0 Hz, 1H), 1.47 (s, 3H), 1.54 (dd, J = 13.2, 6.8 Hz, 1H), 1.50-1.80 (brs, 1H), 2.18-2.26 (m, 2H), 2.34 (s, 3H), 2.68 (dd, J = 14.0, 6.8 Hz, 1H), 3.51 (ddd, J = 11.6, 6.8, 2.0 Hz, 1H), 3.99 (dd, J = 6.8, 2.4 Hz, 1H), 7.08-7.21 (m, 4H).

Synthesis Example 67

Synthesis of (1S,2R,4R)-2-((2-fluorobenzyl)oxy)-4-isopropyl-1-methyl-5-(o-tolyl)-7-oxabicyclo[2.2.1]heptane (1-925)

To a dimethylformamide solution (1 mL) of (1S,2R,4R)-4-isopropyl-1-methyl-5-(o-tolyl)-7-oxabicyclo[2.2.1]heptan-2-ol (20.0 mg, 0.0768 mmol), sodium hydride (55% dispersion in mineral oil, 6.0 mg, 0.14 mmol) was added, followed by stirring at 0°C for 1 hour. To the reaction mixture, 2-fluorobenzyl bromide (0.011 mL, 0.090 mmol) was added, followed by stirring at room temperature (25°C) for 38 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (25.9 mg, 91%).
$^1$H NMR (400 MHz, $CDCl_3$) δ 0.65 (d, J = 6.8 Hz, 3H), 0.96 (d, J = 6.8 Hz, 3H), 1.50-1.62 (m, 2H), 1.54 (s, 3H), 2.18-2.27 (m, 2H), 2.40 (s, 3H), 2.59 (dd, J = 13.2, 6.8 Hz, 1H), 3.57 (ddd, J = 12.0, 6.4, 1.6 Hz, 1H), 3.85 (dd, J = 6.8, 2.4 Hz, 1H), 4.60 (d, J = 12.8 Hz, 1H), 4.68 (d, J = 12.8 Hz, 1H), 7.01-7.21 (m, 6H), 7.24-7.31 (m, 1H), 7.50 (td, J = 7.6, 1.6 Hz, 1H).

Synthesis Example 68

Synthesis of tert-butyl(((I S,2R,4R)-5-(4-fluorophenyl)-4-isopropyl-1-methyl-7-oxabicyclo[2.2.1]hept-5-en-2-yl)oxy)dimethylsilane

To a 1,2-dimethoxyethane solution (1.1 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (143 mg, 0.332 mmol), potassium carbonate (69.0 mg, 0.499 mmol) and 4-fluorophenylboronic acid (51.0 mg, 0.364 mmol) were added, and argon was bubbled through the mixture for 5 minutes. To the reaction mixture, tetrakistriphenylphosphine palladium (77.0 mg, 0.0666 mmol) was added, followed by stirring at room temperature (25°C) for 15 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 69.5 mg, 56%).
$^1$H NMR (400 MHz, $CDCl_3$) δ 0.057 (s, 3H), 0.063 (s, 3H), 0.83 (d, J = 6.8 Hz, 3H), 0.91 (s, 9H), 1.08 (d, J = 6.8 Hz, 3H), 1.54 (s, 3H), 1.67 (dd, J = 11.2, 2.0 Hz, 1H), 1.98 (dd, J = 11.2, 6.4 Hz, 1H), 2.34 (sept, J = 6.8 Hz, 1H), 3.94 (dd, J = 6.4, 2.0 Hz, 1H), 5.96 (s, 1H), 6.97-7.04 (m, 2H), 7.14-7.11 (m, 2H).

Synthesis Example 69

Synthesis of (1S,2R,4R)-5-(4-fluorophenyl)-4-isopropyl-1-methyl-7-oxabicyclo[2.2.1]heptan-2-ol

To a methanol solution (1 mL) of tert-butyl(((1S,2R,4R)-5-(4-fluorophenyl)-4-isopropyl-1-methyl-7-oxabicyclo[2.2.1]hept-5-en-2-yl)oxy)dimethylsilane (69.3 mg, 0.184 mmol), palladium carbon (10.0 mg, water content 55 wt%) was added, followed by stirring under a hydrogen atmosphere at room temperature (25°C) for 3 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 40.3 mg, 83%).
$^1$H NMR (400 MHz, $CDCl_3$) δ 0.90 (d, J = 6.8 Hz, 3H), 0.91 (d, J = 6.8 Hz, 3H), 1.31 (dt, J = 14.0, 2.4 Hz, 1H), 1.48 (s, 3H), 1.55-1.70 (m, 2H), 2.02 (sept, J = 6.8 Hz, 1H), 2.15 (dd, J = 13.6, 12.0 Hz, 1H), 2.47 (dd, J = 13.6, 6.8 Hz, 1H), 3.33 (ddd, J = 12.0, 6.8, 2.4 Hz, 1H), 3.89-3.97 (m, 1H), 6.95-7.03 (m, 2H), 7.13-7.20 (m, 2H).

Synthesis Example 70

Synthesis of (1S,2R,4R)-2-((2-fluorobenzyl)oxy)-5-(4-fluorophenyl)-4-isopropyl-1-methyl-7-oxabicyclo[2.2.1]heptane (1-924)

To a dimethylformamide solution (1 mL) of (1S,2R,4R)-5-(4-fluorophenyl)-4-isopropyl-1-methyl-7-oxabicyclo [2.2.1]heptan-2-ol (40.0 mg, 0.151 mmol), sodium hydride (55% dispersion in mineral oil, 10.0 mg, 0.229 mmol) was added, followed by stirring at 0°C for 1 hour. To the reaction mixture, 2-fluorobenzyl bromide (0.022 mL, 0.18 mmol) was added, followed by stirring at room temperature (25°C) for 14 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (35.0 mg, 62%).

$^1H$ NMR (400 MHz, $CDCl_3$) δ 0.88 (d, J = 6.8 Hz, 3H), 0.94 (d, J = 6.8 Hz, 3H), 1.50-1.61 (m, 2H), 1.53 (s, 3H), 2.07 (sept, J = 6.8 Hz, 1H), 2.11 (dd, J = 13.2, 11.6 Hz, 1H), 2.36 (dd, J = 13.2, 6.8 Hz, 1H), 3.36 (ddd, J = 11.6, 6.8, 1.6 Hz, 1H), 3.78 (dd, J = 6.8, 2.4 Hz, 1H), 4.56 (d, J = 12.8 Hz, 1H), 4.65 (d, J = 12.8 Hz, 1H), 6.95-7.07 (m, 3H), 7.11-7.18 (m, 3H), 7.23-7.31 (m, 1H), 7.45 (dt, J = 7.6, 1.6 Hz, 1H).

Synthesis Example 71

Synthesis of tert-butyl(((I S,2R,4R)-5-(3-fluorophenyl)-4-isopropyl-1-methyl-7-oxabicyclo[2.2.1]hept-5-en-2-yl)oxy)dimethylsilane

To a 1,2-dimethoxyethane solution (2.2 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (230 mg, 0.534 mmol), potassium carbonate (133 mg, 0.962 mmol) and 3-fluorophenylboronic acid (99.0 mg, 0.708 mmol) were added, and argon was bubbled through the mixture for 5 minutes. To the reaction mixture, tetrakistriphenylphosphine palladium (148 mg, 0.128 mmol) was added, followed by stirring at room temperature (25°C) for 18 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 158 mg, 66%).

$^1H$ NMR (400 MHz, $CDCl_3$) δ 0.06 (s, 3H), 0.06 (s, 3H), 0.84 (d, J = 6.8 Hz, 3H), 0.91 (s, 9H), 1.09 (d, J = 6.8 Hz, 3H), 1.55 (s, 3H), 1.69 (dd, J = 11.6, 2.4 Hz, 1H), 1.98 (dd, J = 11.6, 6.4 Hz, 1H), 2.37 (sept, J = 6.8 Hz, 1H), 3.93 (dd, J = 6.4, 2.0 Hz, 1H), 6.03 (s, 1H), 6.88-7.02 (m, 2H), 7.03-7.10 (m, 1H), 7.23-7.30 (m, 1H).

Synthesis Example 72

Synthesis of (1S,2R,4R)-5-(3-fluorophenyl)-4-isopropyl-1-methyl-7-oxabicyclo[2.2.1]heptan-2-ol

To a methanol solution (1.6 mL) of tert-butyl(((1S,2R,4R)-5-(3-fluorophenyl)-4-isopropyl-1-methyl-7-oxabicyclo [2.2.1]hept-5-en-2-yl)oxy)dimethylsilane (158 mg, 0.420 mmol), palladium carbon (15.0 mg, 55 wt % water content) was added, followed by stirring under a hydrogen atmosphere at room temperature (25°C) for 24 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 63.0 mg, 57%).

$^1H$ NMR (400 MHz, $CDCl_3$) δ 0.90 (d, J = 6.8 Hz, 3H), 0.93 (d, J = 6.8 Hz, 3H), 1.33 (dt, J = 14.4, 2.0 Hz, 1H), 1.47 (s, 3H), 1.59-1.69 (m, 2H), 2.04 (sept, J = 6.8 Hz, 1H), 2.15 (dd, J = 14.4, 12.0 Hz, 1H), 2.49 (dd, J = 14.4, 6.4 Hz, 1H), 3.35 (ddd, J = 12.0, 6.4, 2.0 Hz, 1H), 3.94 (t, J = 6.8 Hz, 1H), 6.88-6.96 (m, 2H), 7.00 (d, J = 7.6 Hz, 1H), 7.22-7.30 (m, 1H).

Synthesis Example 73

Synthesis of (1S,2R,4R)-2-((2-fluorobenzyl)oxy)-5-(3-fluorophenyl)-4-isopropyl-1-methyl-7-oxabicyclo[2.2.1]heptane (1-923)

To a dimethylformamide solution (1 mL) of (1S,2R,4R)-5-(3-fluorophenyl)-4-isopropyl-1-methyl-7-oxabicyclo [2.2.1]heptan-2-ol (70.0 mg, 0.265 mmol), sodium hydride (55% dispersion in mineral oil, 17.0 mg, 0.390 mmol) was added, followed by stirring at 0°C for 1 hour. To the reaction mixture, 2-fluorobenzyl bromide (0.038 mL, 0.31 mmol) was added, followed by stirring at room temperature (25°C) for 21 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (74.4 mg, 75%).

$^1$H NMR (400 MHz, $CDCl_3$) δ 0.90 (d, J = 7.2 Hz, 3H), 0.91 (d, J = 7.2 Hz, 3H), 1.51-1.63 (m, 2H), 1.53 (s, 3H), 2.08 (sept, J = 7.2 Hz, 1H), 2.15 (dd, 13.2, 11.6 Hz, 1H), 2.38 (dd, J = 13.2, 6.4 Hz, 1H), 3.37 (ddd, J = 11.6, 6.4, 2.0 Hz, 1H), 3.79 (dd, J = 6.8, 2.8 Hz, 1H), 4.56 (d, J = 12.8 Hz, 1H), 4.65 (d, J = 12.8 Hz, 1H), 6.87-6.95 (m, 2H), 6.99 (d, J = 7.6 Hz, 1H), 7.04 (td, J = 8.0, 1.6 Hz, 1H), 7.15 (td, J = 7.6, 0.8 Hz, 1H), 7.22-7.31 (m, 2H), 7.48 (td, J = 7.6, 1.6 Hz, 1H).

Synthesis Example 74

Synthesis of tert-butyl(((I S,2R,4R)-5-(2-fluorophenyl)-4-isopropyl-1-methyl-7-oxabicyclo[2.2.1]hept-5-en-2-yl)oxy)dimethylsilane

To a 1,2-dimethoxyethane solution (2.2 mL) of (1R,4S,5R)-5-((tert-butyldimethylsilyl)oxy)-1-isopropyl-4-methyl-7-oxabicyclo[2.2.1]hept-2-en-2-yl trifluoromethanesulfonate (230 mg, 0.534 mmol), potassium carbonate (133 mg, 0.962 mmol) and 2-fluorophenylboronic acid (99.0 mg, 0.708 mmol) were added, and argon was bubbled through the mixture for 5 minutes. To the reaction mixture, tetrakistriphenylphosphine palladium (148 mg, 0.128 mmol) was added, followed by stirring at room temperature (25°C) for 30 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 138 mg, 57%).

$^1$H NMR (400 MHz, $CDCl_3$) δ 0.07 (s, 3H), 0.07 (s, 3H), 0.83 (d, J = 6.8 Hz, 3H), 0.92 (s, 9H), 1.04 (d, J = 6.8 Hz, 3H), 1.55 (s, 3H), 1.61 (dd, J = 11.4, 2.4 Hz, 1H), 2.19-2.33 (m, 2H), 3.98 (dd, J = 6.4, 1.6 Hz, 1H), 6.11 (s, 1H), 7.00-7.12 (m, 2H), 7.14-7.28 (m, 2H).

Synthesis Example 75

Synthesis of (1S,2R,4R)-5-(2-fluorophenyl)-4-isopropyl-1-methyl-7-oxabicyclo[2.2.1]heptan-2-ol

To a methanol solution (1.4 mL) of tert-butyl(((1S,2R,4R)-5-(2-fluorophenyl)-4-isopropyl-1-methyl-7-oxabicyclo [2.2.1]hept-5-en-2-yl)oxy)dimethylsilane (138 mg, 0.366 mmol), palladium carbon (13.0 mg, water content 55 wt%) was added, followed by stirring under a hydrogen atmosphere at room temperature (25°C) for 51 hours. The reaction mixture was filtered through Celite, and the filtrate was concentrated under reduced pressure. The concentrate was purified by silica gel column chromatography (eluent: ethyl acetate/n-hexane) to obtain a colorless liquid title compound (yield 22.2 mg, 23%).

$^1$H NMR (400 MHz, $CDCl_3$) δ 0.87 (d, J = 6.8 Hz, 3H), 0.90 (d, J = 6.8 Hz, 3H), 1.32 (dt, J = 14.4, 2.0 Hz, 1H), 1.48 (s, 3H), 1.60-1.74 (m, 2H), 2.04-2.19 (m, 2H), 2.57 (dd, J = 14.4, 6.8 Hz, 1H), 3.66 (ddd, J = 11.2, 6.8, 2.0 Hz, 1H), 3.94-4.03 (brs, 1H), 6.97-7.05 (m, 1H), 7.08-7.14 (m1H), 7.16-7.24 (m, 2H).

Synthesis Example 76

Synthesis of (1S,2R,4R)-2-((2-fluorobenzyl)oxy)-5-(2-fluorophenyl)-4-isopropyl-1-methyl-7-oxabicyclo[2.2.1]heptane (1-922)

To a dimethylformamide solution (1 mL) of (1S,2R,4R)-5-(2-fluorophenyl)-4-isopropyl-1-methyl-7-oxabicyclo [2.2.1]heptan-2-ol (79.6 mg, 0.301 mmol), sodium hydride (55% dispersion in mineral oil, 19.0 mg, 0.435 mmol) was added, followed by stirring at 0°C for 1 hour. To the reaction mixture, 2-fluorobenzyl bromide (0.044 mL, 0.36 mmol) was added, followed by stirring at room temperature (25°C) for 27 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (68.9 mg, 61%).

$^1$H NMR (400 MHz, $CDCl_3$) δ 0.84 (d, J = 6.8 Hz, 3H), 0.94 (d, J = 6.8 Hz, 3H), 1.52-1.56 (m, 1H), 1.53 (s, 3H), 1.63 (dd, J = 13.2, 6.0 Hz, 1H), 2.08-2.18 (m, 2H), 2.44 (dd, J = 13.6, 6.8 Hz, 1H), 3.68 (ddd, J = 12.0, 6.8, 1.6 Hz, 1H), 3.83 (dd, J = 6.0, 2.8 Hz, 1H), 4.56 (d, J = 12.4 Hz, 1H), 4.65 (d, J = 12.4 Hz, 1H), 6.97-7.22 (m, 6H), 7.23-7.31 (m, 1H), 7.49 (dt, 7.2, 1.2 Hz, 1H).

Synthesis Example 77

Synthesis of (1R,2S,4S,5R)-5-((2-fluorobenzyl)oxy)-1-isopropyl-4-methyl-7-oxabicyclo[2.2.1]heptan-2-yl trifluoromethanesulfonate (1-525)

To a dichloromethane solution (0.67 mL) of (1R,2S,4S,5R)-5-((2-fluorobenzyl)oxy)-1-isopropyl-4-methyl-7-oxabicyclo[2.2.1]heptan-2-ol (39.5 mg, 0.134 mmol), trifluoromethanesulfonic anhydride (0.052 mL, 0.32 mmol) and pyridine (0.022 mL, 0.27 mmol) were added, followed by stirring at room temperature (25°C) for 4 hours. A saturated ammonium chloride aqueous solution was poured into the reaction mixture, which was then extracted with dichloromethane. The extract was dried over anhydrous magnesium sulfate and then concentrated under reduced pressure. The concentrate was purified by silica gel column chromatography (eluent: ethyl acetate/n-hexane) to obtain a colorless liquid title compound (43.8 mg, 77%).

$^1$H NMR (400 MHz, $CDCl_3$) δ 1.04 (d, J = 6.8 Hz, 3H), 1.05 (dJ = 6.8 Hz, 3H), 1.45 (s, 3H), 1.63 (dd, J = 13.6, 2.4 Hz, 1H), 1.70 (dt, J = 13.6, 1.6 Hz, 1H), 2.16 (sept, J = 6.8 Hz, 1H), 2.24 (dd, J = 13.6, 10.0 Hz, 1H), 2.59 (dd, J = 13.6, 6.8 Hz, 1H), 3.72 (dd, J = 6.8, 2.4 Hz, 1H), 4.53 (d, J = 12.0 Hz, 1H), 4.62 (d, J = 12.0 Hz, 1H), 5.01-5.08 (m, 1H), 6.99-7.08 (m, 1H), 7.14 (td, J = 7.2, 0.8 Hz, 1H), 7.25-7.32 (m, 1H), 7.42 (td, J = 7.2, 1.2 Hz, 1H).

Synthesis Example 78

Synthesis of tert-butyl((((1S,2R,4R,5S)-5-chloro-4-isopropyl-1-methyl-7-oxabicyclo[2.2.1]heptan-2-yl)oxy)dimethylsilane

A thionyl chloride solution (4 mL) of (1R,2S,4S,5R)-5-((tert-butyldimethylsilyl)oxy)-1-isopropyl-4-methyl-7-oxabicyclo[2.2.1]heptan-2-ol (96.1 mg, 0.320 mmol) was stirred under heating and reflux for 3 hours. The reaction mixture was concentrated under reduced pressure, and the concentrate was purified by silica gel column chromatography (eluent: ethyl acetate/n-hexane) to obtain a colorless liquid title compound (yield 61.7 mg, 60%).

$^1$H NMR (400 MHz, $CDCl_3$) δ 0.06 (s, 6H), 0.90 (s, 9H), 1.04 (d, J = 6.8 Hz, 3H), 1.05 (d, J = 6.8 Hz, 3H), 1.32 (s, 3H), 1.40-1.50 (m, 2H), 2.11 (sept, J = 6.8 Hz, 1H), 2.25 (dd, J = 13.2, 10.4 Hz, 1H), 2.80 (dd, J = 13.2, 6.8 Hz, 1H), 3.86 (dd, J = 6.8, 2.4 Hz, 1H), 4.01 (ddd, J = 10.4, 6.8, 2.0 Hz, 1H).

Synthesis Example 79

Synthesis of (1S,2R,4R,5S)-5-chloro-4-isopropyl-1-methyl-7-oxabicyclo[2.2.1]heptan-2-ol

To a tetrahydrofuran solution (1.9 mL) of tert-butyl((((1S,2R,4R,5S)-5-chloro-4-isopropyl-1-methyl-7-oxabicyclo [2.2.1]heptan-2-yl)oxy)dimethylsilane (61.7 mg, 0.193 mmol), tetrabutylammonium fluoride (0.29 mL, 1 mol/L tetrahydrofuran solution, 0.29 mmol) was added, followed by stirring at room temperature (25°C) for 21 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a white solid title compound (yield 29.4 mg, 75%).

$^1$H NMR (400 MHz, $CDCl_3$) δ 1.04 (dd, J = 6.8 Hz, 3H), 1.05 (dd, J = 6.8 Hz, 3H), 1.38 (s, 3H), 1.42 (dt, J = 14.0, 2.0 Hz, 1H), 1.45-1.65 (brs, 1H), 1.49 (dd, J = 13.6, 4.4 Hz, 1H), 2.09 (sept, J = 6.8 Hz, 1H), 2.28 (dd, J = 13.6, 10.4 Hz, 1H), 2.87 (dd, J = 14.0, 6.8 Hz, 1H), 3.83 (dd, J = 4.4, 2.0 Hz, 1H), 4.00 (ddd, J = 10.4, 6.8, 2.0 Hz, 1H).

Synthesis Example 80

Synthesis of (1R,2S,4S,5R)-2-chloro-5-((2-fluorobenzyl)oxy(-1-isopropyl-4-methyl-7-oxabicyclo[2.2.1]heptane (1-12)

To a dimethylformamide solution (1.4 mL) of (1S,2R,4R,5S)-5-chloro-4-isopropyl-1-methyl-7-oxabicyclo[2.2.1] heptan-2-ol (29.4 mg, 0.144 mmol), sodium hydride (55% dispersion in mineral oil, 31.5 mg, 0.722 mmol) was added, followed by stirring at 0°C for 1 hour. To the reaction mixture, 2-fluorobenzyl bromide (0.052 mL, 0.42 mmol) was added, followed by stirring at room temperature (25°C) for 16 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (43.5 mg, 97%).

$^1$H NMR (400 MHz, $CDCl_3$) δ 1.06 (d, J = 7.2 Hz, 6H), 1.44 (s, 3H), 1.52 (dd, J = 13.2, 4.8 Hz, 1H), 1.63 (dt, J = 13.2, 2.0 Hz, 1H), 2.13 (sept, J = 7.2 Hz, 1H), 2.26 (dd, J = 13.2, 10.4 Hz, 1H), 2.80 (dd, J = 13.2, 6.8 Hz, 1H), 3.68 (dd, J = 6.8, 2.0 Hz, 1H), 4.03 (ddd, J = 10.4, 4.8, 2.0 Hz, 1H), 4.52 (d, J = 12.4 Hz, 1H), 4.62 (d, J = 12.4 Hz, 1H), 7.00-7.06 (m, 1H), 7.13 (td, J = 7.2, 0.8 Hz, 1H), 7.23-7.30 (m, 1H), 7.44 (td, J = 7.2, 0.8 Hz, 1H).

Synthesis Example 81

Synthesis of (1R,4S,5R)-1-isopropyl-4-methyl-5-((3-methylbenzyl)oxy)-7-oxabicyclo[2.2.1]heptan-2-one (2-352)

The same reaction and treatment as in Synthesis Example 18 were performed using 3-methylbenzyl bromide instead of 2-methylbenzyl bromide to obtain a colorless liquid title compound (yield 47%).

$^1$H NMR (400 MHz, $CDCl_3$) δ 1.06 (d, J = 6.8 Hz, 3H), 1.07 (d, J = 6.8 Hz, 3H), 1.59 (s, 3H), 1.82 (dd, J = 13.6, 2.8 Hz, 1H), 1.96 (dd, J = 13.6, 6.8 Hz, 1H), 2.05 (d, J = 18.0 Hz, 1H), 2.17 (sept, J = 6.8 Hz, 1H), 2.22 (d, J = 18.0 Hz, 1H), 2.35 (s, 3H), 3.69 (dd, J = 6.8, 2.8 Hz, 1H), 4.41 (d, J = 12.4 Hz, 1H), 4.56 (d, J = 12.4 Hz, 1H), 7.08-7.15 (m, 3H), 7.23 (t, J = 7.2 Hz, 1H).

Synthesis Example 82

Synthesis of (1R,4S,5R)-1-isopropyl-4-methyl-5-[(4-methylbenzyl)oxy]-7-oxabicyclo[2.2.1]heptan-2-one (2-350)

The same reaction and treatment as in Synthesis Example 18 were performed using 4-methylbenzyl bromide instead of 2-methylbenzyl bromide to obtain a colorless liquid title compound (yield 38%).

$^1$H NMR (400 MHz, $CDCl_3$) δ 1.06 (d, J = 6.8 Hz, 3H), 1.07 (d, J = 6.8 Hz, 3H), 1.57 (s, 3H), 1.81 (dd, J = 13.6, 2.8 Hz, 1H), 1.95 (dd, J = 13.6, 6.8 Hz, 1H), 2.04 (d, J = 17.2 Hz, 1H), 2.17 (sept, J = 6.8 Hz, 1H), 2.21 (d, J = 17.2 Hz, 1H), 2.35 (s, 3H), 3.68 (dd, J = 6.8, 2.8 Hz, 1H), 4.40 (d, J = 12.4 Hz, 1H), 4.56 (d, J = 12.4 Hz, 1H), 7.15 (d, J = 8.0 Hz, 2H), 7.21 (d, J = 8.0 Hz, 2H).

Synthesis Example 83

Synthesis of (1R,4S,5R)-5-((3-fluorobenzyl)oxy)- 1 -isopropyl-4-methyl-7-oxabicyclo[2.2.1]heptan-2-one (2-351)

The same reaction and treatment as in Synthesis Example 18 were performed using 3-fluorobenzyl bromide instead of 2-methylbenzyl bromide to obtain a colorless liquid title compound (yield 45%).

$^1$H NMR (400 MHz, $CDCl_3$) δ 1.07 (d, J = 6.8 Hz, 3H), 1.08 (d, J = 6.8 Hz, 3H), 1.60 (s, 3H), 1.82 (dd, J = 13.6, 2.8 Hz, 1H), 1.99 (dd, J = 13.6, 6.4 Hz, 1H), 2.06 (d, J = 17.2 Hz, 1H), 2.18 (sept, J = 6.8 Hz, 1H), 2.24 (d, J = 17.2 Hz, 1H), 3.71 (dd, J = 6.4, 2.8 Hz, 1H), 4.44 (d, J = 12.4 Hz, 1H), 4.58 (d, J = 12.4 Hz, 1H), 6.98 (td, J = 8.4, 2.4 Hz, 1H), 7.03-7.11 (m, 2H), 7.30 (td, J = 8.4, 6.0 Hz, 1H).

Synthesis Example 84

Synthesis of (1R,4S,5R)-5-((4-fluorobenzyl)oxy)-1-isopropyl-4-methyl-7-oxabicyclo[2.2.1]heptan-2-one (2-349)

The same reaction and treatment as in Synthesis Example 18 were performed using 4-fluorobenzyl bromide instead of 2-methylbenzyl bromide to obtain a colorless liquid title compound (yield 47%).

$^1$H NMR (400 MHz, $CDCl_3$) δ 1.06 (d, J = 6.8 Hz, 3H), 1.07 (d, J = 6.8 Hz, 3H), 1.58 (s, 3H), 1.81 (dd, J = 13.6, 2.8 Hz, 1H), 1.98 (dd, J = 13.6, 6.4 Hz, 1H), 2.06 (d, J = 17.2 Hz, 1H), 2.17 (sept, J = 6.8 Hz, 1H), 2.23 (d, J = 17.2 Hz, 1H), 3.69 (dd, J = 6.4, 2.8 Hz, 1H), 4.40 (d, J = 12.4 Hz, 1H), 4.55 (d, J = 12.4 Hz, 1H), 6.99-7.07 (m, 2H), 7.26-7.33 (m, 2H).

Synthesis Example 85

Synthesis of (1R,4S,5R)-1-isopropyl-5-((2-methoxybenzyl)oxy)-4-methyl-7-oxabicyclo[2.2.1]heptan-2-one (2-7)

The same reaction and treatment as in Synthesis Example 18 were performed using 2-methoxybenzyl iodide instead of 2-methylbenzyl bromide to obtain a colorless liquid title compound (yield 32%).

$^1$H NMR (400 MHz, $CDCl_3$) δ 1.06 (d, J = 6.8 Hz, 3H), 1.07 (d, J = 6.8 Hz, 3H), 1.60 (s, 3H), 1.86 (dd, J = 13.6, 2.8 Hz, 1H), 1.99 (dd, J = 13.6, 6.4 Hz, 1H), 2.07 (d, J = 18.0 Hz, 1H), 2.17 (sept, J = 6.8 Hz, 1H), 2.23 (d, J = 18.0 Hz, 1H), 3.74 (dd, J = 6.4, 2.8 Hz, 1H), 3.82 (s, 3H), 4.51 (d, J = 12.8 Hz, 1H), 4.60 (d, J = 12.8 Hz, 1H), 6.86 (d, J = 8.4 Hz, 1H), 6.96 (t, J = 7.2 Hz, 1H), 7.27 (td, J = 7.2, 2.0 Hz, 1H), 7.40 (dd, J = 7.2, 2.0 Hz, 1H).

Synthesis Example 86

Synthesis of (1R,4S,5R)-1-isopropyl-5-((3-methoxybenzyl)oxy)-4-methyl-7-oxabicyclo[2.2.1]heptan-2-one (2-347)

The same reaction and treatment as in Synthesis Example 18 were performed using 3-methoxybenzyl chloride instead of 2-methylbenzyl bromide to obtain a colorless liquid title compound (yield 21%).

$^1$H NMR (400 MHz, $CDCl_3$) δ 1.06 (d, J = 6.8 Hz, 3H), 1.07 (d, J = 6.8 Hz, 3H), 1.60 (s, 3H), 1.82 (dd, J = 14.0, 2.8 Hz, 1H), 1.97 (dd, J = 14.0, 6.8 Hz, 1H), 2.05 (d, J = 18.0 Hz, 1H), 2.17 (sept, J = 6.8 Hz, 1H), 2.22 (d, J = 18.0 Hz, 1H), 3.70 (dd, J = 6.8, 2.8 Hz, 1H), 3.81 (s, 3H), 4.42 (d, J = 12.4 Hz, 1H), 4.58 (d, J = 12.4 Hz, 1H), 6.81-6.86 (m, 1H), 6.88-6.92 (m, 2H), 7.23-7.29 (m, 1H).

Synthesis Example 87

Synthesis of (1R,4S,5R)-1-isopropyl-5-((4-methoxybenzyl)oxy)-4-methyl-7-oxabicyclo[2.2.1]heptan-2-one (2-346)

The same reaction and treatment as in Synthesis Example 18 were performed using 4-methoxybenzyl iodide instead of 2-methylbenzyl bromide to obtain a colorless liquid title compound (yield 25%).

$^1$H NMR (400 MHz, $CDCl_3$) δ 1.06 (d, J = 6.8 Hz, 3H), 1.07 (d, J = 6.8 Hz, 3H), 1.56 (s, 3H), 1.80 (dd, J = 13.6, 2.8 Hz, 1H), 1.95 (dd, J = 13.6, 6.8 Hz, 1H), 2.04 (d, J = 17.6 Hz, 1H), 2.17 (sept, J = 6.8 Hz, 1H), 2.21 (d, J = 17.6 Hz, 1H), 3.67 (dd, J = 6.8, 2.8 Hz, 1H), 3.81 (s, 3H), 4.38 (d, J = 12.0 Hz, 1H), 4.54 (d, J = 12.0 Hz, 1H), 6.85-6.90 (m, 2H), 7.22-7.27 (m, 2H).

Synthesis Example 88

Synthesis of (1R,4S,5R)-5-((2-chlorobenzyl)oxy)-1-isopropyl-4-methyl-7-oxabicyclo[2.2.1]heptan-2-one (2-11)

The same reaction and treatment as in Synthesis Example 18 were performed using 2-chlorobenzyl bromide instead of 2-methylbenzyl bromide to obtain a colorless liquid title compound (yield 49%).

$^1$H NMR (400 MHz, $CDCl_3$) δ 1.07 (d, J = 6.8 Hz, 3H), 1.08 (d, J = 6.8 Hz, 3H), 1.61 (s, 3H), 1.87 (dd, J = 13.6, 2.8 Hz, 1H), 2.03 (dd, J = 13.6, 6.8 Hz, 1H), 2.09 (d, J = 17.2 Hz, 1H), 2.18 (sept, J = 6.8 Hz, 1H), 2.25 (d, J = 17.2 Hz, 1H), 3.77 (dd, J = 6.8, 2.8 Hz, 1H), 4.54 (d, J = 13.6 Hz, 1H), 4.65 (d, J = 13.6 Hz, 1H), 7.20-7.30 (m, 2H), 7.35 (dd, J = 7.2, 1.2 Hz, 1H), 7.48-7.52 (m, 1H).

Synthesis Example 89

Synthesis of (1R,4S,5R)-5-((2-bromobenzyl)oxy)-1-isopropyl-4-methyl-7-oxabicyclo[2.2.1]heptan-2-one (2-13)

The same reaction and treatment as in Synthesis Example 18 were performed using 2-bromobenzyl bromide instead of 2-methylbenzyl bromide to obtain a colorless liquid title compound (yield 32%).

$^1$H NMR (400 MHz, $CDCl_3$) δ 1.07 (d, J = 6.8 Hz, 3H), 1.08 (d, J = 6.8 Hz, 3H), 1.62 (s, 3H), 1.87 (dd, J = 13.6, 2.8 Hz, 1H), 2.04 (dd, J = 13.6, 6.4 Hz, 1H), 2.10 (d, J = 17.6 Hz, 1H), 2.18 (sept, J = 6.8 Hz, 1H), 2.25 (d, J = 17.6 Hz, 1H), 3.78 (dd, J = 6.4, 2.8 Hz, 1H), 4.51 (d, J = 13.2 Hz, 1H), 4.61 (d, J = 13.2 Hz, 1H), 7.16 (td, J = 7.6, 1.2 Hz, 1H), 7.32 (t, J = 7.6 Hz, 1H), 7.50 (dd, J = 7.6, 1.2 Hz, 1H), 7.54 (d, J = 7.6 Hz, 1H).

Synthesis Example 90

Synthesis of 2-((((1S,2R,4R)-4-isopropyl-1-methyl-5-oxo-7-oxabicyclo[2.2.1]heptan-2-yl)oxy)methyl)benzonitrile (2-354)

The same reaction and treatment as in Synthesis Example 18 were performed using 2-cyanobenzyl bromide instead of 2-methylbenzyl bromide to obtain a colorless liquid title compound (yield 56%).

$^1$H NMR (400 MHz, $CDCl_3$) δ 1.076 (d, J = 6.8 Hz, 3H), 1.080 (d, J = 6.8 Hz, 3H), 1.61 (s, 3H), 1.88 (dd, J = 13.6, 2.4 Hz, 1H), 2.07 (dd, J = 13.6, 6.8 Hz, 1H), 2.12 (d, J = 17.2 Hz, 1H), 2.19 (sept, J = 6.8 Hz, 1H), 2.26 (d, J = 17.2 Hz, 1H), 3.82 (dd, J = 6.8, 2.4 Hz, 1H), 4.62 (d, J = 12.4 Hz, 1H), 4.73 (d, J = 12.4 Hz, 1H), 7.38-7.45 (m, 1H), 7.57-7.70 (m, 3H).

Synthesis Example 91

Synthesis of (1R,4S,5R)-1-isopropyl-4-methyl-5-((2-(trifluoromethyl)benzyl)oxy)-7-oxabicyclo[2.2.1]heptan-2-one (2-29)

The same reaction and treatment as in Synthesis Example 18 were performed using 2-(trifluoromethyl)benzyl bromide instead of 2-methylbenzyl bromide to obtain a colorless liquid title compound (yield 22%).
$^1$H NMR (400 MHz, $CDCl_3$) δ 1.07 (d, J = 6.8 Hz, 3H), 1.08 (d, J = 6.8 Hz, 3H), 1.61 (s, 3H), 1.85 (dd, J = 14.0, 2.8 Hz, 1H), 2.03 (dd, J = 14.0, 6.8 Hz, 1H), 2.09 (d, J = 17.2 Hz, 1H), 2.18 (sept, J = 6.8 Hz, 1H), 2.26 (d, J = 18.0 Hz, 1H), 3.76 (dd, J = 6.8, 2.8 Hz, 1H), 4.63 (d, J = 13.2 Hz, 1H), 4.73 (d, J = 13.2 Hz, 1H), 7.39 (t, J = 8.0 Hz, 1H), 7.56 (t, J = 8.0 Hz, 1H), 7.64 (d, J = 8.0 Hz, 1H), 7.72 (d, J = 8.0 Hz, 1H).

Synthesis Example 92

Synthesis of (1R,4S,5R)-1-isopropyl-4-methyl-5-((2-(trifluoromethoxy)benzyl)oxy)-7-oxabicyclo[2.2.1]heptan-2-one (2-348)

The same reaction and treatment as in Synthesis Example 18 were performed using 2-(trifluoromethoxy)benzyl bromide instead of 2-methylbenzyl bromide to obtain a colorless liquid title compound (yield 54%).
$^1$H NMR (400 MHz, $CDCl_3$) δ 1.07 (d, J = 6.8 Hz, 3H), 1.08 (d, J = 6.8 Hz, 3H), 1.59 (s, 3H), 1.84 (dd, J = 13.6, 2.8 Hz, 1H), 2.02 (dd, J = 13.6, 6.4 Hz, 1H), 2.08 (d, J = 18.0 Hz, 1H), 2.18 (sept, J = 6.8 Hz, 1H), 2.25 (d, J = 18.0 Hz, 1H), 3.74 (dd, J = 6.4, 2.8 Hz, 1H), 4.52 (d, J = 12.8 Hz, 1H), 4.63 (d, J = 12.8 Hz, 1H), 7.21-7.26 (m, 1H), 7.27-7.37 (m, 2H), 7.54 (dd, J = 7.2, 2.0 Hz, 1H).

Synthesis Example 93

Synthesis of (1R,4S,5R)-5-(benzyloxy)-1-isopropyl-4-methyl-7-oxabicyclo[2.2.1]heptan-2-one (2-1)

The same reaction and treatment as in Synthesis Example 18 were performed using benzyl bromide instead of 2-methylbenzyl bromide to obtain a colorless liquid title compound (yield 72%).
$^1$H NMR (400 MHz, $CDCl_3$) δ 1.06 (d, J = 6.8 Hz, 3H), 1.07 (d, J = 6.8 Hz, 3H), 1.59 (s, 3H), 1.82 (dd, J = 13.6, 2.8 Hz, 1H), 1.97 (dd, J = 13.6, 6.8 Hz, 1H), 2.07 (d, J = 17.2 Hz, 1H), 2.17 (sept, J = 6.8 Hz, 1H), 2.23 (d, J = 17.2 Hz, 1H), 3.70 (dd, J = 6.8, 2.8 Hz, 1H), 4.45 (d, J = 12.4 Hz, 1H), 4.60 (d, J = 12.4 Hz, 1H), 7.26-7.38 (m, 5H).

Synthesis Example 94

Synthesis of (1R,4S,5R)-5-((2,3-difluorobenzyl)oxy)-1-isopropyl-4-methyl-7-oxabicyclo[2.2.1]heptan-2-one (2-14)

The same reaction and treatment as in Synthesis Example 18 were performed using 2,3-difluorobenzyl bromide instead of 2-methylbenzyl bromide to obtain a colorless liquid title compound (yield 40%).
$^1$H NMR (400 MHz, $CDCl_3$) δ 1.067 (d, J = 6.8 Hz, 3H), 1.072 (d, J = 6.8 Hz, 3H), 1.58 (s, 3H), 1.83 (dd, J = 13.6, 2.8 Hz, 1H), 2.02 (dd, J = 13.6, 6.8 Hz, 1H), 2.09 (d, J = 17.2 Hz, 1H), 2.18 (sept, J = 6.8 Hz, 1H), 2.25 (d, J = 17.2 Hz, 1H), 3.74 (dd, J = 6.8, 2.8 Hz, 1H), 4.53 (dd, J = 12.4, 0.8 Hz, 1H), 4.63 (dd, J = 12.4, 0.8 Hz, 1H), 7.04-7.22 (m, 3H).

Synthesis Example 95

Synthesis of (1R,4S,5R)-5-((2,4-difluorobenzyl)oxy)-1-isopropyl-4-methyl-7-oxabicyclo[2.2.1]heptan-2-one (2-16)

The same reaction and treatment as in Synthesis Example 18 were performed using 2,4-difluorobenzyl bromide instead of 2-methylbenzyl bromide to obtain a colorless liquid title compound (yield 50%).
$^1$H NMR (400 MHz, $CDCl_3$) δ 1.055 (d, J = 6.8 Hz, 3H), 1.062 (d, J = 6.8 Hz, 3H), 1.55 (s, 3H), 1.81 (dd, J = 13.6, 2.8 Hz, 1H), 2.00 (dd, J = 13.6, 6.4 Hz, 1H), 2.07 (d, J = 17.6 Hz, 1H), 2.16 (sept, J = 6.8 Hz, 1H), 2.23 (d, J = 17.6 Hz, 1H), 3.71 (dd, J = 6.4, 2.8 Hz, 1H), 4.46 (d, J = 12.0 Hz, 1H), 4.56 (d, J = 12.0 Hz, 1H), 6.76-6.83 (m, 1H), 6.84-6.90 (m, 1H), 7.38 (td, J = 8.0, 6.4 Hz, 1H).

Synthesis Example 96

Synthesis of (1R,4S,5R)-5-((2,5-difluorobenzyl)oxy)-1-isopropyl-4-methyl-7-oxabicyclo[2.2.1]heptan-2-one (2-18)

The same reaction and treatment as in Synthesis Example 18 were performed using 2,5-difluorobenzyl bromide

instead of 2-methylbenzyl bromide to obtain a colorless liquid title compound (yield 43%).

$^1$H NMR (400 MHz, $CDCl_3$) δ 1.07 (d, J = 6.8 Hz, 3H), 1.08 (d, J = 6.8 Hz, 3H), 1.60 (s, 3H), 1.83 (dd, J = 13.6, 2.8 Hz, 1H), 2.02 (dd, J = 13.6, 6.8 Hz, 1H), 2.09 (d, J = 17.6 Hz, 1H), 2.18 (sept, J = 6.8 Hz, 1H), 2.25 (d, J = 17.6 Hz, 1H), 3.74 (dd, J = 6.8, 2.8 Hz, 1H), 4.49 (d, J = 12.8 Hz, 1H), 4.59 (d, J = 12.8 Hz, 1H), 6.91-7.03 (m, 2H), 7.12-7.19 (m, 1H).

Synthesis Example 97

Synthesis of (1R,4S,5R)-5-((2,6-difluorobenzyl)oxy)-1-isopropyl-4-methyl-7-oxabicyclo[2.2.1]heptan-2-one (2-20)

The same reaction and treatment as in Synthesis Example 18 were performed using 2,6-difluorobenzyl bromide instead of 2-methylbenzyl bromide to obtain a colorless liquid title compound (yield 48%).

$^1$H NMR (400 MHz, $CDCl_3$) δ 1.056 (d, J = 6.8 Hz, 3H), 1.063 (d, J = 6.8 Hz, 3H), 1.51 (s, 3H), 1.83 (dd, J = 14.0, 2.8 Hz, 1H), 2.01 (dd, J = 14.0, 6.8 Hz, 1H), 2.07 (d, J = 17.6 Hz, 1H), 2.16 (sept, J = 6.8 Hz, 1H), 2.22 (d, J = 17.6 Hz, 1H), 3.71 (dd, J = 6.8, 2.8 Hz, 1H), 4.54 (d, J = 10.8 Hz, 1H), 4.63 (d, J = 10.8 Hz, 1H), 6.87-6.93 (m, 2H), 7.26-7.33 (m, 1H).

Synthesis Example 98

Synthesis of (1R,4S,5R)-5-((2,6-dimethylbenzyl)oxy)-1-isopropyl-4-methyl-7-oxabicyclo[2.2.1]heptan-2-one (2-5)

The same reaction and treatment as in Synthesis Example 18 were performed using 2,6-dimethylbenzyl bromide instead of 2-methylbenzyl bromide to obtain a colorless liquid title compound (yield 43%).

Melting point: 68.0°C to 73.7°C; 'H NMR (400 MHz, $CDCl_3$) δ 1.06 (d, J = 7.2 Hz, 3H), 1.07 (d, J = 6.8 Hz, 3H), 1.52 (s, 3H), 1.85 (dd, J = 13.6, 2.4 Hz, 1H), 2.00 (dd, J = 13.6, 6.8 Hz, 1H), 2.07 (d, J = 17.6 Hz, 1H), 2.18 (sept, J = 6.8 Hz, 1H), 2.22 (d, J = 17.6 Hz, 1H), 2.39 (s, 6H), 3.65 (dd, J = 6.8, 2.4 Hz, 1H), 4.46 (d, J = 10.4 Hz, 1H), 4.57 (d, J = 10.4 Hz, 1H), 6.99-7.04 (m, 2H), 7.08-7.14 (m, 1H).

Synthesis Example 99

Synthesis of (1R,4S,5R)-5-((2,6-dichlorobenzyl)oxy)-1-isopropyl-4-methyl-7-oxabicyclo[2.2.1]heptan-2-one (2-26)

The same reaction and treatment as in Synthesis Example 18 were performed using 2,6-dichlorobenzyl bromide instead of 2-methylbenzyl bromide to obtain a colorless liquid title compound (yield 46%).

Melting point: 101°C to 104°C; $^1$H NMR (400 MHz, $CDCl_3$) δ 1.06 (d, J = 6.8 Hz, 3H), 1.07 (d, J = 6.8 Hz, 3H), 1.54 (s, 3H), 1.92 (dd, J = 13.6, 2.4 Hz, 1H), 2.03 (dd, J = 13.6, 6.4 Hz, 1H), 2.08 (d, J = 17.2 Hz, 1H), 2.18 (sept, J = 6.8 Hz, 1H), 2.23 (d, J = 17.2 Hz, 1H), 3.71 (dd, J = 6.4, 2.4 Hz, 1H), 4.71 (d, J = 10.4 Hz, 1H), 4.79 (d, J = 10.4 Hz, 1H), 7.17-7.23 (m, 2H), 7.32 (d, J = 8.4 Hz, 1H).

Synthesis Example 100

Synthesis of (1R,4S,5R)-5-((2-fluoro-6-methylbenzyl)oxy)-1-isopropyl-4-methyl-7-oxabicyclo[2.2.1]heptan-2-one (2-353)

The same reaction and treatment as in Synthesis Example 18 were performed using 2-fluoro-6-methylbenzyl bromide instead of 2-methylbenzyl bromide to obtain a colorless liquid title compound (yield 20%).

$^1$H NMR (400 MHz, $CDCl_3$) δ 1.06 (d, J = 6.8 Hz, 3H), 1.07 (d, J = 6.8 Hz, 3H), 1.50 (s, 3H), 1.83 (dd, J = 14.0, 2.4 Hz, 1H), 2.02 (dd, J = 14.0, 6.8 Hz, 1H), 2.05 (d, J = 17.6 Hz, 1H), 2.17 (sept, J = 6.8 Hz, 1H), 2.20 (d, J = 17.6 Hz, 1H), 2.42 (s, 3H), 3.64 (dd, J = 6.8, 2.4 Hz, 1H), 4.54 (dd, J = 11.2, 2.0 Hz, 1H), 4.60 (dd, J = 11.2, 2.0 Hz, 1H), 6.89 (t, J = 8.4 Hz, 1H), 6.98 (d, J = 8.4 Hz, 1H), 7.19 (td, J = 8.4, 6.0 Hz, 1H).

Synthesis Example 101

Synthesis of (1R,4S,5R)-5-((2-chloro-6-fluorobenzyl)oxy)-1-isopropyl-4-methyl-7-oxabicyclo[2.2.1]heptan-2-one (2-28)

The same reaction and treatment as in Synthesis Example 18 were performed using 2-chloro-6-fluorobenzyl bromide instead of 2-methylbenzyl bromide to obtain a colorless liquid title compound (yield 53%).

$^1$H NMR (400 MHz, $CDCl_3$) δ 1.06 (d, J = 6.8 Hz, 3H), 1.07 (d, J = 6.8 Hz, 3H), 1.52 (s, 3H), 1.87 (dd, J = 14.0, 2.8 Hz, 1H), 2.02 (dd, J = 14.0, 6.4 Hz, 1H), 2.07 (d, J = 17.6 Hz, 1H), 2.17 (sept, J = 6.8 Hz, 1H), 2.22 (d, J = 17.6 Hz, 1H), 3.70

(dd, J = 6.4, 2.8 Hz, 1H), 4.60 (dd, J = 11.2, 2.4 Hz, 1H), 4.69 (dd, J = 11.2, 2.4 Hz, 1H), 6.97 - 7.03 (m, 1H), 7.18-7.31 (m, 2H).

Synthesis Example 102

Synthesis of (1R,4S,5R)-5-((2-bromo-6-fluorobenzyl)oxy)-1-isopropyl-4-methyl-7-oxabicyclo[2.2.1]heptan-2-one (2-355)

The same reaction and treatment as in Synthesis Example 18 were performed using 2-bromo-6-fluorobenzyl bromide instead of 2-methylbenzyl bromide to obtain a colorless liquid title compound (yield 38%).
$^{1}H$ NMR (400 MHz, $CDCl_3$) δ 1.06 (d, J = 6.8 Hz, 3H), 1.07 (d, J = 6.8 Hz, 3H), 1.53 (s, 3H), 1.89 (dd, J = 13.6, 2.8 Hz, 1H), 2.02 (dd, J = 13.6, 6.8 Hz, 1H), 2.08 (d, J = 17.2 Hz, 1H), 2.17 (sept, J = 6.8 Hz, 1H), 2.22 (d, J = 17.2 Hz, 1H), 3.70 (dd, J = 6.8, 2.8 Hz, 1H), 4.60 (dd, J = 11.2, 2.4 Hz, 1H), 4.69 (dd, J = 11.2, 2.4 Hz, 1H), 7.04 (t, J = 8.4 Hz, 1H), 7.19 (td, J = 8.4, 6.0 Hz, 1H), 7.37-7.41 (m, 1H).

Synthesis Example 103

Synthesis of (1S,2R,4R)-5-(2-fluorophenyl)-4-isopropyl-1-methyl-7-oxabicyclo[2.2.1]hept-5-en-2-ol

To a tetrahydrofuran solution (8.5 mL) of tert-butyl(((1S,2R,4R)-5-(2-fluorophenyl)-4-isopropyl-1-methyl-7-oxabicyclo[2.2.1]hept-5-en-2-yl)oxy)dimethylsilane (296 mg, 0.845 mmol), tetrabutylammonium fluoride (1.28 mL, 1 mol/L tetrahydrofuran solution, 1.28 mmol) was added, followed by stirring at room temperature (25°C) for 15 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 130 mg, 58%).
$^{1}H$ NMR (400 MHz, $CDCl_3$) δ 0.84 (d, J = 6.8 Hz, 3H), 1.03 (d, J = 6.8 Hz, 3H), 1.50 (d, J = 9.6 Hz, 1H), 1.59-1.63 (m, 1H), 1.61 (s, 3H), 2.30 (septd, J = 6.8, 1.2 Hz, 1H), 2.39 (ddd, J = 12.4, 6.8, 1.2 Hz, 1H), 3.98 (ddd, J = 9.6, 6.8, 2.0 Hz, 1H), 6.13 (s, 1H), 6.99-7.13 (m, 2H), 7.15-7.28 (m, 2H).

Synthesis Example 104

Synthesis of (1R,4S,5R)-5-((2-fluorobenzyl)oxy)-2-(2-fluorophenyl)-1-isopropyl-4-methyl-7-oxabicyclo[2.2.1]hept-2-ene (3-686)

To a dimethylformamide solution (1.6 mL) of (1S,2R,4R)-5-(2-fluorophenyl)-4-isopropyl-1-methyl-7-oxabicyclo[2.2.1]hept-5-en-2-ol (130 mg, 0.492 mmol), sodium hydride (55% dispersion in mineral oil, 33.0 mg, 0.756 mmol) was added, followed by stirring at 0°C for 1 hour. To the reaction mixture, 2-fluorobenzyl bromide (0.070 mL, 0.59 mmol) was added, followed by stirring at room temperature (25°C) for 15 hours. A saturated ammonium chloride aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (85.6 mg, 47%).
$^{1}H$ NMR (400 MHz, $CDCl_3$) δ 0.84 (d, J = 6.8 Hz, 3H), 1.04 (d, J = 6.8 Hz, 3H), 1.66 (s, 3H), 1.82 (dd, J = 12.0, 2.0 Hz, 1H), 2.22 (dd, J = 12.0, 6.4 Hz, 1H), 2.30 (sept, J = 6.8 Hz, 1H), 3.77 (dd, J = 6.4, 2.0 Hz, 1H), 4.59 (d, J = 12.4 Hz, 1H), 4.68 (d, J = 12.4 Hz, 1H), 6.11 (s, 1H), 6.98-7.31 (m, 7H), 7.49 (td, J = 7.2, 1.2 Hz, 1H).

Synthesis Example 105

Synthesis of (1S,2R,4R,5S)-2-((2,6-difluorobenzyl)oxy)-5-(1-ethoxyethoxy)-4-isopropyl-1-methyl-7-oxabicyclo[2.2.1]heptane (1-223)

The same reaction and treatment as in Synthesis Example 8 were performed using 2,6-difluorobenzyl bromide instead of 2-methylbenzyl bromide to obtain a colorless liquid title compound (yield 85%).
$^{1}H$ NMR (400 MHz, $CDCl_3$) δ 0.98 (d, J = 6.8 Hz, 1.5H), 0.99 (d, J = 6.8 Hz, 1.5H), 1.01 (d, J = 6.8 Hz, 1.5H), 1.02 (d, J = 6.8 Hz, 1.5H), 1.19 (t, J = 6.8 Hz, 1.5H), 1.20 (t, J = 6.8 Hz, 1.5H), 1.25-1.39 (m, 1H), 1.27 (d, J = 6.8 Hz, 1.5H), 1.28 (d, J = 6.8 Hz, 1.5H), 1.34 (s, 1.5H), 1.35 (s, 1.5H), 1.46-1.55 (m, 1H), 1.88 (ddd, J = 12.8, 9.6 Hz, 0.5H), 1.94 (ddd, J = 12.8, 9.6 Hz, 0.5H), 2.02-2.12 (m, 1H), 2.64 (ddd, J = 12.8, 6.8, 1.6 Hz, 1H), 3.40-3.53 (m, 1H), 3.56-3.69 (m, 2H), 3.93 (dddd, J = 74.0, 9.6, 6.8, 1.6 Hz, 1H), 4.48-4.65 (m, 3H), 6.83-6.91 (m, 2H), 7.20-7.30 (m, 1H).

Synthesis Example 106

Synthesis of (1R,2S,4S,5R)-5-((2,6-difluorobenzyl)oxy)-1-isopropyl-4-methyl-7-oxabicyclo[2,2,1]heptan-2-ol (1-41)

To a tetrahydrofuran solution (2.8 mL) of (1S,2R,4R,5S)-2-((2,6-difluorobenzyl)oxy)-5-(1-ethoxyethoxy)-4-isopropyl-1-methyl-7-oxabicyclo[2.2.1]heptane (232 mg, 0.603 mmol), 3N hydrochloric acid (0.40 mL) was added, followed by stirring at room temperature (25°C) for 4 days. A saturated sodium hydrogen carbonate aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 182 mg, 96%).

$^1$H NMR (400 MHz, $CDCl_3$) δ 1.02 (d, J = 6.8 Hz, 3H), 1.02 (d, J = 6.8 Hz, 3H), 1.21 (dd, J = 3.6, 13.2 Hz, 1H), 1.34 (s, 3H), 1.49-1.53 (m, 2H), 2.03 (dd, J = 13.2, 10.0 Hz, 1H), 2.10 (sept, J = 6.8 Hz, 1H), 2.67 (dd, J = 13.2, 6.8 Hz, 1H), 3.66 (dd, J = 6.8, 2.0 Hz, 1H), 4.13-4.17 (m, 1H), 4.52 (dt, J = 11.2, 1.2 Hz, 1H), 4.54 (dt, J = 11.2, 1.2 Hz, 1H), 6.83-6.92 (m, 2H), 7.21-7.31 (m, 1H).

Synthesis Example 107

Synthesis of (1S,2R,4R,5S)-2-((2,6-difluorobenzyl)oxy)-5-fluoro-4-isopropyl-1-methyl-7-oxabicyclo[2.2.1]heptane (1-7)

To a dichloromethane solution (5 mL) of (1R,25,4S,5R)-5-((2,6-difluorobenzyl)oxy)-1-isopropyl-4-methyl-7-oxabicyclo[2.2.1]heptan-2-ol (162 mg, 0.519 mmol), DAST (0.34 mL, 2.6 mmol) was added, followed by stirring under heating and reflux for 19 hours. A saturated sodium hydrogen carbonate aqueous solution was poured into the reaction mixture, which was then extracted with dichloromethane. The extract was dried over anhydrous magnesium sulfate and then concentrated under reduced pressure. The concentrate was purified by silica gel column chromatography (eluent: ethyl acetate/n-hexane) to obtain a colorless liquid title compound (141 mg, 86%).

$^1$H NMR (400 MHz, $CDCl_3$) δ 0.99 (d, J = 6.8 Hz, 3H), 1.01 (d, J = 6.8 Hz, 3H), 1.36 (s, 3H), 1.45-1.60 (m, 2H), 1.96-2.08 (m, 1H), 2.10 (sept, J = 6.8 Hz, 1H), 2.59 (ddd, J = 13.2, 6.8, 3.6 Hz, 1H), 3.70 (dd, J = 6.8, 2.0 Hz, 1H), 4.52 (dt, J = 11.2, 1.2 Hz, 1H), 4.63 (dt, J = 11.2, 1.2 Hz, 1H), 4.81 (dddd, J = 18.4, 10.0, 2.4, 3.6 Hz, 1H), 6.84-6.92 (m, 2H), 7.22-7.32 (m, 1H).

Synthesis Example 108

Synthesis of (1S,2R,4R)-2-((2,6-difluorobenzyl)oxy)-4-isopropyl-1,5-dimethyl-7-oxabicyclo[2.2.1]heptane (1-111)

The same reaction and treatment as in Synthesis Example 15 were performed using 2,6-difluorobenzyl bromide instead of 2-methylbenzyl bromide to obtain a colorless liquid title compound (yield 78%).

$^1$H NMR (400 MHz, $CDCl_3$) δ 0.84-0.94 (m, 3.06H), 0.95-1.03 (m, 8.94H), 1.34 (s, 1.98H), 1.36 (s, 1.02H), 1.46-1.52 (m, 0.66H), 1.63-1.89 (m, 2H), 1.98-2.20 (m, 1.68H), 2.31 (dd, J = 13.2, 6.8 Hz, 0.66H), 3.47 (dd, J = 6.4, 2.8 Hz, 0.34H), 3.50 (dd, J = 6.4, 2.8 Hz, 0.66H), 4.49 (d, J = 10.8 Hz, 1H), 4.58 (d, J = 10.8 Hz, 1H), 6.82-6.91 (m, 2H), 7.20-7.30 (m, 1H).

The $^1$H-NMR spectrum ($CDCl_3$) $_6$ (ppm) values of compounds according to the present invention that are obtained in the following synthesis examples are shown in Tables 4 to 6 below.

Synthesis Example 109

Synthesis of (1S,2R,4R,5S)-5-bromo-2-[(2-fluorophenyl)methoxy]-1-methyl-4-isopropyl-7-oxabicyclo[2.2.1]heptane (1-19)

To a chloroform solution (10 mL) of (1S,6R)-6-[(2-fluorophenyl)methoxy]-4-isopropyl-1-methyl-cyclohex-3-en-1-ol (300 mg, 1.08 mmol), N-bromosuccinimide (211 mg, 1.19 mmol) was added in an ice bath (0°C), followed by stirring at room temperature (25°C) for 3 hours. A 10% sodium thiosulfate aqueous solution was added to the reaction solution, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (319 mg, 83%).

Synthesis Example 110

Synthesis of (1S,2R,4R,5S)-5-bromo-1-methyl-2-phenylmethoxy-4-isopropyl-7-oxabicyclo[2.2.1]heptane (1-15)

To a dimethylformamide solution (2 mL) of (1S,2R,4R,5S)-5-bromo-4-isopropyl-1-methyl-7-oxabicyclo[2.2.1]heptan-2-ol (318 mg, 1.28 mmol), sodium hydride (55% dispersion in mineral oil, 66.8 mg, 1.53 mmol) was added, followed by stirring in an ice bath (0°C) for 10 minutes. To the reaction mixture, benzyl bromide (262 mg, 1.53 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (340 mg, 79%).

Synthesis Example 111

Synthesis of (1S,2R,4R,5S)-5-bromo-1-methyl-2-[(2-methylphenyl)methoxy]-4-isopropyl-7-oxabicyclo[2.2.1]heptane (1-16)

The same reaction and treatment as in Synthesis Example 110 were performed using 2-methylbenzyl bromide instead of benzyl bromide to obtain a colorless liquid title compound (yield 82%).

Synthesis Example 112

Synthesis of (1S,2R,4R,5S)-5-bromo-1-methyl-2-(2-phenoxyethoxy)-4-isopropyl-7-oxabicyclo[2.2.1]heptane (1-724)

The same reaction and treatment as in Synthesis Example 110 were performed using 2-bromoethoxybenzene instead of benzyl bromide to obtain a colorless liquid title compound (yield 17%).

Synthesis Example 113

Synthesis of (1S,2R,4R,5S)-5-bromo-1-methyl-4-isopropyl-2-prop-2-enoxy-7-oxabicyclo[2.2.1]heptane (1-671)

The same reaction and treatment as in Synthesis Example 110 were performed using 3-bromoprop-1-ene instead of benzyl bromide to obtain a colorless liquid title compound (yield 22%).

Synthesis Example 114

Synthesis of (1S,2R,4R,5S)-5-bromo-2-methoxy-1-methyl-4-isopropyl-7-oxabicyclo[2.2.1]heptane (1-668)

The same reaction and treatment as in Synthesis Example 110 were performed using iodomethane instead of benzyl bromide to obtain a colorless liquid title compound (yield 24%).

Synthesis Example 115

Synthesis of (1S,2R,4R,5S)-5-bromo-1-methyl-4-isopropyl-2-prop-2-ynoxy-7-oxabicyclo[2.2.1]heptane (1-673)

The same reaction and treatment as in Synthesis Example 110 were performed using 3-bromoprop-1-yne instead of benzyl bromide to obtain a colorless liquid title compound (yield 56%).

Synthesis Example 116

Synthesis of (1S,2R,4R,5S)-5-bromo-2-(methoxymethoxy)-1-methyl-4-isopropyl-7-oxabicyclo[2.2.1]heptane (1-677)

The same reaction and treatment as in Synthesis Example 110 were performed using chloro(methoxy)methane instead of benzyl bromide to obtain a colorless liquid title compound (yield 65%).

Synthesis Example 117

Synthesis of (1S,2R,4R,5S)-5-bromo-1-methyl-2-(2-phenylmethoxyethoxy)-4-isopropyl-7-oxabicyclo[2.2.1]heptane (1-725)

The same reaction and treatment as in Synthesis Example 110 were performed using 2-bromoethoxymethylbenzene instead of benzyl bromide to obtain a colorless liquid title compound (yield 35%).

Synthesis Example 118

Synthesis of 2-[[(1S,2R,4R,5S)-5-bromo-1-methyl-4-isopropyl-7-oxabicyclo[2.2.1]heptan-2-yl]oxymethyl]benzonitrile (1-703)

The same reaction and treatment as in Synthesis Example 110 were performed using 2-(chloromethyl) benzonitrile instead of benzyl bromide to obtain a colorless liquid title compound (yield 34%).

Synthesis Example 119

Synthesis of (1S,2R,4R,5S)-5-bromo-2-[(2-bromophenyl)methoxy]-1-methyl-4-isopropyl-7-oxabicyclo[2.2.1]heptane (1-694)

The same reaction and treatment as in Synthesis Example 110 were performed using 1-bromo-2-(bromomethyl) benzene instead of benzyl bromide to obtain a colorless liquid title compound (yield 63%).

Synthesis Example 120

Synthesis of (1S,2R,4R,5S)-5-bromo-1-methyl-4-isopropyl-2-(2,2,2-trifluoroethoxy)-7-oxabicyclo[2.2.1]heptane (1-670)

The same reaction and treatment as in Synthesis Example 110 were performed using 1, 1,1-trifluoro-2-iodoethane instead of benzyl bromide to obtain a colorless liquid title compound (yield 56%).

Synthesis Example 121

Synthesis of (1S,2R,4R,5S)-5-bromo-1-methyl-2-(3-methylbut-2-enoxy)-4-isopropyl-7-oxabicyclo[2.2.1]heptane (1-672)

The same reaction and treatment as in Synthesis Example 110 were performed using 1-bromo-3-methyl-but-2-ene instead of benzyl bromide to obtain a colorless liquid title compound (yield 15%).

Synthesis Example 122

Synthesis of 2-[[(1S,2R,4R,5S)-5-bromo-1-methyl-4-isopropyl-7-oxabicyclo[2.2.1]heptan-2-yl]oxymethyl]pyridine (1-718)

The same reaction and treatment as in Synthesis Example 110 were performed using 2-(chloromethyl)pyridine hydrochloride instead of benzyl bromide to obtain a colorless liquid title compound (yield 50%).

Synthesis Example 123

Synthesis of (1S,2R,4R,5S)-5-bromo-2-[(2,4-dichlorophenyl)methoxy]-1-methyl-4-isopropyl-7-oxabicyclo[2.2.1]heptane (1-717)

The same reaction and treatment as in Synthesis Example 110 were performed using 2,4-dichloro-1-(chloromethyl)benzene instead of benzyl bromide to obtain a colorless liquid title compound (yield 82%).

Synthesis Example 124

Synthesis of (1S,2R,4R,5S)-5-bromo-2-but-2-ynoxy-1-methyl-4-isopropyl-7-oxabicyclo[2.2.1]heptane (1-674)

The same reaction and treatment as in Synthesis Example 110 were performed using 1-bromobut-2-yne instead of benzyl bromide to obtain a colorless liquid title compound (yield 57%).

Synthesis Example 125

Synthesis of (1S,2R,4R,5S)-5-bromo-2-hexoxy-1-methyl-4-isopropyl-7-oxabicyclo[2.2.1]heptane (1-669)

The same reaction and treatment as in Synthesis Example 110 were performed using 1-bromohexane instead of benzyl bromide to obtain a colorless liquid title compound (yield 41%).

Synthesis Example 126

Synthesis of (1S,2R,4R,5S)-5-bromo-2-(cyclohexylmethoxy)-1-methyl-4-isopropyl-7-oxabicyclo[2.2.1]heptane (1-676)

The same reaction and treatment as in Synthesis Example 110 were performed using bromomethylcyclohexane instead of benzyl bromide to obtain a colorless liquid title compound (yield 15%).

Synthesis Example 127

Synthesis of (1S,2R,4R,5S)-5-bromo-2-[(2,6-difluorophenyl)methoxy]-1-methyl-4-isopropyl-7-oxabicyclo[2.2.1]heptane (1-21)

The same reaction and treatment as in Synthesis Example 110 were performed using 2-(chloromethyl)-1,3-difluoro-benzene instead of benzyl bromide to obtain a colorless liquid title compound (yield 66%).

Synthesis Example 128

Synthesis of (1S,2R,4R,5S)-5-bromo-2-(2-methoxyethoxymethoxy)-1-methyl-4-isopropyl-7-oxabicyclo[2.2.1]heptane (1-727)

The same reaction and treatment as in Synthesis Example 110 were performed using 1-(chloromethoxy)-2-methoxy-ethane instead of benzyl bromide to obtain a colorless liquid title compound (yield 13%).

Synthesis Example 129

Synthesis of (1S,2R,4R,5S)-5-bromo-2-[(4-fluorophenyl)methoxy]-1-methyl-4-isopropyl-7-oxabicyclo[2.2.1]heptane (1-691)

The same reaction and treatment as in Synthesis Example 110 were performed using 1-(bromomethyl)-4-fluoro-benzene instead of benzyl bromide to obtain a colorless liquid title compound (yield 63%).

Synthesis Example 130

Synthesis of (1S,2R,4R,5S)-5-bromo-2-[(3-fluorophenyl)methoxy]-1-methyl-4-isopropyl-7-oxabicyclo[2.2.1]heptane (1-690)

The same reaction and treatment as in Synthesis Example 110 were performed using 1-(bromomethyl)-3-fluoro-benzene instead of benzyl bromide to obtain a colorless liquid title compound (yield 84%).

Synthesis Example 131

Synthesis of (1S,2R,4R,5S)-5-bromo-1-methyl-2-[(4-methylphenyl)methoxy]-4-isopropyl-7-oxabicyclo[2.2.1]heptane (1-681)

The same reaction and treatment as in Synthesis Example 110 were performed using 1-(chloromethyl)-4-methyl-benzene instead of benzyl bromide to obtain a colorless liquid title compound (yield 74%).

Synthesis Example 132

Synthesis of (1S,2R,4R,5S)-5-bromo-1-methyl-2-[(3-methylphenyl)methoxy]-4-isopropyl-7-oxabicyclo[2.2.1]heptane (1-680)

The same reaction and treatment as in Synthesis Example 110 were performed using 1-(chloromethyl)-3-methylbenzene instead of benzyl bromide to obtain a colorless liquid title compound (yield 81%).

Synthesis Example 133

Synthesis of (1S,2R,4R,5S)-5-bromo-2-[(4-chlorophenyl)methoxy]-1-methyl-4-isopropyl-7-oxabicyclo[2.2.1]heptane (1-693)

The same reaction and treatment as in Synthesis Example 110 were performed using 1-chloro-4-(chloromethyl) benzene instead of benzyl bromide to obtain a colorless liquid title compound (yield 83%).

Synthesis Example 134

Synthesis of (1S,2R,4R,5S)-5-bromo-2-[(2-chlorophenyl)methoxy]-1-methyl-4-isopropyl-7-oxabicyclo[2.2.1]heptane (1-20)

The same reaction and treatment as in Synthesis Example 110 were performed using 1-(bromomethyl)-2-chlorobenzene instead of benzyl bromide to obtain a colorless liquid title compound (yield 77%).

Synthesis Example 135

Synthesis of (1S,2R,4R,5S)-5-bromo-2-[(3-chlorophenyl)methoxy]-1-methyl-4-isopropyl-7-oxabicyclo[2.2.1]heptane (1-692)

The same reaction and treatment as in Synthesis Example 110 were performed using 1-chloro-3-(chloromethyl) benzene instead of benzyl bromide to obtain a colorless liquid title compound (yield 73%).

Synthesis Example 136

Synthesis of (1S,2R,4R,5S)-5-bromo-1-methyl-4-isopropyl-2-(thiophen-3-ylmethoxy)-7-oxabicyclo[2.2.1]heptane (1-722)

The same reaction and treatment as in Synthesis Example 110 were performed using 3-(chloromethyl)thiophene instead of benzyl bromide to obtain a pale yellow liquid title compound (yield 72%).

Synthesis Example 137

Synthesis of 4-[[(1S,2R,4R,5S)-5-bromo-1-methyl-4-isopropyl-7-oxabicyclo[2.2.1]heptan-2-yl]oxymethyl]benzonitrile (1-705)

The same reaction and treatment as in Synthesis Example 110 were performed using 4-(chloromethyl) benzonitrile instead of benzyl bromide to obtain a colorless liquid title compound (yield 31%).

Synthesis Example 138

Synthesis of (1S,2R,4R,5S)-5-bromo-2-[(4-bromophenyl)methoxy]-1-methyl-4-isopropyl-7-oxabicyclo[2.2.1]heptane (1-696)

The same reaction and treatment as in Synthesis Example 110 were performed using 1-bromo-4-(bromomethyl) benzene instead of benzyl bromide to obtain a colorless liquid title compound (yield 84%).

Synthesis Example 139

Synthesis of 4-[[(1S,2R,4R,5S)-5-bromo-1-methyl-4-isopropyl-7-oxabicyclo[2.2.1]heptan-2-yl]oxymethyl]-3,5-dimethyl-1,2-oxazole (1-723)

The same reaction and treatment as in Synthesis Example 110 were performed using 4-(chloromethyl)-3,5-dimethyl-isoxazole instead of benzyl bromide to obtain a colorless liquid title compound (yield 76%).

Synthesis Example 140

Synthesis of 3-[[(1S,2R,4R,5S)-5-bromo-1-methyl-4-isopropyl-7-oxabicyclo[2.2.1]heptan-2-yl]oxymethyl]benzonitrile (1-704)

The same reaction and treatment as in Synthesis Example 110 were performed using 3-(chloromethyl) benzonitrile instead of benzyl bromide to obtain a colorless liquid title compound (yield 86%).

Synthesis Example 141

Synthesis of (1S,2R,4R,5S)-5-bromo-2-[(3-bromophenyl)methoxy]-1-methyl-4-isopropyl-7-oxabicyclo[2.2.1]heptane (1-695)

The same reaction and treatment as in Synthesis Example 110 were performed using 1-bromo-3-(bromomethyl) benzene instead of benzyl bromide to obtain a colorless liquid title compound (yield 84%).

Synthesis Example 142

Synthesis of (1S,2R,4R,5S)-5-bromo-1-methyl-2-(naphthalen-1-ylmethoxy)-4-isopropyl-7-oxabicyclo[2.2.1]heptane (1-715)

The same reaction and treatment as in Synthesis Example 110 were performed using 1-(chloromethyl) naphthalene instead of benzyl bromide to obtain a colorless liquid title compound (yield 48%).

Synthesis Example 143

Synthesis of (1S,2R,4R,5S)-5-bromo-l-methyl-4-isopropyl-2-[[2-(trifluoromethyl)phenyl]methoxy]-7-oxabicyclo[2.2.1] heptane (1-700)

The same reaction and treatment as in Synthesis Example 110 were performed using 1-(bromomethyl)-2-(trifluoromethyl)benzene instead of benzyl bromide to obtain a colorless liquid title compound (yield 64%).

Synthesis Example 144

Synthesis of (1S,2R,4R,5S)-5-bromo-l-methyl-4-isopropyl-2-[[3-(trifluoromethyl)phenyl]methoxy]-7-oxabicyclo[2.2.1] heptane (1-701)

The same reaction and treatment as in Synthesis Example 110 were performed using 1-(bromomethyl)-3-(trifluoromethyl)benzene instead of benzyl bromide to obtain a colorless liquid title compound (yield 86%).

Synthesis Example 145

Synthesis of (1S,2R,4R,5S)-5-bromo-1-methyl-4-isopropyl-2-[[4-(trifluoromethyl)phenyl]methoxy]-7-oxabicyclo[2.2.1] heptane (1-702)

The same reaction and treatment as in Synthesis Example 110 were performed using 1-(chloromethyl)-4-(trifluoromethyl)benzene instead of benzyl bromide to obtain a colorless liquid title compound (yield 64%).

Synthesis Example 146

Synthesis of (1S,2R,4R,5S)-5-bromo-1-methyl-2-(naphthalen-2-ylmethoxy)-4-isopropyl-7-oxabicyclo[2.2.1]heptane (1-716)

The same reaction and treatment as in Synthesis Example 110 were performed using 2-(bromomethyl) naphthalene instead of benzyl bromide to obtain a colorless liquid title compound (yield 58%).

Synthesis Example 147

Synthesis of (1S,2R,4R,SS)-5-bromo-1-methyl-4-isopropyl-2-[[2-(trifluoromethoxy)phenyl]methoxy]-7-oxabicyclo [2.2.1]heptane (1-687)

The same reaction and treatment as in Synthesis Example 110 were performed using 1-(bromomethyl)-2-(trifluoromethoxy)benzene instead of benzyl bromide to obtain a colorless liquid title compound (yield 74%).

Synthesis Example 148

Synthesis of (1S,2R,4R,SS)-5-bromo-1-methyl-4-isopropyl-2-[[3-(trifluoromethoxy)phenyl]methoxy]-7-oxabicyclo [2.2.1]heptane (1-688)

The same reaction and treatment as in Synthesis Example 110 were performed using 1-(bromomethyl)-3-(trifluoromethoxy)benzene instead of benzyl bromide to obtain a colorless liquid title compound (yield 74%).

Synthesis Example 149

Synthesis of (1S,2R,4R,SS)-5-bromo-1-methyl-4-isopropyl-2-[[4-(trifluoromethoxy)phenyl]methoxy]-7-oxabicyclo [2.2.1]heptane (1-689)

The same reaction and treatment as in Synthesis Example 110 were performed using 1-(bromomethyl)-4-(trifluoromethoxy)benzene instead of benzyl bromide to obtain a colorless liquid title compound (yield 77%).

Synthesis Example 150

Synthesis of (1S,2R,4R,5S)-5-bromo-2-[(2-iodophenyl)methoxy]-1-methyl-4-isopropyl-7-oxabicyclo[2.2.1]heptane (1-697)

The same reaction and treatment as in Synthesis Example 110 were performed using 1-(bromomethyl)-2-iodobenzene instead of benzyl bromide to obtain a colorless liquid title compound (yield 67%).

Synthesis Example 151

Synthesis of (1S,2R,4R,5S)-5-bromo-2-[(3-iodophenyl)methoxy]-1-methyl-4-isopropyl-7-oxabicyclo[2.2.1]heptane (1-698)

The same reaction and treatment as in Synthesis Example 110 were performed using 1-(bromomethyl)-3-iodobenzene instead of benzyl bromide to obtain a colorless liquid title compound (yield 67%).

Synthesis Example 152

Synthesis of (1S,2R,4R,5S)-5-bromo-2-[(3-methoxyphenyl)methoxy]-1-methyl-4-isopropyl-7-oxabicyclo[2.2.1]heptane (1-685)

The same reaction and treatment as in Synthesis Example 110 were performed using 1-(chloromethyl)-3-methoxy-benzene instead of benzyl bromide to obtain a colorless liquid title compound (yield 71%).

Synthesis Example 153

Synthesis of (1S,2R,4R,5S)-5-bromo-2-[(4-methoxyphenyl)methoxy]-1-methyl-4-isopropyl-7-oxabicyclo[2.2.1]heptane (1-686)

The same reaction and treatment as in Synthesis Example 110 were performed using 1-(bromomethyl)-4-methoxy-benzene instead of benzyl bromide to obtain a colorless liquid title compound (yield 30%).

Synthesis Example 154

Synthesis of (1S,2R,4R,5S)-5-bromo-2-[(4-iodophenyl)methoxy]-1-methyl-4-isopropyl-7-oxabicyclo[2.2.1]heptane (1-699)

The same reaction and treatment as in Synthesis Example 110 were performed using 1-(bromomethyl)-4-iodo-benzene instead of benzyl bromide to obtain a colorless liquid title compound (yield 70%).

Synthesis Example 155

Synthesis of (1S,2R,4R,5S)-5-bromo-2-[(2-ethylphenyl)methoxy]-1-methyl-4-isopropyl-7-oxabicyclo[2.2.1]heptane (1-682)

The same reaction and treatment as in Synthesis Example 110 were performed using 1-(chloromethyl)-2-ethyl-benzene instead of benzyl bromide to obtain a colorless liquid title compound (yield 95%).

Synthesis Example 156

Synthesis of (1S,2R,4R,5S)-5-bromo-2-[(2-methoxyphenyl)methoxy]-1-methyl-4-isopropyl-7-oxabicyclo[2.2.1]heptane (1-18)

The same reaction and treatment as in Synthesis Example 110 were performed using 1-(bromomethyl)-2-methoxy-benzene instead of benzyl bromide to obtain a colorless liquid title compound (yield 47%).

Synthesis Example 157

Synthesis of methyl 2-[[(1S,2R,4R,5S)-5-bromo-1-methyl-4-isopropyl-7-oxabicyclo[2.2.1]heptan-2-yl]oxymethyl] benzoate (1-709)

The same reaction and treatment as in Synthesis Example 110 were performed using methyl 2-(chloromethyl) benzoate instead of benzyl bromide to obtain a brown liquid title compound (yield 10%).

Synthesis Example 158

Synthesis of 2-[[(1S,2R,4R,5S)-5-bromo-1-methyl-4-isopropyl-7-oxabicyclo[2.2.1]heptan-2-yl]oxymethyl]benzoic acid (1-706)

The same reaction and treatment as in Synthesis Example 110 were performed using methyl 2-(chloromethyl) benzoate instead of benzyl bromide to obtain a brown solid title compound (yield 23%).
Melting point: 80.0°C to 85.0°C

Synthesis Example 159

Synthesis of (1S,2R,4R,5S)-5-bromo-1-methyl-4-isopropyl-2-(thiophen-2-ylmethoxy)-7-oxabicyclo[2.2.1]heptane (1-721)

The same reaction and treatment as in Synthesis Example 110 were performed using 2-(chloromethyl)thiophene instead of benzyl bromide to obtain a colorless liquid title compound (yield 79%).

Synthesis Example 160

Synthesis of (1S,2R,4R,5S)-5-bromo-2-[(2-chloro-6-fluorophenyl)methoxy]-1-methyl-4-isopropyl-7-oxabicyclo[2.2.1] heptane (1-713)

The same reaction and treatment as in Synthesis Example 110 were performed using 2-(bromomethyl)-1-chloro-3-fluoro-benzene instead of benzyl bromide to obtain a colorless liquid title compound (yield 57%).

Synthesis Example 161

Synthesis of (1S,2R,4R,5S)-5-bromo-2-[(2,6-dichlorophenyl)methoxy]-1-methyl-4-isopropyl-7-oxabicyclo[2.2.1]heptane (1-712)

The same reaction and treatment as in Synthesis Example 110 were performed using 2-(bromomethyl)-1,3-dichloro-benzene instead of benzyl bromide to obtain a colorless liquid title compound (yield 76%).

Synthesis Example 162

Synthesis of (1S,2R,4R,5S)-5-bromo-2-[(2,6-dimethylphenyl)methoxy]-1-methyl-4-isopropyl-7-oxabicyclo[2.2.1]heptane (1-17)

The same reaction and treatment as in Synthesis Example 110 were performed using 2-(chloromethyl)-1,3-dimethyl-benzene instead of benzyl bromide to obtain a colorless liquid title compound (yield 85%).

Synthesis Example 163

Synthesis of (1S,2R,4R,5S)-5-bromo-2-[(2-bromo-6-fluorophenyl)methoxy]-1-methyl-4-isopropyl-7-oxabicyclo[2.2.1] heptane (1-1600)

The same reaction and treatment as in Synthesis Example 110 were performed using 1-bromo-2-(bromomethyl)-3-fluoro-benzene instead of benzyl bromide to obtain a colorless liquid title compound (yield 16%).

Synthesis Example 164

Synthesis of (1R,2S,4S,5R)-5-bromo-2-[(2-fluorophenyl)methoxy]-1-methyl-4-isopropyl-7-oxabicyclo[2.2.1]heptane (1-1028)

To a dimethylformamide solution (2 mL) of (1R,25,4S,5R)-5-bromo-4-isopropyl-1-methyl-7-oxabicyclo[2.2.1] heptan-2-ol (200 mg, 0.803 mmol), sodium hydride (55% dispersion in mineral oil, 42.0 mg, 0.963 mmol) was added, followed by stirring in an ice bath (0°C) for 10 minutes. To the reaction mixture, 1-(bromomethyl)-2-fluoro-benzene (182 mg, 0.963 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (127 mg, 44%).

Synthesis Example 165

Synthesis of (1R,28,48,5R)-5-bromo-2-[(2,6-difluorophenyl)methoxy]-1-methyl-4-isopropyl-7-oxabicyclo[2.2.1]heptane (1-1052)

The same reaction and treatment as in Synthesis Example 109 were performed using (1R,6S)-6-[(2,6-difluorophenyl)methoxy]-4-isopropyl-1-methyl-cyclohex-3-en-1-ol instead of (1S,6R)-6-[(2-fluorophenyl)methoxy]-4-isopropyl-1-methyl-cyclohex-3-en-1-ol to obtain a pale yellow liquid title compound (yield 70%).

Synthesis Example 166

Synthesis of (1R,2S,4S,5R)-5-bromo-2-[(2,4-dichlorophenyl)methoxy]-1-methyl-4-isopropyl-7-oxabicyclo[2.2.1]heptane (1-1058)

The same reaction and treatment as in Synthesis Example 164 were performed using 2,4-dichloro-1-(chloromethyl)benzene instead of 1-(bromomethyl)-2-fluoro-benzene to obtain a colorless liquid title compound (yield 88%).

Synthesis Example 167

Synthesis of (1R,2S,4S,5R)-5-bromo-2-[(2-chloro-6-fluorophenyl)methoxy]-1-methyl-4-isopropyl-7-oxabicyclo[2.2.1]heptane (1-1054)

The same reaction and treatment as in Synthesis Example 164 were performed using 2-(bromomethyl)-1-chloro-3-fluoro-benzene instead of 1-(bromomethyl)-2-fluoro-benzene to obtain a colorless liquid title compound (yield 51%).

Synthesis Example 168

Synthesis of (1R,2S,4S,5R)-5-bromo-2-[(2,6-dichlorophenyl)methoxy]-1-methyl-4-isopropyl-7-oxabicyclo[2.2.1]heptane (1-1053)

The same reaction and treatment as in Synthesis Example 164 were performed using 2-(bromomethyl)-1,3-dichloro-benzene instead of 1-(bromomethyl)-2-fluoro-benzene to obtain a colorless liquid title compound (yield 12%).

Synthesis Example 169

Synthesis of 2-[[(1R,2S,4S,5R)-5-bromo-1-methyl-4-isopropyl-7-oxabicyclo[2.2.1]heptan-2-yl]oxymethyl]pyridine (1-1059)

The same reaction and treatment as in Synthesis Example 164 were performed using 2-(chloromethyl)pyridine hydrochloride instead of 1-(bromomethyl)-2-fluoro-benzene to obtain a colorless liquid title compound (yield 59%).

Synthesis Example 170

Synthesis of 3-[[(1R,2S,4S,5R)-5-bromo-1-methyl-4-isopropyl-7-oxabicyclo[2.2.1]heptan-2-yl]oxymethyl]pyridine (1-1060)

The same reaction and treatment as in Synthesis Example 164 were performed using 3-(chloromethyl)pyridine hydrochloride instead of 1-(bromomethyl)-2-fluoro-benzene to obtain a colorless liquid title compound (yield 59%).

Synthesis Example 171

Synthesis of (1R,2S,4S,5R)-5-bromo-2-[(2,6-dimethylphenyl)methoxy]-1-methyl-4-isopropyl-7-oxabicyclo[2.2.1]heptane (1-1608)

The same reaction and treatment as in Synthesis Example 164 were performed using 2-(chloromethyl)-1,3-dimethyl-benzene instead of 1-(bromomethyl)-2-fluoro-benzene to obtain a colorless liquid title compound (yield 54%).

Synthesis Example 172

Synthesis of (1R,2S,4S,5R)-5-bromo-2-[(2-bromo-6-fluorophenyl)methoxy]-1-methyl-4-isopropyl-7-oxabicyclo[2.2.1]heptane (1-1609)

The same reaction and treatment as in Synthesis Example 164 were performed using 1-bromo-2-(bromomethyl)-3-fluoro-benzene instead of 1-(bromomethyl)-2-fluoro-benzene to obtain a colorless liquid title compound (yield 40%).

Synthesis Example 173

Synthesis of 4-[[(1R,28,4S,5R)-5-bromo-1-methyl-4-isopropyl-7-oxabicyclo[2.2.1]heptan-2-yl]oxymethyl]pyridine (1-1061)

The same reaction and treatment as in Synthesis Example 164 were performed using 4-(chloromethyl)pyridine

hydrochloride instead of 1-(bromomethyl)-2-fluoro-benzene to obtain a colorless liquid title compound (yield 37%).

Synthesis Example 174

Synthesis of 4-[[(1R,2S,4S,5R)-5-bromo-1-methyl-4-isopropyl-7-oxabicyclo[2.2.1]heptan-2-yl]oxymethyl]-3,5-dimethyl-1,2-oxazole (1-1064)

The same reaction and treatment as in Synthesis Example 164 were performed using 4-(chloromethyl)-3,5-dimethyl-isoxazole instead of 1-(bromomethyl)-2-fluoro-benzene to obtain a colorless liquid title compound (yield 56%).

Synthesis Example 175

Synthesis of (1R,2S,4S,5R)-5-bromo-1-methyl-4-isopropyl-2-[[2-(trifluoromethoxy)phenyl]methoxy]-7-oxabicyclo [2.2.1]heptane (1-1025)

The same reaction and treatment as in Synthesis Example 164 were performed using 1-(bromomethyl)-2-(trifluoromethoxy)benzene instead of 1-(bromomethyl)-2-fluoro-benzene to obtain a colorless liquid title compound (yield 21%).

Synthesis Example 176

Synthesis of (1R,2S,4S,5R)-5-bromo-2-[(2-iodophenyl)methoxy]-1-methyl-4-isopropyl-7-oxabicyclo[2.2.1]heptane (1-1037)

The same reaction and treatment as in Synthesis Example 164 were performed using 1-(bromomethyl)-2-iodobenzene instead of 1-(bromomethyl)-2-fluoro-benzene to obtain a colorless liquid title compound (yield 21%).

Synthesis Example 177

Synthesis of (1R,2S,4S,5R)-5-bromo-2-[(2-bromophenyl)methoxy]-1-methyl-4-isopropyl-7-oxabicyclo[2.2.1]heptane (1-1034)

The same reaction and treatment as in Synthesis Example 164 were performed using 1-bromo-2-(bromomethyl) benzene instead of 1-(bromomethyl)-2-fluoro-benzene to obtain a colorless liquid title compound (yield 24%).

Synthesis Example 178

Synthesis of (1R,2S,4S,SR)-5-bromo-1-methyl-2-(3-methylbut-2-enoxy)-4-isopropyl-7-oxabicyclo[2.2.1]heptane (1-1007)

The same reaction and treatment as in Synthesis Example 164 were performed using 1-bromo-3-methyl-but-2-ene instead of 1-(bromomethyl)-2-fluoro-benzene to obtain a colorless liquid title compound (yield 31%).

Synthesis Example 179

Synthesis of 2-[[(1R,2S,4S,5R)-5-bromo-1-methyl-4-isopropyl-7-oxabicyclo[2.2.1]heptan-2-yl]oxymethyl]benzonitrile (1-1043)

The same reaction and treatment as in Synthesis Example 164 were performed using 2-(chloromethyl) benzonitrile instead of 1-(bromomethyl)-2-fluoro-benzene to obtain a colorless liquid title compound (yield 34%).

Synthesis Example 180

Synthesis of (1R,2S,4S,5R)-5-bromo-2-but-2-ynoxy-1-methyl-4-isopropyl-7-oxabicyclo[2.2.1]heptane (1-1009)

The same reaction and treatment as in Synthesis Example 164 were performed using 1-bromobut-2-yne instead of 1-(bromomethyl)-2-fluoro-benzene to obtain a yellow liquid title compound (yield 29%).

Synthesis Example 181

Synthesis of (1R,2S,4S,5R)-5-bromo-1-methyl-2-phenylmethoxy-4-isopropyl-7-oxabicyclo[2.2.1]heptane (1-1015)

The same reaction and treatment as in Synthesis Example 164 were performed using benzyl bromide instead of 1-(bromomethyl)-2-fluoro-benzene to obtain a colorless liquid title compound (yield 58%).

Synthesis Example 182

Synthesis of (1R,2S,4S,SR)-5-bromo-2-[(2-chlorophenyl)methoxy]-1-methyl-4-isopropyl-7-oxabicyclo[2.2.1]heptane (1-1031)

The same reaction and treatment as in Synthesis Example 164 were performed using 1-(bromomethyl)-2-chlorobenzene instead of 1-(bromomethyl)-2-fluoro-benzene to obtain a colorless liquid title compound (yield 27%).

Synthesis Example 183

Synthesis of (1R,2S,4S,5R)-5-bromo-1-methyl-2-[(2-methylphenyl)methoxy]-4-isopropyl-7-oxabicyclo[2.2.1]heptane (1-1016)

The same reaction and treatment as in Synthesis Example 164 were performed using 1-(chloromethyl)-2-methyl-benzene instead of 1-(bromomethyl)-2-fluoro-benzene to obtain a colorless liquid title compound (yield 49%).

Synthesis Example 184

Synthesis of (1R,2S,4S,5R)-5-bromo-2-[(2-methoxyphenyl)methoxy]-1-methyl-4-isopropyl-7-oxabicyclo[2.2.1]heptane (1-1022)

The same reaction and treatment as in Synthesis Example 164 were performed using 1-(bromomethyl)-2-methoxy-benzene instead of 1-(bromomethyl)-2-fluoro-benzene to obtain a colorless liquid title compound (yield 20%).

Synthesis Example 185

Synthesis of (1R,2S,4S,5R)-5-bromo-1-methyl-4-isopropyl-2-[[2-(trifluoromethyl)phenyl]methoxy]-7-oxabicyclo[2.2.1]heptane (1-1040)

The same reaction and treatment as in Synthesis Example 164 were performed using 1-(bromomethyl)-2-(trifluoromethyl)benzene instead of 1-(bromomethyl)-2-fluoro-benzene to obtain a colorless liquid title compound (yield 24%).

Synthesis Example 186

Synthesis of 5-bromo-1-methyl-2-(3-methylbut-2-enoxy)-4-isopropyl-7-oxabicyclo[2.2.1]heptane (1-1334)

To a dimethylformamide solution (2 mL) of 5-bromo-4-isopropyl-1-methyl-7-oxabicyclo[2.2.1]heptan-2-ol (200 mg, 0.803 mmol), sodium hydride (55% dispersion in mineral oil, 42.0 mg, 0.963 mmol) was added, followed by stirring in an ice bath (0°C) for 10 minutes. To the reaction mixture, 1-bromo-3-methyl-but-2-ene (144 mg, 0.963 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (210 mg, 82%).

Synthesis Example 187

Synthesis of (1S,2R,4R)-5-bromo-2-but-2-ynoxy-1-methyl-4-isopropyl-7-oxabicyclo[2.2.1]heptane (1-1336)

The same reaction and treatment as in Synthesis Example 186 were performed using 1-bromobut-2-yne instead of 1-bromo-3-methyl-but-2-ene to obtain a colorless liquid title compound (yield 45%).

Synthesis Example 188

Synthesis of 5-bromo-1-methyl-2-phenylmethoxy-4-isopropyl-7-oxabicyclo[2.2.1]heptane (1-1342)

The same reaction and treatment as in Synthesis Example 186 were performed using benzyl bromide instead of 1-bromo-3-methyl-but-2-ene to obtain a colorless liquid title compound (yield 87%).

Synthesis Example 189

Synthesis of 5-bromo-1-methyl-2-[(2-methylphenyl)methoxy]-4-isopropyl-7-oxabicyclo[2.2.1]heptane (1-1343)

The same reaction and treatment as in Synthesis Example 186 were performed using 1-(chloromethyl)-2-methyl-benzene instead of 1-bromo-3-methyl-but-2-ene to obtain a colorless liquid title compound (yield 61%).

Synthesis Example 190

Synthesis of methyl 2-[(5-bromo-1-methyl-4-isopropyl-7-oxabicyclo[2.2.1]heptan-2-yl)oxymethyl]benzoate (1-1376)

The same reaction and treatment as in Synthesis Example 186 were performed using methyl 2-(chloromethyl) benzoate instead of 1-bromo-3-methyl-but-2-ene to obtain a yellow liquid title compound (yield 31%).

Synthesis Example 191

Synthesis of 4-[(5-bromo-1-methyl-4-isopropyl-7-oxabicyclo[2.2.1]heptan-2-yl)oxymethyl]benzonitrile (1-1372)

The same reaction and treatment as in Synthesis Example 186 were performed using 4-(chloromethyl) benzonitrile instead of 1-bromo-3-methyl-but-2-ene to obtain a colorless liquid title compound (yield 28%).

Synthesis Example 192

Synthesis of 2-[(5-bromo-1-methyl-4-isopropyl-7-oxabicyclo[2.2.1]heptan-2-yl)oxymethyl]benzonitrile (1-1370)

The same reaction and treatment as in Synthesis Example 186 were performed using 2-(chloromethyl) benzonitrile instead of 1-bromo-3-methyl-but-2-ene to obtain a light orange liquid title compound (yield 54%).

Synthesis Example 193

Synthesis of 3-[(5-bromo-1-methyl-4-isopropyl-7-oxabicyclo[2.2.1]heptan-2-yl)oxymethyl]benzonitrile (1-1371)

The same reaction and treatment as in Synthesis Example 186 were performed using 3-(chloromethyl) benzonitrile instead of 1-bromo-3-methyl-but-2-ene to obtain a white solid title compound (yield 86%).
Melting point: 71.2°C to 74.0°C

Synthesis Example 194

Synthesis of 5-bromo-2-[(2-fluorophenyl)methoxy]-1-methyl-4-isopropyl-7-oxabicyclo[2.2.1]heptane (1-1355)

The same reaction and treatment as in Synthesis Example 186 were performed using 1-(bromomethyl)-2-fluorobenzene instead of 1-bromo-3-methyl-but-2-ene to obtain a white solid title compound (yield 51%).
Melting point: 61.9°C to 64.2°C

Synthesis Example 195

Synthesis of 5-bromo-2-[(3-bromophenyl)methoxy]-1-methyl-4-isopropyl-7-oxabicyclo[2.2.1]heptane (1-1362)

The same reaction and treatment as in Synthesis Example 186 were performed using 1-bromo-3-(bromomethyl) benzene instead of 1-bromo-3-methyl-but-2-ene to obtain a colorless liquid title compound (yield 91%).

Synthesis Example 196

Synthesis of 5-bromo-2-[(2-bromo-6-fluorophenyl)methoxy]-1-methyl-4-isopropyl-7-oxabicyclo[2.2.1]heptane (1-1598)

The same reaction and treatment as in Synthesis Example 186 were performed using 1-bromo-2-(bromomethyl)-3-fluoro-benzene instead of 1-bromo-3-methyl-but-2-ene to obtain a colorless liquid title compound (yield 26%).

Synthesis Example 197

Synthesis of 5-bromo-2-[(2-bromophenyl)methoxy]-1-methyl-4-isopropyl-7-oxabicyclo[2.2.1]heptane (1-1361)

The same reaction and treatment as in Synthesis Example 186 were performed using 1-bromo-2-(bromomethyl) benzene instead of 1-bromo-3-methyl-but-2-ene to obtain a colorless liquid title compound (yield 38%).

Synthesis Example 198

Synthesis of 5-bromo-2-[(2,6-difluorophenyl)methoxy]-1-methyl-4-isopropyl-7-oxabicyclo[2.2.1]heptane (1-1379)

The same reaction and treatment as in Synthesis Example 186 were performed using 2-(chloromethyl)-1,3-difluoro-benzene instead of 1-bromo-3-methyl-but-2-ene to obtain a colorless liquid title compound (yield 53%).

Synthesis Example 199

Synthesis of 5-bromo-2-[(2,4-dichlorophenyl)methoxy]-1-methyl-4-isopropyl-7-oxabicyclo[2.2.1]heptane (1-1385)

The same reaction and treatment as in Synthesis Example 186 were performed using 2,4-dichloro-1-(chloromethyl)benzene instead of 1-bromo-3-methyl-but-2-ene to obtain a colorless liquid title compound (yield 49%).

Synthesis Example 200

Synthesis of 5-bromo-2-[(2-chlorophenyl)methoxy]-1-methyl-4-isopropyl-7-oxabicyclo[2.2.1]heptane (1-1358)

The same reaction and treatment as in Synthesis Example 186 were performed using 1-(bromomethyl)-2-chloro-benzene instead of 1-bromo-3-methyl-but-2-ene to obtain a colorless liquid title compound (yield 73%).

Synthesis Example 201

Synthesis of 5-bromo-1-methyl-2-(naphthalen-1-ylmethoxy)-4-isopropyl-7-oxabicyclo[2.2.1]heptane (1-1383)

The same reaction and treatment as in Synthesis Example 186 were performed using 1-(chloromethyl) naphthalene instead of 1-bromo-3-methyl-but-2-ene to obtain a colorless liquid title compound (yield 58%).

Synthesis Example 202

Synthesis of 5-bromo-1-methyl-2-(naphthalen-2-ylmethoxy)-4-isopropyl-7-oxabicyclo[2.2.1]heptane (1-1384)

The same reaction and treatment as in Synthesis Example 186 were performed using 2-(bromomethyl) naphthalene instead of 1-bromo-3-methyl-but-2-ene to obtain a colorless liquid title compound (yield 51%).

Synthesis Example 203

Synthesis of 5-bromo-2-[(4-chlorophenyl)methoxy]-1-methyl-4-isopropyl-7-oxabicyclo[2.2.1]heptane (1-1360)

The same reaction and treatment as in Synthesis Example 186 were performed using 1-chloro-4-(chloromethyl) benzene instead of 1-bromo-3-methyl-but-2-ene to obtain a colorless liquid title compound (yield 83%).

Synthesis Example 204

Synthesis of 5-bromo-1-methyl-4-isopropyl-2-[[3-(trifluoromethyl)phenyl]methoxy]-7-oxabicyclo[2.2.1]heptane (1-1368)

The same reaction and treatment as in Synthesis Example 186 were performed using 1-(bromomethyl)-3-(trifluoromethyl)benzene instead of 1-bromo-3-methyl-but-2-ene to obtain a colorless liquid title compound (yield 80%).

Synthesis Example 205

Synthesis of 5-bromo-1-methyl-4-isopropyl-2-[[4-(trifluoromethyl)phenyl]methoxy]-7-oxabicyclo[2.2.1]heptane (1-1369)

The same reaction and treatment as in Synthesis Example 186 were performed using 1-(chloromethyl)-4-(trifluoromethyl)benzene instead of 1-bromo-3-methyl-but-2-ene to obtain a colorless liquid title compound (yield 43%).

Synthesis Example 206

Synthesis of 5-bromo-2-[(3-chlorophenyl)methoxy]-1-methyl-4-isopropyl-7-oxabicyclo[2.2.1]heptane (1-1359)

The same reaction and treatment as in Synthesis Example 186 were performed using 1-chloro-3-(chloromethyl)benzene instead of 1-bromo-3-methyl-but-2-ene to obtain a colorless liquid title compound (yield 93%).

Synthesis Example 207

Synthesis of 5-bromo-1-methyl-4-isopropyl-2-[[2-(trifluoromethyl)phenyl]methoxy]-7-oxabicyclo[2.2.1]heptane (1-1367)

The same reaction and treatment as in Synthesis Example 186 were performed using 1-(bromomethyl)-3-(trifluoromethyl)benzene instead of 1-bromo-2-methyl-but-2-ene to obtain a colorless liquid title compound (yield 76%).

Synthesis Example 208

Synthesis of 5-bromo-1-methyl-4-isopropyl-2-[[2-(trifluoromethoxy)phenyl]methoxy]-7-oxabicyclo[2.2.1]heptane (1-1352)

The same reaction and treatment as in Synthesis Example 186 were performed using 1-(bromomethyl)-2-(trifluoromethoxy)benzene instead of 1-bromo-3-methyl-but-2-ene to obtain a colorless liquid title compound (yield 77%).

Synthesis Example 209

Synthesis of 5-bromo-1-methyl-4-isopropyl-2-[[3-(trifluoromethoxy)phenyl]methoxy]-7-oxabicyclo[2.2.1]heptane (1-1353)

The same reaction and treatment as in Synthesis Example 186 were performed using 1-(bromomethyl)-3-(trifluoromethoxy)benzene instead of 1-bromo-3-methyl-but-2-ene to obtain a colorless liquid title compound (yield 77%).

Synthesis Example 210

Synthesis of 5-bromo-2-[(2-methoxyphenyl)methoxy]-1-methyl-4-isopropyl-7-oxabicyclo[2.2.1]heptane (1-1349)

The same reaction and treatment as in Synthesis Example 186 were performed using 1-(bromomethyl)-2-methoxy-benzene instead of 1-bromo-3-methyl-but-2-ene to obtain a colorless liquid title compound (yield 67%).

Synthesis Example 211

Synthesis of 5-bromo-1-methyl-4-isopropyl-2-[[4-(trifluoromethoxy)phenyl]methoxy]-7-oxabicyclo[2.2.1]heptane (1-1354)

The same reaction and treatment as in Synthesis Example 186 were performed using 1-(bromomethyl)-4-(trifluoromethoxy)benzene instead of 1-bromo-3-methyl-but-2-ene to obtain a colorless liquid title compound (yield 79%).

Synthesis Example 212

Synthesis of 5-bromo-2-[(3-methoxyphenyl)methoxy]-1-methyl-4-isopropyl-7-oxabicyclo[2.2.1]heptane (1-1350)

The same reaction and treatment as in Synthesis Example 186 were performed using 1-(chloromethyl)-3-methoxy-benzene instead of 1-bromo-3-methyl-but-2-ene to obtain a colorless liquid title compound (yield 81%).

Synthesis Example 213

Synthesis of 5-bromo-2-[(3-iodophenyl)methoxy]-1-methyl-4-isopropyl-7-oxabicyclo[2.2.1]heptane (1-1365)

The same reaction and treatment as in Synthesis Example 186 were performed using 1-(bromomethyl)-3-iodo-benzene instead of 1-bromo-3-methyl-but-2-ene to obtain a colorless liquid title compound (yield 72%).

Synthesis Example 214

Synthesis of 5-bromo-2-[(4-iodophenyl)methoxy]-1-methyl-4-isopropyl-7-oxabicyclo[2.2.1]heptane (1-1366)

The same reaction and treatment as in Synthesis Example 186 were performed using 1-(bromomethyl)-4-iodo-benzene instead of 1-bromo-3-methyl-but-2-ene to obtain a colorless liquid title compound (yield 70%).

Synthesis Example 215

Synthesis of 5-bromo-2-[(4-methoxyphenyl)methoxy]-1-methyl-4-isopropyl-7-oxabicyclo[2.2.1]heptane (1-1351)

The same reaction and treatment as in Synthesis Example 186 were performed using 1-(bromomethyl)-4-methoxy-benzene instead of 1-bromo-3-methyl-but-2-ene to obtain a colorless liquid title compound (yield 30%).

Synthesis Example 216

Synthesis of 5-bromo-2-[(2-iodophenyl)methoxy]-1-methyl-4-isopropyl-7-oxabicyclo[2.2.1]heptane (1-1364)

The same reaction and treatment as in Synthesis Example 186 were performed using 1-(bromomethyl)-2-iodo-benzene instead of 1-bromo-3-methyl-but-2-ene to obtain a colorless liquid title compound (yield 83%).

Synthesis Example 217

Synthesis of 5-bromo-2-[(2-ethylphenyl)methoxy]-1-methyl-4-isopropyl-7-oxabicyclo[2.2.1]heptane (1-1346)

The same reaction and treatment as in Synthesis Example 186 were performed using 1-(chloromethyl)-2-ethyl-benzene instead of 1-bromo-3-methyl-but-2-ene to obtain a colorless liquid title compound (yield 93%).

Synthesis Example 218

Synthesis of 4-[(5-bromo-1-methyl-4-isopropyl-7-oxabicyclo[2.2.1]heptan-2-yl)oxymethyl]-3,5-dimethyl-1,2-oxazole (1-1391)

The same reaction and treatment as in Synthesis Example 186 were performed using 4-(chloromethyl)-3,5-dimethyl-isoxazole instead of 1-bromo-3-methyl-but-2-ene to obtain a colorless liquid title compound (yield 61%).

Synthesis Example 219

Synthesis of 5-bromo-1-methyl-4-isopropyl-2-(thiophen-2-ylmethoxy)-7-oxabicyclo[2.2.1]heptane (1-1389)

The same reaction and treatment as in Synthesis Example 186 were performed using 2-(chloromethyl)thiophene instead of 1-bromo-3-methyl-but-2-ene to obtain a yellow liquid title compound (yield 55%).

Synthesis Example 220

Synthesis of 5-bromo-1-methyl-4-isopropyl-2-(thiophen-3-ylmethoxy)-7-oxabicyclo[2.2.1]heptane (1-1390)

The same reaction and treatment as in Synthesis Example 186 were performed using 3-(chloromethyl)thiophene instead of 1-bromo-3-methyl-but-2-ene to obtain a pale yellow liquid title compound (yield 66%).

Synthesis Example 221

Synthesis of 5-bromo-1-methyl-2-[(3-methylphenyl)methoxy]-4-isopropyl-7-oxabicyclo[2.2.1]heptane (1-1344)

The same reaction and treatment as in Synthesis Example 186 were performed using 1-(chloromethyl)-3-methyl-benzene instead of 1-bromo-3-methyl-but-2-ene to obtain a colorless liquid title compound (yield 75%).

Synthesis Example 222

Synthesis of 5-bromo-1-methyl-2-[(4-methylphenyl)methoxy]-4-isopropyl-7-oxabicyclo[2.2.1]heptane (1-1345)

The same reaction and treatment as in Synthesis Example 186 were performed using 1-(chloromethyl)-4-methyl-benzene instead of 1-bromo-3-methyl-but-2-ene to obtain a colorless liquid title compound (yield 80%).

Synthesis Example 223

Synthesis of 5-bromo-2-[(3-fluorophenyl)methoxy]-1-methyl-4-isopropyl-7-oxabicyclo[2.2.1]heptane (1-1356)

The same reaction and treatment as in Synthesis Example 186 were performed using 1-(bromomethyl)-3-fluoro-benzene instead of 1-bromo-3-methyl-but-2-ene to obtain a colorless liquid title compound (yield 92%).

Synthesis Example 224

Synthesis of 5-bromo-2-[(4-fluorophenyl)methoxy]-1-methyl-4-isopropyl-7-oxabicyclo[2.2.1]heptane (1-1357)

The same reaction and treatment as in Synthesis Example 186 were performed using 1-(bromomethyl)-4-fluoro-benzene instead of 1-bromo-3-methyl-but-2-ene to obtain a colorless liquid title compound (yield 92%).

Synthesis Example 225

Synthesis of 5-bromo-2-[(2,6-dichlorophenyl)methoxy]-1-methyl-4-isopropyl-7-oxabicyclo[2.2.1]heptane (1-1380)

The same reaction and treatment as in Synthesis Example 186 were performed using 2-(bromomethyl)-1,3-dichloro-benzene instead of 1-bromo-3-methyl-but-2-ene to obtain a colorless liquid title compound (yield 79%).

Synthesis Example 226

Synthesis of 5-bromo-2-[(2,6-dimethylphenyl)methoxy]-1-methyl-4-isopropyl-7-oxabicyclo[2.2.1]heptane (1-1599)

The same reaction and treatment as in Synthesis Example 186 were performed using 2-(chloromethyl)-1,3-dimethyl-benzene instead of 1-bromo-3-methyl-but-2-ene to obtain a colorless liquid title compound (yield 88%).

Synthesis Example 227

Synthesis of 5-bromo-2-[(2-chloro-6-fluorophenyl)methoxy]-1-methyl-4-isopropyl-7-oxabicyclo[2.2.1]heptane (1-1381)

The same reaction and treatment as in Synthesis Example 186 were performed using 2-(bromomethyl)-1-chloro-3-fluoro-benzene instead of 1-bromo-3-methyl-but-2-ene to obtain a colorless liquid title compound (yield 80%).

Synthesis Example 228

Synthesis of 5-bromo-2-[(4-bromophenyl)methoxy]-1-methyl-4-isopropyl-7-oxabicyclo[2.2.1]heptane (1-1363)

The same reaction and treatment as in Synthesis Example 186 were performed using 1-bromo-4-(bromomethyl) benzene instead of 1-bromo-3-methyl-but-2-ene to obtain a colorless liquid title compound (yield 98%).

Synthesis Example 229

Synthesis of 2-[(5-bromo-1-methyl-4-isopropyl-7-oxabicyclo[2.2.1]heptan-2-yl)oxymethyl]pyridine (1-1386)

The same reaction and treatment as in Synthesis Example 186 were performed using 2-(chloromethyl)pyridine hydrochloride instead of 1-bromo-3-methyl-but-2-ene to obtain a white solid title compound (yield 91%).
Melting point: 93.4°C to 96.0°C

Synthesis Example 230

Synthesis of 2-[[(1S,2R,4R,5S)-5-bromo-1-methyl-4-isopropyl-7-oxabicyclo[2.2.1]heptan-2-yl]oxy]pyridine (1-678)

To a toluene solution (5 mL) of (1S,2R,4R,5S)-5-bromo-4-isopropyl-1-methyl-7-oxabicyclo[2.2.1]heptan-2-ol (200 mg, 0.803 mmol), 2-chloropyridine (91.1 mg, 0.803 mmol), 85.5% potassium hydroxide (149 mg, 2.26 mmol), and 18-CROWN-6 (10.6 mg, 0.0401 mmol) were added, followed by stirring under heated reflux for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a white solid title compound (70.0 mg, 27%).
Melting point: 70.0°C to 75.0°C

Synthesis Example 231

Synthesis of 2-[[(1S,2R,4R,5S)-5-bromo-1-methyl-4-isopropyl-7-oxabicyclo[2.2.1]heptan-2-yl]oxy]-3-methylpyridine (1-679)

The same reaction and treatment as in Synthesis Example 230 were performed using 2-chloro-3-methylpyridine instead of 2-chloropyridine to obtain a yellow solid title compound (yield 26%).
Melting point: 85.1°C to 87.8°C

Synthesis Example 232

Synthesis of (1S,2R,4R,5S)-5-iodo-1-methyl-2-[(2-methylphenyl)methoxy]-4-isopropyl-7-oxabicyclo[2.2.1]heptane (1-741)

To a chloroform solution (10 mL) of (1S,6R)-4-isopropyl-1-methyl-6-(o-tolylmethoxy)cyclohex-3-en-1-ol (300 mg, 1.08 mmol), N-iodosuccinimide (211 mg, 1.19 mmol) was added in an ice bath (0°C), followed by stirring at room temperature (25°C) for 3 hours. A 10% sodium thiosulfate aqueous solution was added to the reaction solution, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (319 mg, 83%).

Synthesis Example 233

Synthesis of (1S,2R,4R,SS)-2-[(2-fluorophenyl)methoxy]-5-iodo-1-methyl-4-isopropyl-7-oxabicyclo[2.2.1]heptane (1-753)

The same reaction and treatment as in Synthesis Example 232 were performed using (1S,6R)-6-[(2-fluorophenyl)methoxy]-4-isopropyl-1-methyl-cyclohex-3-en-1-ol instead of (1S,6R)-4-isopropyl-1-methyl-6-(o-tolylmethoxy) cyclohex-3-en-1-ol to obtain a colorless liquid title compound (yield 46%).

Synthesis Example 234

Synthesis of (1R,2S,4S,5R)-2-[(2-fluorophenyl)methoxy]-5-iodo-1-methyl-4-isopropyl-7-oxabicyclo[2.2.1]heptane (1-1094)

The same reaction and treatment as in Synthesis Example 232 were performed using (1S,2R,4R,5S)-5-[(2-fluorophenyl)methoxy]-2-iodo-1-isopropyl-4-methyl-7-oxabicyclo[2.2.1]heptane instead of (1 S,6R)-4-isopropyl-1-methyl-6-(o-tolylmethoxy)cyclohex-3-en-1-ol to obtain a brown liquid title compound (yield 83%).

Synthesis Example 235

Synthesis of (1R,2S,4S,5R)-2-iodo-1-isopropyl-4-methyl-5-(3-methylbut-2-enoxy)-7-oxabicyclo[2.2.1]heptane (1-732)

To a dimethylformamide solution (3 mL) of (1S,2R,4R,5S)-5-iodo-4-isopropyl-1-methyl-7-oxabicyclo[2.2.1]heptan-2-ol (200 mg, 0.675 mmol), sodium hydride (55% dispersion in mineral oil, 35.4 mg, 0.810 mmol) was added, followed by stirring in an ice bath (0°C) for 10 minutes. To the reaction mixture, 1-bromo-3-methyl-but-2-ene (121 mg, 0.810 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (180 mg, 73%).

Synthesis Example 236

Synthesis of (1R,2S,4S,5R)-5-but-2-ynoxy-2-iodo-1-isopropyl-4-methyl-7-oxabicyclo[2.2.1]heptane (1-734)

The same reaction and treatment as in Synthesis Example 235 were performed using 1-bromobut-2-yne instead of 1-bromo-3-methyl-but-2-ene to obtain a colorless liquid title compound (yield 60%).

Synthesis Example 237

Synthesis of (1R,2S,4S,5R)-5-(cyclohexylmethoxy)-2-iodo-1-isopropyl-4-methyl-7-oxabicyclo[2.2.1]heptane (1-736)

The same reaction and treatment as in Synthesis Example 235 were performed using bromomethylcyclohexane instead of 1-bromo-3-methyl-but-2-ene to obtain a colorless liquid title compound (yield 8%).

Synthesis Example 238

Synthesis of (1R,2S,4S,5R)-5-benzyloxy-2-iodo-1-isopropyl-4-methyl-7-oxabicyclo[2.2.1]heptane (1-740)

The same reaction and treatment as in Synthesis Example 235 were performed using benzyl bromide instead of 1-bromo-3-methyl-but-2-ene to obtain a colorless liquid title compound (yield 100%).

Synthesis Example 239

Synthesis of (1R,2S,4S,5R)-2-iodo-1-isopropyl-5-[(2-methoxyphenyl)methoxy]-4-methyl-7-oxabicyclo[2.2.1]heptane (1-747)

The same reaction and treatment as in Synthesis Example 235 were performed using 1-(bromomethyl)-2-methoxy-benzene instead of 1-bromo-3-methyl-but-2-ene to obtain a colorless liquid title compound (yield 46%).

Synthesis Example 240

Synthesis of (1R,2S,4S,5R)-2-iodo-1-isopropyl-4-methyl-5-[[2-(trifluoromethoxy)phenyl]methoxy]-7-oxabicyclo[2.2.1]heptane (1-750)

The same reaction and treatment as in Synthesis Example 235 were performed using 1-(bromomethyl)-2-(trifluoromethoxy)benzene instead of 1-bromo-3-methyl-but-2-ene to obtain a colorless liquid title compound (yield 88%).

Synthesis Example 241

Synthesis of (1R,2S,4S,5R)-5-[(2-chlorophenyl)methoxy]-2-iodo-1-isopropyl-4-methyl-7-oxabicyclo[2.2.1]heptane (1-756)

The same reaction and treatment as in Synthesis Example 235 were performed using 1-(bromomethyl)-2-chlorobenzene instead of 1-bromo-3-methyl-but-2-ene to obtain a colorless liquid title compound (yield 99%).

Synthesis Example 242

Synthesis of (1R,2S,4S,5R)-5-[(2-bromophenyl)methoxy]-2-iodo-1-isopropyl-4-methyl-7-oxabicyclo[2.2.1]heptane (1-759)

The same reaction and treatment as in Synthesis Example 235 were performed using 1-bromo-2-(bromomethyl) benzene instead of 1-bromo-3-methyl-but-2-ene to obtain a colorless liquid title compound (yield 80%).

Synthesis Example 243

Synthesis of (1R,2S,4S,5R)-2-iodo-5-[(2-iodophenyl)methoxy]-1-isopropyl-4-methyl-7-oxabicyclo[2.2.1]heptane (1-762)

The same reaction and treatment as in Synthesis Example 235 were performed using 1-(bromomethyl)-2-iodobenzene instead of 1-bromo-3-methyl-but-2-ene to obtain a colorless liquid title compound (yield 72%).

Synthesis Example 244

Synthesis of (1R,2S,4S,5R)-2-iodo-1-isopropyl-4-methyl-5-[[2-(trifluoromethyl)phenyl]methoxy]-7-oxabicyclo[2.2.1] heptane (1-765)

The same reaction and treatment as in Synthesis Example 235 were performed using 1-(bromomethyl)-3-(trifluoromethyl)benzene instead of 1-bromo-2-methyl-but-2-ene to obtain a colorless liquid title compound (yield 91%).

Synthesis Example 245

Synthesis of 2-[[(l S,2R,4R,5S)-5-iodo-4-isopropyl-l-methyl-7-oxabicyclo[2.2.1]heptan-2-yl]oxymethyl]benzonitrile (1-768)

The same reaction and treatment as in Synthesis Example 235 were performed using 2-(chloromethyl) benzonitrile instead of 1-bromo-3-methyl-but-2-ene to obtain an orange liquid title compound (yield 20%).

Synthesis Example 246

Synthesis of (1R,2S,4S,5R)-5-[(2,6-dimethylphenyl)methoxy]-2-iodo-1-isopropyl-4-methyl-7-oxabicyclo[2.2.1]heptane (1-777)

The same reaction and treatment as in Synthesis Example 235 were performed using 2-(chloromethyl)-1,3-dimethyl-benzene instead of 1-bromo-3-methyl-but-2-ene to obtain a colorless liquid title compound (yield 82%).

Synthesis Example 247

Synthesis of (1R,2S,4S,5R)-5-[(2,6-difluorophenyl)methoxy]-2-iodo-1-isopropyl-4-methyl-7-oxabicyclo[2.2.1]heptane (1-778)

The same reaction and treatment as in Synthesis Example 235 were performed using 2-(chloromethyl)-1,3-difluoro-benzene instead of 1-bromo-3-methyl-but-2-ene to obtain a colorless liquid title compound (yield 98%).

Synthesis Example 248

Synthesis of (1R,2S,4S,SR)-5-[(2,6-dichlorophenyl)methoxy]-2-iodo-1-isopropyl-4-methyl-7-oxabicyclo[2.2.1]heptane (1-779)

The same reaction and treatment as in Synthesis Example 235 were performed using 2-(bromomethyl)-1,3-dichloro-benzene instead of 1-bromo-3-methyl-but-2-ene to obtain a colorless liquid title compound (yield 94%).

Synthesis Example 249

Synthesis of (1R,2S,4S,5R)-5-[(2-chloro-6-fluoro-phenyl)methoxy]-2-iodo-1-isopropyl-4-methyl-7-oxabicyclo[2.2.1] heptane (1-780)

The same reaction and treatment as in Synthesis Example 235 were performed using 2-(bromomethyl)-1-chloro-3-fluoro-benzene instead of 1-bromo-3-methyl-but-2-ene to obtain a colorless liquid title compound (yield 100%).

Synthesis Example 250

Synthesis of 2-[[(18,2R,4R,58)-5-iodo-4-isopropyl-1-methyl-7-oxabicyclo[2.2.1]heptan-2-yl]oxymethyl]pyridine (1-785)

The same reaction and treatment as in Synthesis Example 235 were performed using 2-(chlomethyl)pyridine hydrochloride instead of 1-bromo-3-methyl-but-2-ene to obtain a colorless liquid title compound (yield 84%).

Synthesis Example 251

Synthesis of 3-[[(1S,2R,4R,5S)-5-iodo-4-isopropyl-1-methyl-7-oxabicyclo[2.2.1]heptan-2-yl]oxymethyl]pyridine (1-786)

The same reaction and treatment as in Synthesis Example 235 were performed using 3-(chlomethyl)pyridine hydrochloride instead of 1-bromo-3-methyl-but-2-ene to obtain a colorless liquid title compound (yield 92%).

Synthesis Example 252

Synthesis of (1R,2S,4S,5R)-2-iodo-1-isopropyl-4-methyl-5-(2-thienylmethoxy)-7-oxabicyclo[2.2.1]heptane (1-788)

The same reaction and treatment as in Synthesis Example 235 were performed using 2-(chloromethyl)thiophene instead of 1-bromo-3-methyl-but-2-ene to obtain a yellow liquid title compound (yield 53%).

Synthesis Example 253

Synthesis of (1R,2S,4S,5R)-2-iodo-1-isopropyl-4-methyl-5-(3-thienylmethoxy)-7-oxabicyclo[2.2.1]heptane (1-789)

The same reaction and treatment as in Synthesis Example 235 were performed using 3-(chloromethyl)thiophene instead of 1-bromo-3-methyl-but-2-ene to obtain a yellow liquid title compound (yield 10%).

Synthesis Example 254

Synthesis of 4-[[(1S,2R,4R,5S)-5-iodo-4-isopropyl-1-methyl-7-oxabicyclo [2.2.1] heptane-2-yl]oxymethyl]-3,5-dimethyl-isoxazole (1-790)

The same reaction and treatment as in Synthesis Example 235 were performed using 4-(chloromethyl)-3,5-dimethyl-isoxazole instead of 1-bromo-3-methyl-but-2-ene to obtain a colorless liquid title compound (yield 66%).

Synthesis Example 255

Synthesis of (1R,2S,4S,5R)-5-[(2-bromo-6-fluoro-phenyl)methoxy]-2-iodo-1-isopropyl-4-methyl-7-oxabicyclo[2.2.1] heptane (1-1614)

The same reaction and treatment as in Synthesis Example 235 were performed using 1-bromo-2-(bromomethyl)-3-fluoro-benzene instead of 1-bromo-3-methyl-but-2-ene to obtain a colorless liquid title compound (yield 70%).

Synthesis Example 256

Synthesis of (1R,2S,4S,SR)-5-iodo-1-methyl-2-phenylmethoxy-4-isopropyl-7-oxabicyclo[2.2.1]heptane (1-1081)

To a dimethylformamide solution (3 mL) of (1R,2S,4S,5R)-5-iodo-4-isopropyl-1-methyl-7-oxabicyclo[2.2.1]heptan-2-ol (200 mg, 0.675 mmol), sodium hydride (55% dispersion in mineral oil, 44.2 mg, 1.01 mmol) was added, followed by stirring in an ice bath (0°C) for 10 minutes. To the reaction mixture, benzyl bromide (127 mg, 0.743 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (142 mg, 54%).

Synthesis Example 257

Synthesis of 2-[[(1R,2S,4S,5R)-5-iodo-1-methyl-4-isopropyl-7-oxabicyclo[2.2.1]heptan-2-yl]oxymethyl]pyridine (1-1126)

The same reaction and treatment as in Synthesis Example 256 were performed using 2-(chloromethyl)pyridine; hydrochloride instead of benzyl bromide to obtain a white solid title compound (yield 30%).
Melting point: <60°C

Synthesis Example 258

Synthesis of 3-[[(1R,2S,4S,5R)-5-iodo-1-methyl-4-isopropyl-7-oxabicyclo[2.2.1]heptan-2-yl]oxymethyl]pyridine (1-1127)

The same reaction and treatment as in Synthesis Example 256 were performed using 3-(chloromethyl)pyridine; hydrochloride instead of benzyl bromide to obtain a colorless liquid title compound (yield 96%).

Synthesis Example 259

Synthesis of (1R,2S,4S,5R)-2-[(2,6-dimethylphenyl)methoxy]-5-iodo-1-methyl-4-isopropyl-7-oxabicyclo[2.2.1]heptane (1-1118)

The same reaction and treatment as in Synthesis Example 256 were performed using 2-(chloromethyl)-1,3-dimethyl-benzene instead of benzyl bromide to obtain a colorless liquid title compound (yield 75%).

Synthesis Example 260

Synthesis of (1R,2S,4S,5R)-2-[(2-bromo-6-fluorophenyl)methoxy]-5-iodo-1-methyl-4-isopropyl-7-oxabicyclo[2.2.1]heptane (1-1601)

The same reaction and treatment as in Synthesis Example 256 were performed using 1-bromo-2-(bromomethyl)-3-fluoro-benzene instead of benzyl bromide to obtain a colorless liquid title compound (yield 58%).

Synthesis Example 261

Synthesis of 4-[[(1R,2S,4S,5R)-5-iodo-1-methyl-4-isopropyl-7-oxabicyclo[2.2.1]heptan-2-yl]oxymethyl]pyridine (1-1128)

The same reaction and treatment as in Synthesis Example 256 were performed using 4-(chloromethyl)pyridine; hydrochloride instead of benzyl bromide to obtain a brown liquid title compound (yield 30%).

Synthesis Example 262

Synthesis of 4-[[(1R,2S,4S,5R)-5-iodo-1-methyl-4-isopropyl-7-oxabicyclo[2.2.1]heptan-2-yl]oxymethyl]-3,5-dimethyl-1,2-oxazole (1-1131)

The same reaction and treatment as in Synthesis Example 256 were performed using 4-(chloromethyl)-3,5-dimethyl-isoxazole instead of benzyl bromide to obtain a colorless liquid title compound (yield 73%).

Synthesis Example 263

Synthesis of methyl 2-[[(1R,2S,4S,5R)-5-iodo-1-methyl-4-isopropyl-7-oxabicyclo[2.2.1]heptan-2-yl]oxymethyl]benzoate (1-1115)

The same reaction and treatment as in Synthesis Example 256 were performed using methyl 2-(chloromethyl) benzoate instead of benzyl bromide to obtain a colorless liquid title compound (yield 20%).

Synthesis Example 264

Synthesis of (1R,2S,4S,5R)-2-[(2,6-dichlorophenyl)methoxy]-5-iodo-1-methyl-4-isopropyl-7-oxabicyclo[2.2.1]heptane (1-1120)

The same reaction and treatment as in Synthesis Example 256 were performed using 1,3-dichloro-2-(chloromethyl)benzene instead of benzyl bromide to obtain a colorless liquid title compound (yield 94%).

Synthesis Example 265

Synthesis of (1R,2S,4S,5R)-2-[(2,6-difluorophenyl)methoxy]-5-iodo-1-methyl-4-isopropyl-7-oxabicyclo[2.2.1]heptane (1-1119)

The same reaction and treatment as in Synthesis Example 256 were performed using 2-(chloromethyl)-1,3-difluoro-benzene instead of benzyl bromide to obtain a colorless liquid title compound (yield 88%).

Synthesis Example 266

Synthesis of (1R,2S,4S,5R)-2-[(2-chloro-6-fluorophenyl)methoxy]-5-iodo-1-methyl-4-isopropyl-7-oxabicyclo[2.2.1]heptane (1-1121)

The same reaction and treatment as in Synthesis Example 256 were performed using 2-(bromomethyl)-1-chloro-3-fluoro-benzene instead of benzyl bromide to obtain a colorless liquid title compound (yield 98%).

Synthesis Example 267

Synthesis of (1R,2S,4S,5R)-5-iodo-1-methyl-2-(3-methylbut-2-enoxy)-4-isopropyl-7-oxabicyclo[2.2.1]heptane (1-1073)

The same reaction and treatment as in Synthesis Example 256 were performed using 1-bromo-3-methyl-but-2-ene instead of benzyl bromide to obtain a colorless liquid title compound (yield 51%).

Synthesis Example 268

Synthesis of 2-[[(1R,2S,4S,5R)-5-iodo-1-methyl-4-isopropyl-7-oxabicyclo[2.2.1]heptan-2-yl]oxymethyl]benzonitrile (1-1109)

The same reaction and treatment as in Synthesis Example 256 were performed using 2-(chloromethyl) benzonitrile instead of benzyl bromide to obtain a pale yellow liquid title compound (yield 71%).

Synthesis Example 269

Synthesis of (1R,2S,4S,5R)-2-but-2-ynoxy-5-iodo-1-methyl-4-isopropyl-7-oxabicyclo[2.2.1]heptane (1-1075)

The same reaction and treatment as in Synthesis Example 256 were performed using 1-bromobut-2-yne instead of benzyl bromide to obtain a pale yellow liquid title compound (yield 40%).

Synthesis Example 270

Synthesis of (1R,2S,4S,5R)-5-iodo-2-[(2-iodophenyl)methoxy]-1-methyl-4-isopropyl-7-oxabicyclo[2.2.1]heptane (1-1103)

The same reaction and treatment as in Synthesis Example 256 were performed using 1-(bromomethyl)-2-iodobenzene instead of benzyl bromide to obtain a colorless liquid title compound (yield 74%).

Synthesis Example 271

Synthesis of (1R,2S,4S,5R)-2-[(2-bromophenyl)methoxy]-5-iodo-1-methyl-4-isopropyl-7-oxabicyclo[2.2.1]heptane (1-1100)

The same reaction and treatment as in Synthesis Example 256 were performed using 1-bromo-2-(bromomethyl) benzene instead of benzyl bromide to obtain a colorless liquid title compound (yield 74%).

Synthesis Example 272

Synthesis of (1R,2S,4S,5R)-5-iodo-1-methyl-4-isopropyl-2-[[2-(trifluoromethoxy)phenyl]methoxy]-7-oxabicyclo[2.2.1] heptane (1-1091)

The same reaction and treatment as in Synthesis Example 256 were performed using 1-(bromomethyl)-2-(trifluoromethoxy)benzene instead of benzyl bromide to obtain a colorless liquid title compound (yield 72%).

Synthesis Example 273

Synthesis of (1R,2S,4S,5R)-5-iodo-1-methyl-4-isopropyl-2-[[2-(trifluoromethyl)phenyl]methoxy]-7-oxabicyclo[2.2.1] heptane (1-1106)

The same reaction and treatment as in Synthesis Example 256 were performed using 1-(bromomethyl)-2-(trifluoromethyl)benzene instead of benzyl bromide to obtain a colorless liquid title compound (yield 77%).

Synthesis Example 274

Synthesis of (1R,2S,4S,5R)-2-[(2-chlorophenyl)methoxy]-5-iodo-1-methyl-4-isopropyl-7-oxabicyclo[2.2.1]heptane (1-1097)

The same reaction and treatment as in Synthesis Example 256 were performed using 1-(bromomethyl)-2-chlorobenzene instead of benzyl bromide to obtain a colorless liquid title compound (yield 33%).

Synthesis Example 275

Synthesis of (1R,2S,4S,5R)-5-iodo-2-[(2-methoxyphenyl)methoxy]-1-methyl-4-isopropyl-7-oxabicyclo[2.2.1]heptane (1-1088)

The same reaction and treatment as in Synthesis Example 256 were performed using 1-(bromomethyl)-2-methoxy-benzene instead of benzyl bromide to obtain a colorless liquid title compound (yield 38%).

Synthesis Example 276

Synthesis of (1R,2S,4S,5R)-5-iodo-1-methyl-2-[(2-methylphenyl)methoxy]-4-isopropyl-7-oxabicyclo[2.2.1]heptane (1-1082)

The same reaction and treatment as in Synthesis Example 256 were performed using 1-(chloromethyl)-2-methylbenzene instead of benzyl bromide to obtain a colorless liquid title compound (yield 74%).

Synthesis Example 277

Synthesis of 5-iodo-1-methyl-2-phenylmethoxy-4-isopropyl-7-oxabicyclo[2.2.1]heptane (1-1408)

To a dimethylformamide solution (3 mL) of 5-iodo-4-isopropyl-1-methyl-7-oxabicyclo[2.2.1]heptan-2-ol (200 mg, 0.675 mmol), sodium hydride (55% dispersion in mineral oil, 44.2 mg, 1.01 mmol) was added, followed by stirring in an ice bath (0°C) for 10 minutes. To the reaction mixture, benzyl bromide (127 mg, 0.743 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (219 mg, 84%).

Synthesis Example 278

Synthesis of 2-[(5-iodo-1-methyl-4-isopropyl-7-oxabicyclo[2.2.1]heptan-2-yl)oxymethyl]benzonitrile (1-1436)

The same reaction and treatment as in Synthesis Example 277 were performed using 2-(chloromethyl) benzonitrile instead of benzyl bromide to obtain a colorless liquid title compound (yield 73%).

Synthesis Example 279

Synthesis of 5-iodo-1-methyl-2-(3-methylbut-2-enoxy)-4-isopropyl-7-oxabicyclo[2.2.1]heptane (1-1400)

The same reaction and treatment as in Synthesis Example 277 were performed using 1-bromo-3-methyl-but-2-ene instead of benzyl bromide to obtain a colorless liquid title compound (yield 80%).

Synthesis Example 280

Synthesis of 5-iodo-1-methyl-2-[(2-methylphenyl)methoxy]-4-isopropyl-7-oxabicyclo[2.2.1]heptane (1-1409)

The same reaction and treatment as in Synthesis Example 277 were performed using 1-(chloromethyl)-2-methyl-benzene instead of benzyl bromide to obtain a colorless liquid title compound (yield 68%).

Synthesis Example 281

Synthesis of 5-iodo-2-[(2-methoxyphenyl)methoxy]-1-methyl-4-isopropyl-7-oxabicyclo[2.2.1]heptane (1-1415)

The same reaction and treatment as in Synthesis Example 277 were performed using 1-(bromomethyl)-2-methoxy-benzene instead of benzyl bromide to obtain a colorless liquid title compound (yield 37%).

Synthesis Example 282

Synthesis of 2-[(2-fluorophenyl)methoxy]-5-iodo-1-methyl-4-isopropyl-7-oxabicyclo[2.2.1]heptane (1-1421)

The same reaction and treatment as in Synthesis Example 277 were performed using 1-(bromomethyl)-2-fluorobenzene instead of benzyl bromide to obtain a colorless liquid title compound (yield 81%).

Synthesis Example 283

Synthesis of 5-but-2-ynoxy-2-iodo-1-isopropyl-4-methyl-7-oxabicyclo[2.2.1]heptane (1-1402)

The same reaction and treatment as in Synthesis Example 277 were performed using 1-bromobut-2-yne instead

of benzyl bromide to obtain a pale yellow liquid title compound (yield 46%).

Synthesis Example 284

Synthesis of 2-iodo-1-isopropyl-4-methyl-5-[[2-(trifluoromethoxy)phenyl]methoxy]-7-oxabicyclo[2.2.1]heptane (1-1418)

The same reaction and treatment as in Synthesis Example 277 were performed using 1-(bromomethyl)-2-(trifluoromethoxy)benzene instead of benzyl bromide to obtain a colorless liquid title compound (yield 73%).

Synthesis Example 285

Synthesis of 5-[(2-chlorophenyl)methoxy]-2-iodo-1-isopropyl-4-methyl-7-oxabicyclo[2.2.1]heptane (1-1424)

The same reaction and treatment as in Synthesis Example 277 were performed using 1-(bromomethyl)-2-chlorobenzene instead of benzyl bromide to obtain a colorless liquid title compound (yield 87%).

Synthesis Example 286

Synthesis of 5-[(2-bromophenyl)methoxy]-2-iodo-1-isopropyl-4-methyl-7-oxabicyclo[2.2.1]heptane (1-1427)

The same reaction and treatment as in Synthesis Example 277 were performed using 1-bromo-2-(bromomethyl) benzene instead of benzyl bromide to obtain a colorless liquid title compound (yield 69%).

Synthesis Example 287

Synthesis of 2-iodo-5-[(2-iodophenyl)methoxy]-1-isopropyl-4-methyl-7-oxabicyclo[2.2.1]heptane (1-1430)

The same reaction and treatment as in Synthesis Example 277 were performed using 1-(bromomethyl)-2-iodobenzene instead of benzyl bromide to obtain a colorless liquid title compound (yield 81%).

Synthesis Example 288

Synthesis of 2-iodo-1-isopropyl-4-methyl-5-[[2-(trifluoromethyl)phenyl]methoxy]-7-oxabicyclo[2.2.1]heptane (1-1433)

The same reaction and treatment as in Synthesis Example 277 were performed using 1-(bromomethyl)-2-(trifluoromethyl)benzene instead of benzyl bromide to obtain a colorless liquid title compound (yield 70%).

Synthesis Example 289

Synthesis of methyl 2-[(5-iodo-4-isopropyl-1-methyl-7-oxabicyclo[2.2.1]heptan-2-yl)oxymethyl]benzoate (1-1442)

The same reaction and treatment as in Synthesis Example 277 were performed using methyl 2-(chloromethyl) benzoate instead of benzyl bromide to obtain a colorless liquid title compound (yield 25%).

Synthesis Example 290

Synthesis of 5-[(2,6-dimethylphenyl)methoxy]-2-iodo-1-isopropyl-4-methyl-7-oxabicyclo[2.2.1]heptane (1-1445)

The same reaction and treatment as in Synthesis Example 277 were performed using 2-(chloromethyl)-1,3-dimethyl-benzene instead of benzyl bromide to obtain a colorless liquid title compound (yield 71%).

Synthesis Example 291

Synthesis of 5-[(2,6-difluorophenyl)methoxy]-2-iodo-1-isopropyl-4-methyl-7-oxabicyclo[2.2.1]heptane (1-1446)

The same reaction and treatment as in Synthesis Example 277 were performed using 2-(chloromethyl)-1,3-difluoro-benzene instead of benzyl bromide to obtain a colorless liquid title compound (yield 74%).

Synthesis Example 292

Synthesis of 5-[(2,6-dichlorophenyl)methoxy]-2-iodo-1-isopropyl-4-methyl-7-oxabicyclo[2.2.1]heptane (1-1447)

The same reaction and treatment as in Synthesis Example 277 were performed using 2-(bromomethyl)-1,3-dichloro-benzene instead of benzyl bromide to obtain a colorless liquid title compound (yield 76%).

Synthesis Example 293

Synthesis of 5-[(2-chloro-6-fluoro-phenyl)methoxy]-2-iodo-1-isopropyl-4-methyl-7-oxabicyclo[2.2.1]heptane (1-1448)

The same reaction and treatment as in Synthesis Example 277 were performed using 2-(bromomethyl)-1-chloro-3-fluoro-benzene instead of benzyl bromide to obtain a colorless liquid title compound (yield 67%).

Synthesis Example 294

Synthesis of 2-iodo-1-isopropyl-4-methyl-5-(2-thienylmethoxy)-7-oxabicyclo[2.2.1]heptane (1-1456)

The same reaction and treatment as in Synthesis Example 277 were performed using 2-(chloromethyl)thiophene instead of benzyl bromide to obtain a yellow liquid title compound (yield 60%).

Synthesis Example 295

Synthesis of 2-iodo-1-isopropyl-4-methyl-5-(3-thienylmethoxy)-7-oxabicyclo[2.2.1]heptane (1-1457)

The same reaction and treatment as in Synthesis Example 277 were performed using 3-(chloromethyl)thiophene instead of benzyl bromide to obtain a yellow liquid title compound (yield 49%).

Synthesis Example 296

Synthesis of 5-[(2-bromo-6-fluoro-phenyl)methoxy]-2-iodo-1-isopropyl-4-methyl-7-oxabicyclo[2.2.1]heptane (1-1612)

The same reaction and treatment as in Synthesis Example 277 were performed using 1-bromo-2-(bromomethyl)-3-fluoro-benzene instead of benzyl bromide to obtain a colorless liquid title compound (yield 67%).

Synthesis Example 297

Synthesis of (1R,2S,4S,SR)-5-benzyloxy-2-chloro-1-isopropyl-4-methyl-7-oxabicyclo[2.2.1]heptane (1-8)

To a dimethylformamide solution (3 mL) of (1S,2R,4R,5S)-5-chloro-4-isopropyl-1-methyl-7-oxabicyclo[2.2.1]heptan-2-ol (200 mg, 0.977 mmol), sodium hydride (55% dispersion in mineral oil, 58.6 mg, 1.47 mmol) was added, followed by stirring in an ice bath (0°C) for 10 minutes. To the reaction mixture, benzyl bromide (175 mg, 1.03 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (230 mg, 80%).

Synthesis Example 298

Synthesis of (1R,2S,4S,5R)-2-chloro-1-isopropyl-4-methyl-5-(o-tolylmethoxy)-7-oxabicyclo[2.2.1]heptane (1-9)

The same reaction and treatment as in Synthesis Example 297 were performed using 1-(chloromethyl)-2-methyl-benzene instead of benzyl bromide to obtain a colorless liquid title compound (yield 77%).

Synthesis Example 299

Synthesis of (1R,2S,4S,5R)-2-chloro-5-[(2,6-dimethylphenyl)methoxy]-1-isopropyl-4-methyl-7-oxabicyclo[2.2.1]heptane (1-10)

The same reaction and treatment as in Synthesis Example 297 were performed using 2-(chloromethyl)-1,3-dimethyl-benzene instead of benzyl bromide to obtain a pale yellow liquid title compound (yield 40%).

Synthesis Example 300

Synthesis of (1R,2S,4S,5R)-2-chloro-1-isopropyl-5-[(2-methoxyphenyl)methoxy]-4-methyl-7-oxabicyclo[2.2.1]heptane (1-11)

The same reaction and treatment as in Synthesis Example 297 were performed using 1-(bromomethyl)-2-methoxy-benzene instead of benzyl bromide to obtain a pale yellow liquid title compound (yield 62%).

Synthesis Example 301

Synthesis of (1R,2S,4S,5R)-2-chloro-5-[(2-chlorophenyl)methoxy]-1-isopropyl-4-methyl-7-oxabicyclo[2.2.1]heptane (1-13)

The same reaction and treatment as in Synthesis Example 297 were performed using 1-(bromomethyl)-2-chloro-benzene instead of benzyl bromide to obtain a colorless liquid title compound (yield 68%).

Synthesis Example 302

Synthesis of (1R,2S,4S,5R)-2-chloro-5-[(2,6-difluorophenyl)methoxy]-1-isopropyl-4-methyl-7-oxabicyclo[2.2.1]heptane (1-14)

The same reaction and treatment as in Synthesis Example 297 were performed using 2-(chloromethyl)-1,3-difluoro-benzene instead of benzyl bromide to obtain a colorless liquid title compound (yield 94%).

Synthesis Example 303

Synthesis of (1R,2S,4S,5R)-2-chloro-1-isopropyl-4-methyl-5-(3-methylbut-2-enoxy)-7-oxabicyclo[2.2.1]heptane (1-612)

The same reaction and treatment as in Synthesis Example 297 were performed using 1-bromo-3-methyl-but-2-ene instead of benzyl bromide to obtain a colorless liquid title compound (yield 94%).

Synthesis Example 304

Synthesis of (1R,2S,4S,5R)-5-but-2-ynoxy-2-chloro-1-isopropyl-4-methyl-7-oxabicyclo[2.2.1]heptane (1-614)

The same reaction and treatment as in Synthesis Example 297 were performed using 1-bromobut-2-yne instead of benzyl bromide to obtain a pale yellow liquid title compound (yield 96%).

Synthesis Example 305

Synthesis of (1R,2S,4S,SR)-2-chloro-1-isopropyl-4-methyl-5-[[2-(trifluoromethoxy)phenyl]methoxy]-7-oxabicyclo[2.2.1]heptane (1-627)

The same reaction and treatment as in Synthesis Example 297 were performed using 1-(bromomethyl)-2-(trifluoromethoxy)benzene instead of benzyl bromide to obtain a colorless liquid title compound (yield 88%).

Synthesis Example 306

Synthesis of (1R,2S,4S,5R)-2-chloro-1-isopropyl-4-methyl-5-[[2-(trifluoromethyl)phenyl]methoxy]-7-oxabicyclo[2.2.1]heptane (1-640)

The same reaction and treatment as in Synthesis Example 297 were performed using 1-(bromomethyl)-2-(trifluoromethyl)benzene instead of benzyl bromide to obtain a colorless liquid title compound (yield 81%).

Synthesis Example 307

Synthesis of (1R,2S,4S,5R)-2-chloro-5-[(2,6-dichlorophenyl)methoxy]-1-isopropyl-4-methyl-7-oxabicyclo[2.2.1]heptane (1-652)

The same reaction and treatment as in Synthesis Example 297 were performed using 2-(bromomethyl)-1,3-dichloro-benzene instead of benzyl bromide to obtain a colorless liquid title compound (yield 80%).

Synthesis Example 308

Synthesis of (1R,2S,4S,5R)-2-chloro-5-[(2-chloro-6-fluoro-phenyl)methoxy]-1-isopropyl-4-methyl-7-oxabicyclo[2.2.1]heptane (1-653)

The same reaction and treatment as in Synthesis Example 297 were performed using 2-(bromomethyl)-1-chloro-3-fluoro-benzene instead of benzyl bromide to obtain a colorless liquid title compound (yield 77%).

Synthesis Example 309

Synthesis of 2-[[(1S,2R,4R,5S)-5-chloro-4-isopropyl-1-methyl-7-oxabicyclo[2.2.1]heptan-2-yl]oxymethyl]pyridine (1-658)

The same reaction and treatment as in Synthesis Example 297 were performed using 2-(chlomethyl)pyridine hydrochloride instead of benzyl bromide to obtain a pale yellow liquid title compound (yield 5%).

Synthesis Example 310

Synthesis of (1R,2S,4S,5R)-2-chloro-1-isopropyl-4-methyl-5-(2-thienylmethoxy)-7-oxabicyclo[2.2.1]heptane (1-661)

The same reaction and treatment as in Synthesis Example 297 were performed using 2-(chloromethyl)thiophene instead of benzyl bromide to obtain a yellow liquid title compound (yield 37%).

Synthesis Example 311

Synthesis of (1R,2S,4S,5R)-2-chloro-1-isopropyl-4-methyl-5-(3-thienylmethoxy)-7-oxabicyclo[2.2.1]heptane (1-662)

The same reaction and treatment as in Synthesis Example 297 were performed using 3-(chloromethyl)thiophene instead of benzyl bromide to obtain a yellow liquid title compound (yield 37%).

Synthesis Example 312

Synthesis of 4-[[(1S,2R,4R,5S)-5-chloro-4-isopropyl-1-methyl-7-oxabicyclo[2.2.1]heptan-2-yl]oxymethyl]-3,5-dimethyl-isoxazole (1-663)

The same reaction and treatment as in Synthesis Example 297 were performed using 4-(chloromethyl)-3,5-dimethyl-isoxazole instead of benzyl bromide to obtain a colorless liquid title compound (yield 79%).

Synthesis Example 313

Synthesis of (1R,2S,4S,5R)-5-[(2-bromo-6-fluoro-phenyl)methoxy]-2-chloro-1-isopropyl-4-methyl-7-oxabicyclo[2.2.1]heptane (1-1613)

The same reaction and treatment as in Synthesis Example 297 were performed using 1-bromo-2-(bromomethyl)-3-fluoro-benzene instead of benzyl bromide to obtain a pale yellow liquid title compound (yield 82%).

Synthesis Example 314

Synthesis of 5-chloro-1-methyl-2-phenylmethoxy-4-isopropyl-7-oxabicyclo[2.2.1]heptane (1-1276)

To a dimethylformamide solution (3 mL) of 5-chloro-4-isopropyl-l-methyl-7-oxabicyclo[2.2.l ]heptan-2-ol (200 mg, 0.977 mmol), sodium hydride (55% dispersion in mineral oil, 51.2 mg, 1.17 mmol) was added, followed by stirring in an ice bath (0°C) for 10 minutes. To the reaction mixture, benzyl bromide (167 mg, 1.17 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (190 mg, 66%).

Synthesis Example 315

Synthesis of 5-chloro-2-[(2-chlorophenyl)methoxy]-1-methyl-4-isopropyl-7-oxabicyclo[2.2.1]heptane (1-1292)

The same reaction and treatment as in Synthesis Example 314 were performed using 1-(bromomethyl)-2-chloro-benzene instead of benzyl bromide to obtain a colorless liquid title compound (yield 68%).

Synthesis Example 316

Synthesis of 5-chloro-2-[(2-iodophenyl)methoxy]-1-methyl-4-isopropyl-7-oxabicyclo[2.2.1]heptane (1-1298)

The same reaction and treatment as in Synthesis Example 314 were performed using 1-(bromomethyl)-2-iodo-benzene instead of benzyl bromide to obtain a colorless liquid title compound (yield 75%).

Synthesis Example 317

Synthesis of 5-chloro-1-methyl-4-isopropyl-2-[[2-(trifluoromethyl)phenyl]methoxy]-7-oxabicyclo[2.2.1]heptane (1-1301)

The same reaction and treatment as in Synthesis Example 314 were performed using 1-(bromomethyl)-2-(trifluoromethyl)benzene instead of benzyl bromide to obtain a colorless liquid title compound (yield 87%).

Synthesis Example 318

Synthesis of 5-chloro-2-[(2,6-difluorophenyl)methoxy]-1-methyl-4-isopropyl-7-oxabicyclo[2.2.1]heptane (1-1313)

The same reaction and treatment as in Synthesis Example 314 were performed using 2-(chloromethyl)-1,3-difluoro-benzene instead of benzyl bromide to obtain a colorless liquid title compound (yield 87%).

Synthesis Example 319

Synthesis of 5-chloro-2-[(2-chloro-6-fluorophenyl)methoxy]-1-methyl-4-isopropyl-7-oxabicyclo[2.2.1]heptane (1-1315)

The same reaction and treatment as in Synthesis Example 314 were performed using 2-(bromomethyl)-1-chloro-3-fluoro-benzene instead of benzyl bromide to obtain a colorless liquid title compound (yield 32%).

Synthesis Example 320

Synthesis of 2-[(2-bromo-6-fluorophenyl)methoxy]-5-chloro-1-methyl-4-isopropyl-7-oxabicyclo[2.2.1]heptane (1-1611)

The same reaction and treatment as in Synthesis Example 314 were performed using 1-bromo-2-(bromomethyl)-3-fluoro-benzene instead of benzyl bromide to obtain a colorless liquid title compound (yield 60%).

Synthesis Example 321

Synthesis of 5-chloro-2-[(2,6-dichlorophenyl)methoxy]-1-methyl-4-isopropyl-7-oxabicyclo[2.2.1]heptane (1-1314)

The same reaction and treatment as in Synthesis Example 314 were performed using 2-(bromomethyl)-1,3-dichloro-benzene instead of benzyl bromide to obtain a colorless liquid title compound (yield 67%).

Synthesis Example 322

Synthesis of 5-chloro-2-[(2,6-dimethylphenyl)methoxy]-1-methyl-4-isopropyl-7-oxabicyclo[2.2.1]heptane (1-1610)

The same reaction and treatment as in Synthesis Example 314 were performed using 2-(chloromethyl)-1,3-dimethyl-benzene instead of benzyl bromide to obtain a colorless liquid title compound (yield 61%).

Synthesis Example 323

Synthesis of 5-chloro-1-methyl-2-(3-methylbut-2-enoxy)-4-isopropyl-7-oxabicyclo[2.2.1]heptane (1-1268)

The same reaction and treatment as in Synthesis Example 314 were performed using 1-bromo-3-methyl-but-2-ene instead of benzyl bromide to obtain a colorless liquid title compound (yield 49%).

Synthesis Example 324

Synthesis of 2-[(5-chloro-1-methyl-4-isopropyl-7-oxabicyclo[2.2.1]heptan-2-yl)oxymethyl]benzonitrile (1-1304)

The same reaction and treatment as in Synthesis Example 314 were performed using 2-(chloromethyl) benzonitrile instead of benzyl bromide to obtain a yellow liquid title compound (yield 15%).

Synthesis Example 325

Synthesis of 2-but-2-ynoxy-5-chloro-1-methyl-4-isopropyl-7-oxabicyclo[2.2.1]heptane (1-1270)

The same reaction and treatment as in Synthesis Example 314 were performed using 1-bromobut-2-yne instead of benzyl bromide to obtain a colorless liquid title compound (yield 44%).

Synthesis Example 326

Synthesis of 5-chloro-2-[(2-fluorophenyl)methoxy]-1-methyl-4-isopropyl-7-oxabicyclo[2.2.1]heptane (1-1289)

The same reaction and treatment as in Synthesis Example 314 were performed using 1-(chloromethyl)-2-fluoro-benzene instead of benzyl bromide to obtain a colorless liquid title compound (yield 75%).

Synthesis Example 327

Synthesis of 5-chloro-1-methyl-2-[(2-methylphenyl)methoxy]-4-isopropyl-7-oxabicyclo[2.2.1]heptane (1-1277)

The same reaction and treatment as in Synthesis Example 314 were performed using 1-(chloromethyl)-2-methyl-benzene instead of benzyl bromide to obtain a colorless liquid title compound (yield 80%).

Synthesis Example 328

Synthesis of 5-chloro-2-[(2-methoxyphenyl)methoxy]-1-methyl-4-isopropyl-7-oxabicyclo[2.2.1]heptane (1-1283)

The same reaction and treatment as in Synthesis Example 314 were performed using 1-(bromomethyl)-2-methoxy-benzene instead of benzyl bromide to obtain a colorless liquid title compound (yield 26%).

Synthesis Example 329

Synthesis of 2-[(2-bromophenyl)methoxy]-5-chloro-1-methyl-4-isopropyl-7-oxabicyclo[2.2.1]heptane (1-1295)

The same reaction and treatment as in Synthesis Example 314 were performed using 1-bromo-2-(bromomethyl) benzene instead of benzyl bromide to obtain a colorless liquid title compound (yield 47%).

Synthesis Example 330

Synthesis of 2-chloro-1-isopropyl-4-methyl-5-[[2-(trifluoromethoxy)phenyl]methoxy]-7-oxabicyclo[2.2.1]heptane (1-1286)

The same reaction and treatment as in Synthesis Example 314 were performed using 1-(bromomethyl)-2-(trifluoromethoxy)benzene instead of benzyl bromide to obtain a pale yellow liquid title compound (yield 54%).

Synthesis Example 331

Synthesis of methyl 2-[(5-chloro-4-isopropyl-1-methyl-7-oxabicyclo[2.2.1]heptan-2-yl)oxymethyl]benzoate (1-1310)

The same reaction and treatment as in Synthesis Example 314 were performed using methyl 2-(chloromethyl) benzoate instead of benzyl bromide to obtain a pale yellow liquid title compound (yield 26%).

Synthesis Example 332

Synthesis of 2-[(5-chloro-4-isopropyl-1-methyl-7-oxabicyclo[2.2.1]heptan-2-yl)oxymethyl]pyridine (1-1320)

The same reaction and treatment as in Synthesis Example 314 were performed using 2-(chlomethyl)pyridine hydrochloride instead of benzyl bromide to obtain a light brown solid title compound (yield 62%).
Melting point: 88.0°C to 90.5°C

Synthesis Example 333

Synthesis of 3-[(5-chloro-4-isopropyl-1-methyl-7-oxabicyclo[2.2.1]heptan-2-yl)oxymethyl]pyridine (1-1321)

The same reaction and treatment as in Synthesis Example 314 were performed using 3-(chlomethyl)pyridine hydrochloride instead of benzyl bromide to obtain a brown liquid title compound (yield 8%).

Synthesis Example 334

Synthesis of 4-[(5-chloro-4-isopropyl-1-methyl-7-oxabicyclo[2.2.1]heptan-2-yl)oxymethyl]pyridine (1-1322)

The same reaction and treatment as in Synthesis Example 314 were performed using 4-(chlomethyl)pyridine hydrochloride instead of benzyl bromide to obtain a brown liquid title compound (yield 5%).

Synthesis Example 335

Synthesis of 4-[(5-chloro-4-isopropyl-1-methyl-7-oxabicyclo[2.2.1]heptan-2-yl)oxymethyl]-3,5-dimethyl-isoxazole (1-1325)

The same reaction and treatment as in Synthesis Example 314 were performed using 4-(chloromethyl)-3,5-dimethyl-isoxazole instead of benzyl bromide to obtain a white solid title compound (yield 65%).
Melting point: <60°C

Synthesis Example 336

Synthesis of 2-chloro-1-isopropyl-4-methyl-5-(2-thienylmethoxy)-7-oxabicyclo[2.2.1]heptane (1-1323)

The same reaction and treatment as in Synthesis Example 314 were performed using 2-(chloromethyl)thiophene instead of benzyl bromide to obtain a yellow liquid title compound (yield 48%).

Synthesis Example 337

Synthesis of 2-chloro-1-isopropyl-4-methyl-5-(3-thienylmethoxy)-7-oxabicyclo[2.2.1]heptane (1-1324)

The same reaction and treatment as in Synthesis Example 314 were performed using 3-(chloromethyl)thiophene instead of benzyl bromide to obtain a yellow liquid title compound (yield 44%).

Synthesis Example 338

Synthesis of (1R,4S,5R)-4-methyl-5-phenylmethoxy-1-isopropyl-7-oxabicyclo[2.2.1]hept-2-ene (3-1)

To a tetrahydrofuran solution (20 mL) of (1R,2S,4S,SR)-5-benzyloxy-2-bromo-1-isopropyl-4-methyl-7-oxabicyclo[2.2.1]heptane (9.60 g, 28.0 mmol), tert-butoxypotassium (9.50 g, 85.0 mmol) was added, followed by stirring under heated reflux for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (6.20 mg, 85%).

Synthesis Example 339

Synthesis of (1R,4S,SR)-5-[(2-fluorophenyl)methoxy]-4-methyl-1-isopropyl-7-oxabicyclo[2.2.1]hept-2-ene (3-5)

The same reaction and treatment as in Synthesis Example 338 were performed using (1R,2S,4S,5R)-2-bromo-5-[(2-fluorophenyl)methoxy]-1-isopropyl-4-methyl-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a colorless liquid title compound (yield 64%).

Synthesis Example 340

Synthesis of (1R,4S,5R)-4-methyl-5-[(2-methylphenyl)methoxy]-1-isopropyl-7-oxabicyclo[2.2.1]hept-2-ene (3-2)

The same reaction and treatment as in Synthesis Example 338 were performed using (1R,2S,4S,5R)-2-bromo-1-isopropyl-4-methyl-5-(o-tolylmethoxy)-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a colorless liquid title compound (yield 57%).

Synthesis Example 341

Synthesis of 2-[[(1S,2R,4R)-1-methyl-4-isopropyl-7-oxabicyclo[2.2.1]hept-5-en-2-yl]oxymethyl]pyridine (3-738)

The same reaction and treatment as in Synthesis Example 338 were performed using 2-[[(1S,2R,4R,5S)-5-bromo-4-isopropyl-1-methyl-7-oxabicyclo[2.2.1]heptan-2-yl]oxymethyl]pyridine instead of (1R,2S,4S,5R)-5-benzyloxy-2-bromo-1-isopropyl-4-methyl-7-oxabicyclo[2.2.1]heptane to obtain a colorless liquid title compound (yield 57%).

Synthesis Example 342

Synthesis of (1R,4S,5R)-5-[(2,6-dimethylphenyl)methoxy]-4-methyl-1-isopropyl-7-oxabicyclo[2.2.1]hept-2-ene (3-3)

The same reaction and treatment as in Synthesis Example 338 were performed using (1R,2S,4S,5R)-2-bromo-5-[(2,6-dimethylphenyl)methoxy]-1-isopropyl-4-methyl-7-oxabicyclo[2.2.1]heptane instead of (1R,2S,4S,SR)-5-benzyloxy-2-bromo-1-isopropyl-4-methyl-7-oxabicyclo[2.2.1]heptane to obtain a colorless liquid title compound (yield 85%).

Synthesis Example 343

Synthesis of (1R,4S,5R)-5-[(2,6-difluorophenyl)methoxy]-4-methyl-1-isopropyl-7-oxabicyclo[2.2.1]hept-2-ene (3-7)

The same reaction and treatment as in Synthesis Example 338 were performed using (1R,2S,4S,5R)-2-bromo-5-[(2,6-difluorophenyl)methoxy]-1-isopropyl-4-methyl-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a yellow liquid title compound (yield 39%).

Synthesis Example 344

Synthesis of (1R,4S,5R)-5-[(2,6-dichlorophenyl)methoxy]-4-methyl-1-isopropyl-7-oxabicyclo[2.2.1]hept-2-ene (3-732)

The same reaction and treatment as in Synthesis Example 338 were performed using (1R,2S,4S,5R)-2-bromo-5-[(2,6-dichlorophenyl)methoxy]-1-isopropyl-4-methyl-7-oxabicyclo[2.2.1]heptane instead of (1R,2S,4S,SR)-5-benzyloxy-2-bromo-1-isopropyl-4-methyl-7-oxabicyclo[2.2.1]heptane to obtain a yellow liquid title compound (yield 72%).

Synthesis Example 345

Synthesis of (1S,4R,5S)-4-methyl-5-phenylmethoxy-1-isopropyl-7-oxabicyclo[2.2.1]hept-2-ene (3-748)

The same reaction and treatment as in Synthesis Example 338 were performed using (1S,2R,4R,5S)-5-benzyloxy-2-bromo-1-isopropyl-4-methyl-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a colorless liquid title compound (yield 52%).

Synthesis Example 346

Synthesis of (1S,4R,5S)-5-[(2-fluorophenyl)methoxy]-4-methyl-1-isopropyl-7-oxabicyclo[2.2.1]hept-2-ene (3-750)

The same reaction and treatment as in Synthesis Example 338 were performed using (1S,2R,4R,5S)-2-bromo-5-[(2-fluorophenyl)methoxy]-1-isopropyl-4-methyl-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a colorless liquid title compound (yield 83%).

Synthesis Example 347

Synthesis of (1S,4R,5S)-4-methyl-5-[(2-methylphenyl)methoxy]-1-isopropyl-7-oxabicyclo[2.2.1]hept-2-ene (3-749)

The same reaction and treatment as in Synthesis Example 338 were performed using (1S,2R,4R,5S)-2-bromo-1-isopropyl-4-methyl-5-(o-tolylmethoxy)-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a colorless liquid title compound (yield 100%).

Synthesis Example 348

Synthesis of (1S,4R,5S)-5-[(2,4-dichlorophenyl)methoxy]-4-methyl-1-isopropyl-7-oxabicyclo[2.2.1]hept-2-ene (3-751)

The same reaction and treatment as in Synthesis Example 338 were performed using (1S,2R,4R,5S)-2-bromo-5-[(2,4-dichlorophenyl)methoxy]-1-isopropyl-4-methyl-7-oxabicyclo[2.2.1]heptane instead of (1R,2S,4S,SR)-5-benzyloxy-2-bromo-1-isopropyl-4-methyl-7-oxabicyclo[2.2.1]heptane to obtain a colorless liquid title compound (yield 35%).

Synthesis Example 349

Synthesis of 4-methyl-5-[(2-methylphenyl)methoxy]-1-isopropyl-7-oxabicyclo[2.2.1]hept-2-ene (3-753)

The same reaction and treatment as in Synthesis Example 338 were performed using 2-bromo-1-isopropyl-4-methyl-5-(o-tolylmethoxy)-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a colorless liquid title compound (yield 69%).

Synthesis Example 350

Synthesis of 4-methyl-5-phenylmethoxy-1-isopropyl-7-oxabicyclo[2.2.1]hept-2-ene (3-752)

The same reaction and treatment as in Synthesis Example 338 were performed using 5-benzyloxy-2-bromo-1-isopropyl-4-methyl-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a colorless liquid title compound (yield 74%).

Synthesis Example 351

Synthesis of 5-[(2-fluorophenyl)methoxy]-4-methyl-1-isopropyl-7-oxabicyclo[2.2.1]hept-2-ene (3-754)

The same reaction and treatment as in Synthesis Example 338 were performed using 2-bromo-5-[(2-fluorophenyl)methoxy]-1-isopropyl-4-methyl-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a colorless liquid title compound (yield 57%).

Synthesis Example 352

Synthesis of 2-[(1-methyl-4-isopropyl-7-oxabicyclo[2.2.1]hept-5-en-2-yl)oxymethyl]pyridine (3-755)

The same reaction and treatment as in Synthesis Example 338 were performed using 2-[(5-bromo-4-isopropyl-1-methyl-7-oxabicyclo[2.2.1]heptan-2-yl)oxymethyl]pyridine instead of (1R,2S,4S,5R)-5-benzyloxy-2-bromo-1-isopropyl-4-methyl-7-oxabicyclo[2.2.1]heptane to obtain a pale yellow liquid title compound (yield 66%).

Synthesis Example 353

Synthesis of (1R,4S,5R)-1-isopropyl-4-methyl-5-(3-methylbut-2-enoxy)-7-oxabicyclo[2.2.1]hept-2-ene (3-693)

The same reaction and treatment as in Synthesis Example 338 were performed using (1R,2S,4S,5R)-2-iodo-1-isopropyl-4-methyl-5-(3-methylbut-2-enoxy)-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a colorless liquid title compound (yield 30%).

Synthesis Example 354

Synthesis of (1R,4S,5R)-5-(cyclohexylmethoxy)-1-isopropyl-4-methyl-7-oxabicyclo[2.2.1]hept-2-ene (3-697)

The same reaction and treatment as in Synthesis Example 338 were performed using (1R,2S,4S,5R)-5-(cyclohexylmethoxy)-2-iodo-1-isopropyl-4-methyl-7-oxabicyclo[2.2.1]heptane instead of (1R,2S,4S,5R)-5-benzyloxy-2-bromo-1-isopropyl-4-methyl-7-oxabicyclo[2.2.1]heptane to obtain a pale yellow liquid title compound (yield 100%).

Synthesis Example 355

Synthesis of 1-isopropyl-4-methyl-5-(2-thienylmethoxy)-7-oxabicyclo[2.2.1]hept-2-ene (3-756)

The same reaction and treatment as in Synthesis Example 338 were performed using 2-iodo-1-isopropyl-4-methyl-5-(2-thienylmethoxy)-7-oxabicyclo[2.2.1]heptane instead of (1R,2S,4S,SR)-5-benzyloxy-2-bromo-1-isopropyl-4-methyl-7-oxabicyclo[2.2.1]heptane to obtain a colorless liquid title compound (yield 20%).

Synthesis Example 356

Synthesis of 1-isopropyl-4-methyl-5-(3-thienylmethoxy)-7-oxabicyclo[2.2.1]hept-2-ene (3-757)

The same reaction and treatment as in Synthesis Example 338 were performed using 2-iodo-1-isopropyl-4-methyl-5-(3-thienylmethoxy)-7-oxabicyclo[2.2.1]heptane instead of (1R,2S,4S,SR)-5-benzyloxy-2-bromo-1-isopropyl-4-methyl-7-oxabicyclo[2.2.1]heptane to obtain a colorless liquid title compound (yield 20%).

Synthesis Example 357

Synthesis of 2-[[(1S,2R,4R)-1-methyl-4-isopropyl-7-oxabicyclo[2.2.1]hept-5-en-2-yl]oxy]pyridine (3-688)

To a toluene solution (5 mL) of (1S,2R,4R)-4-isopropyl-1-methyl-7-oxabicyclo[2.2.1]hept-5-en-2-ol (200 mg, 1.19 mmol), 2-chloropyridine (135 mg, 1.19 mmol), 85.5% potassium hydroxide (220 mg, 3.35 mmol), and 18-CROWN-6 (15.7 mg, 0.0134 mmol) were added, followed by stirring under heated reflux for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a yellow solid title compound (55.0 mg, 19%).
Melting point: 62.1°C to 64.3°C

Synthesis Example 358

Synthesis of (1S,2R,4R,5S)-2-[(2-fluorophenyl)methoxy]-1,5-dimethyl-4-isopropyl-7-oxabicyclo[2.2.1]heptane (1-93)

To a dimethylformamide solution (5 mL) of (1S,2R,4R,5S)-4-isopropyl-1,5-dimethyl-7-oxabicyclo[2.2.1]heptan-2-ol (480 mg, 2.60 mmol), sodium hydride (55% dispersion in mineral oil, 130 mg, 3.10 mmol) was added, followed by stirring in an ice bath (0°C) for 10 minutes. To the reaction mixture, 1-(bromomethyl)-2-fluoro-benzene (590 mg, 3.10 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (450 mg, 59%).

Synthesis Example 359

Synthesis of (1S,2R,4R,5R)-2-[(2-fluorophenyl)methoxy]-1,5-dimethyl-4-isopropyl-7-oxabicyclo[2.2.1]heptane (1-94)

The same reaction and treatment as in Synthesis Example 358 were performed using (1S,2R,4R,5R)-4-isopropyl-1,5-dimethyl-7-oxabicyclo[2.2.1]heptan-2-ol instead of (1S,2R,4R,5S)-4-isopropyl-1,5-dimethyl-7-oxabicyclo [2.2.1]heptan-2-ol to obtain a colorless liquid title compound (yield 84%).

Synthesis Example 360

Synthesis of (1R,2S,4S,5S)-2-[(2-fluorophenyl)methoxy]-1,5-dimethyl-4-isopropyl-7-oxabicyclo[2.2.1]heptane (1-1222)

The same reaction and treatment as in Synthesis Example 358 were performed using (1R,2S,4S,5S)-4-isopropyl-1,5-dimethyl-7-oxabicyclo[2.2.1]heptan-2-ol instead of (1S,2R,4R,5S)-4-isopropyl-1,5-dimethyl-7-oxabicyclo [2.2.1]heptan-2-ol to obtain a pale yellow liquid title compound (yield 39%).

Synthesis Example 361

Synthesis of (1R,2S,4S,5R)-2-[(2-fluorophenyl)methoxy]-1,5-dimethyl-4-isopropyl-7-oxabicyclo[2.2.1]heptane (1-1221)

The same reaction and treatment as in Synthesis Example 358 were performed using (1R,2S,4S,5R)-4-isopropyl-1,5-dimethyl-7-oxabicyclo[2.2.1]heptan-2-ol instead of (1S,2R,4R,5S)-4-isopropyl-1,5-dimethyl-7-oxabicyclo [2.2.1]heptan-2-ol to obtain a colorless liquid title compound (yield 87%).

Synthesis Example 362

Synthesis of (1SR,2RS,4RS,5RS)-2-[(2-fluorophenyl)methoxy]-1,5-dimethyl-4-isopropyl-7-oxabicyclo[2.2.1]heptane (1-1597)

The same reaction and treatment as in Synthesis Example 358 were performed using a 1:1 mixture (racemate) of (1S,2R,4R,5R)-4-isopropyl-1,5-dimethyl-7-oxabicyclo[2.2.1]heptan-2-ol and (1R,2S,4S,5S)-4-isopropyl-1,5-dimethyl-7-oxabicyclo[2.2.1]heptan-2-ol instead of (1S,2R,4R,5S)-4-isopropyl-1,5-dimethyl-7-oxabicyclo[2.2.1]heptan-2-ol to obtain a colorless liquid title compound (yield 41%).

Synthesis Example 363

Synthesis of (1SR,2RS,4RS,5SR)-2-[(2-fluorophenyl)methoxy]-1,5-dimethyl-4-isopropyl-7-oxabicyclo[2.2.1]heptane (1-1555)

The same reaction and treatment as in Synthesis Example 358 were performed using a 1:1 mixture (racemate) of (1S,2R,4R,5S)-4-isopropyl-1,5-dimethyl-7-oxabicyclo[2.2.1]heptan-2-ol and (1R,2S,4S,5R)-4-isopropyl-1,5-dimethyl-7-oxabicyclo[2.2.1]heptan-2-ol instead of (1S,2R,4R,5S)-4-isopropyl-1,5-dimethyl-7-oxabicyclo[2.2.1]heptan-2-ol to obtain a white solid title compound (yield 52%).
Melting point: 41.3°C to 43.6°C

Synthesis Example 364

Synthesis of (1R,2R,4S,5R)-5-[(2-fluorophenyl)methoxy]-4-methyl-1-isopropyl-7-oxabicyclo[2.2.1]heptan-2-ol (1-68)

To a tetrahydrofuran solution (10 mL) of (1R,3S,4R,6R)-4-[(2-fluorophenyl)methoxy]-6-isopropyl-3-methyl-7-oxabicyclo[4.1.0]heptan-3-ol (490 mg, 1.66 mmol), tosylic acid monohydrate (317 mg, 1.66 mmol) was added, followed by stirring at room temperature (25°C) for 3 hours. Saturated sodium bicarbonate water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a white solid title compound (186 mg, 38%).
Melting point: 91°C to 95°C

Synthesis Example 365

Synthesis of (1R,2S,4S,5R)-5-[(2-fluorophenyl)methoxy]-1-isopropyl-4-methyl-2-propyl-7-oxabicyclo[2.2.1]heptan-2-ol (1-934)

To a tetrahydrofuran solution (10 mL) of (1R,4S,5R)-5-[(2-fluorophenyl)methoxy]-1-isopropyl-4-methyl-7-oxabicyclo[2.2.1]heptan-2-one (500 mg, 1.71 mmol), a 2 mol/L bromo(propyl)magnesium tetrahydrofuran solution (1.28 mL) was added in an ice bath (0°C), followed by stirring for 1 hour in an ice bath (0°C). Dilute hydrochloric acid was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (140 mg, 24%).

Synthesis Example 366

Synthesis of (1S,2R,4R,5R)-2-[(2-fluorophenyl)methoxy]-1-methyl-4-isopropyl-5-propyl-7-oxabicyclo[2.2.1]heptane (1-914)

To a dichloromethane solution (5 mL) of (1R,2S,4S,5R)-5-[(2-fluorophenyl)methoxy]-1-isopropyl-4-methyl-2-propyl-7-oxabicyclo[2.2.1]heptan-2-ol (120 mg, 0.357 mmol), triethylsilane (241 mg, 2.07 mmol) and boron trifluoride diethyl etherate (810 mg, 5.71 mmol) were added in an ice bath (0°C), followed by stirring at room temperature (25°C) for 12 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (53.6 mg, 47%).

Synthesis Example 367

Synthesis of (1S,2R,4R,5R)-2-[(2-fluorophenyl)methoxy]-5-methoxy-1-methyl-4-isopropyl-7-oxabicyclo[2.2.1]heptane (1-173)

To a dimethylformamide solution (1 mL) of (1R,2R,4S,SR)-5-[(2-fluorophenyl)methoxy]-1-isopropyl-4-methyl-7-oxabicyclo[2.2.1]heptan-2-ol (150 mg, 0.510 mmol), sodium hydride (55% dispersion in mineral oil, 28.9 mg, 0.662 mmol) was added, followed by stirring in an ice bath (0°C) for 10 minutes. To the reaction mixture, iodomethane (94.0 mg, 0.662 mmol) was added, followed by stirring at room temperature (25° C.) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (108 mg, 69%).

Synthesis Example 368

Synthesis of (1S,2R,4R,SR)-5-ethoxy-2-[(2-fluorophenyl)methoxy]-1-methyl-4-isopropyl-7-oxabicyclo[2.2.1]heptane (1-916)

The same reaction and treatment as in Synthesis Example 367 were performed using iodoethane instead of iodomethane to obtain a colorless liquid title compound (yield 61%).

Synthesis Example 369

Synthesis of (1S,2R,4R,5R)-2-[(2-fluorophenyl)methoxy]-1-methyl-4-isopropyl-5-propoxy-7-oxabicyclo[2.2.1]heptane (1-917)

The same reaction and treatment as in Synthesis Example 367 were performed using 1-iodopropane instead of iodomethane to obtain a colorless liquid title compound (yield 18%).

Synthesis Example 370

Synthesis of (1S,2R,4R,5R)-2-[(2-fluorophenyl)methoxy]-1-methyl-4-isopropyl-5-prop-2-enoxy-7-oxabicyclo[2.2.1]heptane (1-930)

The same reaction and treatment as in Synthesis Example 367 were performed using 3-bromoprop-1-ene instead of iodomethane to obtain a colorless liquid title compound (yield 59%).

Synthesis Example 371

Synthesis of (1S,2R,4R,5R)-2-[(2-fluorophenyl)methoxy]-1-methyl-4-isopropyl-5-prop-2-ynoxy-7-oxabicyclo[2.2.1]heptane (1-931)

The same reaction and treatment as in Synthesis Example 367 were performed using 3-bromoprop-1-yne instead of iodomethane to obtain a colorless liquid title compound (yield 35%).

Synthesis Example 372

Synthesis of (1S,2R,4R,SR)-5-(cyclopropylmethoxy)-2-[(2-fluorophenyl)methoxy]-1-methyl-4-isopropyl-7-oxabicyclo[2.2.1]heptane (1-921)

The same reaction and treatment as in Synthesis Example 367 were performed using bromomethylcyclopropane instead of iodomethane to obtain a colorless liquid title compound (yield 23%).

Synthesis Example 373

Synthesis of (1S,2R,4R,5R)-2-[(2-fluorophenyl)methoxy]-1-methyl-5-phenylmethoxy-4-isopropyl-7-oxabicyclo[2.2.1]heptane (1-918)

The same reaction and treatment as in Synthesis Example 367 were performed using bromomethylbenzene instead of iodomethane to obtain a colorless liquid title compound (yield 49%).

Synthesis Example 374

Synthesis of 2-[[(1R,2R,4S,5R)-5-[(2-fluorophenyl)methoxy]-4-methyl-1-isopropyl-7-oxabicyclo[2.2.1]heptan-2-yl]oxymethyl]pyridine (1-919)

The same reaction and treatment as in Synthesis Example 367 were performed using 2-(chloromethyl)pyridine hydrochloride instead of iodomethane to obtain a colorless liquid title compound (yield 31%).

Synthesis Example 375

Synthesis of [(1R,2R,4S,5R)-5-[(2-fluorophenyl)methoxy]-4-methyl-1-isopropyl-7-oxabicyclo[2.2.1]heptan-2-yl]acetate (1-932)

The same reaction and treatment as in Synthesis Example 367 were performed using acetyl chloride instead of iodomethane to obtain a colorless liquid title compound (yield 41%).

Synthesis Example 376

Synthesis of [(1R,2R,4S,5R)-5-[(2-fluorophenyl)methoxy]-4-methyl-1-isopropyl-7-oxabicyclo[2.2.1]heptan-2-yl] N-ethylcarbamate (1-933)

To a toluene solution (1 mL) of (1R,2R,4S,SR)-5-[(2-fluorophenyl)methoxy]-1-isopropyl-4-methyl-7-oxabicyclo [2.2.1]heptan-2-ol (150 mg, 0.510 mmol), isocyanatoethane (47.1 mg, 0.662 mmol) was added, followed by reacting at 100°C for 1 hour using a microwave reaction apparatus. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a white solid title compound (70.0 mg, 38%).
Melting point: 68.5°C to 71.3°C

Synthesis Example 377

Synthesis of (1S,2R,4R,5S)-2-[(2-fluorophenyl)methoxy]-1-methyl-4-isopropyl-5-prop-2-enoxy-7-oxabicyclo[2.2.1]heptane (1-1602)

To a dimethylformamide solution (3 mL) of (1R,2S,4S,5R)-5-[(2-fluorophenyl)methoxy]-1-isopropyl-4-methyl-7-oxabicyclo[2.2.1]heptan-2-ol (200 mg, 0.679 mmol), sodium hydride (55% dispersion in mineral oil, 44.5 mg, 1.02 mmol) was added, followed by stirring in an ice bath (0°C) for 10 minutes. To the reaction mixture, 3-bromoprop-1-ene (107 mg, 0.883 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (109 mg, 48%).

Synthesis Example 378

Synthesis of (1S,2R,4R,5S)-5-ethoxy-2-[(2-fluorophenyl)methoxy]-1-methyl-4-isopropyl-7-oxabicyclo[2.2.1]heptane (1-183)

The same reaction and treatment as in Synthesis Example 377 were performed using iodoethane instead of 3-bromoprop-1-ene to obtain a colorless liquid title compound (yield 55%).

Synthesis Example 379

Synthesis of (1S,2R,4R,SS)-2-[(2-fluorophenyl)methoxy]-1-methyl-4-isopropyl-5-propoxy-7-oxabicyclo[2.2.1]heptane (1-1604)

The same reaction and treatment as in Synthesis Example 377 were performed using 1-bromopropane instead of 3-bromoprop-1-ene to obtain a colorless liquid title compound (yield 57%).

Synthesis Example 380

Synthesis of (1S,2R,4R,5S)-2-[(2-fluorophenyl)methoxy]-1-methyl-4-isopropyl-5-prop-2-ynoxy-7-oxabicyclo[2.2.1]heptane (1-1607)

The same reaction and treatment as in Synthesis Example 377 were performed using 3-bromoprop-1-yne instead of 3-bromoprop-1-ene to obtain a yellow liquid title compound (yield 93%).

Synthesis Example 381

Synthesis of (1S,2R,4R,5S)-5-(cyclopropylmethoxy)-2-[(2-fluorophenyl)methoxy]-1-methyl-4-isopropyl-7-oxabicyclo [2.2.1]heptane (1-1603)

The same reaction and treatment as in Synthesis Example 377 were performed using bromomethylcyclopropane instead of 3-bromoprop-1-ene to obtain a colorless liquid title compound (yield 38%).

Synthesis Example 382

Synthesis of (1S,2R,4R,5S)-2-[(2-fluorophenyl)methoxy]-1-methyl-4-isopropyl-5-(2,2,2-trifluoroethoxy)-7-oxabicyclo [2.2.1]heptane (1-1605)

The same reaction and treatment as in Synthesis Example 377 were performed using 1,1,1-trifluoro-2-iodoethane instead of 3-bromoprop-1-ene to obtain a colorless liquid title compound (yield 34%).

Synthesis Example 383

Synthesis of (1S,2R,4R,SS)-2-[(2-fluorophenyl)methoxy]-1-methyl-5-phenylmethoxy-4-isopropyl-7-oxabicyclo[2.2.1] heptane (1-1606)

The same reaction and treatment as in Synthesis Example 377 were performed using bromomethylbenzene instead of 3-bromoprop-1-ene to obtain a colorless liquid title compound (yield 69%).

Synthesis Example 384

Synthesis of [(1R,2S,4S,SR)-5-[(2-fluorophenyl)methoxy]-4-methyl-1-isopropyl-7-oxabicyclo[2.2.1]heptan-2-yl]acetate (1-480)

The same reaction and treatment as in Synthesis Example 377 were performed using acetyl chloride instead of 3-bromoprop-1-ene to obtain a colorless liquid title compound (yield 83%).

Synthesis Example 385

Synthesis of (1R,2S,4S,5R)-5-benzyloxy-1-isopropyl-2-methoxy-4-methyl-7-oxabicyclo[2.2.1]heptane (1-150)

To a dimethylformamide solution (3 mL) of (1S,2R,4R,5S)-4-isopropyl-5-methoxy-1-methyl-7-oxabicyclo[2.2.1] heptan-2-ol (200 mg, 0.999 mmol), sodium hydride (55% dispersion in mineral oil, 52.3 mg, 1.20 mmol) was added, followed by stirring in an ice bath (0°C) for 10 minutes. To the reaction mixture, benzyl bromide (188 mg, 1.10 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (140 mg, 48%).

Synthesis Example 386

Synthesis of (1R,2S,4S,SR)-1-isopropyl-2-methoxy-4-methyl-5-(3-methylbut-2-enoxy)-7-oxabicyclo[2.2.1]heptane (1-799)

The same reaction and treatment as in Synthesis Example 385 were performed using 1-bromo-3-methyl-but-2-ene instead of benzyl bromide to obtain a colorless liquid title compound (yield 75%).

Synthesis Example 387

Synthesis of (1R,2S,4S,SR)-5-but-2-ynoxy-1-isopropyl-2-methoxy-4-methyl-7-oxabicyclo[2.2.1]heptane (1-801)

The same reaction and treatment as in Synthesis Example 385 were performed using 1-bromobut-2-yne instead of benzyl bromide to obtain a colorless liquid title compound (yield 63%).

Synthesis Example 388

Synthesis of [(1R,2S,4S,5R)-5-[(2-fluorophenyl)methoxy]-1-isopropyl-4-methyl-7-oxabicyclo[2.2.1]heptan-2-yl] N-ethylcarbamate (1-1615)

The same reaction and treatment as in Synthesis Example 376 were performed using (1R,2S,4S,5R)-5-[(2-fluorophenyl)methoxy]-1-isopropyl-4-methyl-7-oxabicyclo[2.2.1]heptan-2-ol instead of (1R,2R,4S,5R)-5-[(2-fluorophenyl)methoxy]-1-isopropyl-4-methyl-7-oxabicyclo[2.2.1]heptan-2-ol to obtain a colorless liquid title compound (yield 19%).

Synthesis Example 389

Synthesis of (1S,2R,4R,5S)-2,5-bis[(2-fluorophenyl)methoxy]-1-methyl-4-isopropyl-7-oxabicyclo[2.2.1]heptane (1-603)

To a dimethylformamide solution (3 mL) of (1R,25,45,5R)-1-isopropyl-4-methyl-7-oxabicyclo[2.2.1]heptane-2,5-diol (200 mg, 1.07 mmol), sodium hydride (55% dispersion in mineral oil, 117 mg, 2.68 mmol) was added, followed by stirring in an ice bath (0°C) for 10 minutes. To the reaction mixture, 1-(bromomethyl)-2-fluoro-benzene (609 mg, 3.22 mmol) was added, followed by stirring at room temperature (25°C) for 3 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a colorless liquid title compound (357 mg, 83%).

Synthesis Example 390

Synthesis of (1S,2R,4R,5S)-1-methyl-2,5-bis[(2-methylphenyl)methoxy]-4-isopropyl-7-oxabicyclo[2.2.1]heptane (1-597)

The same reaction and treatment as in Synthesis Example 389 were performed using 1-(bromomethyl)-2-methyl-benzene instead of 1-(bromomethyl)-2-fluoro-benzene to obtain a colorless liquid title compound (yield 86%).

Synthesis Example 391

Synthesis of (1R,4S,SR)-5-[(2-fluorophenyl)methoxy]-1-isopropyl-4-methyl-7-oxabicyclo[2.2.1]hept-2-en-2-yl]trifluoromethanesulfonate (3-680)

To a tetrahydrofuran solution (56.0 mL) of (1R,4S,SR)-S-[(2-fluorophenyl)methoxy]-1-isopropyl-4-methyl-7-oxabicyclo[2.2.1]heptan-2-one (2.00 g, 6.84 mmol), lithium bis(trimethylsilyl)amide (7.90 mL, 1 mol/L tetrahydrofuran solution, 10.3 mmol) was added, followed by stirring for 3 hours. To the reaction mixture, N-phenylbis(trifluoromethanesulfonimide) (3.67 g, 10.3 mmol) was added, followed by stirring at room temperature for 3 hours. A saturated ammonium chloride aqueous solution was poured into the reaction mixture, which was then extracted with ethyl acetate. The extract was dried over anhydrous magnesium sulfate and then concentrated under reduced pressure, and the concentrate was purified by silica gel column chromatography (eluent: ethyl acetate/n-hexane) to obtain a colorless liquid title compound (1.30 g, 45%).

The $^1H$ NMR spectrum ($CDCl_3$) σ (ppm) values of the compounds according to the present invention that are produced based on the above-described Synthesis Examples and the above-described production method are shown in Tables 4, 5, and 6. $^1H$ NMR data was measured using a JNM-ECS400 spectrometer (manufactured by JEOL Ltd.) or the like.

[Table 81]

Table 4

| No. | Physical property (melting point °C) | Form | $^1H$ NMR spectrum σ ppm: |
|---|---|---|---|
| 1-2 | | Colorless liquid | 1.00 (d, J = 6.8 Hz, 3H), 1.02 (d, J = 6.8 Hz, 3H), 1.42 (s, 3H), 1.44-1.61 (m, 2H), 1.95-2.18 (m, 2H), 2.33 (s, 3H), 2.51-2.59 (m, 1H), 3.69 (dd, J = 6.8, 2.4 Hz, 1H), 4.40 (d, J = 12.0 Hz, 1H), 4.59 (d, J = 12.0 Hz, 1H), 4.83 (dddd, J = 58.4, 9.2, 2.4, 1.6 Hz, 1H), 7.13-7.23 (m, 3H), 7.31-7.35 (m, 1H). |
| 1-5 | | Colorless liquid | 1.00 (dd, J = 6.8, 0.8 Hz, 3H), 1.01 (dd, J = 6.8, 0.8 Hz, 3H), 1.43 (s, 3H), 1.44-1.62 (m, 2H), 1.97-2.15 (m, 2H), 2.52 (ddd, J = 12.8, 6.8, 3.6 Hz, 1H), 3.72 (dd, J = 6.8, 2.8 Hz, 1H), 4.53 (d, J = 12.4 Hz, 1H), 4.63 (d, J = 12.4 Hz, 1H), 4.90 (ddq, J = 58.4, 9.2, 1.2 Hz, 1H), 6.99-7.08 (m, 1H), 7.10-7.17 (m, 1H), 7.23-7.30 (m, 1H), 7.41-7.49 (m, 1H). |

(continued)

| Table 4 | | | |
|---|---|---|---|
| No. | Physical property (melting point °C) | Form | $^1$H NMR spectrum σ ppm: |
| 1-7 | | Colorless liquid | 0.99 (d, J = 6.8 Hz, 3H), 1.01 (d, J = 6.8 Hz, 3H), 1.36 (s, 3H), 1.45-1.60 (m, 2H), 1.96-2.08 (m, 1H), 2.10 (sept, J = 6.8 Hz, 1H), 2.59 (ddd, J = 13.2, 6.8, 3.6 Hz, 1H), 3.70 (dd, J = 6.8, 2.0 Hz, 1H), 4.52 (dt, J = 11.2, 1.2 Hz, 1H), 4.63 (dt, J = 11.2, 1.2 Hz, 1H), 4.81 (dddd, J = 18.4, 10.0, 2.4, 3.6 Hz, 1H), 6.84-6.92 (m, 2H), 7.22-7.32 (m, 1H). |
| 1-8 | | Colorless liquid | 1.06 (d, J = 6.9 Hz, 6H), 1.45 (s, 3H), 1.50 (dd, J = 13.5, 4.4 Hz, 1H), 1.62 (dt, J = 13.6, 2.2 Hz, 1H), 2.08-2.19 (m, 1H), 2.25 (dd, J = 13.3, 10.5 Hz, 1H), 2.77 (dd, J = 13.3, 6.9 Hz, 1H), 3.65 (dd, J = 6.9, 2.3 Hz, 1H), 4.03 (dq, J = 10.6, 2.1 Hz, 1H), 4.52 (dd, J = 60.4, 12.4 Hz, 2H), 7.30-7.34 (m, 5H). |
| 1-9 | | Colorless liquid | 1.07 (d, J = 6.9 Hz, 6H), 1.43 (s, 3H), 1.51 (dd, J = 13.3, 4.6 Hz, 1H), 1.64 (dt, J = 13.3, 2.3 Hz, 1H), 2.09-2.15 (m, 1H), 2.25 (dd, J = 13.3, 10.5 Hz, 1H), 2.33 (s, 3H), 2.78 (dd, J = 13.5, 6.6 Hz, 1H), 3.65 (dd, J = 6.9, 2.3 Hz, 1H), 4.03 (dq, J = 10.6, 2.1 Hz, 1H), 4.49 (dd, J = 71.4, 12.4 Hz, 2H), 7.15-7.23 (m, 3H), 7.33 (dd, J = 6.4, 1.8 Hz, 1H). |
| 1-10 | | Pale yellow liquid | 1.06 (d, J = 6.9 Hz, 6H), 1.38 (s, 3H), 1.52 (dd, J = 13.5, 4.4 Hz, 1H), 1.65 (dt, J = 13.3, 2.3 Hz, 1H), 2.10-2.17 (m, 1H), 2.25 (dd, J = 13.3, 10.5 Hz, 1H), 2.40 (s, 6H), 2.81 (dd, J = 13.3, 6.9 Hz, 1H), 3.60 (dd, J = 6.9, 2.3 Hz, 1H), 4.02 (dq, J = 10.6, 2.1 Hz, 1H), 4.50 (dd, J = 49.5, 11.0 Hz, 2H), 7.02 (d, J = 7.8 Hz, 2H), 7.10 (dd, J = 8.2, 6.9 Hz, 1H). |
| 1-11 | | Colorless liquid | 1.06 (d, J = 6.4 Hz, 6H), 1.46 (s, 3H), 1.52 (dd, J = 13.5, 3.9 Hz, 1H), 1.66 (d, J = 13.3 Hz, 1H), 2.13 (dd, J = 13.7, 6.9 Hz, 1H), 2.26 (dd, J = 13.1, 10.8 Hz, 1H), 2.80 (dd, J = 13.3, 6.9 Hz, 1H), 3.69 (dd, J = 6.9, 1.8 Hz, 1H), 3.83 (s, 3H), 4.03 (dq, J = 10.5, 2.0 Hz, 1H), 4.55 (dd, J = 38.5, 13.3 Hz, 2H), 6.86 (d, J = 8.2 Hz, 1H), 6.96 (t, J = 7.6 Hz, 1H), 7.25-7.26 (m, 1H), 7.42 (d, J = 7.3 Hz, 1H). |
| 1-12 | | Colorless liquid | 1.06 (d, J = 7.2 Hz, 6H), 1.44 (s, 3H), 1.52 (dd, J = 13.2, 4.8 Hz, 1H), 1.63 (dt, J = 13.2, 2.0 Hz, 1H), 2.13 (sept, J = 7.2 Hz, 1H), 2.26 (dd, J = 13.2, 10.4 Hz, 1H), 2.80 (dd, J = 13.2, 6.8 Hz, 1H), 3.68 (dd, J = 6.8, 2.0 Hz, 1H), 4.03 (ddd, J = 10.4, 4.8, 2.0 Hz, 1H), 4.52 (d, J = 12.4 Hz, 1H), 4.62 (d, J = 12.4 Hz, 1H), 7.00-7.06 (m, 1H), 7.13 (td, J = 7.2, 0.8 Hz, 1H), 7.23-7.30 (m, 1H), 7.44 (td, J = 7.2, 0.8 Hz, 1H). |

[Table 82]

| Table 4-Continued | | | |
|---|---|---|---|
| No. | Physical property (melting point °C) | Form | $^1$H NMR spectrum σ ppm: |
| 1-13 | | Colorless liquid | 1.07 (d, J = 6.9 Hz, 6H), 1.47 (s, 3H), 1.54 (dd, J = 13.5, 4.4 Hz, 1H), 1.66 (dt, J = 13.3, 2.3 Hz, 1H), 2.11-2.18 (m, 1H), 2.28 (dd, J = 13.5, 10.8 Hz, 1H), 2.83 (dd, J = 13.3, 6.9 Hz, 1H), 3.72 (dd, J = 6.9, 2.3 Hz, 1H), 4.04 (dq, J = 10.6, 2.1 Hz, 1H), 4.59 (dd, J = 44.9, 13.3 Hz, 2H), 7.22-7.27 (m, 2H), 7.34 (dd, J = 7.3, 1.4 Hz, 1H), 7.51 (d, J = 7.3 Hz, 1H). |
| 1-14 | | Colorless liquid | 1.05 (d, J = 6.9 Hz, 6H), 1.37 (s, 3H), 1.52 (dd, J = 13.5, 4.4 Hz, 1H), 1.63 (dt, J = 13.3, 2.3 Hz, 1H), 2.10-2.13 (m, 1H), 2.25 (dd, J = 13.3, 10.5 Hz, 1H), 2.81 (dd, J = 13.5, 6.6 Hz, 1H), 3.65 (dd, J = 6.9, 2.3 Hz, 1H), 4.02 (dq, J = 10.5, 2.1 Hz, 1H), 4.57 (ddt, J = 39.4, 11.0, 1.4 Hz, 2H), 6.85-6.92 (m, 2H), 7.26-7.28 (m, 1H). |

(continued)

| Table 4-Continued | | | |
|---|---|---|---|
| No. | Physical property (melting point °C) | Form | $^1$H NMR spectrum σ ppm: |
| 1-15 | | Colorless liquid | 1.07-1.08 (m, 3H), 1.09-1.10 (m, 3H), 1.46 (s, 3H), 1.62 (dd, J = 13.5, 4.8 Hz, 1H), 1.69-1.75 (m, 1H), 2.11-2.19 (m, 1H), 2.30 (dd, J = 13.5, 10.8 Hz, 1H), 2.83 (dd, J = 13.7, 6.9 Hz, 1H), 3.63 (dd, J = 6.6, 2.5 Hz, 1H), 4.03 (dq, J = 10.6, 2.3 Hz, 1H), 4.45 (d, J = 12.4 Hz, 1H), 4.60 (d, J = 12.4 Hz, 1H), 7.28-7.36 (m, 5H). |
| 1-16 | | Colorless liquid | 1.09 (dd, J = 6.9, 2.3 Hz, 6H), 1.44 (s, 3H), 1.64 (dd, J = 13.5, 4.8 Hz, 1H), 1.72 (dt, J = 13.4, 2.4 Hz, 1H), 2.13-2.17 (m, 1H), 2.30 (dd, J = 13.3, 10.1 Hz, 1H), 2.33 (s, 3H), 2.84 (dd, J = 13.3, 6.9 Hz, 1H), 3.64 (dd, J = 6.9, 2.4 Hz, 1H), 4.03 (dq, J = 10.8, 2.4 Hz, 1H), 4.40 (d, J = 12.4 Hz, 1H), 4.57 (d, J = 12.4 Hz, 1H), 7.16-7.19 (m, 3H), 7.31-7.33 (m, 1H). |
| 1-17 | | Colorless liquid | 1.08 (d, J = 6.9 Hz, 6H), 1.39 (s, 3H), 1.65 (dd, J = 13.5, 4.8 Hz, 1H), 1.71-1.74 (m, 1H), 2.14-2.15 (m, 1H), 2.29 (dd, J = 13.7, 10.5 Hz, 1H), 2.40 (s, 6H), 2.87 (dd, J = 13.3, 6.9 Hz, 1H), 3.58 (dd, J = 6.4, 2.3 Hz, 1H), 3.88-4.10 (m, 1H), 4.44 (d, J = 11.0 Hz, 1H), 4.56 (d, J = 11.0 Hz, 1H), 7.00-7.02 (m, 2H), 7.09-7.11 (m, 1H). |
| 1-18 | | Colorless liquid | 1.07 (d, J = 1.8 Hz, 3H), 1.09 (d, J = 1.8 Hz, 3H), 1.47 (s, 3H), 1.65 (dd, J = 13.5, 4.8 Hz, 1H), 1.71-1.76 (m, 1H), 2.12-2.19 (m, 1H), 2.30 (dd, J = 13.5, 10.8 Hz, 1H), 2.86 (dd, J = 13.7, 6.9 Hz, 1H), 3.68 (dd, J = 6.6, 2.5 Hz, 1H), 3.83 (s, 3H), 4.03 (dq, J = 10.6, 2.3 Hz, 1H), 4.50 (d, J = 12.8 Hz, 1H), 4.60 (d, J = 13.3 Hz, 1H), 6.86 (d, J = 8.2 Hz, 1H), 6.96 (t, J = 7.3 Hz, 1H), 7.23-7.28 (m, 1H), 7.41 (d, J = 6.9 Hz, 1H). |
| 1-19 | | Colorless liquid | 1.08 (dd, J = 7.1, 2.5 Hz, 6H), 1.45 (s, 3H), 1.65 (dd, J = 13.7, 4.6 Hz, 1H), 1.71 (dt, J = 13.4, 2.4 Hz, 1H), 2.15 (ddd, J = 23.5, 11.3, 5.8 Hz, 1H), 2.31 (dd, J = 13.5, 10.8 Hz, 1H), 2.87 (dd, J = 13.3, 6.9 Hz, 1H), 3.67 (dd, J = 6.9, 2.3 Hz, 1H), 4.03 (ddd, J = 10.6, 2.3, 1.1 Hz, 1H), 4.52 (d, J = 12.4 Hz, 1H), 4.62 (d, J = 12.8 Hz, 1H), 7.01-7.06 (m, 1H), 7.13 (td, J = 7.4, 1.1 Hz, 1H), 7.27 (tt, J = 9.6, 2.8 Hz, 1H), 7.44 (td, J = 7.3, 1.8 Hz, 1H). |
| 1-20 | | Colorless liquid | 1.09 (dd, J = 7.1, 2.5 Hz, 6H), 1.48 (s, 3H), 1.67 (dd, J = 13.7, 4.6 Hz, 1H), 1.74 (dt, J = 13.3, 2.3 Hz, 1H), 2.12-2.19 (m, 1H), 2.32 (dd, J = 13.5, 10.8 Hz, 1H), 2.90 (dd, J = 13.7, 6.9 Hz, 1H), 3.71 (dd, J = 6.9, 2.3 Hz, 1H), 4.04 (dq, J = 10.6, 2.3 Hz, 1H), 4.53 (d, J = 13.3 Hz, 1H), 4.64 (d, J = 13.3 Hz, 1H), 7.20-7.29 (m, 2H), 7.34 (dd, J = 7.6, 1.6 Hz, 1H), 7.51 (d, J = 7.3 Hz, 1H). |

[Table 83]

| Table 4-Continued | | | |
|---|---|---|---|
| No. | Physical property (melting point °C) | Form | $^1$H NMR spectrum σ ppm: |
| 1-21 | | Colorless liquid | 1.07 (d, J = 2.7 Hz, 3H), 1.08 (d, J = 2.7 Hz, 3H), 1.38 (s, 3H), 1.64 (dd, J = 13.5, 4.8 Hz, 1H), 1.71 (dt, J = 13.4, 2.4 Hz, 1H), 2.11-2.17 (m, 1H), 2.29 (dd, J = 13.5, 10.8 Hz, 1H), 2.87 (dd, J = 13.5, 6.6 Hz, 1H), 3.64 (dd, J = 6.4, 2.3 Hz, 1H), 4.02 (dq, J = 10.5, 2.3 Hz, 1H), 4.51 (d, J = 11.0 Hz, 1H), 4.61 (d, J = 11.0 Hz, 1H), 6.86-6.92 (m, 2H), 7.23-7.31 (m, 1H). |

(continued)

| Table 4-Continued | | | |
|---|---|---|---|
| No. | Physical property (melting point °C) | Form | $^1$H NMR spectrum σ ppm: |
| 1-24 | | Colorless liquid | 1.02 (d, J = 6.8 Hz, 3H), 1.03 (d, J = 6.8 Hz, 3H), 1.19 (dd, J = 13.2, 3.6 Hz, 1H), 1.39 (s, 3H), 1.46-1.55 (m, 2H), 1.99-2.14 (m, 2H), 2.32 (s, 3H), 2.64 (dd, J = 12.8, 6.8 Hz, 1H), 3.64 (dd, J = 6.8, 2.4 Hz, 1H), 4.12-4.18 (m, 1H), 4.39 (d, J = 12.4 Hz, 1H), 4.58 (d, J = 12.4 Hz, 1H), 7.12-7.22 (m, 3H), 7.30-7.35 (m, 1H). |
| 1-30 | | Colorless liquid | 1.02 (d, J = 6.8 Hz, 3H), 1.03 (d, J = 6.8 Hz, 3H), 1.21 (dd, J = 13.2, 3.6 Hz, 1H), 1.41 (s, 3H), 1.48-1.55 (m, 2H), 2.01-2.2.16 (m, 2H), 2.67 (dd, J = 12.8, 6.8 Hz, 1H), 3.69 (dd, J = 6.8, 2.0 Hz, 1H), 4.13-4.20 (m, 1H), 4.53 (d, J = 12.8 Hz, 1H), 4.64 (d, J = 12.8 Hz, 1H), 6.99-7.05 (m, 1H), 7.13 (td, J = 7.2, 0.8 Hz, 1H), 7.22-7.30 (m, 1H), 7.55 (td, J = 7.2, 2.0 Hz, 1H). |
| 1-41 | | Colorless liquid | 1.02 (d, J = 6.8 Hz, 3H), 1.02 (d, J = 6.8 Hz, 3H), 1.21 (dd, J = 3.6, 13.2 Hz, 1H), 1.34 (s, 3H), 1.49-1.53 (m, 2H), 2.03 (dd, J = 13.2, 10.0 Hz, 1H), 2.10 (sept, J = 6.8 Hz, 1H), 2.67 (dd, J = 13.2, 6.8 Hz, 1H), 3.66 (dd, J = 6.8, 2.0 Hz, 1H), 4.13-4.17 (m, 1H), 4.52 (dt, J = 11.2, 1.2 Hz, 1H), 4.54 (dt, J = 11.2, 1.2 Hz, 1H), 6.83-6.92 (m, 2H), 7.21-7.31 (m, 1H). |
| 1-68 | 91.0-95.0 | White solid | 0.94 (d, J = 6.9 Hz, 3H), 1.06 (d, J = 6.9 Hz, 3H), 1.34 (dd, J = 14.2, 1.8 Hz, 1H), 1.49 (s, 3H), 1.53-1.59 (m, 2H), 1.69 (dd, J = 13.5, 2.1 Hz, 1H), 2.00 (dd, J = 13.7, 6.9 Hz, 1H), 2.35-2.42 (m, 1H), 3.44 (dd, J = 6.4, 1.8 Hz, 1H), 3.82-3.84 (m, 1H), 4.50 (d, J = 12.8 Hz, 1H), 4.60 (d, J = 12.8 Hz, 1H), 7.03 (t, J = 9.2 Hz, 1H), 7.13 (t, J = 7.3 Hz, 1H), 7.24-7.25 (m, 1H), 7.43 (t, J = 7.6 Hz, 1H). |
| 1-83 | | Colorless liquid | 0.85-1.06 (m, 9H), 1.41 (s, 2.16H), 1.44 (s, 0.84H), 1.49-1.91 (m, 3H), 2.01-2.21 (m, 2H), 2.25-2.32 (m, 1H), 2.32 (s, 3H), 3.47 (dd, J = 6.8, 2.4 Hz, 0.28H), 3.50 (dd, J = 6.8, 2.4 Hz, 0.72H), 4.37 (d, J = 12.4 Hz, 0.28H), 4.39 (d, J = 12.4 Hz, 0.72H), 4.55 (d, J = 12.4 Hz, 1H), 7.13-7.22 (m, 3H), 7.31-7.36 (m, 1H). |
| 1-92 | | Colorless liquid | 0.82-1.05 (m, 9H), 1.41 (s, 2.01H), 1.44 (s, 0.99H), 1.47-1.53 (m, 0.67H), 1.63-1.91 (m, 2.66H), 1.99-2.20 (m, 1.67H), 2.25-2.32 (dd, J = 13.2, 6.4 Hz, 1H), 3.49 (dd, J = 6.8, 2.4 Hz, 0.33H), 3.52 (dd, J = 6.8, 2.4 Hz, 0.67H), 4.50 (d, J = 12.8 Hz, 1H), 4.59 (d, J = 12.8 Hz, 0.33H), 4.60 (d, J = 12.8 Hz, 0.67H), 7.00 (t, J = 8.8 Hz, 1H), 7.08-7.15 (m, 1H), 7.20-7.28 (m, 1H), 7.41-7.48 (m, 1H). |
| 1-93 | | Colorless liquid | 0.86 (d, J = 6.9 Hz, 3H), 0.89 (d, J = 6.4 Hz, 3H), 1.45 (s, 3H), 1.45 (dd, J = 10.0, 5.0 Hz, 1H), 1.45 (s, 3H), 1.66-1.88 (m, 4H), 2.12-2.22 (m, 1H), 3.51 (dd, J = 6.0, 3.2 Hz, 1H), 4.51 (d, J = 12.8 Hz, 1H), 4.60 (d, J = 12.8 Hz, 1H), 6.99-7.04 (m, 1H), 7.13 (td, J = 7.6, 1.2 Hz, 1H), 7.23-7.27 (m, 1H), 7.46 (td, J = 7.6, 1.7 Hz, 1H). |

[Table 84]

| Table 4-Continued | | | |
|---|---|---|---|
| No. | Physical property (melting point °C) | Form | $^1$H NMR spectrum σ ppm: |
| 1-94 | | Colorless liquid | 0.91 (dd, J = 12.4, 5.5 Hz, 1H), 0.97 (d, J = 6.9 Hz, 3H), 1.00 (d, J = 6.9 Hz, 3H), 1.03 (d, J = 6.9 Hz, 3H), 1.42 (s, 3H), 1.51 (dt, J = 13.3, 2.3 Hz, 1H), 1.88 (t, J = 11.7 Hz, 1H), 2.03-2.18 (m, 2H), 2.32 (dd, J = 13.3, 6.9 Hz, 1H), 3.54 (dd, J = 6.9, 2.7 Hz, 1H), 4.51 (d, J = 12.8 Hz, 1H), 4.61 (d, J = 12.8 Hz, 1H), 6.99-7.04 (m, 1H), 7.12 (td, J = 7.4, 1.1 Hz, 1H), 7.22-7.28 (m, 1H), 7.46 (td, J = 7.4, 1.7 Hz, 1H). |

(continued)

| Table 4-Continued | | | |
|---|---|---|---|
| No. | Physical property (melting point °C) | Form | $^1$H NMR spectrum σ ppm: |
| 1-111 | | Colorless liquid | 0.84-0.94 (m, 3.06H), 0.95-1.03 (m, 8.94H), 1.34 (s, 1.98H), 1.36 (s, 1.02H), 1.46-1.52 (m, 0.66H), 1.63-1.89 (m, 2H), 1.98-2.20 (m, 1.68H), 2.31 (dd, J = 13.2, 6.8 Hz, 0.66H), 3.47 (dd, J = 6.4, 2.8 Hz, 0.34H), 3.50 (dd, J = 6.4, 2.8 Hz, 0.66H), 4.49 (d, J = 10.8 Hz, 1H), 4.58 (d, J = 10.8 Hz, 1H), 6.82-6.91 (m, 2H), 7.20-7.30 (m, 1H). |
| 1-123 | | Colorless liquid | 0.77-1.06 (m, 11H), 1.42 (s, 2.1H), 1.45 (s, 0.9H), 1.51-1.71 (m, 3H), 1.80-1.92 (m, 1.3H), 2.05 (sept, J = 6.8 Hz, 0.7H), 2.16-2.26 (m, 1H), 2.32 (s, 2.1H), 2.33 (s, 0.9H), 3.48 (dd, J = 6.4, 2.8 Hz, 0.3H), 3.49 (dd, J = 6.4, 2.8 Hz, 0.7H), 4.37 (d, J = 12.4 Hz, 1H), 4.54 (d, J = 12.4 Hz, 0.7H), 4.55 (d, J = 12.4 Hz, 0.3H), 7.13-7.21 (m, 3H), 7.31-7.36 (m, 1H). |
| 1-126 | | Colorless liquid | 0.80-1.06 (m, 11H), 1.43 (s, 2.1H), 1.46 (s, 0.9H), 1.50-1.70 (m, 3H), 1.81-1.93 (m, 1.3H), 2.05 (sept, J = 6.8 Hz, 0.7H), 2.16-2.28 (m, 1H), 3.51-3.54 (m, 1H), 4.50 (d, J = 12.8 Hz, 0.7H), 4.51 (d, J = 12.8 Hz, 0.3H), 4.55 (d, J = 12.8 Hz, 1H), 6.99-7.04 (m, 1H), 7.10-7.15 (m, 1H), 7.22-7.28 (m, 1H), 7.44-7.48 (m, 1H). |
| 1-133 | | Colorless liquid | 0.89 (d, J = 6.8 Hz, 3H), 0.91 (d, J = 6.8 Hz, 3H), 1.00-1.05 (m, 1H), 1.05 (d, J = 7.2 Hz, 3H), 1.07 (d, J = 7.2 Hz, 3H), 1.33 (sept, J = 7.2 Hz, 1H), 1.40 (s, 3H), 1.69-1.84 (m, 3H), 1.99 (sept, J = 6.8 Hz, 1H), 2.12 (dd, J = 13.2, 6.8 Hz, 1H), 3.51 (dd, J = 6.8, 2.8 Hz, 1H), 4.49 (d, J = 13.2 Hz, 1H), 4.58 (d, J = 13.2 Hz, 1H), 7.00 (t, J = 7.2 Hz, 1H), 7.11 (t, J = 7.2 Hz, 1H), 7.20-7.28 (m, 1H), 7.45 (dt, J = 7.2, 1.6 Hz, 1H). |
| 1-150 | | Colorless liquid | 0.99 (dd, J = 6.9, 2.3 Hz, 6H), 1.26 (dd, J = 12.8, 3.0 Hz, 1H), 1.42 (s, 3H), 1.47 (dt, J = 12.8, 2.3 Hz, 1H), 1.85 (dd, J = 12.8, 9.2 Hz, 1H), 2.03-2.10 (m, 1H), 2.54 (dd, J = 12.8, 6.9 Hz, 1H), 3.24 (s, 3H), 3.56-3.61 (m, 2H), 4.49 (dd, J = 65.9, 12.8 Hz, 2H), 7.27-7.30 (m, 5H). |
| 1-151 | | Colorless liquid | 0.99 (d, J = 6.8 Hz, 6H), 1.28 (dd, J = 12.8, 3.2 Hz, 1H), 1.37 (s, 3H), 1.45-1.53 (m, 1H), 1.86 (dd, J = 12.8, 9.6 Hz, 1H), 2.08 (sept, J = 6.8, Hz, 1H), 2.32 (s, 3H), 2.56 (dd, J = 12.8, 6.8 Hz, 1H), 3.26 (s, 3H), 3.57-3.62 (m, 2H), 4.37 (d, J = 12.4 Hz, 1H), 4.57 (d, J = 12.4 Hz, 1H)7.12-7.22 (m, 3H), 7.31-7.36 (m, 1H). |
| 1-156 | | Colorless liquid | 0.99 (d, J = 6.8 Hz, 6H), 1.30 (dd, J = 12.8, 3.2 Hz, 1H), 1.43 (s, 3H), 1.46-1.52 (m, 1H), 1.88 (dd, J = 12.8, 9.2 Hz, 1H), 2.08 (sept, J = 6.8, Hz, 1H), 2.60 (dd, J = 12.8, 6.8 Hz, 1H), 3.27 (s, 3H), 3.60 (ddd, J = 9.2, 3.2, 2.0 Hz, 1H), 3.65 (dd, J = 7.2, 2.4 Hz, 1H), 4.51 (d, J = 12.8 Hz, 1H), 4.62 (d, J = 12.8 Hz, 1H), 6.99-7.05 (m, 1H), 7.13 (td, J = 7.2, 1.6 Hz, 1H), 7.22-7.29 (m, 1H), 7.46 (td, J = 7.2, 1.6 Hz, 1H). |

[Table 85]

| Table 4-Continued | | | |
|---|---|---|---|
| No. | Physical property (melting point °C) | Form | $^1$H NMR spectrum σ ppm: |
| 1-173 | | Colorless liquid | 0.90 (d, J = 6.9 Hz, 3H), 0.98 (dd, J = 23.4, 6.9 Hz, 1H), 1.04 (d, J = 6.9 Hz, 3H), 1.47 (dd, J = 13.3, 2.3 Hz, 1H), 1.51 (s, 3H), 1.66 (dd, J = 13.5, 2.5 Hz, 1H), 1.77 (dd, J = 13.3, 6.4 Hz, 1H), 2.44-2.50 (m, 1H), 3.21 (s, 3H), 3.41 (dd, J = 6.4, 2.3 Hz, 1H), 3.45 (dd, J = 6.4, 2.3 Hz, 1H), 4.50 (d, J = 12.8 Hz, 1H), 4.59 (d, J = 12.8 Hz, 1H), 6.99-7.04 (m, 1H), 7.13 (td, J = 7.6, 1.1 Hz, 1H), 7.26 (tt, J = 9.4, 3.0 Hz, 1H), 7.43-7.45 (m, 1H). |
| 1-183 | | Colorless liquid | 0.99 (d, J = 6.9 Hz, 6H), 1.13 (t, J = 7.1 Hz, 3H), 1.25 (dd, J = 12.6, 3.2 Hz, 1H), 1.41 (s, 3H), 1.47 (dt, J = 12.8, 1.9 Hz, 1H), 1.86 (dd, J = 12.6, 9.4 Hz, 1H), 2.05-2.08 (m, 1H), 2.62 (dd, J = 12.8, 6.9 Hz, 1H), 3.32-3.46 (m, 2H), 3.63-3.68 (m, 2H), 4.55 (dd, J = 43.5, 12.8 Hz, 2H), 6.98-7.03 (m, 1H), 7.11 (td, J = 7.6, 1.1 Hz, 1H), 7.21-7.25 (m, 1H), 7.45 (td, J = 7.6, 1.9 Hz, 1H). |
| 1-208 | | Colorless liquid | 0.98-1.06 (m, 6H), 1.15-1.22 (m, 3H), 1.24-1.29 (m, 4H), 1.32-1.38 (m, 1H), 1.40 (s, 1.5H), 1.41 (s, 1.5H), 1.84-1.99 (m, 1H), 2.01-2.13 (m, 1H), 2.32 (s, 3H), 2.57-2.66 (m, 1H), 3.38-3.53 (m, 1H), 3.54-3.67 (m, 2H), 3.81-3.88 (m, 0.5H), 4.00-4.06 (m, 0.5H), 4.39 (d, 12.4 Hz, 1H), 4.57 (d, 12.4 Hz, 1H), 4.59-4.68 (m, 1H), 7.13-7.22 (m, 3H), 7.32-7.36 (m, 1H). |
| 1-213 | | Colorless liquid | 0.97-1.05 (m, 6H), 1.17 (t, J = 7.2, 1.5H), 1.19 (t, J = 7.2 Hz, 1.5H), 1.23-1.28 (m, 3H), 1.33-1.38 (m, 1H), 1.408 (s, 1.5H), 1.413 (s, 1.5H), 1.47-1.54 (m, 1H), 1.85-1.99 (m, 1H), 2.01-2.13 (m, 1H), 2.63 (dd, J = 12.8, 6.8, 1H), 3.38-3.52 (m, 1H), 3.54-3.68 (m, 2H), 3.80-3.87 (m, 0.5H), 4.00-4.06 (m, 0.5H), 4.35-4.67 (m, 3H), 6.97-7.49 (m, 4H). |
| 1-223 | | Colorless liquid | 0.98 (d, J = 6.8 Hz, 1.5H), 0.99 (d, J = 6.8 Hz, 1.5H), 1.01 (d, J = 6.8 Hz, 1.5H), 1.02 (d, J = 6.8 Hz, 1.5H), 1.19 (t, J = 6.8 Hz, 1.5H), 1.20 (t, J = 6.8 Hz, 1.5H), 1.25-1.39 (m, 1H), 1.27 (d, J = 6.8 Hz, 1.5H), 1.28 (d, J = 6.8 Hz, 1.5H), 1.34 (s, 1.5H), 1.35 (s, 1.5H), 1.46-1.55 (m, 1H), 1.88 (ddd, J = 12.8, 9.6 Hz, 0.5H), 1.94 (ddd, J = 12.8, 9.6 Hz, 0.5H), 2.02-2.12 (m, 1H), 2.64 (ddd, J = 12.8, 6.8, 1.6 Hz, 1H), 3.40-3.53 (m, 1H), 3.56-3.69 (m, 2H), 3.93 (dddd, J = 74.0, 9.6, 6.8, 1.6 Hz, 1H), 4.48-4.65 (m, 3H), 6.83-6.91 (m, 2H), 7.20-7.30 (m, 1H). |
| 1-313 | | Colorless liquid | 0.90 (d, J = 7.2 Hz, 3H), 0.95 (d, J = 7.2 Hz, 3H), 1.51-1.60 (m, 1H), 1.53 (s, 3H), 1.63 (dd, J = 12.8, 6.0 Hz, 1H), 2.05-2.19 (m, 2H), 2.34 (s, 3H), 2.39 (dd, J = 12.8, 6.0 Hz, 1H), 3.35-3.43 (m, 1H), 3.78 (d, J = 6.0 Hz, 1H), 4.44 (d, J = 12.8, 1H), 4.60 (d, J = 12.8, 1H), 7.11-7.33 (m, 8H), 7.36-7.41 (m, 1H). |
| 1-322 | | Colorless liquid | 0.89 (d, J = 6.8 Hz, 3H), 0.95 (d, J = 6.8 Hz, 3H), 1.51-1.58 (m, 1H), 1.53 (s, 3H), 1.64 (dd, J = 13.2, 6.4 Hz, 1H), 2.04-2.19 (m, 2H), 2.42 (dd, J = 13.2, 6.8 Hz, 1H), 3.39 (ddd, J = 11.2, 6.4, 1.6 Hz, 1H), 3.82 (dd, J = 6.8, 3.6 Hz, 1H), 4.57 (d, J = 12.4, 1H), 4.66 (d, J = 12.4, 1H), 6.89-7.35 (m, 8H), 7.46-7.55 (m, 1H). |
| 1-480 | | Colorless liquid | 0.96 (t, J = 6.4 Hz, 6H), 1.20 (dd, J = 13.7, 3.4 Hz, 1H), 1.42 (s, 3H), 1.56-1.60 (m, 1H), 2.02 (s, 3H), 2.07-2.11 (m, 1H), 2.17 (dd, J = 13.7, 10.1 Hz, 1H), 2.62 (dd, J = 13.3, 6.9 Hz, 1H), 3.64 (dd, J = 6.9, 2.7 Hz, 1H), 4.56 (dd, J = 40.5, 12.6 Hz, 2H), 4.88 (dq, J = 10.1, 1.7 Hz, 1H), 7.01 (td, J = 10.1, 8.4 Hz, 1H), 7.12 (td, J = 7.4, 1.7 Hz, 1H), 7.22-7.28 (m, 1H), 7.44 (td, J = 7.4, 1.7 Hz, 1H). |

[Table 86]

| Table 4-Continued | | | |
|---|---|---|---|
| No. | Physical property (melting point °C) | Form | $^1$H NMR spectrum σ ppm: |
| 1-525 | | Colorless liquid | 1.04 (d, J = 6.8 Hz, 3H), 1.05 (dJ = 6.8 Hz, 3H), 1.45 (s, 3H), 1.63 (dd, J = 13.6, 2.4 Hz, 1H), 1.70 (dt, J = 13.6, 1.6 Hz, 1H), 2.16 (sept, J = 6.8 Hz, 1H), 2.24 (dd, J = 13.6, 10.0 Hz, 1H), 2.59 (dd, J = 13.6, 6.8 Hz, 1H), 3.72 (dd, J = 6.8, 2.4 Hz, 1H), 4.53 (d, J = 12.0 Hz, 1H), 4.62 (d, J = 12.0 Hz, 1H), 5.01-5.08 (m, 1H), 6.99-7.08 (m, 1H), 7.14 (td, J = 7.2, 0.8 Hz, 1H), 7.25-7.32 (m, 1H), 7.42 (td, J = 7.2, 1.2 Hz, 1H). |
| 1-597 | | Colorless liquid | 1.00 (dd, J = 7.1, 5.3 Hz, 6H), 1.34 (dd, J = 12.6, 3.0 Hz, 1H), 1.42 (s, 3H), 1.50-1.55 (m, 1H), 1.88 (dd, J = 12.8, 9.2 Hz, 1H), 2.06-2.12 (m, 1H), 2.29 (s, 3H), 2.32 (s, 3H), 2.69 (dd, J = 12.8, 6.9 Hz, 1H), 3.63 (dd, J = 6.9, 2.7 Hz, 1H), 3.84 (dq, J = 9.3, 1.5 Hz, 1H), 4.37 (dd, J = 12.1, 3.0 Hz, 2H), 4.46 (d, J = 11.9 Hz, 1H), 4.56 (d, J = 12.4 Hz, 1H), 7.13-7.23 (m, 6H), 7.29-7.34 (m, 2H). |
| 1-603 | | Colorless liquid | 0.98-1.00 (m, 6H), 1.36 (dd, J = 12.4, 3.2 Hz, 1H), 1.43 (s, 3H), 1.52 (d, J = 12.8 Hz, 1H), 1.91 (dd, J = 12.6, 9.4 Hz, 1H), 2.03-2.13 (m, 1H), 2.70 (dd, J = 12.8, 6.9 Hz, 1H), 3.67 (dd, J = 6.9, 2.3 Hz, 1H), 3.84 (dd, J = 9.4, 2.1 Hz, 1H), 4.51-4.59 (m, 4H), 6.99-7.07 (m, 2H), 7.10-7.16 (m, 2H), 7.21-7.30 (m, 2H), 7.36-7.37 (m, 1H), 7.44-7.46 (m, 1H). |
| 1-612 | | Colorless liquid | 1.05 (d, J = 6.9 Hz, 6H), 1.40 (s, 3H), 1.52-1.55 (m, 2H), 1.66 (s, 3H), 1.74 (s, 3H), 2.09-2.16 (m, 1H), 2.25 (dd, J = 13.5, 10.8 Hz, 1H), 2.75 (dd, J = 13.5, 6.6 Hz, 1H), 3.58 (dd, J = 6.9, 2.7 Hz, 1H), 3.90 (dd, J = 11.4, 6.9 Hz, 1H), 4.00-4.02 (m, 2H), 5.32 (tt, J = 6.6, 1.4 Hz, 1H). |
| 1-614 | | Pale yellow liquid | 1.05 (d, J = 6.9 Hz, 6H), 1.40 (s, 3H), 1.56 (dd, J = 12.8, 3.7 Hz, 2H), 1.86 (t, J = 2.3 Hz, 3H), 2.06-2.17 (m, 1H), 2.27 (dd, J = 13.3, 10.5 Hz, 1H), 2.76 (dd, J = 13.3, 6.9 Hz, 1H), 3.80 (dd, J = 6.9, 2.3 Hz, 1H), 4.01-4.17 (m, 3H). |
| 1-627 | | Colorless liquid | 1.07 (d, J = 6.9 Hz, 6H), 1.46 (s, 3H), 1.53 (dd, J = 13.5, 4.4 Hz, 1H), 1.64 (dt, J = 13.7, 2.3 Hz, 1H), 2.09-2.19 (m, 1H), 2.28 (dd, J = 13.5, 10.8 Hz, 1H), 2.82 (dd, J = 13.5, 6.6 Hz, 1H), 3.69 (dd, J = 6.6, 2.5 Hz, 1H), 4.04 (dq, J = 10.6, 2.1 Hz, 1H), 4.57 (dd, J = 44.2, 13.1 Hz, 2H), 7.22-7.24 (m, 1H), 7.27-7.34 (m, 2H), 7.56 (dd, J = 6.9, 2.3 Hz, 1H). |
| 1-640 | | Colorless liquid | 1.07 (d, J = 6.9 Hz, 6H), 1.48 (s, 3H), 1.54 (dd, J = 13.5, 4.4 Hz, 1H), 1.65 (dt, J = 13.3, 2.3 Hz, 1H), 2.09-2.20 (m, 1H), 2.28 (dd, J = 13.5, 10.8 Hz, 1H), 2.83 (dd, J = 13.3, 6.9 Hz, 1H), 3.70 (dd, J = 6.9, 2.7 Hz, 1H), 4.05 (dq, J = 10.8, 2.1 Hz, 1H), 4.68 (dd, J = 42.6, 13.3 Hz, 2H), 7.37 (t, J = 7.6 Hz, 1H), 7.55 (t, J = 7.6 Hz, 1H), 7.63 (d, J = 7.8 Hz, 1H), 7.73 (d, J = 7.8 Hz, 1H). |
| 1-652 | | Colorless liquid | 1.06 (d, J = 6.9 Hz, 6H), 1.40 (s, 3H), 1.55 (dd, J = 13.5, 4.4 Hz, 1H), 1.71 (dt, J = 13.6, 2.1 Hz, 1H), 2.11-2.14 (m, 1H), 2.26 (dd, J = 13.3, 10.5 Hz, 1H), 2.82 (dd, J = 13.5, 6.6 Hz, 1H), 3.65 (dd, J = 6.9, 2.3 Hz, 1H), 4.02 (dq, J = 10.6, 2.1 Hz, 1H), 4.73 (dd, J = 39.6, 10.3 Hz, 2H), 7.18 (dd, J = 8.5, 7.6 Hz, 1H), 7.31 (d, J = 8.2 Hz, 2H). |

[Table 87]

| Table 4-Continued | | | |
|---|---|---|---|
| No. | Physical property (melting point °C) | Form | $^1$H NMR spectrum σ ppm: |
| 1-653 | | Colorless liquid | 1.06 (d, J = 6.9 Hz, 6H), 1.38 (s, 3H), 1.52 (dd, J = 13.5, 4.4 Hz, 1H), 1.67 (dt, J = 13.7, 2.3 Hz, 1H), 2.10-2.14 (m, 1H), 2.25 (dd, J = 13.5, 10.8 Hz, 1H), 2.81 (dd, J = 13.5, 6.6 Hz, 1H), 3.65 (dd, J = 6.6, 2.5 Hz, 1H), 4.02 (dq, J = 10.6, 2.1 Hz, 1H), 4.63 (ddd, J = 41.7, 10.8, 2.1 Hz, 2H), 6.97-7.02 (m, 1H), 7.20-7.23 (m, 2H). |
| 1-658 | | Pale yellow liquid | 1.07 (d, J = 6.9 Hz, 6H), 1.49 (s, 3H), 1.54 (dd, J = 13.5, 4.4 Hz, 1H), 1.65 (dt, J = 13.6, 2.2 Hz, 1H), 2.09-2.16 (m, 1H), 2.28 (dd, J = 13.5, 10.8 Hz, 1H), 2.83 (dd, J = 13.3, 6.9 Hz, 1H), 3.74 (dd, J = 6.9, 2.7 Hz, 1H), 4.04 (dq, J = 10.6, 2.1 Hz, 1H), 4.64 (dd, J = 40.5, 13.5 Hz, 2H), 7.19 (dd, J = 7.1, 5.3 Hz, 1H), 7.49 (d, J = 7.8 Hz, 1H), 7.70 (td, J = 7.7, 1.7 Hz, 1H), 8.53 (d, J = 4.6 Hz, 1H). |
| 1-661 | | Yellow liquid | 1.05 (d, J = 0.5 Hz, 3H), 1.07 (d, J = 0.5 Hz, 3H), 1.41 (s, 3H), 1.49 (dd, J = 13.5, 4.4 Hz, 1H), 1.60 (dt, J = 13.5, 2.1 Hz, 1H), 2.10-2.17 (m, 1H), 2.25 (dd, J = 13.5, 10.5 Hz, 1H), 2.75 (dd, J = 13.5, 6.6 Hz, 1H), 3.68 (dd, J = 6.6, 2.1 Hz, 1H), 4.02 (dq, J = 10.8, 2.1 Hz, 1H), 4.62 (d, J = 12.8 Hz, 1H), 4.74 (d, J = 12.8 Hz, 1H), 6.97-6.99 (m, 2H), 7.28 (dd, J = 4.6, 1.8 Hz, 1H). |
| 1-662 | | Colorless liquid | 1.05 (d, J = 0.5 Hz, 3H), 1.07 (d, J = 0.5 Hz, 3H), 1.42 (s, 3H), 1.50 (dd, J = 13.5, 4.4 Hz, 1H), 1.59 (dt, J = 13.5, 2.7 Hz, 1H), 2.11-2.16 (m, 1H), 2.25 (dd, J = 13.5, 10.6 Hz, 1H), 2.75 (dd, J = 13.5, 6.6 Hz, 1H), 3.64 (dd, J = 6.6, 2.7 Hz, 1H), 4.02 (dq, J = 10.6, 2.7 Hz, 1H), 4.46 (d, J = 12.4 Hz, 1H), 4.58 (d, J = 12.4 Hz, 1H), 7.06 (dd, J = 2.5, 1.4 Hz, 1H), 7.19 (dd, J = 1.4, 0.7 Hz, 1H), 7.30 (dd, J = 2.5, 1.4 Hz, 1H). |
| 1-663 | | Colorless liquid | 1.06 (d, J = 6.9 Hz, 6H), 1.37 (s, 3H), 1.51-1.56 (m, 2H), 2.10-2.16 (m, 1H), 2.27 (s, 3H), 2.27 (t, J = 12.1 Hz, 1H), 2.38 (s, 3H), 2.78 (dd, J = 13.3, 6.9 Hz, 1H), 3.56 (dd, J = 6.9, 2.3 Hz, 1H), 4.03 (dq, J = 10.6, 2.1 Hz, 1H), 4.24 (dd, J = 64.8, 12.1 Hz, 2H). |
| 1-668 | | Colorless liquid | 1.07 (d, J = 1.4 Hz, 3H), 1.09 (d, J = 1.8 Hz, 3H), 1.40 (s, 3H), 1.58-1.68 (m, 2H), 2.10-2.17 (m, 1H), 2.30 (dd, J = 13.5, 10.8 Hz, 1H), 2.81 (dd, J = 13.3, 6.9 Hz, 1H), 3.30 (s, 3H), 3.46 (dd, J = 6.9, 2.7 Hz, 1H), 4.02 (dq, J = 10.9, 2.3 Hz, 1H). |
| 1-669 | | Colorless liquid | 0.87-0.90 (m, 3H), 1.04-1.10 (m, 6H), 1.26-1.38 (m, 6H), 1.40 (s, 3H), 1.52-1.67 (m, 4H), 2.10-2.18 (m, 1H), 2.30 (dd, J = 13.5, 10.8 Hz, 1H), 2.82 (dd, J = 13.5, 6.6 Hz, 1H), 3.27-3.34 (m, 1H), 3.42-3.49 (m, 1H), 3.51 (dd, J = 6.6, 2.5 Hz, 1H), 4.02 (dq, J = 10.5, 2.3 Hz, 1H). |
| 1-670 | | Colorless liquid | 1.10-1.11 (m, 7H), 1.51 (s, 3H), 1.69 (dd, J = 13.7, 4.6 Hz, 1H), 1.91 (d, J = 14.2 Hz, 1H), 2.11-2.20 (m, 1H), 2.37 (dd, J = 13.7, 10.5 Hz, 1H), 2.91 (dd, J = 14.2, 6.9 Hz, 1H), 4.04 (dq, J = 10.8, 2.3 Hz, 1H), 4.40 (dd, J = 6.9, 2.3 Hz, 1H), 4.74 (d, J = 4.1 Hz, 1H). |
| 1-671 | | Colorless liquid | 1.07 (d, J = 2.3 Hz, 3H), 1.09 (d, J = 2.3 Hz, 3H), 1.42 (s, 3H), 1.61-1.67 (m, 2H), 2.11-2.18 (m, 1H), 2.27-2.36 (m, 1H), 2.83 (dd, J = 13.3, 6.9 Hz, 1H), 3.60 (dd, J = 6.9, 2.7 Hz, 1H), 3.89-3.94 (m, 1H), 4.00-4.06 (m, 2H), 5.15-5.19 (m, 1H), 5.28 (dq, J = 17.3, 1.7 Hz, 1H), 5.89 (yt, J = 13.7, 4.7 Hz, 1H). |

[Table 88]

| Table 4-Continued | | | |
|---|---|---|---|
| No. | Physical property (melting point °C) | Form | $^1$H NMR spectrum σ ppm: |
| 1-672 | | Colorless liquid | 1.06 (d, J = 2.3 Hz, 3H), 1.08 (d, J = 2.3 Hz, 3H), 1.41 (s, 3H), 1.60-1.65 (m, 2H), 1.66 (s, 3H), 1.74 (s, 3H), 2.10-2.17 (m, 1H), 2.30 (dd, J = 13.5, 10.8 Hz, 1H), 2.82 (dd, J = 13.3, 6.9 Hz, 1H), 3.57 (dd, J = 6.6, 2.5 Hz, 1H), 3.88-3.93 (m, 1H), 3.97-4.04 (m, 2H), 5.30-5.34 (m, 1H). |
| 1-673 | | Colorless liquid | 1.07 (s, 3H), 1.08 (s, 3H), 1.41 (s, 3H), 1.63-1.71 (m, 2H), 2.10-2.16 (m, 1H), 2.32 (dd, J = 13.5, 10.8 Hz, 1H), 2.41 (t, J = 2.3 Hz, 1H), 2.85 (dd, J = 13.3, 6.9 Hz, 1H), 3.82 (dd, J = 6.9, 2.3 Hz, 1H), 4.03 (dq, J = 10.9, 2.3 Hz, 1H), 4.15 (ddd, J = 37.1, 16.0, 2.3 Hz, 2H). |
| 1-674 | | Colorless liquid | 1.06 (s, 3H), 1.08 (s, 3H), 1.41 (s, 3H), 1.61-1.72 (m, 2H), 1.86 (t, J = 2.3 Hz, 3H), 2.10-2.17 (m, 1H), 2.31 (dd, J = 13.7, 11.0 Hz, 1H), 2.82 (dd, J = 13.7, 6.9 Hz, 1H), 3.78 (dd, J = 6.9, 2.3 Hz, 1H), 4.00-4.19 (m, 3H). |
| 1-676 | | Colorless liquid | 0.87-0.95 (m, 2H), 1.06 (d, J = 3.2 Hz, 3H), 1.08 (d, J = 3.2 Hz, 3H), 1.13-1.26 (m, 4H), 1.40 (s, 3H), 1.56-1.57 (m, 1H), 1.60 (t, J = 2.5 Hz, 1H), 1.62 (d, J = 4.6 Hz, 1H), 1.65 (d, J = 4.6 Hz, 1H), 1.68-1.76 (m, 3H), 2.10-2.17 (m, 1H), 2.29 (dd, J = 13.5, 10.8 Hz, 1H), 2.81 (dd, J = 13.3, 6.9 Hz, 1H), 3.10 (dd, J = 8.9, 6.6 Hz, 1H), 3.25 (dd, J = 9.2, 6.4 Hz, 1H), 3.49 (dd, J = 6.6, 2.5 Hz, 1H), 4.02 (dq, J = 11.0, 2.3 Hz, 1H). |
| 1-677 | | Colorless liquid | 1.07 (s, 3H), 1.09 (s, 3H), 1.42 (s, 3H), 1.63-1.69 (m, 2H), 2.14 (t, J = 6.9 Hz, 1H), 2.32 (dd, J = 13.7, 10.5 Hz, 1H), 2.90 (dd, J = 13.3, 6.9 Hz, 1H), 3.37 (s, 3H), 3.87 (dd, J = 6.9, 2.3 Hz, 1H), 4.02 (dq, J = 10.8, 2.3 Hz, 1H), 4.61 (d, J = 6.4 Hz, 1H), 4.68 (d, J = 6.9 Hz, 1H). |
| 1-678 | 70.0-75.0 | White solid | 1.08 (d, J = 0.9 Hz, 3H), 1.10 (d, J = 1.4 Hz, 3H), 1.42 (s, 3H), 1.71 (dt, J = 14.0, 2.4 Hz, 1H), 1.87 (dd, J = 13.7, 5.0 Hz, 1H), 2.13-2.20 (m, 1H), 2.35-2.41 (m, 1H), 3.13 (dd, J = 14.0, 7.1 Hz, 1H), 4.09 (dq, J = 10.8, 2.4 Hz, 1H), 5.26 (dd, J = 6.9, 2.3 Hz, 1H), 6.78 (d, J = 8.2 Hz, 1H), 6.84-6.88 (m, 1H), 7.54-7.59 (m, 1H), 8.12 (dd, J = 5.0, 1.4 Hz, 1H). |
| 1-679 | 85.1-87.8 | Yellow solid | 1.08 (s, 3H), 1.10 (s, 3H), 1.43 (s, 3H), 1.69 (dt, J = 14.0, 2.5 Hz, 1H), 1.87 (dd, J = 13.5, 4.8 Hz, 1H), 2.12-2.18 (m, 1H), 2.20 (s, 3H), 2.38 (dd, J = 13.5, 10.8 Hz, 1H), 3.14 (dd, J = 13.7, 6.9 Hz, 1H), 4.09 (dq, J = 10.8, 2.4 Hz, 1H), 5.20 (dd, J = 7.1, 2.5 Hz, 1H), 6.78 (dd, J = 6.9, 5.0 Hz, 1H), 7.37-7.39 (m, 1H), 7.96 (dd, J = 5.0, 1.4 Hz, 1H). |
| 1-680 | | Colorless liquid | 1.08 (d, J = 1.8 Hz, 3H), 1.09 (d, J = 1.8 Hz, 3H), 1.46 (s, 3H), 1.62 (dd, J = 13.5, 4.8 Hz, 1H), 1.70 (dt, J = 13.3, 2.3 Hz, 1H), 2.13-2.18 (m, 1H), 2.29 (dd, J = 13.5, 10.8 Hz, 1H), 2.35 (s, 3H), 2.83 (dd, J = 13.5, 6.6 Hz, 1H), 3.63 (dd, J = 6.6, 2.5 Hz, 1H), 4.03 (d, J = 8.2 Hz, 1H), 4.41 (d, J = 12.4 Hz, 1H), 4.56 (d, J = 12.4 Hz, 1H), 7.11 (dd, J = 15.1, 8.2 Hz, 3H), 7.21-7.25 (m, 1H). |

[Table 89]

| Table 4-Continued | | | |
|---|---|---|---|
| No. | Physical property (melting point °C) | Form | $^1$H NMR spectrum σ ppm: |
| 1-681 | | Colorless liquid | 1.07 (d, J = 1.4 Hz, 3H), 1.09 (d, J = 1.8 Hz, 3H), 1.44 (s, 3H), 1.61 (dd, J = 14.4, 3.9 Hz, 1H), 1.69 (dt, J = 13.6, 2.4 Hz, 1H), 2.12-2.18 (m, 1H), 2.28 (dd, J = 13.5, 10.8 Hz, 1H), 2.35 (s, 3H), 2.81 (dd, J = 13.5, 6.6 Hz, 1H), 3.61 (dd, J = 6.6, 2.5 Hz, 1H), 4.02 (dq, J = 10.6, 2.3 Hz, 1H), 4.40 (d, J = 12.4 Hz, 1H), 4.55 (d, J = 11.9 Hz, 1H), 7.15 (d, J = 7.8 Hz, 2H), 7.22 (d, J = 7.8 Hz, 2H). |
| 1-682 | | Colorless liquid | 1.08 (d, J = 1.8 Hz, 3H), 1.10 (d, J = 1.8 Hz, 3H), 1.23 (t, J = 7.6 Hz, 3H), 1.44 (s, 3H), 1.64 (dd, J = 13.5, 4.6 Hz, 1H), 1.72 (dt, J = 13.5, 2.3 Hz, 1H), 2.13-2.17 (m, 1H), 2.30 (dd, J = 13.5, 11.0 Hz, 1H), 2.66-2.72 (m, 2H), 2.84 (dd, J = 13.5, 6.9 Hz, 1H), 3.65 (dd, J = 6.9, 2.3 Hz, 1H), 4.03 (dq, J = 11.0, 2.3 Hz, 1H), 4.43 (d, J = 12.4 Hz, 1H), 4.61 (d, J = 12.4 Hz, 1H), 7.16-7.28 (m, 3H), 7.35 (d, J = 7.3 Hz, 1H). |
| 1-685 | | Colorless liquid | 1.08 (d, J = 1.8 Hz, 3H), 1.09 (d, J = 1.8 Hz, 3H), 1.46 (s, 3H), 1.62 (dd, J = 13.7, 4.6 Hz, 1H), 1.70 (dt, J = 13.3, 2.3 Hz, 1H), 2.12-2.19 (m, 1H), 2.30 (dd, J = 13.5, 10.8 Hz, 1H), 2.83 (dd, J = 13.3, 6.9 Hz, 1H), 3.63 (dd, J = 6.6, 2.5 Hz, 1H), 3.81 (s, 3H), 4.02 (dq, J = 10.6, 2.3 Hz, 1H), 4.42 (d, J = 12.8 Hz, 1H), 4.57 (d, J = 12.8 Hz, 1H), 6.81-6.84 (m, 1H), 6.89-6.93 (m, 2H), 7.26 (t, J = 8.9 Hz, 1H). |
| 1-686 | | Colorless liquid | 1.07 (d, J = 1.8 Hz, 3H), 1.09 (d, J = 1.8 Hz, 3H), 1.43 (s, 3H), 1.61 (dd, J = 13.5, 4.8 Hz, 1H), 1.68 (dt, J = 13.4, 2.4 Hz, 1H), 2.11-2.18 (m, 1H), 2.29 (dd, J = 13.5, 10.8 Hz, 1H), 2.81 (dd, J = 13.7, 6.9 Hz, 1H), 3.60 (dd, J = 6.6, 2.5 Hz, 1H), 3.81 (s, 3H), 4.02 (dq, J = 10.9, 2.3 Hz, 1H), 4.37 (d, J = 11.9 Hz, 1H), 4.52 (d, J = 11.9 Hz, 1H), 6.86-6.90 (m, 2H), 7.24-7.27 (m, 2H). |
| 1-687 | | Colorless liquid | 1.08 (d, J = 2.3 Hz, 3H), 1.10 (d, J = 2.7 Hz, 3H), 1.47 (s, 3H), 1.66 (dd, J = 13.5, 4.8 Hz, 1H), 1.72 (dt, J = 13.7, 2.3 Hz, 1H), 2.12-2.19 (m, 1H), 2.32 (dd, J = 13.7, 10.5 Hz, 1H), 2.89 (dd, J = 13.5, 6.6 Hz, 1H), 3.67 (dd, J = 6.6, 2.5 Hz, 1H), 4.04 (dq, J = 10.6, 2.3 Hz, 1H), 4.52 (d, J = 13.3 Hz, 1H), 4.63 (d, J = 13.3 Hz, 1H), 7.20-7.35 (m, 3H), 7.54-7.57 (m, 1H). |
| 1-688 | | Colorless liquid | 1.08 (d, J = 2.3 Hz, 3H), 1.10 (d, J = 2.3 Hz, 3H), 1.47 (s, 3H), 1.64 (dd, J = 13.5, 4.8 Hz, 1H), 1.69 (dt, J = 13.3, 2.3 Hz, 1H), 2.12-2.19 (m, 1H), 2.32 (dd, J = 13.5, 10.8 Hz, 1H), 2.85 (dd, J = 13.5, 6.6 Hz, 1H), 3.64 (dd, J = 6.6, 2.5 Hz, 1H), 4.03 (dq, J = 10.9, 2.3 Hz, 1H), 4.45 (d, J = 12.8 Hz, 1H), 4.60 (d, J = 12.4 Hz, 1H), 7.13 (d, J = 8.2 Hz, 1H), 7.20-7.26 (m, 2H), 7.36 (t, J = 7.8 Hz, 1H). |
| 1-689 | | Colorless liquid | 1.08 (d, J = 2.3 Hz, 3H), 1.09 (d, J = 2.3 Hz, 3H), 1.46 (s, 3H), 1.61-1.72 (m, 2H), 2.12-2.19 (m, 1H), 2.31 (dd, J = 13.7, 10.5 Hz, 1H), 2.85 (dd, J = 13.3, 6.9 Hz, 1H), 3.64 (dd, J = 6.6, 2.5 Hz, 1H), 4.03 (dq, J = 10.6, 2.3 Hz, 1H), 4.43 (d, J = 12.4 Hz, 1H), 4.57 (d, J = 12.4 Hz, 1H), 7.19 (d, J = 7.8 Hz, 2H), 7.36 (d, J = 8.7 Hz, 2H). |
| 1-690 | | Colorless liquid | 1.08 (d, J = 2.3 Hz, 3H), 1.10 (d, J = 2.3 Hz, 3H), 1.47 (s, 3H), 1.63 (dd, J = 13.5, 4.8 Hz, 1H), 1.70 (dt, J = 13.6, 2.4 Hz, 1H), 2.12-2.19 (m, 1H), 2.31 (dd, J = 13.7, 11.0 Hz, 1H), 2.85 (dd, J = 13.3, 6.9 Hz, 1H), 3.64 (dd, J = 6.9, 2.7 Hz, 1H), 4.03 (dq, J = 10.9, 2.3 Hz, 1H), 4.43 (d, J = 12.8 Hz, 1H), 4.58 (d, J = 12.8 Hz, 1H), 6.94-6.99 (m, 1H), 7.04-7.11 (m, 2H), 7.30 (td, J = 7.8, 6.0 Hz, 1H). |

[Table 90]

| Table 4-Continued | | | |
|---|---|---|---|
| No. | Physical property (melting point °C) | Form | $^1$H NMR spectrum σ ppm: |
| 1-691 | | Colorless liquid | 1.07 (d, J = 2.3 Hz, 3H), 1.09 (d, J = 2.3 Hz, 3H), 1.44 (s, 3H), 1.63 (dd, J = 13.7, 4.6 Hz, 1H), 1.68 (dt, J = 13.6, 2.4 Hz, 1H), 2.11-2.18 (m, 1H), 2.30 (dd, J = 13.7, 10.5 Hz, 1H), 2.84 (dd, J = 13.3, 6.9 Hz, 1H), 3.62 (dd, J = 6.6, 2.5 Hz, 1H), 4.03 (dq, J = 10.5, 2.3 Hz, 1H), 4.40 (d, J = 11.9 Hz, 1H), 4.54 (d, J = 12.4 Hz, 1H), 7.00-7.05 (m, 2H), 7.27-7.33 (m, 2H). |
| 1-692 | | Colorless liquid | 1.09 (dd, J = 7.1, 2.1 Hz, 6H), 1.46 (s, 3H), 1.64 (dd, J = 13.7, 4.6 Hz, 1H), 1.69 (dt, J = 13.6, 2.4 Hz, 1H), 2.12-2.19 (m, 1H), 2.31 (dd, J = 13.7, 11.0 Hz, 1H), 2.85 (dd, J = 13.5, 6.6 Hz, 1H), 3.63 (dd, J = 6.6, 2.5 Hz, 1H), 4.03 (dq, J = 10.9, 2.3 Hz, 1H), 4.41 (d, J = 12.4 Hz, 1H), 4.56 (d, J = 12.8 Hz, 1H), 7.19-7.29 (m, 3H), 7.33 (s, 1H). |
| 1-693 | | Colorless liquid | 1.08 (dd, J = 6.9, 2.3 Hz, 6H), 1.44 (s, 3H), 1.63 (dd, J = 13.7, 4.6 Hz, 1H), 1.68 (dt, J = 13.4, 2.4 Hz, 1H), 2.11-2.18 (m, 1H), 2.30 (dd, J = 13.7, 11.0 Hz, 1H), 2.84 (dd, J = 13.3, 6.9 Hz, 1H), 3.62 (dd, J = 6.6, 2.5 Hz, 1H), 4.03 (dq, J = 10.9, 2.3 Hz, 1H), 4.40 (d, J = 12.4 Hz, 1H), 4.55 (d, J = 12.4 Hz, 1H), 7.25-7.32 (m, 4H). |
| 1-694 | | Colorless liquid | 1.08 (d, J = 2.3 Hz, 3H), 1.10 (d, J = 2.3 Hz, 3H), 1.49 (s, 3H), 1.67 (dd, J = 13.5, 4.8 Hz, 1H), 1.75 (dt, J = 13.4, 2.4 Hz, 1H), 2.13-2.20 (m, 1H), 2.30-2.36 (m, 1H), 2.90 (dd, J = 13.5, 6.6 Hz, 1H), 3.72 (dd, J = 6.9, 2.7 Hz, 1H), 4.04 (dq, J = 10.6, 2.3 Hz, 1H), 4.49 (d, J = 13.3 Hz, 1H), 4.60 (d, J = 13.3 Hz, 1H), 7.15 (td, J = 7.8, 1.5 Hz, 1H), 7.31 (td, J = 7.4, 1.1 Hz, 1H), 7.50-7.54 (m, 2H). |
| 1-695 | | Colorless liquid | 1.06 (d, J = 2.3 Hz, 3H), 1.08 (d, J = 2.3 Hz, 3H), 1.45 (s, 3H), 1.60-1.70 (m, 2H), 2.11-2.18 (m, 1H), 2.30 (dd, J = 13.7, 11.0 Hz, 1H), 2.83 (dd, J = 13.7, 6.9 Hz, 1H), 3.62 (dd, J = 6.6, 2.5 Hz, 1H), 4.02 (dq, J = 10.9, 2.3 Hz, 1H), 4.39 (d, J = 12.8 Hz, 1H), 4.54 (d, J = 12.8 Hz, 1H), 7.17-7.25 (m, 2H), 7.39 (d, J = 7.8 Hz, 1H), 7.47 (s, 1H). |
| 1-696 | | Colorless liquid | 1.06 (d, J = 1.8 Hz, 3H), 1.08 (d, J = 2.3 Hz, 3H), 1.43 (s, 3H), 1.61 (dd, J = 13.5, 4.8 Hz, 1H), 1.66 (dd, J = 13.3, 2.3 Hz, 1H), 2.10-2.17 (m, 1H), 2.29 (dd, J = 13.5, 10.8 Hz, 1H), 2.82 (dd, J = 13.5, 6.6 Hz, 1H), 3.60 (dd, J = 6.9, 2.3 Hz, 1H), 4.01 (dq, J = 10.6, 2.3 Hz, 1H), 4.37 (d, J = 12.4 Hz, 1H), 4.51 (d, J = 12.4 Hz, 1H), 7.20 (d, J = 8.7 Hz, 2H), 7.45 (d, J = 8.2 Hz, 2H). |
| 1-697 | | Colorless liquid | 1.09 (d, J = 2.3 Hz, 3H), 1.10 (d, J = 2.7 Hz, 3H), 1.50 (s, 3H), 1.68 (dd, J = 13.5, 4.8 Hz, 1H), 1.76 (dt, J = 13.4, 2.4 Hz, 1H), 2.13-2.18 (m, 1H), 2.33 (dd, J = 13.7, 10.5 Hz, 1H), 2.90 (dd, J = 13.5, 6.6 Hz, 1H), 3.73 (dd, J = 6.6, 2.5 Hz, 1H), 4.05 (dq, J = 10.5, 2.3 Hz, 1H), 4.40 (d, J = 12.8 Hz, 1H), 4.52 (d, J = 13.3 Hz, 1H), 6.99 (td, J = 7.6, 1.7 Hz, 1H), 7.35 (t, J = 7.6 Hz, 1H), 7.46 (d, J = 7.8 Hz, 1H), 7.81 (d, J = 7.8 Hz, 1H). |
| 1-698 | | Colorless liquid | 1.08 (d, J = 2.3 Hz, 3H), 1.10 (d, J = 2.7 Hz, 3H), 1.46 (s, 3H), 1.61-1.71 (m, 2H), 2.12-2.19 (m, 1H), 2.31 (dd, J = 13.5, 10.8 Hz, 1H), 2.84 (dd, J = 13.3, 6.9 Hz, 1H), 3.62 (dd, J = 6.9, 2.7 Hz, 1H), 4.03 (dq, J = 10.6, 2.3 Hz, 1H), 4.37 (d, J = 12.4 Hz, 1H), 4.52 (d, J = 12.4 Hz, 1H), 7.08 (t, J = 7.8 Hz, 1H), 7.29 (d, J = 7.8 Hz, 1H), 7.61 (d, J = 7.8 Hz, 1H), 7.69 (s, 11H). |

[Table 91]

| Table 4-Continued | | | |
|---|---|---|---|
| No. | Physical property (melting point °C) | Form | $^1$H NMR spectrum σ ppm: |
| 1-699 | | Colorless liquid | 1.07 (d, J = 2.3 Hz, 3H), 1.09 (d, J = 2.3 Hz, 3H), 1.44 (s, 3H), 1.60-1.70 (m, 2H), 2.11-2.19 (m, 1H), 2.30 (dd, J = 13.5, 10.8 Hz, 1H), 2.83 (dd, J = 13.3, 6.9 Hz, 1H), 3.61 (dd, J = 6.9, 2.7 Hz, 1H), 4.03 (dq, J = 10.9, 2.3 Hz, 1H), 4.37 (d, J = 12.8 Hz, 1H), 4.52 (d, J = 12.8 Hz, 1H), 7.08 (d, J = 8.2 Hz, 2H), 7.67 (d, J = 8.2 Hz, 2H). |
| 1-700 | | Colorless liquid | 1.09 (dd, J = 7.1, 2.5 Hz, 6H), 1.48 (s, 3H), 1.66 (dd, J = 13.5, 4.8 Hz, 1H), 1.73 (dt, J = 13.7, 2.3 Hz, 1H), 2.13-2.20 (m, 1H), 2.33 (dd, J = 13.5, 10.8 Hz, 1H), 2.89 (dd, J = 13.7, 6.9 Hz, 1H), 3.69 (dd, J = 6.9, 2.3 Hz, 1H), 4.04 (dq, J = 10.9, 2.3 Hz, 1H), 4.63 (d, J = 13.3 Hz, 1H), 4.73 (d, J = 13.3 Hz, 1H), 7.37 (t, J = 7.6 Hz, 1H), 7.55 (t, J = 7.6 Hz, 1H), 7.63 (d, J = 8.2 Hz, 1H), 7.73 (d, J = 7.8 Hz, 1H). |
| 1-701 | | Colorless liquid | 1.09 (dd, J = 6.9, 2.7 Hz, 6H), 1.47 (s, 3H), 1.65 (dd, J = 13.7, 4.6 Hz, 1H), 1.71 (dt, J = 13.4, 2.4 Hz, 1H), 2.12-2.19 (m, 1H), 2.32 (dd, J = 13.7, 11.0 Hz, 1H), 2.87 (dd, J = 13.3, 6.9 Hz, 1H), 3.66 (dd, J = 6.6, 2.5 Hz, 1H), 4.04 (dq, J = 10.9, 2.3 Hz, 1H), 4.48 (d, J = 12.4 Hz, 1H), 4.63 (d, J = 6.2 Hz, 1H), 7.50-7.57 (m, 4H). |
| 1-702 | | Colorless liquid | 1.09 (dd, J = 6.9, 2.3 Hz, 6H), 1.47 (s, 3H), 1.64 (dd, J = 13.7, 4.6 Hz, 1H), 1.70 (dt, J = 13.3, 2.3 Hz, 1H), 2.15-2.17 (m, 1H), 2.32 (dd, J = 13.7, 10.5 Hz, 1H), 2.86 (dd, J = 13.5, 6.6 Hz, 1H), 3.65 (dd, J = 6.6, 2.5 Hz, 1H), 4.04 (dq, J = 10.6, 2.3 Hz, 1H), 4.49 (d, J = 12.8 Hz, 1H), 4.64 (d, J = 12.8 Hz, 1H), 7.45 (d, J = 7.8 Hz, 2H), 7.60 (d, J = 8.2 Hz, 2H). |
| 1-703 | | Colorless liquid | 1.08 (d, J = 3.2 Hz, 3H), 1.10 (d, J = 3.2 Hz, 3H), 1.48 (s, 3H), 1.69 (dd, J = 13.5, 4.8 Hz, 1H), 1.75 (dt, J = 13.7, 2.3 Hz, 1H), 2.13-2.19 (m, 1H), 2.34 (dd, J = 13.5, 10.8 Hz, 1H), 2.92 (dd, J = 13.5, 6.6 Hz, 1H), 3.75 (dd, J = 6.9, 2.3 Hz, 1H), 4.05 (dq, J = 10.9, 2.3 Hz, 1H), 4.62 (d, J = 12.8 Hz, 1H), 4.74 (d, J = 12.8 Hz, 1H), 7.37-7.41 (m, 1H), 7.58-7.61 (m, 2H), 7.65 (d, J = 7.3 Hz, 1H). |
| 1-704 | | Colorless liquid | 1.07 (d, J = 2.7 Hz, 3H), 1.08 (d, J = 2.7 Hz, 3H), 1.45 (s, 3H), 1.61-1.70 (m, 2H), 2.11-2.18 (m, 1H), 2.32 (dd, J = 13.5, 10.8 Hz, 1H), 2.86 (dd, J = 13.7, 6.9 Hz, 1H), 3.64 (dd, J = 6.6, 2.5 Hz, 1H), 4.03 (dq, J = 10.9, 2.3 Hz, 1H), 4.44 (d, J = 12.4 Hz, 1H), 4.58 (d, J = 12.4 Hz, 1H), 7.43 (t, J = 7.6 Hz, 1H), 7.54-7.57 (m, 2H), 7.62 (s, 1H). |
| 1-705 | | Colorless liquid | 1.06 (d, J = 2.3 Hz, 3H), 1.08 (d, J = 2.3 Hz, 3H), 1.45 (s, 3H), 1.61-1.70 (m, 2H), 2.08-2.17 (m, 1H), 2.31 (dd, J = 13.5, 10.8 Hz, 1H), 2.86 (dd, J = 13.5, 6.6 Hz, 1H), 3.64 (dd, J = 6.6, 2.5 Hz, 1H), 4.02 (dq, J = 10.9, 2.3 Hz, 1H), 4.47 (d, J = 13.3 Hz, 1H), 4.61 (d, J = 13.3 Hz, 1H), 7.43 (d, J = 8.2 Hz, 2H), 7.62 (d, J = 8.2 Hz, 2H). |
| 1-706 | 80.0-85.0 | Brown solid | 1.08 (d, J = 3.2 Hz, 3H), 1.10 (d, J = 3.2 Hz, 3H), 1.50 (s, 3H), 1.69 (dd, J = 13.3, 4.6 Hz, 1H), 1.73-1.79 (m, 1H), 2.12-2.19 (m, 1H), 2.34 (dd, J = 13.5, 10.8 Hz, 1H), 2.87-2.95 (m, 1H), 3.78 (dd, J = 6.9, 2.3 Hz, 1H), 4.02-4.08 (m, 1H), 4.84 (d, J = 14.2 Hz, 1H), 4.96 (d, J = 14.7 Hz, 1H), 7.39 (t, J = 7.6 Hz, 1H), 7.58 (t, J = 7.1 Hz, 1H), 7.73 (d, J = 7.8 Hz, 1H), 8.08 (d, J = 6.4 Hz, 1H). |

[Table 92]

| [Table 4] Continued | | | |
|---|---|---|---|
| No. | Physical property (melting point °C) | Form | $^1$H NMR spectrum σ ppm: |
| 1-709 | | Brown liquid | 1.08 (d, J = 2.7 Hz, 3H), 1.10 (d, J = 2.7 Hz, 3H), 1.58 (s, 3H), 1.67 (dd, J = 13.7, 4.6 Hz, 1H), 1.74 (dt, J = 13.4, 2.4 Hz, 1H), 2.12-2.22 (m, 1H), 2.32 (dd, J = 13.5, 10.8 Hz, 1H), 2.90 (dd, J = 13.3, 6.9 Hz, 1H), 3.73 (dd, J = 6.9, 2.7 Hz, 1H), 3.90 (s, 3H), 4.04 (dq, J = 10.5, 2.3 Hz, 1H), 4.84 (d, J = 12.8 Hz, 1H), 4.96 (d, J = 14.7 Hz, 1H), 7.31-7.36 (m, 1H), 7.54 (t, J = 7.6 Hz, 1H), 7.73 (d, J = 7.8 Hz, 1H), 7.95 (d, J = 7.8 Hz, 1H). |
| 1-712 | | Colorless liquid | 1.07 (d, J = 1.4 Hz, 3H), 1.09 (d, J = 1.4 Hz, 3H), 1.41 (s, 3H), 1.64 (dd, J = 26.6, 13.3 Hz, 1H), 1.79 (dt, J = 13.3, 6.6 Hz, 1H), 2.14-2.16 (m, 1H), 2.30 (dd, J = 13.5, 10.8 Hz, 1H), 2.88 (dd, J = 13.7, 6.9 Hz, 1H), 3.64 (dd, J = 6.9, 2.3 Hz, 1H), 4.02 (dq, J = 5.4, 2.9 Hz, 1H), 4.68 (d, J = 10.1 Hz, 1H), 4.78 (d, J = 10.1 Hz, 1H), 7.18 (dd, J = 8.0, 4.0 Hz, 1H), 7.31 (m, J = 8.2 Hz, 2H). |
| 1-713 | | Colorless liquid | 1.07 (d, J = 7.0 Hz, 3H), 1.09 (d, J = 7.0 Hz, 3H), 1.39 (s, 3H), 1.64 (dd, J = 13.4, 4.8 Hz, 1H), 1.75 (dt, J = 13.4, 2.3 Hz, 1H), 2.11-2.18 (m, 1H), 2.30 (dd, J = 13.5, 10.8 Hz, 1H), 2.87 (dd, J = 13.5, 6.6 Hz, 1H), 3.64 (dd, J = 6.4, 2.3 Hz, 1H), 4.01-4.03 (m, 1H), 4.57 (dd, J = 10.5, 1.8 Hz, 1H), 4.68 (dd, J = 10.5, 2.3 Hz, 1H), 6.98-7.01 (m, 1H), 7.20-7.23 (m, 2H). |
| 1-715 | | Colorless liquid | 1.07 (dd, J = 7.1, 3.4 Hz, 6H), 1.39 (s, 3H), 1.58 (dd, J = 13.7, 4.6 Hz, 1H), 1.75 (dt, J = 13.6, 2.4 Hz, 1H), 2.11-2.18 (m, 1H), 2.26 (dd, J = 13.5, 10.8 Hz, 1H), 2.82 (dd, J = 13.5, 6.6 Hz, 1H), 3.66 (dd, J = 6.6, 2.5 Hz, 1H), 4.01 (dq, J = 10.5, 2.3 Hz, 1H), 4.83 (d, J = 12.4 Hz, 1H), 5.06 (d, J = 12.4 Hz, 1H), 7.42-7.44 (m, 1H), 7.46-7.53 (m, 3H), 7.80 (d, J = 7.8 Hz, 1H), 7.85-7.86 (m, 1H), 8.11-8.12 (m, 1H). |
| 1-716 | | Colorless liquid | 1.09 (dd, J = 7.1, 2.1 Hz, 6H), 1.48 (s, 3H), 1.60 (dd, J = 13.7, 4.6 Hz, 1H), 1.74 (dt, J = 13.6, 2.4 Hz, 1H), 2.14-2.18 (m, 1H), 2.29 (dd, J = 13.7, 11.0 Hz, 1H), 2.84 (dd, J = 13.5, 6.6 Hz, 1H), 3.66 (dd, J = 6.6, 2.5 Hz, 1H), 4.02 (dq, J = 10.6, 2.3 Hz, 1H), 4.58 (d, J = 12.4 Hz, 1H), 4.74 (d, J = 12.4 Hz, 1H), 7.46-7.48 (m, 3H), 7.77 (s, 1H), 7.81-7.83 (m, 3H). |
| 1-717 | | Colorless liquid | 1.08 (d, J = 2.3 Hz, 3H), 1.10 (d, J = 2.7 Hz, 3H), 1.47 (s, 3H), 1.64-1.74 (m, 2H), 2.12-2.19 (m, 1H), 2.33 (dd, J = 13.7, 11.0 Hz, 1H), 2.90 (dd, J = 13.3, 6.9 Hz, 1H), 3.70 (dd, J = 6.6, 2.5 Hz, 1H), 4.04 (dq, J = 10.6, 2.3 Hz, 1H), 4.47 (d, J = 13.3 Hz, 1H), 4.59 (d, J = 13.3 Hz, 1H), 7.23-7.26 (m, 1H), 7.36 (d, J = 2.3 Hz, 1H), 7.45 (d, J = 8.2 Hz, 1H). |
| 1-718 | | Colorless liquid | 1.08 (d, J = 1.8 Hz, 3H), 1.10 (d, J = 2.3 Hz, 3H), 1.49 (s, 3H), 1.66 (dd, J = 13.7, 4.6 Hz, 1H), 1.73 (dt, J = 13.6, 2.2 Hz, 1H), 2.12-2.19 (m, 1H), 2.32 (dd, J = 13.5, 10.8 Hz, 1H), 2.86-2.91 (m, 1H), 3.73 (dd, J = 6.9, 2.3 Hz, 1H), 4.04 (dq, J = 10.8, 2.2 Hz, 1H), 4.59 (d, J = 13.7 Hz, 1H), 4.69 (d, J = 13.7 Hz, 1H), 7.19 (t, J = 6.2 Hz, 1H), 7.49 (d, J = 8.2 Hz, 1H), 7.70 (td, J = 7.8, 1.4 Hz, 1H), 8.53 (d, J = 5.0 Hz, 1H). |
| 1-721 | | Colorless liquid | 1.07 (d, J = 1.4 Hz, 3H), 1.09 (d, J = 1.8 Hz, 3H), 1.42 (s, 3H), 1.62 (dd, J = 13.5, 4.8 Hz, 1H), 1.67 (dt, J = 13.5, 2.4 Hz, 1H), 2.12-2.17 (m, 1H), 2.29 (dd, J = 13.5, 10.8 Hz, 1H), 2.81 (dd, J = 13.5, 6.6 Hz, 1H), 3.67 (dd, J = 6.6, 2.4 Hz, 1H), 4.02 (dq, J = 10.8, 2.4 Hz, 1H), 4.61 (d, J = 12.8 Hz, 1H), 4.74 (d, J = 12.8 Hz, 1H), 6.97-6.98 (m, 2H), 7.28 (dd, J = 4.6, 1.8 Hz, 1H). |

[Table 93]

| Table 4-Continued | | | |
|---|---|---|---|
| No. | Physical property (melting point °C) | Form | $^1$H NMR spectrum σ ppm: |
| 1-722 | | Pale yellow liquid | 1.07 (d, J = 1.8 Hz, 3H), 1.09 (d, J = 1.8 Hz, 3H), 1.43 (s, 3H), 1.67 (dt, J = 13.1, 2.2 Hz, 1H), 2.09-2.20 (m, 1H), 2.11-2.18 (m, 1H), 2.29 (dd, J = 13.5, 10.8 Hz, 1H), 2.82 (dd, J = 13.5, 6.6 Hz, 1H), 3.63 (dd, J = 6.9, 2.7 Hz, 1H), 4.02 (dq, J = 11.0, 2.3 Hz, 1H), 4.46 (d, J = 12.4 Hz, 1H), 4.58 (d, J = 12.4 Hz, 1H), 7.06 (dd, J = 5.0, 0.9 Hz, 1H), 7.20 (td, J = 2.1, 0.9 Hz, 1H), 7.30 (dd, J = 5.0, 2.7 Hz, 1H). |
| 1-723 | | Colorless liquid | 1.07 (d, J = 2.3 Hz, 3H), 1.09 (d, J = 2.3 Hz, 3H), 1.38 (s, 3H), 1.62-1.64 (m, 2H), 2.10-2.16 (m, 1H), 2.27 (s, 3H), 2.31 (dd, J = 13.5, 10.6 Hz, 1H), 2.38 (s, 3H), 2.84 (dd, J = 13.5, 6.6 Hz, 1H), 3.55 (dd, J = 6.6, 2.3 Hz, 1H), 4.03 (dq, J = 10.6, 2.3 Hz, 1H), 4.16 (d, J = 11.9 Hz, 1H), 4.32 (d, J = 11.9 Hz, 1H). |
| 1-724 | | Colorless liquid | 1.07 (d, J = 1.8 Hz, 3H), 1.08 (d, J = 1.8 Hz, 3H), 1.42 (s, 3H), 1.64-1.70 (m, 2H), 2.11-2.18 (m, 1H), 2.31 (dd, J = 13.5, 10.8 Hz, 1H), 2.87 (dd, J = 13.7, 6.9 Hz, 1H), 3.70 (dd, J = 6.9, 2.3 Hz, 1H), 3.74-3.85 (m, 2H), 4.03 (dq, J = 10.5, 2.3 Hz, 1H), 4.11 (t, J = 5.3 Hz, 2H), 6.89-6.98 (m, 3H), 7.26-7.31 (m, 2H). |
| 1-725 | | Colorless liquid | 1.06 (d, J = 2.3 Hz, 3H), 1.08 (d, J = 2.7 Hz, 3H), 1.42 (s, 3H), 1.62-1.67 (m, 2H), 2.10-2.17 (m, 1H), 2.30 (dd, J = 13.7, 10.5 Hz, 1H), 2.83 (dd, J = 13.5, 6.6 Hz, 1H), 3.56-3.68 (m, 5H), 4.02 (dq, J = 10.8, 2.2 Hz, 1H), 4.57 (s, 2H), 7.27-7.35 (m, 5H). |
| 1-727 | | Colorless liquid | 1.07 (s, 3H), 1.08 (s, 3H), 1.41 (s, 3H), 1.62-1.69 (m, 2H), 2.10-2.17 (m, 1H), 2.31 (dd, J = 13.5, 10.8 Hz, 1H), 2.90 (dd, J = 13.7, 6.9 Hz, 1H), 3.41 (s, 3H), 3.57 (t, J = 4.6 Hz, 2H), 3.68-3.74 (m, 2H), 3.92 (dd, J = 6.9, 2.3 Hz, 1H), 4.02 (dq, J = 10.8, 2.3 Hz, 1H), 4.71 (d, J = 6.9 Hz, 1H), 4.76 (d, J = 7.3 Hz, 1H). |
| 1-732 | | Colorless liquid | 1.08 (dd, J = 6.6, 4.4 Hz, 6H), 1.40 (s, 3H), 1.65 (s, 3H), 1.70-1.72 (m, 5H), 2.11-2.18 (m, 1H), 2.26 (dd, J = 13.3, 11.0 Hz, 1H), 2.80 (dd, J = 13.5, 6.6 Hz, 1H), 3.51 (dd, J = 6.4, 1.8 Hz, 1H), 3.89-3.96 (m, 3H), 5.30 (td, J = 6.8, 1.2 Hz, 1H). |
| 1-734 | | Colorless liquid | 1.08 (dd, J = 6.9, 2.3 Hz, 6H), 1.40 (s, 3H), 1.72-1.76 (m, 2H), 1.85 (t, J = 2.3 Hz, 3H), 2.12-2.16 (m, 1H), 2.28 (dd, J = 13.5, 10.8 Hz, 1H), 2.80 (dd, J = 13.7, 6.9 Hz, 1H), 3.72 (dd, J = 6.6, 2.5 Hz, 1H), 3.93 (dq, J = 10.9, 2.6 Hz, 1H), 4.01-4.15 (m, 2H). |
| 1-736 | | Colorless liquid | 0.84-0.94 (m, 2H), 1.07 (t, J = 7.1 Hz, 6H), 1.14-1.23 (m, 3H), 1.39 (s, 3H), 1.62-1.78 (m, 8H), 2.11-2.18 (m, 1H), 2.26 (dd, J = 13.3, 11.0 Hz, 1H), 2.78 (dd, J = 13.3, 6.9 Hz, 1H), 3.08 (dd, J = 9.2, 6.9 Hz, 1H), 3.22 (dd, J = 9.2, 6.4 Hz, 1H), 3.43 (dd, J = 6.9, 2.3 Hz, 1H), 3.92 (dq, J = 11.0, 2.6 Hz, 1H). |
| 1-740 | | Colorless liquid | 1.09 (dd, J = 7.1, 3.0 Hz, 6H), 1.45 (s, 3H), 1.68 (dd, J = 13.3, 5.5 Hz, 1H), 1.80 (dt, J = 13.6, 2.5 Hz, 1H), 2.14-2.18 (m, 1H), 2.26 (dd, J = 13.5, 10.8 Hz, 1H), 2.81 (dd, J = 13.5, 6.6 Hz, 1H), 3.58 (dd, J = 6.6, 2.5 Hz, 1H), 3.93 (dq, J = 11.0, 2.6 Hz, 1H), 4.50 (dd, J = 59.3, 12.6 Hz, 2H), 7.26-7.36 (m, 5H). |

[Table 94]

| Table 4-Continued | | | |
|---|---|---|---|
| No. | Physical property (melting point °C) | Form | $^1$H NMR spectrum σ ppm: |
| 1-741 | | Colorless liquid | 1.10 (dd, J = 7.1, 3.9 Hz, 6H), 1.45 (s, 3H), 1.71 (dd, J = 13.5, 5.3 Hz, 1H), 1.82 (dt, J = 13.4, 2.4 Hz, 1H), 2.14-2.21 (m, 1H), 2.28 (dd, J = 13.5, 10.8 Hz, 1H), 2.32 (s, 3H), 2.83 (dd, J = 13.3, 6.9 Hz, 1H), 3.59 (dd, J = 6.9, 2.3 Hz, 1H), 3.95 (dq, J = 11.0, 2.6 Hz, 1H), 4.39 (d, J = 11.9 Hz, 1H), 4.56 (d, J = 12.4 Hz, 1H), 7.15-7.23 (m, 3H), 7.33-7.34 (m, 1H). |
| 1-747 | | Colorless liquid | 1.09 (dd, J = 7.1, 3.0 Hz, 6H), 1.46 (s, 3H), 1.71 (dd, J = 13.5, 5.3 Hz, 1H), 1.83 (dt, J = 13.6, 2.6 Hz, 1H), 2.12-2.18 (m, 1H), 2.27 (dd, J = 13.3, 11.0 Hz, 1H), 2.84 (dd, J = 13.5, 6.6 Hz, 1H), 3.62 (dd, J = 6.6, 2.5 Hz, 1H), 3.81 (s, 3H), 3.94 (dq, J = 10.9, 2.7 Hz, 1H), 4.53 (dd, J = 36.2, 13.3 Hz, 2H), 6.84 (d, J = 8.2 Hz, 1H), 6.94 (d, J = 15.1 Hz, 1H), 7.21-7.22 (m, 1H), 7.40 (dt, J = 7.3, 0.9 Hz, 1H). |
| 1-750 | | Colorless liquid | 1.09 (dd, J = 6.9, 5.0 Hz, 6H), 1.46 (s, 3H), 1.72 (dd, J = 13.5, 5.3 Hz, 1H), 1.81 (dt, J = 13.4, 2.5 Hz, 1H), 2.13-2.20 (m, 1H), 2.29 (dd, J = 13.3, 11.0 Hz, 1H), 2.86 (dd, J = 13.5, 6.6 Hz, 1H), 3.62 (dd, J = 6.9, 2.3 Hz, 1H), 3.94 (dq, J = 11.0, 2.6 Hz, 1H), 4.55 (dd, J = 42.4, 13.1 Hz, 2H), 7.20-7.23 (m, 1H), 7.28-7.31 (m, 2H), 7.54 (dd, J = 7.1, 2.1 Hz, 1H). |
| 1-753 | | Colorless liquid | 1.10 (dd, J = 7.1, 4.4 Hz, 6H), 1.45 (s, 3H), 1.72 (dd, J = 13.6, 5.3 Hz, 1H), 1.82 (dt, J = 13.6, 2.4 Hz, 1H), 2.14-2.21 (m, 1H), 2.29 (dd, J = 13.7, 11.0 Hz, 1H), 2.86 (dd, J = 13.5, 6.6 Hz, 1H), 3.62 (dd, J = 6.9, 2.3 Hz, 1H), 3.95 (dq, J = 11.0, 2.6 Hz, 1H), 4.51 (d, J = 12.4 Hz, 1H), 4.61 (d, J = 12.4 Hz, 1H), 7.03 (td, J = 9.2, 1.1 Hz, 1H), 7.13 (td, J = 7.4, 1.1 Hz, 1H), 7.25-7.28 (m, 1H), 7.44 (dt, J = 7.6, 3.8 Hz, 1H). |
| 1-756 | | Colorless liquid | 1.10 (dd, J = 7.1, 4.4 Hz, 6H), 1.48 (s, 3H), 1.73 (dd, J = 13.5, 5.3 Hz, 1H), 1.83 (dt, J = 13.6, 2.5 Hz, 1H), 2.15-2.19 (m, 1H), 2.29 (dd, J = 13.3, 11.0 Hz, 1H), 2.87 (dd, J = 13.5, 6.6 Hz, 1H), 3.65 (dd, J = 6.6, 2.5 Hz, 1H), 3.95 (dq, J = 11.0, 2.6 Hz, 1H), 4.56 (dd, J = 42.8, 13.5 Hz, 2H), 7.21-7.25 (m, 2H), 7.33 (dd, J = 7.8, 1.4 Hz, 1H), 7.50 (dd, J = 4.6, 2.3 Hz, 1H). |
| 1-759 | | Colorless liquid | 1.10 (dd, J = 6.9, 4.6 Hz, 6H), 1.48 (s, 3H), 1.73 (dd, J = 13.5, 5.3 Hz, 1H), 1.84 (dt, J = 13.6, 2.4 Hz, 1H), 2.13-2.19 (m, 1H), 2.29 (dd, J = 13.7, 11.0 Hz, 1H), 2.85-2.90 (m, 1H), 3.66 (dd, J = 6.6, 2.5 Hz, 1H), 3.95 (dq, J = 10.9, 2.6 Hz, 1H), 4.53 (dd, J = 42.8, 13.5 Hz, 2H), 7.13 (td, J = 7.8, 1.4 Hz, 1H), 7.30 (t, J = 7.6 Hz, 1H), 7.50 (t, J = 12.1 Hz, 2H). |
| 1-762 | | Colorless liquid | 1.10 (dd, J = 6.9, 4.6 Hz, 6H), 1.49 (s, 3H), 1.74 (dd, J = 13.5, 5.3 Hz, 1H), 1.85 (dt, J = 13.4, 2.5 Hz, 1H), 2.14-2.19 (m, 1H), 2.30 (dd, J = 13.7, 11.0 Hz, 1H), 2.88 (dd, J = 13.3, 6.9 Hz, 1H), 3.67 (dd, J = 6.9, 2.3 Hz, 1H), 3.95 (dq, J = 11.0, 2.6 Hz, 1H), 4.43 (dd, J = 46.0, 13.1 Hz, 2H), 6.97 (td, J = 7.6, 1.4 Hz, 1H), 7.33 (t, J = 7.6 Hz, 1H), 7.45 (d, J = 6.4 Hz, 1H), 7.79 (d, J = 4.4 Hz, 1H). |
| 1-765 | | Colorless liquid | 1.10 (dd, J = 7.1, 4.8 Hz, 6H), 1.48 (s, 3H), 1.72 (dd, J = 13.5, 5.3 Hz, 1H), 1.82 (dt, J = 13.6, 2.5 Hz, 1H), 2.16-2.18 (m, 1H), 2.29 (dd, J = 13.7, 11.0 Hz, 1H), 2.86 (dd, J = 13.5, 6.6 Hz, 1H), 3.63 (dd, J = 6.6, 2.5 Hz, 1H), 3.95 (dq, J = 11.0, 2.6 Hz, 1H), 4.66 (dd, J = 40.5, 13.5 Hz, 2H), 7.36 (t, J = 7.8 Hz, 1H), 7.53 (t, J = 7.6 Hz, 1H), 7.62 (d, J = 7.8 Hz, 1H), 7.71 (d, J = 7.8 Hz, 1H). |

[Table 95]

| Table 4-Continued | | | |
|---|---|---|---|
| No. | Physical property (melting point °C) | Form | $^1$H NMR spectrum σ ppm: |
| 1-768 | | Orange liquid | 1.10 (t, J = 6.4 Hz, 6H), 1.48 (s, 3H), 1.74 (dd, J = 13.5, 5.3 Hz, 1H), 1.84 (dt, J = 13.4, 2.4 Hz, 1H), 2.15-2.19 (m, 1H), 2.30 (dd, J = 13.5, 10.8 Hz, 1H), 2.90 (dd, J = 13.5, 6.6 Hz, 1H), 3.69 (dd, J = 6.9, 2.3 Hz, 1H), 3.95 (dq, J = 11.0, 2.6 Hz, 1H), 4.66 (dd, J = 45.8, 12.8 Hz, 2H), 7.36-7.39 (m, 1H), 7.55-7.60 (m, 2H), 7.64 (d, J = 7.8 Hz, 1H). |
| 1-777 | | Colorless liquid | 1.08 (d, J = 6.9 Hz, 6H), 1.38 (s, 3H), 1.71 (dd, J = 13.3, 5.5 Hz, 1H), 1.83 (dt, J = 13.4, 2.4 Hz, 1H), 2.12-2.17 (m, 1H), 2.26 (dd, J = 13.3, 11.0 Hz, 1H), 2.38 (s, 6H), 2.85 (dd, J = 13.5, 6.6 Hz, 1H), 3.53 (dd, J = 6.6, 2.5 Hz, 1H), 3.93 (dq, J = 10.9, 2.7 Hz, 1H), 4.47 (dd, J = 51.3, 10.5 Hz, 2H), 7.00 (d, J = 7.8 Hz, 2H), 7.09 (dd, J = 8.2, 6.4 Hz, 1H). |
| 1-778 | | Colorless liquid | 1.08 (dd, J = 6.9, 4.1 Hz, 6H), 1.37 (s, 3H), 1.70 (dd, J = 13.7, 5.5 Hz, 1H), 1.80 (dt, J = 13.6, 2.4 Hz, 1H), 2.11-2.18 (m, 1H), 2.26 (dd, J = 13.7, 11.0 Hz, 1H), 2.84 (dd, J = 13.7, 6.9 Hz, 1H), 3.58 (dd, J = 6.4, 2.3 Hz, 1H), 3.92 (dq, J = 11.0, 2.6 Hz, 1H), 4.54 (dd, J = 38.5, 11.0 Hz, 2H), 6.87 (t, J = 9.2 Hz, 2H), 7.26-7.29 (m, 1H). |
| 1-779 | | Colorless liquid | 1.08 (t, J = 3.7 Hz, 6H), 1.40 (s, 3H), 1.71 (dd, J = 13.3, 5.5 Hz, 1H), 1.89 (dt, J = 13.6, 2.5 Hz, 1H), 2.15-2.16 (m, 1H), 2.26 (dd, J = 13.3, 11.0 Hz, 1H), 2.86 (dd, J = 13.5, 6.6 Hz, 1H), 3.58 (dd, J = 6.4, 2.3 Hz, 1H), 3.93 (dq, J = 11.0, 2.6 Hz, 1H), 4.70 (dd, J = 41.4, 10.3 Hz, 2H), 7.17 (dd, J = 8.7, 7.3 Hz, 1H), 7.29 (d, J = 8.2 Hz, 2H). |
| 1-780 | | Colorless liquid | 1.08 (dd, J = 6.9, 3.2 Hz, 6H), 1.38 (s, 3H), 1.70 (dd, J = 13.3, 5.5 Hz, 1H), 1.84 (dt, J = 13.6, 2.4 Hz, 1H), 2.14-2.16 (m, 1H), 2.26 (dd, J = 13.7, 11.0 Hz, 1H), 2.85 (dd, J = 13.7, 6.4 Hz, 1H), 3.58 (dd, J = 6.4, 2.3 Hz, 1H), 3.93 (dq, J = 10.9, 2.7 Hz, 1H), 4.60 (ddd, J = 41.4, 10.5, 2.1 Hz, 2H), 6.98 (td, J = 8.6, 1.7 Hz, 1H), 7.19-7.22 (m, 2H). |
| 1-785 | | Colorless liquid | 1.09 (dd, J = 7.1, 4.4 Hz, 6H), 1.49 (s, 3H), 1.72 (dd, J = 13.5, 5.3 Hz, 1H), 1.82 (dt, J = 13.6, 2.4 Hz, 1H), 2.13-2.20 (m, 1H), 2.29 (dd, J = 13.7, 11.0 Hz, 1H), 2.86 (dd, J = 13.5, 6.6 Hz, 1H), 3.67 (dd, J = 6.6, 2.5 Hz, 1H), 3.95 (dq, J = 10.9, 2.6 Hz, 1H), 4.61 (dd, J = 38.5, 13.7 Hz, 2H), 7.18 (dd, J = 7.1, 5.3 Hz, 1H), 7.47 (d, J = 7.8 Hz, 1H), 7.68 (td, J = 7.7, 1.7 Hz, 1H), 8.52 (dd, J = 2.7, 1.4 Hz, 1H). |
| 1-786 | | Colorless liquid | 1.09 (dd, J = 6.9, 5.5 Hz, 6H), 1.44 (s, 3H), 1.70 (dd, J = 13.5, 5.3 Hz, 1H), 1.79 (dt, J = 13.6, 2.5 Hz, 1H), 2.13-2.20 (m, 1H), 2.29 (dd, J = 13.3, 11.0 Hz, 1H), 2.85 (dd, J = 13.5, 6.6 Hz, 1H), 3.60 (dd, J = 6.9, 2.3 Hz, 1H), 3.94 (dq, J = 11.0, 2.6 Hz, 1H), 4.50 (dd, J = 58.2, 12.4 Hz, 2H), 7.26-7.28 (m, 1H), 7.67 (d, J = 7.8 Hz, 1H), 8.54 (dd, J = 11.2, 2.5 Hz, 2H). |
| 1-788 | | Yellow liquid | 1.09 (d, J = 1.8 Hz, 3H), 1.11 (d, J = 2.3 Hz, 3H), 1.43 (s, 3H), 1.69 (dd, J = 13.3, 5.4 Hz, 1H), 1.78 (dt, J = 13.3, 3.1 Hz, 1H), 2.13-2.20 (m, 1H), 2.27 (dd, J = 13.3, 11.4 Hz, 1H), 2.80 (dd, J = 13.3, 6.2 Hz, 1H), 3.62 (dd, J = 6.2, 2.6 Hz, 1H), 3.94 (dq, J = 11.0, 2.6 Hz, 1H), 4.61 (d, J = 12.4 Hz, 1H), 4.73 (d, J = 12.4 Hz, 1H), 6.96-7.00 (m, 2H), 7.28 (dd, J = 4.1, 1.4 Hz, 1H). |

[Table 96]

| Table 4-Continued | | | |
|---|---|---|---|
| No. | Physical property (melting point °C) | Form | $^1$H NMR spectrum σ ppm: |
| 1-789 | | Yellow liquid | 1.09 (d, J = 3.7 Hz, 3H), 1.11 (d, J = 3.7 Hz, 3H), 1.44 (s, 3H), 1.70 (dd, J = 13.3, 5.3 Hz, 1H), 1.77 (dt, J = 13.3, 2.4 Hz, 1H), 2.09-2.20 (m, 1H), 2.27 (dd, J = 13.3, 11.0 Hz, 1H), 2.81 (dd, J = 13.3, 6.6 Hz, 1H), 3.58 (dd, J = 6.6, 2.6 Hz, 1H), 3.94 (dq, J = 11.0, 2.6 Hz, 1H), 4.45 (d, J = 12.4 Hz, 1H), 4.57 (d, J = 12.4 Hz, 1H), 7.05 (dd, J = 5.0, 0.9 Hz, 1H), 7.19 (dd, J = 2.7, 0.9 Hz, 1H), 7.30 (dd, J = 5.0, 2.7 Hz, 1H). |
| 1-790 | | Colorless liquid | 1.08 (dd, J = 7.1, 4.8 Hz, 6H), 1.37 (s, 3H), 1.70-1.72 (m, 2H), 2.13-2.17 (m, 1H), 2.25 (s, 3H), 2.28 (dd, J = 13.3, 10.5 Hz, 1H), 2.36 (s, 3H), 2.82 (dd, J = 13.3, 6.4 Hz, 1H), 3.49 (dd, J = 6.4, 2.3 Hz, 1H), 3.93 (dq, J = 10.9, 2.5 Hz, 1H), 4.22 (dd, J = 63.2, 11.9 Hz, 2H). |
| 1-799 | | Colorless liquid | 0.97 (dd, J = 6.9, 1.8 Hz, 6H), 1.28 (dd, J = 12.8, 3.2 Hz, 1H), 1.36 (s, 3H), 1.61-1.62 (m, 1H), 1.62 (d, J = 7.8 Hz, 3H), 1.71 (s, 3H), 1.85 (dd, J = 12.6, 9.4 Hz, 1H), 2.01-2.08 (m, 1H), 2.51 (dd, J = 12.8, 6.9 Hz, 1H), 3.25 (s, 3H), 3.52-3.57 (m, 2H), 3.89-3.95 (m, 2H), 5.30 (dd, J = 7.3, 6.0 Hz, 1H). |
| 1-801 | | Colorless liquid | 0.97 (dd, J = 6.9, 1.5 Hz, 6H), 1.32 (dd, J = 13.1, 3.0 Hz, 1H), 1.37 (s, 3H), 1.37-1.40 (m, 1H), 1.83 (t, J = 2.3 Hz, 3H), 1.85-1.87 (m, 1H), 2.04 (dt, J = 14.8, 6.1 Hz, 1H), 2.52 (dd, J = 13.1, 7.1 Hz, 1H), 3.26 (s, 3H), 3.57 (dq, J = 9.3, 1.5 Hz, 1H), 3.75 (dd, J = 7.1, 2.3 Hz, 1H), 3.99-4.14 (m, 2H). |
| 1-914 | | Colorless liquid | 0.86 (d, J = 6.9 Hz, 3H), 0.89 (t, J = 7.1 Hz, 3H), 1.09 (d, J = 6.9 Hz, 3H), 1.23-1.39 (m, 5H), 1.45-1.50 (m, 3H), 1.64-1.72 (m, 3H), 2.07-2.11 (m, 1H), 3.40 (dd, J = 9.4, 1.1 Hz, 1H), 3.60 (dd, J = 8.7, 4.1 Hz, 1H), 4.61 (d, J = 12.8 Hz, 1H), 4.66 (d, J = 12.8 Hz, 1H), 7.02 (t, J = 9.2 Hz, 1H), 7.13 (td, J = 7.6, 1.1 Hz, 1H), 7.25-7.28 (m, 1H), 7.50 (dt, J = 7.6, 3.8 Hz, 1H). |
| 1-915 | | Colorless liquid | 0.87 (d, J = 6.8 Hz, 3H), 0.89 (d, J = 6.8 Hz, 3H), 0.96-1.03 (m, 2H), 1.02 (d, J = 6.8 Hz, 3H), 1.05 (d, J = 6.8 Hz, 3H), 1.26 (ddd, J = 12.8, 9.2, 2.4 Hz, 1H), 1.43 (s, 3H), 1.47-1.58 (m, 2H), 1.84 (t, J = 12.4 Hz, 1H), 1.99-2.11 (m, 2H), 2.26 (dd, J = 12.8, 6.8 Hz, 1H), 3.53 (dd, J = 6.8, 2.4 Hz, 1H), 4.50 (d, J = 12.8 Hz, 1H), 4.60 (d, J = 12.8 Hz, 1H), 6.98-7.05 (m, 1H), 7.12 (td, J = 7.2, 1.6 Hz, 1H), 7.21-7.28 (m, 1H), 7.46 (td, J = 7.2, 1.6 Hz, 1H). |
| 1-916 | | Colorless liquid | 0.90 (d, J = 6.9 Hz, 3H), 1.04 (d, J = 6.9 Hz, 3H), 1.17 (t, J = 7.1 Hz, 3H), 1.45-1.50 (m, 1H), 1.50 (s, 3H), 1.62 (brs, 2H), 1.79 (dd, J = 13.1, 6.6 Hz, 1H), 2.49-2.52 (m, 1H), 3.23-3.25 (m, 1H), 3.43-3.46 (m, 1H), 3.49 (dd, J = 6.9, 2.3 Hz, 2H), 4.50 (d, J = 12.8 Hz, 1H), 4.59 (d, J = 12.8 Hz, 1H), 6.99-7.04 (m, 1H), 7.13 (td, J = 7.6, 1.1 Hz, 1H), 7.25-7.26 (m, 1H), 7.44 (td, J = 7.6, 1.7 Hz, 1H). |
| 1-917 | | Colorless liquid | 0.91 (t, J = 10.0 Hz, 3H), 0.91 (d, J = 6.9 Hz, 3H), 1.04 (d, J = 6.9 Hz, 3H), 1.47 (dd, J = 13.3, 2.3 Hz, 1H), 1.50 (s, 3H), 1.52-1.66 (m, 4H), 1.78 (dd, J = 13.3, 6.4 Hz, 1H), 2.51-2.53 (m, 1H), 3.06-3.10 (m, 1H), 3.38-3.48 (m, 3H), 4.50 (d, J = 12.8 Hz, 1H), 4.58 (d, J = 12.8 Hz, 1H), 7.01-7.03 (m, 1H), 7.12-7.14 (m, 1H), 7.25-7.26 (m, 1H), 7.44 (td, J = 7.4, 1.5 Hz, 1H). |

[Table 97]

| Table 4-Continued | | | |
|---|---|---|---|
| No. | Physical property (melting point °C) | Form | $^1$H NMR spectrum σ ppm: |
| 1-918 | | Colorless liquid | 0.86 (d, J = 6.9 Hz, 3H), 1.05 (d, J = 6.9 Hz, 3H), 1.52 (s, 3H), 1.53-1.66 (m, 3H), 1.79 (dd, J = 13.3, 6.4 Hz, 1H), 2.58-2.59 (m, 1H), 3.44 (dd, J = 6.9, 2.3 Hz, 1H), 3.56 (dd, J = 6.4, 2.3 Hz, 1H), 4.27 (d, J = 11.9 Hz, 1H), 4.48-4.60 (m, 3H), 7.00-7.03 (m, 1H), 7.12 (td, J = 7.4, 1.1 Hz, 1H), 7.21-7.37 (m, 6H), 7.43 (td, J = 7.4, 1.5 Hz, 1H). |
| 1-919 | | Colorless liquid | 0.91 (d, J = 6.9 Hz, 3H), 1.08 (d, J = 6.9 Hz, 3H), 1.52 (s, 3H), 1.57-1.70 (m, 3H), 1.87 (dd, J = 13.3, 6.4 Hz, 1H), 2.59-2.65 (m, 1H), 3.47 (dd, J = 6.4, 2.7 Hz, 1H), 3.70 (dd, J = 6.4, 2.3 Hz, 1H), 4.42 (d, J = 13.3 Hz, 1H), 4.51 (d, J = 12.8 Hz, 1H), 4.60 (d, J = 12.8 Hz, 1H), 4.64 (d, J = 13.3 Hz, 1H), 7.00-7.05 (m, 1H), 7.14-7.17 (m, 2H), 7.25-7.26 (m, 1H), 7.44 (td, J = 7.6, 1.7 Hz, 1H), 7.50 (d, J = 7.8 Hz, 1H), 7.69 (td, J = 7.6, 1.7 Hz, 1H), 8.50 (d, J = 4.1 Hz, 1H). |
| 1-920 | | Colorless liquid | 1.02-1.17 (m, 2H), 1.06 (d, J = 6.8 Hz, 3H), 1.08 (d, J = 6.8 Hz, 3H), 1.42 (s, 3H), 1.45-1.81 (m, 10H), 1.91-2.03 (m, 2H), 2.16 (dd, J = 13.2, 6.8 Hz, 1H), 3.53 (dd, J = 6.8, 2.8 Hz, 1H), 4.50 (d, J = 12.8 Hz, 1H), 4.60 (d, J = 12.8 Hz, 1H), 6.98-7.04 (m, 1H), 7.12 (td, J = 7.6, 1.6 Hz, 1H), 7.22-7.28 (m, 1H), 7.46 (dt, J = 7.6, 1.6 Hz, 1H) |
| 1-921 | | Colorless liquid | 0.10-0.21 (m, 2H), 0.42-0.53 (m, 2H), 0.91 (d, J = 7.2 Hz, 3H), 0.95-1.08 (m, 1H), 1.05 (d, J = 7.2 Hz, 3H), 1.44-1.50 (m, 1H), 1.51 (s, 3H), 1.54-1.70 (m, 2H), 1.78 (dd, J = 34.8, 17.4 Hz, 1H), 2.46-2.58 (m, 1H), 3.04 (dd, J = 29.3, 15.8 Hz, 1H), 3.30 (dd, J = 29.3, 14.7 Hz, 1H), 3.45 (dd, J = 20.1, 10.1 Hz, 1H), 3.54 (dd, J = 20.1, 12.1 Hz, 1H), 4.50 (d, J = 12.8 Hz, 1H), 4.59 (d, J = 12.8 Hz, 1H), 7.01-7.03 (m, 1H), 7.13 (dq, J = 7.6, 0.9 Hz, 1H), 7.24-7.26 (m, 1H), 7.44 (td, J = 7.3, 1.5 Hz, 1H). |
| 1-922 | | Colorless liquid | 0.84 (d, J = 6.8 Hz, 3H), 0.94 (d, J = 6.8 Hz, 3H), 1.52-1.56 (m, 1H), 1.53 (s, 3H), 1.63 (dd, J = 13.2, 6.0 Hz, 1H), 2.08-2.18 (m, 2H), 2.44 (dd, J = 13.6, 6.8 Hz, 1H), 3.68 (ddd, J = 12.0, 6.8, 1.6 Hz, 1H), 3.83 (dd, J = 6.0, 2, 8 Hz, 1H), 4.56 (d, J = 12.4 Hz, 1H), 4.65 (d, J = 12.4 Hz, 1H), 6.97-7.22 (m, 6H), 7.23-7.31 (m, 1H), 7.49 (dt, 7.2, 1.2 Hz, 1H). |
| 1-923 | | Colorless liquid | 0.90 (d, J = 7.2 Hz, 3H), 0.91 (d, J = 7.2 Hz, 3H), 1.51-1.63 (m, 2H), 1.53 (s, 3H), 2.08 (sept, J = 7.2 Hz, 1H), 2.15 (dd, 13.2, 11.6 Hz, 1H), 2.38 (dd, J = 13.2, 6.4 Hz, 1H), 3.37 (ddd, J = 11.6, 6.4, 2.0 Hz, 1H), 3.79 (dd, J = 6.8, 2, 8 Hz, 1H), 4.56 (d, J = 12.8 Hz, 1H), 4.65 (d, J = 12.8 Hz, 1H), 6.87-6.95 (m, 2H), 6.99 (d, J = 7.6 Hz, 1H), 7.04 (td, J = 8.0, 1.6 Hz, 1H), 7.15 (td, J = 7.6, 0.8 Hz, 1H), 7.22-7.31 (m, 2H), 7.48 (td, J = 7.6, 1.6 Hz, 1H). |
| 1-924 | | Colorless liquid | 0.88 (d, J = 6.8 Hz, 3H), 0.94 (d, J = 6.8 Hz, 3H), 1.50-1.61 (m, 2H), 1.53 (s, 3H), 2.07 (sept, J = 6.8 Hz, 1H), 2.11 (dd, J = 13.2, 11.6 Hz, 1H), 2.36 (dd, J = 13.2, 6.8 Hz, 1H), 3.36 (ddd, J = 11.6, 6.8, 1.6 Hz, 1H), 3.78 (dd, J = 6.8, 2, 4 Hz, 1H), 4.56 (d, J = 12.8 Hz, 1H), 4.65 (d, J = 12.8 Hz, 1H), 6.95-7.07 (m, 3H), 7.11-7.18 (m, 3H), 7.23-7.31 (m, 1H), 7.45 (dt, J = 7.6, 1.6 Hz, 1H). |
| 1-925 | | Colorless liquid | 0.65 (d, J = 6.8 Hz, 3H), 0.96 (d, J = 6.8 Hz, 3H), 1.50-1.62 (m, 2H), 1.54 (5, 3H), 2.18-2.27 (m, 2H), 2.40 (s, 3H), 2.59 (dd, J = 13.2, 6.8 Hz, 1H), 3.57 (ddd, J = 12.0, 6.4, 1.6 Hz, 1H), 3.85 (dd, J = 6.8, 2, 4 Hz, 1H), 4.60 (d, J = 12.8 Hz, 1H), 4.68 (d, J = 12.8 Hz, 1H), 7.01-7.21 (m, 6H), 7.24-7.31 (m, 1H), 7.50 (td, J = 7.6, 1.6 Hz, 1H). |

[Table 98]

| Table 4-Continued | | | |
|---|---|---|---|
| No. | Physical property (melting point °C) | Form | $^1$H NMR spectrum σ ppm: |
| 1-926 | | Colorless liquid | 0.90 (d, J = 6.8 Hz, 3H), 0.95 (d, J = 6.8 Hz, 3H), 1.51-1.55 (m, 1H), 1.53 (s, 3H), 1.63 (dd, J = 13.2, 6.4 Hz, 1H), 2.04-2.15 (m, 2H), 2.35 (s, 3H), 2.41 (dd, J = 13.2, 6.8 Hz, 1H), 3.35 (ddd, J = 12.0, 6.8, 2.0 Hz, 1H), 3.81 (dd, J = 6.8, 2, 4 Hz, 1H), 4.57 (d, J = 12.8 Hz, 1H), 4.66 (d, J = 12.8 Hz, 1H), 7.00-7.06 (m, 4H), 7.14 (dt, J = 7.2, 0.8 Hz, 1H), 7.19 (dt, J = 17.6, 7.6 Hz, 1H), 7.24-7.30 (m, 1H), 7.50 (td, J = 7.6, 1.6 Hz, 1H). |
| 1-927 | | Colorless liquid | 0.89 (d, J = 6.8 Hz, 3H), 0.95 (d, J = 6.8 Hz, 3H), 1.50-1.53 (m, 1H), 1.53 (s, 3H), 1.63 (dd, J = 13.6, 7.2 Hz, 1H), 2.07 (sept, J = 6.8 Hz, 1H), 2.12-2.16 (m, 1H), 2.32 (s, 3H), 2.46 (dd, J = 13.2, 6.8 Hz, 1H), 3.35 (ddd, J = 12.0, 6.8, 1.6 Hz, 1H), 3.80 (dd, J = 7.2, 2, 4 Hz, 1H), 4.57 (d, J = 12.8 Hz, 1H), 4.65 (d, J = 12.8 Hz, 1H), 7.01-7.16 (m, 6H), 7.24-7.29 (m, 1H), 7.45 (dt, J = 7.6, 1.6 Hz, 1H). |
| 1-928 | | Colorless liquid | 0.97 (d, J = 6.8 Hz, 3H), 0.99 (d, J = 6.8 Hz, 3H), 1.51 (s, 3H), 1.53-1.61 (m, 2H), 2.09 (sept, J = 6.8 Hz, 1H), 2.13 (dd, J = 12.8, 11.6 Hz, 1H), 2.34 (dd, J = 13.6, 6.8 Hz, 1H), 3.45 (ddd, J = 11.6, 6.4, 2.0 Hz, 1H), 3.74 (dd, J = 6.8, 2.8 Hz, 1H), 4.54 (d, J = 12.8 Hz, 1H), 4.63 (d, J = 12.8 Hz, 1H), 6.93-7.06 (m, 3H), 7.14 (td, J = 7.2, 0.8 Hz, 1H), 7.24-7.29 (m, 2H), 7.48 (dt, J = 7.2, 1.6 Hz, 1H). |
| 1-929 | | Colorless liquid | 0.97 (d, J = 7.2 Hz, 3H), 0.99 (d, J = 6.8 Hz, 3H), 1.04 (dd, J = 12.0, 6.8 Hz, 1H), 1.44 (s, 3H), 1.63-1.93 (m, 4H), 1.95-2.07 (m, 2H), 2.26 (dd, J = 13.2, 6.8 Hz, 1H), 2.49 (dd, J = 9.6, 6.8 Hz, 0.44H), 2.53 (dd, J = 9.6, 6.8 Hz, 0.56H), 2.65 (dd, J = 9.6, 4.4 Hz, 0.56H), 2.68 (dd, J = 9.6, 4.4 Hz, 0.44H), 3.55 (dd, J = 6.8, 2.8 Hz1H), 4.50 (d, J = 12.8 Hz, 1H), 4.59 (d, J = 12.8 Hz, 1H), 6.97-7.05 (m, 1H), 7.09-7.31 (m, 7H), 7.45 (td, J = 7.2, 2.0 Hz, 1H). |
| 1-930 | | Colorless liquid | 0.90 (d, J = 7.2 Hz, 3H), 1.05 (d, J = 7.2 Hz, 3H), 1.47-1.48 (m, 1H), 1.57 (s, 3H), 1.58-1.68 (m, 2H), 1.78 (dd, J = 13.1, 6.6 Hz, 1H), 2.51-2.53 (m, 1H), 3.45 (dd, J = 20.2, 10.8 Hz, 1H), 3.56 (dd, J = 20.2, 9.4 Hz, 1H), 3.71-3.78 (m, 1H), 3.97-4.05 (m, 1H), 4.50 (d, J = 12.8 Hz, 1H), 4.59 (d, J = 12.8 Hz, 1H), 5.13-5.16 (m, 1H), 5.23-5.26 (m, 1H), 5.86-5.88 (m, 1H), 7.01-7.03 (m, 1H), 7.13 (td, J = 7.4, 1.1 Hz, 1H), 7.25-7.26 (m, 1H), 7.44 (td, J = 7.4, 1.5 Hz, 1H). |
| 1-931 | | Colorless liquid | 0.92 (d, J = 6.9 Hz, 3H), 1.05 (d, J = 6.9 Hz, 3H), 1.46-1.49 (m, 1H), 1.50 (s, 3H), 1.63-1.69 (m, 2H), 1.81 (dd, J = 13.3, 6.4 Hz, 1H), 2.35-2.36 (m, 1H), 2.44-2.51 (m, 1H), 3.47 (dd, J = 6.2, 2.5 Hz, 1H), 3.84 (dd, J = 6.6, 2.1 Hz, 1H), 3.96 (dd, J = 16.5, 2.3 Hz, 1H), 4.20 (dd, J = 16.3, 2.5 Hz, 1H), 4.50 (d, J = 12.8 Hz, 1H), 4.60 (d, J = 12.4 Hz, 1H), 6.98-7.06 (m, 1H), 7.13 (td, J = 22.9, 11.4 Hz, 1H), 7.22-7.31 (m, 1H), 7.44 (td, J = 22.0, 11.0 Hz, 1H). |
| 1-932 | | Colorless liquid | 0.86 (d, J = 6.9 Hz, 3H), 1.06 (d, J = 6.9 Hz, 3H), 1.43-1.46 (m, 1H), 1.52 (s, 3H), 1.70-1.71 (m, 2H), 2.05-2.10 (m, 1H), 2.07 (s, 3H), 2.37-2.40 (m, 1H), 3.50-3.51 (m, 1H), 4.50 (d, J = 12.8 Hz, 1H), 4.60 (d, J = 12.8 Hz, 1H), 4.86 (dd, J = 7.3, 2.3 Hz, 1H), 7.02-7.05 (m, 1H), 7.14 (td, J = 7.6, 0.9 Hz, 1H), 7.26-7.27 (m, 1H), 7.43 (td, J = 7.6, 1.8 Hz, 1H). |
| 1-933 | 68.5-71.3 | White solid | 0.89 (d, J = 6.9 Hz, 3H), 1.06 (d, J = 3.2 Hz, 3H), 1.12 (t, J = 7.3 Hz, 3H), 1.47-1.51 (m, 1H), 1.50 (s, 3H), 1.68-1.70 (m, 2H), 2.04-2.07 (m, 1H), 2.35-2.36 (m, 1H), 3.18-3.22 (m, 2H), 3.50 (t, J = 4.1 Hz, 1H), 4.50 (d, J = 12.8 Hz, 1H), 4.60 (d, J = 12.4 Hz, 1H), 4.73 (brs, 1H), 4.82-4.84 (m, 1H), 7.02-7.04 (m, 1H), 7.13 (td, J = 7.6, 0.9 Hz, 1H), 7.25-7.27 (m, 1H), 7.43 (td, J = 7.6, 1.8 Hz, 1H). |

[Table 99]

| Table 4-Continued | | | |
|---|---|---|---|
| No. | Physical property (melting point °C) | Form | $^1$H NMR spectrum σ ppm: |
| 1-934 | | Colorless liquid | 0.83 (d, J = 6.9 Hz, 9H), 1.20 (s, 3H), 1.23-1.45 (m, 6H), 1.59 (d, J = 12.8 Hz, 1H), 2.01-2.07 (m, 1H), 2.52-2.54 (m, 1H), 3.60 (dd, J = 6.9, 1.8 Hz, 1H), 4.43 (d, J = 12.4 Hz, 1H), 4.46 (s, 1H), 4.53 (d, J = 11.9 Hz, 1H), 7.16 (d, J = 10.1 Hz, 1H), 7.19 (t, J = 7.3 Hz, 1H), 7.31-7.37 (m, 1H), 7.43 (t, J = 7.6 Hz, 1H). Measurement solvent DMSO-d6 |
| 1-935 | | Colorless liquid | 0.98 (d, J = 6.8 Hz, 3H), 1.04 (d, J = 6.8 Hz, 3H), 1.44 (s, 3H), 1.56 (dd, J = 13.6, 2.8 Hz, 1H), 1.68 (d, J = 12.8 Hz, 1H), 1.87 (d, J = 12.8 Hz, 1H), 2.30 (dd, J = 13.6, 6.8 Hz, 1H), 2.36 (sept, J = 6.8 Hz, 1H), 3.25 (s, 3H), 3.37 (s, 3H), 3.58 (dd, J = 6.8, 2.8 Hz, 1H), 4.52 (d, J = 13.2 Hz, 1H), 4.62 (d, J = 13.2 Hz, 1H), 7.02 (ddd, J = 9.6, 7.2, 0.8 Hz, 1H), 7.13 (dt, J = 7.6, 0.8 Hz, 1H), 7.23-7.29 (m, 1H), 7.45 (dt, J = 7.6, 1.6 Hz, 1H). |
| 1-936 | | Colorless liquid | 0.89 (d, J = 6.8 Hz, 3H), 1.03 (d, J = 6.8 Hz, 3H), 1.44 (s, 3H), 1.64 (dd, J = 13.2, 2.4 Hz, 1H), 1.66 (d, J = 12.8 Hz, 1H), 1.83 (d, J = 12.8 Hz, 1H), 2.34-2.45 (m, 2H), 3.64 (dd, J = 6.8, 2.4 Hz, 1H), 3.75-3.81 (m, 1H), 3.88-3.99 (m, 3H), 4.53 (d, J = 12.8 Hz, 1H), 4.62 (d, J = 12.8 Hz, 1H), 7.00-7.05 (m, 1H), 7.13 (dt, J = 7.2, 0.8 Hz, 1H), 7.23-7.29 (m, 1H), 7.45 (dt, J = 8.0, 1.2 Hz, 1H). |
| 1-1007 | | Colorless liquid | 1.06 (d, J = 2.3 Hz, 3H), 1.08 (d, J = 2.7 Hz, 3H), 1.40 (s, 3H), 1.60-1.65 (m, 2H), 1.66 (s, 3H), 1.74 (s, 3H), 2.11-2.17 (m, 1H), 2.30 (dd, J = 13.5, 10.8 Hz, 1H), 2.82 (dd, J = 13.3, 6.9 Hz, 1H), 3.57 (dd, J = 6.9, 2.7 Hz, 1H), 3.86-3.93 (m, 1H), 3.97-4.05 (m, 2H), 5.32 (t, J = 6.9 Hz, 1H). |
| 1-1009 | | Yellow liquid | 1.06 (s, 3H), 1.08 (s, 3H), 1.41 (s, 3H), 1.61-1.72 (m, 2H), 1.86 (t, J = 2.3 Hz, 3H), 2.10-2.17 (m, 1H), 2.31 (dd, J = 13.7, 11.0 Hz, 1H), 2.82 (dd, J = 13.7, 6.9 Hz, 1H), 3.78 (dd, J = 6.9, 2.3 Hz, 1H), 4.00-4.19 (m, 3H). |
| 1-1015 | | Colorless liquid | 1.08 (d, J = 1.8 Hz, 3H), 1.09 (d, J = 1.8 Hz, 3H), 1.46 (s, 3H), 1.62 (dd, J = 13.5, 4.8 Hz, 1H), 1.71 (dt, J = 13.4, 2.4 Hz, 1H), 2.11-2.19 (m, 1H), 2.30 (dd, J = 13.5, 10.8 Hz, 1H), 2.83 (dd, J = 13.3, 6.9 Hz, 1H), 3.63 (dd, J = 6.6, 2.5 Hz, 1H), 4.03 (dq, J = 10.9, 2.3 Hz, 1H), 4.44 (d, J = 12.4 Hz, 1H), 4.59 (d, J = 12.4 Hz, 1H), 7.27-7.37 (m, 5H). |
| 1-1016 | | Colorless liquid | 1.08 (d, J = 1.8 Hz, 3H), 1.09 (d, J = 2.3 Hz, 3H), 1.44 (s, 3H), 1.64 (dd, J = 13.3, 4.6 Hz, 1H), 1.72 (dt, J = 13.6, 2.4 Hz, 1H), 2.11-2.19 (m, 1H), 2.26-2.35 (m, 4H), 2.84 (dd, J = 13.5, 6.6 Hz, 1H), 3.63 (dd, J = 6.6, 2.5 Hz, 1H), 4.03 (dq, J = 10.6, 2.3 Hz, 1H), 4.39 (d, J = 12.4 Hz, 1H), 4.57 (d, J = 11.9 Hz, 1H), 7.14-7.23 (m, 3H), 7.32-7.35 (m, 1H). |
| 1-1022 | | Colorless liquid | 1.08 (d, J = 1.8 Hz, 3H), 1.09 (d, J = 2.3 Hz, 3H), 1.47 (s, 3H), 1.65 (dd, J = 13.3, 4.6 Hz, 1H), 1.74 (dt, J = 13.3, 2.3 Hz, 1H), 2.11-2.19 (m, 1H), 2.30 (dd, J = 13.5, 10.8 Hz, 1H), 2.86 (dd, J = 13.3, 6.9 Hz, 1H), 3.68 (dd, J = 6.6, 2.5 Hz, 1H), 3.83 (s, 3H), 4.03 (dq, J = 10.9, 2.3 Hz, 1H), 4.50 (d, J = 13.3 Hz, 1H), 4.60 (d, J = 13.3 Hz, 1H), 6.86 (d, J = 7.8 Hz, 1H), 6.94-6.98 (m, 1H), 7.23-7.28 (m, 1H), 7.40-7.43 (m, 1H). |
| 1-1025 | | Colorless liquid | 1.08 (d, J = 2.7 Hz, 3H), 1.10 (d, J = 2.7 Hz, 3H), 1.46 (s, 3H), 1.66 (dd, J = 13.7, 4.6 Hz, 1H), 1.72 (dt, J = 13.3, 2.3 Hz, 1H), 2.12-2.19 (m, 1H), 2.32 (dd, J = 13.5, 10.8 Hz, 1H), 2.88 (dd, J = 13.3, 6.9 Hz, 1H), 3.67 (dd, J = 6.6, 2.5 Hz, 1H), 4.04 (dq, J = 10.9, 2.3 Hz, 1H), 4.52 (d, J = 12.8 Hz, 1H), 4.63 (d, J = 12.8 Hz, 1H), 7.21-7.24 (m, 1H), 7.27-7.34 (m, 2H), 7.53-7.57 (m, 1H). |

[Table 100]

| Table 4-Continued | | | |
|---|---|---|---|
| No. | Physical property (melting point °C) | Form | $^1$H NMR spectrum σ ppm: |
| 1-1028 | | Colorless liquid | 1.08 (d, J = 2.3 Hz, 3H), 1.09 (d, J = 2.7 Hz, 3H), 1.45 (s, 3H), 1.65 (dd, J = 13.7, 4.6 Hz, 1H), 1.72 (dt, J = 13.3, 2.3 Hz, 1H), 2.15 (dt, J = 17.2, 5.3 Hz, 1H), 2.31 (dd, J = 13.7, 11.0 Hz, 1H), 2.87 (dd, J = 13.3, 6.9 Hz, 1H), 3.67 (dd, J = 6.6, 2.5 Hz, 1H), 4.03 (dq, J = 10.6, 2.3 Hz, 1H), 4.57 (dd, J = 40.8, 12.4 Hz, 2H), 7.03 (t, J = 9.2 Hz, 1H), 7.13 (t, J = 7.6 Hz, 1H), 7.24-7.30 (m, 1H), 7.42-7.46 (m, 1H). |
| 1-1031 | | Colorless liquid | 1.08 (d, J = 2.3 Hz, 3H), 1.10 (d, J = 2.3 Hz, 3H), 1.48 (s, 3H), 1.67 (dd, J = 13.7, 4.6 Hz, 1H), 1.74 (dt, J = 13.6, 2.4 Hz, 1H), 2.11-2.20 (m, 1H), 2.33 (dd, J = 13.7, 11.0 Hz, 1H), 2.90 (dd, J = 13.3, 6.9 Hz, 1H), 3.71 (dd, J = 6.6, 2.5 Hz, 1H), 4.04 (dq, J = 10.6, 2.3 Hz, 1H), 4.53 (d, J = 13.3 Hz, 1H), 4.64 (d, J = 13.3 Hz, 1H), 7.19-7.30 (m, 2H), 7.34 (dd, J = 7.6, 1.6 Hz, 1H), 7.50-7.53 (m, 1H). |
| 1-1034 | | Colorless liquid | 1.08 (d, J = 1.8 Hz, 3H), 1.10 (d, J = 2.3 Hz, 3H), 1.49 (s, 3H), 1.67 (dd, J = 13.7, 4.6 Hz, 1H), 1.72-1.78 (m, 1H), 2.10-2.20 (m, 1H), 2.33 (dd, J = 13.5, 10.8 Hz, 1H), 2.90 (dd, J = 13.3, 6.9 Hz, 1H), 3.72 (dd, J = 6.9, 2.3 Hz, 1H), 4.01-4.08 (m, 1H), 4.49 (d, J = 13.3 Hz, 1H), 4.60 (d, J = 13.3 Hz, 1H), 7.15 (t, J = 7.6 Hz, 1H), 7.31 (t, J = 7.6 Hz, 1H), 7.52 (t, J = 7.6 Hz, 2H). |
| 1-1037 | | Colorless liquid | 1.09 (d, J = 2.3 Hz, 3H), 1.10 (d, J = 2.3 Hz, 3H), 1.50 (s, 3H), 1.68 (dd, J = 13.3, 4.6 Hz, 1H), 1.76 (dt, J = 13.6, 2.4 Hz, 1H), 2.11-2.20 (m, 1H), 2.33 (dd, J = 13.7, 10.5 Hz, 1H), 2.90 (dd, J = 13.7, 6.9 Hz, 1H), 3.73 (dd, J = 6.9, 2.3 Hz, 1H), 4.05 (dq, J = 10.9, 2.3 Hz, 1H), 4.40 (d, J = 13.3 Hz, 1H), 4.52 (d, J = 13.3 Hz, 1H), 6.99 (td, J = 7.7, 1.5 Hz, 1H), 7.35 (td, J = 7.4, 1.2 Hz, 1H), 7.47 (dd, J = 7.6, 1.6 Hz, 1H), 7.81 (dd, J = 7.8, 0.9 Hz, 1H). |
| 1-1040 | | Colorless liquid | 1.08 (d, J = 2.3 Hz, 3H), 1.10 (d, J = 2.3 Hz, 3H), 1.48 (s, 3H), 1.66 (dd, J = 13.5, 4.8 Hz, 1H), 1.73 (dt, J = 13.7, 2.3 Hz, 1H), 2.11-2.20 (m, 1H), 2.33 (dd, J = 13.5, 10.8 Hz, 1H), 2.89 (dd, J = 13.5, 6.6 Hz, 1H), 3.69 (dd, J = 6.9, 2.3 Hz, 1H), 4.04 (dq, J = 10.5, 2.1 Hz, 1H), 4.63 (d, J = 13.3 Hz, 1H), 4.73 (d, J = 13.7 Hz, 1H), 7.37 (t, J = 7.8 Hz, 1H), 7.55 (t, J = 7.6 Hz, 1H), 7.63 (d, J = 8.2 Hz, 1H), 7.73 (d, J = 7.8 Hz, 1H). |
| 1-1043 | | Colorless liquid | 1.08 (d, J = 3.7 Hz, 3H), 1.10 (d, J = 3.7 Hz, 3H), 1.48 (s, 3H), 1.68 (dd, J = 13.5, 4.8 Hz, 1H), 1.75 (dt, J = 13.4, 2.4 Hz, 1H), 2.13-2.20 (m, 1H), 2.34 (dd, J = 13.5, 10.8 Hz, 1H), 2.92 (dd, J = 13.5, 6.6 Hz, 1H), 3.75 (dd, J = 6.9, 2.3 Hz, 1H), 4.04 (dq, J = 10.8, 2.2 Hz, 1H), 4.62 (d, J = 12.8 Hz, 1H), 4.74 (d, J = 12.8 Hz, 1H), 7.37-7.41 (m, 1H), 7.58-7.61 (m, 2H), 7.65 (d, J = 7.3 Hz, 1H). |
| 1-1052 | | Pale yellow liquid | 1.06 (d, J = 2.3 Hz, 3H), 1.08 (d, J = 2.7 Hz, 3H), 1.38 (s, 3H), 1.64 (dd, J = 13.7, 4.6 Hz, 1H), 1.71 (dt, J = 13.4, 2.4 Hz, 1H), 2.09-2.19 (m, 1H), 2.29 (dd, J = 13.5, 10.8 Hz, 1H), 2.86 (dd, J = 13.3, 6.9 Hz, 1H), 3.64 (dd, J = 6.9, 2.3 Hz, 1H), 4.02 (dq, J = 10.8, 2.4 Hz, 1H), 4.56 (dt, J = 33.1, 10.3 Hz, 2H), 6.85-6.92 (m, 1H), 7.26-7.28 (m, 2H). |
| 1-1053 | | Colorless liquid | 1.08 (d, J = 7.3 Hz, 6H), 1.41 (s, 3H), 1.54-1.56 (m, 1H), 1.64 (dd, J = 15.0, 6.0 Hz, 1H), 2.14-2.16 (m, 1H), 2.30 (dd, J = 13.7, 5.6 Hz, 1H), 2.89 (dd, J = 13.5, 6.6 Hz, 1H), 3.64 (d, J = 6.9 Hz, 1H), 4.03 (dd, J = 6.9, 3.4 Hz, 1H), 4.68 (dd, J = 5.0, 2.5 Hz, 1H), 4.78 (dd, J = 4.8, 2.4 Hz, 1H), 7.17-7.20 (m, 1H), 7.30-7.32 (m, 2H). |

[Table 101]

| Table 4-Continued | | | |
|---|---|---|---|
| No. | Physical property (melting point °C) | Form | $^{1}$H NMR spectrum σ ppm: |
| 1-1054 | | Colorless liquid | 1.07 (d, J = 2.3 Hz, 3H), 1.09 (d, J = 2.7 Hz, 3H), 1.39 (s, 3H), 1.64 (dd, J = 13.7, 5.0 Hz, 1H), 1.75 (dt, J = 8.9, 6.6 Hz, 1H), 2.14-2.15 (m, 1H), 2.30 (dd, J = 13.7, 10.5 Hz, 1H), 2.87 (dd, J = 13.3, 6.9 Hz, 1H), 3.64 (dd, J = 6.9, 2.3 Hz, 1H), 4.00-4.04 (m, 1H), 4.57 (dd, J = 10.5, 1.8 Hz, 1H), 4.68 (dd, J = 10.5, 2.3 Hz, 1H), 6.98-7.01 (m, 1H), 7.18-7.26 (m, 2H). |
| 1-1058 | | Colorless liquid | 1.08 (d, J = 2.3 Hz, 3H), 1.10 (d, J = 2.7 Hz, 3H), 1.47 (s, 3H), 1.67-1.71 (m, 2H), 2.15-2.18 (m, 1H), 2.33 (dd, J = 13.5, 10.8 Hz, 1H), 2.90 (dd, J = 13.5, 6.6 Hz, 1H), 3.70 (dd, J = 6.6, 2.5 Hz, 1H), 4.04 (dq, J = 10.8, 2.2 Hz, 1H), 4.53 (dd, J = 45.6, 13.5 Hz, 2H), 7.25 (dd, J = 8.5, 2.1 Hz, 1H), 7.36 (d, J = 1.8 Hz, 1H), 7.45 (d, J = 8.2 Hz, 1H). |
| 1-1059 | | Colorless liquid | 1.08 (d, J = 7.2 Hz, 3H), 1.09 (d, J = 7.2 Hz, 3H), 1.49 (s, 3H), 1.66 (dd, J = 13.7, 4.6 Hz, 1H), 1.72-1.75 (m, 1H), 2.12-2.19 (m, 1H), 2.32 (dd, J = 13.5, 10.8 Hz, 1H), 2.89 (dd, J = 13.7, 6.9 Hz, 1H), 3.73 (dd, J = 6.6, 2.5 Hz, 1H), 4.03-4.06 (m, 1H), 4.59 (d, J = 13.2 Hz, 1H), 4.69 (d, J = 13.2 Hz, 1H), 7.18-7.20 (m, 1H), 7.48-7.50 (m, 1H), 7.68-7.72 (m, 1H), 8.52-8.54 (m, 1H). |
| 1-1060 | | Colorless liquid | 1.08 (t, J = 7.2 Hz, 3H), 1.10 (t, J = 7.2 Hz, 3H), 1.45 (brs, 3H), 1.64-1.69 (m, 2H), 2.15-2.17 (m, 1H), 2.32 (dd, J = 13.5, 10.8 Hz, 1H), 2.87 (dd, J = 13.7, 6.9 Hz, 1H), 3.66-3.67 (m, 1H), 4.03-4.05 (m, 1H), 4.45 (d, J = 12.4 Hz, 1H), 4.60 (d, J = 12.8 Hz, 1H), 7.29 (d, J = 4.4 Hz, 1H), 7.29 (dd, J = 7.8, 4.4 Hz, 1H), 7.69 (d, J = 7.8 Hz, 1H), 8.55 (s, 1H). |
| 1-1061 | | Colorless liquid | 1.08 (d, J = 7.2 Hz, 3H), 1.10 (d, J = 7.2 Hz, 3H), 1.49 (s, 3H), 1.61-1.76 (m, 2H), 2.11-2.23 (m, 1H), 2.33 (dd, J = 13.7, 10.5 Hz, 1H), 2.88 (dd, J = 13.3, 6.9 Hz, 1H), 3.66 (dd, J = 6.6, 2.5 Hz, 1H), 4.04 (dq, J = 10.6, 2.3 Hz, 1H), 4.45 (d, J = 13.7 Hz, 1H), 4.59 (d, J = 13.7 Hz, 1H), 7.27 (d, J = 5.8 Hz, 2H), 8.58 (d, J = 5.8 Hz, 2H). |
| 1-1064 | | Colorless liquid | 1.07 (d, J = 2.3 Hz, 3H), 1.09 (d, J = 2.3 Hz, 3H), 1.38 (s, 3H), 1.62-1.64 (m, 2H), 2.10-2.16 (m, 1H), 2.27 (s, 3H), 2.31 (dd, J = 13.5, 10.6 Hz, 1H), 2.38 (s, 3H), 2.84 (dd, J = 13.5, 6.6 Hz, 1H), 3.55 (dd, J = 6.6, 2.3 Hz, 1H), 4.03 (dq, J = 10.6, 2.3 Hz, 1H), 4.16 (d, J = 11.9 Hz, 1H), 4.32 (d, J = 11.9 Hz, 1H). |
| 1-1073 | | Colorless liquid | 1.07 (d, J = 4.6 Hz, 3H), 1.08 (d, J = 4.6 Hz, 3H), 1.40 (s, 3H), 1.65 (s, 3H), 1.73 (s, 3H), 1.69-1.74 (m, 2H), 2.11-2.18 (m, 1H), 2.26 (dd, J = 13.3, 11.0 Hz, 1H), 2.80 (dd, J = 13.53, 6.6 Hz, 1H), 3.51 (dd, J = 6.9, 2.3 Hz, 1H), 3.85-4.00 (m, 3H), 5.28-5.32 (m, 1H). |
| 1-1075 | | Pale yellow liquid | 1.07 (d, J = 2.3 Hz, 3H), 1.08 (d, J = 2.3 Hz, 3H), 1.40 (s, 3H), 1.71-1.77 (m, 2H), 1.85 (t, J = 2.3 Hz, 3H), 2.10-2.17 (m, 1H), 2.27 (dd, J = 13.5, 10.8 Hz, 1H), 2.80 (dd, J = 13.7, 6.9 Hz, 1H), 3.72 (dd, J = 6.6, 2.5 Hz, 1H), 3.93 (dq, J = 10.9, 2.6 Hz, 1H), 4.01-4.15 (m, 2H). |
| 1-1081 | | Colorless liquid | 1.08 (d, J = 3.2 Hz, 3H), 1.10 (d, J = 3.2 Hz, 3H), 1.45 (s, 3H), 1.68 (dd, J = 13.7, 4.6 Hz, 1H), 1.80 (dt, J = 13.6, 2.5 Hz, 1H), 2.13-2.20 (m, 1H), 2.26 (dd, J = 13.7, 11.0 Hz, 1H), 2.81 (dd, J = 13.5, 6.6 Hz, 1H), 3.58 (dd, J = 6.9, 2.3 Hz, 1H), 3.93 (dq, J = 10.9, 2.6 Hz, 1H), 4.43 (d, J = 12.4 Hz, 1H), 4.57 (d, J = 12.4 Hz, 1H), 7.25-7.36 (m, 5H). |

[Table 102]

| Table 4-Continued | | | |
|---|---|---|---|
| No. | Physical property (melting point °C) | Form | $^1$H NMR spectrum σ ppm: |
| 1-1082 | | Colorless liquid | 1.08 (d, J = 4.1 Hz, 3H), 1.10 (d, J = 4.1 Hz, 3H), 1.43 (s, 3H), 1.70 (dd, J = 13.5, 5.3 Hz, 1H), 1.81 (dt, J = 13.4, 2.5 Hz, 1H), 2.13-2.20 (m, 1H), 2.27 (dd, J = 13.3, 11.0 Hz, 1H), 2.31 (s, 3H), 2.82 (dd, J = 13.3, 6.9 Hz, 1H), 3.58 (dd, J = 6.6, 2.5 Hz, 1H), 3.94 (dq, J = 11.0, 2.6 Hz, 1H), 4.38 (d, J = 11.9 Hz, 1H), 4.55 (d, J = 12.4 Hz, 1H), 7.14-7.22 (m, 3H), 7.30-7.33 (m, 1H). |
| 1-1088 | | Colorless liquid | 1.08 (d, J = 2.7 Hz, 3H), 1.10 (d, J = 3.2 Hz, 3H), 1.46 (s, 3H), 1.71 (dd, J = 13.5, 5.3 Hz, 1H), 1.83 (dt, J = 13.6, 2.5 Hz, 1H), 2.12-2.19 (m, 1H), 2.27 (dd, J = 13.7, 11.0 Hz, 1H), 2.84 (dd, J = 13.3, 6.9 Hz, 1H), 3.62 (dd, J = 6.9, 2.3 Hz, 1H), 3.82 (s, 3H), 3.94 (dq, J = 10.8, 2.6 Hz, 1H), 4.48 (d, J = 12.8 Hz, 1H), 4.57 (d, J = 13.3 Hz, 1H), 6.84 (d, J = 8.2 Hz, 1H), 6.95 (td, J = 7.3 Hz, 0.9 Hz, 1H), 7.22-7.26 (m, 1H), 7.40 (d, J = 7.3 Hz, 1H). |
| 1-1091 | | Colorless liquid | 1.09 (d, J = 5.0 Hz, 3H), 1.10 (d, J = 5.0 Hz, 3H), 1.46 (s, 3H), 1.72 (dd, J = 13.5, 5.3 Hz, 1H), 1.81 (dt, J = 13.6, 2.5 Hz, 1H), 2.13-2.20 (m, 1H), 2.29 (dd, J = 13.5, 10.8 Hz, 1H), 2.86 (dd, J = 13.7, 6.9 Hz, 1H), 3.62 (dd, J = 6.6, 2.5 Hz, 1H), 3.94 (dq, J = 11.0, 2.6 Hz, 1H), 4.50 (d, J = 12.8 Hz, 1H), 4.60 (d, J = 13.3 Hz, 1H), 7.20-7.33 (m, 3H), 7.53 (dt, J = 8.2, 2.6 Hz, 1H). |
| 1-1094 | | Brown liquid | 1.09 (d, J = 4.1 Hz, 3H), 1.11 (d, J = 4.6 Hz, 3H), 1.45 (s, 3H), 1.72 (dd, J = 13.7, 5.5 Hz, 1H), 1.82 (dt, J = 13.6, 2.4 Hz, 1H), 2.14-2.21 (m, 1H), 2.29 (dd, J = 13.3, 11.0 Hz, 1H), 2.86 (dd, J = 13.5, 6.6 Hz, 1H), 3.62 (dd, J = 6.6, 2.5 Hz, 1H), 3.95 (dq, J = 11.0, 2.6 Hz, 1H), 4.56 (dd, J = 39.8, 12.8 Hz, 2H), 7.03 (t, J = 9.2 Hz, 1H), 7.11-7.15 (m, 1H), 7.25-7.29 (m, 1H), 7.44 (td, J = 7.6, 1.7 Hz, 1H). |
| 1-1097 | | Colorless liquid | 1.09 (d, J = 4.6 Hz, 3H), 1.10 (d, J = 4.6 Hz, 3H), 1.48 (s, 3H), 1.73 (dd, J = 13.5, 5.3 Hz, 1H), 1.84 (dt, J = 13.6, 2.4 Hz, 1H), 2.13-2.20 (m, 1H), 2.29 (dd, J = 13.3, 11.0 Hz, 1H), 2.87 (dd, J = 13.5, 6.6 Hz, 1H), 3.65 (dd, J = 6.6, 2.5 Hz, 1H), 3.95 (dq, J = 11.0, 2.6 Hz, 1H), 4.51 (d, J = 13.3 Hz, 1H), 4.62 (d, J = 13.7 Hz, 1H), 7.18-7.27 (m, 3H), 7.50 (dd, J = 7.1, 2.1 Hz, 1H). |
| 1-1100 | | Colorless liquid | 1.09 (d, J = 4.1 Hz, 3H), 1.11 (d, J = 4.6 Hz, 3H), 1.48 (s, 3H), 1.73 (dd, J = 13.5, 5.3 Hz, 1H), 1.84 (dt, J = 13.6, 2.4 Hz, 1H), 2.14-2.21 (m, 1H), 2.29 (dd, J = 13.3, 11.0 Hz, 1H), 2.88 (dd, J = 13.7, 6.9 Hz, 1H), 3.66 (dd, J = 6.9, 2.3 Hz, 1H), 3.95 (dq, J = 10.9, 2.7 Hz, 1H), 4.47 (d, J = 13.3 Hz, 1H), 4.58 (d, J = 13.3 Hz, 1H), 7.13 (td, J = 7.7 Hz, 1.5 Hz, 1H), 7.30 (td, J = 7.6 Hz, 1.1 Hz, 1H), 7.50 (td, J = 8.0 Hz, 1.2 Hz, 2H). |
| 1-1103 | | Colorless liquid | 1.09 (d, J = 4.1 Hz, 3H), 1.11 (d, J = 4.6 Hz, 3H), 1.49 (s, 3H), 1.74 (dd, J = 13.3, 5.5 Hz, 1H), 1.85 (dt, J = 13.6, 2.4 Hz, 1H), 2.14-2.21 (m, 1H), 2.30 (dd, J = 13.7, 11.0 Hz, 1H), 2.88 (dd, J = 13.5, 6.6 Hz, 1H), 3.67 (dd, J = 6.4, 2.3 Hz, 1H), 3.95 (dq, J = 10.9, 2.5 Hz, 1H), 4.38 (d, J = 12.4 Hz, 1H), 4.49 (d, J = 13.3 Hz, 1H), 6.97 (t, J = 7.6 Hz, 1H), 7.33 (t, J = 7.6 Hz, 1H), 7.45 (d, J = 7.8 Hz, 1H), 7.79 (d, J = 7.8 Hz, 1H). |
| 1-1106 | | Colorless liquid | 1.09 (d, J = 4.6 Hz, 3H), 1.11 (d, J = 4.6 Hz, 3H), 1.48 (s, 3H), 1.72 (dd, J = 13.5, 5.3 Hz, 1H), 1.82 (dt, J = 13.6, 2.4 Hz, 1H), 2.13-2.20 (m, 1H), 2.29 (dd, J = 13.3, 11.0 Hz, 1H), 2.86 (dd, J = 13.5, 6.6 Hz, 1H), 3.63 (dd, J = 6.9, 2.3 Hz, 1H), 3.95 (dq, J = 10.9, 2.6 Hz, 1H), 4.61 (d, J = 13.3 Hz, 1H), 4.71 (d, J = 13.3 Hz, 1H), 7.36 (t, J = 7.6 Hz, 1H), 7.53 (t, J = 7.6 Hz, 1H), 7.62 (d, J = 7.8 Hz, 1H), 7.71 (d, J = 7.8 Hz, 1H). |

[Table 103]

| Table 4-Continued | | | |
|---|---|---|---|
| No. | Physical property (melting point °C) | Form | $^1H$ NMR spectrum σ ppm: |
| 1-1109 | | Pale yellow liquid | 1.09 (d, J = 6.0 Hz, 3H), 1.10 (d, J = 6.4 Hz, 3H), 1.48 (s, 3H), 1.74 (dd, J = 13.3, 5.5 Hz, 1H), 1.84 (dt, J = 13.6, 2.5 Hz, 1H), 2.12-2.20 (m, 1H), 2.30 (dd, J = 13.7, 11.0 Hz, 1H), 2.90 (dd, J = 13.5, 6.6 Hz, 1H), 3.69 (dd, J = 6.9, 2.3 Hz, 1H), 3.95 (dq, J = 11.0, 2.6 Hz, 1H), 4.60 (d, J = 12.8 Hz, 1H), 4.71 (d, J = 12.8 Hz, 1H), 7.35-7.40 (m, 1H), 7.57-7.59 (m, 2H), 7.64 (d, J = 7.8 Hz, 1H). |
| 1-1115 | | Colorless liquid | 1.11 (t, J = 6.2 Hz, 6H), 1.75 (dd, J = 13.7, 5.0 Hz, 1H), 1.85 (dt, J = 13.4, 2.2 Hz, 1H), 2.19-2.20 (m, 4H), 2.31 (dd, J = 13.5, 11.2 Hz, 1H), 2.89 (dd, J = 13.3, 6.9 Hz, 1H), 3.69 (dd, J = 6.4, 2.3 Hz, 1H), 3.90 (s, 3H), 3.97 (dq, J = 11.0, 2.6 Hz, 1H), 4.84 (d, J = 14.7 Hz, 1H), 4.95 (d, J = 14.7 Hz, 1H), 7.33 (t, J = 7.6 Hz, 1H), 7.53 (t, J = 7.6 Hz, 1H), 7.73 (d, J = 7.8 Hz, 1H), 7.95 (d, J = 7.8 Hz, 1H). |
| 1-1118 | | Colorless liquid | 1.10 (d, J = 6.9 Hz, 6H), 1.40 (s, 3H), 1.72 (dd, J = 13.5, 5.3 Hz, 1H), 1.84 (d, J = 13.3 Hz, 1H), 2.13-2.19 (m, 1H), 2.27 (dd, J = 13.1, 11.7 Hz, 1H), 2.40 (s, 6H), 2.87 (dd, J = 13.5, 6.6 Hz, 1H), 3.55 (d, J = 6.4 Hz, 1H), 3.93-3.97 (m, 1H), 4.42 (d, J = 10.5 Hz, 1H), 4.55 (d, J = 11.0 Hz, 1H), 7.02 (d, J = 7.3 Hz, 2H), 7.10 (t, J = 7.3 Hz, 1H). |
| 1-1119 | | Colorless liquid | 1.08 (dd, J = 6.9, 4.1 Hz, 6H), 1.37 (s, 3H), 1.70 (dd, J = 19.2, 9.6 Hz, 1H), 1.80 (dt, J = 13.4, 2.1 Hz, 1H), 2.11-2.18 (m, 1H), 2.26 (dd, J = 13.3, 11.0 Hz, 1H), 2.84 (dd, J = 13.5, 6.6 Hz, 1H), 3.58 (dd, J = 6.6, 2.1 Hz, 1H), 3.92 (dq, J = 11.0, 2.6 Hz, 1H), 4.49 (d, J = 11.0 Hz, 1H), 4.59 (d, J = 11.0 Hz, 1H), 6.87 (t, J = 7.6 Hz, 2H), 7.25-7.29 (m, 1H). |
| 1-1120 | | Colorless liquid | 1.10 (d, J = 7.3 Hz, 6H), 1.41 (s, 3H), 1.73 (dd, J = 13.5, 5.3 Hz, 1H), 1.89 (dt, J = 24.3, 12.1 Hz, 1H), 2.13-2.20 (m, 1H), 2.28 (dd, J = 24.7, 12.4 Hz, 1H), 2.88 (dd, J = 13.5, 6.6 Hz, 1H), 3.60 (dd, J = 6.4, 2.3 Hz, 1H), 3.95 (dq, J = 10.6, 2.6 Hz, 1H), 4.67 (d, J = 10.5 Hz, 1H), 4.76 (d, J = 9.6 Hz, 1H), 7.18 (t, J = 13.7 Hz, 1H), 7.31 (d, J = 7.8 Hz, 2H). |
| 1-1121 | | Colorless liquid | 1.08 (dd, J = 6.9, 3.2 Hz, 6H), 1.38 (s, 3H), 1.70 (dd, J = 13.3, 5.5 Hz, 1H), 1.84 (dt, J = 13.6, 2.4 Hz, 1H), 2.13-2.17 (m, 1H), 2.26 (dd, J = 24.7, 12.4 Hz, 1H), 2.85 (dd, J = 13.7, 6.4 Hz, 1H), 3.58 (dd, J = 6.4, 2.3 Hz, 1H), 3.90-3.92 (m, 1H), 4.55 (dd, J = 12.4, 6.2 Hz, 1H), 4.65 (dd, J = 10.5, 2.3 Hz, 1H), 6.98 (dt, J = 18.3, 9.2 Hz, 1H), 7.18-7.23 (m, 2H). |
| 1-1126 | <60 | White solid | 1.11 (dd, J = 6.6, 4.4 Hz, 6H), 1.50 (s, 3H), 1.73 (dd, J = 13.3, 5.5 Hz, 1H), 1.84 (dt, J = 6.6, 3.3 Hz, 1H), 2.14-2.21 (m, 1H), 2.30 (dd, J = 13.5, 11.2 Hz, 1H), 2.87 (dd, J = 13.5, 6.6 Hz, 1H), 3.68 (dd, J = 6.9, 1.8 Hz, 1H), 3.96 (dq, J = 10.9, 2.6 Hz, 1H), 4.58 (d, J = 14.2 Hz, 1H), 4.68 (d, J = 13.7 Hz, 1H), 7.17 (t, J = 14.9 Hz, 1H), 7.47 (d, J = 10.5 Hz, 1H), 7.69 (t, J = 6.9 Hz, 1H), 8.53 (d, J = 4.6 Hz, 1H). |
| 1-1127 | | Colorless liquid | 1.09 (dd, J = 6.6, 5.3 Hz, 6H), 1.44 (s, 3H), 1.70 (dd, J = 13.5, 5.3 Hz, 1H), 1.79 (dt, J = 18.3, 9.2 Hz, 1H), 2.13-2.20 (m, 1H), 2.29 (dd, J = 13.3, 11.0 Hz, 1H), 2.85 (dd, J = 13.5, 6.6 Hz, 1H), 3.60 (dd, J = 6.9, 2.3 Hz, 1H), 3.94 (dq, J = 11.0, 2.6 Hz, 1H), 4.43 (d, J = 12.4 Hz, 1H), 4.58 (d, J = 12.4 Hz, 1H), 7.27 (dd, J = 7.8, 5.0 Hz, 1H), 7.65 (d, J = 21.5 Hz, 1H), 8.53 (d, J = 2.3 Hz, 1H), 8.56 (s, 1H). |

[Table 104]

| [Table 4] Continued | | | |
|---|---|---|---|
| No. | Physical property (melting point °C) | Form | $^1H$ NMR spectrum σ ppm: |
| 1-1128 | | Brown liquid | 1.11 (dd, J = 6.9, 4.6 Hz, 6H), 1.49 (s, 3H), 1.72 (dd, J = 16.2, 4.0 Hz, 1H), 1.81 (dt, J = 13.6, 2.5 Hz, 1H), 2.14-2.22 (m, 1H), 2.31 (dd, J = 24.3, 12.1 Hz, 1H), 2.87 (dd, J = 13.5, 6.6 Hz, 1H), 3.62 (dd, J = 6.6, 2.5 Hz, 1H), 3.96 (dq, J = 8.0, 2.9 Hz, 1H), 4.44 (dd, J = 15.8, 7.1 Hz, 1H), 4.59 (t, J = 6.9 Hz, 1H), 7.25 (d, J = 10.0 Hz, 2H), 8.57 (dd, J = 4.6, 1.8 Hz, 2H). |
| 1-1131 | | Colorless liquid | 1.10 (dd, J = 6.9, 5.0 Hz, 6H), 1.38 (s, 3H), 1.71-1.75 (m, 2H), 2.15-2.18 (m, 1H), 2.26 (s, 3H), 2.31 (dd, J = 8.9, 4.8 Hz, 1H), 2.38 (s, 3H), 2.84 (dd, J = 13.5, 6.6 Hz, 1H), 3.51 (dd, J = 6.4, 2.3 Hz, 1H), 3.95 (dq, J = 10.9, 2.5 Hz, 1H), 4.15 (d, J = 9.4 Hz, 1H), 4.31 (d, J = 11.9 Hz, 1H). |
| 1-1221 | | Colorless liquid | 0.91 (dd, J = 12.1, 5.3 Hz, 1H), 0.97 (d, J = 6.9 Hz, 3H), 1.00 (d, J = 6.9 Hz, 3H), 1.03 (d, J = 6.9 Hz, 3H), 1.43 (s, 3H), 1.51 (dt, J = 13.3, 2.3 Hz, 1H), 1.88 (t, J = 11.9 Hz, 1H), 2.03-2.18 (m, 2H), 2.32 (dd, J = 13.1, 6.6 Hz, 1H), 3.54 (dd, J = 6.4, 2.7 Hz, 1H), 4.56 (dd, J = 40.3, 12.8 Hz, 2H), 6.99-7.04 (m, 1H), 7.13 (td, J = 7.6, 0.9 Hz, 1H), 7.22-7.28 (m, 1H), 7.46 (td, J = 7.6, 1.7 Hz, 1H). |
| 1-1222 | | Pale yellow liquid | 0.86 (d, J = 6.9 Hz, 3H), 0.89 (d, J = 6.4 Hz, 3H), 1.01 (t, J = 3.4 Hz, 1H), 1.04 (d, J = 6.9 Hz, 3H), 1.45 (s, 3H), 1.70-1.74 (m, 3H), 1.81-1.85 (m, 1H), 2.17 (td, J = 10.3, 5.8 Hz, 1H), 3.51 (dd, J = 6.2, 3.0 Hz, 1H), 4.55 (dd, J = 36.6, 12.8 Hz, 2H), 7.02 (t, J = 9.2 Hz, 1H), 7.13 (t, J = 7.1 Hz, 1H), 7.24-7.27 (m, 1H), 7.46 (td, J = 7.4, 1.5 Hz, 1H). |
| 1-1268 | | Colorless liquid | 1.04 (s, 3H), 1.06 (s, 3H), 1.40 (s, 3H), 1.50-1.61 (m, 2H), 1.66 (s, 3H), 1.74 (s, 3H), 2.08-2.17 (m, 1H), 2.25 (dd, J = 13.5, 10.8 Hz, 1H), 2.76 (dd, J = 13.5, 6.6 Hz, 1H), 3.58 (dd, J = 6.9, 2.7 Hz, 1H), 3.87-3.94 (m, 1H), 3.97-4.04 (m, 2H), 5.32 (t, J = 6.6 Hz, 1H). |
| 1-1270 | | Colorless liquid | 1.04 (s, 3H), 1.06 (s, 3H), 1.40 (s, 3H), 1.53-1.62 (m, 2H), 1.86 (t, J = 2.3 Hz, 3H), 2.08-2.15 (m, 1H), 2.27 (dd, J = 13.3, 10.5 Hz, 1H), 2.76 (dd, J = 13.5, 6.6 Hz, 1H), 3.80 (dd, J = 6.9, 2.7 Hz, 1H), 4.00-4.17 (m, 3H). |
| 1-1276 | | Colorless liquid | 1.06 (s, 3H), 1.07 (s, 3H), 1.45 (s, 3H), 1.50 (dd, J = 13.5, 4.4 Hz, 1H), 1.62 (dt, J = 13.3, 2.3 Hz, 1H), 2.10-2.18 (m, 1H), 2.25 (dd, J = 13.5, 10.8 Hz, 1H), 2.77 (dd, J = 13.5, 6.6 Hz, 1H), 3.65 (dd, J = 6.6, 2.5 Hz, 1H), 4.03 (dq, J = 10.5, 2.1 Hz, 1H), 4.45 (d, J = 12.8 Hz, 1H), 4.60 (d, J = 12.4 Hz, 1H), 7.26-7.35 (m, 5H). |
| 1-1277 | | Colorless liquid | 1.06 (s, 3H), 1.08 (s, 3H), 1.43 (s, 3H), 1.51 (dd, J = 13.5, 4.4 Hz, 1H), 1.64 (dt, J = 13.6, 2.2 Hz, 1H), 2.10-2.18 (m, 1H), 2.25 (dd, J = 13.3, 10.5 Hz, 1H), 2.33 (s, 3H), 2.78 (dd, J = 13.5, 6.6 Hz, 1H), 3.65 (dd, J = 6.6, 2.5 Hz, 1H), 4.03 (dq, J = 10.6, 2.1 Hz, 1H), 4.40 (d, J = 12.4 Hz, 1H), 4.58 (d, J = 12.4 Hz, 1H), 7.15-7.23 (m, 3H), 7.32-7.35 (m, 1H). |
| 1-1283 | | Colorless liquid | 1.05 (s, 3H), 1.07 (s, 3H), 1.46 (s, 3H), 1.52 (dd, J = 13.5, 4.4 Hz, 1H), 1.66 (dt, J = 13.4, 2.2 Hz, 1H), 2.10-2.17 (m, 1H), 2.26 (dd, J = 13.5, 10.8 Hz, 1H), 2.80 (dd, J = 13.3, 6.9 Hz, 1H), 3.69 (dd, J = 6.9, 2.7 Hz, 1H), 3.83 (s, 3H), 4.03 (dq, J = 10.6, 2.1 Hz, 1H), 4.50 (d, J = 13.3 Hz, 1H), 4.60 (d, J = 13.3 Hz, 1H), 6.85 (d, J = 8.2 Hz, 1H), 6.96 (t, J = 7.3 Hz, 1H), 7.23-7.28 (m, 1H), 7.41 (d, J = 6.9 Hz, 1H). |

[Table 105]

| Table 4-Continued | | | |
|---|---|---|---|
| No. | Physical property (melting point °C) | Form | $^1$H NMR spectrum σ ppm: |
| 1-1286 | | Pale yellow liquid | 1.07 (d, J = 6.9 Hz, 6H), 1.46 (s, 3H), 1.53 (dd, J = 13.4, 4.4 Hz, 1H), 1.64 (dt, J = 13.4, 2.2 Hz, 1H), 2.13-2.17 (m, 1H), 2.28 (dd, J = 13.3, 10.5 Hz, 1H), 2.82 (dd, J = 13.3, 6.9 Hz, 1H), 3.69 (dd, J = 6.9, 2.7 Hz, 1H), 4.04 (dq, J = 10.6, 2.1 Hz, 1H), 4.58 (dd, J = 44.4, 13.3 Hz, 2H), 7.22-7.24 (m, 1H), 7.31-7.33 (m, 2H), 7.53-7.57 (m, 1H). |
| 1-1289 | | Colorless liquid | 1.05 (s, 3H), 1.07 (s, 3H), 1.44 (s, 3H), 1.52 (dd, J = 13.5, 4.4 Hz, 1H), 1.63 (dt, J = 13.3, 2.3 Hz, 1H), 2.10-2.18 (m, 1H), 2.27 (dd, J = 13.5, 10.8 Hz, 1H), 2.81 (dd, J = 13.3, 6.9 Hz, 1H), 3.68 (dd, J = 6.6, 2.5 Hz, 1H), 4.03 (dq, J = 10.6, 2.1 Hz, 1H), 4.52 (d, J = 12.4 Hz, 1H), 4.63 (d, J = 12.8 Hz, 1H), 7.01-7.06 (m, 1H), 7.13 (td, J = 7.4, 1.2 Hz, 1H), 7.24-7.30 (m, 1H), 7.44 (td, J = 7.3, 1.8 Hz, 1H). |
| 1-1292 | | Colorless liquid | 1.06 (s, 3H), 1.08 (s, 3H), 1.47 (s, 3H), 1.54 (dd, J = 13.5, 4.4 Hz, 1H), 1.66 (dt, J = 13.3, 2.3 Hz, 1H), 2.10-2.18 (m, 1H), 2.28 (dd, J = 13.5, 10.8 Hz, 1H), 2.83 (dd, J = 13.5, 6.6 Hz, 1H), 3.72 (dd, J = 6.9, 2.3 Hz, 1H), 4.05 (dq, J = 10.6, 2.1 Hz, 1H), 4.53 (d, J = 13.3 Hz, 1H), 4.65 (d, J = 13.3 Hz, 1H), 7.20-7.29 (m, 2H), 7.34 (dd, J = 7.6, 1.6 Hz, 1H), 7.50-7.53 (m, 1H). |
| 1-1295 | | Colorless liquid | 1.06 (s, 3H), 1.08 (s, 3H), 1.48 (s, 3H), 1.55 (dd, J = 13.5, 4.4 Hz, 1H), 1.67 (dt, J = 13.3, 2.3 Hz, 1H), 2.10-2.18 (m, 1H), 2.28 (dd, J = 13.3, 10.5 Hz, 1H), 2.84 (dd, J = 13.3, 6.9 Hz, 1H), 3.73 (dd, J = 6.9, 2.3 Hz, 1H), 4.05 (dq, J = 10.8, 2.1 Hz, 1H), 4.49 (d, J = 13.3 Hz, 1H), 4.61 (d, J = 13.3 Hz, 1H), 7.15 (td, J = 7.7, 1.5 Hz, 1H), 7.31 (td, J = 7.4, 1.1 Hz, 1H), 7.50-7.54 (m, 2H). |
| 1-1298 | | Colorless liquid | 1.07 (s, 3H), 1.08 (s, 3H), 1.49 (s, 3H), 1.55 (dd, J = 13.5, 4.4 Hz, 1H), 1.65-1.70 (m, 1H), 2.11-2.18 (m, 1H), 2.28 (dd, J = 13.5, 10.8 Hz, 1H), 2.84 (dd, J = 13.5, 6.6 Hz, 1H), 3.74 (dd, J = 6.6, 2.5 Hz, 1H), 4.02-4.07 (m, 1H), 4.40 (d, J = 12.8 Hz, 1H), 4.52 (d, J = 12.8 Hz, 1H), 6.99 (t, J = 7.6 Hz, 1H), 7.35 (t, J = 7.3 Hz, 1H), 7.47 (d, J = 7.8 Hz, 1H), 7.81 (d, J = 7.8 Hz, 1H). |
| 1-1301 | | Colorless liquid | 1.06 (s, 3H), 1.08 (s, 3H), 1.48 (s, 3H), 1.50-1.58 (m, 1H), 1.62-1.68 (m, 1H), 2.11-2.18 (m, 1H), 2.28 (dd, J = 13.5, 10.8 Hz, 1H), 2.83 (dd, J = 13.5, 6.6 Hz, 1H), 3.70 (dd, J = 6.9, 2.3 Hz, 1H), 4.05 (dq, J = 10.8, 2.1 Hz, 1H), 4.63 (d, J = 13.7 Hz, 1H), 4.74 (d, J = 13.7 Hz, 1H), 7.37 (t, J = 7.6 Hz, 1H), 7.55 (t, J = 7.6 Hz, 1H), 7.63 (d, J = 7.8 Hz, 1H), 7.73 (d, J = 7.8 Hz, 1H). |
| 1-1304 | | Yellow liquid | 1.06 (s, 3H), 1.08 (s, 3H), 1.48 (s, 3H), 1.53-1.59 (m, 1H), 1.65-1.70 (m, 1H), 2.11-2.18 (m, 1H), 2.29 (dd, J = 13.7, 10.5 Hz, 1H), 2.86 (dd, J = 13.5, 6.6 Hz, 1H), 3.76 (dd, J = 6.6, 2.5 Hz, 1H), 4.05 (dq, J = 10.6, 2.1 Hz, 1H), 4.62 (d, J = 12.8 Hz, 1H), 4.74 (d, J = 12.8 Hz, 1H), 7.37-7.41 (m, 1H), 7.58-7.61 (m, 2H), 7.65 (d, J = 7.8 Hz, 1H). |
| 1-1310 | | Pale yellow liquid | 1.05 (d, J = 6.9 Hz, 6H), 1.47 (s, 3H), 1.53 (dd, J = 13.5, 4.4 Hz, 1H), 1.64 (dt, J = 13.5, 2.3 Hz, 1H), 2.10-2.14 (m, 1H), 2.26 (dd, J = 13.3, 10.5 Hz, 1H), 2.82 (dd, J = 13.3, 6.9 Hz, 1H), 3.72 (dd, J = 6.9, 2.3 Hz, 1H), 3.88 (s, 3H), 4.03 (dq, J = 10.8, 2.1 Hz, 1H), 4.89 (dd, J = 46.7, 14.7 Hz, 2H), 7.31 (t, J = 7.8 Hz, 1H), 7.52 (td, J = 7.8, 1.4 Hz, 1H), 7.71 (dd, J = 7.8, 0.9 Hz, 1H), 7.93 (dd, J = 7.8, 1.4 Hz, 1H). |
| 1-1313 | | Colorless liquid | 1.04 (d, J = 6.9 Hz, 6H), 1.35 (s, 3H), 1.50 (dd, J = 13.3, 4.6 Hz, 1H), 1.61 (dt, J = 13.5, 2.2 Hz, 1H), 2.08-2.15 (m, 1H), 2.24 (dd, J = 13.5, 10.8 Hz, 1H), 2.79 (dd, J = 13.3, 6.9 Hz, 1H), 3.64 (dd, J = 6.6, 2.5 Hz, 1H), 4.00 (dq, J = 10.6, 2.2 Hz, 1H), 4.55 (dd, J = 39.4, 11.0 Hz, 2H), 6.87 (t, J = 7.8 Hz, 2H), 7.23-7.28 (m, 1H). |

[Table 106]

| Table 4-Continued | | | |
|---|---|---|---|
| No. | Physical property (melting point °C) | Form | $^1$H NMR spectrum σ ppm: |
| 1-1314 | | Colorless liquid | 1.05 (d, J = 7.3 Hz, 6H), 1.38 (s, 3H), 1.51 (dd, J = 13.3, 4.6 Hz, 1H), 1.70 (dt, J = 13.6, 2.2 Hz, 1H), 2.08-2.15 (m, 1H), 2.24 (dd, J = 13.5, 10.8 Hz, 1H), 2.81 (dd, J = 13.5, 6.6 Hz, 1H), 3.63 (dd, J = 6.9, 2.3 Hz, 1H), 4.01 (dq, J = 10.5, 2.2 Hz, 1H), 4.72 (dd, J = 39.6, 10.3 Hz, 2H), 7.17 (td, J = 7.8, 0.6 Hz, 1H), 7.29 (d, J = 7.8 Hz, 2H). |
| 1-1315 | | Colorless liquid | 1.04 (d, J = 6.9 Hz, 6H), 1.37 (s, 3H), 1.50 (dd, J = 13.3, 4.6 Hz, 1H), 1.65 (dt, J = 13.4, 2.2 Hz, 1H), 2.08-2.15 (m, 1H), 2.24 (dd, J = 13.5, 10.8 Hz, 1H), 2.79 (dd, J = 13.5, 6.6 Hz, 1H), 3.63 (dd, J = 6.6, 2.5 Hz, 1H), 4.01 (dq, J = 10.5, 2.1 Hz, 1H), 4.62 (ddd, J = 41.7, 10.5, 2.3 Hz, 2H), 6.98 (td, J = 8.5, 1.5 Hz, 1H), 7.19-7.22 (m, 2H). |
| 1-1320 | 88.0-90.5 | Light brown solid | 1.05 (d, J = 6.9 Hz, 6H), 1.47 (s, 3H), 1.52 (dd, J = 13.5, 4.4 Hz, 1H), 1.64 (dt, J = 13.7, 2.1 Hz, 1H), 2.09-2.16 (m, 1H), 2.27 (dd, J = 13.5, 10.8 Hz, 1H), 2.81 (dd, J = 13.5, 6.6 Hz, 1H), 3.73 (dd, J = 6.9, 2.7 Hz, 1H), 4.03 (dq, J = 10.5, 2.1 Hz, 1H), 4.62 (dd, J = 40.5, 13.5 Hz, 2H), 7.17 (dd, J = 7.3, 5.0 Hz, 1H), 7.47 (d, J = 7.8 Hz, 1H), 7.68 (td, J = 7.8, 1.8 Hz, 1H), 8.52 (d, J = 5.0 Hz, 1H). |
| 1-1321 | | Brown liquid | 1.05 (d, J = 7.3 Hz, 6H), 1.43 (s, 3H), 1.51 (dd, J = 13.5, 4.4 Hz, 1H), 1.59-1.65 (m, 1H), 2.13 (dd, J = 14.4, 7.6 Hz, 1H), 2.26 (dd, J = 13.5, 10.8 Hz, 1H), 2.79 (dd, J = 13.5, 6.6 Hz, 1H), 3.66 (dd, J = 6.6, 2.1 Hz, 1H), 4.02 (dq, J = 10.8, 2.1 Hz, 1H), 4.51 (dd, J = 60.4, 12.4 Hz, 2H), 7.27 (dd, J = 7.8, 5.0 Hz, 1H), 7.67 (d, J = 7.8 Hz, 1H), 8.53 (dd, J = 5.0, 1.8 Hz, 1H), 8.56 (d, J = 1.8 Hz, 1H). |
| 1-1322 | | Brown liquid | 1.05 (d, J = 6.9 Hz, 6H), 1.46 (s, 3H), 1.51 (dd, J = 13.5, 4.4 Hz, 1H), 1.58-1.63 (m, 1H), 2.11-2.15 (m, 1H), 2.27 (dd, J = 13.5, 10.8 Hz, 1H), 2.80 (dd, J = 13.5, 6.6 Hz, 1H), 3.66 (dd, J = 6.6, 2.5 Hz, 1H), 4.02-4.04 (m, 1H), 4.51 (dd, J = 57.7, 13.7 Hz, 2H), 7.25 (d, J = 6.0 Hz, 2H), 8.55 (d, J = 6.0 Hz, 2H). |
| 1-1323 | | Yellow liquid | 1.05 (d, J = 0.5 Hz, 3H), 1.07 (d, J = 0.5 Hz, 3H), 1.41 (s, 3H), 1.49 (dd, J = 13.5, 4.4 Hz, 1H), 1.60 (dt, J = 13.5, 2.1 Hz, 1H), 2.10-2.17 (m, 1H), 2.25 (dd, J = 13.5, 10.5 Hz, 1H), 2.75 (dd, J = 13.5, 6.6 Hz, 1H), 3.68 (dd, J = 6.6, 2.1 Hz, 1H), 4.02 (dq, J = 10.8, 2.1 Hz, 1H), 4.62 (d, J = 12.8 Hz, 1H), 4.74 (d, J = 12.8 Hz, 1H), 6.97-6.99 (m, 2H), 7.28 (dd, J = 4.6, 1.8 Hz, 1H). |
| 1-1324 | | Yellow liquid | 1.05 (d, J = 0.5 Hz, 3H), 1.07 (d, J = 0.5 Hz, 3H), 1.42 (s, 3H), 1.50 (dd, J = 13.5, 4.4 Hz, 1H), 1.59 (dt, J = 13.5, 2.7 Hz, 1H), 2.11-2.16 (m, 1H), 2.25 (dd, J = 13.5, 10.6 Hz, 1H), 2.75 (dd, J = 13.5, 6.6 Hz, 1H), 3.64 (dd, J = 6.6, 2.7 Hz, 1H), 4.02 (dq, J = 10.6, 2.7 Hz, 1H), 4.46 (d, J = 12.4 Hz, 1H), 4.58 (d, J = 12.4 Hz, 1H), 7.06 (dd, J = 2.5, 1.4 Hz, 1H), 7.19 (dd, J = 1.4, 0.7 Hz, 1H), 7.30 (dd, J = 2.5, 1.4 Hz, 1H). |
| 1-1325 | <60 | White solid | 1.04 (d, J = 6.9 Hz, 6H), 1.35 (s, 3H), 1.51-1.56 (m, 3H), 2.08-2.16 (m, 1H), 2.25 (s, 3H), 2.36 (s, 3H), 2.76 (dd, J = 13.3, 6.9 Hz, 1H), 3.55 (dd, J = 6.6, 2.5 Hz, 1H), 4.01 (dq, J = 10.8, 2.1 Hz, 1H), 4.22 (dd, J = 65.0, 11.9 Hz, 2H). |
| 1-1334 | | Colorless liquid | 1.06 (dd, J = 6.9, 1.4 Hz, 6H), 1.39 (s, 3H), 1.61 (td, J = 6.6, 2.7 Hz, 2H), 1.65 (s, 3H), 1.73 (s, 3H), 2.10-2.15 (m, 1H), 2.28 (dd, J = 13.1, 11.2 Hz, 1H), 2.80 (dd, J = 13.3, 6.9 Hz, 1H), 3.55 (dd, J = 6.9, 1.8 Hz, 1H), 3.87-3.90 (m, 1H), 3.98-4.00 (m, 2H), 5.30 (td, J = 5.5, 1.4 Hz, 1H). |

[Table 107]

| Table 4-Continued | | | |
|---|---|---|---|
| No. | Physical property (melting point °C) | Form | $^1$H NMR spectrum σ ppm: |
| 1-1336 | | Colorless liquid | 1.06 (d, J = 7.3 Hz, 6H), 1.39 (s, 3H), 1.62-1.68 (m, 2H), 1.85 (t, J = 2.3 Hz, 3H), 2.09-2.14 (m, 1H), 2.29 (dd, J = 13.1, 11.2 Hz, 1H), 2.81 (dd, J = 15.1, 3.7 Hz, 1H), 3.78 (dd, J = 9.6, 4.1 Hz, 1H), 3.97-4.19 (m, 3H). |
| 1-1342 | | Colorless liquid | 1.07 (d, J = 1.8 Hz, 3H), 1.08 (d, J = 1.8 Hz, 3H), 1.45 (s, 3H), 1.61 (dd, J = 13.7, 4.6 Hz, 1H), 1.69 (dd, J = 13.4, 2.4 Hz, 1H), 2.11-2.18 (m, 1H), 2.28 (dd, J = 13.5, 10.8 Hz, 1H), 2.82 (dd, J = 13.7, 6.9 Hz, 1H), 3.62 (dd, J = 6.6, 2.5 Hz, 1H), 4.01 (dq, J = 10.6, 2.3 Hz, 1H), 4.43 (d, J = 12.4 Hz, 1H), 4.58 (d, J = 12.4 Hz, 1H), 7.254-7.36 (m, 5H). |
| 1-1343 | | Colorless liquid | 1.07 (d, J = 1.8 Hz, 3H), 1.08 (d, J = 2.3 Hz, 3H), 1.43 (s, 3H), 1.63 (dd, J = 13.5, 4.8 Hz, 1H), 1.71 (dt, J = 13.3, 2.3 Hz, 1H), 2.11-2.18 (m, 1H), 2.32 (s, 3H), 2.26-2.33 (m, 1H), 2.83 (dd, J = 13.5, 6.6 Hz, 1H), 3.62 (dd, J = 6.6, 2.5 Hz, 1H), 4.02 (dq, J = 10.6, 2.3 Hz, 1H), 4.38 (d, J = 12.4 Hz, 1H), 4.56 (d, J = 12.4 Hz, 1H), 7.14-7.20 (m, 3H), 7.31-7.33 (m, 1H). |
| 1-1344 | | Colorless liquid | 1.06 (d, J = 1.8 Hz, 3H), 1.08 (d, J = 2.3 Hz, 3H), 1.45 (s, 3H), 1.61 (dd, J = 13.5, 4.8 Hz, 1H), 1.69 (dt, J = 13.3, 2.3 Hz, 1H), 2.11-2.18 (m, 1H), 2.28 (dd, J = 13.7, 10.5 Hz, 1H), 2.34 (s, 3H), 2.82 (dd, J = 13.5, 6.6 Hz, 1H), 3.61 (dd, J = 6.9, 2.3 Hz, 1H), 4.01 (dq, J = 10.8, 2.4 Hz, 1H), 4.39 (d, J = 12.4 Hz, 1H), 4.54 (d, J = 12.4 Hz, 1H), 7.07-7.16 (m, 3H), 7.22 (t, J = 7.3 Hz, 1H). |
| 1-1345 | | Colorless liquid | 1.06 (d, J = 1.4 Hz, 3H), 1.08 (d, J = 1.8 Hz, 3H), 1.43 (s, 3H), 1.60 (dd, J = 13.5, 4.8 Hz, 1H), 1.68 (dt, J = 13.3, 2.3 Hz, 1H), 2.10-2.17 (m, 1H), 2.27 (dd, J = 13.7, 10.5 Hz, 1H), 2.34 (s, 3H), 2.80 (dd, J = 13.5, 6.9 Hz, 1H), 3.60 (dd, J = 6.9, 2.3 Hz, 1H), 4.01 (dq, J = 10.5, 2.3 Hz, 1H), 4.39 (d, J = 12.4 Hz, 1H), 4.54 (d, J = 12.4 Hz, 1H), 7.14 (d, J = 8.2 Hz, 2H), 7.21 (d, J = 7.8 Hz, 2H). |
| 1-1346 | | Colorless liquid | 1.08 (d, J = 1.8 Hz, 3H), 1.10 (d, J = 1.8 Hz, 3H), 1.23 (t, J = 7.8 Hz, 3H), 1.44 (s, 3H), 1.64 (dd, J = 13.5, 4.6 Hz, 1H), 1.72 (dt, J = 13.5, 2.3 Hz, 1H), 2.13-2.17 (m, 1H), 2.30 (dd, J = 13.5, 10.8 Hz, 1H), 2.68-2.72 (m, 2H), 2.84 (dd, J = 13.5, 6.9 Hz, 1H), 3.65 (dd, J = 6.9, 2.3 Hz, 1H), 4.03 (dq, J = 10.5, 2.3 Hz, 1H), 4.43 (d, J = 11.9 Hz, 1H), 4.60 (d, J = 11.9 Hz, 1H), 7.16-7.28 (m, 3H), 7.35-7.37 (m, 1H). |
| 1-1349 | | Colorless liquid | 1.08 (d, J = 1.4 Hz, 3H), 1.09 (d, J = 1.8 Hz, 3H), 1.47 (s, 3H), 1.65 (dd, J = 13.5, 4.8 Hz, 1H), 1.74 (dt, J = 13.3, 2.3 Hz, 1H), 2.12-2.19 (m, 1H), 2.30 (dd, J = 13.5, 10.8 Hz, 1H), 2.86 (dd, J = 13.5, 6.6 Hz, 1H), 3.68 (dd, J = 6.6, 2.5 Hz, 1H), 3.83 (s, 3H), 4.03 (dq, J = 10.9, 2.3 Hz, 1H), 4.50 (d, J = 13.3 Hz, 1H), 4.60 (d, J = 13.3 Hz, 1H), 6.85 (d, J = 8.2 Hz, 1H), 6.96 (t, J = 7.3 Hz, 1H), 7.26 (t, J = 7.8 Hz, 1H), 7.41 (d, J = 7.3 Hz, 1H). |
| 1-1350 | | Colorless liquid | 1.08 (d, J = 1.4 Hz, 3H), 1.09 (d, J = 1.4 Hz, 3H), 1.46 (s, 3H), 1.62 (dd, J = 13.7, 4.6 Hz, 1H), 1.70 (dt, J = 13.4, 2.4 Hz, 1H), 2.12-2.19 (m, 1H), 2.29 (dd, J = 13.7, 10.5 Hz, 1H), 2.83 (dd, J = 13.3, 6.9 Hz, 1H), 3.63 (dd, J = 6.9, 2.7 Hz, 1H), 3.81 (s, 3H), 4.02 (dq, J = 10.6, 2.3 Hz, 1H), 4.42 (d, J = 12.4 Hz, 1H), 4.57 (d, J = 12.4 Hz, 1H), 6.81-6.84 (m, 1H), 6.89-6.92 (m, 2H), 7.23-7.28 (m, 1H). |

[Table 108]

| Table 4-Continued | | | |
|---|---|---|---|
| No. | Physical property (melting point °C) | Form | $^1$H NMR spectrum σ ppm: |
| 1-1351 | | Colorless liquid | 1.07 (d, J = 1.8 Hz, 3H), 1.09 (d, J = 2.3 Hz, 3H), 1.43 (s, 3H), 1.61 (dd, J = 13.5, 4.8 Hz, 1H), 1.68 (dt, J = 13.3, 2.3 Hz, 1H), 2.09-2.18 (m, 1H), 2.29 (dd, J = 13.5, 10.8 Hz, 1H), 2.81 (dd, J = 13.5, 6.6 Hz, 1H), 3.60 (dd, J = 6.9, 2.7 Hz, 1H), 3.81 (d, J = 2.3 Hz, 3H), 4.02 (dq, J = 10.6, 2.3 Hz, 1H), 4.37 (d, J = 11.9 Hz, 1H), 4.52 (d, J = 11.9 Hz, 1H), 6.86-6.90 (m, 2H), 7.24-7.28 (m, 2H). |
| 1-1352 | | Colorless liquid | 1.08 (d, J = 2.7 Hz, 3H), 1.10 (d, J = 2.7 Hz, 3H), 1.47 (s, 3H), 1.66 (dd, J = 13.3, 4.6 Hz, 1H), 1.72 (dt, J = 13.6, 2.4 Hz, 1H), 2.12-2.19 (m, 1H), 2.32 (dd, J = 13.7, 10.5 Hz, 1H), 2.89 (dd, J = 13.3, 6.9 Hz, 1H), 3.67 (dd, J = 6.6, 2.5 Hz, 1H), 4.04 (dq, J = 10.5, 2.3 Hz, 1H), 4.52 (d, J = 13.3 Hz, 1H), 4.63 (d, J = 12.8 Hz, 1H), 7.21-7.24 (m, 1H), 7.27-7.34 (m, 2H), 7.54-7.57 (m, 1H). |
| 1-1353 | | Colorless liquid | 1.08 (d, J = 2.3 Hz, 3H), 1.10 (d, J = 2.7 Hz, 3H), 1.47 (s, 3H), 1.64 (dd, J = 13.5, 4.8 Hz, 1H), 1.69 (dt, J = 13.3, 2.3 Hz, 1H), 2.12-2.19 (m, 1H), 2.32 (dd, J = 13.5, 10.8 Hz, 1H), 2.85 (dd, J = 13.5, 6.6 Hz, 1H), 3.64 (dd, J = 6.6, 2.5 Hz, 1H), 4.04 (dq, J = 10.6, 2.3 Hz, 1H), 4.45 (d, J = 12.8 Hz, 1H), 4.60 (d, J = 12.4 Hz, 1H), 7.13 (d, J = 7.8 Hz, 1H), 7.20-7.26 (m, 2H), 7.36 (t, J = 7.8 Hz, 1H). |
| 1-1354 | | Colorless liquid | 1.08 (d, J = 1.8 Hz, 3H), 1.09 (d, J = 2.3 Hz, 3H), 1.46 (s, 3H), 1.67 (tt, J = 15.6, 4.0 Hz, 2H), 2.12-2.19 (m, 1H), 2.32 (dd, J = 13.7, 11.0 Hz, 1H), 2.85 (dd, J = 13.5, 6.6 Hz, 1H), 3.64 (dd, J = 6.9, 2.3 Hz, 1H), 4.03 (dq, J = 10.6, 2.3 Hz, 1H), 4.43 (d, J = 12.4 Hz, 1H), 4.57 (d, J = 12.4 Hz, 1H), 7.19 (d, J = 8.2 Hz, 2H), 7.36 (d, J = 8.7 Hz, 2H). |
| 1-1355 | 61.9-64.2 | White solid | 1.07 (dd, J = 7.1, 2.1 Hz, 6H), 1.43 (s, 3H), 1.61-1.73 (m, 2H), 2.12-2.16 (m, 1H), 2.29 (dd, J = 13.5, 10.8 Hz, 1H), 2.85 (dd, J = 13.5, 6.6 Hz, 1H), 3.65 (dd, J = 6.6, 2.5 Hz, 1H), 4.00-4.03 (m, 1H), 4.56 (dd, J = 40.8, 12.4 Hz, 2H), 6.99-7.04 (m, 1H), 7.12 (td, J = 7.4, 1.2 Hz, 1H), 7.23-7.28 (m, 1H), 7.43 (td, J = 7.4, 1.2 Hz, 1H). |
| 1-1356 | | Colorless liquid | 1.06 (d, J = 1.8 Hz, 3H), 1.08 (d, J = 2.3 Hz, 3H), 1.45 (s, 3H), 1.62 (dd, J = 13.7, 4.6 Hz, 1H), 1.68 (dt, J = 13.3, 2.3 Hz, 1H), 2.11-2.18 (m, 1H), 2.30 (dd, J = 13.7, 11.0 Hz, 1H), 2.83 (dd, J = 13.3, 6.9 Hz, 1H), 3.62 (dd, J = 6.6, 2.5 Hz, 1H), 4.02 (dq, J = 10.6, 2.3 Hz, 1H), 4.42 (d, J = 12.4 Hz, 1H), 4.56 (d, J = 12.8 Hz, 1H), 6.95 (td, J = 8.5 Hz, 2.3 Hz, 1H), 7.03-7.08 (m, 2H), 7.25-7.31 (m, 1H). |
| 1-1357 | | Colorless liquid | 1.06 (d, J = 2.3 Hz, 3H), 1.08 (d, J = 2.3 Hz, 3H), 1.43 (s, 3H), 1.61 (dd, J = 13.7, 4.6 Hz, 1H), 1.67 (dt, J = 13.6, 2.4 Hz, 1H), 2.10-2.17 (m, 1H), 2.29 (dd, J = 13.5, 10.8 Hz, 1H), 2.82 (dd, J = 13.3, 6.9 Hz, 1H), 3.61 (dd, J = 6.9, 2.3 Hz, 1H), 4.01 (dq, J = 10.9, 2.3 Hz, 1H), 4.38 (d, J = 11.9 Hz, 1H), 4.53 (d, J = 11.9 Hz, 1H), 6.98-7.05 (m, 2H), 7.27-7.30 (m, 2H). |
| 1-1358 | | Colorless liquid | 1.08 (dd, J = 6.9, 2.3 Hz, 6H), 1.47 (s, 3H), 1.65 (dd, J = 13.5, 4.8 Hz, 1H), 1.73 (dt, J = 13.4, 2.4 Hz, 1H), 2.11-2.18 (m, 1H), 2.31 (dd, J = 13.5, 10.8 Hz, 1H), 2.88 (dd, J = 13.3, 6.9 Hz, 1H), 3.70 (dd, J = 6.6, 2.5 Hz, 1H), 4.03 (dq, J = 10.6, 2.3 Hz, 1H), 4.52 (d, J = 13.3 Hz, 1H), 4.63 (d, J = 13.3 Hz, 1H), 7.18-7.28 (m, 2H), 7.33 (dd, J = 7.8, 1.4 Hz, 1H), 7.50 (d, J = 7.3 Hz, 1H). |

[Table 109]

| Table 4-Continued | | | |
|---|---|---|---|
| No. | Physical property (melting point °C) | Form | $^1$H NMR spectrum σ ppm: |
| 1-1359 | | Colorless liquid | 1.07 (dd, J = 7.1, 2.5 Hz, 6H), 1.45 (s, 3H), 1.62 (dd, J = 23.4, 11.7 Hz, 1H), 1.68 (dt, J = 13.4, 2.4 Hz, 1H), 2.11-2.18 (m, 1H), 2.30 (dd, J = 13.5, 10.8 Hz, 1H), 2.84 (dd, J = 13.5, 6.6 Hz, 1H), 3.62 (dd, J = 6.9, 2.3 Hz, 1H), 4.02 (dq, J = 10.8, 2.2 Hz, 1H), 4.39 (d, J = 12.4 Hz, 1H), 4.54 (d, J = 12.8 Hz, 1H), 7.18-7.28 (m, 3H), 7.32 (s, 1H). |
| 1-1360 | | Colorless liquid | 1.06-1.12 (m, 6H), 1.43 (s, 3H), 1.61 (dd, J = 13.5, 4.8 Hz, 1H), 1.67 (dt, J = 13.6, 2.4 Hz, 1H), 2.11-2.16 (m, 1H), 2.29 (dd, J = 13.5, 10.8 Hz, 1H), 2.82 (dd, J = 13.3, 6.9 Hz, 1H), 3.60 (dd, J = 6.9, 2.7 Hz, 1H), 4.01 (dq, J = 10.9, 2.3 Hz, 1H), 4.38 (d, J = 12.4 Hz, 1H), 4.53 (d, J = 12.4 Hz, 1H), 7.24-7.26 (m, 2H), 7.29-7.31 (m, 2H). |
| 1-1361 | | Colorless liquid | 1.08 (dd, J = 6.9, 2.3 Hz, 6H), 1.48 (s, 3H), 1.66 (dd, J = 13.5, 4.6 Hz, 1H), 1.74 (dt, J = 13.3, 2.3 Hz, 1H), 2.11-2.18 (m, 1H), 2.31 (dd, J = 13.5, 10.8 Hz, 1H), 2.89 (dd, J = 13.3, 6.9 Hz, 1H), 3.71 (dd, J = 6.9, 2.3 Hz, 1H), 4.03 (dq, J = 10.6, 2.3 Hz, 1H), 4.53 (dd, J = 44.4, 13.3 Hz, 2H), 7.14 (t, J = 7.8 Hz, 1H), 7.30 (t, J = 7.3 Hz, 1H), 7.51 (t, J = 7.3 Hz, 2H). |
| 1-1362 | | Colorless liquid | 1.06 (d, J = 2.3 Hz, 3H), 1.08 (d, J = 2.7 Hz, 3H), 1.45 (s, 3H), 1.62 (dd, J = 13.5, 4.8 Hz, 1H), 1.67 (dt, J = 13.4, 2.4 Hz, 1H), 2.11-2.18 (m, 1H), 2.30 (dd, J = 13.5, 10.8 Hz, 1H), 2.84 (dd, J = 13.5, 6.6 Hz, 1H), 3.62 (dd, J = 6.9, 2.3 Hz, 1H), 4.02 (dq, J = 10.6, 2.3 Hz, 1H), 4.39 (d, J = 12.4 Hz, 1H), 4.54 (d, J = 12.8 Hz, 1H), 7.18-7.25 (m, 2H), 7.40 (d, J = 7.8 Hz, 1H), 7.47 (s, 1H). |
| 1-1363 | | Colorless liquid | 1.06 (d, J = 1.8 Hz, 3H), 1.08 (d, J = 2.3 Hz, 3H), 1.43 (s, 3H), 1.61 (dd, J = 13.5, 4.8 Hz, 1H), 1.66 (dt, J = 13.3, 2.3 Hz, 1H), 2.10-2.17 (m, 1H), 2.29 (dd, J = 13.5, 10.8 Hz, 1H), 2.82 (dd, J = 13.5, 6.6 Hz, 1H), 3.60 (dd, J = 6.9, 2.3 Hz, 1H), 4.01 (dq, J = 10.6, 2.3 Hz, 1H), 4.37 (d, J = 12.4 Hz, 1H), 4.52 (d, J = 12.8 Hz, 1H), 7.20 (d, J = 8.2 Hz, 2H), 7.45 (d, J = 8.2 Hz, 2H). |
| 1-1364 | | Colorless liquid | 1.09 (d, J = 2.3 Hz, 3H), 1.10 (d, J = 2.3 Hz, 3H), 1.50 (s, 3H), 1.67 (dd, J = 13.7, 4.6 Hz, 1H), 1.76 (dt, J = 13.6, 2.4 Hz, 1H), 2.13-2.20 (m, 1H), 2.33 (dd, J = 13.5, 10.8 Hz, 1H), 2.90 (dd, J = 13.5, 6.6 Hz, 1H), 3.73 (dd, J = 6.6, 2.5 Hz, 1H), 4.05 (dq, J = 10.9, 2.3 Hz, 1H), 4.40 (d, J = 13.3 Hz, 1H), 4.52 (d, J = 12.8 Hz, 1H), 6.99 (td, J = 7.6, 1.7 Hz, 1H), 7.35 (td, J = 7.4, 1.1 Hz, 1H), 7.46 (d, J = 7.3 Hz, 1H), 7.81 (dd, J = 7.8, 1.4 Hz, 1H). |
| 1-1365 | | Colorless liquid | 1.08 (d, J = 2.3 Hz, 3H), 1.10 (d, J = 2.3 Hz, 3H), 1.46 (s, 3H), 1.60-1.70 (m, 2H), 2.10-2.19 (m, 1H), 2.31 (dd, J = 13.5, 10.8 Hz, 1H), 2.84 (dd, J = 13.7, 6.9 Hz, 1H), 3.62 (dd, J = 6.9, 2.3 Hz, 1H), 4.03 (dq, J = 10.6, 2.3 Hz, 1H), 4.37 (d, J = 12.4 Hz, 1H), 4.52 (d, J = 12.4 Hz, 1H), 7.08 (t, J = 7.8 Hz, 1H), 7.29 (d, J = 7.8 Hz, 1H), 7.61 (d, J = 7.8 Hz, 1H), 7.69 (s, 1H). |
| 1-1366 | | Colorless liquid | 1.07 (d, J = 1.8 Hz, 3H), 1.09 (d, J = 2.3 Hz, 3H), 1.44 (s, 3H), 1.60-1.70 (m, 2H), 2.11-2.19 (m, 1H), 2.30 (dd, J = 13.5, 10.8 Hz, 1H), 2.83 (dd, J = 13.3, 6.9 Hz, 1H), 3.61 (dd, J = 6.6, 2.5 Hz, 1H), 4.03 (dq, J = 10.9, 2.3 Hz, 1H), 4.37 (d, J = 12.8 Hz, 1H), 4.52 (d, J = 12.4 Hz, 1H), 7.08 (d, J = 7.8 Hz, 2H), 7.67 (d, J = 8.7 Hz, 2H). |

[Table 110]

| Table 4-Continued | | | |
|---|---|---|---|
| No. | Physical property (melting point °C) | Form | $^1$H NMR spectrum σ ppm: |
| 1-1367 | | Colorless liquid | 1.07-1.13 (m, 6H), 1.47 (s, 3H), 1.65 (dd, J = 13.5, 4.8 Hz, 1H), 1.71 (dt, J = 13.6, 2.4 Hz, 1H), 2.11-2.18 (m, 1H), 2.31 (dd, J = 13.7, 11.0 Hz, 1H), 2.87 (dd, J = 13.5, 6.6 Hz, 1H), 3.68 (dd, J = 6.9, 2.3 Hz, 1H), 4.03 (dq, J = 10.6, 2.3 Hz, 1H), 4.61 (d, J = 13.3 Hz, 1H), 4.72 (d, J = 13.3 Hz, 1H), 7.36 (t, J = 7.8 Hz, 1H), 7.54 (t, J = 7.6 Hz, 1H), 7.62 (d, J = 7.8 Hz, 1H), 7.72 (d, J = 7.8 Hz, 1H). |
| 1-1368 | | Colorless liquid | 1.08-1.14 (m, 6H), 1.47 (s, 3H), 1.65 (dd, J = 13.7, 4.6 Hz, 1H), 1.71 (dt, J = 13.3, 2.3 Hz, 1H), 2.15-2.17 (m, 1H), 2.32 (dd, J = 13.7, 11.0 Hz, 1H), 2.87 (dd, J = 13.3, 6.9 Hz, 1H), 3.66 (dd, J = 6.6, 2.5 Hz, 1H), 4.04 (dq, J = 10.9, 2.3 Hz, 1H), 4.48 (d, J = 12.4 Hz, 1H), 4.63 (d, J = 6.2 Hz, 1H), 7.41-7.55 (m, 3H), 7.59 (s, 1H). |
| 1-1369 | | Colorless liquid | 1.09 (dd, J = 7.1, 2.1 Hz, 6H), 1.47 (s, 3H), 1.64 (dd, J = 19.7, 9.8 Hz, 1H), 1.70 (dt, J = 18.3, 9.2 Hz, 1H), 2.13-2.17 (m, 1H), 2.32 (td, J = 9.8, 4.0 Hz, 1H), 2.87 (dd, J = 13.3, 6.9 Hz, 1H), 3.65 (dd, J = 6.6, 2.5 Hz, 1H), 4.04 (dq, J = 10.5, 2.3 Hz, 1H), 4.49 (d, J = 12.8 Hz, 1H), 4.64 (d, J = 12.8 Hz, 1H), 7.45 (d, J = 7.8 Hz, 2H), 7.60 (d, J = 8.2 Hz, 2H). |
| 1-1370 | | Light orange liquid | 1.06 (d, J = 3.2 Hz, 3H), 1.08 (d, J = 3.2 Hz, 3H), 1.47 (s, 3H), 1.67 (dd, J = 13.7, 4.6 Hz, 1H), 1.73 (dt, J = 13.3, 2.3 Hz, 1H), 2.11-2.18 (m, 1H), 2.32 (dd, J = 13.7, 10.5 Hz, 1H), 2.90 (dd, J = 13.5, 6.6 Hz, 1H), 3.73 (dd, J = 6.9, 2.3 Hz, 1H), 4.03 (dq, J = 11.0, 2.3 Hz, 1H), 4.60 (d, J = 12.8 Hz, 1H), 4.72 (d, J = 12.4 Hz, 1H), 7.35-7.39 (m, 1H), 7.57-7.59 (m, 2H), 7.63 (d, J = 7.3 Hz, 1H). |
| 1-1371 | 71.2-74.0 | White solid | 1.06 (d, J = 2.7 Hz, 3H), 1.08 (d, J = 2.7 Hz, 3H), 1.45 (s, 3H), 1.61-1.70 (m, 2H), 2.11-2.18 (m, 1H), 2.31 (dd, J = 13.7, 11.0 Hz, 1H), 2.86 (dd, J = 13.7, 6.9 Hz, 1H), 3.64 (dd, J = 6.6, 2.5 Hz, 1H), 4.03 (dq, J = 10.9, 2.3 Hz, 1H), 4.44 (d, J = 12.8 Hz, 1H), 4.58 (d, J = 12.4 Hz, 1H), 7.43 (t, J = 7.8 Hz, 1H), 7.54-7.57 (m, 2H), 7.62 (s, 1H). |
| 1-1372 | | Colorless liquid | 1.06 (d, J = 2.3 Hz, 3H), 1.08 (d, J = 2.3 Hz, 3H), 1.45 (s, 3H), 1.61-1.70 (m, 2H), 2.10-2.17 (m, 1H), 2.31 (dd, J = 13.5, 10.8 Hz, 1H), 2.86 (dd, J = 13.5, 6.6 Hz, 1H), 3.64 (dd, J = 6.9, 2.7 Hz, 1H), 4.03 (dq, J = 10.9, 2.3 Hz, 1H), 4.47 (d, J = 13.3 Hz, 1H), 4.61 (d, J = 13.3 Hz, 1H), 7.43 (d, J = 7.8 Hz, 2H), 7.62 (d, J = 8.2 Hz, 2H). |
| 1-1376 | | Yellow liquid | 1.08 (d, J = 2.7 Hz, 3H), 1.10 (d, J = 2.7 Hz, 3H), 1.56 (s, 3H), 1.67 (dd, J = 13.7, 4.6 Hz, 2H), 1.74 (dt, J = 13.4, 2.5 Hz, 1H), 2.12-2.19 (m, 1H), 2.32 (dd, J = 13.5, 10.8 Hz, 1H), 2.90 (dd, J = 13.5, 6.6 Hz, 1H), 3.73 (dd, J = 6.6, 2.5 Hz, 1H), 3.90 (s, 2H), 4.01-4.08 (m, 1H), 4.84 (d, J = 14.7 Hz, 1H), 4.96 (d, J = 14.7 Hz, 1H), 7.33 (t, J = 7.3 Hz, 1H), 7.53 (t, J = 6.9 Hz, 1H), 7.73 (d, J = 8.2 Hz, 1H), 7.95 (d, J = 6.9 Hz, 1H). |
| 1-1379 | | Colorless liquid | 1.06 (dd, J = 7.1, 3.0 Hz, 6H), 1.36 (s, 3H), 1.63 (dd, J = 13.5, 4.8 Hz, 1H), 1.70 (dt, J = 13.3, 2.3 Hz, 1H), 2.09-2.16 (m, 1H), 2.28 (dd, J = 13.5, 10.8 Hz, 1H), 2.85 (dd, J = 13.5, 6.6 Hz, 1H), 3.63 (dd, J = 6.6, 2.5 Hz, 1H), 4.00 (dq, J = 10.8, 2.4 Hz, 1H), 4.55 (dd, J = 38.9, 11.0 Hz, 2H), 6.87 (t, J = 7.8 Hz, 2H), 7.26 (dd, J = 29.8, 14.9 Hz, 1H). |

[Table 111]

| Table 4-Continued | | | |
|---|---|---|---|
| No. | Physical property (melting point °C) | Form | $^1$H NMR spectrum σ ppm: |
| 1-1380 | | Colorless liquid | 1.07 (d, J = 0.9 Hz, 3H), 1.09 (d, J = 1.4 Hz, 3H), 1.41 (s, 3H), 1.65 (dd, J = 13.5, 4.8 Hz, 1H), 1.77-1.82 (m, 1H), 2.12-2.18 (m, 1H), 2.30 (dd, J = 13.5, 10.8 Hz, 1H), 2.89 (dd, J = 13.5, 6.6 Hz, 1H), 3.64 (dd, J = 6.4, 2.3 Hz, 1H), 4.02-4.03 (m, 1H), 4.68 (d, J = 10.5 Hz, 1H), 4.78 (d, J = 10.5 Hz, 1H), 7.17-7.20 (m, 1H), 7.31-7.31 (m, 2H). |
| 1-1381 | | Colorless liquid | 1.07 (d, J = 7.2 Hz, 3H), 1.08 (d, J = 7.2 Hz, 3H), 1.39 (s, 3H), 1.64 (dd, J = 13.5, 4.8 Hz, 1H), 1.73-1.77 (m, 1H), 2.11-2.18 (m, 1H), 2.30 (dd, J = 13.5, 10.8 Hz, 1H), 2.87 (dd, J = 13.5, 6.6 Hz, 1H), 3.64 (dd, J = 6.4, 2.3 Hz, 1H), 4.01-4.03 (m, 1H), 4.58 (dd, J = 10.5, 1.8 Hz, 1H), 4.68 (dd, J = 10.5, 2.3 Hz, 1H), 6.98-7.01 (m, 1H), 7.20-7.23 (m, 2H). |
| 1-1383 | | Colorless liquid | 1.07 (dd, J = 20.0, 10.4 Hz, 6H), 1.41 (s, 3H), 1.58 (dd, J = 13.1, 4.4 Hz, 1H), 1.75 (dt, J = 13.3, 2.3 Hz, 1H), 2.13-2.18 (m, 1H), 2.26 (dd, J = 13.5, 10.8 Hz, 1H), 2.81 (dd, J = 28.9, 14.4 Hz, 1H), 3.67 (dd, J = 6.9, 2.3 Hz, 1H), 4.01 (dq, J = 10.6, 2.3 Hz, 1H), 4.83 (d, J = 12.8 Hz, 1H), 5.07 (d, J = 12.8 Hz, 1H), 7.41-7.53 (m, 4H), 7.81 (d, J = 8.2 Hz, 1H), 7.84-7.87 (m, 1H), 8.12 (dd, J = 7.6, 5.7 Hz, 1H). |
| 1-1384 | | Colorless liquid | 1.08 (dd, J = 7.1, 2.1 Hz, 6H), 1.47 (s, 3H), 1.60 (dd, J = 13.5, 4.8 Hz, 1H), 1.74 (dt, J = 13.3, 2.3 Hz, 1H), 2.15-2.16 (m, 1H), 2.28 (dd, J = 13.5, 10.8 Hz, 1H), 2.84 (dd, J = 13.5, 6.6 Hz, 1H), 3.66 (dd, J = 6.9, 2.3 Hz, 1H), 4.02 (dq, J = 10.8, 2.3 Hz, 1H), 4.58 (d, J = 12.4 Hz, 1H), 4.74 (d, J = 12.8 Hz, 1H), 7.44-7.48 (m, 3H), 7.76 (s, 1H), 7.81-7.83 (m, 3H). |
| 1-1385 | | Colorless liquid | 1.07 (dd, J = 6.9, 2.7 Hz, 6H), 1.45 (s, 3H), 1.64-1.67 (m, 1H), 1.68-1.72 (m, 1H), 2.11-2.18 (m, 1H), 2.31 (dd, J = 13.7, 11.0 Hz, 1H), 2.88 (dd, J = 13.5, 6.6 Hz, 1H), 3.68 (dd, J = 6.6, 2.5 Hz, 1H), 4.03 (dq, J = 10.6, 2.3 Hz, 1H), 4.52 (dd, J = 45.6, 13.5 Hz, 2H), 7.23 (dd, J = 8.2, 2.3 Hz, 1H), 7.35 (d, J = 2.3 Hz, 1H), 7.44 (d, J = 8.2 Hz, 1H). |
| 1-1386 | 93.4-96.0 | White solid | 1.09 (dd, J = 6.9, 1.8 Hz, 6H), 1.49 (s, 3H), 1.66 (dd, J = 13.7, 4.6 Hz, 1H), 1.73 (dt, J = 13.3, 2.3 Hz, 1H), 2.11-2.21 (m, 1H), 2.32 (dd, J = 13.5, 10.8 Hz, 1H), 2.89 (dd, J = 13.5, 6.6 Hz, 1H), 3.73 (dd, J = 6.9, 2.3 Hz, 1H), 4.04 (dq, J = 11.0, 2.3 Hz, 1H), 4.59 (d, J = 13.7 Hz, 1H), 4.69 (d, J = 13.7 Hz, 1H), 7.17-7.21 (m, 1H), 7.49 (d, J = 8.2 Hz, 1H), 7.70 (td, J = 7.7, 1.7 Hz, 1H), 8.53 (d, J = 4.6 Hz, 1H). |
| 1-1389 | | Yellow liquid | 1.07 (d, J = 1.4 Hz, 3H), 1.09 (d, J = 1.4 Hz, 3H), 1.42 (s, 3H), 1.62 (dd, J = 13.3, 4.6 Hz, 1H), 1.67 (dt, J = 13.3, 2.4 Hz, 1H), 2.11-2.17 (m, 1H), 2.29 (dd, J = 13.3, 10.8 Hz, 1H), 2.81 (dd, J = 13.3, 6.9 Hz, 1H), 3.67 (dd, J = 6.6, 2.4 Hz, 1H), 4.02 (dq, J = 10.8, 2.4 Hz, 1H), 4.61 (d, J = 12.8 Hz, 1H), 4.74 (d, J = 13.3 Hz, 1H), 6.97-6.99 (m, 2H), 7.28 (dd, J = 4.4, 1.6 Hz, 1H). |
| 1-1390 | | Pale yellow liquid | 1.07 (d, J = 1.4 Hz, 3H), 1.09 (d, J = 2.3 Hz, 3H), 1.43 (s, 3H), 1.63-1.66 (m, 2H), 2.09-2.18 (m, 1H), 2.30 (dd, J = 13.5, 10.8 Hz, 1H), 2.82 (dd, J = 13.3, 6.9 Hz, 1H), 3.63 (dd, J = 6.6, 2.5 Hz, 1H), 4.02 (dq, J = 10.6, 2.3 Hz, 1H), 4.46 (d, J = 12.4 Hz, 1H), 4.58 (d, J = 12.4 Hz, 1H), 7.06 (dd, J = 4.8, 1.1 Hz, 1H), 7.19 (dd, J = 2.7, 0.9 Hz, 1H), 7.30 (dd, J = 5.0, 2.7 Hz, 1H). |

[Table 112]

| [Table 4] Continued | | | |
|---|---|---|---|
| No. | Physical property (melting point °C) | Form | $^1$H NMR spectrum σ ppm: |
| 1-1391 | | Colorless liquid | 1.07 (d, J = 2.7 Hz, 3H), 1.09 (d, J = 2.7 Hz, 3H), 1.38 (s, 3H), 1.61-1.66 (m, 2H), 2.11-2.18 (m, 1H), 2.26 (s, 3H), 2.31 (dd, J = 13.3, 10.8 Hz, 1H), 2.38 (s, 3H), 2.84 (dd, J = 13.3, 6.6 Hz, 1H), 3.55 (dd, J = 6.6, 2.2 Hz, 1H), 4.03 (dq, J = 10.8, 2.2 Hz, 1H), 4.16 (d, J = 12.4 Hz, 1H), 4.32 (d, J = 12.4 Hz, 1H). |
| 1-1400 | | Colorless liquid | 1.07 (d, J = 4.1 Hz, 3H), 1.08 (d, J = 4.1 Hz, 3H), 1.40 (s, 3H), 1.65 (s, 3H), 1.73 (s, 3H), 1.69-1.74 (m, 2H), 2.11-2.18 (m, 1H), 2.26 (dd, J = 13.7, 11.0 Hz, 1H), 2.80 (dd, J = 13.3, 6.9 Hz, 1H), 3.51 (dd, J = 6.9, 2.3 Hz, 1H), 3.85-4.00 (m, 3H), 5.30 (tt, J = 6.9, 1.4 Hz, 1H). |
| 1-1402 | | Pale yellow liquid | 1.07 (d, J = 1.7 Hz, 3H), 1.08 (d, J = 2.3 Hz, 3H), 1.40 (s, 3H), 1.71-1.77 (m, 1H), 1.85 (t, J = 2.3 Hz, 3H), 1.69-1.74 (m, 2H), 2.10-2.17 (m, 1H), 2.27 (dd, J = 13.7, 11.0 Hz, 1H), 2.80 (dd, J = 13.5, 6.6 Hz, 1H), 3.72 (dd, J = 6.6, 2.5 Hz, 1H), 3.72 (dq, J = 11.0, 2.6 Hz, 1H), 4.01-4.15 (m, 1H). |
| 1-1408 | | Colorless liquid | 1.08 (d, J = 2.7 Hz, 3H), 1.10 (d, J = 3.2 Hz, 3H), 1.45 (s, 3H), 1.68 (dd, J = 13.7, 5.5 Hz, 1H), 1.80 (dt, J = 13.6, 2.5 Hz, 1H), 2.13-2.20 (m, 1H), 2.26 (dd, J = 13.7, 11.0 Hz, 1H), 2.81 (dd, J = 13.5, 6.6 Hz, 1H), 3.58 (dd, J = 6.9, 2.3 Hz, 1H), 3.93 (dq, J = 10.9, 2.6 Hz, 1H), 4.43 (d, J = 12.4 Hz, 1H), 4.57 (d, J = 12.4 Hz, 1H), 7.25-7.36 (m, 5H). |
| 1-1409 | | Colorless liquid | 1.08 (d, J = 3.7 Hz, 3H), 1.10 (d, J = 4.1 Hz, 3H), 1.44 (s, 3H), 1.70 (dd, J = 13.5, 5.3 Hz, 1H), 1.81 (dt, J = 13.4, 2.4 Hz, 1H), 2.13-2.20 (m, 1H), 2.27 (dd, J = 13.7, 11.0 Hz, 1H), 2.31 (s, 3H), 2.82 (dd, J = 13.5, 6.6 Hz, 1H), 3.58 (dd, J = 6.6, 2.5 Hz, 1H), 3.94 (dq, J = 11.0, 2.6 Hz, 1H), 4.38 (d, J = 12.4 Hz, 1H), 4.55 (d, J = 12.4 Hz, 1H), 7.14-7.21 (m, 3H), 7.31-7.33 (m, 1H). |
| 1-1415 | | Colorless liquid | 1.08 (d, J = 3.2 Hz, 3H), 1.10 (d, J = 3.2 Hz, 3H), 1.46 (s, 3H), 1.71 (dd, J = 13.5, 5.3 Hz, 1H), 1.83 (dt, J = 13.4, 2.5 Hz, 1H), 2.12-2.19 (m, 1H), 2.27 (dd, J = 13.7, 11.0 Hz, 1H), 2.84 (dd, J = 13.7, 6.9 Hz, 1H), 3.62 (dd, J = 6.6, 2.5 Hz, 1H), 3.81 (s, 3H), 3.94 (dq, J = 10.8, 2.6 Hz, 1H), 4.48 (d, J = 13.3 Hz, 1H), 4.57 (d, J = 13.3 Hz, 1H), 6.84 (d, J = 8.2 Hz, 1H), 6.94 (t, J = 7.6 Hz, 1H), 7.22-7.26 (m, 1H), 7.40 (d, J = 7.3 Hz, 1H). |
| 1-1418 | | Colorless liquid | 1.08 (d, J = 4.6 Hz, 3H), 1.10 (d, J = 5.0 Hz, 3H), 1.46 (s, 3H), 1.72 (dd, J = 13.7, 5.5 Hz, 1H), 1.81 (dt, J = 13.6, 2.5 Hz, 1H), 2.13-2.20 (m, 1H), 2.29 (dd, J = 13.3, 11.0 Hz, 1H), 2.86 (dd, J = 13.5, 6.6 Hz, 1H), 3.62 (dd, J = 6.6, 2.5 Hz, 1H), 3.94 (dq, J = 11.0, 2.6 Hz, 1H), 4.50 (d, J = 13.3 Hz, 1H), 4.60 (d, J = 13.3 Hz, 1H), 7.20-7.33 (m, 3H), 7.51-7.55 (m, 1H). |
| 1-1421 | | Colorless liquid | 1.08 (d, J = 4.1 Hz, 3H), 1.10 (d, J = 4.6 Hz, 3H), 1.44 (s, 3H), 1.71 (dd, J = 13.5, 5.3 Hz, 1H), 1.81 (d, J = 13.7 Hz, 1H), 2.12-2.19 (m, 1H), 2.28 (dd, J = 13.3, 11.0 Hz, 1H), 2.84 (dd, J = 13.5, 6.6 Hz, 1H), 3.61 (dd, J = 6.9, 2.3 Hz, 1H), 3.94 (dq, J = 10.8, 2.6 Hz, 1H), 4.50 (d, J = 12.8 Hz, 1H), 4.60 (d, J = 12.8 Hz, 1H), 7.02 (t, J = 9.2 Hz, 1H), 7.12 (t, J = 7.3 Hz, 1H), 7.23-7.28 (m, 1H), 7.42 (t, J = 7.3 Hz, 1H). |

[Table 113]

| Table 4-Continued | | | |
|---|---|---|---|
| No. | Physical property (melting point °C) | Form | $^1H$ NMR spectrum σ ppm: |
| 1-1424 | | Colorless liquid | 1.09 (d, J = 4.1 Hz, 3H), 1.11 (d, J = 4.6 Hz, 3H), 1.48 (s, 3H), 1.73 (dd, J = 13.5, 5.3 Hz, 1H), 1.84 (dt, J = 13.6, 2.4 Hz, 1H), 2.13-2.20 (m, 1H), 2.29 (dd, J = 13.3, 11.0 Hz, 1H), 2.87 (dd, J = 13.5, 6.6 Hz, 1H), 3.65 (dd, J = 6.9, 2.3 Hz, 1H), 3.95 (dq, J = 11.0, 2.6 Hz, 1H), 4.51 (d, J = 13.3 Hz, 1H), 4.62 (d, J = 13.3 Hz, 1H), 7.19-7.27 (m, 2H), 7.32-7.34 (m, 1H), 7.50 (d, J = 7.8 Hz, 1H). |
| 1-1427 | | Colorless liquid | 1.09 (d, J = 4.6 Hz, 3H), 1.11 (d, J = 4.6 Hz, 3H), 1.48 (s, 3H), 1.73 (dd, J = 13.5, 5.3 Hz, 1H), 1.84 (dt, J = 13.4, 2.4 Hz, 1H), 2.12-2.21 (m, 1H), 2.29 (dd, J = 13.7, 11.0 Hz, 1H), 2.88 (dd, J = 13.5, 6.6 Hz, 1H), 3.66 (dd, J = 6.6, 2.5 Hz, 1H), 3.95 (dq, J = 11.0, 2.6 Hz, 1H), 4.47 (d, J = 13.3 Hz, 1H), 4.58 (d, J = 13.3 Hz, 1H), 7.11-7.15 (m, 1H), 7.30 (t, J = 7.6 Hz, 1H), 7.50 (t, J = 7.8 Hz, 2H). |
| 1-1430 | | Colorless liquid | 1.09 (d, J = 4.6 Hz, 3H), 1.11 (d, J = 6.4 Hz, 3H), 1.49 (s, 3H), 1.74 (dd, J = 13.5, 5.3 Hz, 1H), 1.85 (dt, J = 13.4, 2.5 Hz, 1H), 2.12-2.21 (m, 1H), 2.30 (dd, J = 13.7, 11.0 Hz, 1H), 2.88 (dd, J = 13.5, 6.6 Hz, 1H), 3.67 (dd, J = 6.6, 2.5 Hz, 1H), 3.95 (dq, J = 11.0, 2.6 Hz, 1H), 4.38 (d, J = 13.3 Hz, 1H), 4.49 (d, J = 12.8 Hz, 1H), 6.97 (td, J = 7.7, 1.5 Hz, 1H), 7.33 (td, J = 7.6, 1.2 Hz, 1H), 7.45 (dd, J = 7.8, 1.4 Hz, 1H), 7.79 (dd, J = 7.8, 0.9 Hz, 1H). |
| 1-1433 | | Colorless liquid | 1.09 (d, J = 4.6 Hz, 3H), 1.10 (d, J = 5.0 Hz, 3H), 1.48 (s, 3H), 1.72 (dd, J = 13.5, 5.3 Hz, 1H), 1.82 (dt, J = 13.6, 2.4 Hz, 1H), 2.12-2.20 (m, 1H), 2.29 (dd, J = 13.3, 11.0 Hz, 1H), 2.86 (dd, J = 13.5, 6.6 Hz, 1H), 3.63 (dd, J = 6.9, 2.3 Hz, 1H), 3.95 (dq, J = 10.9, 2.7 Hz, 1H), 4.61 (d, J = 13.3 Hz, 1H), 4.71 (d, J = 13.7 Hz, 1H), 7.36 (t, J = 7.6 Hz, 1H), 7.53 (t, J = 7.6 Hz, 1H), 7.62 (d, J = 8.2 Hz, 1H), 7.71 (d, J = 7.8 Hz, 1H). |
| 1-1436 | | Colorless liquid | 1.09 (d, J = 6.4 Hz, 3H), 1.10 (d, J = 6.4 Hz, 3H), 1.48 (s, 3H), 1.74 (dd, J = 13.5, 5.3 Hz, 1H), 1.84 (dt, J = 13.6, 2.5 Hz, 1H), 2.13-2.20 (m, 1H), 2.30 (dd, J = 13.3, 11.0 Hz, 1H), 2.90 (dd, J = 13.5, 6.6 Hz, 1H), 3.69 (dd, J = 6.9, 2.3 Hz, 1H), 3.95 (dq, J = 11.0, 2.6 Hz, 1H), 4.60 (d, J = 12.8 Hz, 1H), 4.71 (d, J = 12.8 Hz, 1H), 7.34-7.40 (m, 1H), 7.55-7.60 (m, 2H), 7.64 (d, J = 7.8 Hz, 1H). |
| 1-1442 | | Colorless liquid | 1.09 (t, J = 6.4 Hz, 6H), 1.49 (s, 3H), 1.73 (dd, J = 13.3, 5.0 Hz, 1H), 1.83 (d, J = 13.7 Hz, 1H), 2.13-2.20 (m, 1H), 2.29 (dd, J = 13.3, 11.0 Hz, 1H), 2.88 (dd, J = 13.5, 6.6 Hz, 1H), 3.67 (dd, J = 6.4, 1.8 Hz, 1H), 3.88 (s, 3H), 3.93-3.97 (m, 1H), 4.82 (d, J = 14.7 Hz, 1H), 4.93 (d, J = 14.7 Hz, 1H), 7.32 (t, J = 7.6 Hz, 1H), 7.52 (t, J = 7.3 Hz, 1H), 7.71 (d, J = 7.8 Hz, 1H), 7.93 (d, J = 7.8 Hz, 1H). |
| 1-1445 | | Colorless liquid | 1.09 (d, J = 6.9 Hz, 6H), 1.38 (s, 3H), 1.71 (dd, J = 13.5, 5.3 Hz, 1H), 1.84 (dt, J = 13.3, 2.3 Hz, 1H), 2.12-2.19 (m, 1H), 2.26 (dd, J = 13.5, 10.8 Hz, 1H), 2.38 (s, 6H), 2.85 (dd, J = 13.3, 6.9 Hz, 1H), 3.53 (dd, J = 6.4, 2.3 Hz, 1H), 3.94 (dq, J = 11.0, 2.6 Hz, 1H), 4.41 (d, J = 10.5 Hz, 1H), 4.54 (d, J = 10.5 Hz, 1H), 7.00 (d, J = 7.8 Hz, 2H), 7.09 (t, J = 7.6 Hz, 1H). |
| 1-1446 | | Colorless liquid | 1.07 (d, J = 4.1 Hz, 3H), 1.08 (d, J = 4.1 Hz, 3H), 1.37 (s, 3H), 1.70 (dd, J = 13.5, 5.3 Hz, 1H), 1.80 (dt, J = 13.4, 2.4 Hz, 1H), 2.11-2.18 (m, 1H), 2.26 (dd, J = 13.7, 11.0 Hz, 1H), 2.84 (dd, J = 13.5, 6.6 Hz, 1H), 3.58 (dd, J = 6.6, 2.1 Hz, 1H), 3.92 (dq, J = 10.9, 2.7 Hz, 1H), 4.49 (d, J = 11.0 Hz, 1H), 4.58 (d, J = 10.5 Hz, 1H), 6.84-6.90 (m, 2H), 7.22-7.29 (m, 1H). |

[Table 114]

| Table 4-Continued | | | |
|---|---|---|---|
| No. | Physical property (melting point °C) | Form | $^1$H NMR spectrum σ ppm: |
| 1-1447 | | Colorless liquid | 1.08 (d, J = 6.9 Hz, 6H), 1.40 (s, 3H), 1.71 (dd, J = 13.5, 5.3 Hz, 1H), 1.89 (dt, J = 13.7, 2.3 Hz, 1H), 2.12-2.19 (m, 1H), 2.26 (dd, J = 13.3, 11.0 Hz, 1H), 2.86 (dd, J = 13.7, 6.4 Hz, 1H), 3.58 (dd, J = 6.4, 2.3 Hz, 1H), 3.93 (dq, J = 10.9, 2.6 Hz, 1H), 4.65 (d, J = 10.5 Hz, 1H), 4.75 (d, J = 10.5 Hz, 1H), 7.17 (t, J = 8.0 Hz, 1H), 7.29 (d, J = 7.8 Hz, 2H). |
| 1-1448 | | Colorless liquid | 1.07 (d, J = 3.2 Hz, 3H), 1.09 (d, J = 3.2 Hz, 3H), 1.38 (s, 3H), 1.70 (dd, J = 13.3, 5.5 Hz, 1H), 1.84 (dt, J = 13.6, 2.4 Hz, 1H), 2.11-2.18 (m, 1H), 2.26 (dd, J = 13.3, 11.0 Hz, 1H), 2.84 (dd, J = 13.7, 6.4 Hz, 1H), 3.58 (dd, J = 6.9, 2.3 Hz, 1H), 3.93 (dq, J = 10.9, 2.6 Hz, 1H), 4.55 (dd, J = 10.5, 1.8 Hz1H), 4.65 (dd, J = 10.5, 2.3 Hz, 1H), 6.96-7.00 (m, 1H), 7.17-7.24 (m, 2H). |
| 1-1456 | | Yellow liquid | 1.09 (d, J = 1.8 Hz, 3H), 1.11 (d, J = 2.3 Hz, 3H), 1.43 (s, 3H), 1.69 (dd, J = 13.3, 5.4 Hz, 1H), 1.78 (dt, J = 13.3, 3.1 Hz, 1H), 2.13-2.20 (m, 1H), 2.27 (dd, J = 13.3, 11.4 Hz, 1H), 2.80 (dd, J = 13.3, 6.2 Hz, 1H), 3.62 (dd, J = 6.2, 2.6 Hz, 1H), 3.94 (dq, J = 11.0, 2.6 Hz, 1H), 4.61 (d, J = 12.4 Hz, 1H), 4.73 (d, J = 12.4 Hz, 1H), 6.96-7.00 (m, 2H), 7.28 (dd, J = 4.1, 1.4 Hz, 1H). |
| 1-1457 | | Yellow liquid | 1.09 (d, J = 3.7 Hz, 3H), 1.11 (d, J = 3.7 Hz, 3H), 1.44 (s, 3H), 1.70 (dd, J = 13.3, 5.3 Hz, 1H), 1.77 (dt, J = 13.3, 2.4 Hz, 1H), 2.09-2.20 (m, 1H), 2.27 (dd, J = 13.3, 11.0 Hz, 1H), 2.81 (dd, J = 13.3, 6.6 Hz, 1H), 3.58 (dd, J = 6.6, 2.6 Hz, 1H), 3.94 (dq, J = 11.0, 2.6 Hz, 1H), 4.45 (d, J = 12.4 Hz, 1H), 4.57 (d, J = 12.4 Hz, 1H), 7.05 (dd, J = 5.0, 0.9 Hz, 1H), 7.19 (dd, J = 2.7, 0.9 Hz, 1H), 7.30 (dd, J = 5.0, 2.7 Hz, 1H). |
| 1-1555 | 41.3-43.6 | White solid | 0.90 (dd, J = 12.4, 5.5 Hz, 1H), 0.97 (dd, J = 15.3, 7.1 Hz, 6H), 1.02 (d, J = 6.9 Hz, 3H), 1.41 (s, 3H), 1.50 (dt, J = 13.3, 2.7 Hz, 1H), 1.87 (t, J = 11.9 Hz, 1H), 2.02-2.16 (m, 2H), 2.31 (dd, J = 13.3, 6.9 Hz, 1H), 3.53 (dd, J = 6.9, 2.7 Hz, 1H), 4.54 (dd, J = 39.8, 12.8 Hz, 2H), 7.00 (t, J = 9.2 Hz, 1H), 7.11 (t, J = 7.6 Hz, 1H), 7.22-7.25 (m, 1H), 7.45 (t, J = 7.6 Hz, 1H). |
| 1-1597 | | Colorless liquid | 0.86 (dd, J = 14.9, 7.1 Hz, 6H), 1.02-1.03 (m, 4H), 1.44 (s, 3H), 1.67-1.77 (m, 3H), 1.80-1.81 (m, 1H), 2.15-2.16 (m, 1H), 3.49 (dd, J = 6.2, 3.0 Hz, 1H), 4.54 (dd, J = 36.6, 12.8 Hz, 2H), 7.00 (t, J = 9.2 Hz, 1H), 7.12 (t, J = 7.6 Hz, 1H), 7.23-7.25 (m, 1H), 7.44 (t, J = 7.6 Hz, 1H). |
| 1-1598 | | Colorless liquid | 1.07 (d, J = 7.2 Hz, 3H), 1.09 (d, J = 7.2 Hz, 3H), 1.40 (s, 3H), 1.65 (dd, J = 13.5, 4.8 Hz, 1H), 1.75-1.78 (m, 1H), 2.13-2.17 (m, 1H), 2.30 (dd, J = 13.6, 10.8 Hz, 1H), 2.87 (dd, J = 13.6, 6.9 Hz, 1H), 3.64 (dd, J = 6.4, 2.3 Hz, 1H), 4.01-4.04 (m, 1H), 4.57 (dd, J = 10.7, 5.3 Hz, 1H), 4.68 (dd, J = 10.7, 5.4 Hz, 1H), 7.02-7.04 (m, 1H), 7.16-7.18 (m, 1H), 7.37-7.39 (m, 1H). |
| 1-1599 | | Colorless liquid | 1.08 (d, J = 6.9 Hz, 6H), 1.39 (s, 3H), 1.65 (dd, J = 13.5, 4.8 Hz, 1H), 1.72-1.75 (m, 1H), 2.12-2.15 (m, 1H), 2.29 (dd, J = 13.5, 10.8 Hz, 1H), 2.39 (s, 6H), 2.87 (dd, J = 13.3, 6.9 Hz, 1H), 3.57-3.59 (m, 1H), 4.01-4.04 (m, 1H), 4.43 (d, J = 10.5 Hz, 1H), 4.56 (d, J = 10.5 Hz, 1H), 7.00-7.02 (m, 2H), 7.10 (dd, J = 8.5, 6.6 Hz, 1H). |

[Table 115]

| Table 4-Continued | | | |
|---|---|---|---|
| No. | Physical property (melting point °C) | Form | $^1$H NMR spectrum σ ppm: |
| 1-1600 | | Colorless liquid | 1.07 (d, J = 7.2 Hz, 3H), 1.10 (d, J = 7.2 Hz, 3H), 1.40 (s, 3H), 1.65 (dd, J = 13.5, 4.8 Hz, 1H), 1.77 (dt, J = 13.5, 2.4 Hz, 1H), 2.13-2.16 (m, 1H), 2.30 (dd, J = 13.7, 11.0 Hz, 1H), 2.87 (dd, J = 13.6, 6.6 Hz, 1H), 3.64 (dd, J = 6.9, 2.3 Hz, 1H), 4.01-4.03 (m, 1H), 4.58 (dd, J = 10.5, 2.3 Hz, 1H), 4.68 (dd, J = 10.5, 2.3 Hz, 1H), 7.01-7.05 (m, 1H), 7.16-7.18 (m, 1H), 7.37-7.38 (m, 1H). |
| 1-1601 | | Colorless liquid | 1.08 (dd, J = 7.1, 2.5 Hz, 6H), 1.39 (s, 3H), 1.71 (dd, J = 13.5, 5.3 Hz, 1H), 1.86 (dt, J = 13.7, 6.9 Hz, 1H), 2.14-2.16 (m, 1H), 2.26 (dd, J = 13.3, 11.0 Hz, 1H), 2.85 (dd, J = 13.5, 6.6 Hz, 1H), 3.59 (dd, J = 3.2, 1.6 Hz, 1H), 3.91-3.95 (m, 1H), 4.55 (dd, J = 10.5, 1.8 Hz, 1H), 4.66 (dd, J = 10.5, 1.8 Hz, 1H), 7.02 (t, J = 8.7 Hz, 1H), 7.13-7.17 (m, 1H), 7.36 (d, J = 8.2 Hz, 1H). |
| 1-1602 | | Colorless liquid | 0.99 (dd, J = 6.9, 1.8 Hz, 6H), 1.28 (dd, J = 12.8, 3.2 Hz, 1H), 1.41 (s, 3H), 1.49 (d, J = 12.8 Hz, 1H), 1.86 (dd, J = 12.8, 9.4 Hz, 1H), 2.04-2.11 (m, 1H), 2.65 (dd, J = 12.8, 6.9 Hz, 1H), 3.65 (dd, J = 6.9, 2.7 Hz, 1H), 3.72-3.75 (m, 1H), 3.90 (dtt, J = 39.2, 7.9, 2.3 Hz, 2H), 4.55 (dd, J = 44.0, 12.8 Hz, 2H), 5.19 (ddd, J = 40.1, 13.7, 1.6 Hz, 2H), 5.81-5.91 (m, 1H), 7.00 (t, J = 9.2 Hz, 1H), 7.11 (t, J = 7.3 Hz, 1H), 7.21-7.26 (m, 1H), 7.44 (t, J = 7.3 Hz, 1H). |
| 1-1603 | | Colorless liquid | 0.15 (dd, J = 8.0, 1.6 Hz, 2H), 0.48 (dd, J = 8.0, 1.6 Hz, 2H), 0.99 (dd, J = 7.1, 2.1 Hz, 6H), 1.27 (dd, J = 12.8, 3.2 Hz, 2H), 1.41 (s, 3H), 1.48 (d, J = 12.8 Hz, 1H), 1.86 (dd, J = 12.8, 9.2 Hz, 1H), 2.03-2.10 (m, 1H), 2.64 (dd, J = 12.8, 6.9 Hz, 1H), 3.21 (ddd, J = 41.4, 10.3, 6.6 Hz, 2H), 3.68 (ddd, J = 24.3, 8.0, 2.1 Hz, 2H), 4.55 (dd, J = 44.0, 12.8 Hz, 2H), 7.00 (t, J = 8.7 Hz, 1H), 7.11 (t, J = 7.3 Hz, 1H), 7.23 (dd, J = 14.2, 5.0 Hz, 1H), 7.45 (t, J = 7.6 Hz, 1H). |
| 1-1604 | | Colorless liquid | 0.88 (dd, J = 9.2, 5.5 Hz, 3H), 0.99 (d, J = 6.9 Hz, 6H), 1.26 (dd, J = 12.4, 3.2 Hz, 1H), 1.41 (s, 3H), 1.45-1.56 (m, 3H), 1.85 (dd, J = 12.6, 9.4 Hz, 1H), 2.03-2.10 (m, 1H), 2.64 (dd, J = 12.8, 6.9 Hz, 1H), 3.28 (ddt, J = 36.3, 11.5, 4.5 Hz, 2H), 3.64 (dt, J = 11.6, 3.9 Hz, 2H), 4.55 (dd, J = 44.4, 12.8 Hz, 2H), 7.00 (t, J = 9.2 Hz, 1H), 7.11 (t, J = 7.3 Hz, 1H), 7.21-7.26 (m, 1H), 7.45 (dd, J = 7.3, 6.0 Hz, 1H). |
| 1-1605 | | Colorless liquid | 1.00 (dd, J = 6.9, 4.1 Hz, 6H), 1.42 (s, 3H), 1.44 (d, J = 13.3 Hz, 1H), 1.51 (s, 1H), 2.01 (d, J = 10.1 Hz, 1H), 2.10 (t, J = 6.9 Hz, 1H), 2.60 (dd, J = 13.3, 6.9 Hz, 1H), 3.44 (t, J = 9.2 Hz, 2H), 3.67 (dd, J = 6.9, 2.7 Hz, 1H), 4.49 (s, 1H), 4.56 (dd, J = 43.5, 12.8 Hz, 2H), 7.01 (t, J = 9.6 Hz, 1H), 7.12 (t, J = 7.6 Hz, 1H), 7.21-7.23 (m, 1H), 7.44 (t, J = 7.6 Hz, 1H). |
| 1-1606 | | Colorless liquid | 1.00 (dd, J = 8.7, 6.9 Hz, 6H), 1.33 (dd, J = 12.8, 3.2 Hz, 1H), 1.42 (s, 3H), 1.53 (t, J = 12.6 Hz, 1H), 1.87 (dd, J = 12.6, 9.4 Hz, 1H), 2.08 (td, J = 13.2, 6.1 Hz, 1H), 2.71 (dd, J = 12.8, 6.9 Hz, 1H), 3.66 (dd, J = 6.9, 2.3 Hz, 1H), 3.81 (dd, J = 9.2, 2.3 Hz, 1H), 4.49 (dd, J = 12.4, 6.0 Hz, 2H), 4.51 (dd, J = 79.7, 12.4 Hz, 2H), 7.00 (t, J = 9.2 Hz, 1H), 7.11 (t, J = 7.3 Hz, 1H), 7.21-7.22 (m, 1H), 7.28 (d, J = 7.3 Hz, 3H), 7.31-7.35 (m, 2H), 7.44 (t, J = 7.6 Hz, 1H). |
| 1-1607 | | Yellow liquid | 1.00 (dd, J = 7.1, 3.0 Hz, 6H), 1.33 (dd, J = 12.8, 3.0 Hz, 1H), 1.42 (s, 3H), 1.49 (d, J = 12.8 Hz, 1H), 1.91 (dd, J = 12.8, 6.4 Hz, 1H), 2.04-2.11 (m, 1H), 2.39 (t, J = 2.7 Hz, 1H), 2.61 (dd, J = 12.8, 6.9 Hz, 1H), 3.64 (dd, J = 6.9, 2.7 Hz, 1H), 3.95 (dd, J = 9.4, 1.6 Hz, 1H), 4.07 (ddd, J = 57.2, 16.0, 2.3 Hz, 2H), 4.55 (dd, J = 43.3, 12.6 Hz, 2H), 7.00 (t, J = 9.2 Hz, 1H), 7.11 (t, J = 7.3 Hz, 1H), 7.21-7.26 (m, 1H), 7.43 (t, J = 7.3 Hz, 1H). |

[Table 116]

| Table 4-Continued | | | |
|---|---|---|---|
| No. | Physical property (melting point °C) | Form | $^1$H NMR spectrum σ ppm: |
| 1-1608 | | Colorless liquid | 1.08 (d, J = 6.9 Hz, 6H), 1.39 (s, 3H), 1.65 (dd, J = 13.7, 4.6 Hz, 1H), 1.72-1.75 (m, 1H), 2.14-2.15 (m, 1H), 2.29 (dd, J = 13.5, 10.8 Hz, 1H), 2.40 (s, 6H), 2.87 (dd, J = 13.3, 6.4 Hz, 1H), 3.58 (dd, J = 3.2, 1.6 Hz, 1H), 4.01-4.03 (m, 1H), 4.43 (d, J = 11.0 Hz, 1H), 4.56 (d, J = 11.0 Hz, 1H), 7.01 (d, J = 7.8 Hz, 2H), 7.09-7.11 (m, 1H). |
| 1-1609 | | Colorless liquid | 1.07 (d, J = 1.4 Hz, 3H), 1.09 (d, J = 2.3 Hz, 3H), 1.39 (d, J = 7.3 Hz, 3H), 1.65 (dd, J = 13.5, 4.8 Hz, 1H), 1.77 (d, J = 13.3 Hz, 1H), 2.11-2.18 (m, 1H), 2.30 (dd, J = 13.3, 11.0 Hz, 1H), 2.87 (dd, J = 13.3, 6.9 Hz, 1H), 3.64 (dd, J = 5.0, 2.5 Hz, 1H), 4.01-4.03 (m, 1H), 4.58 (dd, J = 10.5, 5.3 Hz, 1H), 4.68 (dd, J = 10.5, 5.3 Hz, 1H), 7.03 (t, J = 8.7 Hz, 1H), 7.17 (dd, J = 14.7, 8.2 Hz, 1H), 7.38 (d, J = 7.8 Hz, 1H). |
| 1-1610 | | Colorless liquid | 1.05 (d, J = 6.9 Hz, 6H), 1.37 (s, 3H), 1.51 (dd, J = 13.5, 4.4 Hz, 1H), 1.64 (d, J = 13.3 Hz, 1H), 2.08-2.15 (m, 1H), 2.24 (dd, J = 13.3, 11.0 Hz, 1H), 2.39 (s, 6H), 2.79 (dd, J = 13.3, 6.4 Hz, 1H), 3.58 (dd, J = 6.6, 2.1 Hz, 1H), 3.99-4.03 (m, 1H), 4.49 (dd, J = 49.5, 10.5 Hz, 2H), 6.99-7.11 (m, 3H). |
| 1-1611 | | Colorless liquid | 1.05 (d, J = 6.9 Hz, 6H), 1.38 (s, 3H), 1.51 (dd, J = 13.5, 4.4 Hz, 1H), 1.67 (dt, J = 13.5, 2.2 Hz, 1H), 2.09-2.15 (m, 1H), 2.24 (dd, J = 13.5, 10.8 Hz, 1H), 2.80 (dd, J = 13.5, 6.6 Hz, 1H), 3.64 (dd, J = 6.9, 2.3 Hz, 1H), 4.01 (dq, J = 10.8, 2.1 Hz, 1H), 4.62 (ddd, J = 42.5, 10.6, 2.2 Hz, 2H), 7.00-7.04 (m, 1H), 7.14-7.17 (m, 1H), 7.36 (d, J = 7.8 Hz, 1H). |
| 1-1612 | | Colorless liquid | 1.07 (d, J = 2.3 Hz, 3H), 1.09 (d, J = 2.7 Hz, 3H), 1.39 (s, 3H), 1.71 (dd, J = 13.5, 5.3 Hz, 1H), 1.86 (dt, J = 13.6, 2.4 Hz, 1H), 2.12-2.19 (m, 1H), 2.26 (dd, J = 13.5, 10.8 Hz, 1H), 2.85 (dd, J = 13.5, 6.6 Hz, 1H), 3.59 (dd, J = 6.6, 2.1 Hz, 1H), 3.93 (dq, J = 10.8, 2.6 Hz, 1H), 4.55 (dd, J = 10.5, 1.8 Hz1H), 4.66 (dd, J = 10.5, 1.8 Hz1H), 7.02 (t, J = 8.7 Hz, 1H), 7.13-7.18 (m, 1H), 7.36 (d, J = 7.8 Hz, 1H). |
| 1-1613 | | Pale yellow liquid | 1.06 (d, J = 6.9 Hz, 6H), 1.39 (s, 3H), 1.52 (dd, J = 13.3, 4.6 Hz, 1H), 1.68 (d, J = 13.3 Hz, 1H), 2.11-2.14 (m, 1H), 2.25 (dd, J = 13.5, 10.8 Hz, 1H), 2.81 (dd, J = 13.5, 6.6 Hz, 1H), 3.65 (dd, J = 6.6, 2.1 Hz, 1H), 4.02 (dq, J = 10.9, 2.0 Hz, 1H), 4.63 (ddd, J = 42.5, 10.6, 1.9 Hz, 2H), 7.03 (t, J = 8.7 Hz, 1H), 7.17 (td, J = 8.0, 6.4 Hz, 1H), 7.38 (dd, J = 8.0, 0.7 Hz, 1H). |
| 1-1614 | | Colorless liquid | 1.08 (dd, J = 6.6, 2.1 Hz, 6H), 1.39 (s, 3H), 1.71 (dd, J = 13.5, 5.3 Hz, 1H), 1.86 (d, J = 13.3 Hz, 1H), 2.15-2.16 (m, 1H), 2.26 (dd, J = 12.1, 6.1 Hz, 1H), 2.85 (dd, J = 13.5, 6.6 Hz, 1H), 3.59 (d, J = 6.4 Hz, 1H), 3.93 (dd, J = 9.6, 4.1 Hz, 1H), 4.60 (dd, J = 43.0, 10.5 Hz, 2H), 7.02 (t, J = 8.7 Hz, 1H), 7.16 (dd, J = 14.4, 8.0 Hz, 1H), 7.36 (d, J = 7.8 Hz, 1H). |
| 1-1615 | | Colorless liquid | 0.96 (dd, J = 9.4, 7.1 Hz, 6H), 1.12 (t, J = 7.3 Hz, 3H), 1.24-1.27 (m, 1H), 1.42 (s, 3H), 1.55 (d, J = 12.8 Hz, 1H), 2.09-2.13 (m, 2H), 2.55 (dd, J = 13.1, 6.6 Hz, 1H), 3.16-3.23 (m, 2H), 3.63 (d, J = 4.6 Hz, 1H), 4.56 (dd, J = 38.0, 11.9 Hz, 2H), 4.85-4.88 (m, 1H), 6.99-7.03 (m, 1H), 7.12 (td, J = 7.6, 1.7 Hz, 1H), 7.24-7.26 (m, 2H), 7.44 (td, J = 7.6, 1.7 Hz, 1H). |

[Table 117]

| Table 5 | | |
|---|---|---|
| No. | Form | $^1$H NMR spectrum σ ppm: |
| 2-1 | Colorless liquid | 1.06 (d, J = 6.8 Hz, 3H), 1.07 (d, J = 6.8 Hz, 3H), 1.59 (s, 3H), 1.82 (dd, J = 13.6, 2.8 Hz, 1H), 1.97 (dd, J = 13.6, 6.8 Hz, 1H), 2.07 (d, J = 17.2 Hz, 1H), 2.17 (sept, J = 6.8 Hz, 1H), 2.23 (d, J = 17.2 Hz, 1H), 3.70 (dd, J = 6.8, 2.8 Hz, 1H), 4.45 (d, J = 12.4 Hz, 1H), 4.60 (d, J = 12.4 Hz, 1H), 7.26-7.38 (m, 5H). |
| 2-3 | Colorless liquid | 1.07 (d, J = 6.8 Hz, 3H), 1.08 (d, J = 6.8 Hz, 3H), 1.57 (s, 3H), 1.83 (dd, J = 14.0, 2.8 Hz, 1H), 1.98 (dd, J = 14.0, 6.8 Hz, 1H), 2.01-2.26 (m, 3H), 2.34 (s, 3H), 3.69 (dd, J = 6.4, 2.8 Hz, 1H), 4.41 (d, J = 12.4 Hz, 1H), 4.59 (d, J = 12.4 Hz, 1H), 7.10-7.25 (m, 3H), 7.29-7.33 (m, 1H). |
| 2-5 | Colorless liquid | 1.06 (d, J = 7.2 Hz, 3H), 1.07 (d, J = 6.8 Hz, 3H), 1.52 (s, 3H), 1.85 (dd, J = 13.6, 2.4 Hz, 1H), 2.00 (dd, J = 13.6, 6.8 Hz, 1H), 2.07 (d, J = 17.6 Hz, 1H), 2.18 (sept, J = 6.8 Hz, 1H), 2.22 (d, J = 17.6 Hz, 1H), 2.39 (s, 6H), 3.65 (dd, J = 6.8, 2.4 Hz, 1H), 4.46 (d, J = 10.4 Hz, 1H), 4.57 (d, J = 10.4 Hz, 1H), 6.99-7.04 (m, 2H), 7.08-7.14 (m, 1H). |
| 2-7 | Colorless liquid | 1.06 (d, J = 6.8 Hz, 3H), 1.07 (d, J = 6.8 Hz, 3H), 1.60 (s, 3H), 1.86 (dd, J = 13.6, 2.8 Hz, 1H), 1.99 (dd, J = 13.6, 6.4 Hz, 1H), 2.07 (d, J = 18.0 Hz, 1H), 2.17 (sept, J = 6.8 Hz, 1H), 2.23 (d, J = 18.0 Hz, 1H), 3.74 (dd, J = 6.4, 2.8 Hz, 1H), 3.82 (s, 3H), 4.51 (d, J = 12.8 Hz, 1H), 4.60 (d, J = 12.8 Hz, 1H), 6.86 (d, J = 8.4 Hz, 1H), 6.96 (t, J = 7.2 Hz, 1H), 7.27 (td, J = 7.2, 2.0 Hz, 1H), 7.40 (dd, J = 7.2, 2.0 Hz, 1H). |
| 2-9 | Colorless liquid | 1.06 (d, J = 6.8 Hz, 3H), 1.07 (d, J = 6.8 Hz, 3H), 1.58 (s, 3H), 1.83 (dd, J = 11.2, 2.8 Hz, 1H), 2.00 (dd, J = 9.2, 6.8 Hz, 1H), 2.04-2.27 (m, 3H), 3.73 (dd, J = 6.8, 2.8 Hz, 1H), 4.53 (d, J = 12.4 Hz, 1H), 4.63 (d, J = 12.4 Hz, 1H), 7.00-7.07 (m, 1H), 7.14 (td, J = 7.2, 0.8 Hz, 1H), 7.25-7.32 (m, 1H), 7.43 (td, J = 7.2, 1.6 Hz, 1H). |
| 2-11 | Colorless liquid | 1.07 (d, J = 6.8 Hz, 3H), 1.08 (d, J = 6.8 Hz, 3H), 1.61 (s, 3H), 1.87 (dd, J = 13.6, 2.8 Hz, 1H), 2.03 (dd, J = 13.6, 6.8 Hz, 1H), 2.09 (d, J = 17.2 Hz, 1H), 2.18 (sept, J = 6.8 Hz, 1H), 2.25 (d, J = 17.2 Hz, 1H), 3.77 (dd, J = 6.8, 2.8 Hz, 1H), 4.54 (d, J = 13.6 Hz, 1H), 4.65 (d, J = 13.6 Hz, 1H), 7.20-7.30 (m, 2H), 7.35 (dd, J = 7.2, 1.2 Hz, 1H), 7.48-7.52 (m, 1H). |
| 2-13 | Colorless liquid | 1.07 (d, J = 6.8 Hz, 3H), 1.08 (d, J = 6.8 Hz, 3H), 1.62 (s, 3H), 1.87 (dd, J = 13.6, 2.8 Hz, 1H), 2.04 (dd, J = 13.6, 6.4 Hz, 1H), 2.10 (d, J = 17.6 Hz, 1H), 2.18 (sept, J = 6.8 Hz, 1H), 2.25 (d, J = 17.6 Hz, 1H), 3.78 (dd, J = 6.4, 2.8 Hz, 1H), 4.51 (d, J = 13.2 Hz, 1H), 4.61 (d, J = 13.2 Hz, 1H), 7.16 (td, J = 7.6, 1.2 Hz, 1H), 7.32 (t, J = 7.6 Hz, 1H), 7.50 (dd, J = 7.6, 1.2 Hz, 1H), 7.54 (d, J = 7.6 Hz, 1H). |
| 2-14 | Colorless liquid | 1.067 (d, J = 6.8 Hz, 3H), 1.072 (d, J = 6.8 Hz, 3H), 1.58 (s, 3H), 1.83 (dd, J = 13.6, 2.8 Hz, 1H), 2.02 (dd, J = 13.6, 6.8 Hz, 1H), 2.00 (d, J = 17.2 Hz, 1H), 2.18 (sept, J = 6.8 Hz, 1H), 2.25 (d, J = 17.2 Hz, 1H), 3.74 (dd, J = 6.8, 2.8 Hz, 1H), 4.53 (dd, J = 12.4, 0.8 Hz, 1H), 4.63 (dd, J = 12.4, 0.8 Hz, 1H), 7.04-7.22 (m, 3H). |

[Table 118]

| Table 5-Continued | | |
|---|---|---|
| No. | Form | $^1$H NMR spectrum σ ppm: |
| 2-16 | Colorless liquid | 1.055 (d, J = 6.8 Hz, 3H), 1.062 (d, J = 6.8 Hz, 3H), 1.55 (s, 3H), 1.81 (dd, J = 13.6, 2.8 Hz, 1H), 2.00 (dd, J = 13.6, 6.4 Hz, 1H), 2.07 (d, J = 17.6 Hz, 1H), 2.16 (sept, J = 6.8 Hz, 1H), 2.23 (d, J = 17.6 Hz, 1H), 3.71 (dd, J = 6.4, 2.8 Hz, 1H), 4.46 (d, J = 12.0 Hz, 1H), 4.56 (d, J = 12.0 Hz, 1H), 6.76-6.83 (m, 1H), 6.84-6.90 (m, 1H), 7.38 (td, J = 8.0, 6.4 Hz, 1H) |
| 2-18 | Colorless liquid | 1.07 (d, J = 6.8 Hz, 3H), 1.08 (d, J = 6.8 Hz, 3H), 1.60 (s, 3H), 1.83 (dd, J = 13.6, 2.8 Hz, 1H), 2.02 (dd, J = 13.6, 6.8 Hz, 1H), 2.00 (d, J = 17.6 Hz, 1H), 2.18 (sept, J = 6.8 Hz, 1H), 2.25 (d, J = 17.6 Hz, 1H), 3.74 (dd, J = 6.8, 2.8 Hz, 1H), 4.49 (d, J = 12.8 Hz, 1H), 4.59 (d, J = 12.8 Hz, 1H), 6.91-7.03 (m, 2H), 7.12-7.19 (m, 1H). |

(continued)

| Table 5-Continued | | |
|---|---|---|
| No. | Form | $^1$H NMR spectrum σ ppm: |
| 2-20 | Colorless liquid | 1.056 (d, J = 6.8 Hz, 3H), 1.063 (d, J = 6.8 Hz, 3H), 1.51 (s, 3H), 1.83 (dd, J = 14.0, 2.8 Hz, 1H), 2.01 (dd, J = 14.0, 6.8 Hz, 1H), 2.07 (d, J = 17.6 Hz, 1H), 2.16 (sept, J = 6.8 Hz, 1H), 2.22 (d, J = 17.6 Hz, 1H), 3.71 (dd, J = 6.8, 2.8 Hz, 1H), 4.54 (d, J = 10.8 Hz, 1H), 4.63 (d, J = 10.8 Hz, 1H), 6.87-6.93 (m, 2H), 7.26-7.33 (m, 1H) |
| 2-26 | Colorless liquid | 1.06 (d, J = 6.8 Hz, 3H), 1.07 (d, J = 6.8 Hz, 3H), 1.54 (s, 3H), 1.92 (dd, J = 13.6, 2.4 Hz, 1H), 2.03 (dd, J = 13.6, 6.4 Hz, 1H), 2.08 (d, J = 17.2 Hz, 1H), 2.18 (sept, J = 6.8 Hz, 1H), 2.23 (d, J = 17.2 Hz, 1H), 3.71 (dd, J = 6.4, 2.4 Hz, 1H), 4.71 (d, J = 10.4 Hz, 1H), 4.79 (d, J = 10.4 Hz, 1H), 7.17-7.23 (m, 2H), 7.32 (d, J = 8.4 Hz, 1H). |
| 2-28 | Colorless liquid | 1.06 (d, J = 6.8 Hz, 3H), 1.07 (d, J = 6.8 Hz, 3H), 1.52 (s, 3H), 1.87 (dd, J = 14.0, 2.8 Hz, 1H), 2.02 (dd, J = 14.0, 6.4 Hz, 1H), 2.07 (d, J = 17.6 Hz, 1H), 2.17 (sept, J = 6.8 Hz, 1H), 2.22 (d, J = 17.6 Hz, 1H), 3.70 (dd, J = 6.4, 2.8 Hz, 1H), 4.60 (dd, J = 11.2, 2.4 Hz, 1H), 4.69 (dd, J = 11.2, 2.4 Hz, 1H), 6.97-7.03 (m, 1H), 7.18-7.31 (m, 2H). |
| 2-29 | Colorless liquid | 1.07 (d, J = 6.8 Hz, 3H), 1.08 (d, J = 6.8 Hz, 3H), 1.61 (s, 3H), 1.85 (dd, J = 14.0, 2.8 Hz, 1H), 2.03 (dd, J = 14.0, 6.8 Hz, 1H), 2.00 (d, J = 17.2 Hz, 1H), 2.18 (sept, J = 6.8 Hz, 1H), 2.26 (d, J = 18.0 Hz, 1H), 3.76 (dd, J = 6.8, 2.8 Hz, 1H), 4.63 (d, J = 13.2 Hz, 1H), 4.73 (d, J = 13.2 Hz, 1H), 7.39 (t, J = 8.0 Hz, 1H), 7.56 (t, J = 8.0 Hz, 1H), 7.64 (d, J = 8.0 Hz, 1H), 7.72 (d, J = 8.0 Hz, 1H). |
| 2-62 | Colorless liquid | 1.07 (d, J = 6.8 Hz, 3H), 1.08 (d, J = 6.8 Hz, 3H), 1.59 (s, 3H), 1.77 (dd, J = 12.4, 2.8 Hz, 1H), 2.14-2.23 (m, 2H), 2.25 (s, 3H), 2.29-2.41 (m, 2H), 4.31 (dd, J = 6.8, 2.8 Hz, 1H), 4.83 (t, J = 2.4 Hz, 1H), 4.89 (t, J = 2.4 Hz, 1H), 6.69 (d, J = 8.0 Hz, 1H), 6.84 (td, J = 8.0, 0.8 Hz, 1H), 7.06-7.17 (m, 2H). |
| 2-72 | Colorless liquid | 1.08 (d, J = 6.8 Hz, 3H), 1.11 (d, J = 6.8 Hz, 3H), 1.49 (s, 3H), 1.82 (dd, J = 12.4, 2.8 Hz, 1H), 1.92 (dd, J = 12.4, 6.8 Hz, 1H), 2.13-2.29 (m, 3H), 2.32 (s, 3H), 3.61 (dd, J = 6.8, 2.8 Hz, 1H), 4.37 (d, J = 12.4 Hz, 1H), 4.55 (d, J = 12.4 Hz, 1H), 4.74 (t, J = 2.4 Hz, 1H), 4.83 (t, J = 2.4 Hz, 1H), 7.13-7.22 (m, 3H), 7.30-7.34 (m, 1H). |

[Table 119]

| Table 5-Continued | | |
|---|---|---|
| No. | Form | $^1$H NMR spectrum σ ppm: |
| 2-88 | Colorless liquid | 1.08 (d, J = 6.8 Hz, 3H), 1.11 (d, J = 6.8 Hz, 3H), 1.40 (s, 3H), 1.82 (dd, J = 12.4, 2.8 Hz, 1H), 1.95 (dd, J = 12.4, 6.8 Hz, 1H), 2.14-2.31 (m, 3H), 3.65 (dd, J = 6.8, 2.8 Hz, 1H), 4.50 (d, J = 12.8 Hz, 1H), 4.60 (d, J = 12.8 Hz, 1H), 4.75 (t, J = 2.4 Hz, 1H), 4.84 (t, J = 2.4 Hz, 1H), 6.98-7.05 (m, 1H), 7.13 (td, J = 7.2, 0.8 Hz, 1H), 7.22-7.29 (m, 1H), 7.45 (td, J = 7.2, 1.6 Hz, 1H). |
| 2-191 | Colorless liquid | 1.11 (d, J = 7.2 Hz, 3H), 1.12 (d, J = 7.2 Hz, 3H), 1.54 (s, 3H), 1.58 (s, 1H), 1.84 (dd, J = 12.8, 2.8 Hz, 1H), 2.08 (dd, J = 12.8, 2.8 Hz, 1H), 2.26 (sept, J = 7.2 Hz, 1H), 2.33 (s, 3H), 2.36 (s, 2H), 3.62 (dd, J = 6.8, 2.8 Hz, 1H), 4.39 (d, J = 12.4 Hz, 1H), 4.57 (d, J = 12.4 Hz, 1H), 7.14-7.24 (m, 3H), 7.30 (d, J = 7.2 Hz, 1H). |
| 2-197 | Colorless liquid | 1.11 (d, J = 6.8 Hz, 6H), 1.55 (s, 3H), 1.58 (s, 1H), 1.84 (dd, J = 12.8, 2.4 Hz, 1H), 2.08 (dd, J = 12.8, 2.4 Hz, 1H), 2.26 (sept, J = 6.8 Hz, 1H), 2.38 (s, 2H), 3.67 (dd, J = 6.8, 2.8 Hz, 1H), 4.51 (d, J = 12.4 Hz, 1H), 4.61 (d, J = 12.4 Hz, 1H), 7.00-7.06 (m, 1H), 7.14 (td, J = 7.2, 2.0 Hz, 1H), 7.24-7.31 (m, 1H), 7.43 (td, J = 7.2, 2.0 Hz, 1H). |
| 2-346 | Colorless liquid | 1.06 (d, J = 6.8 Hz, 3H), 1.07 (d, J = 6.8 Hz, 3H), 1.56 (s, 3H), 1.80 (dd, J = 13.6, 2.8 Hz, 1H), 1.95 (dd, J = 13.6, 6.8 Hz, 1H), 2.04 (d, J = 17.6 Hz, 1H), 2.17 (sept, J = 6.8 Hz, 1H), 2.21 (d, J = 17.6 Hz, 1H), 3.67 (dd, J = 6.8, 2.8 Hz, 1H), 3.81 (s, 3H), 4.38 (d, J = 12.0 Hz, 1H), 4.54 (d, J = 12.0 Hz, 1H), 6.85-6.90 (m, 2H), 7.22-7.27 (m, 2H) |

(continued)

| Table 5-Continued | | |
|---|---|---|
| No. | Form | $^{1}$H NMR spectrum σ ppm: |
| 2-347 | Colorless liquid | 1.06 (d, J = 6.8 Hz, 3H), 1.07 (d, J = 6.8 Hz, 3H), 1.60 (s, 3H), 1.82 (dd, J = 14.0, 2.8 Hz, 1H), 1.97 (dd, J = 14.0, 6.8 Hz, 1H), 2.05 (d, J = 18.0 Hz, 1H), 2.17 (sept, J = 6.8 Hz, 1H), 2.22 (d, J = 18.0 Hz, 1H), 3.70 (dd, J = 6.8, 2.8 Hz, 1H), 3.81 (s, 3H), 4.42 (d, J = 12.4 Hz, 1H), 4.58 (d, J = 12.4 Hz, 1H), 6.81-6.86 (m, 1H), 6.88-6.92 (m, 2H), 7.23-7.29 (m, 1H). |
| 2-348 | Colorless liquid | 1.07 (d, J = 6.8 Hz, 3H), 1.08 (d, J = 6.8 Hz, 3H), 1.59 (s, 3H), 1.84 (dd, J = 13.6, 2.8 Hz, 1H), 2.02 (dd, J = 13.6, 6.4 Hz, 1H), 2.08 (d, J = 18.0 Hz, 1H), 2.18 (sept, J = 6.8 Hz, 1H), 2.25 (d, J = 18.0 Hz, 1H), 3.74 (dd, J = 6.4, 2.8 Hz, 1H), 4.52 (d, J = 12.8 Hz, 1H), 4.63 (d, J = 12.8 Hz, 1H), 7.21-7.26 (m, 1H), 7.27-7.37 (m, 2H), 7.54 (dd, J = 7.2, 2.0 Hz, 1H). |
| 2-349 | Colorless liquid | 1.06 (d, J = 6.8 Hz, 3H), 1.07 (d, J = 6.8 Hz, 3H), 1.58 (s, 3H), 1.81 (dd, J = 13.6, 2.8 Hz, 1H), 1.98 (dd, J = 13.6, 6.4 Hz, 1H), 2.06 (d, J = 17.2 Hz, 1H), 2.17 (sept, J = 6.8 Hz, 1H), 2.23 (d, J = 17.2 Hz, 1H), 3.69 (dd, J = 6.4, 2.8 Hz, 1H), 4.40 (d, J = 12.4 Hz, 1H), 4.55 (d, J = 12.4 Hz, 1H), 6.99-7.07 (m, 2H), 7.26-7.33 (m, 2H). |
| 2-350 | Colorless liquid | 1.06 (d, J = 6.8 Hz, 3H), 1.07 (d, J = 6.8 Hz, 3H), 1.57 (s, 3H), 1.81 (dd, J = 13.6, 2.8 Hz, 1H), 1.95 (dd, J = 13.6, 6.8 Hz, 1H), 2.04 (d, J = 17.2 Hz, 1H), 2.17 (sept, J = 6.8 Hz, 1H), 2.21 (d, J = 17.2 Hz, 1H), 2.35 (s, 3H), 3.68 (dd, J = 6.8, 2.8 Hz, 1H), 4.40 (d, J = 12.4 Hz, 1H), 4.56 (d, J = 12.4 Hz, 1H), 7.15 (d, J = 8.0 Hz, 2H), 7.21 (d, J = 8.0 Hz, 2H). |

[Table 120]

| Table 5-Continued | | |
|---|---|---|
| No. | Form | $^{1}$H NMR spectrum σ ppm: |
| 2-351 | Colorless liquid | 1.07 (d, J = 6.8 Hz, 3H), 1.08 (d, J = 6.8 Hz, 3H), 1.60 (s, 3H), 1.82 (dd, J = 13.6, 2.8 Hz, 1H), 1.99 (dd, J = 13.6, 6.4 Hz, 1H), 2.06 (d, J = 17.2 Hz, 1H), 2.18 (sept, J = 6.8 Hz, 1H), 2.24 (d, J = 17.2 Hz, 1H), 3.71 (dd, J = 6.4, 2.8 Hz, 1H), 4.44 (d, J = 12.4 Hz, 1H), 4.58 (d, J = 12.4 Hz, 1H), 6.98 (td, J = 8.4, 2.4 Hz, 1H), 7.03-7.11 (m, 2H), 7.30 (td, J = 8.4, 6.0 Hz, 1H). |
| 2-352 | Colorless liquid | 1.06 (d, J = 6.8 Hz, 3H), 1.07 (d, J = 6.8 Hz, 3H), 1.59 (s, 3H), 1.82 (dd, J = 13.6, 2.8 Hz, 1H), 1.96 (dd, J = 13.6, 6.8 Hz, 1H), 2.05 (d, J = 18.0 Hz, 1H), 2.17 (sept, J = 6.8 Hz, 1H), 2.22 (d, J = 18.0 Hz, 1H), 2.35 (s, 3H), 3.69 (dd, J = 6.8, 2.8 Hz, 1H), 4.41 (d, J = 12.4 Hz, 1H), 4.56 (d, J = 12.4 Hz, 1H), 7.08-7.15 (m, 3H), 7.23 (t, J = 7.2 Hz, 1H). |
| 2-353 | Colorless liquid | 1.06 (d, J = 6.8 Hz, 3H), 1.07 (d, J = 6.8 Hz, 3H), 1.50 (s, 3H), 1.83 (dd, J = 14.0, 2.4 Hz, 1H), 2.02 (dd, J = 14.0, 6.8 Hz, 1H), 2.05 (d, J = 17.6 Hz, 1H), 2.17 (sept, J = 6.8 Hz, 1H), 2.20 (d, J = 17.6 Hz, 1H), 2.42 (s, 3H), 3.64 (dd, J = 6.8, 2.4 Hz, 1H), 4.54 (dd, J = 11.2, 2.0 Hz, 1H), 4.60 (dd, J = 11.2, 2.0 Hz, 1H), 6.89 (t, J = 8.4 Hz, 1H), 6.98 (d, J = 8.4 Hz, 1H), 7.19 (td, J = 8.4, 6.0 Hz, 1H). |
| 2-354 | Colorless liquid | 1.076 (d, J = 6.8 Hz, 3H), 1.080 (d, J = 6.8 Hz, 3H), 1.61 (s, 3H), 1.88 (dd, J = 13.6, 2.4 Hz, 1H), 2.07 (dd, J = 13.6, 6.8 Hz, 1H), 2.12 (d, J = 17.2 Hz, 1H), 2.19 (sept, J = 6.8 Hz, 1H), 2.26 (d, J = 17.2 Hz, 1H), 3.82 (dd, J = 6.8, 2.4 Hz, 1H), 4.62 (d, J = 12.4 Hz, 1H), 4.73 (d, J = 12.4 Hz, 1H), 7.38-7.45 (m, 1H), 7.57-7.70 (m, 3H). |
| 2-355 | Colorless liquid | 1.06 (d, J = 6.8 Hz, 3H), 1.07 (d, J = 6.8 Hz, 3H), 1.53 (s, 3H), 1.89 (dd, J = 13.6, 2.8 Hz, 1H), 2.02 (dd, J = 13.6, 6.8 Hz, 1H), 2.08 (d, J = 17.2 Hz, 1H), 2.17 (sept, J = 6.8 Hz, 1H), 2.22 (d, J = 17.2 Hz, 1H), 3.70 (dd, J = 6.8, 2.8 Hz, 1H), 4.60 (dd, J = 11.2, 2.4 Hz, 1H), 4.69 (dd, J = 11.2, 2.4 Hz, 1H), 7.04 (t, J = 8.4 Hz, 1H), 7.19 (td, 8.4, 6.0 Hz, 1H), 7.37-7.41 (m, 1H). |

[Table 121]

| Table 6 | | | |
|---|---|---|---|
| No. | Physical property (melting point °C) | Form | $^1$H NMR spectrum σ ppm: |
| 3-1 | | Colorless liquid | 1.03 (d, J = 4.1 Hz, 3H), 1.05 (d, J = 4.1 Hz, 3H), 1.48 (dd, J = 11.7, 2.1 Hz, 1H), 1.62 (s, 3H), 1.85 (dd, J = 11.7, 6.2 Hz, 1H), 2.12-2.18 (m, 1H), 3.61 (dd, J = 6.2, 2.1 Hz, 1H), 4.45 (d, J = 12.4 Hz, 1H), 4.62 (d, J = 12.4 Hz, 1H), 6.04 (d, J = 6.0 Hz, 1H), 6.33 (d, J = 5.5 Hz, 1H), 7.25-7.36 (m, 5H). |
| 3-2 | | Colorless liquid | 1.05 (dd, J = 6.6, 5.3 Hz, 6H), 1.49 (dd, J = 11.4, 1.8 Hz, 1H), 1.59 (s, 3H), 1.86 (dd, J = 11.7, 6.2 Hz, 1H), 2.13-2.19 (m, 1H), 2.33 (s, 3H), 3.60 (dd, J = 6.2, 2.1 Hz, 1H), 4.40 (d, J = 12.4 Hz, 1H), 4.61 (d, J = 12.4 Hz, 1H), 6.05 (d, J = 5.5 Hz, 1H), 6.33 (d, J = 5.5 Hz, 1H), 7.17-7.20 (m, 3H), 7.33-7.34 (m, 1H). |
| 3-3 | | Colorless liquid | 1.05 (t, J = 6.9 Hz, 6H), 1.48-1.52 (m, 1H), 1.54 (s, 3H), 1.89 (dd, J = 11.4, 6.0 Hz, 1H), 2.10-2.19 (m, 1H), 2.40 (s, 6H), 3.55 (dd, J = 6.2, 2.1 Hz, 1H), 4.47 (d, J = 11.0 Hz, 1H), 4.59 (d, J = 11.0 Hz, 1H), 6.04 (d, J = 5.5 Hz, 1H), 6.34 (d, J = 5.5 Hz, 1H), 7.00 (d, J = 7.8 Hz, 2H), 7.09 (dd, J = 8.5, 6.6 Hz, 1H). |
| 3-5 | | Colorless liquid | 1.04 (6H, dd, J = 5.5, 2.7 Hz), 1.49 (1H, d, J = 11.4 Hz), 1.57 (1H, d, J = 0.9 Hz), 1.61 (3H, s), 1.89 (1H, dd, J = 11.7, 6.2 Hz), 2.12-2.19 (1H, m), 3.65 (1H, d, J = 6.4 Hz), 4.59 (2H, dd, J = 41.7, 12.8 Hz), 6.07 (1H, d, J = 5.5 Hz), 6.35 (1H, d, J = 5.5 Hz), 7.02 (1H, t, J = 9.2 Hz), 7.13 (1H, t, J = 7.6 Hz), 7.26 (1H, t, J = 10.3 Hz), 7.47 (1H, t, J = 7.6 Hz). |
| 3-7 | | Yellow liquid | 1.03 (t, J = 6.4 Hz, 6H), 1.46-1.49 (m, 1H), 1.54 (s, 3H), 1.87-1.92 (m, 1H), 2.10-2.20 (m, 1H), 3.63 (dd, J = 6.2, 2.1 Hz, 1H), 4.53 (d, J = 11.0 Hz, 1H), 4.64 (d, J = 11.0 Hz, 1H), 6.05 (d, J = 6.0 Hz, 1H), 6.34 (d, J = 5.5 Hz, 1H), 6.86-6.90 (m, 2H), 7.23-7.30 (m, 1H). |
| 3-175 | | Colorless liquid | 0.85 (d, J = 6.8 Hz, 3H), 1.09 (d, J = 6.8 Hz, 3H), 1.64 (s, 3H), 1.86 (dd, J = 12.0, 2.4 Hz, 1H), 1.97 (dd, J = 12.0, 6.4 Hz, 1H), 2.35 (s, 3H), 2.43 (sept, J = 6.8 Hz, 1H), 3.68 (dd, J = 6.4, 1.6 Hz, 1H), 4.45 (d, J = 12.4 Hz, 1H), 4.63 (d, J = 12.4 Hz, 1H), 5.97 (s, 1H), 7.11-7.35 (m, 9H). |
| 3-177 | | Colorless liquid | 0.85 (d, J = 6.8 Hz, 3H), 1.09 (d, J = 6.8 Hz, 3H), 1.65 (s, 3H), 1.84-1.91 (m, 1H), 1.88 (dd, J = 12.8, 9.2 Hz, 1H), 2.43 (sept, J = 6.8 Hz, 1H), 3.72 (dd, J = 6.0, 1.6 Hz, 1H), 4.59 (d, J = 12.8 Hz, 1H), 4.68 (d, J = 12.8 Hz, 1H), 5.98 (s, 1H), 7.02 (t, J = 8.0 Hz, 1H), 7.13 (t, J = 7.2 Hz, 1H), 7.18-7.36 (m, 6H), 7.48 (td, J = 7.2 Hz, 1H). |
| 3-680 | | Colorless liquid | 1.07 (dd, J = 6.9, 6.0 Hz, 6H), 1.60 (s, 3H), 1.64 (dd, J = 11.9, 1.8 Hz, 1H), 2.06 (dd, J = 12.1, 6.2 Hz, 1H), 2.10-2.16 (m, 1H), 3.77 (dd, J = 6.2, 2.1 Hz, 1H), 4.59 (dd, J = 33.7, 12.6 Hz, 2H), 5.80 (s, 1H), 7.00-7.05 (m, 1H), 7.12 (td, J = 7.6, 0.9 Hz, 1H), 7.24-7.29 (m, 1H), 7.42 (td, J = 7.4, 1.5 Hz, 1H). |

[Table 122]

| Table 6-Continued | | | |
|---|---|---|---|
| No. | Physical property (melting point °C) | Form | $^1$H NMR spectrum σ ppm: |
| 3-686 | | Colorless liquid | 0.84 (d, J = 6.8 Hz, 3H), 1.04 (d, J = 6.8 Hz, 3H), 1.66 (s, 3H), 1.82 (dd, J = 12.0, 2.0 Hz, 1H), 2.22 (dd, J = 12.0, 6.4 Hz, 1H), 2.30 (sept, J = 6.8 Hz, 1H), 3.77 (dd, J = 6.4, 2.0 Hz, 1H), 4.59 (d, J = 12.4 Hz, 1H), 4.68 (d, J = 12.4 Hz, 1H), 6.11 (s, 1H), 6.98-7.31 (m, 7H), 7.49 (td, 7.2, 1.2 Hz, 1H). |

(continued)

| Table 6-Continued | | | |
|---|---|---|---|
| No. | Physical property (melting point °C) | Form | $^1$H NMR spectrum σ ppm: |
| 3-688 | 62.1-64.3 | Yellow solid | 1.05 (d, J = 0.9 Hz, 3H), 1.06 (d, J = 0.9 Hz, 3H), 1.47-1.50 (m, 1H), 1.60 (s, 3H), 2.13-2.24 (m, 2H), 5.14 (dd, J = 6.6, 2.1 Hz, 1H), 6.17 (d, J = 6.0 Hz, 1H), 6.44 (d, J = 5.5 Hz, 1H), 6.80-6.86 (m, 2H), 7.54-7.58 (m, 1H), 8.10 (d, J = 3.2 Hz, 1H). |
| 3-693 | | Colorless liquid | 1.03 (dd, J = 6.9, 4.6 Hz, 6H), 1.40 (dd, J = 11.4, 1.8 Hz, 1H), 1.56 (s, 3H), 1.65 (s, 3H), 1.73 (s, 3H), 1.83 (dd, J = 11.7, 6.2 Hz, 1H), 2.13 (t, J = 6.9 Hz, 1H), 3.56 (dd, J = 6.2, 2.1 Hz, 1H), 3.90-4.01 (m, 2H), 5.34 (t, J = 6.6 Hz, 1H), 6.07 (d, J = 5.5 Hz, 1H), 6.33 (d, J = 6.0 Hz, 1H). |
| 3-697 | | Pale yellow liquid | 0.85-0.98 (m, 2H), 1.03 (dd, J = 6.9, 3.2 Hz, 6H), 1.13-1.26 (m, 4H), 1.35 (dd, J = 11.4, 1.8 Hz, 1H), 1.56 (s, 3H), 1.69-1.72 (m, 4H), 1.83 (dd, J = 11.7, 6.2 Hz, 2H), 2.12-2.16 (m, 1H), 3.11 (dd, J = 9.2, 6.9 Hz, 1H), 3.26 (dd, J = 9.2, 6.0 Hz, 1H), 3.47 (dd, J = 6.4, 2.3 Hz, 1H), 6.06 (d, J = 6.0 Hz, 1H), 6.32 (d, J = 5.5 Hz, 1H). |
| 3-732 | | Yellow liquid | 1.04 (t, J = 7.1 Hz, 6H), 1.53-1.58 (m, 4H), 1.92 (dd, J = 11.7, 6.2 Hz, 1H), 2.12-2.19 (m, 1H), 3.61 (dd, J = 6.0, 1.8 Hz, 1H), 4.71 (d, J = 10.5 Hz, 1H), 4.80 (d, J = 10.5 Hz, 1H), 6.05 (d, J = 5.5 Hz, 1H), 6.36 (d, J = 5.5 Hz, 1H), 7.17 (dd, J = 8.7, 7.3 Hz, 1H), 7.25-7.32 (m, 2H). |
| 3-738 | | Colorless liquid | 1.04 (d, J = 3.2 Hz, 3H), 1.06 (d, J = 3.7 Hz, 3H), 1.51 (dd, J = 11.4, 1.8 Hz, 1H), 1.66 (s, 3H), 1.92 (dd, J = 11.7, 6.2 Hz, 1H), 2.10-2.20 (m, 1H), 3.71 (dd, J = 6.2, 2.1 Hz, 1H), 4.60 (d, J = 13.7 Hz, 1H), 4.70 (d, J = 13.7 Hz, 1H), 6.08 (d, J = 5.5 Hz, 1H), 6.35 (d, J = 6.0 Hz, 1H), 7.17 (dd, J = 7.3, 5.0 Hz, 1H), 7.53 (d, J = 7.8 Hz, 1H), 7.70 (td, J = 7.8, 1.8 Hz, 1H), 8.51-8.52 (m, 1H). |
| 3-748 | | Colorless liquid | 1.03 (d, J = 4.1 Hz, 3H), 1.05 (d, J = 4.1 Hz, 3H), 1.48 (dd, J = 11.4, 1.8 Hz, 1H), 1.62 (s, 3H), 1.85 (dd, J = 11.7, 6.2 Hz, 1H), 2.12-2.18 (m, 1H), 3.61 (dd, J = 6.2, 2.1 Hz, 1H), 4.45 (d, J = 12.8 Hz, 1H), 4.62 (d, J = 12.4 Hz, 1H), 6.04 (d, J = 5.5 Hz, 1H), 6.33 (d, J = 6.0 Hz, 1H), 7.25-7.36 (m, 5H). |
| 3-749 | | Colorless liquid | 1.04 (d, J = 5.5 Hz, 3H), 1.06 (d, J = 5.5 Hz, 3H), 1.50 (d, J = 6.4 Hz, 1H), 1.60 (s, 3H), 1.86 (dd, J = 11.4, 6.0 Hz, 1H), 2.10-2.21 (m, 1H), 2.33 (s, 3H), 3.60 (dd, J = 6.2, 2.1 Hz, 1H), 4.51 (dd, J = 81.1, 12.4 Hz, 2H), 6.05 (d, J = 6.0 Hz, 1H), 6.33 (d, J = 6.0 Hz, 1H), 7.16-7.20 (m, 3H), 7.33 (d, J = 6.9 Hz, 1H). |

[Table 123]

| Table 6-Continued | | | |
|---|---|---|---|
| No. | Physical property (melting point °C) | Form | $^1$H NMR spectrum σ ppm: |
| 3-750 | | Colorless liquid | 1.04 (d, J = 4.1 Hz, 3H), 1.05 (d, J = 4.6 Hz, 3H), 1.49 (dd, J = 11.4, 1.8 Hz, 1H), 1.61 (s, 3H), 1.89 (dd, J = 11.7, 6.2 Hz, 1H), 2.12-2.18 (m, 1H), 3.65 (dd, J = 6.2, 2.1 Hz, 1H), 4.59 (dd, J = 41.7, 12.8 Hz, 2H), 6.06 (d, J = 5.5 Hz, 1H), 6.35 (d, J = 6.0 Hz, 1H), 6.99-7.04 (m, 1H), 7.13 (td, J = 7.6, 0.9 Hz, 1H), 7.24-7.27 (m, 1H), 7.47 (td, J = 7.4, 1.7 Hz, 1H). |

(continued)

Table 6-Continued

| No. | Physical property (melting point °C) | Form | $^1$H NMR spectrum σ ppm: |
|---|---|---|---|
| 3-751 | | Colorless liquid | 1.04 (d, J = 4.1 Hz, 3H), 1.06 (d, J = 4.1 Hz, 3H), 1.49 (dd, J = 11.4, 1.8 Hz, 1H), 1.63 (s, 3H), 1.92 (dd, J = 11.7, 6.2 Hz, 1H), 2.13-2.17 (m, 1H), 3.67 (dd, J = 6.2, 2.1 Hz, 1H), 4.55 (dd, J = 42.8, 13.5 Hz, 2H), 6.08 (d, J = 6.0 Hz, 1H), 6.36 (d, J = 5.5 Hz, 1H), 7.25 (dd, J = 9.4, 3.0 Hz, 1H), 7.35 (d, J = 1.8 Hz, 1H), 7.49 (d, J = 8.2 Hz, 1H). |
| 3-752 | | Colorless liquid | 1.04 (6H, dd, J = 6.9, 4.1 Hz), 1.48 (1H, dd, J = 11.7, 2.1 Hz), 1.62 (3H, s), 1.85 (1H, dd, J = 11.7, 6.2 Hz), 2.12-2.18 (1H, m), 3.61 (1H, dd, J = 6.2, 2.1 Hz), 4.45 (1H, d, J = 12.4 Hz), 4.62 (1H, d, J = 12.4 Hz), 6.05 (1H, d, J = 6.0 Hz), 6.33 (1H, d, J = 6.0 Hz), 7.29-7.34 (5H, m). |
| 3-753 | | Colorless liquid | 1.05 (6H, dd, J = 6.4, 5.5 Hz), 1.50 (1H, dd, J = 11.4, 1.8 Hz), 1.60 (3H, s), 1.86 (1H, dd, J = 11.4, 6.0 Hz), 2.10-2.21 (1H, m), 2.33 (3H, s), 3.60 (1H, dd, J = 6.0, 1.8 Hz), 4.40 (1H, d, J = 12.4 Hz), 4.61 (1H, d, J = 11.9 Hz), 6.05 (1H, d, J = 6.0 Hz), 6.33 (1H, d, J = 5.5 Hz), 7.15-7.22 (3H, m), 7.33 (1H, d, J = 7.3 Hz). |
| 3-754 | | Colorless liquid | 1.04 (6H, dd, J = 6.6, 4.4 Hz), 1.49 (1H, dd, J = 11.4, 2.1 Hz), 1.61 (3H, s), 1.89 (1H, dd, J = 11.4, 6.4 Hz), 2.10-2.18 (1H, m), 3.65 (1H, dd, J = 6.4, 2.1 Hz), 4.54 (1H, d, J = 12.8 Hz), 4.64 (1H, d, J = 12.8 Hz), 6.06 (1H, d, J = 5.5 Hz), 6.34 (1H, d, J = 5.5 Hz), 7.02 (1H, t, J = 9.2 Hz), 7.13 (1H, td, J = 7.6, 1.1 Hz), 7.24-7.27 (1H, m), 7.47 (1H, td, J = 7.6, 1.5 Hz). |
| 3-755 | | Pale yellow liquid | 1.05 (6H, dd, J = 6.9, 3.2 Hz), 1.51 (1H, dd, J = 11.7, 2.1 Hz), 1.67 (3H, d, J = 14.7 Hz), 1.92 (1H, dd, J = 11.7, 6.4 Hz), 2.13-2.18 (1H, m), 3.71 (1H, dd, J = 6.4, 2.1 Hz), 4.60 (1H, d, J = 13.7 Hz), 4.70 (1H, d, J = 13.7 Hz), 6.08 (1H, d, J = 6.0 Hz), 6.35 (1H, d, J = 6.0 Hz), 7.18 (1H, dd, J = 7.1, 5.3 Hz), 7.53 (1H, d, J = 7.8 Hz), 7.70 (1H, td, J = 7.8, 1.8 Hz), 8.52 (1H, d, J = 4.6 Hz). |
| 3-756 | | Colorless liquid | 1.03 (d, J = 4.6 Hz, 3H), 1.05 (d, J = 4.6 Hz, 3H), 1.45 (d, J = 11.4 Hz, 1H), 1.58 (s, 3H), 1.84 (dd, J = 11.4, 6.4 Hz, 1H), 2.10-2.20 (m, 1H), 3.66 (d, J = 6.4 Hz, 1H), 4.62 (d, J = 12.8 Hz, 1H), 4.78 (d, J = 12.8 Hz, 1H), 6.04 (d, J = 5.5 Hz, 1H), 6.33 (d, J = 5.5 Hz, 1H), 6.95-6.96 (m, 2H), 7.26-7.28 (m, 1H). |
| 3-757 | | Colorless liquid | 1.03 (d, J = 3.7 Hz, 3H), 1.05 (d, J = 3.7 Hz, 3H), 1.44 (dd, J = 11.4, 1.8 Hz, 1H), 1.59 (s, 3H), 1.83 (dd, J = 11.4, 6.2 Hz, 1H), 2.09-2.19 (m, 1H), 3.61 (dd, J = 6.2, 1.8 Hz, 1H), 4.47 (d, J = 12.4 Hz, 1H), 4.60 (d, J = 12.4 Hz, 1H), 6.05 (d, J = 5.5 Hz, 1H), 6.33 (d, J = 5.5 Hz, 1H), 7.06 (d, J = 5.0 Hz, 1H), 7.18 (dd, J = 1.8, 0.9 Hz, 1H), 7.29 (dd, J = 5.0, 3.0 Hz, 1H). |

The following Reference Examples show Synthesis Examples in which starting materials for the above synthesis are synthesized from commercially available products, but the present invention is not limited thereto.

Reference Example 1

Synthesis of (1S,6R)-6-((tert-butyldimethylsilyl)oxy)-4-isopropyl-1-methylcyclohex-3-en-1-ol

The synthesis was performed according to the following procedure with reference to the method described in Tetrahedron, Vol. 51, 1995, pp. 1345-1376.

To a mixed solution of tert-butanol (140 mL) and water (140 mL) of AD-mix-β (40.0 g, a mixture of $(DHQD)_2PHAL$, potassium carbonate, potassium ferricyanide, and potassium osmate dihydrate, manufactured by Sigma-Aldrich, product number 392766), methanesulfonamide (2.70 g, 28.2 mmol) was added, followed by stirring at room temperature for 30 minutes. To the mixed solution, γ-terpinene (4.60 mL, 28.7 mmol) was added at 0°C, followed by stirring at 0°C for 22 hours. To the reaction mixture, sodium sulfite (20.0 g) was added, and the temperature was raised to room temperature. The reaction mixture was extracted with ethyl acetate, and the extract was dried over anhydrous magnesium sulfate and then

concentrated under reduced pressure to obtain a crude product of (1S,2R)-4-isopropyl-1-methylcyclohex-4-ene-1,2-diol.

To a dimethylformamide solution (146 mL) of the crude product of (1S,2R)-4-isopropyl-1-methylcyclohex-4-ene-1,2-diol, imidazole (3.81 g, 56.0 mmol) and tert-butylchlorodimethylsilane (6.32 g, 41.9 mmol) were added, followed by stirring at room temperature for 19 hours. A saturated sodium hydrogen carbonate aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 6.65 mg, 83%).

$^1$H NMR (400 MHz, $CDCl_3$) δ 0.08 (s, 3H), 0.09 (s, 3H), 0.90 (s, 9H), 0.98 (d, J = 6.8 Hz, 6H), 1.15 (s, 3H), 2.02-2.29 (m, 6H), 3.64 (t, J = 6.0 Hz, 1H), 5.22-5.27 (m, 1H).

Reference Example 2

Synthesis of (1S,3S,4R,6S)-4-((tert-butyldimethylsilyl)oxy)-6-isopropyl-3-methyl-7-oxabicyclo[4.1.0]heptan-3-ol and (1R,3S,4R,6R)-4-((tert-butyldimethylsilyl)oxy)-6-isopropyl-3-methyl-7-oxabicyclo[4.1.0]heptan-3-ol

To a dichloromethane solution (40 mL) of (1S,6R)-6-((tert-butyldimethylsilyl)oxy)-4-isopropyl-1-methylcyclohex-3-en-1-ol (3.93 g, 13.8 mmol), 3-chloroperbenzoic acid (5.18 g, 20.7 mmol) was added, followed by stirring at room temperature for 3 hours. A saturated sodium hydrogen carbonate aqueous solution was poured into the reaction mixture, which was then extracted with dichloromethane. The extract was dried over anhydrous magnesium sulfate and then concentrated under reduced pressure. The concentrate was purified by silica gel column chromatography (eluent: ethyl acetate/n-hexane) to obtain a colorless liquid (1S,3S,4R,6S)-4-((tertbutyldimethylsilyl)oxy)-6-isopropyl-3-methyl-7-oxabicyclo[4.1.0]heptan-3-ol (yield 548 mg, 13%) and a colorless liquid (1R,3S,4R,6R)-4-((tert-butyldimethylsilyl)oxy)-6-isopropyl-3-methyl-7-oxabicyclo[4.1.0]heptan-3-ol (yield 2.14 g, 51%).

(1S,3S,4R,6S)-4-((tert-butyldimethylsilyl)oxy)-6-isopropyl-3-methyl-7-oxabicyclo[4.1.0]heptan-3-ol

$^1$H NMR (400 MHz, $CDCl_3$) δ 0.06 (s, 3H), 0.09 (s, 3H), 0.91 (s, 9H), 0.96 (d, J = 6.8 Hz, 3H), 0.99 (d, J = 6.8 Hz, 3H), 1.13 (s, 3H), 1.51 (sept, J = 6.8 Hz, 1H), 1.85-2.03 (m, 4H), 2.14 (dd, J = 16.0, 5.2 Hz, 1H), 2.90 (d, J = 5.2 Hz, 1H), 3.61 (dd, J = 9.2, 5.2 Hz, 1H).

(1R,3S,4R,6R)-4-((tert-butyldimethylsilyl)oxy)-6-isopropyl-3-methyl-7-oxabicyclo[4.1.0]heptan-3-ol

$^1$H NMR (400 MHz, $CDCl_3$) δ 0.08 (s, 3H), 0.09 (s, 3H), 0.92 (s, 9H), 0.95 (d, J = 6.8 Hz, 3H), 0.98 (d, J = 6.8 Hz, 3H), 1.14 (s, 3H), 1.49 (sept, J = 6.8 Hz, 1H), 1.83 (15.6, 2.0 Hz, 1H), 1.92-2.03 (m, 2H), 2.26 (dd, J = 15.6, 2.0 Hz, 1H), 3.03 (t, J = 2.0 Hz, 1H), 3.43 (dd, J = 10.6, 6.8 Hz, 1H).

Reference Example 3

Synthesis of (1R,2S,4S,5R)-5-((tert-butyldimethylsilyl)oxy)-1-isopropyl-4-methyl-7-oxabicyclo[2.2.1]heptan-2-ol

To a tetrahydrofuran solution (36 mL) of (1S,3S,4R,6S)-4-((tert-butyldimethylsilyl)oxy)-6-isopropyl-3-methyl-7-oxabicyclo[4.1.0]heptan-3-ol (548 mg, 1.82 mmol), p-toluenesulfonic acid monohydrate (520 mg, 2.74 mmol) was added, followed by stirring at room temperature for 3 hours. A saturated sodium hydrogen carbonate aqueous solution was poured into the reaction mixture, which was then extracted with ethyl acetate. The extract was dried over anhydrous magnesium sulfate and then concentrated under reduced pressure. The concentrate was purified by silica gel column chromatography (eluent: ethyl acetate/n-hexane) to obtain a colorless liquid title compound (yield 449 mg, 83%).

$^1$H NMR (400 MHz, $CDCl_3$) δ 0.06 (s, 6H), 0.90 (s, 9H), 1.01 (d, J = 6.8 Hz, 3H), 1.02 (d, J = 6.8 Hz, 3H), 1.17 (dd, J = 13.0, 3.6 Hz, 1H), 1.20-1.28 (m, 1H), 1.29 (s, 3H), 1.31-1.37 (m, 1H), 2.00-2.14 (m, 2H), 2.69 (dd, J = 13.0, 7.6 Hz, 1H), 3.89 (dd, J = 6.8, 2.4 Hz, 1H), 4.14 (dq, J = 10.0, 2.4 Hz, 1H).

Reference Example 4

Synthesis of (1S,2R)-4-isopropyl-1-methyl-cyclohex-4-ene-1,2-diol

$(DHQD)_2PHAL$ (1.20 g, 1.60 mmol), potassium carbonate (65.0 g, 470 mmol), potassium ferricyanide (150 g, 470 mmol), and potassium osmate dihydrate (690 mg, 1.90 mmol) were dissolved in a mixed solution of tert-butanol (400 mL) and water (400 mL), and then methanesulfonamide (15.0 g, 160 mmol) was added, followed by stirring at room temperature for 30 minutes. To the mixed solution, γ-terpinene (21.0 g, 160 mmol) was added at 0°C, followed by stirring

at 0°C for 5 hours. To the reaction mixture, sodium sulfite (220 g) was added, and the temperature was raised to room temperature. The reaction mixture was extracted with ethyl acetate, and the extract was washed with brine and then concentrated under reduced pressure. The obtained concentrate was purified by silica gel column chromatography (eluent: ethyl acetate/n-hexane) to obtain a white solid title compound (26.1 g, 98%).

$^1$H-NMR ($CDCl_3$) δ: 1.01 (6H, dd, J = 6.9, 1.8 Hz), 1.22 (3H, s), 1.88 (1H, d, J = 6.9 Hz), 2.24 (5H, m, J = 52.9, 25.9, 9.8 Hz), 3.65 (1H, q, J = 5.8 Hz), 4.68 (1H, s), 5.31 (1H, brs).
Melting point: 66.0°C to 68.5°C

The same reaction and treatment as in Reference Example 4 was performed using $(DHQ)_2$PHAL instead of $(DHQD)_2$PHAL to obtain a white solid (1R,2S)-4-isopropyl-1-methyl-cyclohex-4-ene-1,2-diol in a yield of 73%.

$^1$H-NMR ($CDCl_3$) δ: 1.01 (6H, dd, J = 6.9, 1.8 Hz), 1.22 (3H, s), 1.88 (1H, d, J = 6.9 Hz), 2.24 (5H, m, J = 52.9, 25.9, 9.8 Hz), 3.65 (1H, q, J = 5.8 Hz), 4.68 (1H, s), 5.31 (1H, brs).
Melting point: 66.0°C to 70.1°C

The same reaction and treatment as in Synthesis Example 4 were performed without adding $(DHQD)_2$PHAL to obtain a white solid 1:1 mixture (racemate) of (1S,2R)-4-isopropyl-1-methyl-cyclohex-4-ene-1,2-diol and (1R,2S)-4-isopropyl-1-methyl-cyclohex-4-ene-1,2-diol in a yield of 75%.

$^1$H-NMR ($CDCl_3$) δ: 1.01 (6H, dd, J = 6.9, 1.8 Hz), 1.22 (3H, s), 1.88 (1H, d, J = 6.9 Hz), 2.24 (5H, m, J = 52.9, 25.9, 9.8 Hz), 3.65 (1H, q, J = 5.8 Hz), 4.68 (1H, s), 5.31 (1H, brs).
Melting point: 61.9°C to 64.3°C

The enantiomeric excess of the target diol compound obtained in Reference Example 4, can be determined by the following procedure with reference to the method described in Organic Letter, Vol. 8, 2006, pp. 609-612.

To deuterated chloroform (2 mL), the diol compound (4-isopropyl-1-methyl-cyclohex-4-ene-1,2-diol, 30.0 mg, 0.176 mmol), (2-formylphenyl)boronic acid (26.4 mg, 0.176 mmol), and (1S)-1-phenylethanamine (23.5 mg, 0.194 mmol) were added, followed by stirring at room temperature for 10 minutes, after which the reaction solution was used as is for $^1$H-NMR measurement.

In the case of (1S,2R)-4-isopropyl-1-methyl-cyclohex-4-ene-1,2-diol, a peak derived from imine is observed at a chemical shift of around 7.90 ppm, and in the case of (1R,2S)-4-isopropyl-1-methyl-cyclohex-4-ene-1,2-diol, a peak derived from imine is observed at a chemical shift of around 7.94 ppm. The enantiomeric excess of the target diol compound is determined from the integral ratio of this peak.

Reference Example 5

Synthesis of (1S,6R)-6-[(2-fluorophenyl)methoxy]-4-isopropyl-1-methyl-cyclohex-3-en-1-ol

To a dimethylformamide solution (300 mL) of (1S,2R)-4-isopropyl-1-methyl-cyclohex-4-ene-1,2-diol (29.2 g, 172 mmol), sodium hydride (55% dispersion in mineral oil, 8.98 g, 206 mmol) was added in an ice bath (0°C), followed by stirring in an ice bath (0°C) for 30 minutes. To the reaction mixture, 1-(bromomethyl)-2-fluoro-benzene (38.9 mg, 206 mmol) was added, followed by stirring at room temperature (25°C) for 5 hours. Water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a yellow liquid title compound (38.0 mg, 80%).

$^1$H NMR ($CDCl_3$) δ: 7.41 (1H, td, J = 7.6, 1.7 Hz), 7.31-7.25 (1H, m), 7.13 (1H, t, J = 7.3 Hz), 7.04 (1H, t, J = 9.2 Hz), 5.31 (1H, s), 4.73 (1H, d, J = 11.9 Hz), 4.60 (1H, d, J = 11.9 Hz), 3.46 (1H, t, J = 4.8 Hz), 2.35 (1H, s), 2.25-2.20 (4H, m), 2.07 (1H, d, J = 18.3 Hz), 1.20 (3H, s), 1.00 (5H, dd, J = 6.9, 3.7 Hz).

Reference Example 6

Synthesis of (1S,2R,4R,5S)-5-bromo-4-isopropyl-1-methyl-7-oxabicyclo[2.2.1]heptan-2-ol

To a dichloromethane solution (5 mL) of (1S,2R)-4-isopropyl-1-methyl-cyclohex-4-ene-1,2-diol (300 mg, 1.76 mmol), N-bromosuccinimide (344 mg, 1.94 mmol) was added, followed by stirring at room temperature for 5 hours. A 10% sodium thiosulfate aqueous solution was poured into the reaction mixture, which was then extracted with ethyl acetate. The extract was washed with brine and then concentrated under reduced pressure, and the concentrate was purified by

silica gel column chromatography (eluent: ethyl acetate/n-hexane) to obtain a white solid title compound (38.0 mg, 80%).

$^1$H NMR ($CDCl_3$) $\delta$: 1.06-1.09 (m, 6H), 1.40 (s, 3H), 1.51 (dd, J = 8.9, 7.1 Hz, 1H), 1.60 (d, J = 5.5 Hz, 1H), 1.63 (d, J = 5.5 Hz, 1H), 2.05-2.14 (m, 1H), 2.32 (dd, J = 13.7, 10.5 Hz, 1H), 2.93 (dd, J = 14.2, 6.9 Hz, 1H), 3.82 (d, J = 6.9 Hz, 1H), 4.01 (dq, J = 10.5, 2.4 Hz, 1H).
Melting point: 62.9°C to 65.0°C

Reference Example 7

Synthesis of (1S,2R,4R,5S)-5-chloro-4-isopropyl-1-methyl-7-oxabicyclo[2.2.1]heptan-2-ol

To a dichloromethane solution (30 mL) of (1S,2R)-4-isopropyl-1-methyl-cyclohex-4-ene-1,2-diol (2.00 g, 11.7 mmol), N-bromosuccinimide (2.04 g, 15.3 mmol) and N,N-dimethylpyridin-4-amine were added, followed by stirring at room temperature for 5 hours. A 10% sodium thiosulfate aqueous solution was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a white solid title compound (1.49 mg, 62%).

$^1$H NMR ($CDCl_3$) $\delta$: 1.04 (dd, J = 6.9, 4.1 Hz, 6H), 1.37 (s, 3H), 1.42 (dt, J = 14.2, 1.8 Hz, 1H), 1.48 (dd, J = 13.5, 4.8 Hz, 1H), 2.07 (td, J = 13.3, 6.3 Hz, 1H), 2.27 (dd, J = 13.3, 10.5 Hz, 1H), 2.86 (dd, J = 13.5, 6.9 Hz, 1H), 3.80-3.85 (m, 1H), 3.99 (dq, J = 10.6, 2.3 Hz, 1H).
Melting point: 79.7°C to 84.3°C

Reference Example 8

Synthesis of (1S,2R,4R)-4-isopropyl-1-methyl-7-oxabicyclo[2.2.1]hept-5-en-2-ol

To a 1,2-dichloroethane solution (5 mL) of (1R,4S,5R)-5-benzyloxy-1-isopropyl-4-methyl-7-oxabicyclo[2.2.1]hept-2-ene (150 mg, 0.581 mmol), 2,3-dichloro-5,6-dicyano-1,4-benzoquinone (527 mg, 2.32 mmol) was added, followed by stirring at room temperature (25°C) for 5 hours. Saturated sodium bicarbonate water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a white solid title compound (45 mg, 46%).

$^1$H NMR ($CDCl_3$) $\delta$: 1.03 (s, 3H), 1.04 (s, 3H), 1.27 (d, J = 10.5 Hz, 1H), 1.41-1.46 (m, 1H), 1.55 (s, 3H), 2.03-2.13 (m, 2H), 3.85-3.88 (m, 1H), 6.07 (d, J = 6.0 Hz, 1H), 6.35 (d, J = 6.0 Hz, 1H).
Melting point: <60°C

Reference Example 9

Synthesis of (1R,2R,4S,5R)-5-[(2-fluorophenyl)methoxy]-1-isopropyl-4-methyl-7-oxabicyclo[2.2.1]heptan-2-ol

(1R,3S,4R,6R)-4-[(2-fluorophenyl)methoxy]-6-isopropyl-3-methyl-7-oxabicyclo[4.1.0]heptan-3-ol (490 mg, 1.66 mmol) and tosylic acid monohydrate (317 mg, 1.66 mmol) were dissolved in tetrahydrofuran (10 mL) and stirred under heating and reflux for 5 hours. Saturated sodium bicarbonate water was poured into the reaction mixture, which was then extracted with ethyl acetate. The extract was washed with brine and then concentrated under reduced pressure, and the concentrate was purified by silica gel column chromatography (eluent: ethyl acetate/n-hexane) to obtain a white solid title compound (186 mg, 38%).

$^1$H NMR ($CDCl_3$) $\delta$: 0.94 (3H, d, J = 6.9 Hz), 1.06 (3H, d, J = 6.9 Hz), 1.34 (1H, dd, J = 14.2, 1.8 Hz), 1.49 (3H, s), 1.53-1.59 (2H, m), 1.69 (1H, dd, J = 13.5, 2.1 Hz), 2.00 (1H, dd, J = 13.7, 6.9 Hz), 2.35-2.42 (1H, m), 3.44 (1H, dd, J = 6.4, 1.8 Hz), 3.82-3.84 (1H, m), 4.50 (1H, d, J = 12.8 Hz), 4.60 (1H, d, J = 12.8 Hz), 7.03 (1H, t, J = 9.2 Hz), 7.13 (1H, t, J = 7.3 Hz), 7.24-7.25 (1H, m), 7.43 (1H, t, J = 7.6 Hz).
Melting point: 91.0°C to 95.0°C

Next, a method for formulating the compound of the present invention as a herbicide will be specifically explained using the following Formulation Examples. The herbicide is not limited to these Formulation Examples, and can also be formulated by mixing it with various other additives in any ratio.

Unless otherwise specified, "parts" means parts by mass, and "%" means % by mass.

Formulation Example 1 (Granule)

To 1 part of the compound in Synthesis Example 3, 1 part of calcium lignosulfonate, 1 part of lauryl sulfate, 30 parts of bentonite, and 67 parts of talc, 15 parts of water were added, followed by kneading in a kneader and then granulating in an extrusion granulator. The granules were dried in a fluidized bed dryer to obtain granules containing 1% of a herbicidal active ingredient. Further, the same method as in Formulation Example 1 was used except that compounds shown in Tables 1, 2, and 3 were used instead of the compound in Synthesis Example 3 to obtain respective granules.

Formulation Example 2 (Flowable Formulation)

A flowable formulation containing 20% of a herbicidal active ingredient can be obtained by uniformly mixing and grinding 20.0 parts of the compound in Synthesis Example 3, 2.0 parts of sodium di-2-ethylhexyl sulfosuccinate, 2.0 parts of polyoxyethylene nonylphenyl ether, 5.0 parts of propylene glycol, 0.5 parts of an antifoaming agent, and 70.5 parts of water in a wet ball mill. Further, the same method as in Formulation Example 2 was used except that compounds shown in Tables 1, 2, and 3 were used instead of the compound in Synthesis Example 3 to obtain respective flowable formulations.

Formulation Example 3 (Dry Flowable Formulation)

A dry flowable formulation (water dispersible granule) containing 75% of a herbicidal active ingredient can be obtained by uniformly mixing and finely grinding 75 parts of the compound in Synthesis Example 3, 10 parts of a naphthalenesulfonic acid formaldehyde condensate, 5 parts of sodium lauryl sulfate, 5 parts of white carbon, and 5 parts of clay. Further, the same method as in Formulation Example 3 was used except that compounds shown in Tables 1, 2, and 3 were used instead of the compound in Synthesis Example 3 to obtain respective dry flowable formulations (water dispersible granules).

Formulation Example 4 (Wettable Powder)

A wettable powder containing 15% of a herbicidal active ingredient can be obtained by uniformly mixing 15 parts of the compound in Synthesis Example 3, 15 parts of white carbon, 3 parts of calcium lignosulfonate, 2 parts of polyoxyethylene alkyl ether, 5 parts of diatomaceous earth, and 60 parts of clay in a grinding mixer. Further, the same method as in Formulation Example 4 was used except that compounds shown in Tables 1, 2, and 3 were used instead of the compound in Synthesis Example 3 to obtain respective wettable powders.

Formulation Example 5 (Emulsifiable Concentrate)

An emulsifiable concentrate containing 20% of a herbicidal active ingredient can be obtained by mixing 20 parts of the compound in Synthesis Example 3, 18 parts of polyoxyethylene styrylphenyl ether, 2 parts of calcium dodecylbenzenesulfonate and 60 parts of xylene. Further, the same method as in Formulation Example 5 was used except that compounds shown in Tables 1, 2, and 3 were used instead of the compound in Synthesis Example 3 to obtain respective emulsifiable concentrates.

Formulation Example 6 (Dustable Powder)

A dustable powder containing 0.5% of a herbicidal active ingredient can be obtained by uniformly mixing and grinding 0.5 parts of the compound in Synthesis Example 3, 0.5 parts of white carbon, 0.5 parts of calcium stearate, 50.0 parts of clay, and 48.5 parts of talc. Further, the same method as in Formulation Example 6 was used except that compounds shown in Tables 1, 2, and 3 were used instead of the compound in Synthesis Example 3 to obtain respective dustable powders.

Formulation Example 7 (Jumbo Formulation)

15 parts of the compound in Synthesis Example 3, 2 parts of sodium lauryl sulfate, 5 parts of sodium di-2-ethylhexyl sulfosuccinate, 5 parts of carboxymethylcellulose sodium salt, 35 parts of silica balloons, 10 parts of lactose, and 28 parts of expanded perlite were mixed, and then 35 parts of water was added, followed by kneading in a kneader and granulating in an extrusion granulator. The granules were dried in a fluidized bed dryer to obtain a jumbo formulation containing 15% of a herbicidal active ingredient. Further, the same method as in Formulation Example 7 was used except

that compounds shown in Tables 1, 2, and 3 were used instead of the compound in Synthesis Example 3 to obtain respective jumbo formulations.

Next, test examples will be shown to illustrate the herbicidal effect of the 1,4-cineole derivative of the present invention.

Test Example 1

Herbicidal Effect Test by Soil Application in Paddy Rice

Wagner pots with an area of 1/10,000 acre were filled with paddy soil, followed by addition of water and incorporation of chemical fertilizer (N:P:K = 16:16:16), after which puddling was performed. After that, seeds of Echinochloa crus-galli var. formosensis, broad-leaved weeds (Lindernia pyxidaria and Monochoria vaginalis), and Scirpus juncoides were sown at a depth of 0 cm to 1 cm, 30 seeds each. Immediately after sowing, the pots were flooded and a water depth of approximately 3 cm was maintained. Subsequent management was conducted in a glass greenhouse. Immediately thereafter, an emulsifiable concentrate prepared according to Formulation Example 5 using the compound shown below was diluted with water, and a specified amount of the diluted solution was applied by dropper. The amount of the active ingredient to be applied was equivalent to 120 g per 10 ares.

The present test was conducted in duplicate for each chemical concentration group, and the weed inhibition rate (%) was determined 14 days after chemical application using the following formula (Math. 1).

$$\text{Weed inhibition rate (\%)} = (1 - (\text{Average dry weight (g) of plants in application area / Average dry weight (g) of plants in non-application area})) \times 100 \quad \text{[Math. 1]}$$

As representative examples, the compounds of the above-mentioned compound Nos. 1-2, 1-5, 1-7, 1-8, 1-9, 1-10, 1-11, 1-12, 1-13, 1-14, 1-15, 1-16, 1-17, 1-18, 1-19, 1-20, 1-21, 1-24, 1-30, 1-41, 1-68, 1-83, 1-92, 1-93, 1-94, 1-111, 1-123, 1-126, 1-133, 1-150, 1-151, 1-156, 1-183, 1-208, 1-213, 1-223, 1-313, 1-322, 1-480, 1-525, 1-597, 1-603, 1-612, 1-614, 1-627, 1-640, 1-652, 1-653, 1-658, 1-661, 1-662, 1-663, 1-669, 1-672, 1-673, 1-674, 1-676, 1-680, 1-681, 1-682, 1-690, 1-691, 1-692, 1-693, 1-694, 1-695, 1-696, 1-700, 1-701, 1-703, 1-705, 1-712, 1-713, 1-715, 1-716, 1-717, 1-718, 1-721, 1-722, 1-723, 1-732, 1-734, 1-736, 1-740, 1-741, 1-747, 1-750, 1-753, 1-756, 1-759, 1-762, 1-765, 1-768, 1-777, 1-778, 1-779, 1-780, 1-785, 1-788, 1-789, 1-790, 1-799, 1-801, 1-914, 1-915, 1-920, 1-921, 1-922, 1-923, 1-924, 1-926, 1-928, 1-929, 1-930, 1-931, 1-932, 1-935, 1-936, 1-1007, 1-1015, 1-1016, 1-1022, 1-1031, 1-1034, 1-1037, 1-1043, 1-1052, 1-1053, 1-1054, 1-1059, 1-1073, 1-1081, 1-1088, 1-1094, 1-1097, 1-1109, 1-1118, 1-1119, 1-1120, 1-1121, 1-1126, 1-1292, 1-1295, 1-1298, 1-1301, 1-1304, 1-1313, 1-1314, 1-1315, 1-1320, 1-1323, 1-1324, 1-1325, 1-1334, 1-1336, 1-1342, 1-1343, 1-1344, 1-1345, 1-1346, 1-1349, 1-1350, 1-1351, 1-1352, 1-1355, 1-1356, 1-1357, 1-1358, 1-1359, 1-1360, 1-1361, 1-1362, 1-1363, 1-1364, 1-1365, 1-1367, 1-1370, 1-1376, 1-1379, 1-1380, 1-1381, 1-1383, 1-1385, 1-1386, 1-1389, 1-1390, 1-1400, 1-1402, 1-1408, 1-1409, 1-1415, 1-1418, 1-1421, 1-1424, 1-1427, 1-1430, 1-1433, 1-1436, 1-1445, 1-1446, 1-1447, 1-1448, 1-1456, 1-1457, 1-1555, 1-1597, 1-1598, 1-1599, 1-1600, 1-1601, 1-1602, 1-1603, 1-1604, 1-1605, 1-1606, 1-1607, 1-1608, 1-1609, 1-1610, 1-1611, 1-1612, 1-1613, 1-1614, 1-1615, 2-1, 2-3, 2-5, 2-7, 2-9, 2-11, 2-13, 2-14, 2-16, 2-18, 2 -20, 2-26, 2-28, 2-29, 2-72, 2-88, 2-191, 2-197, 2-347, 2-348, 2-349, 2-350, 2-351, 2-352, 2-353, 2-354, 2-355, 3-1, 3-2, 3-3, 3-5, 3-7, 3-175, 3-177, 3-680, 3-693, 3-697, 3-732, 3-738, 3-748, 3-749, 3-750, 3-752, 3-753, 3-754, 3-755, 3-756, and 3-757 exhibited a weed inhibition rate of 80% or higher against Echinochloa crus-galli var. formosensis.

As representative examples, the compounds of the above-mentioned compound Nos. 1-7, 1-16, 1-19, 1-603, 1-703, 1-741, 1-921, 1-922, 1-924, 1-930, 1-931, 1-935, 2-3, 2-5, 2-20, 2-72, 2-88, 2-197, 2-349, 2-351 and 3-5 exhibited a weed inhibition rate of 80% or higher against Lindernia pyxidaria.

As representative examples, the compounds of the above-mentioned compound Nos. 1-2, 1-5, 1-7, 1-12, 1-15, 1-16, 1-19, 1-20, 1-21, 1-24, 1-30, 1-41, 1-83, 1-92, 1-94, 1-111, 1-123, 1-126, 1-133, 1-151, 1-156, 1-183, 1-208, 1-213, 1-223, 1-525, 1-597, 1-603, 1-672, 1-680, 1-681, 1-690, 1-691, 1-692, 1-693, 1-694, 1-703, 1-712, 1-713, 1-717, 1-721, 1-722, 1 -741, 1-753, 1-914, 1-915, 1-920, 1-921, 1-922, 1-924, 1-929, 1-930, 1-931, 1-935, 1-936, 1-1355, 1-1600, 1-1603, 1-1604, 2-1, 2-3, 2-5, 2-9, 2-13, 2-14, 2-20, 2-26, 2-28, 2-72, 2-88, 2-191, 2-197, 2-349, 2-350, 2-351, 2-353, 2-354, 2-355, 3-1, 3-2, 3-3, 3-5, 3-7, 3-175, and 3-177 exhibited a weed inhibition rate of 80% or higher against Monochoria vaginalis.

As representative examples, the compounds of the above-mentioned compound Nos. 1-2, 1-5, 1-7, 1-8, 1-9, 1-10, 1-11, 1-12, 1-13, 1-14, 1-15, 1-16, 1-19, 1-20, 1-21, 1-24, 1-30, 1-41, 1-83, 1-92, 1-94, 1-111, 1-126, 1-133, 1-150, 1-151, 1-156, 1-183, 1-208, 1-213, 1-223, 1-480, 1-525, 1-597, 1-603, 1-612, 1-614, 1-627, 1-640, 1-652, 1-653, 1-658, 1-661, 1-662, 1-663, 1-672, 1-674, 1-676, 1-680, 1-681, 1-690, 1-691, 1-692, 1-693, 1-703, 1-712, 1-713, 1-717, 1-721, 1-722, 1-732, 1-734, 1-736, 1-740, 1-741, 1-747, 1-750, 1-753, 1-756, 1-759, 1-762, 1-765, 1-768, 1-777, 1-778, 1-779,

1-780, 1-785, 1-786, 1-788, 1-789, 1-790, 1-799, 1-801, 1-914, 1-915, 1-920, 1-921, 1-922, 1-923, 1-924, 1-926, 1-929, 1-930, 1-931, 1-935, 1-936, 1-1115, 1-1119, 1-1126, 1-1292, 1-1295, 1-1298, 1-1301, 1-1304, 1-1310, 1-1313, 1-1314, 1-1315, 1-1320, 1-1321, 1-1322, 1-1323, 1-1324, 1-1325, 1-1334, 1-1336, 1-1342, 1-1355, 1-1386, 1-1389, 1-1390, 1-1400, 1-1402, 1-1408, 1-1409, 1-1415, 1-1418, 1-1421, 1-1424, 1-1427, 1-1430, 1-1433, 1-1436, 1-1442, 1-1445, 1-1446, 1-1447, 1-1448, 1-1456, 1-1457, 1-1555, 1-1600, 1-1602, 1-1603, 1-1604, 1-1607, 1-1610, 1-1611, 1-1612, 1-1613, 1-1614, 1-1615, 2-3, 2-5, 2-9, 2-11, 2-13, 2-14, 2-16, 2-18, 2-20, 2-26, 2-28, 2-29, 2-72, 2-88, 2-191, 2-197, 2-349, 2-350, 2-351, 2-352, 2-353, 2-354, 2-355, 3-1, 3-2, 3-3, 3-5, 3-7, 3-177, 3-680, 3-693, 3-697, 3-732, 3-738, 3-748, 3-749, 3-750, 3-752, 3-753, 3-754, 3-755, 3-756, and 3-757 exhibited a weed inhibition rate of 80% or higher against Scirpus juncoides.

Test Example 2

Herbicidal Effect Test by Application During Growth Period of Paddy Rice

Wagner pots with an area of 1/10,000 acre were filled with paddy soil, followed by addition of water and incorporation of chemical fertilizer (N:P:K = 16:16:16), after which puddling was performed. After that, seeds of Echinochloa crus-galli var. formosensis, broad-leaved weeds (Monochoria vaginalis), and Scirpus juncoides were sown at a depth of 0 cm to 1 cm, 30 seeds each. Immediately after sowing, the pots were flooded and a water depth of approximately 3 cm was maintained. Subsequent management was conducted in a glass greenhouse. Seven days after sowing, an emulsifiable concentrate prepared according to Formulation Example 5 using the compound shown below was diluted with water, and a specified amount of the diluted solution was applied by dropper. The amount of the active ingredient to be applied was equivalent to 120 g per 10 ares. The test was conducted in duplicate for each chemical concentration group, and the herbicidal effect was evaluated 14 days after chemical application using the same criteria as in Test Example 1.

As representative examples, the compounds of the above-mentioned compound Nos. 1-2, 1-5, 1-7, 1-8, 1-9, 1-10, 1-11, 1-12, 1-13, 1-14, 1-15, 1-16, 1-17, 1-18, 1-19, 1-20, 1-21, 1-24, 1-30, 1-41, 1-68, 1-83, 1-92, 1-93, 1-94, 1-111, 1-123, 1-126, 1-133, 1-150, 1-151, 1-156, 1-183, 1-208, 1-213, 1-223, 1-313, 1-322, 1-480, 1-525, 1 -597, 1-603, 1-612, 1-614, 1-627, 1-640, 1-652, 1-653, 1-658, 1-661, 1-662, 1-663, 1-669, 1-672, 1-673, 1-674, 1-676, 1-677, 1-680, 1-681, 1-682, 1-690, 1-691, 1-692, 1-693, 1-694, 1-695, 1-696, 1-700, 1-701, 1-703, 1-705, 1-712, 1-713, 1-715, 1-716, 1-717, 1-718, 1-721, 1-722, 1-723, 1-725, 1-732, 1-734, 1-736, 1-740, 1-741, 1-747, 1-750, 1-753, 1-756, 1-759, 1-762, 1-765, 1-768, 1-777, 1-778, 1-779, 1-780, 1-785, 1-788, 1-789, 1-790, 1-799, 1-801, 1-914, 1-915, 1-920, 1-921, 1-922, 1-923, 1-924, 1-926, 1-928, 1-929, 1-930, 1-931, 1-932, 1-934, 1-935, 1-936, 1-1007, 1-1015, 1-1016, 1-1022, 1-1031, 1-1034, 1-1037, 1-1043, 1-1052, 1-1053, 1-1054, 1-1059, 1-1073, 1-1081, 1-1088, 1-1094, 1-1097, 1-1109, 1-1118, 1-1119, 1-1120, 1-1121, 1-1126, 1-1292, 1-1295, 1-1298, 1-1301, 1-1304, 1-1313, 1-1314, 1-1315, 1-1320, 1-1323, 1-1324, 1-1325, 1-1334, 1-1336, 1-1342, 1-1343, 1-1344, 1-1345, 1-1346, 1-1349, 1-1350, 1-1351, 1-1352, 1-1355, 1-1356, 1-1357, 1-1358, 1-1359, 1-1360, 1-1361, 1-1362, 1-1363, 1-1364, 1-1365, 1-1367, 1-1370, 1-1376, 1-1379, 1-1380, 1-1381, 1-1383, 1-1385, 1-1386, 1-1389, 1-1390, 1-1400, 1-1402, 1-1408, 1-1409, 1-1415, 1-1418, 1-1421, 1-1424, 1-1427, 1-1430, 1-1433, 1-1436, 1-1445, 1-1446, 1-1447, 1-1448, 1-1456, 1-1457, 1-1555, 1-1597, 1-1598, 1-1599, 1-1600, 1-1601, 1-1602, 1-1603, 1-1604, 1-1605, 1-1606, 1-1607, 1-1608, 1-1609, 1-1610, 1-1611, 1-1612, 1-1613, 1-1614, 1-1615, 2-1, 2-3, 2-5, 2-7, 2-9, 2-11, 2-13, 2-14, 2-16, 2-18, 2-20, 2-26, 2-28, 2-29, 2-72, 2-88, 2-191, 2-197, 2-346, 2-348, 2-349, 2-350, 2-351, 2-352, 2-353, 2-354, 2-355, 3-1, 3-2, 3-3, 3-5, 3-7, 3-175, 3-177, 3-680, 3-693, 3-697, 3-732, 3-738, 3-748, 3-749, 3-750, 3-752, 3-753, 3-754, 3-755, 3-756, and 3-757 exhibited a weed inhibition rate of 80% or higher against Echinochloa crus-galli var. formosensis.

As representative examples, the compounds of the above-mentioned compound Nos. 1-2, 1-19, 1-672, 1-703, 1-921, 1-930, 1-931, 1-935, 2-3, 2-5, 2-11, 2-20, 2-72, 2-88, 2-349, 2-351, 2-352, and 3-5 exhibited a weed inhibition rate of 80% or higher against Lindernia pyxidaria.

As representative examples, the compounds of the above-mentioned compound Nos. 1-2, 1-5, 1-7, 1-12, 1-15, 1-19, 1-20, 1-21, 1-24, 1-30, 1-41, 1-83, 1-92, 1-94, 1-111, 1-133, 1-151, 1-156, 1-183, 1-597, 1-603, 1-672, 1-680, 1-681, 1-690, 1-691, 1-692, 1-693, 1-694, 1-703, 1-712, 1-713, 1-717, 1-721, 1-722, 1-741, 1-753, 1-914, 1-915, 1-920, 1-921, 1-922, 1-929, 1-930, 1-931, 1-935, 1-1355, 1-1600, 1-1602, 1-1603, 1-1604, 2-1, 2-3, 2-5, 2-9, 2-11, 2-13, 2-18, 2-20, 2-28, 2-72, 2-88, 2-191, 2-197, 2-349, 2-351, 2-353, 2-355, 3-1, 3-2, 3-5, 3-175, and 3-177 exhibited a weed inhibition rate of 80% or higher against Monochoria vaginalis.

As representative examples, the compounds of the above-mentioned compound Nos. 1-2, 1-5, 1-7, 1-8, 1-9, 1-10, 1-11, 1-12, 1-13, 1-14, 1-15, 1-19, 1-20, 1-21, 1-24, 1-30, 1-41, 1-83, 1-92, 1-111, 1-133, 1-150, 1-151, 1-156, 1-183, 1-208, 1-213, 1-223, 1-480, 1-597, 1-603, 1-612, 1-614, 1-627, 1-640, 1-652, 1-653, 1-658, 1-661, 1-662, 1-663, 1-669, 1-672, 1-674, 1-680, 1-681, 1-690, 1-691, 1-692, 1-693, 1-703, 1-712, 1-713, 1-717, 1-722, 1-732, 1-734, 1-736, 1-740, 1-741, 1-747, 1-750, 1-753, 1-756, 1-759, 1-762, 1-765, 1-768, 1-777, 1-778, 1-779, 1-780, 1-785, 1-786, 1-788, 1-789, 1-790, 1-799, 1-801, 1-914, 1-915, 1-920, 1-921, 1-930, 1-931, 1-935, 1-1115, 1-1119, 1-1126, 1-1292, 1-1295, 1-1298,

1-1301, 1-1304, 1-1310, 1-1313, 1-1314, 1-1315, 1-1320, 1-1321, 1-1322, 1-1323, 1-1324, 1-1325, 1-1334, 1-1336, 1-1342, 1-1355, 1-1386, 1-1389, 1-1390, 1-1400, 1-1402, 1-1408, 1-1409, 1-1415, 1-1418, 1-1421, 1-1424, 1-1427, 1-1430, 1-1433, 1-1436, 1-1442, 1-1445, 1-1446, 1-1447, 1-1448, 1-1456, 1-1457, 1-1555,1-1600, 1-1602, 1-1603, 1-1604, 1-1607, 1-1610, 1-1611, 1-1612, 1-1613, 1-1614, 1-1615, 2-3, 2-9, 2-11, 2-13, 2-18, 2-20, 2-28, 2-72, 2-88, 2-191, 2-197, 2-351, 2-352, 2-353, 2-354, 2-355, 3-1, 3-2, 3-3, 3-5, 3-7, 3-177, 3-680, 3-693, 3-697, 3-732, 3-738, 3-748, 3-749, 3-750, 3-752, 3-753, 3-754, 3-755, 3-756, and 3-757 exhibited a weed inhibition rate of 80% or higher against Scirpus juncoides.

Test Example 3

Herbicidal Effect Test by Soil Application in Upland Field

Pots with an area of 36 $cm^2$ were filled with upland soil (alluvial loam), twenty seeds each of weeds of Digitaria ciliaris, Echinochloa crus-galli, Chenopodium album, and Amaranthus viridis were uniformly mixed with the top 1 cm layer of soil and the surface was lightly pressed down. One day after sowing, an emulsifiable concentrate prepared according to Formulation Example 5 using the compound shown below was diluted with water, and the diluted solution was sprayed onto the soil surface at a rate of 100 liters per 10 ares. The amount of the active ingredient to be applied was equivalent to 120 g per 10 ares. Fourteen days after chemical application, the herbicidal effect was evaluated according to the same criteria as in Test Example 1.

As representative examples, the compounds of the above-mentioned compound Nos. 1-2, 1-5, 1-7, 1-8, 1-9, 1-10, 1-11, 1-12, 1-13, 1-14, 1-15, 1-16, 1-17, 1-19, 1-20, 1-21, 1-24, 1-30, 1-41, 1-68, 1-83, 1-92, 1-93, 1-94, 1-111, 1-123, 1-126, 1-133, 1-150, 1-151, 1-156, 1-183, 1-208, 1-213, 1-223, 1-480, 1- 525, 1-603, 1-612, 1-614, 1-652, 1-653, 1-658, 1-661, 1-662, 1-669, 1-672, 1-674, 1-676, 1-680, 1-681, 1-682, 1-690, 1-691, 1-692, 1-693, 1-694, 1-695, 1-696, 1-700, 1-701, 1-703, 1-705, 1-712, 1-713, 1-717, 1-718, 1-721, 1-722, 1-732, 1-734, 1-736, 1-740, 1-741, 1-747, 1-753, 1-756, 1-759, 1-762, 1-768, 1-777, 1-778, 1-779, 1-780, 1-785, 1-788, 1-789, 1-799, 1-801, 1-914, 1-915, 1-921, 1-927, 1-929, 1-930, 1-931, 1-935, 1-936, 1-1007, 1-1015, 1-1016, 1-1022, 1-1031, 1-1034, 1-1037, 1-1043, 1-1052, 1-1053, 1-1054, 1-1059, 1-1073, 1-1081, 1-1088, 1-1094, 1-1097, 1-1109, 1-1118, 1-1119, 1-1120, 1-1121, 1-1126, 1-1292, 1-1295, 1-1298, 1-1304, 1-1313, 1-1314, 1-1315, 1-1320, 1-1323, 1-1324, 1-1334, 1-1336, 1-1342, 1-1343, 1-1344, 1-1345, 1-1346, 1-1349, 1-1350, 1-1351, 1-1352, 1-1355, 1-1356, 1-1357, 1-1358, 1-1359, 1-1360, 1-1361, 1-1362, 1-1363, 1-1364, 1-1365, 1-1367, 1-1370, 1-1376, 1-1379, 1-1380, 1-1381, 1-1383, 1-1385, 1-1386, 1-1389, 1-1390, 1-1400, 1-1402, 1-1408, 1-1409, 1-1415, 1-1421, 1-1424, 1-1427, 1-1430, 1-1436, 1-1445, 1-1446, 1-1447, 1-1448, 1-1456, 1-1457, 1-1555, 1-1597, 1-1598, 1-1599, 1-1600, 1-1601, 1-1602, 1-1603, 1-1604, 1-1605, 1-1606, 1-1607, 1-1608, 1-1609, 1-1610, 1-1611, 1-1612, 1-1613, 1-1614, 1-1615, 2-1, 2-3, 2-5, 2-9, 2-11, 2-13, 2-14, 2-16, 2-18, 2-20, 2-26, 2-28, 2-29, 2-72, 2-88, 2-191, 2-197, 2-348, 2-349, 2-350, 2-351, 2-352, 2-353, 2-354, 2-355, 3-1, 3-2, 3-3, 3-5, 3-7, 3-175, 3-177, 3-680, 3-693, 3-697, 3-732, 3-738, 3-748, 3-749, 3-750, 3-752, 3-753, 3-754, 3-755, 3-756, and 3-757 exhibited a weed inhibition rate of 80% or higher against Digitaria ciliaris.

As representative examples, the compounds of the above-mentioned compound Nos. 1-2, 1-5, 1-7, 1-8, 1-9, 1-10, 1-11, 1-12, 1-13, 1-14, 1-15, 1-16, 1-17, 1-19, 1-20, 1-21, 1-24, 1-30, 1-41, 1-68, 1-83, 1-92, 1-93, 1-94, 1-111, 1-123, 1-126, 1-133, 1-150, 1-151, 1-156, 1-183, 1-208, 1-213, 1-223, 1-313, 1-322, 1-480, 1-525, 1-603, 1-612, 1-614, 1-652, 1-653, 1-658, 1-661, 1-662, 1-669, 1-672, 1-674, 1-676, 1-680, 1-681, 1-682, 1-690, 1-691, 1-692, 1-693, 1-694, 1-695, 1-696, 1-700, 1-701, 1-703, 1-705, 1-712, 1-713, 1-717, 1-718, 1-721, 1-722, 1-732, 1-734, 1-736, 1-740, 1-741, 1-747, 1-753, 1-756, 1-759, 1-762, 1-768, 1-777, 1-778, 1-779, 1-780, 1-785, 1-788, 1-789, 1-799, 1-801, 1-914, 1-915, 1-921, 1-922, 1-923, 1-924, 1-926, 1-927, 1-929, 1-930, 1-931, 1- 935, 1-936, 1-1007, 1-1015, 1-1016, 1-1022, 1-1031, 1-1034, 1-1037, 1-1043, 1-1052, 1-1053, 1-1054, 1-1059, 1-1073, 1-1081, 1-1088, 1-1094, 1-1097, 1-1109, 1-1118, 1-1119, 1-1120, 1-1121, 1-1126, 1-1292, 1-1295, 1-1298, 1-1304, 1-1313, 1-1314, 1-1315, 1-1320, 1-1323, 1-1324, 1-1334, 1-1336, 1-1342, 1-1343, 1-1344, 1-1345, 1-1346, 1-1349, 1-1350, 1-1351, 1-1352, 1-1355, 1-1356, 1-1357, 1-1358, 1-1359, 1-1360, 1-1361, 1-1362, 1-1363, 1-1364, 1- 1365, 1-1367, 1-1370, 1-1376, 1-1379, 1-1380, 1-1381, 1-1383, 1-1385, 1-1386, 1-1389, 1-1390, 1-1400, 1-1402, 1-1408, 1-1409, 1-1415, 1-1421, 1-1424, 1-1427, 1-1430, 1-1436, 1-1445, 1-1446, 1-1447, 1-1448, 1-1456, 1-1457, 1-1555, 1-1597, 1-1598, 1-1599, 1-1600, 1-1601, 1-1602, 1-1603, 1-1604, 1-1605, 1-1606, 1-1607, 1-1608, 1-1609, 1-1610, 1-1611, 1-1612, 1-1613, 1-1614, 1-1615, 2-1, 2-3, 2-5, 2-9, 2-11, 2-13, 2-14, 2-16, 2-18, 2-20, 2-26, 2-28, 2-29, 2-72, 2-88, 2-191, 2-197, 2-347, 2-348, 2-349, 2-350, 2-351, 2-352, 2-353, 2-354, 2-355, 3-1, 3-2, 3-3, 3-5, 3-7, 3-175, 3-177, 3-680, 3-693, 3-697, 3-732, 3-738, 3-748, 3-749, 3-750, 3-752, 3-753, 3-754, 3-755, 3-756, and 3-757 exhibited a weed inhibition rate of 80% or higher against Echinochloa crus-galli.

As representative examples, the compounds of the above-mentioned compound Nos. 1-2, 1-5, 1-7, 1-19, 1-30, 1-41, 1-83, 1-111, 1-123, 1-126, 1-156, 1-322, 1-603, 1-921, 1-926, 1-930, 1-931, 2-3, 2-9, 2-11, 2-20, 2-29, 2-72, 2-88, 2-349, 2-350, and 3-5 exhibited a weed inhibition rate of 80% or higher against Chenopodium album.

As representative examples, the compounds of the above-mentioned compound Nos. 1-7, 1-30, 1-41, 1-68, 1-156, 1-208, 1-213, 1-223, 1-672, 1-694, 1-703, 1-725, 1-753, 1-921, 1-926, 1-930, 1-931, 2-9, 2-11, 2-20, 2-29, 2-72,

2-88, 2-197, 2-346, 2-349, 2-351, 3-2, and 3-5 exhibited a weed inhibition rate of 80% or higher against Amaranthus viridis.

Test Example 4

Herbicidal Effect Test by Stem and Leaf Treatment in Upland Field

Pots with an area of 36 $cm^2$ were filled with upland soil (alluvial loam), twenty seeds each of weeds of Digitaria ciliaris, Echinochloa crus-galli, and Amaranthus viridis were uniformly mixed with the top 1 cm layer of soil and the surface was lightly pressed down. Seven days after sowing, an emulsifiable concentrate prepared according to Formulation Example 5 using the compound shown below was diluted with water, and the diluted solution was sprayed onto the soil surface at a rate of 100 liters per 10 ares. The amount of the active ingredient to be applied was equivalent to 120 g per 10 ares. Fourteen days after chemical application, the herbicidal effect was evaluated according to the same criteria as in Test Example 1.

As representative examples, the compounds of the above-mentioned compound Nos. 1-2, 1-5, 1-7, 1-8, 1-9, 1-10, 1-11, 1-12, 1-13, 1-14, 1-15, 1-16, 1-17, 1-18, 1-19, 1-20, 1-21, 1-24, 1-30, 1-41, 1-83, 1-92, 1-93, 1-94, 1-111, 1-123, 1-126, 1-133, 1-150, 1-151, 1-156, 1-183, 1-208, 1-213, 1-223, 1-322, 1-480, 1-525, 1-597, 1-603, 1-612, 1-614, 1-652, 1-653, 1-658, 1-661, 1-662, 1-669, 1-672, 1-674, 1-676, 1-680, 1-681, 1-682, 1-686, 1-690, 1-691, 1-692, 1-693, 1-695, 1-696, 1-700, 1-701, 1-703, 1-705, 1-706, 1-709, 1-712, 1-713, 1-717, 1- 718, 1-721, 1-722, 1-732, 1-734, 1-736, 1-740, 1-741, 1-747, 1-756, 1-759, 1-762, 1-768, 1-777, 1-778, 1-779, 1-780, 1-785, 1-788, 1-789, 1-799, 1-801, 1-914, 1-915, 1-916, 1-920, 1-921, 1-922, 1-924, 1-928, 1-929, 1-930, 1-931, 1-934, 1-935, 1-1007, 1-1015, 1-1016, 1-1022, 1-1028, 1-1031, 1-1034, 1-1037, 1-1043, 1-1052, 1-1053, 1-1054, 1-1059, 1-1073, 1-1081, 1-1088, 1-1094, 1-1097, 1-1109, 1-1118, 1-1119, 1-1120, 1-1121, 1-1126, 1-1221, 1-1292, 1-1295, 1-1298, 1-1304, 1-1313, 1-1314, 1-1315, 1-1320, 1-1323, 1-1324, 1-1334, 1-1336, 1-1342, 1-1343, 1-1344, 1-1345, 1-1346, 1-1349, 1-1350, 1-1351, 1-1352, 1-1355, 1-1356, 1-1357, 1-1358, 1-1359, 1-1360, 1-1361, 1-1362, 1-1363, 1-1364, 1-1365, 1-1367, 1-1370, 1-1376, 1-1379, 1-1380, 1-1381, 1-1383, 1-1385, 1-1386, 1-1389, 1-1390, 1-1400, 1-1402, 1-1408, 1-1409, 1-1415, 1-1421, 1-1424, 1-1427, 1-1430, 1-1436, 1-1445, 1-1446, 1-1447, 1-1448, 1-1456, 1-1457, 1-1555, 1-1597, 1-1598, 1-1599, 1-1600, 1-1601, 1-1602, 1-1603, 1-1604, 1-1605, 1-1606, 1-1607, 1-1608, 1-1609, 1-1610, 1-1611, 1-1612, 1-1613, 1-1614, 1-1615, 2-1, 2-3, 2-5, 2-7, 2-9, 2-11, 2-13, 2-14, 2-16, 2-18, 2-20, 2-26, 2-28, 2-29, 2-72, 2-88, 2-191, 2-197, 2-348, 2-349, 2-350, 2-351, 2-352, 2-353, 2-354, 2-355, 3-1, 3-3, 3-5, 3-7, 3-175, 3-177, 3-680, 3-693, 3-697, 3-732, 3-738, 3-748, 3-749, 3-750, 3-752, 3-753, 3-754, 3-755, 3-756, and 3-757 exhibited a weed inhibition rate of 80% or higher against Digitaria ciliaris.

As representative examples, the compounds of the above-mentioned compound Nos. 1-2, 1-5, 1-7, 1-8, 1-9, 1-10, 1-11, 1-12, 1-13, 1-14, 1-15, 1-16, 1-17, 1-18, 1-19, 1-20, 1-21, 1-24, 1-30, 1-41, 1-68, 1-83, 1-92, 1-93, 1-94, 1-111, 1-123, 1-126, 1-133, 1-150, 1-151, 1-156, 1-183, 1-208, 1-213, 1-223, 1-313, 1-322, 1-480, 1-525, 1-597, 1-603, 1-612, 1-614, 1-652, 1-653, 1-658, 1-661, 1-662, 1-669, 1-671, 1-672, 1-674, 1-676, 1-680, 1-681, 1-682, 1-686, 1-690, 1-691, 1-692, 1-693, 1-694, 1-695, 1-696, 1-700, 1-701, 1-703, 1-705, 1-706, 1-709, 1-712, 1-713, 1-717, 1-718, 1-721, 1-722, 1-725, 1-732, 1-734, 1-736, 1-740, 1-741, 1-747, 1-753, 1-756, 1-759, 1-762, 1-768, 1-777, 1-778, 1-779, 1-780, 1-785, 1-788, 1-789, 1-799, 1-801, 1-914, 1-915, 1-916, 1-920, 1-921, 1-922, 1-923, 1-924, 1-928, 1-929, 1-930, 1-931, 1-934, 1-935, 1-936, 1-1007, 1-1015, 1-1016, 1-1022, 1-1028, 1-1031, 1-1034, 1-1037, 1-1043, 1-1052, 1-1053, 1-1054, 1-1059, 1-1073, 1-1081, 1-1088, 1-1094, 1-1097, 1-1109, 1-1118, 1-1119, 1-1120, 1-1121, 1-1126, 1-1222, 1-1292, 1-1295, 1-1298, 1-1304, 1-1313, 1-1314, 1-1315, 1-1320, 1-1323, 1-1324, 1-1334, 1-1336, 1-1342, 1-1343, 1-1344, 1-1345, 1-1346, 1-1349, 1-1350, 1-1351, 1-1352, 1-1355, 1-1356, 1-1357, 1-1358, 1-1359, 1-1360, 1-1361, 1-1362, 1-1363, 1-1364, 1-1365, 1-1367, 1-1370, 1-1376, 1-1379, 1-1380, 1-1381, 1-1383, 1-1385, 1-1386, 1-1389, 1-1390, 1-1400, 1-1402, 1-1408, 1-1409, 1-1415, 1-1421, 1-1424, 1-1427, 1-1430, 1-1436, 1-1445, 1-1446, 1-1447, 1-1448, 1-1456, 1-1457, 1-1555, 1-1597, 1-1598, 1-1599, 1-1600, 1-1601, 1-1602, 1-1603, 1-1604, 1-1605, 1-1606, 1-1607, 1-1608, 1-1609, 1-1610, 1-1611, 1-1612, 1-1613, 1-1614, 1-1615, 2-1, 2-3, 2-5, 2-7, 2-9, 2-11, 2-13, 2-14, 2-16, 2-18, 2 -20, 2-26, 2-28, 2-29, 2-72, 2-88, 2-191, 2-197, 2-348, 2-349, 2-350, 2-351, 2-352, 2-353, 2-354, 2-355, 3-1, 3-2, 3-3, 3-5, 3-7, 3-175, 3-177, 3-680, 3-693, 3-697, 3-732, 3-738, 3-748, 3-749, 3-750, 3-752, 3-753, 3-754, 3-755, 3-756, and 3-757 exhibited a weed inhibition rate of 80% or higher against Echinochloa crus-galli.

As representative examples, the compounds of the above-mentioned compound Nos. 1-2, 1-5, 1-7, 1-19, 1-83, 1-92, 1-603, 1-921, 1-924, 1-930, 1-931, 2-3, 2-72, 2-88, 2-351, and 3-5 exhibited a weed inhibition rate of 80% or higher against Chenopodium album.

As representative examples, the compounds of the above-mentioned compound Nos. 1-2, 1-92, 1-921, 1-930, 1-931, 2-20, 2-191, 2-197, 2-349, and 3-5 exhibited a weed inhibition rate of 80% or higher against Amaranthus viridis.

Test Example 5

Herbicidal Effect Test by Soil Application in Paddy Rice

Wagner pots with an area of 1/10,000 acre were filled with paddy soil, followed by addition of water and incorporation of chemical fertilizer (N:P:K = 16:16:16), after which puddling was performed. After that, seeds of Echinochloa crus-galli var. formosensis, broad-leaved weeds (Monochoria vaginalis), and Scirpus juncoides were sown at a depth of 0 cm to 1 cm, 30 seeds each. Immediately after sowing, the pots were flooded and a water depth of approximately 3 cm was maintained. Subsequent management was conducted in a glass greenhouse. Immediately thereafter, an emulsifiable concentrate prepared according to Formulation Example 5 using the compound shown below was diluted with water, and a specified amount of the diluted solution was applied by dropper. The amount of the active ingredient to be applied was equivalent to 7.5 g per 10 ares.

The present test was conducted in duplicate for each chemical concentration group, and the herbicidal effect was evaluated 14 days after chemical application using the same criteria as in Test Example 1.

As representative examples, the compounds of the above-mentioned compound Nos. 1-2, 1-5, 1-7, 1-8, 1-9, 1-10, 1-11, 1-12, 1-13, 1-14, 1-15, 1-16, 1-17, 1-18, 1-19, 1-20, 1-21, 1-24, 1-30, 1-41, 1-68, 1-83, 1-92, 1-94, 1-111, 1-123, 1-126, 1-133, 1-150, 1-151, 1-156, 1-183, 1-208, 1-213, 1-223, 1-313, 1-322, 1-525, 1-603, 1-612, 1-614, 1-652, 1-653, 1-658, 1-661, 1-662, 1-672, 1-674, 1-676, 1-680, 1-681, 1-682, 1-690, 1-691, 1-692, 1-693, 1-694, 1-703, 1-712, 1-713, 1-715, 1-717, 1-718, 1-721, 1-722, 1-723, 1-732, 1-734, 1-736, 1-740, 1-741, 1-747, 1-753, 1-756, 1-759, 1-762, 1-768, 1-777, 1-778, 1-779, 1-780, 1-785, 1-788, 1-789, 1-799, 1-801, 1-914, 1-915, 1-920, 1-921, 1-922, 1-928, 1-929, 1-930, 1-931, 1-932, 1-935, 1 -1119, 1-1292, 1-1295, 1-1298, 1-1304, 1-1313, 1-1314, 1-1315, 1-1320, 1-1323, 1-1324, 1-1334, 1-1342, 1-1343, 1-1349, 1-1355, 1-1357, 1-1358, 1-1361, 1-1379, 1-1381, 1-1386, 1-1389, 1-1400, 1-1402, 1-1408, 1-1409, 1-1415, 1-1421, 1-1424, 1-1427, 1-1430, 1-1436, 1-1445, 1-1446, 1-1447, 1-1448, 1-1456, 1-1457, 1-1555, 1-1598, 1-1600, 1-1602, 1-1603, 1-1604, 1-1610, 1-1611, 1-1612, 1-1613, 1-1614, 1-1615, 2-3, 2-5, 2-7, 2-9, 2-11, 2-13, 2-14, 2-16, 2-18, 2-20, 2-26, 2-28, 2-29, 2-72, 2-88, 2-191, 2-197, 2-349, 2-350, 2-351, 2-352, 2-353, 2-354, 2-355, 3-1, 3-2, 3-3, 3-5, 3-7, 3-680, 3-693, 3-697, 3-752, 3-753, 3-754, 3-755, 3-756, and 3-757 exhibited a weed inhibition rate of 80% or higher against Echinochloa crus-galli var. formosensis.

As representative examples, the compounds of the above-mentioned compound Nos. 1-603, 1-741, 1-921, 1-930, 1-931, 2-5, and 2-20 exhibited a weed inhibition rate of 80% or higher against Lindernia pyxidaria.

As representative examples, the compounds of the above-mentioned compound Nos. 1-2, 1-5, 1-15, 1-20, 1-21, 1-24, 1-83, 1-92, 1-94, 1-133, 1-156, 1-183, 1-603, 1-672, 1-690, 1-691, 1-692, 1-694, 1-712, 1-713, 1-721, 1-722, 1-741, 1-753, 1-921, 1-930, 1-931, 1-1355, 1-1603, 1-1604, 2-3, 2-5, 2-9, 2-13, 2-14, 2-20, 2-28, 2-72, 2-88, 2-191, 2-197, 2-351, 2-353, 2-355, 3-1, 3-2, 3-3, and 3-7 exhibited a weed inhibition rate of 80% or higher against Monochoria vaginalis.

As representative examples, the compounds of the above-mentioned compound Nos. 1-5, 1-7, 1-15, 1-19, 1-20, 1-21, 1-24, 1-30, 1-41, 1-83, 1-92, 1-94, 1-133, 1-151, 1-156, 1-183, 1-672, 1-691, 1-692, 1-721, 1-722, 1-741, 1-753, 1-915, 1-921, 1-923, 1-930, 1-931, 1-1355, 1-1602, 1-1603, 1-1604, 2-3, 2-5, 2-9, 2-11, 2-13, 2-14, 2-20, 2-28, 2-29, 2-88, 2-351, 2-353, 2-355, 3-1, 3-2, 3-3, 3-5, and 3-7 exhibited a weed inhibition rate of 80% or higher against Scirpus juncoides.

Test Example 6

Herbicidal Effect Test by Application During Growth Period of Paddy Rice

Wagner pots with an area of 1/10,000 acre were filled with paddy soil, followed by addition of water and incorporation of chemical fertilizer (N:P:K = 16:16:16), after which puddling was performed. After that, seeds of Echinochloa crus-galli var. formosensis, broad-leaved weeds (Monochoria vaginalis), and Scirpus juncoides were sown at a depth of 0 cm to 1 cm, 30 seeds each. Immediately after sowing, the pots were flooded and a water depth of approximately 3 cm was maintained. Subsequent management was conducted in a glass greenhouse. Seven days after sowing, an emulsifiable concentrate prepared according to Formulation Example 5 using the compound shown below was diluted with water, and a specified amount of the diluted solution was applied by dropper. The amount of the active ingredient to be applied was equivalent to 7.5 g per 10 ares. The test was conducted in duplicate for each chemical concentration group, and the herbicidal effect was evaluated 14 days after chemical application using the same criteria as in Test Example 1.

As representative examples, the compounds of the above-mentioned compound Nos. 1-2, 1-5, 1-7, 1-8, 1-9, 1-10, 1-11, 1-12, 1-13, 1-14, 1-15, 1-16, 1-18, 1-19, 1-20, 1-21, 1-24, 1-30, 1-41, 1-68, 1-83, 1-92, 1-94, 1-111, 1-123, 1-126, 1-133, 1- 150, 1-151, 1-156, 1-183, 1-208, 1-213, 1-223, 1-313, 1-322, 1-525, 1-603, 1-612, 1-614, 1-652, 1-653, 1-658, 1-661, 1-662, 1-672, 1-674, 1-676, 1-680, 1-681, 1-682, 1-690, 1-691, 1-692, 1-693, 1-694, 1-703, 1-712, 1-713, 1-715, 1-717, 1-718, 1-721, 1-722, 1-723, 1-732, 1-734, 1-736, 1-740, 1-741, 1-747, 1-753, 1-756, 1-759, 1-762, 1-768, 1-777, 1-778, 1-779, 1-780, 1-785, 1-788, 1-789, 1-799, 1-801, 1-914, 1-915, 1-920, 1-921, 1-928, 1-929, 1-930, 1-931, 1-932, 1-935, 1-1119, 1-1292, 1-1295, 1-1298, 1-1304, 1-1313, 1-1314, 1-1315, 1-1320, 1-1323, 1-1324, 1-1334, 1-1342, 1-1343, 1-1349, 1-1355, 1-1357, 1-1358, 1-1361, 1-1379, 1-1381, 1-1386, 1-1389, 1-1400, 1-1402, 1-1408, 1-1409,

1-1415, 1-1421, 1-1424, 1-1427, 1-1430, 1-1436, 1-1445, 1-1446, 1-1447, 1-1448, 1-1456, 1-1457, 1-1555, 1-1598, 1-1600, 1-1602, 1-1603, 1-1604, 1-1610, 1-1611, 1-1612, 1-1613, 1-1614, 1-1615, 2-3, 2-9, 2-11, 2-13, 2-14, 2-16, 2-18, 2-20, 2-26, 2-28, 2-72, 2 -88, 2-191, 2-197, 2-349, 2-350, 2-351, 2-352, 2-353, 2-354, 2-355, 3-1, 3-2, 3-3, 3-5, 3-7, 3-177, 3-680, 3-693, 3-697, 3-752, 3-753, 3-754, 3-755, 3-756, and 3-757 exhibited a weed inhibition rate of 80% or higher against Echinochloa crus-galli var. formosensis.

As representative examples, the compounds of the above-mentioned compound Nos. 1-921, 1-930, 1-931, and 2-20 exhibited a weed inhibition rate of 80% or higher against Lindernia pyxidaria.

As representative examples, the compounds of the above-mentioned compound Nos. 1-2, 1-5, 1-7, 1-15, 1-20, 1-21, 1-24, 1-83, 1-92, 1-94, 1-133, 1-156, 1-183, 1-603, 1-672, 1-681, 1-690, 1-691, 1-694, 1-712, 1-713, 1-721, 1-722, 1-753, 1-921, 1-930, 1-931, 1-1355, 1-1600, 1-1602, 1-1604, 2-3, 2-9, 2-13, 2-28, 2-72, 2-88, 2-191, 2-197, 2-353, 2-355, 3-1, 3-2, and 3-5 exhibited a weed inhibition rate of 80% or higher against Monochoria vaginalis.

As representative examples, the compounds of the above-mentioned compound Nos. 1-2, 1-5, 1-15, 1-21, 1-24, 1-30, 1-41, 1-83, 1-151, 1-156, 1-183, 1-681, 1-691, 1-722, 1-921, 1-930, 1-931, 1-1355, 1-1602, 2-3, 2-9, 2-72, 2-88, 2-353, 2-355, 3-1, 3-2, 3-5, and 3-7 exhibited a weed inhibition rate of 80% or higher against Scirpus juncoides.

Test Example 7

Herbicidal Effect Test by Soil Application in Upland Field

Pots with an area of 36 $cm^2$ were filled with upland soil (alluvial loam), twenty seeds each of weeds of Digitaria ciliaris, and Echinochloa crus-galli were uniformly mixed with the top 1 cm layer of soil and the surface was lightly pressed down. One day after sowing, an emulsifiable concentrate prepared according to Formulation Example 5 using the compound shown below was diluted with water, and the diluted solution was sprayed onto the soil surface at a rate of 100 liters per 10 ares. The amount of the active ingredient to be applied was equivalent to 7.5 g per 10 ares. Fourteen days after chemical application, the herbicidal effect was evaluated according to the same criteria as in Test Example 1.

As representative examples, the compounds of the above-mentioned compound Nos. 1-2, 1-5, 1-7, 1-8, 1-9, 1-10, 1-11, 1-12, 1-13, 1-14, 1-15, 1-19, 1-20, 1-21, 1-24, 1-30, 1-41, 1-68, 1-83, 1-92, 1-94, 1-111, 1-123, 1-126, 1-133, 1-150, 1-151, 1-156, 1-183, 1-208, 1-213, 1-223, 1-525, 1-612, 1-614, 1-652, 1-653, 1-658, 1-661, 1-662, 1-680, 1-682, 1-690, 1-691, 1-692, 1-694, 1-712, 1-713, 1-718, 1-721, 1-722, 1-732, 1-734, 1-740, 1-741, 1-747, 1-753, 1-756, 1-759, 1-762, 1-768, 1-777, 1-778, 1-779, 1-780, 1-785, 1-788, 1-789, 1-799, 1-801, 1-921, 1-930, 1-931, 1-935, 1-1119, 1-1292, 1-1295, 1-1298, 1-1304, 1-1313, 1-1314, 1-1315, 1-1320, 1-1323, 1-1324, 1-1334, 1-1342, 1-1343, 1-1349, 1-1355, 1-1357, 1-1358, 1-1361, 1-1379, 1-1381, 1-1386, 1-1389, 1-1400, 1-1402, 1-1408, 1-1409, 1-1415, 1-1421, 1-1424, 1-1427, 1-1430, 1-1436, 1-1445, 1-1446, 1-1447, 1-1448, 1-1456, 1-1457, 1-1555, 1-1598, 1-1600, 1-1602, 1-1603, 1-1604, 1-1610, 1-1611, 1-1612, 1-1613, 1-1614, 1-1615, 2-3, 2-5, 2-9, 2-11, 2-13, 2-14, 2-16, 2-18, 2-20, 2-26, 2-28, 2-72, 2-88, 2-191, 2-197, 2-349, 2-353, 2-354, 2-355, 3-1, 3-2, 3-5, 3-7, 3-680, 3-693, 3-697, 3-752, 3-753, 3-754, 3-755, 3-756, and 3-757 exhibited a weed inhibition rate of 80% or higher against Digitaria ciliaris.

As representative examples, the compounds of the above-mentioned compound Nos. 1-2, 1-5, 1-7, 1-8, 1-9, 1-10, 1-11, 1-12, 1-13, 1-14, 1-15, 1-16, 1-19, 1-20, 1-21, 1-24, 1-30, 1-41, 1-68, 1-83, 1-92, 1-94, 1-111, 1-126, 1-133, 1-150, 1-151, 1-156, 1-183, 1-208, 1-213, 1-223, 1-612, 1-614, 1-652, 1-653, 1-658, 1-661, 1-662, 1-680, 1-681, 1-682, 1-690, 1-691, 1- 692, 1-693, 1-694, 1-703, 1-712, 1-713, 1-718, 1-721, 1-722, 1-732, 1-734, 1-740, 1-741, 1-747, 1-753, 1-756, 1-759, 1-762, 1-768, 1-777, 1-778, 1-779, 1-780, 1-785, 1-788, 1-789, 1-799, 1-801, 1-921, 1-930, 1-931, 1-1119, 1-1292, 1-1295, 1-1298, 1-1304, 1-1313, 1-1314, 1-1315, 1-1320, 1-1323, 1-1324, 1-1334, 1-1342, 1-1343, 1-1349, 1-1355, 1-1357, 1-1358, 1-1361, 1-1379, 1-1381, 1-1386, 1-1389, 1-1400, 1-1402, 1-1408, 1-1409, 1-1415, 1-1421, 1-1424, 1-1427, 1-1430, 1-1436, 1-1445, 1-1446, 1-1447, 1-1448, 1-1456, 1-1457, 1-1555, 1-1598, 1-1600, 1-1602, 1-1603, 1-1604, 1-1610, 1-1611, 1-1612, 1-1613, 1-1614, 1-1615, 2-3, 2-5, 2-9, 2-11, 2-13, 2-14, 2-16, 2-18, 2-20, 2-26, 2-28, 2-29, 2-72, 2-88, 2-191, 2-197, 2-349, 2-351, 2-353, 2-354, 2-355, 3-1, 3-2, 3-5, 3-7, 3-680, 3-693, 3-697, 3-752, 3-753, 3-754, 3-755, 3-756, and 3-757 exhibited a weed inhibition rate of 80% or higher against Echinochloa crus-galli.

As representative examples, the compounds of the above-mentioned compound Nos. 1-7, 1-921, 1-930, 2-72, and 2-88 exhibited a weed inhibition rate of 80% or higher against Chenopodium album.

As representative examples, the compounds of the above-mentioned compound Nos. 1-7, 1-68, 1-694, 1-753, 1-921, 1-930, 1-931, 2-29, 2-72, 2-88, and 3-2 exhibited a weed inhibition rate of 80% or higher against Amaranthus viridis.

Test Example 8

Herbicidal Effect Test by Stem and Leaf Treatment in Upland Field

Pots with an area of 36 $cm^2$ were filled with upland soil (alluvial loam), twenty seeds each of weeds of Digitaria ciliaris, and Echinochloa crus-galli were uniformly mixed with the top 1 cm layer of soil and the surface was lightly pressed

down. Seven days after sowing, an emulsifiable concentrate prepared according to Formulation Example 5 using the compound shown below was diluted with water, and the diluted solution was sprayed onto the soil surface at a rate of 100 liters per 10 ares. The amount of the active ingredient to be applied was equivalent to 7.5 g per 10 ares. Fourteen days after chemical application, the herbicidal effect was evaluated according to the same criteria as in Test Example 1.

As representative examples, the compounds of the above-mentioned compound Nos. 1-2, 1-5, 1-7, 1-8, 1-9, 1-10, 1-11, 1-12, 1-13, 1-14, 1-15, 1-16, 1-18, 1-19, 1-20, 1-21, 1-24, 1-30, 1-41, 1-83, 1-92, 1-94, 1-111, 1-123, 1-126, 1-150, 1-151, 1-156, 1-183, 1-322, 1-525, 1-612, 1-614, 1-652, 1-653, 1-658, 1-661, 1-662, 1-672, 1-681, 1-682, 1-686, 1-690, 1-691, 1-700, 1-703, 1-706, 1-709, 1-713, 1-717, 1-718, 1-721, 1-732, 1-734, 1-740, 1-741, 1-747, 1-756, 1-759, 1-762, 1-768, 1-777, 1-778, 1-779, 1-780, 1-785, 1-788, 1-789, 1-799, 1-801, 1-915, 1-921, 1-929, 1-930, 1-931, 1-1119, 1-1221, 1-1292, 1-1295, 1-1298, 1-1304, 1-1313, 1-1314, 1-1315, 1-1320, 1-1323, 1-1324, 1-1334, 1-1342, 1-1343, 1-1349, 1-1355, 1-1357, 1-1358, 1-1361, 1-1379, 1-1381, 1-1386, 1-1389, 1-1400, 1-1402, 1-1408, 1-1409, 1-1415, 1-1421, 1-1424, 1-1427, 1-1430, 1-1436, 1-1445, 1-1446, 1-1447, 1-1448, 1-1456, 1-1457, 1-1555, 1-1598, 1-1600, 1-1602, 1-1603, 1-1604, 1-1610, 1-1611, 1-1612, 1-1613, 1-1614, 1-1615, 2-3, 2-5, 2-9, 2-11, 2-13, 2-14, 2-16, 2-18, 2-20, 2-26, 2-28, 2-72, 2-88, 2-191, 2-197, 2-349, 2-351, 2-353, 2-355, 3-1, 3-5, 3-7, 3-680, 3-693, 3-697, 3-752, 3-753, 3-754, 3-755, 3-756, and 3-757 exhibited a weed inhibition rate of 80% or higher against Digitaria ciliaris.

As representative examples, the compounds of the above-mentioned compound Nos. 1-2, 1-5, 1-7, 1-8, 1-9, 1-10, 1-11, 1-12, 1-13, 1-14, 1-15, 1-16, 1-17, 1-18, 1-19, 1-20, 1-21, 1-24, 1-30, 1-41, 1-68, 1-83, 1-92, 1-94, 1-111, 1-123, 1 -126, 1-133, 1-150, 1-151, 1-156, 1-183, 1-208, 1-213, 1-223, 1-322, 1-525, 1-612, 1-614, 1-652, 1-653, 1-658, 1-661, 1-662, 1-672, 1-680, 1-681, 1-682, 1-686, 1-690, 1-691, 1-693, 1-694, 1-706, 1-709, 1-712, 1-713, 1-717, 1-718, 1-721, 1-722, 1-732, 1-734, 1-740, 1-741, 1-747, 1-753, 1-756, 1-759, 1-762, 1-768, 1-777, 1-778, 1-779, 1-780, 1-785, 1-788, 1-789, 1-799, 1-801, 1-915, 1-921, 1-930, 1-931, 1-934, 1-1028, 1-1119, 1-1222, 1-1292, 1-1295, 1-1298, 1-1304, 1-1313, 1-1314, 1-1315, 1-1320, 1-1323, 1-1324, 1-1334, 1-1342, 1-1343, 1-1349, 1-1355, 1-1357, 1-1358, 1-1361, 1-1379, 1-1381, 1-1386, 1-1389, 1-1400, 1-1402, 1-1408, 1-1409, 1-1415, 1-1421, 1-1424, 1-1427, 1-1430, 1-1436, 1-1445, 1-1446, 1-1447, 1-1448, 1-1456, 1-1457, 1-1555, 1-1598, 1-1600, 1-1602, 1-1603, 1-1604, 1-1610, 1-1611, 1-1612, 1-1613, 1-1614, 1-1615, 2-3, 2-5, 2-9, 2-11, 2-13, 2-14, 2-16, 2-18, 2-20, 2-28, 2-29, 2-72, 2-88, 2-191, 2-197, 2-349, 2-350, 2-351, 2-353, 2-355, 3-1, 3-2, 3-5, 3-7, 3-680, 3-693, 3-697, 3-752, 3-753, 3-754, 3-755, 3-756, and 3-757 exhibited a weed inhibition rate of 80% or higher against Echinochloa crus-galli.

As representative examples, the compounds of the above-mentioned compound Nos. 1-2, 1-92, 1-921, 1-930, 2-72, 2-88, and 3-5 exhibited a weed inhibition rate of 80% or higher against Chenopodium album.

As representative examples, the compounds of the above-mentioned compound Nos. 1-2, 1-921, 1-930, and 1-931 exhibited a weed inhibition rate of 80% or higher against Amaranthus viridis.

Comparative Example

The inventors of the present invention have found that the compound according to the present invention is clearly superior to structurally similar compounds known from the related art. As an example, the herbicidal effect on Echinochloa crus-galli var. formosensis at the three-leaf stage was confirmed by the following method, and the results are specifically shown in Table 7 as a comparative example.

Herbicidal Effect Test by Application During Growth Period of Paddy Rice

Wagner pots with an area of 1/10,000 acre were filled with paddy soil, followed by addition of water and incorporation of chemical fertilizer (N:P:K = 16:16:16), after which puddling was performed. After that, 30 seeds of Echinochloa crus-galli var. formosensis were sown at a depth of 0 cm to 1 cm. Immediately after sowing, the pots were flooded and a water depth of approximately 3 cm was maintained. Subsequent management was conducted in a glass greenhouse. When Echinochloa crus-galli var. formosensis reached the three-leaf stage, an emulsifiable concentrate prepared according to Formulation Example 5 using the compound shown below was diluted with water, and a specified amount of the diluted solution was applied by dropper. The amount of the active ingredient to be applied was equivalent to 7.5 g per 10 ares. The test was conducted in triplicate for each chemical concentration group, and the weed inhibition rate (%) was determined 21 days after chemical application using the following formula (Math. 1).

[Table 124]

| Table 7 | |
|---|---|
| Compound | Weed inhibition rate (%) |
| 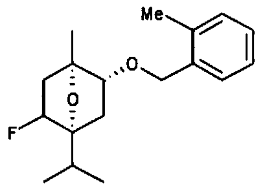 Present Invention No. 1-2 | 94% |
| 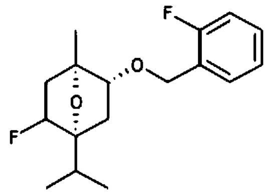 Present Invention No. 1-5 | 100% |
| 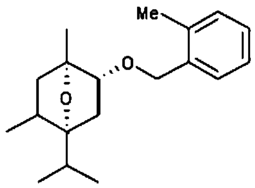 Present Invention No. 1-83 | 99% |
| 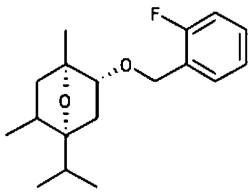 Present Invention No. 1-92 | 100% |
| 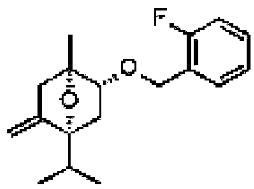 Present Invention No. 2-88 | 80% |
| 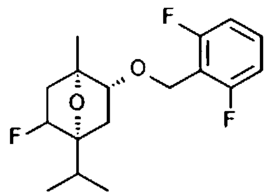 Present Invention No. 1-7 | 100% |

(continued)

| Table 7 | |
|---|---|
| Compound | Weed inhibition rate (%) |
| 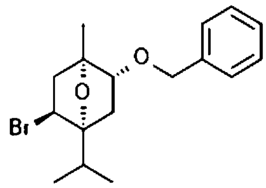 Present Invention No. 1-15 | 80% |

[Table 125]

| Table 7-Continued | |
|---|---|
| Compound | Weed inhibition rate (%) |
| 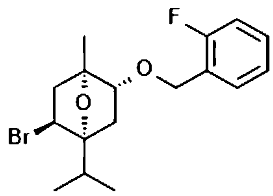 Present Invention No. 1-19 | 88% |
| 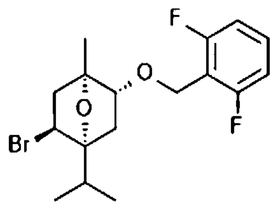 Present Invention No. 1-21 | 93% |
| 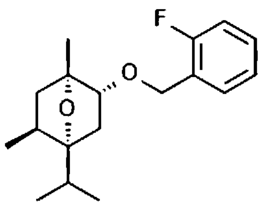 Present Invention No. 1-93 | 100% |
| 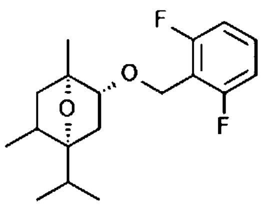 Present Invention No. 1-111 | 100% |
| 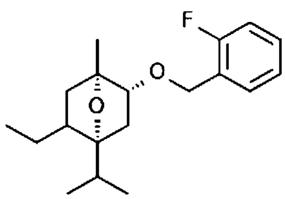 Present Invention No. 1-126 | 100% |

(continued)

| Table 7-Continued | |
|---|---|
| Compound | Weed inhibition rate (%) |
| 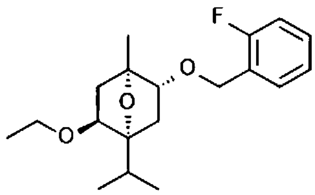 Present Invention No. 1-183 | 93% |
| 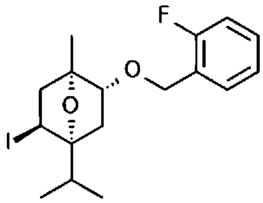 Present Invention No. 1-753 | 93% |

[Table 126]

| Table 7-Continued | |
|---|---|
| Compound | Weed inhibition rate (%) |
| 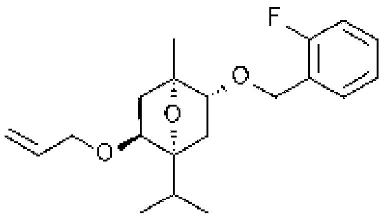 Present Invention No. 1-1602 | 82% |
| 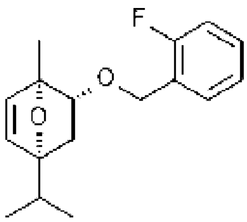 Present Invention No. 3-5 | 82% |
| 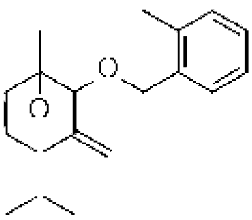 Compound No. 13a in Journal of Pesticide Science, Vol. 48, 2023, pp. 11-16 | 20% |

(continued)

| Table 7-Continued | |
|---|---|
| Compound | Weed inhibition rate (%) |
| 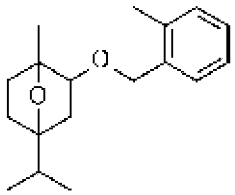 Compound No. 5 in Journal of Agricultural and Food Chemistry, Vol. 58, 2010, pp. 10147-10155 and No. 39 in EP0081893A | 73% |

INDUSTRIAL APPLICABILITY

According to the present invention, a novel 1,4-cineole derivative having excellent herbicidal activity, a synthetic intermediate thereof, and a herbicide containing the 1,4-cineole derivative can be provided.

According to the present invention, a method for using a herbicide and a method for preparing an agrochemical composition can be provided.

Although the present invention has been described in detail with reference to specific embodiments, it is apparent to those skilled in the art that various changes and modifications can be made without departing from the spirit and scope of the present invention.

The present application is based on a Japanese patent application No.2023-076081 filed on May 2, 2023 and a Japanese patent application No.2024-057466 filed on March 29, 2024, the disclosure of which is hereby incorporated by reference herein in its entirety.

## Claims

**1.** A 1,4-cineole derivative represented by the following general formula (1), (1'), (2), (2'), (3) or (3'):

[Chem. 1]

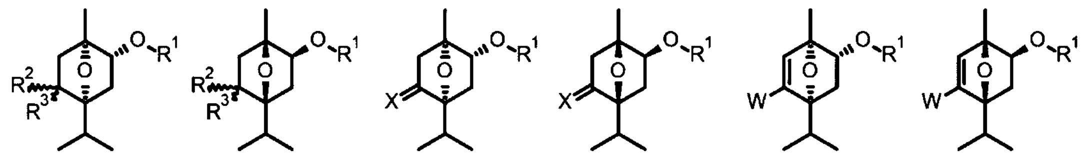

**(1)** **(1')** **(2)** **(2')** **(3)** **(3')**

(in the general formulae (1), (1'), (2), (2'), (3), and (3'),

$R^1$'s each independently represent a C1-C6 alkyl group, a C1-C6 haloalkyl group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a benzoyl C1-C6 alkyl group (which may be mono-substituted or poly-substituted with a halogen atom, a C1-C6 alkyl group, or a C1-C6 haloalkyl group), or a C1-C6 alkoxy C1-C6 alkoxy C1-C6 alkyl group,

$R^{2}$'s and $R^{3}$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyl group, a C2-C6 alkenyloxy group, a C2-C6 alkynyl group, a C2-C6 alkynyloxy group, a C1-C6 alkylcarbonyloxy group, a C1-C6 alkoxycarbonyloxy group, a C1-C6 alkylthiocarbonyloxy group, a C1-C6 alkylthiothiocarbonyloxy group, a C1-C6 alkylsulfonyloxy group, a C1-C6 haloalkylsulfonyloxy group, a C3-C6 cycloalkylsulfonyloxy group, an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), an aminosulfonyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), and at least one of $R^2$ and $R^3$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

X's each independently represent an oxygen atom, a sulfur atom, $CR^4R^5$ or $NR^6$,

$R^4$ and $R^5$ each independently represent a hydrogen atom or a C1-C6 alkyl group,

$R^6$ represents a hydrogen atom, a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and

W's each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkoxy group, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be

mono-substituted or poly-substituted with a halogen atom, a C1-C6 alkyl group, or a C1-C6 haloalkyl group), a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkylsulfonyloxy group, a C1-C6 haloalkylsulfonyloxy group, a C3-C6 cycloalkylsulfonyloxy group, or an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group)).

**2.** The 1,4-cineole derivative according to claim 1, wherein

$R^1$'s each independently represent a C1-C6 alkyl group, a C1-C6 haloalkyl group, a C2-C6 alkenyl group, a C2-C6 alkynyl group, C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a benzoyl C1-C6 alkyl group (which may be mono-substituted or poly-substituted with a halogen atom, a C1-C6 alkyl group, or a C1-C6 haloalkyl group), or a C1-C6 alkoxy C1-C6 alkoxy C1-C6 alkyl group,
$R^2$'s and $R^3$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyloxy group, a C2-C6 alkynyloxy group, a C1-C6 alkylcarbonyloxy group, a C1-C6 alkylthiothiocarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), at least one of $R^2$ and $R^3$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,
X's each independently represent an oxygen atom, a sulfur atom, $CR^4R^5$, or $NR^6$,
$R^4$ and $R^5$ each independently represent a hydrogen atom or a C1-C6 alkyl group,
$R^6$ represents a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and
W's each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or

a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C1-C6 alkylsulfonyloxy group, or a C1-C6 haloalkylsulfonyloxy group.

**3.** The 1,4-cineole derivative according to claim 1, wherein

$R^1$'s each independently represent a C1-C6 alkyl group, a C1-C6 haloalkyl group, a C2-C6 alkenyl group, a C2-C6 alkynyl group, a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkyl group, an aryl group (which may be mono-substituted or poly-substituted with a halogen atom, a C1-C6 alkyl group, or a C1-C6 haloalkyl group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, a C1-C6 haloalkoxy group, a carboxy group, or a C1-C6 alkoxycarbonyl group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), or a C1-C6 alkoxy C1-C6 alkoxy C1-C6 alkyl group,

$R^2$'s and $R^3$'s each independently represent a hydrogen atom, a halogen atom, a hydroxyl group, a C1-C6 alkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a C2-C6 alkenyloxy group, a C2-C6 alkynyloxy group, a C1-C6 alkylcarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), at least one of $R^2$ and $R^3$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

X's each independently represent an oxygen atom, $CR^4R^5$, or $NR^6$,

$R^4$ and $R^5$ each independently represent a hydrogen atom,

$R^6$ represents a hydroxyl group, and

W's each independently represent a hydrogen atom, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), or a C1-C6 haloalkylsulfonyloxy group.

**4.** A synthetic intermediate represented by the following general formula (la), (1a'), (2a), (2a'), (3a) or (3a'), which is useful in producing the 1,4-cineole derivative represented by the general formula (1), (1'), (2), (2'), (3) or (3') according to any one of claims 1 to 3:

[Chem. 2]

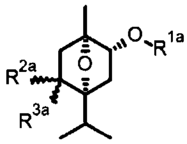
(1a)

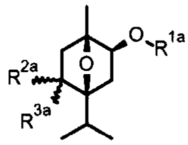
(1a')

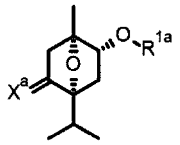
(2a)

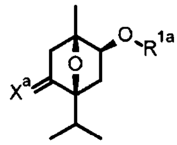
(2a')

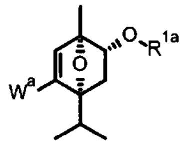
(3a)

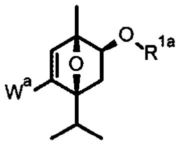
(3a')

(in the general formulae (1a), (1a'), (2a), (2a'), (3a), and (3a'),

$R^{1a}$'s each independently represent a hydrogen atom or a tri-C1-C6 alkylsilyl group which may be the same or different,

$R^{2a}$'s and $R^{3a}$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyl group, a C2-C6 alkenyloxy group, a C2-C6 alkynyl group, a C2-C6 alkynyloxy group, a C1-C6 alkylcarbonyloxy group, a C1-C6 alkoxycarbonyloxy group, a C1-C6 alkylthiocarbonyloxy group, a C1-C6 alkylthiothiocarbonyloxy group, a C1-C6 alkylsulfonyloxy group, a C1-C6 haloalkylsulfonyloxy group, a C3-C6 cycloalkylsulfonyloxy group, an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), an aminosulfonyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), or a carbamoyloxy group (which may be mono-substituted or poly-substituted with a C1-C6 alkyl group, a C3-C6 cycloalkyl group, or an aryl group, and the C1-C6 alkyl groups may also be bonded to each other via an alkylene group to form a 3-membered ring, a 4-membered ring, a 5-membered ring, or a 6-membered ring), and at least one of $R^2$ and $R^3$ is a substituent other than a hydrogen atom, the two adjacent substituents, $R^{2a}$ and $R^{3a}$, together with a carbon atom to which $R^{2a}$ and $R^{3a}$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,

$X^a$'s each independently represent an oxygen atom, a sulfur atom, $CR^{4a}R^{5a}$ or $NR^{6a}$,

$R^{4a}$ and $R^{5a}$ each independently represent a hydrogen atom or a C1-C6 alkyl group,

$R^{6a}$ represents a hydrogen atom, a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and

$W^a$'s each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C1-C6 alkoxy C1-C6 alkoxy group, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy C1-C6 alkyl group (which may be mono-substituted or poly-substituted with a halogen atom, a C1-C6 alkyl group, or a C1-C6 haloalkyl group), a C7-C11 aralkyloxy C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkylsulfonyloxy group, a C1-C6 haloalkylsulfonyloxy group, a C3-C6 cycloalkylsulfonyloxy group, or an arylsulfonyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group)).

**5.** The synthetic intermediate according to claim 4, wherein $R^{1a}$'s each independently represent a hydrogen atom or a tri-C1-C6 alkylsilyl group which may be same or different,

$R^{2a}$'s and $R^{3a}$'s each independently represent a hydrogen atom, a halogen atom, a hydroxyl group, a C1-C6 alkyl group, a C1-C6 haloalkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a C2-C6 alkenyloxy group, a C2-C6 alkynyloxy group, a C1-C6 alkylcarbonyloxy group, a C1-C6 alkylthiothiocarbonyloxy group, a C1-C6 haloalkylsulfonyloxy group, or a carbamoyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), at least one of $R^{2a}$ and $R^{3a}$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,
$X^a$'s each independently represent an oxygen atom, a sulfur atom, $CR^4R^5$, or $NR^6$,
$R^{4a}$ and $R^{5a}$ each independently represent a hydrogen atom or a C1-C6 alkyl group,
$R^{6a}$ represents a hydroxyl group, a C1-C6 alkyl group, a C1-C6 alkoxy group, an amino group, a C1-C6 alkylamino group, an aminocarbonylamino group, or a phenylamino group (which may be mono-substituted or poly-substituted with a halogen atom, a C1-C6 alkyl group, or a C1-C6 haloalkyl group), and
$W^a$'s each independently represent a hydrogen atom, a halogen atom, a C1-C6 alkyl group, a C1-C6 haloalkyl group, a C1-C6 alkoxy group, a C1-C6 haloalkoxy group, a C2-C6 alkenyl group, a C2-C6 alkynyl group, a C3-C6 cycloalkyl group (which may be mono-substituted or poly-substituted with a halogen atom, a C1-C6 alkyl group, or

a C1-C6 haloalkyl group), a C3-C6 cycloalkyl C1-C6 alkyl group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkyl group (which may be mono-substituted or poly-substituted with a halogen atom, a C1-C6 alkyl group, or a C1-C6 haloalkyl group), a phenoxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic oxy group (which may be mono-substituted or poly-substituted with a halogen atom, a C1-C6 alkyl group, or a C1-C6 haloalkyl group), a C1-C6 alkylsulfonyloxy group, or a C1-C6 haloalkylsulfonyloxy group.

**6.** The synthetic intermediate according to claim 4, wherein $R^{1a}$'s each independently represent a hydrogen atom or a tri-C1-C6 alkylsilyl group which may be same or different,

$R^{2a}$'s and $R^{3a}$'s each independently represent a hydrogen atom, a halogen atom, a hydroxyl group, a C1-C6 alkyl group, a C1-C6 alkoxy group, a C1-C6 haloalkoxy group, a C7-C11 aralkyloxy group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic C1-C6 alkoxy group (which may be mono-substituted or poly-substituted with a halogen atom, a C1-C6 alkyl group, or a C1-C6 haloalkyl group), a C1-C6 alkoxy C1-C6 alkoxy group, a C3-C6 cycloalkyl group (which may be mono-substituted or poly-substituted with a halogen atom, a C1-C6 alkyl group, or a C1-C6 haloalkyl group), a C3-C6 cycloalkyl C1-C6 alkoxy group (which may be mono-substituted or poly-substituted with a halogen atom, a C1-C6 alkyl group, or a C1-C6 haloalkyl group), an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a heterocyclic ring (which may be mono-substituted or poly-substituted with a halogen atom, a C1-C6 alkyl group, or a C1-C6 haloalkyl group), a C7-C11 aralkyl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), a C2-C6 alkenyloxy group, a C2-C6 alkynyloxy group, a C1-C6 alkylcarbonyloxy group, a C1-C6 haloalkylsulfonyloxy group or a carbamoyloxy group (which may be mono-substituted or poly-substituted with a C1-C6 alkyl group, a C3-C6 cycloalkyl group, or an aryl group, and the C1-C6 alkyl groups may be bonded to each other via an alkylene group to form a 3-membered ring, a 4-membered ring, a 5-membered ring, or a 6-membered ring), at least one of $R^{2a}$ and $R^{3a}$ is a substituent other than a hydrogen atom, the two adjacent substituents, $R^2$ and $R^3$, together with a carbon atom to which $R^2$ and $R^3$ are bonded, may form a 3-membered to 6-membered carbocyclic ring or a 3-membered to 6-membered heterocyclic ring having 1 to 4 heteroatoms independently selected from an oxygen atom, a sulfur atom and a nitrogen atom, and the ring formed here may have one or more substituents,
$X^a$'s each independently represent an oxygen atom, $CR^4R^5$, or $NR^6$,
$R^{4a}$ and $R^{5a}$ each independently represent a hydrogen atom,
$R^{6a}$ represents a hydroxyl group, and
$W^a$'s each independently represent a hydrogen atom, an aryl group (which may be mono-substituted or poly-substituted with a halogen atom, a cyano group, a nitro group, a C1-C6 alkyl group, a C1-C6 haloalkyl group, a C1-C6 alkoxy group, or a C1-C6 haloalkoxy group), or a C1-C6 haloalkylsulfonyloxy group.

**7.** A herbicide comprising:
the 1,4-cineole derivative according to any one of claims 1 to 3 as an active ingredient.

**8.** The herbicide according to claim 7, which is for use in agricultural land, pastureland, lawn, or non-agricultural land.

**9.** A method for using the herbicide according to claim 7, the method comprising:
applying an effective amount of the 1,4-cineole derivative to at least one selected from a stem and leaf of a weed, soil, and a water surface.

**10.** A method for preparing an agrochemical composition, the method comprising:
a step of mixing the herbicide according to claim 7 with at least one selected from a diluent and a surfactant.

# INTERNATIONAL SEARCH REPORT

International application No.
**PCT/JP2024/016782**

**A. CLASSIFICATION OF SUBJECT MATTER**

***C07D 493/08***(2006.01)i; ***A01N 43/90***(2006.01)i; ***A01P 13/00***(2006.01)i
FI: C07D493/08 CSP; A01P13/00; A01N43/90 101

According to International Patent Classification (IPC) or to both national classification and IPC

**B. FIELDS SEARCHED**

Minimum documentation searched (classification system followed by classification symbols)

C07D493/08; A01N43/90; A01P13/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | US 4670041 A (E. I. DU PONT DE NEMOURS AND COMPANY) 02 June 1987 (1987-06-02)<br>table I, claim 17 | 1-10 |
| X | US 4554366 A (SHELL OIL COMPANY) 19 November 1985 (1985-11-19)<br>table I, columns 4, 5 | 1-10 |
| X | US 4487945 A (SHELL OIL COMPANY) 11 December 1984 (1984-12-11)<br>examples 45, 46, claim 2 | 4-6 |
| A | US 4567283 A (SHELL OIL COMPANY) 28 January 1986 (1986-01-28)<br>entire text | 1-10 |
| A | US 4542244 A (SHELL OIL COMPANY) 17 September 1985 (1985-09-17)<br>entire text | 1-10 |

☐ Further documents are listed in the continuation of Box C.
☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 July 2024** | **23 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/016782**

| Patent document cited in search report | Publication date (day/month/year) | Patent family member(s) | Publication date (day/month/year) |
|---|---|---|---|
| US 4670041 A | 02 June 1987 | JP 58-110591 A | |
| | | US 4542244 A | |
| | | US 4554366 A | |
| | | US 4567283 A | |
| | | EP 81893 A2 | |
| | | KR 10-1989-0000177 B1 | |
| US 4554366 A | 19 November 1985 | JP 58-110591 A | |
| | | US 4670041 A | |
| | | US 4542244 A | |
| | | US 4567283 A | |
| | | EP 81893 A2 | |
| | | KR 10-1989-0000177 B1 | |
| US 4487945 A | 11 December 1984 | (Family: none) | |
| US 4567283 A | 28 January 1986 | JP 58-110591 A | |
| | | US 4670041 A | |
| | | US 4542244 A | |
| | | US 4554366 A | |
| | | EP 81893 A2 | |
| | | KR 10-1989-0000177 B1 | |
| US 4542244 A | 17 September 1985 | JP 58-110591 A | |
| | | US 4567283 A | |
| | | US 4670041 A | |
| | | US 4554366 A | |
| | | EP 81893 A2 | |
| | | KR 10-1989-0000177 B1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0081893 A
- WO 2008008763 A
- JP 2012002571 A
- WO 2008096398 A
- JP 2023076081 A
- JP 2024057466 A

### Non-patent literature cited in the description

- *Journal of Agricultural and Food Chemistry*, 2010, vol. 58, 10147-10155
- *Journal of Pesticide Science*, 2023, vol. 48, 11-16
- Pesticide Formulation Guide. Japan Plant Protection Association, 1997
- *Tetrahedron*, 1995, vol. 51, 1345-1376
- *Organic Letter*, 2006, vol. 8, 609-612